# EUROPEAN PATENT APPLICATION

(11) **EP 2 865 689 A1**
(43) Date of publication of application: **29.04.2015**
(21) Application number: 14195219.2
(22) Date of filing: 08.12.2009
(51) Int. Cl.: C07K 16/30, C12Q 1/68, C07K 14/47, A61K 38/00

(54) **FAM26F polypeptides and polynucleotides, and uses thereof as a drug target for producing drugs and biologics**

(30) Priority: 08.12.2008 US 120540 P
(62) Divisional of application: 09799402.4
(71) Applicant: Compugen Ltd., 69512 Tel Aviv (IL)
(72) Inventor: Levine, Zurit, 46725 Herzliya (IL); Rosenberg, Avi Yeshah, 44622 Kfar Saba (IL); Rotman, Galit, 46725 Herzliya (IL); Toporik, Amir, 58847 Holon (IL); Montia, Eve, 76328 Rehovot (IL); Cohen-Dayag, Anat, 76302 Rehovot (IL); Walach, Shira, 61130 Hod Hasharon (IL); Sameach-Greenwald, Shirley, 61130 Kfar Saba (IL); Dassa, Liat, 56478 Yahud (IL); Beiman, Merav, 70450 Nes Tsiyona (IL); Levy, Ofer, 99770 D.N. Shimshon (IL); Novik, Amit, 30500 Binyamina (IL); Kinar, Yaron, 6326447 Tel Aviv (IL); Nemzar, Sergey, 43352 Ranaana (IL)
(74) Representative: Fuchs Patentanwälte Partnerschaft mbB

(57) **Abstract**

This invention relates to a novel target for production of immune and non-immune based therapeutics and for disease diagnosis. More particularly, the invention provides therapeutic antibodies against FAM26F antigens, which are differentially expressed in cancer, and diagnostic and therapeutic usages. This invention further relates to extracellular domains of FAM26F, proteins and variants, and therapeutic usages thereof.

SEQUENCE LISTING

<110>  Compugen Ltd

<120>  POLYPEPTIDES AND POLYNUCLEOTIDES, AND USES THEREOF AS A DRUG
 TARGET FOR PRODUCING DRUGS AND BIOLOGICS

<130>  0000

<160>  201

<210>  1
<211>  916
<212>  DNA
<213>  Homo sapiens

<400> 1

gggacggagg cggggcccag gttggggcgg ggccgggccc aggtacagcg gccctgcggc      60

tggcgcggcg gacgggatga ggcgctgcag tctctgcgct ttcgacgccg cccgggggcc     120

caggcggctg atgcgtgtgg gcctcgcgct gatcttggtg ggccacgtga acctgctgct     180

gggggccgtg ctgcatggca ccgtcctgcg gcacgtggcc aatccccgcg gcgctgtcac     240

gccggagtac accgtagcca atgtcatctc tgtcggctcg gggctgctga gcgtttccgt     300

gggacttgtg gccctcctgg cgtccaggaa ccttcttcgc cctccactgc actgggtcct     360

gctggcacta gctctggtga acctgctctt gtccgttgcc tgctccctgg gcctccttct     420

tgctgtgtca ctcactgtgg ccaacggtgg ccgccgcctt attgctgact gccacccagg     480

actgctggat cctctggtac cactggatga ggggccggga catactgact gcccctttga     540

ccccacaaga atctatgata cagccttggc tctctggatc ccttctttgc tcatgtctgc     600

aggggaggct gctctatctg gttactgctg tgtggctgca ctcactctac gtggagttgg     660

gccctgcagg aaggacggac ttcaggggca gctagaggaa atgacagagc ttgaatctcc     720

taaatgtaaa aggcaggaaa atgagcagct actggatcaa aatcaagaaa tccgggcatc     780

acagagaagt tgggtttagg acagcaggtg ctgttccgag actcagtcct aaagggtttt     840

ttttcccact aagcaagggg ccctgacctc gggatgagat aacaaattgt aataaagtaa     900

cttctctttt cttcta                                                     916

<210>  2
<211>  78
<212>  DNA
<213>  Artificial

<220>
<223>  Synthetic polynucleotide

<400> 2

gtgagcgcgg caggcgaccc gggcgggggc cgggctcccg gagagcccag caggccaaag      60

gctttgtgtc ttccacag                                                    78

<210>  3
<211>  994
<212>  DNA
<213>  Homo sapiens

<400>  3

gggacggagg cggggcccag gttggggcgg ggccgggccc aggtacagcg gccctgcggc      60

tggcgcggcg gacgggatga ggcgctgcag tctctgcgct ttcgacgccg cccgggggcc     120

caggcggctg atgcgtgtgg gcctcgcgct gatcttggtg ggccacgtga acctgctgct     180

gggggccgtg ctgcatggca ccgtcctgcg gcacgtggcc aatccccgcg gcgctgtcac     240

gccggagtac accgtagcca atgtcatctc tgtcggctcg gggctgctgg tgagcgcggc     300

aggcgacccg ggcgggggcc gggctcccgg agagcccagc aggccaaagg ctttgtgtct     360

tccacagagc gtttccgtgg gacttgtggc cctcctggcg tccaggaacc ttcttcgccc     420

tccactgcac tgggtcctgc tggcactagc tctggtgaac ctgctcttgt ccgttgcctg     480

ctccctgggc ctccttcttg ctgtgtcact cactgtggcc aacggtggcc gccgccttat     540

tgctgactgc cacccaggac tgctggatcc tctggtacca ctggatgagg ggccgggaca     600

tactgactgc ccctttgacc ccacaagaat ctatgataca gccttggctc tctggatccc     660

ttctttgctc atgtctgcag gggaggctgc tctatctggt tactgctgtg tggctgcact     720

cactctacgt ggagttgggc cctgcaggaa ggacggactt caggggcagc tagaggaaat     780

gacagagctt gaatctccta aatgtaaaag gcaggaaaat gagcagctac tggatcaaaa     840

tcaagaaatc cgggcatcac agagaagttg ggtttaggac agcaggtgct gttccgagac     900

tcagtcctaa agggtttttt ttcccactaa gcaaggggcc ctgacctcgg gatgagataa     960

caaattgtaa taaagtaact tctcttttct tcta                                 994

<210>  4
<211>  1332
<212>  DNA
<213>  Homo sapiens

<400>  4

gggacggagg cggggcccag gttggggcgg ggccgggccc aggtacagcg gccctgcggc      60

tggcgcggcg gacgggatga ggcgctgcag tctctgcgct ttcgacgccg cccgggggcc     120

caggcggctg atgcgtgtgg gcctcgcgct gatcttggtg ggccacgtga acctgctgct     180

gggggccgtg ctgcatggca ccgtcctgcg gcacgtggcc aatccccgcg gcgctgtcac     240

gccggagtac accgtagcca atgtcatctc tgtcggctcg gggctgctga gcgtttccgt     300

gggacttgtg gccctcctgg cgtccaggaa ccttcttcgc cctccactgc actgggtcct 360

gctggcacta gctctggtga acctgctctt gtccgttgcc tgctccctgg gcctccttct 420

tgctgtgtca ctcactgtgg ccaacggtgg ccgccgcctt attgctgact gccacccagg 480

actgctggat cctctggtac cactggatga ggggccggga catactgact gcccctttga 540

ccccacaaga atctatgata cagccttggc tctctggatc ccttctttgc tcatgtctgc 600

aggggaggct gctctatctg gttactgctg tgtggctgca ctcactctac gtggagttgg 660

gccctgcagg aaggacggac ttcaggggca ggtaaggaag gcaaacagga agggttcatt 720

ccacagagac tggctgtgtt gaaaagatgc tgaccggccg ggtgcggagg ctcacacctg 780

taatcccagc actttgggag gctgaggcag gcggatcact tgaggtcagg agtttgagac 840

cagcttggac aacatggtga aacctcgtct ctactaaaaa tacaaaaaat taggtggggt 900

ggtgggcgcc tgtaatccca gctacttgga aggctgaggc aggagaatcg cttgaacccg 960

gaaggcggag gttgcagtga accgagatca cgccactgca ctccagcctg ggcaacagag 1020

tgaggctccg tcacaaaaaa caaaacaaaa aaaagatgct gacctatcat tcagggcgag 1080

ggtgtctaac tctatcttct tctgcagcta gaggaaatga cagagcttga atctcctaaa 1140

tgtaaaaggc aggaaaatga gcagctactg gatcaaaatc aagaaatccg ggcatcacag 1200

agaagttggg tttaggacag caggtgctgt tccgagactc agtcctaaag ggtttttttt 1260

cccactaagc aaggggccct gacctcggga tgagataaca aattgtaata aagtaacttc 1320

tcttttcttc ta 1332

<210> 5
<211> 871
<212> DNA
<213> Homo sapiens

<400> 5

gggacggagg cggggcccag gttggggcgg ggccgggccc aggtacagcg gccctgcggc 60

tggcgcggcg gacgggatga ggcgctgcag tctctgcgct ttcgacgccg cccgggggcc 120

caggcggctg atgcgtgtgg gcctcgcgct gatcttggtg ggccacgtga acctgctgct 180

gggggccgtg ctgcatggca ccgtcctgcg gcacgtggcc aatccccgcg gcgctgtcac 240

gccggagtac accgtagcca atgtcatctc tgtcggctcg gggctgctga gcgtttccgt 300

gggacttgtg gccctcctgg cgtccaggaa ccttcttcgc cctccactgc actgggtcct 360

gctggcacta gctctggtga acctgctctt gtccgttgcc tgctccctgg gcctccttct 420

tgctgtgtca ctcactgtgg ccaacggtgg ccgccgcctt attgctgact gccacccagg 480

actgctggat cctctggtac cactggatga ggggccggga catactgact gcccctttga 540

ccccacaaga atctatgata cagccttggc tctctggatc ccttctttgc tcatgtctgc 600

aggggaggct gctctatctg gttactgctg tgtggctgca ctcactctac gtggagttgg 660

gccctgcagg aaggacggac ttcaggggca ggtagtagct gggtgtgacg caagagtgaa 720

acagaaagcc tggcagccac ggtttcctgg gattaaagtc aaagcattat gaatatggca 780

ctaaagtgac tgagctacca gaccaatgat cctgtaaggc agccacagaa ctaaaaaaca 840

acaattatta ttaaactgct ctggattctc a 871

<210> 6
<211> 709
<212> DNA
<213> Homo sapiens

<400> 6

gggacggagg cggggcccag gttggggcgg ggccgggccc aggtacagcg gccctgcggc 60

tggcgcggcg gacgggatga ggcgctgcag tctctgcgct ttcgacgccg cccgggggcc 120

caggcggctg atgcgtgtgg gcctcgcgct gatcttggtg ggccacgtga acctgctgct 180

gggggccgtg ctgcatggca ccgtcctgcg gcacgtggcc aatccccgcg gcgctgtcac 240

gccggagtac accgtagcca atgtcatctc tgtcggctcg gggctgctga gcgtttccgt 300

gggacttgtg gccctcctgg cgtccaggaa ccttcttcgc cctccactgg atacagcctt 360

ggctctctgg atcccttctt tgctcatgtc tgcaggggag gctgctctat ctggttactg 420

ctgtgtggct gcactcactc tacgtggagt tgggccctgc aggaaggacg gacttcaggg 480

gcagctagag gaaatgacag agcttgaatc tcctaaatgt aaaaggcagg aaaatgagca 540

gctactggat caaaatcaag aaatccgggc atcacagaga agttgggttt aggacagcag 600

gtgctgttcc gagactcagt cctaaagggt tttttttccc actaagcaag gggccctgac 660

ctcgggatga gataacaaat tgtaataaag taacttctct tttcttcta 709

<210> 7
<211> 240
<212> PRT
<213> Homo sapiens

<400> 7

Met Arg Arg Cys Ser Leu Cys Ala Phe Asp Ala Ala Arg Gly Pro Arg
1               5                   10                  15

Arg Leu Met Arg Val Gly Leu Ala Leu Ile Leu Val Gly His Val Asn
            20                  25                  30

Leu Leu Leu Gly Ala Val Leu His Gly Thr Val Leu Arg His Val Ala
        35                  40                  45

Asn Pro Arg Gly Ala Val Thr Pro Glu Tyr Thr Val Ala Asn Val Ile
    50                  55                  60

Ser Val Gly Ser Gly Leu Leu Ser Val Ser Val Gly Leu Val Ala Leu
65                  70                  75                  80

Leu Ala Ser Arg Asn Leu Leu Arg Pro Pro Leu His Trp Val Leu Leu
                85                  90                  95

Ala Leu Ala Leu Val Asn Leu Leu Leu Ser Val Ala Cys Ser Leu Gly
            100                 105                 110

Leu Leu Leu Ala Val Ser Leu Thr Val Ala Asn Gly Gly Arg Arg Leu
        115                 120                 125

Ile Ala Asp Cys His Pro Gly Leu Leu Asp Pro Leu Val Pro Leu Asp
    130                 135                 140

Glu Gly Pro Gly His Thr Asp Cys Pro Phe Asp Pro Thr Arg Ile Tyr
145                 150                 155                 160

Asp Thr Ala Leu Ala Leu Trp Ile Pro Ser Leu Leu Met Ser Ala Gly
                165                 170                 175

Glu Ala Ala Leu Ser Gly Tyr Cys Cys Val Ala Ala Leu Thr Leu Arg
            180                 185                 190

Gly Val Gly Pro Cys Arg Lys Asp Gly Leu Gln Gly Gln Leu Glu Glu
        195                 200                 205

Met Thr Glu Leu Glu Ser Pro Lys Cys Lys Arg Gln Glu Asn Glu Gln
    210                 215                 220

Leu Leu Asp Gln Asn Gln Glu Ile Arg Ala Ser Gln Arg Ser Trp Val
225                 230                 235                 240


<210>  8
<211>  240
<212>  PRT
<213>  Homo sapiens

<400> 8

Met Arg Arg Cys Ser Leu Cys Ala Phe Asp Ala Ala Arg Gly Pro Arg
1               5                   10                  15

Arg Leu Met Arg Val Gly Leu Ala Leu Ile Leu Val Gly His Val Asn
            20                  25                  30

Leu Leu Leu Gly Ala Val Leu His Gly Thr Val Leu Arg His Val Ala
        35                  40                  45

Asn Pro Arg Gly Ala Val Thr Pro Glu Tyr Thr Val Ala Asn Val Ile
    50                  55                  60

Ser Val Gly Ser Gly Leu Leu Ser Val Ser Val Gly Leu Val Thr Leu
65                  70                  75                  80

Leu Ala Ser Arg Asn Leu Leu Arg Pro Pro Leu His Trp Val Leu Leu
                85                  90                  95

Ala Leu Ala Leu Val Asn Leu Leu Leu Ser Val Ala Cys Ser Leu Gly
            100                 105                 110

Leu Leu Leu Ala Val Ser Leu Thr Val Ala Asn Gly Gly Arg Arg Leu

115                 120                 125

Ile Ala Asp Cys His Pro Gly Leu Leu Asp Pro Leu Val Pro Leu Asp
    130                 135                 140

Glu Gly Pro Gly His Thr Asp Cys Pro Phe Asp Pro Thr Arg Ile Tyr
145                 150                 155                 160

Asp Thr Ala Leu Ala Leu Trp Ile Pro Ser Leu Leu Met Ser Ala Gly
                165                 170                 175

Glu Ala Ala Leu Ser Gly Tyr Cys Cys Val Ala Ala Leu Thr Leu Arg
            180                 185                 190

Gly Val Gly Pro Cys Arg Lys Asp Gly Leu Gln Gly Gln Leu Glu Glu
        195                 200                 205

Met Thr Glu Leu Glu Ser Pro Lys Cys Lys Arg Gln Glu Asn Glu Gln
    210                 215                 220

Leu Leu Asp Gln Asn Gln Glu Ile Arg Ala Ser Gln Arg Ser Trp Val
225                 230                 235                 240


<210>  9
<211>  48
<212>  DNA
<213>  Artificial

<220>
<223>  Synthetic polynucleotide

<400> 9

gtaaggaagg caaacaggaa gggttcattc cacagagact ggctgtgt                48


<210>  10
<211>  266
<212>  PRT
<213>  Homo sapiens

<400> 10

Met Arg Arg Cys Ser Leu Cys Ala Phe Asp Ala Ala Arg Gly Pro Arg
1               5                   10                  15

Arg Leu Met Arg Val Gly Leu Ala Leu Ile Leu Val Gly His Val Asn
            20                  25                  30

Leu Leu Leu Gly Ala Val Leu His Gly Thr Val Leu Arg His Val Ala
        35                  40                  45

Asn Pro Arg Gly Ala Val Thr Pro Glu Tyr Thr Val Ala Asn Val Ile
    50                  55                  60

Ser Val Gly Ser Gly Leu Leu Val Ser Ala Ala Gly Asp Pro Gly Gly
65                  70                  75                  80

Gly Arg Ala Pro Gly Glu Pro Ser Arg Pro Lys Ala Leu Cys Leu Pro
                85                  90                  95

Gln Ser Val Ser Val Gly Leu Val Ala Leu Leu Ala Ser Arg Asn Leu

100                 105                 110

Leu Arg Pro Pro Leu His Trp Val Leu Leu Ala Leu Ala Leu Val Asn
        115                 120                 125

Leu Leu Leu Ser Val Ala Cys Ser Leu Gly Leu Leu Leu Ala Val Ser
    130                 135                 140

Leu Thr Val Ala Asn Gly Gly Arg Arg Leu Ile Ala Asp Cys His Pro
145                 150                 155                 160

Gly Leu Leu Asp Pro Leu Val Pro Leu Asp Glu Gly Pro Gly His Thr
                165                 170                 175

Asp Cys Pro Phe Asp Pro Thr Arg Ile Tyr Asp Thr Ala Leu Ala Leu
            180                 185                 190

Trp Ile Pro Ser Leu Leu Met Ser Ala Gly Glu Ala Ala Leu Ser Gly
        195                 200                 205

Tyr Cys Cys Val Ala Ala Leu Thr Leu Arg Gly Val Gly Pro Cys Arg
    210                 215                 220

Lys Asp Gly Leu Gln Gly Gln Leu Glu Glu Met Thr Glu Leu Glu Ser
225                 230                 235                 240

Pro Lys Cys Lys Arg Gln Glu Asn Glu Gln Leu Leu Asp Gln Asn Gln
                245                 250                 255

Glu Ile Arg Ala Ser Gln Arg Ser Trp Val
            260                 265

<210>  11
<211>  221
<212>  PRT
<213>  Homo sapiens

<400> 11

Met Arg Arg Cys Ser Leu Cys Ala Phe Asp Ala Ala Arg Gly Pro Arg
1               5                   10                  15

Arg Leu Met Arg Val Gly Leu Ala Leu Ile Leu Val Gly His Val Asn
            20                  25                  30

Leu Leu Leu Gly Ala Val Leu His Gly Thr Val Leu Arg His Val Ala
        35                  40                  45

Asn Pro Arg Gly Ala Val Thr Pro Glu Tyr Thr Val Ala Asn Val Ile
    50                  55                  60

Ser Val Gly Ser Gly Leu Leu Ser Val Ser Val Gly Leu Val Ala Leu
65                  70                  75                  80

Leu Ala Ser Arg Asn Leu Leu Arg Pro Pro Leu His Trp Val Leu Leu
                85                  90                  95

Ala Leu Ala Leu Val Asn Leu Leu Leu Ser Val Ala Cys Ser Leu Gly
            100                 105                 110

Leu Leu Leu Ala Val Ser Leu Thr Val Ala Asn Gly Gly Arg Arg Leu
        115                 120                 125

Ile Ala Asp Cys His Pro Gly Leu Leu Asp Pro Leu Val Pro Leu Asp
    130                 135                 140

Glu Gly Pro Gly His Thr Asp Cys Pro Phe Asp Pro Thr Arg Ile Tyr
145                 150                 155                 160

Asp Thr Ala Leu Ala Leu Trp Ile Pro Ser Leu Leu Met Ser Ala Gly
                165                 170                 175

Glu Ala Ala Leu Ser Gly Tyr Cys Cys Val Ala Ala Leu Thr Leu Arg
            180                 185                 190

Gly Val Gly Pro Cys Arg Lys Asp Gly Leu Gln Gly Gln Val Arg Lys
        195                 200                 205

Ala Asn Arg Lys Gly Ser Phe His Arg Asp Trp Leu Cys
    210                 215                 220


<210>  12
<211>  231
<212>  PRT
<213>  Homo sapiens

<400> 12

Met Arg Arg Cys Ser Leu Cys Ala Phe Asp Ala Ala Arg Gly Pro Arg
1               5                   10                  15

Arg Leu Met Arg Val Gly Leu Ala Leu Ile Leu Val Gly His Val Asn
            20                  25                  30

Leu Leu Leu Gly Ala Val Leu His Gly Thr Val Leu Arg His Val Ala
        35                  40                  45

Asn Pro Arg Gly Ala Val Thr Pro Glu Tyr Thr Val Ala Asn Val Ile
    50                  55                  60

Ser Val Gly Ser Gly Leu Leu Ser Val Ser Val Gly Leu Val Ala Leu
65                  70                  75                  80

Leu Ala Ser Arg Asn Leu Leu Arg Pro Pro Leu His Trp Val Leu Leu
                85                  90                  95

Ala Leu Ala Leu Val Asn Leu Leu Leu Ser Val Ala Cys Ser Leu Gly
            100                 105                 110

Leu Leu Leu Ala Val Ser Leu Thr Val Ala Asn Gly Gly Arg Arg Leu
        115                 120                 125

Ile Ala Asp Cys His Pro Gly Leu Leu Asp Pro Leu Val Pro Leu Asp
    130                 135                 140

Glu Gly Pro Gly His Thr Asp Cys Pro Phe Asp Pro Thr Arg Ile Tyr
145                 150                 155                 160

Asp Thr Ala Leu Ala Leu Trp Ile Pro Ser Leu Leu Met Ser Ala Gly
                165                 170                 175

Glu Ala Ala Leu Ser Gly Tyr Cys Cys Val Ala Ala Leu Thr Leu Arg
            180                 185                 190

Gly Val Gly Pro Cys Arg Lys Asp Gly Leu Gln Gly Gln Val Val Ala
        195                 200                 205

Gly Cys Asp Ala Arg Val Lys Gln Lys Ala Trp Gln Pro Arg Phe Pro
    210                 215                 220

Gly Ile Lys Val Lys Ala Leu
225                 230

<210>  13
<211>  171
<212>  PRT
<213>  Homo sapiens

<400> 13

Met Arg Arg Cys Ser Leu Cys Ala Phe Asp Ala Ala Arg Gly Pro Arg
1               5                   10                  15

Arg Leu Met Arg Val Gly Leu Ala Leu Ile Leu Val Gly His Val Asn
            20                  25                  30

Leu Leu Leu Gly Ala Val Leu His Gly Thr Val Leu Arg His Val Ala
        35                  40                  45

Asn Pro Arg Gly Ala Val Thr Pro Glu Tyr Thr Val Ala Asn Val Ile
    50                  55                  60

Ser Val Gly Ser Gly Leu Leu Ser Val Ser Val Gly Leu Val Ala Leu
65                  70                  75                  80

Leu Ala Ser Arg Asn Leu Leu Arg Pro Pro Leu Asp Thr Ala Leu Ala
                85                  90                  95

Leu Trp Ile Pro Ser Leu Leu Met Ser Ala Gly Glu Ala Ala Leu Ser
            100                 105                 110

Gly Tyr Cys Cys Val Ala Ala Leu Thr Leu Arg Gly Val Gly Pro Cys
        115                 120                 125

Arg Lys Asp Gly Leu Gln Gly Gln Leu Glu Glu Met Thr Glu Leu Glu
    130                 135                 140

Ser Pro Lys Cys Lys Arg Gln Glu Asn Glu Gln Leu Leu Asp Gln Asn
145                 150                 155                 160

Gln Glu Ile Arg Ala Ser Gln Arg Ser Trp Val
                165                 170

<210>  14
<211>  1252
<212>  DNA
<213>  Homo sapiens

<400> 14

gaaggagatc aggcagagga gagaagagtc agtgtgaact aggctggtgg ctctgtccgg      60

tagggagacg gaaagggtgc cgcccatcat agaagtacca gaacttgagc tggactttgc     120

tgatttagct tatggaagag gaaccagaaa tttgtccttg aataatgttt cccgacaacg     180

aagaggcaga aggatctggg cctgtgcgcg acgccccggg ggacgaggct catggagaag 240

tttcgggcgg tgctggacct gcacgtcaag caccacagcg ccttgggcta cggcctggtg 300

accctgctga cggcgggcgg ggagcgcatc ttctccgccg tggcattcca gtgcccgtgc 360

agcgccgcct ggaacctgcc ctacggcctg gtcttcttgc tggtgccggc gctcgcgctc 420

ttcctcctgg gctacgtgct gagcgcacgc acgtggcgcc tgctcaccgg atgctgctcc 480

agcgcccgcg cgagttgcgg atcggcgctg cgcggctccc tggtgtgcgc gcaaatcagc 540

gcggccgccg cgctcgcgcc cctcacctgg gtggccgtgg cgctgctcgg gggcgccttt 600

tacgagtgcg cggccaccgg gagcgcggcc ttcgcgcagc gcctgtgcct cggccgcaac 660

cgcagctgcg ccgcggagct gccgctggtg ccgtgcaacc aggccaaggc gtcggacgtg 720

caggacctcc tgaaggatct gaaggctcag tcgcaggtgt tgggctggat cttgatagca 780

gttgttatca tcattcttct gatttttaca tctgtcaccc gatgcctatc tccagttagt 840

tttctgcagc tgaaattctg gaaaatctat ttggaacagg agcagcagat ccttaaaagt 900

aaagccacag agcatgcaac tgaattggca aaagagaata ttaaatgttt ctttgagggc 960

tcgcatccaa aagaatataa cactccaagc atgaaagagt ggcagcaaat ttcatcactg 1020

tatactttca atccgaaggg ccagtactac agcatgttgc acaaatatgt caacagaaaa 1080

gagaagactc acagtatcag gtctactgaa ggagatacgg tgattcctgt tcttggcttt 1140

gtagattcat ctggtataaa cagcactcct gagttatgac cttttgaatg agtagaaaaa 1200

aaaattgttt tgaattattg ctttattaaa aaataaacat tggtattttt tg 1252

<210> 15
<211> 315
<212> PRT
<213> Homo sapiens

<400> 15

Met Glu Lys Phe Arg Ala Val Leu Asp Leu His Val Lys His His Ser
1               5                   10                  15

Ala Leu Gly Tyr Gly Leu Val Thr Leu Leu Thr Ala Gly Gly Glu Arg
            20                  25                  30

Ile Phe Ser Ala Val Ala Phe Gln Cys Pro Cys Ser Ala Ala Trp Asn
        35                  40                  45

Leu Pro Tyr Gly Leu Val Phe Leu Leu Val Pro Ala Leu Ala Leu Phe
    50                  55                  60

Leu Leu Gly Tyr Val Leu Ser Ala Arg Thr Trp Arg Leu Leu Thr Gly
65                  70                  75                  80

Cys Cys Ser Ser Ala Arg Ala Ser Cys Gly Ser Ala Leu Arg Gly Ser
                85                  90                  95

Leu Val Cys Thr Gln Ile Ser Ala Ala Ala Ala Leu Ala Pro Leu Thr
            100                 105                 110

Trp Val Ala Val Ala Leu Leu Gly Gly Ala Phe Tyr Glu Cys Ala Ala
        115                 120                 125

Thr Gly Ser Ala Ala Phe Ala Gln Arg Leu Cys Leu Gly Arg Asn Arg
    130                 135                 140

Ser Cys Ala Ala Glu Leu Pro Leu Val Pro Cys Asn Gln Ala Lys Ala
145                 150                 155                 160

Ser Asp Val Gln Asp Leu Leu Lys Asp Leu Lys Ala Gln Ser Gln Val
                165                 170                 175

Leu Gly Trp Ile Leu Ile Ala Val Val Ile Ile Ile Leu Leu Ile Phe
            180                 185                 190

Thr Ser Val Thr Arg Cys Leu Ser Pro Val Ser Phe Leu Gln Leu Lys
        195                 200                 205

Phe Trp Lys Ile Tyr Leu Glu Gln Glu Gln Gln Ile Leu Lys Ser Lys
    210                 215                 220

Ala Thr Glu His Ala Thr Glu Leu Ala Lys Glu Asn Ile Lys Cys Phe
225                 230                 235                 240

Phe Glu Gly Ser His Pro Lys Glu Tyr Asn Thr Pro Ser Met Lys Glu
                245                 250                 255

Trp Gln Gln Ile Ser Ser Leu Tyr Thr Phe Asn Pro Lys Gly Gln Tyr
            260                 265                 270

Tyr Ser Met Leu His Lys Tyr Val Asn Arg Lys Glu Lys Thr His Ser
        275                 280                 285

Ile Arg Ser Thr Glu Gly Asp Thr Val Ile Pro Val Leu Gly Phe Val
    290                 295                 300

Asp Ser Ser Gly Ile Asn Ser Thr Pro Glu Leu
305                 310                 315


<210>  16
<211>  143
<212>  PRT
<213>  Homo sapiens

<400> 16

Met Phe Pro Val Leu Gly Trp Ile Leu Ile Ala Val Val Ile Ile Ile
1               5                   10                  15

Leu Leu Ile Phe Thr Ser Val Thr Arg Cys Leu Ser Pro Val Ser Phe
            20                  25                  30

Leu Gln Leu Lys Phe Trp Lys Ile Tyr Leu Glu Gln Glu Gln Gln Ile
        35                  40                  45

Leu Lys Ser Lys Ala Thr Glu His Ala Thr Glu Leu Ala Lys Glu Asn
    50                  55                  60

Ile Lys Cys Phe Phe Glu Gly Ser His Pro Lys Glu Tyr Asn Thr Pro

65                  70                  75                  80

Ser Met Lys Glu Trp Gln Gln Ile Ser Ser Leu Tyr Thr Phe Asn Pro
                85                  90                  95

Lys Gly Gln Tyr Tyr Ser Met Leu His Lys Tyr Val Asn Arg Lys Glu
            100                 105                 110

Lys Thr His Ser Ile Arg Ser Thr Glu Gly Asp Thr Val Ile Pro Val
        115                 120                 125

Leu Gly Phe Val Asp Ser Ser Gly Ile Asn Ser Thr Pro Glu Leu
    130                 135                 140


<210>  17
<211>  158
<212>  PRT
<213>  Homo sapiens

<400> 17

Arg Trp Gly Arg Leu Gly Gly Ala Glu Arg Pro Ser Phe Leu Arg Ala
1               5                   10                  15

Ala Gly Val Leu Gly Trp Ile Leu Ile Ala Val Val Ile Ile Ile Leu
            20                  25                  30

Leu Ile Phe Thr Ser Val Thr Arg Cys Leu Ser Pro Val Ser Phe Leu
        35                  40                  45

Gln Leu Lys Phe Trp Lys Ile Tyr Leu Glu Gln Glu Gln Gln Ile Leu
    50                  55                  60

Lys Ser Lys Ala Thr Glu His Ala Thr Glu Leu Ala Lys Glu Asn Ile
65                  70                  75                  80

Lys Cys Phe Phe Glu Gly Ser His Pro Lys Glu Tyr Asn Thr Pro Ser
                85                  90                  95

Met Lys Glu Trp Gln Gln Ile Ser Ser Leu Tyr Thr Phe Asn Pro Lys
            100                 105                 110

Gly Gln Tyr Tyr Ser Met Leu His Lys Tyr Val Asn Arg Lys Glu Lys
        115                 120                 125

Thr His Ser Ile Arg Ser Thr Glu Gly Asp Thr Val Ile Pro Val Leu
    130                 135                 140

Gly Phe Val Asp Ser Ser Gly Ile Asn Ser Thr Pro Glu Leu
145                 150                 155


<210>  18
<211>  315
<212>  PRT
<213>  Homo sapiens

<400> 18


<210>  19
<211>  194
<212>  PRT
<213>  Homo sapiens

<400> 19

Met Ala Ser Leu Trp Pro Ser Ala Leu Thr Phe Asn Thr Asp Ala Asn
1               5                   10                  15

Ile Pro Gly Pro Leu Gly Phe Cys Gly Gly Trp Val Arg Leu Cys Ser
            20                  25                  30

Leu Ser Ser Leu Thr Pro Pro Cys Gly Arg Arg Leu Val Pro Cys Leu
        35                  40                  45

Ser Ala Pro Ala Pro Asn Ala Pro Arg Leu Pro Ala Pro Ala Arg Cys
    50                  55                  60

Ser Ile Gly Ala Leu Ile Gly Leu Ser Val Ala Ala Val Val Leu Leu
65                  70                  75                  80

Ala Phe Ile Val Thr Ala Cys Val Leu Cys Tyr Leu Phe Ile Ser Ser
                85                  90                  95

Lys Pro His Thr Lys Leu Asp Leu Gly Leu Ser Leu Gln Thr Ala Gly
            100                 105                 110

Pro Glu Glu Val Ser Pro Asp Cys Gln Gly Val Asn Thr Gly Met Ala
        115                 120                 125

Ala Glu Val Pro Lys Val Ser Pro Leu Gln Gln Ser Tyr Ser Cys Leu
    130                 135                 140

Asn Pro Gln Leu Glu Ser Asn Glu Gly Gln Ala Val Asn Ser Lys Arg
145                 150                 155                 160

Leu Leu His His Cys Phe Met Ala Thr Val Thr Thr Ser Asp Ile Pro
                165                 170                 175

Gly Ser Pro Glu Glu Ala Ser Val Pro Asn Pro Asp Leu Cys Gly Pro
            180                 185                 190

Val Pro
<210>  20

<211>  614
<212>  DNA
<213>  Homo sapiens

<400>  20

atggcttctc tctggccctc agctctcacc tttaacacgg atgcgaacat ccccgggccc      60

ctgggttttt gcggaggttg ggtgaggttg tgttcactct cctcactgac cccgccgtgt     120

ggtcgccggc tggtgccttg tctgtctgcc ccagccccga atgccccgag actcccggcg     180

ccggctcggt gcagcattgg cgctctcata ggcctgtccg tagcagcagt ggttcttctc     240

gccttcattg ttaccgcctg tgtgctctgc tacctgttca tcagctctaa gccccacaca     300

aagttggacc tgggcttgag cttacagaca gcaggccctg aggaggtttc tcctgactgc     360

caaggtgtga acacaggcat ggcggcagaa gtgccaaaag tgagccctct ccagcagagt     420

tactcctgct tgaacccgca gctggagagc aatgaggggc aggctgtgaa ctccaaacgc     480

ctcctccatc attgcttcat ggccacagtg accaccagtg acattccagg cagccctgag 540

gaagcctctg tacccaaccc tgacctatgt ggaccagtcc cataaacatt caataaatgt 600

ctccatacca tccc 614

<210> 21
<211> 3517
<212> DNA
<213> Homo sapiens

<400> 21

acgcggctgc acggaccgcc aatgctgctg cctcccagag cagagggagg cagaagagga 60

gggagggaga agagtggaag ggagggggag gcggcacttg ggctgcagct cgcaaccaga 120

ggcagtgtct ctcagctctc ggaaggaccg agagaggcaa ccgcggagta ggatgctctg 180

cggggcgccc agagccgggg gcgcttgaag cagcttcggg gactggggca accgggcagt 240

tttcaccatg catcagtccc tgactcagca gcggtccagc gacatgtccc tgcccgattc 300

catgggagca ttcaatcgga ggaaacgaaa ctccatctat gtcaccgtga ctttgcttat 360

tgtgtccgtg ttaattctca cagtgggcct tgctgcaacc accaggaccc agaatgtgac 420

tgtaggaggt tattaccccg gagttattct cggctttgga tcgttccttg gaatcattgg 480

atcaaacctt attgagaaca aaaggcagat gctggtggct tctatcgtgt ttatcagctt 540

tggtgtgatt gcggcttttt gttgtgccat agttgacggg gtctttgctg ccagacacat 600

tgatctgaaa ccactctacg ctaaccggtg ccattatgtt cccaagacat cacagaagga 660

agctgaggag gtcataagtt cctcaaccaa aaattctcct tccacgaggg ttatgaggaa 720

ccttacccag gcagctagag aggttaactg ccctcacctc agccgtgaat tctgcacacc 780

tcgcatccgg ggcaacacct gcttctgctg tgacctctac aactgtggca accgggtgga 840

gatcactggt gggtactacg aatacatcga tgtcagcagt tgccaagata tcatccacct 900

ctaccacctg ctctggtctg ccaccatcct caacattgtt ggcctgttcc tgggcatcat 960

cactgccgct gtccttggag gctttaagga catgaaccca actctcccag cactgaactg 1020

ttctgttgaa aatacccatc caacagtttc ttactatgct catccccaag tggcatccta 1080

caatacctac taccatagcc ctcctcacct gccaccatat tctgcttatg actttcagca 1140

ttccggtgtc tttccatcct cccctccctc tggactttct gatgagcccc agtctgcctc 1200

tccctcaccc agctacatgt ggtcctcaag tgcaccgccc cgttactctc caccctacta 1260

tccacctttt gaaaagccac caccttacag tccctaaaga ggaatgcctg ctggctattg 1320

agattattgt ggcttttgta tttctgcttc agtggaagtg tgtagggtac aaaatttaaa 1380

gtgtgactct tatgcataaa gttttacaat ggcctgccag gctagggaaa gatagggacg 1440

aagcttattc attattagtg cagagcaggg gtggtcaggc tgaacgcagc acagaagggc 1500

agctcacatt ctctaagcaa gactggggag ccagcccagc aagaagcttg tttggacttg 1560

cattacccta tgctccacct ctgtattcag cagaagtgtg gttgccatct ttttcacttt 1620

atgtaaagga gtgttgccct cgggcccttg gcagattgcc accccagcac ctaggttgaa 1680

gcacctggtt tataggccct atctttccct acccctaaag tcagtcccta aggacaattt 1740

cccagctgat ggggctacac agtagttcca atacagagag ttctggctaa gattttgttt 1800

gcttgtgtct ggatgttgaa aaagactgcc cgtatctctt actccttcct tctctgtgag 1860

tattgtaaaa atggctgttg tgatcactca gctcagcttt tgttattggt acctcctaaa 1920

gggaaaagtg caatattctt gcatcttcag tagtggggaa caggatgtat tgttccggaa 1980

acactgaaat acacagcaac atgtgagatg ttttaagtag atcacttagg agacagtggt 2040

tctactacat gttgcattat tacaaaatac atttgctaca ggagatataa atcttatggt 2100

tgtaattcag agtttaaaaa tgttataaat taggttcttg ggtcgtgata tgaattgtta 2160

ctaatctttg tgactattta atcttcaaat attgtgctta accccagcaa tccgcacgta 2220

tcctgcaccc caccccaaaa gagtcatctg tattttaatg ccactggtct tatcggtcct 2280

tttgtctgtt gagaccagtc atgacagcat tcaagattat gaaagtgtta caatgccgct 2340

tcaagtctgc aaaacctcaa acgtagccaa cttgacaaat atttaagtgt tacggcagat 2400

ttaaaatcca tctggcacac cgtggtaggt atttgtacag ttcttttaat tacacatagc 2460

tttaaaccat caacctgatg agtttaaagc ttttgcaccc atgccttcac ttcagaatga 2520

acaccttcat tgtgatctta tgttaacctg agaattgatt taaaggaaga ttgataatcc 2580

tatactttat aacgtaaaaa tacaggggct acaggagggt acctaattag acagttctcc 2640

aaacacagaa cacacactgg aaaattttcc ggccaatttt gctacctccc aacttgatgg 2700

attagaggta gcgcaaatgc tggtgctccc atctaccttg tagacactta gccatcaaga 2760

atcaaggcac aagaagtgca ctctctcatt aacagtaaat gtttgcaaga tattcagttt 2820

aactttcagc atcatgaatg ttcttatcca gattttgaat ccgaaaaact ataatccttt 2880

tatgttatac aaaattacta tgatttttta cagttctgag catattaaaa ttctactgga 2940

tttcaaaaag agactaatac ccaactgact aactaaacaa atatcaactt gtaatactca 3000

atgaattttt ttgccattta catttgaccg ttggctttag tgaatgtcca tatttaattt 3060

tttaaggcac cattacacag tttatcctac atttatcaca tttcttaaag tgttaagatt 3120

ctatggctca tttctatgta tttttcttac tttacaaaat aacctgaaac agtatagatt 3180

ttgtaacact taatttgagc agctttttta ttacattgaa ttatataaag tgcatgttac 3240

cttagaaaaa ttaatatttg ctgctttact cttttgcaaa acatttgctg taatgaatgg 3300

atttgtattt ccaatatgta tcttgactgc attttgtaat atttactgct ttattcctaa 3360

ttctgcttta aagtactgaa ctgggcatga aacattaaaa tattaatcca gaaactgtat 3420

aaactggatg ttgcttaaaa tctgtatcac tgccatgttg aaaactcaga ctgcttttgt 3480

gatgtttcaa atgaataaaa ctatcctccc ctcgtta 3517

<210> 22
<211> 3445
<212> DNA
<213> Homo sapiens

<400> 22

acgcggctgc acggaccgcc aatgctgctg cctcccagag cagagggagg cagaagagga 60

gggagggaga agagtggaag ggagggggag gcggcacttg ggctgcagct cgcaaccaga 120

ggcagtgtct ctcagctctc ggaaggaccg agagaggcaa ccgcggagta ggatgctctg 180

cggggcgccc agagccgggg gcgcttgaag cagcttcggg gactggggca accgggcagt 240

tttcaccatg catcagtccc tgactcagca gcggtccagc gacatgtccc tgcccgattc 300

catgggagca ttcaatcgga ggaaacgaaa ctccatctat gtcaccgtga ctttgcttat 360

tgtgtccgtg ttaattctca cagtgggcct tgctgcaacc accaggaccc agaatgtgac 420

tgtaggaggt tattaccccg gagttattct cggctttgga tcgttccttg gaatcattgg 480

atcaaacctt attgagaaca aaaggcagat gctggtggct tctatcgtgt ttatcagctt 540

tggtgtgatt gcggcttttt gttgtgccat agttgacggg gtctttgctg ccagacacat 600

tgatctgaaa ccactctacg ctaaccggtg ccattatgtt cccaagacat cacagaagga 660

agctgaggag gttaactgcc ctcacctcag ccgtgaattc tgcacacctc gcatccgggg 720

caacacctgc ttctgctgtg acctctacaa ctgtggcaac cgggtggaga tcactggtgg 780

gtactacgaa tacatcgatg tcagcagttg ccaagatatc atccacctct accacctgct 840

ctggtctgcc accatcctca acattgttgg cctgttcctg ggcatcatca ctgccgctgt 900

ccttggaggc tttaaggaca tgaacccaac tctcccagca ctgaactgtt ctgttgaaaa 960

tacccatcca acagtttctt actatgctca tccccaagtg gcatcctaca atacctacta 1020

ccatagccct cctcacctgc caccatattc tgcttatgac tttcagcatt ccggtgtctt 1080

tccatcctcc cctccctctg gactttctga tgagccccag tctgcctctc cctcacccag 1140

ctacatgtgg tcctcaagtg caccgccccg ttactctcca ccctactatc caccttttga 1200

aaagccacca ccttacagtc cctaaagagg aatgcctgct ggctattgag attattgtgg 1260

cttttgtatt tctgcttcag tggaagtgtg tagggtacaa aatttaaagt gtgactctta 1320

tgcataaagt tttacaatgg cctgccaggc tagggaaaga tagggacgaa gcttattcat 1380

tattagtgca gagcaggggt ggtcaggctg aacgcagcac agaagggcag ctcacattct 1440

ctaagcaaga ctggggagcc agcccagcaa gaagcttgtt tggacttgca ttaccctatg 1500

ctccacctct gtattcagca gaagtgtggt tgccatcttt ttcactttat gtaaaggagt 1560

gttgccctcg ggcccttggc agattgccac cccagcacct aggttgaagc acctggttta 1620

taggccctat ctttccctac ccctaaagtc agtccctaag gacaatttcc cagctgatgg 1680

ggctacacag tagttccaat acagagagtt ctggctaaga ttttgtttgc ttgtgtctgg 1740

atgttgaaaa agactgcccg tatctcttac tccttccttc tctgtgagta ttgtaaaaat 1800

ggctgttgtg atcactcagc tcagcttttg ttattggtac ctcctaaagg gaaaagtgca 1860

atattcttgc atcttcagta gtggggaaca ggatgtattg ttccggaaac actgaaatac 1920

acagcaacat gtgagatgtt ttaagtagat cacttaggag acagtggttc tactacatgt 1980

tgcattatta caaaatacat ttgctacagg agatataaat cttatggttg taattcagag 2040

tttaaaaatg ttataaatta ggttcttggg tcgtgatatg aattgttact aatctttgtg 2100

actatttaat cttcaaatat tgtgcttaac cccagcaatc cgcacgtatc ctgcacccca 2160

ccccaaaaga gtcatctgta ttttaatgcc actggtctta tcggtccttt tgtctgttga 2220

gaccagtcat gacagcattc aagattatga aagtgttaca atgccgcttc aagtctgcaa 2280

aacctcaaac gtagccaact tgacaaatat ttaagtgtta cggcagattt aaaatccatc 2340

tggcacaccg tggtaggtat ttgtacagtt cttttaatta cacatagctt taaaccatca 2400

acctgatgag tttaaagctt ttgcacccat gccttcactt cagaatgaac accttcattg 2460

tgatcttatg ttaacctgag aattgattta aaggaagatt gataatccta tactttataa 2520

cgtaaaaata caggggctac aggagggtac ctaattagac agttctccaa acacagaaca 2580

cacactggaa aattttccgg ccaattttgc tacctcccaa cttgatggat tagaggtagc 2640

gcaaatgctg gtgctcccat ctaccttgta gacacttagc catcaagaat caaggcacaa 2700

gaagtgcact ctctcattaa cagtaaatgt ttgcaagata ttcagtttaa ctttcagcat 2760

catgaatgtt cttatccaga ttttgaatcc gaaaaactat aatcctttta tgttatacaa 2820

aattactatg attttttaca gttctgagca tattaaaatt ctactggatt tcaaaaagag 2880

actaataccc aactgactaa ctaaacaaat atcaacttgt aatactcaat gaattttttt 2940

gccatttaca tttgaccgtt ggctttagtg aatgtccata tttaattttt taaggcacca 3000

ttacacagtt tatcctacat ttatcacatt tcttaaagtg ttaagattct atggctcatt 3060

tctatgtatt tttcttactt tacaaaataa cctgaaacag tatagatttt gtaacactta 3120

atttgagcag cttttttatt acattgaatt atataaagtg catgttacct tagaaaaatt 3180

aatatttgct gctttactct tttgcaaaac atttgctgta atgaatggat ttgtatttcc 3240

aatatgtatc ttgactgcat tttgtaatat ttactgcttt attcctaatt ctgctttaaa 3300

gtactgaact gggcatgaaa cattaaaata ttaatccaga aactgtataa actggatgtt 3360

gcttaaaatc tgtatcactg ccatgttgaa aactcagact gcttttgtga tgtttcaaat 3420

gaataaaact atcctcccct cgtta 3445

<210> 23
<211> 49
<212> DNA
<213> Artificial

<220>
<223> Synthetic polynucleotide

<400> 23

gtaagccgcc tccacttcct actcctggga aggaggatcg caaaagctg 49

<210> 24
<211> 3193
<212> DNA
<213> Homo sapiens

<400> 24

acgcggctgc acggaccgcc aatgctgctg cctcccagag cagagggagg cagaagagga 60

gggagggaga agagtggaag ggagggggag gcggcacttg ggctgcagct cgcaaccaga 120

ggcagtgtct ctcagctctc ggaaggaccg agagaggcaa ccgcggagta ggatgctctg 180

cggggcgccc agagccgggg gcgcttgaag cagcttcggg gactggggca accgggcagt 240

tttcaccatg catcagtccc tgactcagca gcggtccagc gacatgtccc tgcccgattc 300

catgggagca ttcaatcgga ggaaacgaaa ctccatctat gtcaccgtga ctttgcttat 360

tgtgtccgtg ttaattctca cagtgggcct tgctgcaacc accaggaccc agaatgtgac 420

tgtaggaggt tattaccccg gagttattct cggctttgga tcgttccttg gaatcattgg 480

atcaaacctt attgagaaca aaaggcagat gctggtggct tctatcgtgt ttatcagctt 540

tggtgtgatt gcggcttttt gttgtgccat agttgacggg gtctttgctg ccagacacat 600

tgatctgaaa ccactctacg ctaaccggtg ccattatgtt cccaagacat cacagaagga 660

agctgaggag aacccaactc tcccagcact gaactgttct gttgaaaata cccatccaac 720

agtttcttac tatgctcatc cccaagtggc atcctacaat acctactacc atagccctcc 780

tcacctgcca ccatattctg cttatgactt tcagcattcc ggtgtctttc catcctcccc 840

tccctctgga ctttctgatg agccccagtc tgcctctccc tcacccagct acatgtggtc 900

ctcaagtgca ccgccccgtt actctccacc ctactatcca ccttttgaaa agccaccacc 960

ttacagtccc taaagaggaa tgcctgctgg ctattgagat tattgtggct tttgtatttc 1020

tgcttcagtg gaagtgtgta gggtacaaaa tttaaagtgt gactcttatg cataaagttt 1080

tacaatggcc tgccaggcta gggaaagata gggacgaagc ttattcatta ttagtgcaga 1140

gcaggggtgg tcaggctgaa cgcagcacag aagggcagct cacattctct aagcaagact 1200
ggggagccag cccagcaaga agcttgtttg gacttgcatt accctatgct ccacctctgt 1260
attcagcaga agtgtggttg ccatcttttt cactttatgt aaaggagtgt tgccctcggg 1320
cccttggcag attgccaccc cagcacctag gttgaagcac ctggtttata ggccctatct 1380
ttccctaccc ctaaagtcag tccctaagga caatttccca gctgatgggg ctacacagta 1440
gttccaatac agagagttct ggctaagatt ttgtttgctt gtgtctggat gttgaaaaag 1500
actgcccgta tctcttactc cttccttctc tgtgagtatt gtaaaaatgg ctgttgtgat 1560
cactcagctc agcttttgtt attggtacct cctaaaggga aaagtgcaat attcttgcat 1620
cttcagtagt ggggaacagg atgtattgtt ccggaaacac tgaaatacac agcaacatgt 1680
gagatgtttt aagtagatca cttaggagac agtggttcta ctacatgttg cattattaca 1740
aaatacattt gctacaggag atataaatct tatggttgta attcagagtt taaaaatgtt 1800
ataaattagg ttcttgggtc gtgatatgaa ttgttactaa tctttgtgac tatttaatct 1860
tcaaatattg tgcttaaccc cagcaatccg cacgtatcct gcaccccacc ccaaaagagt 1920
catctgtatt ttaatgccac tggtcttatc ggtccttttg tctgttgaga ccagtcatga 1980
cagcattcaa gattatgaaa gtgttacaat gccgcttcaa gtctgcaaaa cctcaaacgt 2040
agccaacttg acaaatattt aagtgttacg gcagatttaa aatccatctg gcacaccgtg 2100
gtaggtattt gtacagttct tttaattaca catagcttta aaccatcaac ctgatgagtt 2160
taaagctttt gcacccatgc cttcacttca gaatgaacac cttcattgtg atcttatgtt 2220
aacctgagaa ttgatttaaa ggaagattga taatcctata ctttataacg taaaaataca 2280
ggggctacag gagggtacct aattagacag ttctccaaac acagaacaca cactggaaaa 2340
ttttccggcc aattttgcta cctcccaact tgatggatta gaggtagcgc aaatgctggt 2400
gctcccatct accttgtaga cacttagcca tcaagaatca aggcacaaga agtgcactct 2460
ctcattaaca gtaaatgttt gcaagatatt cagtttaact ttcagcatca tgaatgttct 2520
tatccagatt ttgaatccga aaaactataa tccttttatg ttatacaaaa ttactatgat 2580
tttttacagt tctgagcata ttaaaattct actggatttc aaaaagagac taatacccaa 2640
ctgactaact aaacaaatat caacttgtaa tactcaatga atttttttgc catttacatt 2700
tgaccgttgg ctttagtgaa tgtccatatt taatttttta aggcaccatt acacagttta 2760
tcctacattt atcacatttc ttaaagtgtt aagattctat ggctcatttc tatgtatttt 2820
tcttacttta caaaataacc tgaaacagta tagattttgt aacacttaat ttgagcagct 2880
tttttattac attgaattat ataaagtgca tgttacctta gaaaaattaa tatttgctgc 2940
tttactcttt tgcaaaacat ttgctgtaat gaatggattt gtatttccaa tatgtatctt 3000
gactgcattt tgtaatattt actgctttat tcctaattct gctttaaagt actgaactgg 3060

gcatgaaaca ttaaaatatt aatccagaaa ctgtataaac tggatgttgc ttaaaatctg 3120

tatcactgcc atgttgaaaa ctcagactgc ttttgtgatg tttcaaatga ataaaactat 3180

cctcccctcg tta 3193

<210> 25
<211> 165
<212> PRT
<213> Homo sapiens

<400> 25

Leu Cys Ser Leu Ser Ser Leu Thr Pro Pro Cys Gly Arg Arg Leu Val
1               5                   10                  15

Pro Cys Leu Ser Ala Pro Ala Pro Asn Ala Pro Arg Leu Pro Ala Pro
            20                  25                  30

Ala Arg Cys Ser Ile Gly Ala Leu Ile Gly Leu Ser Val Ala Ala Val
        35                  40                  45

Val Leu Leu Ala Phe Ile Val Thr Ala Cys Val Leu Cys Tyr Leu Phe
    50                  55                  60

Ile Ser Ser Lys Pro His Thr Lys Leu Asp Leu Gly Leu Ser Leu Gln
65                  70                  75                  80

Thr Ala Gly Pro Glu Glu Val Ser Pro Asp Cys Gln Gly Val Asn Thr
                85                  90                  95

Gly Met Ala Ala Glu Val Pro Lys Val Ser Pro Leu Gln Gln Ser Tyr
            100                 105                 110

Ser Cys Leu Asn Pro Gln Leu Glu Ser Asn Glu Gly Gln Ala Val Asn
        115                 120                 125

Ser Lys Arg Leu Leu His His Cys Phe Met Ala Thr Val Thr Thr Ser
    130                 135                 140

Asp Ile Pro Gly Ser Pro Glu Glu Ala Ser Val Pro Asn Pro Asp Leu
145                 150                 155                 160

Cys Gly Pro Val Pro
                165

<210> 26
<211> 3454
<212> DNA
<213> Homo sapiens

<400> 26

acgcggctgc acggaccgcc aatgctgctg cctcccagag cagagggagg cagaagagga 60

gggagggaga agagtggaag ggagggggag gcggcacttg ggctgcagct cgcaaccaga 120

ggcagtgtct ctcagctctc ggaaggaccg agagaggcaa ccgcggagta ggatgctctg 180

cggggcgccc agagccgggg gcgcttgaag cagcttcggg gactggggca accgggcagt 240

tttcaccatg catcagtccc tgactcagca gcggtccagc gacatgtccc tgcccgattc 300
catgggagca ttcaatcgga ggaaacgaaa ctccatctat gtcaccgtga ctttgcttat 360
tgtgtccgtg ttaattctca cagtgggcct tgctgcaacc accaggaccc agaatgtgac 420
tgtaggaggt tattaccccg gagttattct ggtggcttct atcgtgttta tcagctttgg 480
tgtgattgcg gctttttgtt gtgccatagt tgacggggtc tttgctgcca gacacattga 540
tctgaaacca ctctacgcta accggtgcca ttatgttccc aagacatcac agaaggaagc 600
tgaggaggtc ataagttcct caaccaaaaa ttctccttcc acgagggtta tgaggaacct 660
tacccaggca gctagagagg ttaactgccc tcacctcagc cgtgaattct gcacacctcg 720
catccggggc aacacctgct tctgctgtga cctctacaac tgtggcaacc gggtggagat 780
cactggtggg tactacgaat acatcgatgt cagcagttgc caagatatca tccacctcta 840
ccacctgctc tggtctgcca ccatcctcaa cattgttggc ctgttcctgg gcatcatcac 900
tgccgctgtc cttggaggct ttaaggacat gaacccaact ctcccagcac tgaactgttc 960
tgttgaaaat acccatccaa cagtttctta ctatgctcat ccccaagtgg catcctacaa 1020
tacctactac catagccctc ctcacctgcc accatattct gcttatgact ttcagcattc 1080
cggtgtcttt ccatcctccc ctccctctgg actttctgat gagccccagt ctgcctctcc 1140
ctcacccagc tacatgtggt cctcaagtgc accgccccgt tactctccac cctactatcc 1200
accttttgaa aagccaccac cttacagtcc ctaaagagga atgcctgctg gctattgaga 1260
ttattgtggc ttttgtattt ctgcttcagt ggaagtgtgt agggtacaaa atttaaagtg 1320
tgactcttat gcataaagtt ttacaatggc ctgccaggct agggaaagat agggacgaag 1380
cttattcatt attagtgcag agcaggggtg gtcaggctga acgcagcaca gaagggcagc 1440
tcacattctc taagcaagac tggggagcca gcccagcaag aagcttgttt ggacttgcat 1500
taccctatgc tccacctctg tattcagcag aagtgtggtt gccatctttt tcactttatg 1560
taaaggagtg ttgccctcgg gcccttggca gattgccacc ccagcaccta ggttgaagca 1620
cctggtttat aggccctatc tttccctacc cctaaagtca gtccctaagg acaatttccc 1680
agctgatggg gctacacagt agttccaata cagagagttc tggctaagat tttgtttgct 1740
tgtgtctgga tgttgaaaaa gactgcccgt atctcttact ccttccttct ctgtgagtat 1800
tgtaaaaatg gctgttgtga tcactcagct cagcttttgt tattggtacc tcctaaaggg 1860
aaaagtgcaa tattcttgca tcttcagtag tggggaacag gatgtattgt tccggaaaca 1920
ctgaaataca cagcaacatg tgagatgttt taagtagatc acttaggaga cagtggttct 1980
actacatgtt gcattattac aaaatacatt tgctacagga gatataaatc ttatggttgt 2040
aattcagagt ttaaaaatgt tataaattag gttcttgggt cgtgatatga attgttacta 2100

atctttgtga ctatttaatc ttcaaatatt gtgcttaacc ccagcaatcc gcacgtatcc 2160

tgcaccccac cccaaaagag tcatctgtat tttaatgcca ctggtcttat cggtcctttt 2220

gtctgttgag accagtcatg acagcattca agattatgaa agtgttacaa tgccgcttca 2280

agtctgcaaa acctcaaacg tagccaactt gacaaatatt taagtgttac ggcagattta 2340

aaatccatct ggcacaccgt ggtaggtatt tgtacagttc ttttaattac acatagcttt 2400

aaaccatcaa cctgatgagt ttaaagcttt tgcacccatg ccttcacttc agaatgaaca 2460

ccttcattgt gatcttatgt taacctgaga attgatttaa aggaagattg ataatcctat 2520

actttataac gtaaaaatac aggggctaca ggagggtacc taattagaca gttctccaaa 2580

cacagaacac acactggaaa attttccggc caattttgct acctcccaac ttgatggatt 2640

agaggtagcg caaatgctgg tgctcccatc taccttgtag acacttagcc atcaagaatc 2700

aaggcacaag aagtgcactc tctcattaac agtaaatgtt tgcaagatat tcagtttaac 2760

tttcagcatc atgaatgttc ttatccagat tttgaatccg aaaaactata atccttttat 2820

gttatacaaa attactatga ttttttacag ttctgagcat attaaaattc tactggattt 2880

caaaaagaga ctaataccca actgactaac taaacaaata tcaacttgta atactcaatg 2940

aatttttttg ccatttacat ttgaccgttg gctttagtga atgtccatat ttaatttttt 3000

aaggcaccat tacacagttt atcctacatt tatcacattt cttaaagtgt taagattcta 3060

tggctcattt ctatgtattt ttcttacttt acaaaataac ctgaaacagt atagattttg 3120

taacacttaa tttgagcagc ttttttatta cattgaatta tataaagtgc atgttacctt 3180

agaaaaatta atatttgctg ctttactctt ttgcaaaaca tttgctgtaa tgaatggatt 3240

tgtatttcca atatgtatct tgactgcatt ttgtaatatt tactgcttta ttcctaattc 3300

tgctttaaag tactgaactg ggcatgaaac attaaaatat taatccagaa actgtataaa 3360

ctggatgttg cttaaaatct gtatcactgc catgttgaaa actcagactg cttttgtgat 3420

gtttcaaatg aataaaacta tcctcccctc gtta 3454

<210> 27
<211> 108
<212> DNA
<213> Artificial

<220>
<223> Synthetic polynucleotide

<400> 27

gttgtgttca ctctcctcac tgaccccgcc gtgtggtcgc cggctggtgc cttgtctgtc 60

tgccccagcc ccgaatgccc cgagactccc ggcgccggct cggtgcag 108

<210> 28

<211>  2880
<212>  DNA
<213>  Homo sapiens

<400> 28

catttgctat gaatattctc tataacaaag caagacaaat ttagcagcac ttcattgcat      60

ctggatgggg gagagagctg gacaatttct tgctaacaag agatggttaa ctgccctcac     120

ctcagccgtg aattctgcac acctcgcatc cggggcaaca cctgcttctg ctgtgacctc     180

tacaactgtg gcaaccgggt ggagatcact ggtgggtact acgaatacat cgatgtcagc     240

agttgccaag atatcatcca cctctaccac ctgctctggt ctgccaccat cctcaacatt     300

gttggcctgt tcctgggcat catcactgcc gctgtccttg gaggctttaa ggacatgaac     360

ccaactctcc cagcactgaa ctgttctgtt gaaaataccc atccaacagt ttcttactat     420

gctcatcccc aagtggcatc ctacaatacc tactaccata gccctcctca cctgccacca     480

tattctgctt atgactttca gcattccggt gtctttccat cctcccctcc ctctggactt     540

tctgatgagc cccagtctgc ctctccctca cccagctaca tgtggtcctc aagtgcaccg     600

ccccgttact ctccacccta ctatccacct tttgaaaagc caccacctta cagtccctaa     660

agaggaatgc ctgctggcta ttgagattat tgtggctttt gtatttctgc ttcagtggaa     720

gtgtgtaggg tacaaaattt aaagtgtgac tcttatgcat aaagttttac aatggcctgc     780

caggctaggg aaagataggg acgaagctta ttcattatta gtgcagagca ggggtggtca     840

ggctgaacgc agcacagaag ggcagctcac attctctaag caagactggg gagccagccc     900

agcaagaagc ttgtttggac ttgcattacc ctatgctcca cctctgtatt cagcagaagt     960

gtggttgcca tctttttcac tttatgtaaa ggagtgttgc cctcgggccc ttggcagatt    1020

gccaccccag cacctaggtt gaagcacctg gtttataggc cctatctttc cctaccccta    1080

aagtcagtcc ctaaggacaa tttcccagct gatggggcta cacagtagtt ccaatacaga    1140

gagttctggc taagattttg tttgcttgtg tctggatgtt gaaaaagact gcccgtatct    1200

cttactcctt ccttctctgt gagtattgta aaaatggctg ttgtgatcac tcagctcagc    1260

ttttgttatt ggtacctcct aaagggaaaa gtgcaatatt cttgcatctt cagtagtggg    1320

gaacaggatg tattgttccg gaaacactga aatacacagc aacatgtgag atgttttaag    1380

tagatcactt aggagacagt ggttctacta catgttgcat tattacaaaa tacatttgct    1440

acaggagata taaatcttat ggttgtaatt cagagtttaa aaatgttata aattaggttc    1500

ttgggtcgtg atatgaattg ttactaatct ttgtgactat ttaatcttca aatattgtgc    1560

ttaaccccag caatccgcac gtatcctgca ccccacccca aaagagtcat ctgtatttta    1620

atgccactgg tcttatcggt ccttttgtct gttgagacca gtcatgacag cattcaagat    1680

tatgaaagtg ttacaatgcc gcttcaagtc tgcaaaacct caaacgtagc caacttgaca    1740

aatatttaag tgttacggca gatttaaaat ccatctggca caccgtggta ggtatttgta 1800

cagttctttt aattacacat agctttaaac catcaacctg atgagtttaa agcttttgca 1860

cccatgcctt cacttcagaa tgaacacctt cattgtgatc ttatgttaac ctgagaattg 1920

atttaaagga agattgataa tcctatactt tataacgtaa aaatacaggg gctacaggag 1980

ggtacctaat tagacagttc tccaaacaca gaacacacac tggaaaattt tccggccaat 2040

tttgctacct cccaacttga tggattagag gtagcgcaaa tgctggtgct cccatctacc 2100

ttgtagacac ttagccatca agaatcaagg cacaagaagt gcactctctc attaacagta 2160

aatgtttgca agatattcag tttaactttc agcatcatga atgttcttat ccagattttg 2220

aatccgaaaa actataatcc ttttatgtta tacaaaatta ctatgatttt ttacagttct 2280

gagcatatta aaattctact ggatttcaaa aagagactaa tacccaactg actaactaaa 2340

caaatatcaa cttgtaatac tcaatgaatt tttttgccat ttacatttga ccgttggctt 2400

tagtgaatgt ccatatttaa ttttttaagg caccattaca cagtttatcc tacatttatc 2460

acatttctta aagtgttaag attctatggc tcatttctat gtatttttct tactttacaa 2520

aataacctga aacagtatag attttgtaac acttaatttg agcagctttt ttattacatt 2580

gaattatata aagtgcatgt taccttagaa aaattaatat ttgctgcttt actcttttgc 2640

aaaacatttg ctgtaatgaa tggatttgta tttccaatat gtatcttgac tgcattttgt 2700

aatatttact gctttattcc taattctgct ttaaagtact gaactgggca tgaaacatta 2760

aaatattaat ccagaaactg tataaactgg atgttgctta aaatctgtat cactgccatg 2820

ttgaaaactc agactgcttt tgtgatgttt caaatgaata aaactatcct cccctcgtta 2880

<210> 29
<211> 349
<212> PRT
<213> Homo sapiens

<400> 29

Met His Gln Ser Leu Thr Gln Gln Arg Ser Ser Asp Met Ser Leu Pro
1               5                   10                  15

Asp Ser Met Gly Ala Phe Asn Arg Arg Lys Arg Asn Ser Ile Tyr Val
            20                  25                  30

Thr Val Thr Leu Leu Ile Val Ser Val Leu Ile Leu Thr Val Gly Leu
        35                  40                  45

Ala Ala Thr Thr Arg Thr Gln Asn Val Thr Val Gly Gly Tyr Tyr Pro
    50                  55                  60

Gly Val Ile Leu Gly Phe Gly Ser Phe Leu Gly Ile Ile Gly Ser Asn
65                  70                  75                  80

Leu Ile Glu Asn Lys Arg Gln Met Leu Val Ala Ser Ile Val Phe Ile

                    85                  90                  95

Ser Phe Gly Val Ile Ala Ala Phe Cys Cys Ala Ile Val Asp Gly Val
            100                 105                 110

Phe Ala Ala Arg His Ile Asp Leu Lys Pro Leu Tyr Ala Asn Arg Cys
        115                 120                 125

His Tyr Val Pro Lys Thr Ser Gln Lys Glu Ala Glu Glu Val Ile Ser
    130                 135                 140

Ser Ser Thr Lys Asn Ser Pro Ser Thr Arg Val Met Arg Asn Leu Thr
145                 150                 155                 160

Gln Ala Ala Arg Glu Val Asn Cys Pro His Leu Ser Arg Glu Phe Cys
                165                 170                 175

Thr Pro Arg Ile Arg Gly Asn Thr Cys Phe Cys Cys Asp Leu Tyr Asn
            180                 185                 190

Cys Gly Asn Arg Val Glu Ile Thr Gly Gly Tyr Tyr Glu Tyr Ile Asp
        195                 200                 205

Val Ser Ser Cys Gln Asp Ile Ile His Leu Tyr His Leu Leu Trp Ser
    210                 215                 220

Ala Thr Ile Leu Asn Ile Val Gly Leu Phe Leu Gly Ile Ile Thr Ala
225                 230                 235                 240

Ala Val Leu Gly Gly Phe Lys Asp Met Asn Pro Thr Leu Pro Ala Leu
                245                 250                 255

Asn Cys Ser Val Glu Asn Thr His Pro Thr Val Ser Tyr Tyr Ala His
            260                 265                 270

Pro Gln Val Ala Ser Tyr Asn Thr Tyr Tyr His Ser Pro Pro His Leu
        275                 280                 285

Pro Pro Tyr Ser Ala Tyr Asp Phe Gln His Ser Gly Val Phe Pro Ser
    290                 295                 300

Ser Pro Pro Ser Gly Leu Ser Asp Glu Pro Gln Ser Ala Ser Pro Ser
305                 310                 315                 320

Pro Ser Tyr Met Trp Ser Ser Ser Ala Pro Pro Arg Tyr Ser Pro Pro
                325                 330                 335

Tyr Tyr Pro Pro Phe Glu Lys Pro Gln Pro Tyr Ser Pro
            340                 345


<210>  30
<211>  349
<212>  PRT
<213>  Homo sapiens

<400>  30

Met His Gln Ser Leu Thr Gln Gln Arg Ser Ser Asp Met Ser Leu Pro
1               5                   10                  15

Asp Ser Met Gly Ala Phe Asn Arg Arg Lys Arg Asn Ser Ile Tyr Val
            20                  25                  30

Thr Val Thr Leu Leu Ile Val Ser Val Leu Ile Leu Thr Val Gly Leu
        35                  40                  45

Ala Ala Thr Thr Arg Thr Gln Asn Val Thr Val Gly Gly Tyr Tyr Pro
    50                  55                  60

Gly Val Ile Leu Gly Phe Gly Ser Phe Leu Gly Ile Ile Gly Ser Asn
65                  70                  75                  80

Leu Ile Glu Asn Lys Arg Gln Met Leu Val Ala Ser Ile Val Phe Ile
                85                  90                  95

Ser Phe Gly Val Ile Ala Ala Phe Cys Cys Ala Ile Val Asp Gly Val
            100                 105                 110

Phe Ala Ala Arg His Ile Asp Leu Lys Pro Leu Tyr Ala Asn Arg Cys
        115                 120                 125

His Tyr Val Pro Lys Thr Ser Gln Lys Glu Ala Glu Glu Val Ile Ser
    130                 135                 140

Ser Ser Thr Lys Asn Ser Pro Ser Thr Arg Val Met Arg Asn Leu Thr
145                 150                 155                 160

Gln Ala Ala Arg Glu Val Asn Cys Pro His Leu Ser Arg Glu Phe Cys
                165                 170                 175

Thr Pro Arg Ile Arg Gly Asn Thr Cys Phe Cys Cys Asp Leu Tyr Asn
            180                 185                 190

Cys Gly Asn Arg Val Glu Ile Thr Gly Gly Tyr Tyr Glu Tyr Ile Asp
        195                 200                 205

Val Ser Ser Cys Gln Asp Ile Ile His Leu Tyr His Leu Leu Trp Ser
    210                 215                 220

Ala Thr Ile Leu Asn Ile Val Gly Leu Phe Leu Gly Ile Ile Thr Ala
225                 230                 235                 240

Ala Val Leu Gly Gly Phe Lys Asp Met Asn Pro Thr Leu Pro Ala Leu
                245                 250                 255

Asn Cys Ser Val Glu Asn Thr His Pro Thr Val Ser Tyr Tyr Ala His
            260                 265                 270

Pro Gln Val Ala Ser Tyr Asn Thr Tyr Tyr His Ser Pro Pro His Leu
        275                 280                 285

Pro Pro Tyr Ser Ala Tyr Asp Phe Gln His Ser Gly Val Phe Pro Ser
    290                 295                 300

Ser Pro Pro Ser Gly Leu Ser Asp Glu Pro Gln Ser Ala Ser Pro Ser
305                 310                 315                 320

Pro Ser Tyr Met Trp Ser Ser Ser Ala Pro Pro Arg Tyr Ser Pro Pro
                325                 330                 335

Tyr Tyr Pro Pro Phe Glu Lys Pro Pro Pro Tyr Ser Pro
            340                 345

<210>  31

<211>  349
<212>  PRT
<213>  Homo sapiens

<400> 31

<210>  32
<211>  185
<212>  PRT
<213>  Homo sapiens

<400> 32

Met Val Asn Cys Pro His Leu Ser Arg Glu Phe Cys Thr Pro Arg Ile
1               5                   10                  15

Arg Gly Asn Thr Cys Phe Cys Cys Asp Leu Tyr Asn Cys Gly Asn Arg
            20                  25                  30

Val Glu Ile Thr Gly Gly Tyr Tyr Glu Tyr Ile Asp Val Ser Ser Cys
        35                  40                  45

Gln Asp Ile Ile His Leu Tyr His Leu Leu Trp Ser Ala Thr Ile Leu
    50                  55                  60

Asn Ile Val Gly Leu Phe Leu Gly Ile Ile Thr Ala Ala Val Leu Gly
65                  70                  75                  80

Gly Phe Lys Asp Met Asn Pro Thr Leu Pro Ala Leu Asn Cys Ser Val
                85                  90                  95

Glu Asn Thr His Pro Thr Val Ser Tyr Tyr Ala His Pro Gln Val Ala
            100                 105                 110

Ser Tyr Asn Thr Tyr Tyr His Ser Pro Pro His Leu Pro Pro Tyr Ser
        115                 120                 125

Ala Tyr Asp Phe Gln His Ser Gly Val Phe Pro Ser Ser Pro Pro Ser
    130                 135                 140

Gly Leu Ser Asp Glu Pro Gln Ser Ala Ser Pro Ser Pro Ser Tyr Met
145                 150                 155                 160

Trp Ser Ser Ser Ala Pro Pro Arg Tyr Ser Pro Pro Tyr Tyr Pro Pro
                165                 170                 175

Phe Glu Lys Pro Pro Pro Tyr Ser Pro
            180                 185

<210>  33
<211>  325
<212>  PRT
<213>  Homo sapiens

<400> 33

Met His Gln Ser Leu Thr Gln Gln Arg Ser Ser Asp Met Ser Leu Pro
1               5                   10                  15

Asp Ser Met Gly Ala Phe Asn Arg Arg Lys Arg Asn Ser Ile Tyr Val
            20                  25                  30

Thr Val Thr Leu Leu Ile Val Ser Val Leu Ile Leu Thr Val Gly Leu
        35                  40                  45

Ala Ala Thr Thr Arg Thr Gln Asn Val Thr Val Gly Gly Tyr Tyr Pro
    50                  55                  60

Gly Val Ile Leu Gly Phe Gly Ser Phe Leu Gly Ile Ile Gly Ser Asn
65                  70                  75                  80

Leu Ile Glu Asn Lys Arg Gln Met Leu Val Ala Ser Ile Val Phe Ile
                85                  90                  95

Ser Phe Gly Val Ile Ala Ala Phe Cys Cys Ala Ile Val Asp Gly Val
            100                 105                 110

Phe Ala Ala Arg His Ile Asp Leu Lys Pro Leu Tyr Ala Asn Arg Cys
        115                 120                 125

His Tyr Val Pro Lys Thr Ser Gln Lys Glu Ala Glu Glu Val Asn Cys
    130                 135                 140

Pro His Leu Ser Arg Glu Phe Cys Thr Pro Arg Ile Arg Gly Asn Thr
145                 150                 155                 160

Cys Phe Cys Cys Asp Leu Tyr Asn Cys Gly Asn Arg Val Glu Ile Thr
                165                 170                 175

Gly Gly Tyr Tyr Glu Tyr Ile Asp Val Ser Ser Cys Gln Asp Ile Ile
            180                 185                 190

His Leu Tyr His Leu Leu Trp Ser Ala Thr Ile Leu Asn Ile Val Gly
        195                 200                 205

Leu Phe Leu Gly Ile Ile Thr Ala Ala Val Leu Gly Gly Phe Lys Asp
    210                 215                 220

Met Asn Pro Thr Leu Pro Ala Leu Asn Cys Ser Val Glu Asn Thr His
225                 230                 235                 240

Pro Thr Val Ser Tyr Tyr Ala His Pro Gln Val Ala Ser Tyr Asn Thr
                245                 250                 255

Tyr Tyr His Ser Pro Pro His Leu Pro Pro Tyr Ser Ala Tyr Asp Phe
            260                 265                 270

Gln His Ser Gly Val Phe Pro Ser Ser Pro Pro Ser Gly Leu Ser Asp
        275                 280                 285

Glu Pro Gln Ser Ala Ser Pro Ser Pro Ser Tyr Met Trp Ser Ser Ser
    290                 295                 300

Ala Pro Pro Arg Tyr Ser Pro Pro Tyr Tyr Pro Pro Phe Glu Lys Pro
305                 310                 315                 320

Pro Pro Tyr Ser Pro
                325

<210>  34

<211>  25
<212>  DNA
<213>  Artificial

<220>
<223>  Synthetic polynucleotide

<400> 34

aatcattatg ctgaggattt ggaaa                                              25


<210>  35
<211>  241
<212>  PRT
<213>  Homo sapiens

<400> 35

Met His Gln Ser Leu Thr Gln Gln Arg Ser Ser Asp Met Ser Leu Pro
1               5                   10                  15

Asp Ser Met Gly Ala Phe Asn Arg Arg Lys Arg Asn Ser Ile Tyr Val
            20                  25                  30

Thr Val Thr Leu Leu Ile Val Ser Val Leu Ile Leu Thr Val Gly Leu
        35                  40                  45

Ala Ala Thr Thr Arg Thr Gln Asn Val Thr Val Gly Gly Tyr Tyr Pro
    50                  55                  60

Gly Val Ile Leu Gly Phe Gly Ser Phe Leu Gly Ile Ile Gly Ser Asn
65                  70                  75                  80

Leu Ile Glu Asn Lys Arg Gln Met Leu Val Ala Ser Ile Val Phe Ile
                85                  90                  95

Ser Phe Gly Val Ile Ala Ala Phe Cys Cys Ala Ile Val Asp Gly Val
            100                 105                 110

Phe Ala Ala Arg His Ile Asp Leu Lys Pro Leu Tyr Ala Asn Arg Cys
        115                 120                 125

His Tyr Val Pro Lys Thr Ser Gln Lys Glu Ala Glu Glu Asn Pro Thr
    130                 135                 140

Leu Pro Ala Leu Asn Cys Ser Val Glu Asn Thr His Pro Thr Val Ser
145                 150                 155                 160

Tyr Tyr Ala His Pro Gln Val Ala Ser Tyr Asn Thr Tyr Tyr His Ser
                165                 170                 175

Pro Pro His Leu Pro Pro Tyr Ser Ala Tyr Asp Phe Gln His Ser Gly
            180                 185                 190

Val Phe Pro Ser Ser Pro Pro Ser Gly Leu Ser Asp Glu Pro Gln Ser
        195                 200                 205

Ala Ser Pro Ser Pro Ser Tyr Met Trp Ser Ser Ser Ala Pro Pro Arg
    210                 215                 220

Tyr Ser Pro Pro Tyr Tyr Pro Pro Phe Glu Lys Pro Pro Pro Tyr Ser
225                 230                 235                 240

Pro
<210>  36

<211>  328
<212>  PRT
<213>  Homo sapiens

<400> 36

Met His Gln Ser Leu Thr Gln Gln Arg Ser Ser Asp Met Ser Leu Pro
1               5                   10                  15

Asp Ser Met Gly Ala Phe Asn Arg Arg Lys Arg Asn Ser Ile Tyr Val
            20                  25                  30

Thr Val Thr Leu Leu Ile Val Ser Val Leu Ile Leu Thr Val Gly Leu
        35                  40                  45

Ala Ala Thr Thr Arg Thr Gln Asn Val Thr Val Gly Gly Tyr Tyr Pro
    50                  55                  60

Gly Val Ile Leu Val Ala Ser Ile Val Phe Ile Ser Phe Gly Val Ile
65                  70                  75                  80

Ala Ala Phe Cys Cys Ala Ile Val Asp Gly Val Phe Ala Ala Arg His
                85                  90                  95

Ile Asp Leu Lys Pro Leu Tyr Ala Asn Arg Cys His Tyr Val Pro Lys
            100                 105                 110

Thr Ser Gln Lys Glu Ala Glu Glu Val Ile Ser Ser Ser Thr Lys Asn
        115                 120                 125

Ser Pro Ser Thr Arg Val Met Arg Asn Leu Thr Gln Ala Ala Arg Glu
    130                 135                 140

Val Asn Cys Pro His Leu Ser Arg Glu Phe Cys Thr Pro Arg Ile Arg
145                 150                 155                 160

Gly Asn Thr Cys Phe Cys Cys Asp Leu Tyr Asn Cys Gly Asn Arg Val
                165                 170                 175

Glu Ile Thr Gly Gly Tyr Tyr Glu Tyr Ile Asp Val Ser Ser Cys Gln
            180                 185                 190

Asp Ile Ile His Leu Tyr His Leu Leu Trp Ser Ala Thr Ile Leu Asn
        195                 200                 205

Ile Val Gly Leu Phe Leu Gly Ile Ile Thr Ala Ala Val Leu Gly Gly
    210                 215                 220

Phe Lys Asp Met Asn Pro Thr Leu Pro Ala Leu Asn Cys Ser Val Glu
225                 230                 235                 240

Asn Thr His Pro Thr Val Ser Tyr Tyr Ala His Pro Gln Val Ala Ser
                245                 250                 255

Tyr Asn Thr Tyr Tyr His Ser Pro Pro His Leu Pro Pro Tyr Ser Ala
            260                 265                 270

Tyr Asp Phe Gln His Ser Gly Val Phe Pro Ser Ser Pro Pro Ser Gly
        275                 280                 285

Leu Ser Asp Glu Pro Gln Ser Ala Ser Pro Ser Pro Ser Tyr Met Trp
    290                 295                 300

Ser Ser Ser Ala Pro Pro Arg Tyr Ser Pro Pro Tyr Tyr Pro Pro Phe
305                 310                 315                 320

Glu Lys Pro Pro Pro Tyr Ser Pro
                325

<210>  37
<211>  22
<212>  DNA
<213>  Artificial

<220>
<223>  Synthetic polynucleotide

<400> 37

cctcccatct ccttcatcac at                                                22

<210>  38
<211>  3349
<212>  DNA
<213>  Homo sapiens

<400> 38

ggctggagag gcgtgataat agaaccagtg cttcaacagc aacttctctt ttgcaatcct      60

tgctggtctg acaggctcct gcagaattac atcacttcct aaaaaacagg cacacacctc     120

tccttcaagt cagcaccgac ccaaacaagc aggaaacagg aaatgttcac gtttcagaga     180

ggctgcagcc cggcgcagca tcctgagcgc gcctctgccg aggcgagcgg acatgcaggc     240

tccccgcgca gccctagtct tcgccctggt gatcgcgctc gttcccgtcg gccggggtaa     300

ttatgaggaa ttagaaaact caggagatac aactgtggaa tctgaaagac caaataaagt     360

gactattcca agcacatttg ctgcagtgac catcaaagaa acattaaatg caaatataaa     420

ttctaccaac tttgctccgg atgaaaatca gttagagttt atactgatgg tgttaatccc     480

attgatttta ttggtcctct tacttttatc cgtggtattc cttgcaacat actataaaag     540

aaaaagaact aaacaagaac cttctagcca aggatctcag agtgctttac agacatatga     600

actgggaagt gaaaacgtga aagtccctat ttttgaggaa gatacaccct ctgttatgga     660

aattgaaatg gaagagcttg ataaatggat gaacagcatg aatagaaatg ccgactttga     720

atgtttacct accttgaagg aagagaagga atcaaatcac aacccaagtg acagtgaatc     780

ctaaacctga atggcgctca tgttttccaa gagaagcagc ccctgaggga gtctgctgag     840

gctgccaaca gaggatgaag aggatacaaa tttaattaat ttcaaatcaa catagacaca     900

agaacctttt gctgtttctt ccaacgccca ctcttcctaa tgatggcatc acttgcactt     960

ggaagaatgt gcaattgaga agtactagga aaaggcctgg ctgccatcca tcgctgcctc 1020

tgagggtgga gaaggaggcg ggtgatgtgc tcacttctga tcaacatgtg ttgcctcctc 1080

tcagccaact tctagctcac tgcactcact ctggtcatga taaatgttcg tcacctttct 1140

gcttcattcc ttagggccta aatcaggaag ctgttttatc gatggtttcc ttttgggtca 1200

gtaaccagct ttggataatt tcctctgatt attcaagtcg tgggacaggt aaactacatt 1260

cagcaggaac ttttctcgag gagtgttatg tcatggaaaa gacaccaaac acagcaagta 1320

ttttaatgaa tacaccatcc cagggggtca gtaagctctg cctgccaaga agacacagtg 1380

agaggggtcc acagtcctga tgaggtggcg tttggtaact tgtagaccct agcatggcca 1440

ggtctggtca cccttaagaa cttctcagag aaactaggaa tcttcagtga aagaactaat 1500

gttctcctca gctgaaattc ccttgcttgt cagcatttct gcaaagctca cacttgtttc 1560

accatacctc ccttggatgt gacatgtagg taggaagtat gtgcaggtgg gagtcatctg 1620

tcagccttct atgtttcaga gatcctgaag gtggtttgaa acaaacagaa gaggagcagg 1680

aaatatccgt gcctgtggca gatctcactc atcatgctta gcattctctc ccgccaagct 1740

gggataagcc tcatgtccta acacagcaca acaggaggtc tctgtcagtc catcagagat 1800

gacattctat gtgatatttt tgacatcctt gtgctaaaag caatggcaca aaatggaaaa 1860

gggcctattg accacaccta ctccagtaaa attgttcttc atttattcct taattttcta 1920

aatctgaccc ctttaaagca atctagcaaa ttgagaatcc tcagctctcc ttggatacct 1980

gatattttat ttcaagaaag agacaaagaa ggaaaatttt atttatttta ctacccacat 2040

ataaaccgaa gggagatggg actacccaaa catttgctgc tcaattttgt gtcttgtgct 2100

tgaaagtctg ccctaatgca taacaaaaac tacttgtctc ctaccttttg ggatcccttt 2160

aacaagtatt tgccttctga actacgtgga taatttcaaa ggcagagttc cagaccagag 2220

aggtctttcc atacaatgaa agctataact agctggttgt gtttaaccac tgctatcacg 2280

ctatcctggg actgcataga actttgacaa aagacagact tcatggtaca caacttcaac 2340

agattttctg tcatattctc accagcacat ctgaatgagg ctttgtgttt tccttgctct 2400

cttgcatgtt ccttttcaac tcatggccca cagtgactct cagagattta tgccaaaatt 2460

gcatacaatt gttttctgaa tcataacttg tctatttttc tgcctatgtg tgctactttc 2520

agtttgtttc tcatcaacat tttgactctc agaagagcct ccatttgccc ctttctctct 2580

ttaggtatct aagatctttg aacacctgga cctttacatt tgatccaacc ctatcaaata 2640

atgaacttct cagagaggca tctggggtcc tggaacttca tgttgatgaa gtcatattat 2700

ataatatgat aaaaatattc tcatgcagta ttttaaataa tttcaaattc tagaagaagc 2760

aaatttcagc gacatgtcat tgagttttta tttggtaaag ctataattgt gcagtgtaca 2820

aagcactttt taaaaagata gtttattctg tcagggtata tgaagttagt atacagccag 2880

aacagccaag cctcaattct tgtaccttgt gtctttttat tactgtttaa tcaatagata 2940

tcatatgttt atgacagttt caagaattgt ttttaaaccc aaacttaatt ttatgtttca 3000

gactattgtt agaaaaacaa aacaaaaaac aaaaaaaccc tcactaattt gccctaattg 3060

gatagggcaa tcagaacaaa atctgacttt cgaatattta aaagatatgt aagtttgatt 3120

gcattttcgt acattttaag caaactaggt taacaacaac atagcctagt caaacttctc 3180

aggaaacttg ttttaataaa tatgtaaaaa tacccattca tgactcttga cccaatggtg 3240

tgactcctcc ctctgggaag ggctttgtta cccaatgcaa gaccacagcc tggatccacc 3300

ttgatgtagg tggctcctgg ataacccaca tcaaacaaat gtgataaag 3349

<210> 39
<211> 2767
<212> DNA
<213> Homo sapiens

<400> 39

ggctggagag gcgtgataat agaaccagtg cttcaacagc aacttctctt ttgcaatcct 60

tgctggtctg acaggctcct gcagaattac atcacttcct aaaaaacagg cacacacctc 120

tccttcaagt cagcaccgac ccaaacaagc aggaaacagg aaatgttcac gtttcagaga 180

ggctgcagcc cggcgcagca tcctgagcgc gcctctgccg aggcgagcgg acatgcaggc 240

tccccgcgca gccctagtct tcgccctggt gatcgcgctc gttcccgtcg gccggggtaa 300

ttatgaggaa ttagaaaact caggagatac aactgtggaa tctgaaagac caaataaagt 360

gactattcca agcacatttg ctgcagtgac catcaaagaa acattaaatg caaatataaa 420

ttctaccaac tttgctccgg atgaaaatca gttagagttt atactgatgg tgttaatccc 480

attgatttta ttggtcctct tacttttatc cgtggtattc cttgcaacat actataaaag 540

aaaaagaact aaacaagaac cttctagcca aggatctcag agtgctttac agacatatga 600

actgggaagt gaaaacgtga aagtccctat ttttgaggaa gatacaccct ctgttatgga 660

aattgaaatg gaagagcttg ataaatggat gaacagcatg aatagaaatg ccgactttga 720

atgtttacct accttgaagg aagagaagga atcaaatcac aacccaagtg acagtgaatc 780

ctaaacctga atggcgctca tgttttccaa gagaagcagc ccctgaggga gtctgctgag 840

gctgccaaca gaggatgaag aggatacaaa tttaattaat ttcaaatcaa catagacaca 900

agaacctttt gctgtttctt ccaacgccca ctcttcctaa tgatggcatc acttgcactt 960

ggaagaatgt gcaattgaga agtactagga aaaggcctgg ctgccatcca tcgctgcctc 1020

tgagggtgga gaaggaggcg ggtgatgtgc tcacttctga tcaacatgtg ttgcctcctc 1080

tcagccaact tctagctcac tgcactcact ctggtcatga taaatgttcg tcacctttct 1140

gcttcattcc ttagggccta aatcaggaag ctgttttatc gatggtttcc ttttgggtca 1200

gtaaccagct ttggataatt tcctctgatt attcaagtcg tgggacaggt aaactacatt 1260

cagcaggaac ttttctcgag gagtgttatg tcatggaaaa gacaccaaac acagcaagta 1320

ttttaatgaa tacaccatcc cagggggtca gtaagctctg cctgccaaga agacacagtg 1380

agaggggtcc acagtcctga tgaggtggcg tttggtaact tgtagaccct agcatggcca 1440

ggtctggtca cccttaagaa cttctcagag aaactaggaa tcttcagtga aagaactaat 1500

gttctcctca gctgaaattc ccttgcttgt cagcatttct gcaaagctca cacttgtttc 1560

accatacctc ccttggatgt gacatgtagg taggaagtat gtgcaggtgg gagtcatctg 1620

tcagccttct atgtttcaga gatcctgaag gtggtttgaa acaaacagaa gaggagcagg 1680

aaatatccgt gcctgtggca gatctcactc atcatgctta gcattctctc ccgccaagct 1740

gggataagcc tcatgtccta acacagcaca acaggaggtc tctgtcagtc catcagagat 1800

gacattctat gtgatatttt tgacatcctt gtgctaaaag caatggcaca aaatggaaaa 1860

gggcctattg accacaccta ctccagtaaa attgttcttc atttattcct taattttcta 1920

aatctgaccc ctttaaagca atctagcaaa ttgagaatcc tcagctctcc ttggatacct 1980

gatattttat ttcaagaaag agacaaagaa ggaaaatttt atttatttta ctacccacat 2040

ataaaccgaa gggagatggg actacccaaa catttgctgc tcaattttgt gtcttgtgct 2100

tgaaagtctg ccctaatgca taacaaaaac tacttgtctc ctaccttttg ggatcccttt 2160

aacaagtatt tgccttctga actacgtgga taatttcaaa ggcagagttc cagaccagag 2220

aggtctttcc atacaatgaa agctataact agctggttgt gtttaaccac tgctatcacg 2280

ctatcctggg actgcataga actttgacaa aagacagact tcatggtaca caacttcaac 2340

agattttctg tcatattctc accagcacat ctgaatgagg ctttgtgttt tccttgctct 2400

cttgcatgtt ccttttcaac tcatggccca cagtgactct cagagattta tgccaaaatt 2460

gcatacaatt gttttctgaa tcataacttg tctatttttc tgcctatgtg tgctactttc 2520

agtttgtttc tcatcaacat tttgactctc agaagagcct ccatttgccc ctttctctct 2580

ttaggtatct aagatctttg aacacctgga cctttacatt tgatccaacc ctatcaaata 2640

atgaacttct cagagaggca tctggggtcc tggaacttca tgttgatgaa gtcatattat 2700

ataatatgat aaaaatattc tcatgcagta ttttaaataa tttcaaattc tagaagaagc 2760

aaatttc 2767

<210> 40
<211> 3398
<212> DNA
<213> Homo sapiens

<400> 40

ggctggagag gcgtgataat agaaccagtg cttcaacagc aacttctctt ttgcaatcct 60

tgctggtctg acaggctcct gcagaattac atcacttcct aaaaaacagg cacacacctc 120

tccttcaagt cagcaccgac ccaaacaagc aggaaacagg aaatgttcac gtttcagaga 180

ggctgcagcc cggcgcagca tcctgagcgc gcctctgccg aggcgagcgg acatgcaggc 240

tccccgcgca gccctagtct tcgccctggt gatcgcgctc gttcccgtcg gccggggtaa 300

gccgcctcca cttcctactc ctgggaagga ggatcgcaaa agctggtaat tatgaggaat 360

tagaaaactc aggagataca actgtggaat ctgaaagacc aaataaagtg actattccaa 420

gcacatttgc tgcagtgacc atcaaagaaa cattaaatgc aaatataaat tctaccaact 480

ttgctccgga tgaaaatcag ttagagttta tactgatggt gttaatccca ttgattttat 540

tggtcctctt acttttatcc gtggtattcc ttgcaacata ctataaaaga aaaagaacta 600

aacaagaacc ttctagccaa ggatctcaga gtgctttaca gacatatgaa ctgggaagtg 660

aaaacgtgaa agtccctatt tttgaggaag atacaccctc tgttatggaa attgaaatgg 720

aagagcttga taaatggatg aacagcatga atagaaatgc cgactttgaa tgtttaccta 780

ccttgaagga agagaaggaa tcaaatcaca acccaagtga cagtgaatcc taaacctgaa 840

tggcgctcat gttttccaag agaagcagcc cctgagggag tctgctgagg ctgccaacag 900

aggatgaaga ggatacaaat ttaattaatt tcaaatcaac atagacacaa gaaccttttg 960

ctgtttcttc caacgcccac tcttcctaat gatggcatca cttgcacttg gaagaatgtg 1020

caattgagaa gtactaggaa aaggcctggc tgccatccat cgctgcctct gagggtggag 1080

aaggaggcgg gtgatgtgct cacttctgat caacatgtgt tgcctcctct cagccaactt 1140

ctagctcact gcactcactc tggtcatgat aaatgttcgt cacctttctg cttcattcct 1200

tagggcctaa atcaggaagc tgttttatcg atggtttcct tttgggtcag taaccagctt 1260

tggataattt cctctgatta ttcaagtcgt gggacaggta aactacattc agcaggaact 1320

tttctcgagg agtgttatgt catggaaaag acaccaaaca cagcaagtat tttaatgaat 1380

acaccatccc agggggtcag taagctctgc ctgccaagaa gacacagtga gaggggtcca 1440

cagtcctgat gaggtggcgt ttggtaactt gtagacccta gcatggccag gtctggtcac 1500

ccttaagaac ttctcagaga aactaggaat cttcagtgaa agaactaatg ttctcctcag 1560

ctgaaattcc cttgcttgtc agcatttctg caaagctcac acttgtttca ccatacctcc 1620

cttggatgtg acatgtaggt aggaagtatg tgcaggtggg agtcatctgt cagccttcta 1680

tgtttcagag atcctgaagg tggtttgaaa caaacagaag aggagcagga aatatccgtg 1740

cctgtggcag atctcactca tcatgcttag cattctctcc cgccaagctg ggataagcct 1800

catgtcctaa cacagcacaa caggaggtct ctgtcagtcc atcagagatg acattctatg 1860

tgatattttt gacatccttg tgctaaaagc aatggcacaa aatggaaaag ggcctattga 1920

ccacacctac tccagtaaaa ttgttcttca tttattcctt aattttctaa atctgacccc 1980

tttaaagcaa tctagcaaat tgagaatcct cagctctcct tggatacctg atattttatt 2040

tcaagaaaga gacaaagaag gaaaatttta tttattttac tacccacata taaaccgaag 2100

ggagatggga ctacccaaac atttgctgct caattttgtg tcttgtgctt gaaagtctgc 2160

cctaatgcat aacaaaaact acttgtctcc taccttttgg gatcccttta acaagtattt 2220

gccttctgaa ctacgtggat aatttcaaag gcagagttcc agaccagaga ggtctttcca 2280

tacaatgaaa gctataacta gctggttgtg tttaaccact gctatcacgc tatcctggga 2340

ctgcatagaa ctttgacaaa agacagactt catggtacac aacttcaaca gattttctgt 2400

catattctca ccagcacatc tgaatgaggc tttgtgtttt ccttgctctc ttgcatgttc 2460

cttttcaact catggcccac agtgactctc agagatttat gccaaaattg catacaattg 2520

ttttctgaat cataacttgt ctatttttct gcctatgtgt gctactttca gtttgtttct 2580

catcaacatt ttgactctca gaagagcctc catttgcccc tttctctctt taggtatcta 2640

agatctttga acacctggac ctttacattt gatccaaccc tatcaaataa tgaacttctc 2700

agagaggcat ctggggtcct ggaacttcat gttgatgaag tcatattata taatatgata 2760

aaaatattct catgcagtat tttaaataat ttcaaattct agaagaagca aatttcagcg 2820

acatgtcatt gagtttttat ttggtaaagc tataattgtg cagtgtacaa agcacttttt 2880

aaaaagatag tttattctgt cagggtatat gaagttagta tacagccaga acagccaagc 2940

ctcaattctt gtaccttgtg tctttttatt actgtttaat caatagatat catatgttta 3000

tgacagtttc aagaattgtt tttaaaccca aacttaattt tatgtttcag actattgtta 3060

gaaaaacaaa acaaaaaaca aaaaaaccct cactaatttg ccctaattgg atagggcaat 3120

cagaacaaaa tctgactttc gaatatttaa aagatatgta agtttgattg cattttcgta 3180

cattttaagc aaactaggtt aacaacaaca tagcctagtc aaacttctca ggaaacttgt 3240

tttaataaat atgtaaaaat acccattcat gactcttgac ccaatggtgt gactcctccc 3300

tctgggaagg gctttgttac ccaatgcaag accacagcct ggatccacct tgatgtaggt 3360

ggctcctgga taacccacat caaacaaatg tgataaag 3398

<210> 41
<211> 776
<212> DNA
<213> Homo sapiens

<400> 41

ggctggagag gcgtgataat agaaccagtg cttcaacagc aacttctctt ttgcaatcct 60

tgctggtctg acaggctcct gcagaattac atcacttcct aaaaaacagg cacacacctc 120

tccttcaagt cagcaccgac ccaaacaagc aggaaacagg aaatgttcac gtttcagaga 180

ggctgcagcc cggcgcagca tcctgagcgc gcctctgccg aggcgagcgg acatgcaggc 240

tccccgcgca gccctagtct tcgccctggt gatcgcgctc gttcccgtcg gccggggtaa 300

ttatgaggaa ttagaaaact caggagatac aactgtggaa tctgaaagac caaataaagt 360

gactattcca agcacatttg ctgcagtgac catcaaagaa acattaaatg caaatataaa 420

ttctaccaac tttgctccgg atgaaaatca gttagagttt atactgatgg tgttaatccc 480

attgatttta ttggtcctct tacttttatc cgtggtattc cttgcaacat actataaaag 540

aaaaagaact aaacaagaac cttctagcca aggatctcag agtgctttac agacatgtaa 600

aatacaacta tcatggaagg tcataccagc gttttgcttg gaaagctccc acagaaatgc 660

tttataggag agcatggaca cctgcacact atgtcccctg ggaagttaga agcagacata 720

ctgtttctta ctgcacttgg gtagagcctg gtctgttata ctatactagg aaataa 776

<210> 42
<211> 183
<212> PRT
<213> Homo sapiens

<400> 42

Met Gln Ala Pro Arg Ala Ala Leu Val Phe Ala Leu Val Ile Ala Leu
1               5                   10                  15

Val Pro Val Gly Arg Gly Asn Tyr Glu Glu Leu Glu Asn Ser Gly Asp
            20                  25                  30

Thr Thr Val Glu Ser Glu Arg Pro Asn Lys Val Thr Ile Pro Ser Thr
        35                  40                  45

Phe Ala Ala Val Thr Ile Lys Glu Thr Leu Asn Ala Asn Ile Asn Ser
    50                  55                  60

Thr Asn Phe Ala Pro Asp Glu Asn Gln Leu Glu Phe Ile Leu Met Val
65                  70                  75                  80

Leu Ile Pro Leu Ile Leu Leu Val Leu Leu Leu Leu Ser Val Val Phe
                85                  90                  95

Leu Ala Thr Tyr Tyr Lys Arg Lys Arg Thr Lys Gln Glu Pro Ser Ser
            100                 105                 110

Gln Gly Ser Gln Ser Ala Leu Gln Thr Tyr Glu Leu Gly Ser Glu Asn
        115                 120                 125

Val Lys Val Pro Ile Phe Glu Glu Asp Thr Pro Ser Val Met Glu Ile
    130                 135                 140

Glu Met Glu Glu Leu Asp Lys Trp Met Asn Ser Met Asn Arg Asn Ala
145                 150                 155                 160

Asp Phe Glu Cys Leu Pro Thr Leu Lys Glu Glu Lys Glu Ser Asn His
                165                 170                 175

Asn Pro Ser Asp Ser Glu Ser
                180

<210>  43
<211>  128
<212>  PRT
<213>  Homo sapiens

<400> 43

Glu Thr Leu Asn Ala Asn Ile Asn Ser Thr Asn Phe Ala Pro Asp Glu
1               5                   10                  15

Asn Gln Leu Glu Phe Ile Leu Met Val Leu Ile Pro Leu Ile Leu Leu
            20                  25                  30

Val Leu Leu Leu Leu Ser Val Val Phe Leu Ala Thr Tyr Tyr Lys Arg
        35                  40                  45

Lys Arg Thr Lys Gln Glu Pro Ser Ser Gln Gly Ser Gln Ser Ala Leu
    50                  55                  60

Gln Thr Tyr Glu Leu Gly Ser Glu Asn Val Lys Val Pro Ile Phe Glu
65                  70                  75                  80

Glu Asp Thr Pro Ser Val Met Glu Ile Glu Met Glu Glu Leu Asp Lys
                85                  90                  95

Trp Met Asn Ser Met Asn Arg Asn Ala Asp Phe Glu Cys Leu Pro Thr
            100                 105                 110

Leu Lys Glu Glu Lys Glu Ser Asn His Asn Pro Ser Asp Ser Glu Ser
        115                 120                 125

<210>  44
<211>  183
<212>  PRT
<213>  Homo sapiens

<400> 44

Met Gln Ala Pro Arg Ala Ala Leu Val Phe Ala Leu Val Ile Ala Leu
1               5                   10                  15

Val Pro Val Gly Arg Gly Asn Tyr Glu Glu Leu Glu Asn Ser Gly Asp
            20                  25                  30

Thr Thr Val Glu Ser Glu Arg Pro Asn Lys Val Thr Ile Pro Ser Thr
        35                  40                  45

Phe Ala Ala Val Thr Ile Lys Glu Thr Leu Asn Ala Asn Ile Asn Ser
    50                  55                  60

Thr Asn Phe Ala Pro Asp Glu Asn Gln Leu Glu Phe Ile Leu Met Val
65                  70                  75                  80

Leu Ile Pro Leu Ile Leu Leu Val Leu Leu Leu Leu Phe Val Val Phe
                85                  90                  95

Leu Ala Thr Tyr Tyr Lys Arg Lys Arg Thr Lys Gln Glu Pro Ser Ser

100                 105                 110

Gln Gly Ser Gln Ser Ala Leu Gln Thr Tyr Glu Leu Gly Ser Glu Asn
        115                 120                 125

Val Lys Val Pro Ile Phe Glu Glu Asp Thr Pro Ser Val Met Glu Ile
    130                 135                 140

Glu Met Glu Glu Leu Asp Lys Trp Met Asn Ser Met Asn Arg Asn Ala
145                 150                 155                 160

Asp Phe Glu Cys Leu Pro Thr Leu Lys Glu Glu Lys Glu Ser Asn His
                165                 170                 175

Asn Pro Ser Asp Ser Glu Ser
            180


<210>  45
<211>  105
<212>  PRT
<213>  Homo sapiens

<400> 45


<210>  46
<211>  144
<212>  PRT
<213>  Homo sapiens

<400> 46

Met Gln Ala Pro Arg Ala Ala Leu Val Phe Ala Leu Val Ile Ala Leu
1               5                   10                  15

Val Pro Val Gly Arg Gly Asn Tyr Glu Glu Leu Glu Asn Ser Gly Asp
            20                  25                  30

Thr Thr Val Glu Ser Glu Arg Pro Asn Lys Val Thr Ile Pro Ser Thr
        35                  40                  45

Phe Ala Ala Val Thr Ile Lys Glu Thr Leu Asn Ala Asn Ile Asn Ser
    50                  55                  60

Thr Asn Phe Ala Pro Asp Glu Asn Gln Leu Glu Phe Ile Leu Met Val
65                  70                  75                  80

Leu Ile Pro Leu Ile Leu Leu Val Leu Leu Leu Leu Ser Val Val Phe
                85                  90                  95

Leu Ala Thr Tyr Tyr Lys Arg Lys Arg Thr Lys Gln Glu Pro Ser Ser
            100                 105                 110

Gln Gly Ser Gln Ser Ala Leu Gln Thr Cys Lys Ile Gln Leu Ser Trp
        115                 120                 125

Lys Val Ile Pro Ala Phe Cys Leu Glu Ser Ser His Arg Asn Ala Leu
    130                 135                 140


<210>  47
<211>  21
<212>  PRT
<213>

<213>  Artificial

<220>
<223>  Synthetic polypeptide

<400> 47

Thr Val Leu Arg His Val Ala Asn Pro Arg Gly Ala Val Thr Pro Glu
1               5                   10                  15

Tyr Thr Val Ala Asn
            20


<210>  48
<211>  48
<212>  PRT
<213>  Artificial

<220>
<223>  Synthetic polypeptide

<400> 48

Leu Ala Val Ser Leu Thr Val Ala Asn Gly Gly Arg Arg Leu Ile Ala
1               5                   10                  15

Asp Cys His Pro Gly Leu Leu Asp Pro Leu Val Pro Leu Asp Glu Gly
            20                  25                  30

Pro Gly His Thr Asp Cys Pro Phe Asp Pro Thr Arg Ile Tyr Asp Thr
        35                  40                  45


<210>  49
<211>  20
<212>  PRT
<213>  Artificial

<220>
<223>  Synthetic polypeptide

<400> 49

Met Arg Arg Cys Ser Leu Cys Ala Phe Asp Ala Ala Arg Gly Pro Arg
1               5                   10                  15

Arg Leu Met Arg
            20


<210>  50
<211>  15
<212>  PRT
<213>  Artificial

<220>
<223>  Synthetic polypeptide

<400> 50

Asp Pro Gly Gly Gly Arg Ala Pro Gly Glu Pro Ser Arg Pro Lys
1               5                   10                  15


<210>  51
<211>  57
<212>  PRT
<213>  Artificial

<220>
<223>  Synthetic polypeptide

<400> 51

Cys Cys Val Ala Ala Leu Thr Leu Arg Gly Val Gly Pro Cys Arg Lys
1               5                   10                  15

Asp Gly Leu Gln Gly Gln Leu Glu Glu Met Thr Glu Leu Glu Ser Pro
            20                  25                  30

Lys Cys Lys Arg Gln Glu Asn Glu Gln Leu Leu Asp Gln Asn Gln Glu
        35                  40                  45

Ile Arg Ala Ser Gln Arg Ser Trp Val
    50                  55


<210>  52
<211>  9
<212>  PRT
<213>  Artificial

<220>
<223>  Synthetic polypeptide

<400> 52

Gln Cys Pro Cys Ser Ala Ala Trp Asn
1               5

<210>  53
<211>  51
<212>  PRT
<213>  Artificial

<220>
<223>  Synthetic polypeptide

<400> 53

Glu Cys Ala Ala Thr Gly Ser Ala Ala Phe Ala Gln Arg Leu Cys Leu
1               5                   10                  15

Gly Arg Asn Arg Ser Cys Ala Ala Glu Leu Pro Leu Val Pro Cys Asn
            20                  25                  30

Gln Ala Lys Ala Ser Asp Val Gln Asp Leu Leu Lys Asp Leu Lys Ala
        35                  40                  45

Gln Ser Gln
    50


<210>  54
<211>  9
<212>  PRT
<213>  Artificial

<220>
<223>  Synthetic polypeptide

<400> 54

Thr Thr Arg Thr Gln Asn Val Thr Val
1               5


<210>  55
<211>  116
<212>  PRT
<213>  Homo sapiens

<400> 55

Asp Gly Val Phe Ala Ala Arg His Ile Asp Leu Lys Pro Leu Tyr Ala
1               5                   10                  15

Asn Arg Cys His Tyr Val Pro Lys Thr Ser Gln Lys Glu Ala Glu Glu
            20                  25                  30

Val Ile Ser Ser Ser Thr Lys Asn Ser Pro Ser Thr Arg Val Met Arg
        35                  40                  45

Asn Leu Thr Gln Ala Ala Arg Glu Val Asn Cys Pro His Leu Ser Arg
    50                  55                  60

Glu Phe Cys Thr Pro Arg Ile Arg Gly Asn Thr Cys Phe Cys Cys Asp
65                  70                  75                  80

Leu Tyr Asn Cys Gly Asn Arg Val Glu Ile Thr Gly Gly Tyr Tyr Glu
                85                  90                  95

Tyr Ile Asp Val Ser Ser Cys Gln Asp Ile Ile His Leu Tyr His Leu

100                 105                 110

Leu Trp Ser Ala
        115



<210>  56
<211>  92
<212>  PRT
<213>  Homo sapiens

<400> 56

Asp Gly Val Phe Ala Ala Arg His Ile Asp Leu Lys Pro Leu Tyr Ala
1               5                   10                  15

Asn Arg Cys His Tyr Val Pro Lys Thr Ser Gln Lys Glu Ala Glu Glu
            20                  25                  30

Val Asn Cys Pro His Leu Ser Arg Glu Phe Cys Thr Pro Arg Ile Arg
        35                  40                  45

Gly Asn Thr Cys Phe Cys Cys Asp Leu Tyr Asn Cys Gly Asn Arg Val
    50                  55                  60

Glu Ile Thr Gly Gly Tyr Tyr Glu Tyr Ile Asp Val Ser Ser Cys Gln
65                  70                  75                  80

Asp Ile Ile His Leu Tyr His Leu Leu Trp Ser Ala
                85                  90



<210>  57
<211>  106
<212>  PRT
<213>  Homo sapiens

<400> 57

Gly Gly Phe Lys Asp Met Asn Pro Thr Leu Pro Ala Leu Asn Cys Ser
1               5                   10                  15

Val Glu Asn Thr His Pro Thr Val Ser Tyr Tyr Ala His Pro Gln Val
            20                  25                  30

Ala Ser Tyr Asn Thr Tyr Tyr His Ser Pro Pro His Leu Pro Pro Tyr
        35                  40                  45

Ser Ala Tyr Asp Phe Gln His Ser Gly Val Phe Pro Ser Ser Pro Pro
    50                  55                  60

Ser Gly Leu Ser Asp Glu Pro Gln Ser Ala Ser Pro Ser Pro Ser Tyr
65                  70                  75                  80

Met Trp Ser Ser Ser Ala Pro Pro Arg Tyr Ser Pro Pro Tyr Tyr Pro
                85                  90                  95

Pro Phe Glu Lys Pro Pro Pro Tyr Ser Pro
            100                 105



<210>  58
<211>  132

<212>  PRT
<213>  Homo sapiens

<400> 58

Asp Gly Val Phe Ala Ala Arg His Ile Asp Leu Lys Pro Leu Tyr Ala
1               5                   10                  15

Asn Arg Cys His Tyr Val Pro Lys Thr Ser Gln Lys Glu Ala Glu Glu
            20                  25                  30

Asn Pro Thr Leu Pro Ala Leu Asn Cys Ser Val Glu Asn Thr His Pro
        35                  40                  45

Thr Val Ser Tyr Tyr Ala His Pro Gln Val Ala Ser Tyr Asn Thr Tyr
    50                  55                  60

Tyr His Ser Pro Pro His Leu Pro Pro Tyr Ser Ala Tyr Asp Phe Gln
65                  70                  75                  80

His Ser Gly Val Phe Pro Ser Ser Pro Pro Ser Gly Leu Ser Asp Glu
                85                  90                  95

Pro Gln Ser Ala Ser Pro Ser Pro Ser Tyr Met Trp Ser Ser Ser Ala
            100                 105                 110

Pro Pro Arg Tyr Ser Pro Pro Tyr Tyr Pro Pro Phe Glu Lys Pro Pro
        115                 120                 125

Pro Tyr Ser Pro
    130


<210>  59
<211>  15
<212>  PRT
<213>  Artificial

<220>
<223>  Synthetic polypeptide

<400> 59

Thr Thr Arg Thr Gln Asn Val Thr Val Gly Gly Tyr Tyr Pro Gly
1               5                   10                  15


<210>  60
<211>  55
<212>  PRT
<213>  Homo sapiens

<400> 60

Met Ser Gly Ala Cys Thr Ser Tyr Val Ser Ala Glu Gln Glu Val Val
1               5                   10                  15

Arg Gly Phe Ser Cys Pro Arg Pro Gly Gly Glu Ala Ala Ala Val Phe
            20                  25                  30

Cys Cys Gly Phe Arg Asp His Lys Tyr Cys Cys Asp Asp Pro His Ser
        35                  40                  45

Phe Phe Pro Tyr Glu His Ser
    50                  55

<210>  61
<211>  100
<212>  PRT
<213>  Homo sapiens

<400> 61

Ser Ser Lys Pro His Thr Lys Leu Asp Leu Gly Leu Ser Leu Gln Thr
1               5                   10                  15

Ala Gly Pro Glu Glu Val Ser Pro Asp Cys Gln Gly Val Asn Thr Gly
            20                  25                  30

Met Ala Ala Glu Val Pro Lys Val Ser Pro Leu Gln Gln Ser Tyr Ser
        35                  40                  45

Cys Leu Asn Pro Gln Leu Glu Ser Asn Glu Gly Gln Ala Val Asn Ser
    50                  55                  60

Lys Arg Leu Leu His His Cys Phe Met Ala Thr Val Thr Thr Ser Asp
65                  70                  75                  80

Ile Pro Gly Ser Pro Glu Glu Ala Ser Val Pro Asn Pro Asp Leu Cys
                85                  90                  95

Gly Pro Val Pro
            100

<210>  62
<211>  71
<212>  PRT
<213>  Homo sapiens

<400> 62

Met Ala Ser Leu Trp Pro Ser Ala Leu Thr Phe Asn Thr Asp Ala Asn
1               5                   10                  15

Ile Pro Gly Pro Leu Gly Phe Cys Gly Gly Trp Val Arg Leu Cys Ser
            20                  25                  30

Leu Ser Ser Leu Thr Pro Pro Cys Gly Arg Arg Leu Val Pro Cys Leu
        35                  40                  45

Ser Ala Pro Ala Pro Asn Ala Pro Arg Leu Pro Ala Pro Ala Arg Cys
    50                  55                  60

Ser Ile Gly Ala Leu Ile Gly
65                  70

<210>  63
<211>  51
<212>  PRT
<213>  Homo sapiens

<400> 63

Glu Glu Leu Glu Asn Ser Gly Asp Thr Thr Val Glu Ser Glu Arg Pro
1               5                   10                  15

Asn Lys Val Thr Ile Pro Ser Thr Phe Ala Ala Val Thr Ile Lys Glu
            20                  25                  30

Thr Leu Asn Ala Asn Ile Asn Ser Thr Asn Phe Ala Pro Asp Glu Asn
        35                  40                  45

Gln Leu Glu
    50

<210>  64
<211>  86
<212>  PRT
<213>  Homo sapiens

<400> 64

Ala Thr Tyr Tyr Lys Arg Lys Arg Thr Lys Gln Glu Pro Ser Ser Gln
1               5                   10                  15

Gly Ser Gln Ser Ala Leu Gln Thr Tyr Glu Leu Gly Ser Glu Asn Val
            20                  25                  30

Lys Val Pro Ile Phe Glu Glu Asp Thr Pro Ser Val Met Glu Ile Glu
        35                  40                  45

Met Glu Glu Leu Asp Lys Trp Met Asn Ser Met Asn Arg Asn Ala Asp
    50                  55                  60

Phe Glu Cys Leu Pro Thr Leu Lys Glu Glu Lys Glu Ser Asn His Asn
65                  70                  75                  80

Pro Ser Asp Ser Glu Ser
                85

<210>  65
<211>  21
<212>  DNA
<213>  Artificial

<220>
<223>  Synthetic polynucleotide

<400> 65

aatttgtcaa gtcggtgcag c                                                21

<210>  66
<211>  20
<212>  DNA
<213>  Artificial

<220>
<223>  Synthetic polynucleotide

<400> 66

tcaccccttc atttttgcgt                                                  20

<210>  67
<211>  106
<212>  DNA
<213>  Artificial

<220>
<223>  Synthetic polynucleotide

<400> 67
   aatttgtcaa gtcggtgcag ctggcaagac ctaaaggatt atatgcgtca ggcaggagaa      60
   gtgacttatg cagatgctca caagggacgc aaaaatgaag gggtga                     106


<210>  68
<211>  21
<212>  DNA
<213>  Artificial

<220>
<223>  Synthetic polynucleotide

<400> 68

gctccaggcc ataaggactt c                                                21


<210>  69
<211>  21
<212>  DNA
<213>  Artificial

<220>
<223>  Synthetic polynucleotide

<400> 69

cagcttcaaa ctctcccctg c                                                21


<210>  70
<211>  103
<212>  DNA
<213>  Artificial

<220>
<223>  Synthetic polynucleotide

<400> 70

gctccaggcc ataaggactt cattccaaat atgattacag gagcagccca ggcggatgta      60

gctgttttag ttgtagatgc cagcagggga gagtttgaag ctg                       103


<210>  71
<211>  20
<212>  DNA
<213>  Artificial

<220>
<223>  Synthetic polynucleotide

<400> 71

ttccttgcca ggacctagag                                                    20


<210>  72
<211>  20
<212>  DNA
<213>  Artificial

<220>
<223>  Synthetic polynucleotide

<400> 72

cataaacctt tcgccttgac                                                    20


<210>  73
<211>  128
<212>  DNA
<213>  Artificial

<220>
<223>  Synthetic polynucleotide

<400> 73

ttccttgcca ggacctagag tttgttcagt tccaccccac aggcatatat ggtgctggtt      60

gtctcattac ggaaggatgt cgtggagagg gaggcattct cattaacagt caaggcgaaa     120

ggtttatg                                                                128


<210>  74
<211>  20
<212>  DNA
<213>  Artificial

<220>
<223>  Synthetic polynucleotide

<400> 74

cagccaggtt atgccaacac                                                    20


<210>  75
<211>  21
<212>  DNA
<213>  Artificial

<220>
<223>  Synthetic polynucleotide

<400> 75

tcaaagcagg cgaacttcat c                                                 20

<210>  76
<211>  140
<212>  DNA
<213>  Artificial

<220>
<223>  Synthetic polynucleotide

<400> 76

cagccaggtt atgccaacac tttgagggat gcagctccca aaatgtataa ggaagaaggc      60

ctaaaagcat tctacaaggg ggttgctcct ctctggatga gacagatacc atacaccatg     120

atgaagttcg cctgctttga                                                 140


<210>  77
<211>  20
<212>  DNA
<213>  Artificial

<220>
<223>  Synthetic polynucleotide

<400> 77

cggtttgctg cggtaatcat                                                  20


<210>  78
<211>  21
<212>  DNA
<213>  Artificial

<220>
<223>  Synthetic polynucleotide

<400> 78

tttcttgctg ccagtctgga c                                                21


<210>  79
<211>  122
<212>  DNA
<213>  Artificial

<220>
<223>  Synthetic polynucleotide

<400> 79

cggtttgctg cggtaatcat gaggataaga gagccacgaa ccacggcact gattttcagt      60

tctgggaaaa tggtgtgcac aggagccaag agtgaagaac agtccagact ggcagcaaga     120

aa                                                                    122


<210>  80
<211>  19

<212>  DNA
<213>  Artificial

<220>
<223>  Synthetic polynucleotide

<400> 80

atttgggtcg cggttcttg                                                    19


<210>  81
<211>  21
<212>  DNA
<213>  Artificial

<220>
<223>  Synthetic polynucleotide

<400> 81

tgccttgaca ttctcgatgg t                                                 21


<210>  82
<211>  133
<212>  DNA
<213>  Artificial

<220>
<223>  Synthetic polynucleotide

<400> 82

atttgggtcg cggttcttgt ttgtggatcg ctgtgatcgt cacttgacaa tgcagatctt      60

cgtgaagact ctgactggta agaccatcac cctcgaggtt gagcccagtg acaccatcga     120

gaatgtcaag gca                                                         133


<210>  83
<211>  20
<212>  DNA
<213>  Artificial

<220>
<223>  Synthetic polynucleotide

<400> 83

tgggaacaag agggcatctg                                                   20


<210>  84
<211>  22
<212>  DNA
<213>  Artificial

<220>
<223>  Synthetic polynucleotide

<400> 84

ccaccactgc atcaaattca tg                                                22

<210>  85
<211>  86
<212>  DNA
<213>  Artificial

<220>
<223>  Synthetic polynucleotide

<400> 85

tgggaacaag agggcatctg ctaaagtttc agattccatt tctgctcagt atccagtagt      60

ggatcatgaa tttgatgcag tggtgg                                            86

<210>  86
<211>  21
<212>  DNA
<213>  Artificial

<220>
<223>  Synthetic polynucleotide

<400> 86

tgacactggc aaaacaatgc a                                                 21

<210>  87
<211>  21
<212>  DNA
<213>  Artificial

<220>
<223>  Synthetic polynucleotide

<400> 87

ggtccttttc accagcaagc t                                                 21

<210>  88
<211>  94
<212>  DNA
<213>  Artificial

<220>
<223>  Synthetic polynucleotide

<400> 88

tgacactggc aaaacaatgc agactttgct ttccttggtc aggcagtata atccaaagat      60

ggtcaaggtc gcaagcttgc tggtgaaaag gacc                                   94

<210>  89
<211>  20
<212>  DNA

<213>  Artificial

<220>
<223>  Synthetic polynucleotide

<400> 89

gaggccgtca ccaagaacat                                                    20


<210>  90
<211>  19
<212>  DNA
<213>  Artificial

<220>
<223>  Synthetic polynucleotide

<400> 90

ggacagccgg tcagagctc                                                     19


<210>  91
<211>  111
<212>  DNA
<213>  Artificial

<220>
<223>  Synthetic polynucleotide

<400> 91

gaggccgtca ccaagaacat tcacgagtcc tgcatgagcc agataggctg gaaccgcatc      60

atcgtggaga agcccttcgg gagggacctg cagagctctg accggctgtc c               111


<210>  92
<211>  19
<212>  DNA
<213>  Artificial

<220>
<223>  Synthetic polynucleotide

<400> 92

cccctttgac cccacaaga                                                     19


<210>  93
<211>  21
<212>  DNA
<213>  Artificial

<220>
<223>  Synthetic polynucleotide

<400> 93

cagccacaca gcagtaacca g                                                  21

<210>  94
<211>  107
<212>  DNA
<213>  Artificial

<220>
<223>  Synthetic polynucleotide

<400> 94

cccctttgac cccacaagaa tctatgatac agccttggct ctctggatcc cttctttgct      60

catgtctgca ggggaggctg ctctatctgg ttactgctgt gtggctg                   107


<210>  95
<211>  16
<212>  DNA
<213>  Artificial

<220>
<223>  Synthetic polynucleotide

<400> 95

ggccaaggcg tcggac                                                      16


<210>  96
<211>  25
<212>  DNA
<213>  Artificial

<220>
<223>  Synthetic polynucleotide

<400> 96

gaaaactaac tggagatagg catcg                                            25


<210>  97
<211>  143
<212>  DNA
<213>  Artificial

<220>
<223>  Synthetic polynucleotide

<400> 97

ggccaaggcg tcggacgtgc aggacctcct gaaggatctg aaggctcagt cgcaggtgtt      60

gggctggatc ttgatagcag ttgttatcat cattcttctg atttttacat ctgtcacccg     120

atgcctatct ccagttagtt ttc                                             143


<210>  98
<211>  18
<212>  DNA
<213>  Artificial

<220>
<223>  Synthetic polynucleotide

<400> 98

tcggacgtgc aggacctc                                                    18


<210>  99
<211>  21
<212>  DNA
<213>  Artificial

<220>
<223>  Synthetic polynucleotide

<400> 99

gagataggca tcgggtgaca g                                                21


<210>  100
<211>  121
<212>  DNA
<213>  Artificial

<220>
<223>  Synthetic polynucleotide

<400> 100

tcggacgtgc aggacctcct gaaggatctg aaggctcagt cgcaggtgtt gggctggatc      60

ttgatagcag ttgttatcat cattcttctg atttttacat ctgtcacccg atgcctatct     120

c                                                                     121


<210>  101
<211>  26
<212>  DNA
<213>  Artificial

<220>
<223>  Synthetic polynucleotide

<400> 101

ctggtggctt ctatcgtgtt tatcag                                           26


<210>  102
<211>  23
<212>  DNA
<213>  Artificial

<220>
<223>  Synthetic polynucleotide

<400> 102

cggttagcgt agagtggttt cag                                              23

<210>  103
<211>  116
<212>  DNA
<213>  Artificial

<220>
<223>  Synthetic polynucleotide

<400> 103

ctggtggctt ctatcgtgtt tatcagcttt ggtgtgattg cggctttttg ttgtgccata     60

gttgacgggg tctttgctgc cagacacatt gatctgaaac cactctacgc taaccg        116


<210>  104
<211>  25
<212>  DNA
<213>  Artificial

<220>
<223>  Synthetic polynucleotide

<400> 104

ccttctagcc aaggatctca gagtg                                           25


<210>  105
<211>  26
<212>  DNA
<213>  Artificial

<220>
<223>  Synthetic polynucleotide

<400> 105

cttgggttgt gatttgattc cttctc                                          26


<210>  106
<211>  209
<212>  DNA
<213>  Artificial

<220>
<223>  Synthetic polynucleotide

<400> 106

ccttctagcc aaggatctca gagtgcttta cagacatatg aactgggaag tgaaaacgtg     60

aaagtcccta tttttgagga agatacaccc tctgttatgg aaattgaaat ggaagagctt    120

gataaatgga tgaacagcat gaatagaaat gccgactttg aatgtttacc taccttgaag    180

gaagagaagg aatcaaatca caacccaag                                      209


<210>  107

<211>  21
<212>  DNA
<213>  Artificial

<220>
<223>  Synthetic polynucleotide

<400> 107

cagcattggc gctctcatag g                                                21


<210>  108
<211>  22
<212>  DNA
<213>  Artificial

<220>
<223>  Synthetic polynucleotide

<400> 108

gtgttcacac cttggcagtc ag                                               22


<210>  109
<211>  183
<212>  DNA
<213>  Artificial

<220>
<223>  Synthetic polynucleotide

<400> 109

cagcattggc gctctcatag gcctgtccgt agcagcagtg gttcttctcg ccttcattgt      60

taccgcctgt gtgctctgct acctgttcat cagctctaag ccccacacaa agttggacct     120

gggcttgagc ttacagacag caggccctga ggaggtttct cctgactgcc aaggtgtgaa     180

cac                                                                   183


<210>  110
<211>  1455
<212>  DNA
<213>  Homo sapiens

<400> 110

atgaggcgct gcagtctctg cgctttcgac gccgcccggg ggcccaggcg gctgatgcgt      60

gtgggcctcg cgctgatctt ggtgggccac gtgaacctgc tgctgggggc cgtgctgcat     120

ggcaccgtcc tgcggcacgt ggccaatccc cgcggcgctg tcacgccgga gtacaccgta     180

gccaatgtca tctctgtcgg ctcggggctg ctgagcgttt ccgtgggact tgtggccctc     240

ctggcgtcca ggaaccttct tcgccctcca ctgcactggg tcctgctggc actagctctg     300

gtgaacctgc tcttgtccgt tgcctgctcc ctgggcctcc ttcttgctgt gtcactcact     360

gtggccaacg gtggccgccg ccttattgct gactgccacc caggactgct ggatcctctg 420

gtaccactgg atgaggggcc gggacatact gactgcccct ttgaccccac aagaatctat 480

gatacagcct tggctctctg gatcccttct ttgctcatgt ctgcagggga ggctgctcta 540

tctggttact gctgtgtggc tgcactcact ctacgtggag ttgggccctg caggaaggac 600

ggacttcagg ggcagctaga ggaaatgaca gagcttgaat ctcctaaatg taaaaggcag 660

gaaaatgagc agctactgga tcaaaatcaa gaaatccggg catcacagag aagttgggtt 720

ggaccggtcg ccaccatggt gagcaagggc gaggagctgt tcaccggggt ggtgcccatc 780

ctggtcgagc tggacggcga cgtaaacggc cacaagttca gcgtgtccgg cgagggcgag 840

ggcgatgcca cctacggcaa gctgaccctg aagttcatct gcaccaccgg caagctgccc 900

gtgccctggc ccaccctcgt gaccaccctg acctacggcg tgcagtgctt cagccgctac 960

cccgaccaca tgaagcagca cgacttcttc aagtccgcca tgcccgaagg ctacgtccag 1020

gagcgcacca tcttcttcaa ggacgacggc aactacaaga cccgcgccga ggtgaagttc 1080

gagggcgaca ccctggtgaa ccgcatcgag ctgaagggca tcgacttcaa ggaggacggc 1140

aacatcctgg ggcacaagct ggagtacaac tacaacagcc acaacgtcta tatcatggcc 1200

gacaagcaga agaacggcat caaggtgaac ttcaagatcc gccacaacat cgaggacggc 1260

agcgtgcagc tcgccgacca ctaccagcag aacaccccca tcggcgacgg ccccgtgctg 1320

ctgcccgaca accactacct gagcacccag tccgccctga gcaaagaccc caacgagaag 1380

cgcgatcaca tggtcctgct ggagttcgtg accgccgccg ggatcactct cggcatggac 1440

gagctgtaca agtaa 1455

<210> 111
<211> 1437
<212> DNA
<213> Homo sapiens

<400> 111

atggtgagca agggcgagga gctgttcacc ggggtggtgc ccatcctggt cgagctggac 60

ggcgacgtaa acggccacaa gttcagcgtg tccggcgagg gcgagggcga tgccacctac 120

ggcaagctga ccctgaagtt catctgcacc accggcaagc tgcccgtgcc ctggcccacc 180

ctcgtgacca ccctgaccta cggcgtgcag tgcttcagcc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagca cccagtccgc cctgagcaaa gaccccaacg agaagcgcga tcacatggtc 660

ctgctggagt tcgtgaccgc cgccgggatc actctcggca tggacgagct gtacaagagg 720

cgctgcagtc tctgcgcttt cgacgccgcc cgggggccca ggcggctgat gcgtgtgggc 780

ctcgcgctga tcttggtggg ccacgtgaac ctgctgctgg gggccgtgct gcatggcacc 840

gtcctgcggc acgtggccaa tccccgcggc gctgtcacgc cggagtacac cgtagccaat 900

gtcatctctg tcggctcggg gctgctgagc gtttccgtgg gacttgtggc cctcctggcg 960

tccaggaacc ttcttcgccc tccactgcac tgggtcctgc tggcactagc tctggtgaac 1020

ctgctcttgt ccgttgcctg ctccctgggc ctccttcttg ctgtgtcact cactgtggcc 1080

aacggtggcc gccgccttat tgctgactgc cacccaggac tgctggatcc tctggtacca 1140

ctggatgagg ggccgggaca tactgactgc ccctttgacc ccacaagaat ctatgataca 1200

gccttggctc tctggatccc ttctttgctc atgtctgcag gggaggctgc tctatctggt 1260

tactgctgtg tggctgcact cactctacgt ggagttgggc cctgcaggaa ggacggactt 1320

caggggcagc tagaggaaat gacagagctt gaatctccta aatgtaaaag gcaggaaaat 1380

gagcagctac tggatcaaaa tcaagaaatc cgggcatcac agagaagttg ggtttag 1437

<210> 112
<211> 723
<212> DNA
<213> Homo sapiens

<400> 112

atgaggcgct gcagtctctg cgctttcgac gccgcccggg ggcccaggcg gctgatgcgt 60

gtgggcctcg cgctgatctt ggtgggccac gtgaacctgc tgctgggggc cgtgctgcat 120

ggcaccgtcc tgcggcacgt ggccaatccc cgcggcgctg tcacgccgga gtacaccgta 180

gccaatgtca tctctgtcgg ctcggggctg ctgagcgttt ccgtgggact tgtggccctc 240

ctggcgtcca ggaaccttct tcgccctcca ctgcactggg tcctgctggc actagctctg 300

gtgaacctgc tcttgtccgt tgcctgctcc ctgggcctcc ttcttgctgt gtcactcact 360

gtggccaacg gtggccgccg ccttattgct gactgccacc caggactgct ggatcctctg 420

gtaccactgg atgaggggcc gggacatact gactgcccct ttgaccccac aagaatctat 480

gatacagcct tggctctctg gatcccttct ttgctcatgt ctgcagggga ggctgctcta 540

tctggttact gctgtgtggc tgcactcact ctacgtggag ttgggccctg caggaaggac 600

ggacttcagg ggcagctaga ggaaatgaca gagcttgaat ctcctaaatg taaaaggcag 660

gaaaatgagc agctactgga tcaaaatcaa gaaatccggg catcacagag aagttgggtt 720

tag                                                                    723

<210>  113
<211>  484
<212>  PRT
<213>  Homo sapiens

<400> 113
<210>  114

<211>  478
<212>  PRT
<213>  Homo sapiens

<400> 114


<210>  115
<211>  16
<212>  PRT
<213>  Artificial

<220>
<223>  Synthetic polypeptide

<400> 115

Glu Gln Leu Leu Asp Gln Asn Gln Glu Ile Arg Ala Ser Gln Arg Ser
1               5                   10                  15


<210>  116
<211>  16
<212>  PRT
<213>  Artificial

<220>
<223>  Synthetic polypeptide

<400> 116

Leu Asp Glu Gly Pro Gly His Thr Asp Cys Pro Phe Asp Pro Thr Arg
1               5                   10                  15


<210>  117
<211>  13
<212>  PRT
<213>  Artificial

<220>
<223>  Synthetic polypeptide

<400> 117

Glu Lys Phe Arg Ala Val Leu Asp Leu His Val Lys His
1               5                   10


<210>  118
<211>  15
<212>  PRT
<213>  Artificial

<220>
<223>  Synthetic polypeptide

<400> 118

Cys Asn Gln Ala Lys Ala Ser Asp Val Gln Asp Leu Leu Lys Asp
1               5                   10                  15

<210>  119
<211>  1002
<212>  DNA
<213>  Homo sapiens

<400> 119

atgcatcagt ccctgactca gcagcggtcc agcgacatgt ccctgcccga ttccatggga      60

gcattcaatc ggaggaaacg aaactccatc tatgtcaccg tgactttgct tattgtgtcc     120

gtgttaattc tcacagtggg ccttgctgca accaccagga cccagaatgt gactgtagga     180

ggttattacc ccggagttat tctcggcttt ggatcgttcc ttggaatcat tggatcaaac     240

cttattgaga acaaaaggca gatgctggtg gcttctatcg tgtttatcag ctttggtgtg     300

attgcggctt tttgttgtgc catagttgac ggggtctttg ctgccagaca cattgatctg     360

aaaccactct acgctaaccg gtgccattat gttcccaaga catcacagaa ggaagctgag     420

gaggttaact gccctcacct cagccgtgaa ttctgcacac ctcgcatccg gggcaacacc     480

tgcttctgct gtgacctcta caactgtggc aaccgggtgg agatcactgg tgggtactac     540

gaatacatcg atgtcagcag ttgccaagat atcatccacc tctaccacct gctctggtct     600

gccaccatcc tcaacattgt tggcctgttc ctgggcatca tcactgccgc tgtccttgga     660

ggctttaagg acatgaaccc aactctccca gcactgaact gttctgttga aaatacccat     720

ccaacagttt cttactatgc tcatccccaa gtggcatcct acaataccta ctaccatagc     780

cctcctcacc tgccaccata ttctgcttat gactttcagc attccggtgt ctttccatcc     840

tcccctccct ctggactttc tgatgagccc cagtctgcct ctccctcacc cagctacatg     900

tggtcctcaa gtgcaccgcc ccgttactct ccaccctact atccaccttt tgaaaagcca     960

ccaccttaca gtcccgacta caaagacgat gacgacaagt aa                       1002

<210>  120
<211>  333
<212>  PRT
<213>  Homo sapiens

<400> 120

Met His Gln Ser Leu Thr Gln Gln Arg Ser Ser Asp Met Ser Leu Pro
1               5                   10                  15

Asp Ser Met Gly Ala Phe Asn Arg Arg Lys Arg Asn Ser Ile Tyr Val
            20                  25                  30

Thr Val Thr Leu Leu Ile Val Ser Val Leu Ile Leu Thr Val Gly Leu
        35                  40                  45

Ala Ala Thr Thr Arg Thr Gln Asn Val Thr Val Gly Gly Tyr Tyr Pro

50                  55                  60

Gly Val Ile Leu Gly Phe Gly Ser Phe Leu Gly Ile Ile Gly Ser Asn
65                  70                  75                  80

Leu Ile Glu Asn Lys Arg Gln Met Leu Val Ala Ser Ile Val Phe Ile
                85                  90                  95

Ser Phe Gly Val Ile Ala Ala Phe Cys Cys Ala Ile Val Asp Gly Val
            100                 105                 110

Phe Ala Ala Arg His Ile Asp Leu Lys Pro Leu Tyr Ala Asn Arg Cys
        115                 120                 125

His Tyr Val Pro Lys Thr Ser Gln Lys Glu Ala Glu Glu Val Asn Cys
    130                 135                 140

Pro His Leu Ser Arg Glu Phe Cys Thr Pro Arg Ile Arg Gly Asn Thr
145                 150                 155                 160

Cys Phe Cys Cys Asp Leu Tyr Asn Cys Gly Asn Arg Val Glu Ile Thr
                165                 170                 175

Gly Gly Tyr Tyr Glu Tyr Ile Asp Val Ser Ser Cys Gln Asp Ile Ile
            180                 185                 190

His Leu Tyr His Leu Leu Trp Ser Ala Thr Ile Leu Asn Ile Val Gly
        195                 200                 205

Leu Phe Leu Gly Ile Ile Thr Ala Ala Val Leu Gly Gly Phe Lys Asp
    210                 215                 220

Met Asn Pro Thr Leu Pro Ala Leu Asn Cys Ser Val Glu Asn Thr His
225                 230                 235                 240

Pro Thr Val Ser Tyr Tyr Ala His Pro Gln Val Ala Ser Tyr Asn Thr
                245                 250                 255

Tyr Tyr His Ser Pro Pro His Leu Pro Pro Tyr Ser Ala Tyr Asp Phe
            260                 265                 270

Gln His Ser Gly Val Phe Pro Ser Ser Pro Pro Ser Gly Leu Ser Asp
        275                 280                 285

Glu Pro Gln Ser Ala Ser Pro Ser Pro Ser Tyr Met Trp Ser Ser Ser
    290                 295                 300

Ala Pro Pro Arg Tyr Ser Pro Pro Tyr Tyr Pro Pro Phe Glu Lys Pro
305                 310                 315                 320

Pro Pro Tyr Ser Pro Asp Tyr Lys Asp Asp Asp Asp Lys
                325                 330


<210>  121
<211>  15
<212>  PRT
<213>  Artificial

<220>
<223>  Synthetic polypeptide

<400> 121

Cys His Tyr Val Pro Lys Thr Ser Gln Lys Glu Ala Glu Glu Val
1               5                   10                  15

<210>  122
<211>  23
<212>  DNA
<213>  Artificial

<220>
<223>  Synthetic polynucleotide

<400> 122

ctgaaggatc tgaaggctca gtc                                              23

<210>  123
<211>  20
<212>  DNA
<213>  Artificial

<220>
<223>  Synthetic polynucleotide

<400> 123

ggatctgctg ctcctgttcc                                                  20

<210>  124
<211>  163
<212>  DNA
<213>  Artificial

<220>
<223>  Synthetic polynucleotide

<400> 124

ctgaaggatc tgaaggctca gtcgcaggtg ttgggctgga tcttgatagc agttgttatc      60

atcattcttc tgatttttac atctgtcacc cgatgcctat ctccagttag ttttctgcag     120

ctgaaattct ggaaaatcta tttggaacag gagcagcaga tcc                       163

<210>  125
<211>  669
<212>  DNA
<213>  Homo sapiens

<400> 125
   gaaggagatc aggcagagga gagaagagtc agtgtgaact aggctggtgg ctctgtccgg      60
   tagggagacg gaaagggtgc cgcccatcat agaagtacca gaacttgagc tggactttgc     120
   tgatttagct tatggaagag gaaccagaaa tttgtccttg aataatgttt cccgtgttgg     180
   gctggatctt gatagcagtt gttatcatca ttcttctgat ttttacatct gtcacccgat     240
   gcctatctcc agttagtttt ctgcagctga aattctggaa aatctatttg gaacaggagc 300
   agcagatcct taaaagtaaa gccacagagc atgcaactga attggcaaaa gagaatatta 360
   aatgtttctt tgagggctcg catccaaaag aatataacac tccaagcatg aaagagtggc 420
   agcaaatttc atcactgtat actttcaatc cgaagggcca gtactacagc atgttgcaca 480
   aatatgtcaa cagaaaagag aagactcaca gtatcaggtc tactgaagga gatacggtga 540
   ttcctgttct tggctttgta gattcatctg gtataaacag cactcctgag ttatgacctt 600
   ttgaatgagt agaaaaaaaa attgttttga attattgctt tattaaaaaa taaacattgg 660
   tattttttg 669

<210> 126
<211> 101
<212> DNA
<213> Artificial

<220>
<223> Synthetic polynucleotide

<400> 126

aatcattatg ctgaggattt ggaaagggtg tttattcctc atggactaat tatggacagg 60

actgaacgtc ttgctcgaga tgtgatgaag gagatgggag g 101

<210> 127
<211> 117
<212> PRT
<213> Homo sapiens

<400> 127

Leu Ser Pro Val Ser Phe Leu Gln Leu Lys Phe Trp Lys Ile Tyr Leu
1               5                   10                  15

Glu Gln Glu Gln Gln Ile Leu Lys Ser Lys Ala Thr Glu His Ala Thr
            20                  25                  30

Glu Leu Ala Lys Glu Asn Ile Lys Cys Phe Phe Glu Gly Ser His Pro
        35                  40                  45

Lys Glu Tyr Asn Thr Pro Ser Met Lys Glu Trp Gln Gln Ile Ser Ser
    50                  55                  60

Leu Tyr Thr Phe Asn Pro Lys Gly Gln Tyr Tyr Ser Met Leu His Lys
65                  70                  75                  80

Tyr Val Asn Arg Lys Glu Lys Thr His Ser Ile Arg Ser Thr Glu Gly
                85                  90                  95

Asp Thr Val Ile Pro Val Leu Gly Phe Val Asp Ser Ser Gly Ile Asn
            100                 105                 110

Ser Thr Pro Glu Leu
        115

<210>  128
<211>  24
<212>  DNA
<213>  Artificial

<220>
<223>  Synthetic polynucleotide

<400> 128

aacatcatgg atcagaacaa cagc                                              24


<210>  129
<211>  26
<212>  DNA
<213>  Artificial

<220>
<223>  Synthetic polynucleotide

<400> 129

atcattggac taaagatagg gattcc                                            26


<210>  130
<211>  101
<212>  DNA
<213>  Artificial

<220>
<223>  Synthetic polynucleotide

<400> 130

aacatcatgg atcagaacaa cagcctgcca ccttacgctc agggcttggc ctcccctcag      60

ggtgccatga ctcccggaat ccctatcttt agtccaatga t                          101


<210>  131
<211>  888
<212>  DNA
<213>  Homo sapiens

<400> 131

gtccgctccg gctcctgccg cgttccagca gccgtcactc ccgccgccgg cccccgccgc      60

ccgccccgcc tgcccctacc cctccgcgcg ggccgggcac ctggccgccg ctcggtcctc     120

ggggccccgc gctgctgtct ctgtctcggc ttctctcggc ccctgggtct cttcgtctct     180

gccgttctca ggctcagctc cgtctcgctc ggtccctcgc ttccccgccg ggctctagcc     240

ggccgtctgg tggcccgagg tggcggcggg ctgggcgcgg ggcgcgatga gcggcgcctg     300

cacgagctac gtgagcgcag agcaggaggt ggtgcgcggc ttcagctgcc cgcggccggg     360

gggcgaggcg gccgctgtct tctgctgcgg cttccgcgac cacaagtact gctgcgacga     420

cccgcacagc ttcttcccct acgagcacag ctacatgtgg tggctcagca ttggcgctct 480

cataggcctg tccgtagcag cagtggttct tctcgccttc attgttaccg cctgtgtgct 540

ctgctacctg ttcatcagct ctaagcccca cacaaagttg gacctgggct tgagcttaca 600

gacagcaggc cctgaggagg tttctcctga ctgccaaggt gtgaacacag gcatggcggc 660

agaagtgcca aaagtgagcc ctctccagca gagttactcc tgcttgaacc cgcagctgga 720

gagcaatgag gggcaggctg tgaactccaa acgcctcctc catcattgct tcatggccac 780

agtgaccacc agtgacattc caggcagccc tgaggaagcc tctgtaccca accctgacct 840

atgtggacca gtcccataaa cattcaataa atgtctccat accatccc 888

<210> 132
<211> 72
<212> PRT
<213> Homo sapiens

<400> 132

Met Ser Gly Ala Cys Thr Ser Tyr Val Ser Ala Glu Gln Glu Val Val
1               5                   10                  15

Arg Gly Phe Ser Cys Pro Arg Pro Gly Gly Glu Ala Ala Ala Val Phe
            20                  25                  30

Cys Cys Gly Phe Arg Asp His Lys Tyr Cys Cys Asp Asp Pro His Ser
        35                  40                  45

Phe Phe Pro Tyr Glu His Ser Tyr Met Trp Trp Leu Arg Asp Gly Val
    50                  55                  60

Ser Pro Cys Cys Pro Gly Trp Ser
65                  70

<210> 133
<211> 153
<212> PRT
<213> Homo sapiens

<400> 133

Met Ser Gly Ala Cys Thr Ser Tyr Val Ser Ala Glu Gln Glu Val Val
1               5                   10                  15

Arg Gly Phe Ser Cys Pro Arg Pro Gly Gly Glu Ala Ala Ala Val Phe
            20                  25                  30

Cys Cys Gly Phe Arg Asp His Lys Tyr Cys Cys Asp Asp Pro His Ser
        35                  40                  45

Phe Phe Pro Tyr Glu His Ser Tyr Met Trp Trp Leu Ser Ile Gly Ala
    50                  55                  60

Leu Ile Gly Leu Ser Val Ala Ala Val Val Leu Leu Ala Phe Ile Val
65                  70                  75                  80

Thr Ala Cys Val Leu Cys Tyr Leu Phe Ile Ser Ser Lys Pro His Thr

85                  90                  95

Lys Leu Asp Leu Gly Leu Ser Leu Gln Thr Ala Gly Pro Val Gln Val
            100                 105                 110

Ala Thr Asn Lys Arg Asn Gln Thr Val Leu Thr Leu Lys Gly Leu Thr
        115                 120                 125

Trp Arg Thr Arg Gln Ala His Lys Trp Pro Gly Gly Pro Val Val Ala
    130                 135                 140

Leu His Gly Ala Leu Gly Arg Lys Lys
145                 150


<210>  134
<211>  134
<212>  PRT
<213>  Homo sapiens

<400> 134

Met Trp Trp Leu Ser Ile Gly Ala Leu Ile Gly Leu Ser Val Ala Ala
1               5                   10                  15

Val Val Leu Leu Ala Phe Ile Val Thr Ala Cys Val Leu Cys Tyr Leu
            20                  25                  30

Phe Ile Ser Ser Lys Pro His Thr Lys Leu Asp Leu Gly Leu Ser Leu
        35                  40                  45

Gln Thr Ala Gly Pro Glu Glu Val Ser Pro Asp Cys Gln Gly Val Asn
    50                  55                  60

Thr Gly Met Ala Ala Glu Val Pro Lys Val Ser Pro Leu Gln Gln Ser
65                  70                  75                  80

Tyr Ser Cys Leu Asn Pro Gln Leu Glu Ser Asn Glu Gly Gln Ala Val
                85                  90                  95

Asn Ser Lys Arg Leu Leu His His Cys Phe Met Ala Thr Val Thr Thr
            100                 105                 110

Ser Asp Ile Pro Gly Ser Pro Glu Glu Ala Ser Val Pro Asn Pro Asp
        115                 120                 125

Leu Cys Gly Pro Val Pro
    130


<210>  135
<211>  190
<212>  PRT
<213>  Homo sapiens

<400> 135

Met Ser Gly Ala Cys Thr Ser Tyr Val Ser Ala Glu Gln Glu Val Val
1               5                   10                  15

Arg Gly Phe Ser Cys Pro Arg Pro Gly Gly Glu Ala Ala Ala Val Phe
            20                  25                  30

Cys Cys Gly Phe Arg Asp His Lys Tyr Cys Cys Asp Asp Pro His Ser
        35                  40                  45

Phe Phe Pro Tyr Glu His Ser Tyr Met Trp Trp Leu Ser Ile Gly Ala
    50                  55                  60

Leu Ile Gly Leu Ser Val Ala Ala Val Val Leu Leu Ala Phe Ile Val
65                  70                  75                  80

Thr Ala Cys Val Leu Cys Tyr Leu Phe Ile Ser Ser Lys Pro His Thr
                85                  90                  95

Lys Leu Asp Leu Gly Leu Ser Leu Gln Thr Ala Gly Pro Glu Glu Val
            100                 105                 110

Ser Pro Asp Cys Gln Gly Val Asn Thr Gly Met Ala Ala Glu Val Pro
        115                 120                 125

Lys Val Ser Pro Leu Gln Gln Ser Tyr Ser Cys Leu Asn Pro Gln Leu
    130                 135                 140

Glu Ser Asn Glu Gly Gln Ala Val Asn Ser Lys Arg Leu Leu His His
145                 150                 155                 160

Cys Phe Met Ala Thr Val Thr Thr Ser Asp Ile Pro Gly Ser Pro Glu
                165                 170                 175

Glu Ala Ser Val Pro Asn Pro Asp Leu Cys Gly Pro Val Pro
            180                 185                 190

<210> 136
<211> 2405
<212> DNA
<213> Homo sapiens

<400> 136

tccggcgtgg tgcgcaggcg cggtatcccc cctcccccgc cagctcgacc ccggtgtggt 60

gcgcaggcgc agtctgcgca gggactggcg ggactgcgcg gcggcaacag cagacatgtc 120

gggggtccgg ggcctgtcgc ggctgctgag cgctcggcgc ctggcgctgg ccaaggcgtg 180

gccaacagtg ttgcaaacag gaacccgagg ttttcacttc actgttgatg ggaacaagag 240

ggcatctgct aaagtttcag attccatttc tgctcagtat ccagtagtgg atcatgaatt 300

tgatgcagtg gtggtaggcg ctggaggggc aggcttgcga gctgcatttg gcctttctga 360

ggcagggttt aatacagcat gtgttaccaa gctgtttcct accaggtcac acactgttgc 420

agcacaggga ggaatcaatg ctgctctggg gaacatggag gaggacaact ggaggtggca 480

tttctacgac accgtgaagg gctccgactg gctgggggac caggatgcca tccactacat 540

gacggagcag gcccccgccg ccgtggtcga gctagaaaat tatggcatgc cgtttagcag 600

aactgaagat gggaagattt atcagcgtgc atttggtgga cagagcctca agtttggaaa 660

gggcgggcag gcccatcggt gctgctgtgt ggctgatcgg actggccact cgctattgca 720

caccttatat ggaaggtctc tgcgatatga taccagctat tttgtggagt attttgcctt 780

ggatctcctg atggagaatg gggagtgccg tggtgtcatc gcactgtgca tagaggacgg 840

gtccatccat cgcataagag caaagaacac tgttgttgcc acaggaggct acgggcgcac 900

ctacttcagc tgcacgtctg cccacaccag cactggcgac ggcacggcca tgatcaccag 960

ggcaggcctt ccttgccagg acctagagtt tgttcagttc caccctacag gcatatatgg 1020

tgctggttgt ctcattacgg aaggatgtcg tggagaggga ggcattctca ttaacagtca 1080

aggcgaaagg tttatggagc gatacgcccc tgtcgcgaag gacctggcgt ctagagatgt 1140

ggtgtctcgg tccatgactc tggagatccg agaaggaaga ggctgtggcc ctgagaaaga 1200

tcacgtctac ctgcagctgc accacctacc tccagagcag ctggccacgc gcctgcctgg 1260

catttcagag acagccatga tcttcgctgg cgtggacgtc acgaaggagc cgatccctgt 1320

cctccccacc gtgcattata acatgggcgg cattcccacc aactacaagg ggcaggtcct 1380

gaggcacgtg aatggccagg atcagattgt gcccggcctg tacgcctgtg gggaggccgc 1440

ctgtgcctcg gtacatggtg ccaaccgcct cggggcaaac tcgctcttgg acctggttgt 1500

ctttggtcgg gcatgtgccc tgagcatcga agagtcatgc aggcctggag ataaagtccc 1560

tccaattaaa ccaaacgctg gggaagaatc tgtcatgaat cttgacaaat tgagatttgc 1620

tgatggaagc ataagaacat cggaactgcg actcagcatg cagaagtcaa tgcaaaatca 1680

tgctgccgtg ttccgtgtgg gaagcgtgtt gcaagaaggt tgtgggaaaa tcagcaagct 1740

ctatggagac ctaaagcacc tgaagacgtt cgaccgggga atggtctgga acacggacct 1800

ggtggagacc ctggagctgc agaacctgat gctgtgtgcg ctgcagacca tctacggagc 1860

agaggcacgg aaggagtcac ggggcgcgca tgccagggaa gactacaagg tgcggattga 1920

tgagtacgat tactccaagc ccatccaggg gcaacagaag aagccctttg aggagcactg 1980

gaggaagcac accctgtcct atgtggacgt tggcactggg aaggtcactc tggaatatag 2040

acccgtgatc gacaaaactt tgaacgaggc tgactgtgcc accgtcccgc cagccattcg 2100

ctcctactga tgagacaaga tgtggtgatg acagaatcag cttttgtaat tatgtataat 2160

agctcatgca tgtgtccatg tcataactgt cttcatacgc ttctgcactc tggggaagaa 2220

ggagtacatt gaagggagat tggcacctag tggctgggag cttgccagga acccagtggc 2280

cagggagcgt ggcacttacc tttgtccctt gcttcattct tgtgagatga taaaactggg 2340

cacagctctt aaataaaata taaatgaaca aactttcttt tatttccaaa aaaaaaaaaa 2400

aaaaa 2405

<210> 137
<211> 1435
<212> DNA
<213> Homo sapiens

<400> 137

ggcggggcct gcttctcctc agcttcaggc ggctgcgacg agccctcagg cgaacctctc 60

ggctttcccg cgcggcgccg cctcttgctg cgcctccgcc tcctcctctg ctccgccacc 120

ggcttcctcc tcctgagcag tcagcccgcg cgccggccgg ctccgttatg gcgacccgca 180

gccctggcgt cgtgattagt gatgatgaac caggttatga ccttgattta ttttgcatac 240

ctaatcatta tgctgaggat ttggaaaggg tgtttattcc tcatggacta attatggaca 300

ggactgaacg tcttgctcga gatgtgatga aggagatggg aggccatcac attgtagccc 360

tctgtgtgct caaggggggc tataaattct ttgctgacct gctggattac atcaaagcac 420

tgaatagaaa tagtgataga tccattccta tgactgtaga ttttatcaga ctgaagagct 480

attgtaatga ccagtcaaca ggggacataa aagtaattgg tggagatgat ctctcaactt 540

taactggaaa gaatgtcttg attgtggaag atataattga cactggcaaa acaatgcaga 600

ctttgctttc cttggtcagg cagtataatc caaagatggt caaggtcgca agcttgctgg 660

tgaaaaggac cccacgaagt gttggatata agccagactt tgttggattt gaaattccag 720

acaagtttgt tgtaggatat gcccttgact ataatgaata cttcagggat ttgaatcatg 780

tttgtgtcat tagtgaaact ggaaaagcaa aatacaaagc ctaagatgag agttcaagtt 840

gagtttggaa acatctggag tcctattgac atcgccagta aaattatcaa tgttctagtt 900

ctgtggccat ctgcttagta gagctttttg catgtatctt ctaagaattt tatctgtttt 960

gtactttaga aatgtcagtt gctgcattcc taaactgttt atttgcacta tgagcctata 1020

gactatcagt tccctttggg cggattgttg tttaacttgt aaatgaaaaa attctcttaa 1080

accacagcac tattgagtga aacattgaac tcatatctgt aagaaataaa gagaagatat 1140

attagttttt taattggtat tttaattttt atatatgcag gaaagaatag aagtgattga 1200

atattgttaa ttataccacc gtgtgttaga aaagtaagaa gcagtcaatt ttcacatcaa 1260

agacagcatc taagaagttt tgttctgtcc tggaattatt ttagtagtgt ttcagtaatg 1320

ttgactgtat tttccaactt gttcaaatta ttaccagtga atctttgtca gcagttccct 1380

tttaaatgca aatcaataaa ttcccaaaaa tttaaaaaaa aaaaaaaaaa aaaaa 1435

<210> 138
<211> 2395
<212> DNA
<213> Homo sapiens

<400> 138

agaggcaggg gctggcctgg gatgcgcgcg cacctgccct cgccccgccc cgcccgcacg 60

aggggtggtg gccgaggccc cgccccgcac gcctcgcctg aggcgggtcc gctcagccca 120

ggcgcccgcc cccgcccccg ccgattaaat gggccggcgg ggctcagccc ccggaaacgg 180

tcgtacactt cggggctgcg agcgcggagg gcgacgacga cgaagcgcag acagcgtcat 240

ggcagagcag gtggccctga gccggaccca ggtgtgcggg atcctgcggg aagagctttt 300

ccagggcgat gccttccatc agtcggatac acacatattc atcatcatgg gtgcatcggg 360

tgacctggcc aagaagaaga tctaccccac catctggtgg ctgttccggg atggccttct 420

gcccgaaaac accttcatcg tgggctatgc ccgttcccgc ctcacagtgg ctgacatccg 480

caaacagagt gagcccttct tcaaggccac cccagaggag aagctcaagc tggaggactt 540

ctttgcccgc aactcctatg tggctggcca gtacgatgat gcagcctcct accagcgcct 600

caacagccac atgaatgccc tccacctggg gtcacaggcc aaccgcctct tctacctggc 660

cttgcccccg accgtctacg aggccgtcac caagaacatt cacgagtcct gcatgagcca 720

gataggctgg aaccgcatca tcgtggagaa gcccttcggg agggacctgc agagctctga 780

ccggctgtcc aaccacatct cctccctgtt ccgtgaggac cagatctacc gcatcgacca 840

ctacctgggc aaggagatgg tgcagaacct catggtgctg agatttgcca acaggatctt 900

cggccccatc tggaaccggg acaacatcgc ctgcgttatc ctcaccttca aggagccctt 960

tggcactgag ggtcgcgggg gctatttcga tgaatttggg atcatccggg acgtgatgca 1020

gaaccaccta ctgcagatgc tgtgtctggt ggccatggag aagcccgcct ccaccaactc 1080

agatgacgtc cgtgatgaga aggtcaaggt gttgaaatgc atctcagagg tgcaggccaa 1140

caatgtggtc ctgggccagt acgtggggaa ccccgatgga gagggcgagg ccaccaaagg 1200

gtacctggac gaccccacgg tgccccgcgg gtccaccacc gccacttttg cagccgtcgt 1260

cctctatgtg gagaatgaga ggtgggatgg ggtgcccttc atcctgcgct gcggcaaggc 1320

cctgaacgag cgcaaggccg aggtgaggct gcagttccat gatgtggccg gcgacatctt 1380

ccaccagcag tgcaagcgca acgagctggt gatccgcgtg cagcccaacg aggccgtgta 1440

caccaagatg atgaccaaga agccgggcat gttcttcaac cccgaggagt cggagctgga 1500

cctgacctac ggcaacagat acaagaacgt gaagctccct gacgcctacg agcgcctcat 1560

cctggacgtc ttctgcggga gccagatgca cttcgtgcgc agcgacgagc tccgtgaggc 1620

ctggcgtatt ttcaccccac tgctgcacca gattgagctg gagaagccca agcccatccc 1680

ctatatttat ggcagccgag gccccacgga ggcagacgag ctgatgaaga gagtgggttt 1740

ccagtatgag ggcacctaca agtgggtgaa cccccacaag ctctgagccc tgggcaccca 1800

cctccacccc cgccacggcc accctccttc ccgccgcccg accccgagtc gggaggactc 1860

cgggaccatt gacctcagct gcacattcct ggccccgggc tctggccacc ctggcccgcc 1920

cctcgctgct gctactaccc gagcccagct acattcctca gctgccaagc actcgagacc 1980

atcctggccc ctccagaccc tgcctgagcc caggagctga gtcacctcct ccactcactc 2040

cagcccaaca gaaggaagga ggagggcgcc cattcgtctg tcccagagct tattggccac 2100

tgggtctcac tcctgagtgg ggccagggtg ggagggaggg acaaggggga ggaaaggggc 2160

gagcacccac gtgagagaat ctgcctgtgg ccttgcccgc cagcctcagt gccacttgac 2220

attccttgtc accagcaaca tctcgagccc cctggatgtc ccctgtccca ccaactctgc 2280

actccatggc caccccgtgc cacccgtagg cagcctctct gctataagaa aagcagacgc 2340

agcagctggg acccctccca acctcaatgc cctgccatta aatccgcaaa cagcc 2395

<210> 139
<211> 2201
<212> DNA
<213> Homo sapiens

<400> 139

cgggatttgg gtcgcggttc ttgtttgtgg atcgctgtga tcgtcacttg acaatgcaga 60

tcttcgtgaa gactctgact ggtaagacca tcaccctcga ggttgagccc agtgacacca 120

tcgagaatgt caaggcaaag atccaagata aggaaggcat ccctcctgac cagcagaggc 180

tgatctttgc tggaaaacag ctggaagatg ggcgcaccct gtctgactac aacatccaga 240

aagagtccac cctgcacctg gtgctccgtc tcagaggtgg gatgcaaatc ttcgtgaaga 300

cactcactgg caagaccatc acccttgagg tggagcccag tgacaccatc gagaacgtca 360

aagcaaagat ccaggacaag gaaggcattc ctcctgacca gcagaggttg atctttgccg 420

gaaagcagct ggaagatggg cgcaccctgt ctgactacaa catccagaaa gagtctaccc 480

tgcacctggt gctccgtctc agaggtggga tgcagatctt cgtgaagacc ctgactggta 540

agaccatcac cctcgaggtg gagcccagtg acaccatcga gaatgtcaag gcaaagatcc 600

aagataagga aggcattcct cctgatcagc agaggttgat ctttgccgga aaacagctgg 660

aagatggtcg taccctgtct gactacaaca tccagaaaga gtccaccttg cacctggtac 720

tccgtctcag aggtgggatg caaatcttcg tgaagacact cactggcaag accatcaccc 780

ttgaggtcga gcccagtgac actatcgaga acgtcaaagc aaagatccaa gacaaggaag 840

gcattcctcc tgaccagcag aggttgatct ttgccggaaa gcagctggaa gatgggcgca 900

ccctgtctga ctacaacatc cagaaagagt ctaccctgca cctggtgctc cgtctcagag 960

gtgggatgca gatcttcgtg aagaccctga ctggtaagac catcaccctc gaagtggagc 1020

cgagtgacac cattgagaat gtcaaggcaa agatccaaga caaggaaggc atccctcctg 1080

accagcagag gttgatcttt gccggaaaac agctggaaga tggtcgtacc ctgtctgact 1140

acaacatcca gaaagagtcc accttgcacc tggtgctccg tctcagaggt gggatgcaga 1200

tcttcgtgaa gaccctgact ggtaagacca tcactctcga ggtggagccg agtgacacca 1260

ttgagaatgt caaggcaaag atccaagaca aggaaggcat ccctcctgat cagcagaggt 1320

tgatctttgc tgggaaacag ctggaagatg gacgcaccct gtctgactac aacatccaga 1380

aagagtccac cctgcacctg gtgctccgtc ttagaggtgg gatgcagatc ttcgtgaaga 1440

ccctgactgg taagaccatc actctcgaag tggagccgag tgacaccatt gagaatgtca 1500

aggcaaagat ccaagacaag gaaggcatcc ctcctgacca gcagaggttg atctttgctg 1560

ggaaacagct ggaagatgga cgcaccctgt ctgactacaa catccagaaa gagtccaccc 1620

tgcacctggt gctccgtctt agaggtggga tgcagatctt cgtgaagacc ctgactggta 1680

agaccatcac tctcgaagtg gagccgagtg acaccattga gaatgtcaag gcaaagatcc 1740

aagacaagga aggcatccct cctgaccagc agaggttgat ctttgctggg aaacagctgg 1800

aagatggacg caccctgtct gactacaaca tccagaaaga gtccaccctg cacctggtgc 1860

tccgtctcag aggtgggatg cagatcttcg tgaagaccct gactggtaag accatcaccc 1920

tcgaggtgga gcccagtgac accatcgaga atgtcaaggc aaagatccaa gataaggaag 1980

gcatccctcc tgatcagcag aggttgatct ttgctgggaa acagctggaa gatggacgca 2040

ccctgtctga ctacaacatc cagaaagagt ccactctgca cttggtcctg cgcttgaggg 2100

ggggtgtcta agtttcccct tttaaggttt caacaaattt cattgcactt tcctttcaat 2160

aaagttgttg cattcccaaa aaaaaaaaaa aaaaaaaaaa a 2201

<210> 140
<211> 1867
<212> DNA
<213> Homo sapiens

<400> 140

ggttcgctgt ggcgggcgcc tgggccgccg gctgtttaac ttcgcttccg ctggcccata 60

gtgatctttg cagtgaccca gcagcatcac tgtttcttgg cgtgtgaaga taacccaagg 120

aattgaggaa gttgctgaga agagtgtgct ggagatgctc taggaaaaaa ttgaatagtg 180

agacgagttc cagcgcaagg gtttctggtt tgccaagaag aaagtgaaca tcatggatca 240

gaacaacagc ctgccacctt acgctcaggg cttggcctcc cctcagggtg ccatgactcc 300

cggaatccct atctttagtc caatgatgcc ttatggcact ggactgaccc cacagcctat 360

tcagaacacc aatagtctgt ctattttgga agagcaacaa aggcagcagc agcaacaaca 420

acagcagcag cagcagcagc agcagcaaca gcaacagcag cagcagcagc agcagcagca 480

gcagcagcag cagcagcagc agcagcagca gcaacaggca gtggcagctg cagccgttca 540

gcagtcaacg tcccagcagg caacacaggg aacctcaggc caggcaccac agctcttcca 600

ctcacagact ctcacaactg cacccttgcc gggcaccact ccactgtatc cctcccccat 660

gactcccatg acccccatca ctcctgccac gccagcttcg gagagttctg ggattgtacc 720

gcagctgcaa aatattgtat ccacagtgaa tcttggttgt aaacttgacc taaagaccat 780

tgcacttcgt gcccgaaacg ccgaatataa tcccaagcgg tttgctgcgg taatcatgag 840

gataagagag ccacgaacca cggcactgat tttcagttct gggaaaatgg tgtgcacagg 900

agccaagagt gaagaacagt ccagactggc agcaagaaaa tatgctagag ttgtacagaa 960

gttgggtttt ccagctaagt tcttggactt caagattcag aatatggtgg ggagctgtga 1020

tgtgaagttt cctataaggt tagaaggcct tgtgctcacc caccaacaat ttagtagtta 1080

tgagccagag ttatttcctg gtttaatcta cagaatgatc aaacccagaa ttgttctcct 1140

tatttttgtt tctggaaaag ttgtattaac aggtgctaaa gtcagagcag aaatttatga 1200

agcatttgaa aacatctacc ctattctaaa gggattcagg aagacgacgt aatggctctc 1260

atgtaccctt gcctccccca cccccttctt tttttttttt taaacaaatc agtttgtttt 1320

ggtaccttta aatggtggtg ttgtgagaag atggatgttg agttgcaggg tgtggcacca 1380

ggtgatgccc ttctgtaagt gcccaccgcg ggatgccggg aaggggcatt atttgtgcac 1440

tgagaacacc gcgcagcgtg actgtgagtt gctcataccg tgctgctatc tgggcagcgc 1500

tgcccattta tttatatgta gattttaaac actgctgttg acaagttggt ttgagggaga 1560

aaactttaag tgttaaagcc acctctataa ttgattggac tttttaattt taatgttttt 1620

ccccatgaac cacagttttt atatttctac cagaaaagta aaaatctttt ttaaaagtgt 1680

tgtttttcta atttataact cctaggggtt atttctgtgc cagacacatt ccacctctcc 1740

agtattgcag gacagaatat atgtgttaat gaaaatgaat ggctgtacat atttttttct 1800

ttcttcagag tactctgtac aataaatgca gtttataaaa gtgttaaaaa aaaaaaaaaa 1860

aaaaaaa 1867

<210> 141
<211> 684
<212> PRT
<213> Homo sapiens

<400> 141

Met Ala Arg His Arg Asn Val Arg Gly Tyr Asn Tyr Asp Glu Asp Phe
1               5                   10                  15

Glu Asp Asp Asp Leu Tyr Gly Gln Ser Val Glu Asp Asp Tyr Cys Ile
            20                  25                  30

Ser Pro Ser Thr Ala Ala Gln Phe Ile Tyr Ser Arg Arg Asp Lys Pro
        35                  40                  45

Ser Val Glu Pro Val Glu Glu Tyr Asp Tyr Glu Asp Leu Lys Glu Ser
    50                  55                  60

Ser Asn Ser Val Ser Asn His Gln Leu Ser Gly Phe Asp Gln Ala Arg
65                  70                  75                  80

Leu Tyr Ser Cys Leu Asp His Met Arg Glu Val Leu Gly Asp Ala Val
                85                  90                  95
Pro Asp Glu Ile Leu Ile Glu Ala Val Leu Lys Asn Lys Phe Asp Val
            100                 105                 110
Gln Lys Ala Leu Ser Gly Val Leu Glu Gln Asp Arg Val Gln Ser Leu
        115                 120                 125
Lys Asp Lys Asn Glu Ala Thr Val Ser Thr Gly Lys Ile Ala Lys Gly
    130                 135                 140
Lys Pro Val Asp Ser Gln Thr Ser Arg Ser Glu Ser Glu Ile Val Pro
145                 150                 155                 160
Lys Val Ala Lys Met Thr Val Ser Gly Lys Lys Gln Thr Met Gly Phe
                165                 170                 175
Glu Val Pro Gly Val Ser Ser Glu Glu Asn Gly His Ser Phe His Thr
            180                 185                 190
Pro Gln Lys Gly Pro Pro Ile Glu Asp Ala Ile Ala Ser Ser Asp Val
        195                 200                 205
Leu Glu Thr Ala Ser Lys Ser Ala Asn Pro Pro His Thr Ile Gln Ala
    210                 215                 220
Ser Glu Glu Gln Ser Ser Thr Pro Ala Pro Val Lys Lys Ser Gly Lys
225                 230                 235                 240
Leu Arg Gln Gln Ile Asp Val Lys Ala Glu Leu Glu Lys Arg Gln Gly
                245                 250                 255
Gly Lys Gln Leu Leu Asn Leu Val Val Ile Gly His Val Asp Ala Gly
            260                 265                 270
Lys Ser Thr Leu Met Gly His Met Leu Tyr Leu Leu Gly Asn Ile Asn
        275                 280                 285
Lys Arg Thr Met His Lys Tyr Glu Gln Glu Ser Lys Lys Ala Gly Lys
    290                 295                 300
Ala Ser Phe Ala Tyr Ala Trp Val Leu Asp Glu Thr Gly Glu Glu Arg
305                 310                 315                 320
Glu Arg Gly Val Thr Met Asp Val Gly Met Thr Lys Phe Glu Thr Thr
                325                 330                 335
Thr Lys Val Ile Thr Leu Met Asp Ala Pro Gly His Lys Asp Phe Ile
            340                 345                 350
Pro Asn Met Ile Thr Gly Ala Ala Gln Ala Asp Val Ala Val Leu Val
        355                 360                 365
Val Asp Ala Ser Arg Gly Glu Phe Glu Ala Gly Phe Glu Thr Gly Gly
    370                 375                 380
Gln Thr Arg Glu His Gly Leu Leu Val Arg Ser Leu Gly Val Thr Gln
385                 390                 395                 400
Leu Ala Val Ala Val Asn Lys Met Asp Gln Val Asn Trp Gln Gln Glu
                405                 410                 415

Arg Phe Gln Glu Ile Thr Gly Lys Leu Gly His Phe Leu Lys Gln Ala
            420                 425                 430

Gly Phe Lys Glu Ser Asp Val Gly Phe Ile Pro Thr Ser Gly Leu Ser
        435                 440                 445

Gly Glu Asn Leu Ile Thr Arg Ser Gln Ser Ser Glu Leu Thr Lys Trp
    450                 455                 460

Tyr Lys Gly Leu Cys Leu Leu Glu Gln Ile Asp Ser Phe Lys Pro Pro
465                 470                 475                 480

Gln Arg Ser Ile Asp Lys Pro Phe Arg Leu Cys Val Ser Asp Val Phe
                485                 490                 495

Lys Asp Gln Gly Ser Gly Phe Cys Ile Thr Gly Lys Ile Glu Ala Gly
            500                 505                 510

Tyr Ile Gln Thr Gly Asp Arg Leu Leu Ala Met Pro Pro Asn Glu Thr
        515                 520                 525

Cys Thr Val Lys Gly Ile Thr Leu His Asp Glu Pro Val Asp Trp Ala
    530                 535                 540

Ala Ala Gly Asp His Val Ser Leu Thr Leu Val Gly Met Asp Ile Ile
545                 550                 555                 560

Lys Ile Asn Val Gly Cys Ile Phe Cys Gly Pro Lys Val Pro Ile Lys
                565                 570                 575

Ala Cys Thr Arg Phe Arg Ala Arg Ile Leu Ile Phe Asn Ile Glu Ile
            580                 585                 590

Pro Ile Thr Lys Gly Phe Pro Val Leu Leu His Tyr Gln Thr Val Ser
        595                 600                 605

Glu Pro Ala Val Ile Lys Arg Leu Ile Ser Val Leu Asn Lys Ser Thr
    610                 615                 620

Gly Glu Val Thr Lys Lys Lys Pro Lys Phe Leu Thr Lys Gly Gln Asn
625                 630                 635                 640

Ala Leu Val Glu Leu Gln Thr Gln Arg Pro Ile Ala Leu Glu Leu Tyr
                645                 650                 655

Lys Asp Phe Lys Glu Leu Gly Arg Phe Met Leu Arg Tyr Gly Gly Ser
            660                 665                 670

Thr Ile Ala Ala Gly Val Val Thr Glu Ile Lys Glu
        675                 680


<210>  142
<211>  664
<212>  PRT
<213>  Homo sapiens

<400> 142

<210>  143
<211>  494
<212>  PRT

<213>  Homo sapiens

<400>  143

Met Pro Arg Val Tyr Ile Gly Arg Leu Ser Tyr Gln Ala Arg Glu Arg
1               5                   10                  15

Asp Val Glu Arg Phe Phe Lys Gly Tyr Gly Lys Ile Leu Glu Val Asp
            20                  25                  30

Leu Lys Asn Gly Tyr Gly Phe Val Glu Phe Asp Asp Leu Arg Asp Ala
        35                  40                  45

Asp Asp Ala Val Tyr Glu Leu Asn Gly Lys Asp Leu Cys Gly Glu Arg
    50                  55                  60

Val Ile Val Glu His Ala Arg Gly Pro Arg Arg Asp Gly Ser Tyr Gly
65                  70                  75                  80

Ser Gly Arg Ser Gly Tyr Gly Tyr Arg Arg Ser Gly Arg Asp Lys Tyr
                85                  90                  95

Gly Pro Pro Thr Arg Thr Glu Tyr Arg Leu Ile Val Glu Asn Leu Ser
            100                 105                 110

Ser Arg Cys Ser Trp Gln Asp Leu Lys Asp Tyr Met Arg Gln Ala Gly
        115                 120                 125

Glu Val Thr Tyr Ala Asp Ala His Lys Gly Arg Lys Asn Glu Gly Val
    130                 135                 140

Ile Glu Phe Val Ser Tyr Ser Asp Met Lys Arg Ala Leu Glu Lys Leu
145                 150                 155                 160

Asp Gly Thr Glu Val Asn Gly Arg Lys Ile Arg Leu Val Glu Asp Lys
                165                 170                 175

Pro Gly Ser Arg Arg Arg Arg Ser Tyr Ser Arg Ser Arg Ser His Ser
            180                 185                 190

Arg Ser Arg Ser Arg Ser Arg His Ser Arg Lys Ser Arg Ser Arg Ser
        195                 200                 205

Gly Ser Ser Lys Ser Ser His Ser Lys Ser Arg Ser Arg Ser Arg Ser
    210                 215                 220

Gly Ser Arg Ser Arg Ser Lys Ser Arg Ser Arg Ser Gln Ser Arg Ser
225                 230                 235                 240

Arg Ser Lys Lys Glu Lys Ser Arg Ser Pro Ser Lys Glu Lys Ser Arg
                245                 250                 255

Ser Arg Ser His Ser Ala Gly Lys Ser Arg Ser Lys Ser Lys Asp Gln
            260                 265                 270

Ala Glu Glu Lys Ile Gln Asn Asn Asp Asn Val Gly Lys Pro Lys Ser
        275                 280                 285

Arg Ser Pro Ser Arg His Lys Ser Lys Ser Lys Ser Arg Ser Arg Ser
    290                 295                 300

Gln Glu Arg Arg Val Glu Glu Glu Lys Arg Gly Ser Val Ser Arg Gly
305                 310                 315                 320

Arg Ser Gln Glu Lys Ser Leu Arg Gln Ser Arg Ser Arg Ser Arg Ser
                325                 330                 335

Lys Gly Gly Ser Arg Ser Arg Ser Arg Ser Arg Ser Lys Ser Lys Asp
            340                 345                 350

Lys Arg Lys Gly Arg Lys Arg Ser Arg Glu Glu Ser Arg Ser Arg Ser
        355                 360                 365

Arg Ser Arg Ser Lys Ser Glu Arg Ser Arg Lys Arg Gly Ser Lys Arg
    370                 375                 380

Asp Ser Lys Ala Gly Ser Ser Lys Lys Lys Lys Lys Glu Asp Thr Asp
385                 390                 395                 400

Arg Ser Gln Ser Arg Ser Pro Ser Arg Ser Val Ser Lys Glu Arg Glu
                405                 410                 415

His Ala Lys Ser Glu Ser Ser Gln Arg Glu Gly Arg Gly Glu Ser Glu
            420                 425                 430

Asn Ala Gly Thr Asn Gln Glu Thr Arg Ser Arg Ser Arg Ser Asn Ser
        435                 440                 445

Lys Ser Lys Pro Asn Leu Pro Ser Glu Ser Arg Ser Arg Ser Lys Ser
    450                 455                 460

Ala Ser Lys Thr Arg Ser Arg Ser Lys Ser Arg Ser Arg Ser Ala Ser
465                 470                 475                 480

Arg Ser Pro Ser Arg Ser Arg Ser Arg Ser His Ser Arg Ser
                485                 490


<210>  144
<211>  376
<212>  PRT
<213>  Homo sapiens

<400> 144

Thr Phe Pro Arg Glu Trp Leu Cys Asp Arg His Leu Arg Glu Lys Met
1               5                   10                  15

Phe Ser Ser Val Ala His Leu Ala Arg Ala Asn Pro Phe Asn Thr Pro
            20                  25                  30

His Leu Gln Leu Val His Asp Gly Leu Gly Asp Leu Arg Ser Ser Ser
        35                  40                  45

Pro Gly Pro Thr Gly Gln Pro Arg Arg Pro Arg Asn Leu Ala Ala Ala
    50                  55                  60

Ala Val Glu Glu Tyr Ser Cys Glu Phe Gly Ser Ala Lys Tyr Tyr Ala
65                  70                  75                  80

Leu Cys Gly Phe Gly Gly Val Leu Ser Cys Gly Leu Thr His Thr Ala
                85                  90                  95

Val Val Pro Leu Asp Leu Val Lys Cys Arg Met Gln Val Asp Pro Gln
            100                 105                 110

Lys Tyr Lys Gly Ile Phe Asn Gly Phe Ser Val Thr Leu Lys Glu Asp
        115                 120                 125

Gly Val Arg Gly Leu Ala Lys Gly Trp Ala Pro Thr Phe Leu Gly Tyr
    130                 135                 140

Ser Met Gln Gly Leu Cys Lys Phe Gly Phe Tyr Glu Val Phe Lys Val
145                 150                 155                 160

Leu Tyr Ser Asn Met Leu Gly Glu Glu Asn Thr Tyr Leu Trp Arg Thr
                165                 170                 175

Ser Leu Tyr Leu Ala Ala Ser Ala Ser Ala Glu Phe Phe Ala Asp Ile
            180                 185                 190

Ala Leu Ala Pro Met Glu Ala Ala Lys Val Arg Ile Gln Thr Gln Pro
        195                 200                 205

Gly Tyr Ala Asn Thr Leu Arg Asp Ala Ala Pro Lys Met Tyr Lys Glu
    210                 215                 220

Glu Gly Leu Lys Ala Phe Tyr Lys Gly Val Ala Pro Leu Trp Met Arg
225                 230                 235                 240

Gln Ile Pro Tyr Thr Met Met Lys Phe Ala Cys Phe Glu Arg Thr Val
                245                 250                 255

Glu Ala Leu Tyr Lys Phe Val Val Pro Lys Pro Arg Ser Glu Cys Ser
            260                 265                 270

Lys Pro Glu Gln Leu Val Val Thr Phe Val Ala Gly Tyr Ile Ala Gly
        275                 280                 285

Val Phe Cys Ala Ile Val Ser His Pro Ala Asp Ser Val Val Ser Val
    290                 295                 300

Leu Asn Lys Glu Lys Gly Ser Ser Ala Ser Leu Val Leu Lys Arg Leu
305                 310                 315                 320

Gly Phe Lys Gly Val Trp Lys Gly Leu Phe Ala Arg Ile Ile Met Ile
                325                 330                 335

Gly Thr Leu Thr Ala Leu Gln Trp Phe Ile Tyr Asp Ser Val Lys Val
            340                 345                 350

Tyr Phe Arg Leu Pro Arg Pro Pro Pro Pro Glu Met Pro Glu Ser Leu
        355                 360                 365

Lys Lys Lys Leu Gly Leu Thr Gln
    370                 375


<210>  145
<211>  339
<212>  PRT
<213>  Homo sapiens

<400> 145

Met Asp Gln Asn Asn Ser Leu Pro Pro Tyr Ala Gln Gly Leu Ala Ser
1               5                   10                  15

Pro Gln Gly Ala Met Thr Pro Gly Ile Pro Ile Phe Ser Pro Met Met

20                  25                  30

Pro Tyr Gly Thr Gly Leu Thr Pro Gln Pro Ile Gln Asn Thr Asn Ser
        35                  40                  45

Leu Ser Ile Leu Glu Glu Gln Gln Arg Gln Gln Gln Gln Gln Gln Gln
    50                  55                  60

Gln Gln Gln Gln Gln Gln Gln Gln Gln Gln Gln Gln Gln Gln Gln Gln
65                  70                  75                  80

Gln Gln Gln Gln Gln Gln Gln Gln Gln Gln Gln Gln Gln Gln Gln Ala
                85                  90                  95

Val Ala Ala Ala Ala Val Gln Gln Ser Thr Ser Gln Gln Ala Thr Gln
            100                 105                 110

Gly Thr Ser Gly Gln Ala Pro Gln Leu Phe His Ser Gln Thr Leu Thr
        115                 120                 125

Thr Ala Pro Leu Pro Gly Thr Thr Pro Leu Tyr Pro Ser Pro Met Thr
    130                 135                 140

Pro Met Thr Pro Ile Thr Pro Ala Thr Pro Ala Ser Glu Ser Ser Gly
145                 150                 155                 160

Ile Val Pro Gln Leu Gln Asn Ile Val Ser Thr Val Asn Leu Gly Cys
                165                 170                 175

Lys Leu Asp Leu Lys Thr Ile Ala Leu Arg Ala Arg Asn Ala Glu Tyr
            180                 185                 190

Asn Pro Lys Arg Phe Ala Ala Val Ile Met Arg Ile Arg Glu Pro Arg
        195                 200                 205

Thr Thr Ala Leu Ile Phe Ser Ser Gly Lys Met Val Cys Thr Gly Ala
    210                 215                 220

Lys Ser Glu Glu Gln Ser Arg Leu Ala Ala Arg Lys Tyr Ala Arg Val
225                 230                 235                 240

Val Gln Lys Leu Gly Phe Pro Ala Lys Phe Leu Asp Phe Lys Ile Gln
                245                 250                 255

Asn Met Val Gly Ser Cys Asp Val Lys Phe Pro Ile Arg Leu Glu Gly
            260                 265                 270

Leu Val Leu Thr His Gln Gln Phe Ser Ser Tyr Glu Pro Glu Leu Phe
        275                 280                 285

Pro Gly Leu Ile Tyr Arg Met Ile Lys Pro Arg Ile Val Leu Leu Ile
    290                 295                 300

Phe Val Ser Gly Lys Val Val Leu Thr Gly Ala Lys Val Arg Ala Glu
305                 310                 315                 320

Ile Tyr Glu Ala Phe Glu Asn Ile Tyr Pro Ile Leu Lys Gly Phe Arg
                325                 330                 335

Lys Thr Thr
<210>  146

<211>  26

<212>  PRT
<213>  Artificial

<220>
<223>  Synthetic polypeptide

<400> 146

Val Ser Ala Ala Gly Asp Pro Gly Gly Gly Arg Ala Pro Gly Glu Pro
1               5                   10                  15

Ser Arg Pro Lys Ala Leu Cys Leu Pro Gln
            20                  25


<210>  147
<211>  16
<212>  PRT
<213>  Artificial

<220>
<223>  Synthetic polypeptide

<400> 147

Val Arg Lys Ala Asn Arg Lys Gly Ser Phe His Arg Asp Trp Leu Cys
1               5                   10                  15


<210>  148
<211>  26
<212>  PRT
<213>  Artificial

<220>
<223>  Synthetic polypeptide

<400> 148

Val Val Ala Gly Cys Asp Ala Arg Val Lys Gln Lys Ala Trp Gln Pro
1               5                   10                  15

Arg Phe Pro Gly Ile Lys Val Lys Ala Leu
            20                  25


<210>  149
<211>  18
<212>  PRT
<213>  Artificial

<220>
<223>  Synthetic polypeptide

<400> 149

Arg Trp Gly Arg Leu Gly Gly Ala Glu Arg Pro Ser Phe Leu Arg Ala
1               5                   10                  15

Ala Gly
<210>  150

<211>  56

<212>  PRT
<213>  Artificial

<220>
<223>  Synthetic polypeptide

<400> 150

Met Ser Gly Ala Cys Thr Ser Tyr Val Ser Ala Glu Gln Glu Val Val
1               5                   10                  15

Arg Gly Phe Ser Cys Pro Arg Pro Gly Gly Glu Ala Ala Ala Val Phe
            20                  25                  30

Cys Cys Gly Phe Arg Asp His Lys Tyr Cys Cys Asp Asp Pro His Ser
        35                  40                  45

Phe Phe Pro Tyr Glu His Ser Tyr
    50                  55


<210>  151
<211>  81
<212>  PRT
<213>  Artificial

<220>
<223>  Synthetic polypeptide

<400> 151

Glu Glu Val Ser Pro Asp Cys Gln Gly Val Asn Thr Gly Met Ala Ala
1               5                   10                  15

Glu Val Pro Lys Val Ser Pro Leu Gln Gln Ser Tyr Ser Cys Leu Asn
            20                  25                  30

Pro Gln Leu Glu Ser Asn Glu Gly Gln Ala Val Asn Ser Lys Arg Leu
        35                  40                  45

Leu His His Cys Phe Met Ala Thr Val Thr Thr Ser Asp Ile Pro Gly
    50                  55                  60

Ser Pro Glu Glu Ala Ser Val Pro Asn Pro Asp Leu Cys Gly Pro Val
65                  70                  75                  80

Pro
<210>  152

<211>  130
<212>  PRT
<213>  Artificial

<220>
<223>  Synthetic polypeptide

<400> 152

Ser Ile Gly Ala Leu Ile Gly Leu Ser Val Ala Ala Val Val Leu Leu
1               5                   10                  15

Ala Phe Ile Val Thr Ala Cys Val Leu Cys Tyr Leu Phe Ile Ser Ser
            20                  25                  30

Lys Pro His Thr Lys Leu Asp Leu Gly Leu Ser Leu Gln Thr Ala Gly
        35                  40                  45

Pro Glu Glu Val Ser Pro Asp Cys Gln Gly Val Asn Thr Gly Met Ala
    50                  55                  60

Ala Glu Val Pro Lys Val Ser Pro Leu Gln Gln Ser Tyr Ser Cys Leu
65                  70                  75                  80

Asn Pro Gln Leu Glu Ser Asn Glu Gly Gln Ala Val Asn Ser Lys Arg
                85                  90                  95

Leu Leu His His Cys Phe Met Ala Thr Val Thr Thr Ser Asp Ile Pro
            100                 105                 110

Gly Ser Pro Glu Glu Ala Ser Val Pro Asn Pro Asp Leu Cys Gly Pro
        115                 120                 125

Val Pro
    130


<210>  153
<211>  64
<212>  PRT
<213>  Artificial

<220>
<223>  Synthetic polypeptide

<400> 153

Met Ala Ser Leu Trp Pro Ser Ala Leu Thr Phe Asn Thr Asp Ala Asn
1               5                   10                  15

Ile Pro Gly Pro Leu Gly Phe Cys Gly Gly Trp Val Arg Leu Cys Ser
            20                  25                  30

Leu Ser Ser Leu Thr Pro Pro Cys Gly Arg Arg Leu Val Pro Cys Leu
        35                  40                  45

Ser Ala Pro Ala Pro Asn Ala Pro Arg Leu Pro Ala Pro Ala Arg Cys
    50                  55                  60


<210>  154
<211>  81
<212>  PRT
<213>  Artificial

<220>
<223>  Synthetic polypeptide

<400> 154

Glu Glu Val Ser Pro Asp Cys Gln Gly Val Asn Thr Gly Met Ala Ala
1               5                   10                  15

Glu Val Pro Lys Val Ser Pro Leu Gln Gln Ser Tyr Ser Cys Leu Asn
            20                  25                  30

Pro Gln Leu Glu Ser Asn Glu Gly Gln Ala Val Asn Ser Lys Arg Leu

35                  40                  45

Leu His His Cys Phe Met Ala Thr Val Thr Thr Ser Asp Ile Pro Gly
    50                  55                  60

Ser Pro Glu Glu Ala Ser Val Pro Asn Pro Asp Leu Cys Gly Pro Val
65                  70                  75                  80

Pro
<210>  155

<211>  165
<212>  PRT
<213>  Artificial

<220>
<223>  Synthetic polypeptide

<400> 155

Met His Gln Ser Leu Thr Gln Gln Arg Ser Ser Asp Met Ser Leu Pro
1               5                   10                  15

Asp Ser Met Gly Ala Phe Asn Arg Arg Lys Arg Asn Ser Ile Tyr Val
            20                  25                  30

Thr Val Thr Leu Leu Ile Val Ser Val Leu Ile Leu Thr Val Gly Leu
        35                  40                  45

Ala Ala Thr Thr Arg Thr Gln Asn Val Thr Val Gly Gly Tyr Tyr Pro
    50                  55                  60

Gly Val Ile Leu Gly Phe Gly Ser Phe Leu Gly Ile Ile Gly Ser Asn
65                  70                  75                  80

Leu Ile Glu Asn Lys Arg Gln Met Leu Val Ala Ser Ile Val Phe Ile
                85                  90                  95

Ser Phe Gly Val Ile Ala Ala Phe Cys Cys Ala Ile Val Asp Gly Val
            100                 105                 110

Phe Ala Ala Arg His Ile Asp Leu Lys Pro Leu Tyr Ala Asn Arg Cys
        115                 120                 125

His Tyr Val Pro Lys Thr Ser Gln Lys Glu Ala Glu Glu Val Ile Ser
    130                 135                 140

Ser Ser Thr Lys Asn Ser Pro Ser Thr Arg Val Met Arg Asn Leu Thr
145                 150                 155                 160

Gln Ala Ala Arg Glu
                165


<210>  156
<211>  141
<212>  PRT
<213>  Artificial

<220>
<223>  Synthetic polypeptide

<400> 156

Met His Gln Ser Leu Thr Gln Gln Arg Ser Ser Asp Met Ser Leu Pro
1               5                   10                  15

Asp Ser Met Gly Ala Phe Asn Arg Arg Lys Arg Asn Ser Ile Tyr Val
            20                  25                  30

Thr Val Thr Leu Leu Ile Val Ser Val Leu Ile Leu Thr Val Gly Leu
        35                  40                  45

Ala Ala Thr Thr Arg Thr Gln Asn Val Thr Val Gly Gly Tyr Tyr Pro
    50                  55                  60

Gly Val Ile Leu Gly Phe Gly Ser Phe Leu Gly Ile Ile Gly Ser Asn
65                  70                  75                  80

Leu Ile Glu Asn Lys Arg Gln Met Leu Val Ala Ser Ile Val Phe Ile
                85                  90                  95

Ser Phe Gly Val Ile Ala Ala Phe Cys Cys Ala Ile Val Asp Gly Val
            100                 105                 110

Phe Ala Ala Arg His Ile Asp Leu Lys Pro Leu Tyr Ala Asn Arg Cys
        115                 120                 125

His Tyr Val Pro Lys Thr Ser Gln Lys Glu Ala Glu Glu
    130                 135                 140

<210>  157
<211>  78
<212>  PRT
<213>  Artificial

<220>
<223>  Synthetic polypeptide

<400> 157

Met Leu Val Ala Ser Ile Val Phe Ile Ser Phe Gly Val Ile Ala Ala
1               5                   10                  15

Phe Cys Cys Ala Ile Val Asp Gly Val Phe Ala Ala Arg His Ile Asp
            20                  25                  30

Leu Lys Pro Leu Tyr Ala Asn Arg Cys His Tyr Val Pro Lys Thr Ser
        35                  40                  45

Gln Lys Glu Ala Glu Glu Val Ile Ser Ser Ser Thr Lys Asn Ser Pro
    50                  55                  60

Ser Thr Arg Val Met Arg Asn Leu Thr Gln Ala Ala Arg Glu
65                  70                  75

<210>  158
<211>  141
<212>  PRT
<213>  Artificial

<220>
<223>  Synthetic polypeptide

<400> 158

Met His Gln Ser Leu Thr Gln Gln Arg Ser Ser Asp Met Ser Leu Pro
1               5                   10                  15

Asp Ser Met Gly Ala Phe Asn Arg Arg Lys Arg Asn Ser Ile Tyr Val
            20                  25                  30

Thr Val Thr Leu Leu Ile Val Ser Val Leu Ile Leu Thr Val Gly Leu
        35                  40                  45

Ala Ala Thr Thr Arg Thr Gln Asn Val Thr Val Gly Gly Tyr Tyr Pro
    50                  55                  60

Gly Val Ile Leu Gly Phe Gly Ser Phe Leu Gly Ile Ile Gly Ser Asn
65                  70                  75                  80

Leu Ile Glu Asn Lys Arg Gln Met Leu Val Ala Ser Ile Val Phe Ile
                85                  90                  95

Ser Phe Gly Val Ile Ala Ala Phe Cys Cys Ala Ile Val Asp Gly Val
            100                 105                 110

Phe Ala Ala Arg His Ile Asp Leu Lys Pro Leu Tyr Ala Asn Arg Cys
        115                 120                 125

His Tyr Val Pro Lys Thr Ser Gln Lys Glu Ala Glu Glu
    130                 135                 140


<210>  159
<211>  67
<212>  PRT
<213>  Artificial

<220>
<223>  Synthetic polypeptide

<400> 159

Met His Gln Ser Leu Thr Gln Gln Arg Ser Ser Asp Met Ser Leu Pro
1               5                   10                  15

Asp Ser Met Gly Ala Phe Asn Arg Arg Lys Arg Asn Ser Ile Tyr Val
            20                  25                  30

Thr Val Thr Leu Leu Ile Val Ser Val Leu Ile Leu Thr Val Gly Leu
        35                  40                  45

Ala Ala Thr Thr Arg Thr Gln Asn Val Thr Val Gly Gly Tyr Tyr Pro
    50                  55                  60

Gly Val Ile
65


<210>  160
<211>  120
<212>  PRT
<213>  Artificial

<220>
<223>  Synthetic polypeptide

<400> 160

Met His Gln Ser Leu Thr Gln Gln Arg Ser Ser Asp Met Ser Leu Pro
1               5                   10                  15

Asp Ser Met Gly Ala Phe Asn Arg Arg Lys Arg Asn Ser Ile Tyr Val
            20                  25                  30

Thr Val Thr Leu Leu Ile Val Ser Val Leu Ile Leu Thr Val Gly Leu
        35                  40                  45

Ala Ala Thr Thr Arg Thr Gln Asn Val Thr Val Gly Gly Tyr Tyr Pro
    50                  55                  60

Gly Val Ile Leu Val Ala Ser Ile Val Phe Ile Ser Phe Gly Val Ile
65                  70                  75                  80

Ala Ala Phe Cys Cys Ala Ile Val Asp Gly Val Phe Ala Ala Arg His
                85                  90                  95

Ile Asp Leu Lys Pro Leu Tyr Ala Asn Arg Cys His Tyr Val Pro Lys
            100                 105                 110

Thr Ser Gln Lys Glu Ala Glu Glu
        115                 120


<210>  161
<211>  55
<212>  PRT
<213>  Artificial

<220>
<223>  Synthetic polypeptide

<400> 161

Met Gln Ala Pro Arg Ala Ala Leu Val Phe Ala Leu Val Ile Ala Leu
1               5                   10                  15

Val Pro Val Gly Arg Gly Asn Tyr Glu Glu Leu Glu Asn Ser Gly Asp
            20                  25                  30

Thr Thr Val Glu Ser Glu Arg Pro Asn Lys Val Thr Ile Pro Ser Thr
        35                  40                  45

Phe Ala Ala Val Thr Ile Lys
    50                  55


<210>  162
<211>  23
<212>  PRT
<213>  Artificial

<220>
<223>  Synthetic polypeptide

<400> 162

Cys Lys Ile Gln Leu Ser Trp Lys Val Ile Pro Ala Phe Cys Leu Glu
1               5                   10                  15

Ser Ser His Arg Asn Ala Leu
            20

<210>  163
<211>  33
<212>  DNA
<213>  Artificial

<220>
<223>  Synthetic polynucleotide

<400> 163

ctagctagcc accatgaggc gctgcagtct ctg                                    33

<210>  164
<211>  32
<212>  DNA
<213>  Artificial

<220>
<223>  Synthetic polynucleotide

<400> 164

cgcgaccggt ccaacccaac ttctctgtga tg                                     32

<210>  165
<211>  34
<212>  DNA
<213>  Artificial

<220>
<223>  Synthetic polynucleotide

<400> 165

cgattgtaca agaggcgctg cagtctctgc gctt                                   34

<210>  166
<211>  31
<212>  DNA
<213>  Artificial

<220>
<223>  Synthetic polynucleotide

<400> 166

gcgcgcggcc gcctaaaccc aacttctctg t                                      31

<210>  167
<211>  32
<212>  DNA
<213>  Artificial

<220>

<223>  Synthetic polynucleotide

<400> 167

ctagctagcc accatgcatc agtccctgac tc                                    32


<210>  168
<211>  75
<212>  DNA
<213>  Artificial

<220>
<223>  Synthetic polynucleotide

<400> 168

ataagaatgc ggccgctcat tacttgtcgt catcgtcttt gtagtcggga ctgtaaggtg      60

gtggcttttc aaaag                                                       75


<210>  169
<211>  19
<212>  DNA
<213>  Artificial

<220>
<223>  Synthetic polynucleotide

<400> 169

agagcccagc aggccaaag                                                   19


<210>  170
<211>  19
<212>  DNA
<213>  Artificial

<220>
<223>  Synthetic polynucleotide

<400> 170

agcaggaccc agtgcagtg                                                   19


<210>  171
<211>  111
<212>  DNA
<213>  Artificial

<220>
<223>  Synthetic polynucleotide

<400> 171

agagcccagc aggccaaagg ctttgtgtct tccacagagc gtttccgtgg gacttgtggc      60

cctcctggcg tccaggaacc ttcttcgccc tccactgcac tgggtcctgc t              111

<210>  172
<211>  30
<212>  DNA
<213>  Artificial

<220>
<223>  Synthetic polynucleotide

<400> 172

ctagctagcc accatggaga agtttcgggc                                        30


<210>  173
<211>  68
<212>  DNA
<213>  Artificial

<220>
<223>  Synthetic polynucleotide

<400> 173

cgcaccggtt cattacttgt cgtcatcgtc tttgtagtct aactcaggag tgctgtttat      60

accagatg                                                                68


<210>  174
<211>  972
<212>  DNA
<213>  Artificial

<220>
<223>  Synthetic polynucleotide

<400> 174

atggagaagt ttcgggcggt gctggacctg cacgtcaagc accacagcgc cttgggctac      60

ggcctggtga ccctgctgac ggcgggcggg gagcgcatct tctccgccgt ggcattccag     120

tgcccgtgca gcgccgcctg gaacctgccc tacggcctgg tcttcttgct ggtgccggcg     180

ctcgcgctct tcctcctggg ctacgtgctg agcgcacgca cgtggcgcct gctcaccgga     240

tgctgctcca gcgcccgcgc gagttgcgga tcggcgctgc gcggctccct ggtgtgcgcg     300

caaatcagcg cggccgccgc gctcgcgccc ctcacctggg tggccgtggc gctgctcggg     360

ggcgcctttt acgagtgcgc ggccaccggg agcgcggcct tcgcgcagcg cctgtgcctc     420

ggccgcaacc gcagctgcgc cgcggagctg ccgctggtgc cgtgcaacca ggccaaggcg     480

tcggacgtgc aggacctcct gaaggatctg aaggctcagt cgcaggtgtt gggctggatc     540

ttgatagcag ttgttatcat cattcttctg atttttacat ctgtcacccg atgcctatct     600

ccagttagtt ttctgcagct gaaattctgg aaaatctatt tggaacagga gcagcagatc     660

cttaaaagta aagccacaga gcatgcaact gaattggcaa aagagaatat taaatgtttc     720

tttgagggct cgcatccaaa agaatataac actccaagca tgaaagagtg gcagcaaatt     780

tcatcactgt atactttcaa tccgaagggc cagtactaca gcatgttgca caaatatgtc     840

aacagaaaag agaagactca cagtatcagg tctactgaag gagatacggt gattcctgtt     900

cttggctttg tagattcatc tggtataaac agcactcctg agttagacta caaagacgat     960

gacgacaagt aa                                                         972

<210>  175
<211>  323
<212>  PRT
<213>  Artificial

<220>
<223>  Synthetic polypeptide

<400> 175
   Met Glu Lys Phe Arg Ala Val Leu Asp Leu His Val Lys His His Ser
   1               5                   10                  15
   Ala Leu Gly Tyr Gly Leu Val Thr Leu Leu Thr Ala Gly Gly Glu Arg
               20                  25                  30
   Ile Phe Ser Ala Val Ala Phe Gln Cys Pro Cys Ser Ala Ala Trp Asn
           35                  40                  45
   Leu Pro Tyr Gly Leu Val Phe Leu Leu Val Pro Ala Leu Ala Leu Phe
       50                  55                  60
   Leu Leu Gly Tyr Val Leu Ser Ala Arg Thr Trp Arg Leu Leu Thr Gly
   65                  70                  75                  80
   Cys Cys Ser Ser Ala Arg Ala Ser Cys Gly Ser Ala Leu Arg Gly Ser
                   85                  90                  95
   Leu Val Cys Ala Gln Ile Ser Ala Ala Ala Ala Leu Ala Pro Leu Thr
               100                 105                 110
   Trp Val Ala Val Ala Leu Leu Gly Gly Ala Phe Tyr Glu Cys Ala Ala
           115                 120                 125
   Thr Gly Ser Ala Ala Phe Ala Gln Arg Leu Cys Leu Gly Arg Asn Arg
       130                 135                 140
   Ser Cys Ala Ala Glu Leu Pro Leu Val Pro Cys Asn Gln Ala Lys Ala
   145                 150                 155                 160
   Ser Asp Val Gln Asp Leu Leu Lys Asp Leu Lys Ala Gln Ser Gln Val
                   165                 170                 175
   Leu Gly Trp Ile Leu Ile Ala Val Val Ile Ile Ile Leu Leu Ile Phe
               180                 185                 190
   Thr Ser Val Thr Arg Cys Leu Ser Pro Val Ser Phe Leu Gln Leu Lys
           195                 200                 205
   Phe Trp Lys Ile Tyr Leu Glu Gln Glu Gln Gln Ile Leu Lys Ser Lys
       210                 215                 220
   Ala Thr Glu His Ala Thr Glu Leu Ala Lys Glu Asn Ile Lys Cys Phe
   225                 230                 235                 240
   Phe Glu Gly Ser His Pro Lys Glu Tyr Asn Thr Pro Ser Met Lys Glu
                   245                 250                 255
   Trp Gln Gln Ile Ser Ser Leu Tyr Thr Phe Asn Pro Lys Gly Gln Tyr
               260                 265                 270
   Tyr Ser Met Leu His Lys Tyr Val Asn Arg Lys Glu Lys Thr His Ser
           275                 280                 285
   Ile Arg Ser Thr Glu Gly Asp Thr Val Ile Pro Val Leu Gly Phe Val
       290                 295                 300
   Asp Ser Ser Gly Ile Asn Ser Thr Pro Glu Leu Asp Tyr Lys Asp Asp
   305                 310                 315                 320
   Asp Asp Lys
<210>  176

<211>  20
<212>  DNA
<213>  Artificial

<220>
<223>  Synthetic polynucleotide

<400> 176

tcgtctctgc cgttctcagg                                                   20


<210>  177
<211>  24
<212>  DNA
<213>  Artificial

<220>
<223>  Synthetic polynucleotide

<400> 177

gggatggtat ggagacattt attg                                              24


<210>  178
<211>  60
<212>  DNA
<213>  Artificial

<220>
<223>  Synthetic polynucleotide

<400> 178

ctagctagcc accatggact acaaagacga tgacgacaag agcggcgcct gcacgagcta      60


<210>  179
<211>  30
<212>  DNA
<213>  Artificial

<220>
<223>  Synthetic polynucleotide

<400> 179

cggaattctt atgggactgg tccacatagg                                        30


<210>  180
<211>  37
<212>  DNA
<213>  Artificial

<220>
<223>  Synthetic polynucleotide

<400> 180

ctagctagcc accatgagcg gcgcctgcac gagctac                                37


<210>  181
<211>  57
<212>  DNA
<213>  Artificial

<220>
<223>  Synthetic polynucleotide

<400> 181

ccggaattct tacttgtcgt catcgtcttt gtagtctggg actggtccac ataggtc          57


<210>  182
<211>  597
<212>  DNA
<213>  Artificial

<220>
<223>  Synthetic polynucleotide

<400> 182

atggactaca aagacgatga cgacaagagc ggcgcctgca cgagctacgt gagcgcagag      60

caggaggtgg tgcgcggctt cagctgcccg cggccggggg gcgaggcggc cgctgtcttc     120

tgctgcggct tccgcgacca caagtactgc tgcgacgacc cgcacagctt cttcccctac     180

gagcacagct acatgtggtg gctcagcatt ggcgctctca taggcctgtc cgtagcagca     240

gtggttcttc tcgccttcat tgttaccgcc tgtgtgctct gctacctgtt catcagctct     300

aagccccaca caaagttgga cctgggcttg agcttacaga cagcaggccc tgaggaggtt     360

tctcctgact gccaaggtgt gaacacaggc atggcggcag aagtgccaaa agtgagccct     420

ctccagcaga gttactcctg cttgaacccg cagctggaga gcaatgaggg gcaggctgtg     480

aactccaaac gcctcctcca tcattgcttc atggccacag tgaccaccag tgacattcca     540

ggcagccctg aggaagcctc tgtacccaac cctgacctat gtggaccagt cccataa        597

<210>  183
<211>  597
<212>  DNA
<213>  Artificial

<220>
<223>  Synthetic polynucleotide

<400> 183

atgagcggcg cctgcacgag ctacgtgagc gcagagcagg aggtggtgcg cggcttcagc      60

tgcccgcggc cggggggcga ggcggccgct gtcttctgct gcggcttccg cgaccacaag      120

tactgctgcg acgacccgca cagcttcttc ccctacgagc acagctacat gtggtggctc      180

agcattggcg ctctcatagg cctgtccgta gcagcagtgg ttcttctcgc cttcattgtt      240

accgcctgtg tgctctgcta cctgttcatc agctctaagc cccacacaaa gttggacctg      300

ggcttgagct tacagacagc aggccctgag gaggtttctc ctgactgcca aggtgtgaac      360

acaggcatgg cggcagaagt gccaaaagtg agccctctcc agcagagtta ctcctgcttg      420

aacccgcagc tggagagcaa tgaggggcag gctgtgaact ccaaacgcct cctccatcat      480

tgcttcatgg ccacagtgac caccagtgac attccaggca gccctgagga agcctctgta      540

cccaaccctg acctatgtgg accagtccca gactacaaag acgatgacga caagtaa      597


<210>  184
<211>  198
<212>  PRT
<213>  Artificial

<220>
<223>  Synthetic polynucleotide

<400> 184

Met Asp Tyr Lys Asp Asp Asp Asp Lys Ser Gly Ala Cys Thr Ser Tyr
1               5                   10                  15

Val Ser Ala Glu Gln Glu Val Val Arg Gly Phe Ser Cys Pro Arg Pro
            20                  25                  30

Gly Gly Glu Ala Ala Ala Val Phe Cys Cys Gly Phe Arg Asp His Lys
        35                  40                  45

Tyr Cys Cys Asp Asp Pro His Ser Phe Phe Pro Tyr Glu His Ser Tyr
    50                  55                  60

Met Trp Trp Leu Ser Ile Gly Ala Leu Ile Gly Leu Ser Val Ala Ala
65                  70                  75                  80

Val Val Leu Leu Ala Phe Ile Val Thr Ala Cys Val Leu Cys Tyr Leu
                85                  90                  95

Phe Ile Ser Ser Lys Pro His Thr Lys Leu Asp Leu Gly Leu Ser Leu
            100                 105                 110

Gln Thr Ala Gly Pro Glu Glu Val Ser Pro Asp Cys Gln Gly Val Asn
        115                 120                 125

Thr Gly Met Ala Ala Glu Val Pro Lys Val Ser Pro Leu Gln Gln Ser
    130                 135                 140

Tyr Ser Cys Leu Asn Pro Gln Leu Glu Ser Asn Glu Gly Gln Ala Val
145                 150                 155                 160

Asn Ser Lys Arg Leu Leu His His Cys Phe Met Ala Thr Val Thr Thr
                165                 170                 175

Ser Asp Ile Pro Gly Ser Pro Glu Glu Ala Ser Val Pro Asn Pro Asp
            180                 185                 190

Leu Cys Gly Pro Val Pro
        195


<210>  185
<211>  198
<212>  PRT
<213>  Artificial

<220>
<223>  Synthetic polypeptide

<400> 185

Met Ser Gly Ala Cys Thr Ser Tyr Val Ser Ala Glu Gln Glu Val Val
1               5                   10                  15

Arg Gly Phe Ser Cys Pro Arg Pro Gly Gly Glu Ala Ala Ala Val Phe
            20                  25                  30

Cys Cys Gly Phe Arg Asp His Lys Tyr Cys Cys Asp Asp Pro His Ser
        35                  40                  45

Phe Phe Pro Tyr Glu His Ser Tyr Met Trp Trp Leu Ser Ile Gly Ala
    50                  55                  60

Leu Ile Gly Leu Ser Val Ala Ala Val Val Leu Leu Ala Phe Ile Val
65                  70                  75                  80

Thr Ala Cys Val Leu Cys Tyr Leu Phe Ile Ser Ser Lys Pro His Thr
                85                  90                  95

Lys Leu Asp Leu Gly Leu Ser Leu Gln Thr Ala Gly Pro Glu Glu Val
            100                 105                 110

Ser Pro Asp Cys Gln Gly Val Asn Thr Gly Met Ala Ala Glu Val Pro
        115                 120                 125

Lys Val Ser Pro Leu Gln Gln Ser Tyr Ser Cys Leu Asn Pro Gln Leu
    130                 135                 140

Glu Ser Asn Glu Gly Gln Ala Val Asn Ser Lys Arg Leu Leu His His
145                 150                 155                 160

Cys Phe Met Ala Thr Val Thr Thr Ser Asp Ile Pro Gly Ser Pro Glu
                165                 170                 175

Glu Ala Ser Val Pro Asn Pro Asp Leu Cys Gly Pro Val Pro Asp Tyr
            180                 185                 190

Lys Asp Asp Asp Asp Lys
        195

<210>  186
<211>  18
<212>  PRT
<213>  Artificial

<220>
<223>  Synthetic polynucleotide

<400> 186

Met His Gln Ser Leu Thr Gln Gln Arg Ser Ser Asp Met Ser Leu Pro
1               5                   10                  15

Asp Ser
<210>  187

<211>  38
<212>  DNA
<213>  Artificial

<220>
<223>  Synthetic polynucleotide

<400> 187

ctagctagcc accatgcagg ctccccgcgc agccctag                              38

<210>  188
<211>  53
<212>  DNA
<213>  Artificial

<220>
<223>  Synthetic polynucleotide

<400> 188

ttacttgtcg tcatcgtctt tgtagtcgga ttcactgtca cttgggttgt gat             53

<210>  189
<211>  576
<212>  DNA
<213>  Artificial

<220>
<223>  Synthetic polypeptide

<400> 189

atgcaggctc cccgcgcagc cctagtcttc gccctggtga tcgcgctcgt tcccgtcggc      60

cggggtaatt atgaggaatt agaaaactca ggagatacaa ctgtggaatc tgaaagacca     120

aataaagtga ctattccaag cacatttgct gcagtgacca tcaaagaaac attaaatgca     180

aatataaatt ctaccaactt tgctccggat gaaaatcagt tagagtttat actgatggtg 240

ttaatcccat tgattttatt ggtcctctta cttttatccg tggtattcct tgcaacatac 300

tataaaagaa aaagaactaa acaagaacct tctagccaag gatctcagag tgctttacag 360

acatatgaac tgggaagtga aaacgtgaaa gtccctattt ttgaggaaga tacaccctct 420

gttatggaaa ttgaaatgga agagcttgat aaatggatga acagcatgaa tagaaatgcc 480

gactttgaat gtttacctac cttgaaggaa gagaaggaat caaatcacaa cccaagtgac 540

agtgaatccg actacaaaga cgatgacgac aagtaa 576

<210> 190
<211> 191
<212> PRT
<213> Artificial

<220>
<223> Synthetic polypeptide

<400> 190

Met Gln Ala Pro Arg Ala Ala Leu Val Phe Ala Leu Val Ile Ala Leu
1               5                   10                  15

Val Pro Val Gly Arg Gly Asn Tyr Glu Glu Leu Glu Asn Ser Gly Asp
            20                  25                  30

Thr Thr Val Glu Ser Glu Arg Pro Asn Lys Val Thr Ile Pro Ser Thr
        35                  40                  45

Phe Ala Ala Val Thr Ile Lys Glu Thr Leu Asn Ala Asn Ile Asn Ser
    50                  55                  60

Thr Asn Phe Ala Pro Asp Glu Asn Gln Leu Glu Phe Ile Leu Met Val
65                  70                  75                  80

Leu Ile Pro Leu Ile Leu Leu Val Leu Leu Leu Leu Ser Val Val Phe
                85                  90                  95

Leu Ala Thr Tyr Tyr Lys Arg Lys Arg Thr Lys Gln Glu Pro Ser Ser
            100                 105                 110

Gln Gly Ser Gln Ser Ala Leu Gln Thr Tyr Glu Leu Gly Ser Glu Asn
        115                 120                 125

Val Lys Val Pro Ile Phe Glu Glu Asp Thr Pro Ser Val Met Glu Ile
    130                 135                 140

Glu Met Glu Glu Leu Asp Lys Trp Met Asn Ser Met Asn Arg Asn Ala
145                 150                 155                 160

Asp Phe Glu Cys Leu Pro Thr Leu Lys Glu Glu Lys Glu Ser Asn His
                165                 170                 175

Asn Pro Ser Asp Ser Glu Ser Asp Tyr Lys Asp Asp Asp Asp Lys
            180                 185                 190

<210>  191
<211>  19
<212>  PRT
<213>  Artificial

<220>
<223>  Synthetic polypeptide

<400> 191

Arg Gly Asn Tyr Glu Glu Leu Glu Asn Ser Gly Asp Thr Thr Val Glu
1               5                   10                  15

Ser Glu Arg
<210>  192

<211>  17
<212>  PRT
<213>  Artificial

<220>
<223>  Synthetic polypeptide

<400> 192

Tyr Lys Arg Lys Arg Thr Lys Gln Glu Pro Ser Ser Gln Gly Ser Gln
1               5                   10                  15

Ser
<210>  193

<211>  78
<212>  DNA
<213>  Artificial

<220>
<223>  Synthetic polynucleotide

<400> 193

gtgagcgcgg caggcgaccc gggcgggggc cgggctcccg gagagcccag caggccaaag      60

gctttgtgtc ttccacag                                                    78


<210>  194
<211>  48
<212>  DNA
<213>  Artificial

<220>
<223>  Synthetic polynucleotide

<400> 194

gtaaggaagg caaacaggaa gggttcattc cacagagact ggctgtgt                   48


<210>  195
<211>  20
<212>  DNA
<213>  Artificial

<220>
<223>  Synthetic polynucleotide

<400> 195

ccctccactg gatacagcct                                                   20


<210>  196
<211>  20
<212>  PRT
<213>  Artificial

<220>
<223>  Synthetic polypeptide

<400> 196

Pro Lys Thr Ser Gln Lys Glu Ala Glu Glu Asn Pro Thr Leu Pro Ala
1               5                   10                  15

Leu Asn Cys Ser
            20


<210>  197
<211>  20
<212>  DNA
<213>  Artificial

<220>
<223>  Synthetic polynucleotide

<400> 197

agctgaggag aacccaactc                                                   20


<210>  198
<211>  20
<212>  DNA
<213>  Artificial

<220>
<223>  Synthetic polynucleotide

<400> 198

cggagttatt ctggtggctt                                                   20


<210>  199
<211>  20
<212>  PRT
<213>  Artificial

<220>
<223>  Synthetic polypeptide

<400> 199

Thr Val Gly Gly Tyr Tyr Pro Gly Val Ile Leu Val Ala Ser Ile Val
1               5                   10                  15

Phe Ile Ser Phe
            20

<210>  200
<211>  35
<212>  PRT
<213>  Artificial

<220>
<223>  Synthetic polypeptide

<400> 200

Leu Cys Ser Leu Ser Ser Leu Thr Pro Pro Cys Gly Arg Arg Leu Val
1               5                   10                  15

Pro Cys Leu Ser Ala Pro Ala Pro Asn Ala Pro Arg Leu Pro Ala Pro
            20                  25                  30

Ala Arg Cys
        35

<210>  201
<211>  108
<212>  DNA
<213>  Artificial

<220>
<223>  Synthetic polynucleotide

<400> 201

gttgtgttca ctctcctcac tgaccccgcc gtgtggtcgc cggctggtgc cttgtctgtc      60

tgccccagcc ccgaatgccc cgagactccc ggcgccggct cggtgcag                  108

## Description

### FIELD OF THE INVENTION

This invention relates to KRTCAP3, FAM26F, MGC52498, FAM70A and TMEM154 related polypeptides and polynucleotides that are differentially expressed in some cancers and specific blood cells, and therefore are suitable targets for development of therapeutics and diagnostics, particularly for cancer therapy and treatment of immune related disorders.

### BACKGROUND OF THE INVENTION

Tumor antigens are ideally positioned as biomarkers and drug targets, and they play a critical role in the development of novel strategies for active and passive immunotherapy agents, to be used as stand-alone therapies or in conjunction with conventional therapies for cancer. Tumor antigens can be classified as either tumor-specific antigens (TSAs) where the antigens are expressed only in tumor cells and not in normal tissues, or tumor-associated antigens (TAAs) where the antigens are overexpressed in tumor cells but nonetheless also present at low levels in normal tissues.

TAAs and TSAs are validated as targets for passive (antibody) therapy as well as active immunotherapy using strategies to break immune tolerance and stimulate the immune system. The antigenic epitopes that are targeted by these therapeutic approaches are present at the cell surface, overexpressed in tumor cells compared to non-tumor cells, and are targeted by antibodies that block functional activity, inhibit cell proliferation, or induce cell death.

There are a growing number of tumor-associated antigens against which monoclonal antibodies have been tested or are in use as treatment for cancer. The identification and molecular characterization of novel tumor antigens expressed by human malignancies is an active field in tumor immunology. Several approaches have been used to identify tumor-associated antigens as target candidates for immunotherapy, including high throughput bioinformatic approaches, based on genomics and proteomics. The identification of novel TAAs or TSAs expands the spectrum of tumor antigen targets available for immune recognition and provides new target molecules for the development of therapeutic agents for passive immunotherapy, including monoclonal antibodies, whether unmodified or armed. Such novel antigens may also point the way to more effective therapeutic vaccines for active or adoptive immunotherapy.

Cancer vaccination involves the administration of tumor antigens and is used to break immune tolerance and induce an active T-cell response to the tumor. Vaccine therapy includes the use of naked DNA, peptides, recombinant protein, and whole cell therapy, where the patient's own tumor cells are used as the source of the vaccine. With the identification of specific tumor antigens, vaccinations are more often carried out by dendritic cell therapy, whereby dendritic cells are loaded with the relevant protein or peptide, or transfected with vector DNA or RNA.

The major applications of anti-TAA antibodies for treatment of cancer are therapy with naked antibody, therapy with a drug-conjugated antibody, and fusion therapy with cellular immunity. Ever since their discovery, antibodies were envisioned as "magic bullets" that would deliver toxic agents, such as drugs, toxins, enzymes and radioisotopes, specifically to the diseased site and leaving the non-target normal tissues unaffected. Indeed, antibodies, and in particular antibody fragments, can function as carriers of cytotoxic substances such as radioisotopes, drugs and toxins. Immunotherapy with such immunoconjugates is more effective than with the naked antibody.

In contrast to the overwhelming success of naked (such as Rituxan and Campath) and conjugated antibodies (such as Bexxar and Zevalin) in treating hematological malignancies, only modest success has been achieved in the immunotherapy of solid tumors. One of the major limitations in successful application of immunotherapy to solid tumors is the large molecular size of the intact immunoglobulin that results in prolonged serum half-life but in poor tumor penetration and uptake. Indeed, only a very small amount of administered antibody (as low as 0.01%) reaches the tumor. In addition to their size, antibodies encounter other impediments before reaching their target antigens expressed on the cell surface of solid tumors. Some of the barriers include poor blood flow in large tumors, permeability of vascular endothelium, elevated interstitial fluid pressure of tumor stroma, and heterogenous antigen expression.

With the advent of antibody engineering, small molecular weight antibody fragments exhibiting improved tumor penetration have been generated. Such antibody fragments are often conjugated to specific cytotoxic molecules and are designed to selectively deliver them to cancer cells. Still, solid tumors remain a formidable challenge for therapy, even with immunoconjugated antibody fragments.

The new wave of optimization strategies involves the use of biological modifiers to modulate the impediments posed by solid tumors. Thus, in combination to antibodies or their conjugated antibody fragments, various agents are being used to improve the tumor blood flow, enhance vascular permeability, lower tumor interstitial fluid pressure by modulating stromal cells and extracellular matrix components, upregulate expression of target antigens and improve penetration and retention of the therapeutic agent.

Immunotherapy with antibodies represents an exciting opportunity for combining with standard modalities, such as chemotherapy, as well as combinations with diverse biological agents to obtain a synergistic activity. Indeed, unconjugated mAbs are more effective when used in combination with other therapeutic agents, including other antibodies.

Passive tumor immunotherapy uses the exquisite specificity and lytic capability of the immune system to target tumor specific antigens and treat malignant disease with a minimum of damage to normal tissue. Several approaches have been used to identify tumor-associated antigens as target candidates for immunotherapy. The identification of novel tumor specific antigens expands the spectrum of tumor antigen targets available for immune recognition and provides new target molecules for the development of therapeutic agents for passive immunotherapy, including monoclonal antibodies, whether unmodified or armed. Such novel antigens may also point the way to more effective therapeutic vaccines for active or adoptive immunotherapy.

Despite recent progress in the understanding of cancer biology and cancer treatment, as well as better understanding of the molecules involved in immune responses, the success rate for cancer therapy and for the treatment of immune related disorders remains low. Therefore, there is an unmet need for new therapies which can successfully treat both cancer and immune related disorders.

### BRIEF SUMMARY OF THE INVENTION

In at least some embodiments, the subject invention provides novel amino acid and nucleic acid sequences, which are variants of the corresponding amino acid sequences and nucleic acid sequences for known or "WT" (wild type") KRTCAP3, FAM26F, MGC52498, FAM70A, or TMEM154, respectively. According to at least some embodiments of the present invention, the KRTCAP3, FAM26F, MGC52498, FAM70A, and TMEM154 proteins are differentially expressed by some cancers and specific blood cells, and therefore are suitable targets for cancer therapy, treatment of immune related conditions, and drug development. As described in greater detail below, the terms "polypeptides" and "proteins" are used to describe specific variants, the known proteins themselves or derived amino acid sequences related to KRTCAP3, FAM26F, MGC52498, FAM70A, or TMEM154, or fragments or portions of any of the above.

In at least some embodiments, the subject invention provides novel therapeutic and diagnostic compositions containing at least one of the KRTCAP3, FAM26F, MGC52498, FAM70A, or TMEM154 proteins, or variants disclosed herein, or nucleic acid sequences encoding same.

In at least some embodiments, the subject invention provides discrete portions of KRTCAP3; FAM26F; MGC52498; FAM70A, and TMEM154 proteins, variants, and nucleic acid sequences encoding same or fragments thereof.

In at least some embodiments, the subject invention provides a secreted form of TMEM154 proteins, especially the extracellular domain (ECD) of TMEM154 proteins and nucleic acid sequences encoding same or fragments or portions or homologous or conjugates thereof, and compositions comprising same.

According to at least some embodiments of the present invention, the polypeptides corresponding to an extracellular domain of TMEM154 proteins are used as therapeutic agents for cancer therapy, treatment of immune related conditions, and drug development.

In at least some embodiments, the subject invention provides polypeptides corresponding to an extracellular domain of KRTCAP3, FAM26F, MGC52498, FAM70A, proteins and/or new variants, and nucleic acid sequences encoding same or fragments or homologous thereof.

In at least some embodiments, the subject invention provides therapeutic and diagnostic antibodies, antibody fragments and compositions comprising same, and therapies and diagnostic methods using said antibodies and antibody fragments that specifically bind to any one of KRTCAP3; FAM26F; MGC52498; FAM70A, and TMEM154 proteins, or variants, or a soluble or extracellular portion thereof, especially the ectodomain, or a the unique bridge, edge portion, tail or head portion thereof.

According to at least some embodiments of the present invention, the KRTCAP3, FAM26F, MGC52498, FAM70A, TMEM154 proteins and/or variants polypeptides and nucleic acid sequences are used as novel targets for development of drugs which specifically bind to the KRTCAP3, FAM26F, MGC52498, FAM70A, TMEM154 proteins and/or new variants, and/or drugs which agonize or antagonize the binding of other moieties to the KRTCAP3, FAM26F, MGC52498, FAM70A, TMEM154 proteins and/or new variants.

Thus, in at least some embodiments, the present invention provides KRTCAP3 proteins and novel variants of a KRTCAP3 (SEQ ID NO:7) (Keratinocytes-associated protein 3), discrete portions thereof, and polynucleotides encoding same, and KRTCAP3 polypeptides and discrete portions thereof, and polynucleotides encoding same, which can be used as diagnostic markers and/or as targets for cancer therapy, treatment of immune related conditions, and drug development, and as therapeutic agents which agonize or antagonize the binding of other moieties to the KRTCAP3 proteins and/or which agonize or antagonize at least one KRTCAP3 related biological activity.

According to some embodiments the present invention provides an isolated polypeptide selected from W93943_P13 (SEQ ID NO:10), W93943_P14 (SEQ ID NO:11), W93943_P18 (SEQ ID NO:13), or a fragment or a variant thereof that possesses at least 80, 85, 90, 95, 96, 97, 98 or 99% sequence identity therewith.

According to some embodiments of the present invention there is provided an isolated polypeptide comprising a unique bridge, edge, tail or head portion of KRTCAP3 novel variants, or a homologue or a fragment thereof as well as nucleic acid sequences encoding the unique bridge, edge, tail or head portion, as well as fragments thereof and conjugates and the use thereof as therapeutics and/or for diagnostics.

According to at least some embodiments, the subject invention provides an isolated polypeptide comprising an amino acid sequence fragment of any one of the unique bridge, edge, tail or head portion, selected from the group consisting of any one of SEQ ID NO: 146, corresponding to amino acid residues 72 - 97 of W93943_P13 (SEQ ID NO:10); SEQ ID NO: 147, corresponding to amino acid residues 206 - 221 of W93943_P14 (SEQ ID NO:11), SEQ ID NO: 148 corresponding to amino acid residues 206 - 231 of W93943_P17 (SEQ ID NO:12), or a fragment or variant thereof that possesses at least 80, 85, 90, 95, 96, 97, 98 or 99% sequence identity therewith. According to at least some embodiments, the subject invention provides an isolated polypeptide having an amino acid sequence as set forth in any one of SEQ ID NOs:146-148.

According to at least some embodiments, the subject invention provides polypeptides comprising a sequence of amino acid residues corresponding to discrete portions of the KRTCAP3 proteins, including different portions of the extracellular domain corresponding to residues 42-62 of the KRTCAP3 protein sequence contained in the sequence of W93943_P2 (SEQ ID NO:7), W93943_P14 (SEQ ID NO:11), W93943_P17 (SEQ ID NO:12), and W93943_P18 (SEQ ID NO:13), or residues 115-162 KRTCAP3 protein sequence contained in the sequence of W93943_P2 (SEQ ID NO:7), W93943_P14 (SEQ ID NO:11), and W93943_P17 (SEQ ID NO:12), or residues 1-20 of the KRTCAP3 protein sequence contained in the sequence of W93943_P13 (SEQ ID NO:10), corresponding to amino acid sequence depicted in SEQ ID NO:49, or residues 77-91 of the KRTCAP3 protein sequence contained in the sequence of W93943_P13 (SEQ ID NO:10) corresponding to amino acid sequence depicted in SEQ ID NO:50, or residues 141-188 of the KRTCAP3 protein sequence contained in the sequence of W93943_P13 (SEQ ID NO:10) corresponding to amino acid sequence depicted in SEQ ID NO:48; or residues 115-171 of the KRTCAP3 protein sequence contained in the sequence of W93943_P18 (SEQ ID NO:13), corresponding to amino acid sequence depicted in SEQ ID NO:51, or a fragment, or a variant thereof possessing at least 80%, 85%, 90%, 95, 96, 97, 98 or 99% sequence identity therewith.

According to at least some embodiments, the subject invention provides KRTCAP3 amino acid sequences selected from any of the isolated polypeptides, used for rabbit immunization and specific antibodies production, having an amino acid sequence as set forth in any one of SEQ ID NOs:115, 116, or a fragment, or a variant thereof possessing at least 80%, 85%, 90%, 95, 96, 97, 98 or 99% sequence identity therewith.

According to at least some embodiments, the subject invention provides isolated nucleic acid sequences encoding any of the foregoing KRTCAP3 proteins extracellular domain polypeptides or fragments or homologous thereof.

According to at least some embodiments, the subject invention provides an isolated polynucleotide encoding a polypeptide comprising any one of the amino acid sequence, as set forth in SEQ ID NOs: 10, 11, 13, 47-51, 146-148, or a fragment or variant thereof that possesses at least 80, 85, 90, 95, 96, 97, 98 or 99% sequence identity therewith.

According to at least some embodiments, the subject invention provides an isolated polynucleotide comprising a nucleic acid having a nucleic acid sequence as set forth in any one of W93943_T5 (SEQ ID NO:3), W93943_T8 (SEQ ID NO:4), W93943_T14 (SEQ ID NO:6), or a fragment thereof or a sequence homologous thereto, that possesses at least 80, 85, 90, 95, 96, 97, 98 or 99% sequence identity therewith. According to at least some embodiments, the foregoing fragment is selected from a group comprising any one of SEQ ID NO:2, 9, 94, 193-195, or a fragment thereof, or a sequence homologous thereto. According to another embodiment, the isolated polynucleotide is at least 80, 85, 90, 95, 96, 97, 98 or 99% homologous to a nucleic acid sequence as set forth in any one of SEQ ID NOs: 2, 3, 4, 6, 9, 94, 193-195.

In at least some embodiments, the present invention provides proteins and discrete portion of hypothetical protein LOC441168 (SEQ ID NO:15) (SwissProt accession identifier NP_001010919, FAM26F) or polynucleotides encoding same, which can be used as diagnostic markers and/or as targets for cancer therapy, treatment of immune related conditions, and drug development, and as therapeutic agents which agonize or antagonize the binding of other moieties to the FAM26F proteins and/or which agonize or antagonize at least one FAM26F related biological activity.

According to at least some embodiments, the subject invention provides polypeptides comprising a sequence of amino acid residues corresponding to discrete portions of the FAM26F proteins, including different portions of the extracellular domain corresponding to residues 40-48 of sequences of T82906_P4 (SEQ ID NO:18), corresponding to amino acid sequence depicted in SEQ ID NO: 52, or residues 125-175 of sequences of T82906_P4 (SEQ ID NO:18), corresponding to amino acid sequence depicted in SEQ ID NO: 53, or residues 27-143 of sequences of T82906_P3 (SEQ ID NO:16), corresponding to amino acid sequence depicted in SEQ ID NO: 127, or fragments or variants thereof possessing at least 80%, 85%, 90%, 95, 96, 97, 98 or 99% sequence identity therewith.

According to at least some embodiments, the subject invention provides FAM26A amino acid sequences selected from any of the isolated polypeptides, used for rabbit immunization and specific antibodies production, having an amino acid sequence as set forth in any one of SEQ ID NOs: 117, 118, or a fragment, or a variant thereof possessing at least 80%, 85%, 90%, 95, 96, 97, 98 or 99% sequence identity therewith.

In at least some embodiments, the present invention provides MGC52498 proteins and novel variants of a known hypothetical protein MGC52498 (SEQ ID NO:132) (SwissProt accession identifier NP_872427; LOC348378), discrete portions thereof, and polynucleotides encoding same, and their use as diagnostic markers and/or as targets for cancer therapy, treatment of immune related conditions, and drug development, and as therapeutic agents which agonize or antagonize the binding of other moieties to the MGC52498 proteins and/or which agonize or antagonize at least one MGC52498 related biological activity.

According to some embodiments the present invention provides an isolated polypeptide selected from AA213820_P6 (SEQ ID NO:19), or a fragment or a variant thereof that possesses at least 80, 85, 90, 95, 96, 97, 98 or 99% sequence identity therewith.

According to some embodiments of the present invention there is provided an isolated polypeptide comprising a unique bridge, edge, tail or head portion of MGC52498 novel variants, or a homologue or a fragment thereof as well as nucleic acid sequences encoding the unique bridge, edge, tail or head portion, as well as fragments thereof and conjugates and the use thereof as therapeutics and/or for diagnostics.

According to at least some embodiments, the subject invention provides an isolated polypeptide comprising an amino acid sequence of the unique head portion of AA213820_P6 (SEQ ID NO:19), corresponding to amino acid residues 1 - 64 of AA213820_P6 (SEQ ID NO:19), as set forth in SEQ ID NO: 25, or a fragment or variant thereof that possesses at least 80, 85, 90, 95, 96, 97, 98 or 99% sequence identity therewith.

According to at least some embodiments, the subject invention provides an isolated polypeptide having an amino acid sequence as set forth in SEQ ID NO: 25.

According to at least some embodiments, the subject invention provides polypeptides comprising a sequence of amino acid residues corresponding to discrete portions of the MGC52498 proteins, including different portions of the extracellular domain corresponding to residues 1-55 of the sequence AA213820_P4 (SEQ ID NO:135), corresponding to amino acid sequence depicted in SEQ ID NO:60, or residues 91-190 of the sequences AA213820_P4 (SEQ ID NO:135), corresponding to amino acid sequence depicted in SEQ ID NO:61, or residues 1-71 of the sequences AA213820_P6 (SEQ ID NO:19), corresponding to amino acid sequence depicted in SEQ ID NO:62, or fragments or variants thereof possessing at least 80%, 85%, 90%, 95, 96, 97, 98 or 99% sequence identity therewith.

According to at least some embodiments, the subject invention provides polypeptides comprising a sequence of amino acid residues corresponding to discrete fragments of MGC52498, selected from the group consisting of SEQ ID NOs: 150-154, 200, or fragments or variants thereof possessing at least 80%, 85%, 90%, 95, 96, 97, 98 or 99% sequence identity therewith.

According to at least some embodiments, the subject invention provides isolated nucleic acid sequences encoding any of the foregoing MGC52498 proteins extracellular domain polypeptides or fragments or homologous thereof.

According to at least some embodiments, the subject invention provides an isolated polynucleotide encoding a polypeptide comprising any one of the amino acid sequence, as set forth in SEQ ID NOs: 19, 25, 60, 61, 62, 150-154, 200, or a fragment or variant thereof that possesses at least 80, 85, 90, 95, 96, 97, 98 or 99% sequence identity therewith.

According to at least some embodiments, the subject invention provides an isolated polynucleotide comprising a nucleic acid as set forth in SEQ ID NO:20, or a fragment thereof or a sequence homologous thereto, that possesses at least 80, 85, 90, 95, 96, 97, 98 or 99% sequence identity therewith. According to at least some embodiments, the subject invention further provides an isolated polynucleotide comprising a nucleic acid sequenceselected from a group comprising any one of SEQ ID NOs: 27, 109, 201, or a fragment thereof or a sequence homologous thereto, that possesses at least 80, 85, 90, 95, 96, 97, 98 or 99% sequence identity therewith.

In at least some embodiments, the present invention provides FAM70A proteins and novel variants of a known hypothetical protein FAM70A (SEQ ID NO:29) (SwissProt accession identifier NP_060408), discrete portions thereof, and polynucleotides encoding same, and polynucleotides encoding same, which can be used as diagnostic markers and/or as targets for cancer therapy, treatment of immune related conditions, and drug development, and therapeutic agents which agonize or antagonize the binding of other moieties to the FAM70A proteins and/or which agonize or antagonize at least one FAM70A related biological activity.

According to some embodiments the present invention provides an isolated polypeptide selected from anyone of F10649_P7 (SEQ ID NO:35), F10649_P8 (SEQ ID NO:36), or a fragment or a variant thereof that possesses at least 80, 85, 90, 95, 96, 97, 98 or 99%sequence identity therewith.

According to some embodiments of the present invention there is provided an isolated polypeptide comprising a unique bridge, edge, tail or head portion of FAM70A variants, or a homologue or a fragment thereof as well as nucleic acid sequences encoding the unique bridge, edge, or head portion, as well as fragments thereof and conjugates and the use thereof as therapeutics and/or for diagnostics.

According to at least some embodiments, the subject invention provides an isolated polypeptide comprising an amino acid sequence of anyone of the unique bridge, edge, or head portion corresponding to amino acid residues 1-141 of F10649_P5 (SEQ ID NO:33), as set forth in SEQ ID NO: 156; or corresponding to amino acid residues 1-144 of F10649_P8 (SEQ ID NO:36), as set forth in SEQ ID NO: 159; or corresponding to amino acid sequences set forth in any one of SEQ ID NOs: 155, 157, 158, 160, 196, 199, or a fragment or variant thereof that possesses at least 80, 85, 90, 95, 96, 97, 98 or 99% sequence identity therewith.

According to at least some embodiments, the subject invention provides an isolated polypeptide having an amino acid sequence as set forth in any one of SEQ ID NOs: 155-160, 196, 199.

According to at least some embodiments, the subject invention provides FAM70A amino acid sequences selected from any of the isolated polypeptides, used for rabbit immunization and specific antibodies production, having an amino acid sequence as set forth in any one of SEQ ID NOs: 121, 186, or a fragment, or a variant thereof possessing at least 80%, 85%, 90%, 95, 96, 97, 98 or 99% sequence identity therewith.

According to at least some embodiments, the subject invention provides polypeptides comprising a sequence of amino acid residues corresponding to discrete portions of the FAM70A proteins, including different portions of the extracellular domain corresponding to residues 51-59 of the sequence F10649_P4 (SEQ ID NO:30), F10649_P5 (SEQ ID NO:33), or F10649_P7 (SEQ ID NO:35), corresponding to amino acid sequence depicted in SEQ ID NO:54, or residues 110-225 of the sequence F10649_P4 (SEQ ID NO:30),, corresponding to amino acid sequence depicted in SEQ ID NO:55, or residues 110-201 of the sequence F10649_P5 (SEQ ID NO:33), corresponding to amino acid sequence depicted in SEQ ID NO:56, or residues 110-241 of the sequence F10649_P7 (SEQ ID NO:35), corresponding to amino acid sequence depicted in SEQ ID NO:58, or residues 51-65 of the sequence F10649_P8 (SEQ ID NO:36), corresponding to amino acid sequence depicted in SEQ ID NO:59, or residues 223-328 of the sequence F10649_P8 (SEQ ID NO:36), or residues 80-185 of the sequence F10649_P10 (SEQ ID NO:32), corresponding to amino acid sequence depicted in SEQ ID NO:57, or variants thereof possessing at least 80%, 85%, 90%, 95, 96, 97, 98 or 99% sequence identity therewith.

According to at least some embodiments, the subject invention provides isolated nucleic acid sequences encoding any of the foregoing FAM70A proteins extracellular domain polypeptides or fragments or homologous thereof.

According to at least some embodiments, the subject invention provides an isolated polynucleotide encoding a polypeptide comprising any one of the amino acid sequence, as set forth in SEQ ID NOs: 35, 36, 54-58, 121, 155-160, 186, 196, 199, or a fragment or variant thereof that possesses at least 80, 85, 90, 95, 96, 97, 98 or 99% sequence identity therewith.

According to at least some embodiments, the subject invention provides an isolated polynucleotide comprising a nucleic acid having a nucleic acid sequence as set forth in anyone of F10649_T4 (SEQ ID NO:24), F10649_T6 (SEQ ID NO:26), or a fragment thereof or a sequence at least 80, 85, 90, 95, 96, 97, 98 or 99% homologous thereto. According to at least some embodiments, the foregoing fragment comprises any of the nucleic acid as set forth in any one of SEQ ID NO:103, 197, 198, or a fragment thereof, or a sequence at least 80, 85, 90, 95, 96, 97, 98 or 99% homologous thereto.

In at least some embodiments, the present invention provides proteins and discrete portions of a known hypothetical protein LOC201799 (SEQ ID NO:42) (SwissProt accession identifier NP_689893; TMEM154) or polynucleotides encoding same, which can be used as diagnostic markers and/or as targets for cancer therapy, treatment of immune related conditions, and drug development, and therapeutic agents which agonize or antagonize the binding of other moieties to the TMEM154 proteins and/or which agonize or antagonize at least one TMEM154 related biological activity.

According to at least some embodiments, the subject invention provides isolated polypeptides comprising the soluble ectodomain (ECD) of the TMEM154 proteins and fragments and conjugates thereof, as well as nucleic acid sequences encoding said soluble ectodomain, and the use thereof as therapeutics.

According to at least some embodiments, the subject invention provides polypeptides comprising a sequence of amino acid residues corresponding to discrete portions of the TMEM154 proteins, including different portions of the extracellular domain corresponding to residues 23-75 of the sequence W38346_P3 (SEQ ID NO:42) or W38346_P7 (SEQ ID NO:46), corresponding to amino acid sequence depicted in SEQ ID NO:63, or residues 20-105 of the sequence W38346_P4 (SEQ ID NO:45), corresponding to amino acid sequence depicted in SEQ ID NO:64, or residues 122 - 144 of the sequence of W38346_P7 (SEQ ID NO:46), corresponding to amino acids depicted in SEQ ID NO:162, or fragments thereof or variants thereof possessing at least 80%, 85%, 90%, 95, 96, 97, 98 or 99% sequence identity therewith.

According to at least some embodiments, the subject invention provides TMEM154 amino acid sequences selected from any of the isolated polypeptides, used for rabbit immunization and specific antibodies production, having an amino acid sequence as set forth in any one of SEQ ID NOs:191, 192, or a fragment, or a variant thereof possessing at least 80%, 85%, 90%, 95, 96, 97, 98 or 99% sequence identity therewith.

According to at least some embodiments, the subject invention provides isolated nucleic acid sequences encoding any of the foregoing TMEM154 proteins extracellular domain polypeptides or fragments or homologous thereof.

According to at least some embodiments, the subject invention provides an isolated polynucleotide encoding a polypeptide comprising any one of the amino acid sequence, as set forth in SEQ ID NOs: 63, 64, 161, 162, or a fragment or variant thereof that possesses at least 80, 85, 90, 95, 96, 97, 98 or 99% sequence identity therewith.

According to at least some embodiments, the subject invention provides an isolated polynucleotide comprising a nucleic acid as set forth in any one of SEQ ID NO:23, 106 or a fragment thereof, or a sequence at least 80, 85, 90, 95, 96, 97, 98 or 99% homologous thereto. According to another embodiment, the isolated polynucleotide is at least 80, 85, 90, 95, 96, 97, 98 or 99% homologous to a nucleic acid sequence as set forth in any one of SEQ ID NOs: 23, 106.

According to at least some embodiments, the subject invention provides any of the foregoing polypeptides corresponding to any one of the soluble TMEM154 proteins and/or TMEM154 protein's extracellular domains, wherein said polypeptide blocks or inhibits the interaction of TMEM154 proteins with a corresponding functional ligand.

According to at least some embodiments, the subject invention provides any of the foregoing polypeptides corresponding to any one of the soluble TMEM154 proteins and/or TMEM154 proteins extracellular domains, wherein said polypeptide replaces or augments the interaction of TMEM154 proteins with a corresponding functional ligand.

According to some embodiments of the present invention there is provided a fusion protein, or a nucleic acid encoding same, comprising an isolated or purified TMEM154 proteins and/or TMEM154 proteins extracellular domain or fragments or variants or homologs thereof. According to some embodiments of the present invention, the fusion protein, or a nucleic acid encoding same, optionally may be directly or indirectly attached to a non-TMEM154 protein or nucleic acid sequence, respectively.

According to some embodiments of the present invention the non-TMEM154 protein or nucleic acid sequence is at least a portion of soluble immunoglobulin domain or fragment.

In another embodiment the invention includes any of the foregoing fusion proteins, wherein a polyalkyl oxide moiety such as polyethylene glycol is attached to the polypeptide.

In another embodiment the invention includes any of the foregoing fusion proteins, wherein the immunoglobulin heavy chain constant region is an Fc fragment.

In another embodiment the invention includes any of the foregoing fusion proteins wherein the immunoglobulin heavy chain constant region is an isotype selected from the group consisting of an IgG1, IgG2, IgG3, IgG4, IgM, IgE, IgA and IgD.

In another embodiment the invention includes any of the foregoing fusion proteins, wherein the polypeptide is fused to a VASP domain.

In another embodiment the invention includes any of the foregoing fusion proteins, wherein the fusion protein modulates lymphocyte activation.

In another embodiment the invention includes any of the foregoing polypeptides, attached to a detectable or therapeutic moiety.

According to some embodiments of the present invention there is provided vectors such as plasmids and recombinant viral vectors containing any of the foregoing nucleic acid sequences, and host cells containing the vectors that express any one of discrete portions of KRTCAP3, FAM26F, MGC52498, FAM70A, TMEM154 proteins, its secreted or soluble form and/or the ECD or sequences corresponding to unique bridge, edge, tail or head portion of KRTCAP3, FAM26F, MGC52498, FAM70A, TMEM154 proteins, or homologous thereof or conjugates containing any of the foregoing.

According to still other embodiments there is provided use of any of the forgoing vectors and host cells for producing any one of the KRTCAP3, FAM26F, MGC52498, FAM70A, TMEM154 polypeptides.

In another embodiment the invention includes a method of producing any one of the KRTCAP3, FAM26F, MGC52498, FAM70A, TMEM154 ectodomain polypeptides, sequences corresponding to a unique bridge, edge, tail or head portion of KRTCAP3, FAM26F, MGC52498, FAM70A, TMEM154 polypeptides, or a fragment or a homolog or a conjugate thereof, comprising culturing the foregoing host cell, wherein the cell expresses the polypeptide encoded by the DNA segment or nucleic acid and recovering said polypeptide.

According to another embodiment of the invention, the KRTCAP3, FAM26F, MGC52498, FAM70A, TMEM154 polypeptides, or fragments or homologs thereof, can be produced using any one of biochemically synthesis method known in the art, such as by employing standard solid phase techniques.

In another embodiment the invention includes a pharmaceutical composition comprising any of the foregoing polynucleotide sequences and further comprising a pharmaceutically acceptable diluent or carrier.

In another embodiment the invention includes a pharmaceutical composition comprising the foregoing vector or host cell and further comprising a pharmaceutically acceptable diluent or carrier.

In another embodiment the invention includes a pharmaceutical composition comprising any of the foregoing polypeptides and/or any of the foregoing fusion proteins and further comprising a pharmaceutically acceptable diluent or carrier.

According to some embodiments of the present invention there is provided compounds and use thereof including TMEM154 ectodomain or fragments or variants thereof, and a pharmaceutical composition comprising same, which are suitable for treatment or prevention of cancer and/or immune related conditions.

According to some embodiments of the present invention there is provided a method for treating, or preventing cancer, and/or immune related conditions, comprising administering a subject in need thereof a foregoing pharmaceutical composition, comprising any one of: a molecule having the extracellular domain of TMEM154 polypeptide, or fragment or variant or homologue or conjugate thereof; or polypeptide, comprising a sequence of amino acid residues having at least 80%, 85%, 90%, 95%, 96%, 97%, 98%, 99%, 100% sequence identity with amino acid set forth in SEQ ID NOs:63, 64, or a fusion protein comprising polypeptide having the extracellular domain of TMEM154 polypeptide.

According to at least some embodiments of the present invention there is provided any of the foregoing methods for treating, or preventing cancer, wherein the cancer is selected from the group consisting of solid tumors, sarcomas, hematological malignancies, including but not limited to breast cancer (e.g. breast carcinoma), cervical cancer, ovary cancer (ovary carcinoma), endometrial cancer, melanoma, bladder cancer (bladder carcinoma), lung cancer (e.g. adenocarcinoma and non-small cell lung cancer), pancreatic cancer (e.g. pancreatic carcinoma such as exocrine pancreatic carcinoma), colon cancer (e.g. colorectal carcinoma, such as colon adenocarcinoma and colon adenoma), prostate cancer including the advanced disease, hematopoietic tumors of lymphoid lineage (e.g. leukemia, acute lymphocytic leukemia, chronic lymphocytic leukemia, B-cell lymphoma, Burkitt's lymphoma, multiple myeloma, Hodgkin's lymphoma, Non-Hodgkin's lymphoma, anti CD20 (i.e. Rituximab) resistant lymphoma), myeloid leukemia (for example, acute myelogenous leukemia (AML), chronic myelogenous leukemia), thyroid cancer, thyroid follicular cancer, myelodysplastic syndrome (MDS), tumors of mesenchymal origin (e.g. fibrosarcomas and rhabdomyosarcomas), melanoma, uveal melanoma, teratocarcinoma, neuroblastoma, glioma, glioblastoma, benign tumor of the skin (e.g. keratoacanthomas), renal cancer, anaplastic large-cell lymphoma, esophageal squamous cells carcinoma, hepatocellular carcinoma, follicular dendritic cell carcinoma, intestinal cancer, muscle-invasive cancer, seminal vesicle tumor, epidermal carcinoma, spleen cancer, bladder cancer, head and neck cancer, stomach cancer, liver cancer, bone cancer, brain cancer, cancer of the retina, biliary cancer, small bowel cancer, salivary gland cancer, cancer of uterus, cancer of testicles, cancer of connective tissue, prostatic hypertrophy, myelodysplasia, Waldenstrom's macroglobinaemia, nasopharyngeal, neuroendocrine cancer, myelodysplastic syndrome, mesothelioma, angiosarcoma, Kaposi's sarcoma, carcinoid, oesophagogastric, fallopian tube cancer, peritoneal cancer, papillary serous mullerian cancer, malignant ascites, gastrointestinal stromal tumor (GIST), and a hereditary cancer syndrome such as Li-Fraumeni syndrome and Von Hippel-Lindau syndrome (VHL), and wherein the cancer is non-metastatic, invasive or metastatic.

According to at least some embodiments of the present invention there is provided any of the foregoing methods for treating, or preventing cancer, comprising administering a subject in need thereof a foregoing pharmaceutical composition, comprising any one of: a soluble molecule having the extracellular domain the TMEM154 polypeptides, or fragment or variant or homologue or conjugate thereof; or polypeptide, comprising a sequence of amino acid residues having at least 80%, 85%, 90%, 95%, 96%, 97%, 98%, 99%, 100% sequence identity with amino acid sequence as set forth in anyone of SEQ ID NOs:63, 64, or fusion protein, or a nucleic acid sequence encoding the same, or the expression vector containing the nucleic acid sequences, or host cell comprising the foregoing expression vector, wherein the cancer is selected from lymphoma, especially Non-Hodgkin's Lymphoma, , anti CD20 (i.e. Rituximab) resistant lymphoma, Multiple Myeloma, kidney cancer, and/or pancreatic cancer.

According to at least some embodiments of the present invention there is provided any of the foregoing methods for treating, or preventing immune related condition, disease or disorder, wherein the immune related condition, disease or disorder is selected from a group consisting of but not limited to multiple sclerosis; psoriasis; rheumatoid arthritis; psoriatic arthritis, systemic lupus erythematosus; ulcerative colitis; Crohn's disease; benign lymphocytic angiitis, thrombocytopenic purpura, idiopathic thrombocytopenia, idiopathic autoimmune hemolytic anemia, pure red cell aplasia, Sjogren's syndrome, rheumatic disease, connective tissue disease, inflammatory rheumatism, degenerative rheumatism, extra-articular rheumatism, juvenile rheumatoid arthritis, arthritis uratica, muscular rheumatism, chronic polyarthritis, cryoglobulinemic vasculitis, ANCA-associated vasculitis, antiphospholipid syndrome, myasthenia gravis, autoimmune haemolytic anaemia, Guillian-Barre syndrome, chronic immune polyneuropathy, autoimmune thyroiditis, insulin dependent diabetes mellitus, type I diabetes, Addison's disease, membranous glomerulonephropathy, Goodpasture's disease, autoimmune gastritis, pernicious anaemia, pemphigus vulgarus, cirrhosis, primary biliary cirrhosis, dermatomyositis, polymyositis, fibromyositis, myogelosis, celiac disease, immunoglobulin A nephropathy, Henoch-Schonlein purpura, Evans syndrome, atopic dermatitis, psoriasis, psoriasis arthropathica, Graves' disease, Graves' ophthalmopathy, scleroderma, systemic scleroderma, asthma, allergy, primary biliary cirrhosis, Hashimoto's thyroiditis, primary myxedema, sympathetic ophthalmia, autoimmune uveitis, hepatitis, chronic action hepatitis, collagen diseases, ankylosing spondylitis, periarthritis humeroscapularis, panarteritis nodosa, chondrocalcinosis, Wegener's granulomatosis, microscopic polyangiitis, chronic urticaria, bullous skin disorders, pemphigoid, atopic eczema, Devic's disease, childhood autoimmune hemolytic anemia, Refractory or chronic Autoimmune Cytopenias, Prevention of development of Autoimmune Anti-Factor VIII Antibodies in Acquired Hemophilia A, Cold Agglutinin Disease, Neuromyelitis Optica, Stiff Person Syndrome, gingivitis, periodontitis, pancreatitis, myocarditis, vasculitis, gastritis, gout, gouty arthritis, and inflammatory skin disorders, selected from the group consisting of psoriasis, atopic dermatitis, eczema, rosacea, urticaria, and acne, normocomplementemic urticarial vasculitis, pericarditis, myositis, anti-synthetase syndrome, scleritis, macrophage activation syndrome, Bechet's Syndrome, PAPA Syndrome, Blau's Syndrome, gout, adult and juvenile Still's disease, cryropyrinopathy, Muckle-Wells syndrome, familial cold-induced auto-inflammatory syndrome, neonatal onset multisystemic inflammatory disease, familial Mediterranean fever, chronic infantile neurologic, cutaneous and articular syndrome, systemic juvenile idiopathic arthritis, Hyper IgD syndrome, Schnitzler's syndrome, and TNF receptor-associated periodic syndrome (TRAPS), immune disorders associated with graft transplantation rejection, such as acute and chronic rejection of organ transplantation, allogenic stem cell transplantation, autologous stem cell transplantation, bone marrow tranplantation, graft versus host disease, inflammatory bowel disease, Good pasture's syndrome, pernicious anemia, autoimmune atrophic gastritis, ulceratis colitis, mixed connective tissue disease, panarteriitis nodosa, progressive systemic scleroderma, peptic ulcers, ulcers, chronic bronchitis, acute lung injury, pulmonary inflammation, airway hyper-responsiveness, septic shock, inflammatory skin disorders, myogelosis, chondrocalcinosis, thyroditis, allergic oedema, and granulomas.

According to other embodiments of the present invention, there is provided monoclonal or polyclonal antibodies and antibody fragments and conjugates containing such, that specifically bind any one of KRTCAP3, FAM26F, MGC52498, FAM70A, TMEM154 proteins, optionally and preferably by specifically binding a sequence selected from the group consisting of any of SEQ ID NOs: 7, 8, 10-13, 15-19, 29-33, 35, 36, 42-46, 127, 132-135, or a fragment, or a variant, or a homologue thereof, or a unique bridge, edge, tail or head portion selected from any one of SEQ ID NOs:25, 146-162, 196, 199, 200, or a fragment, or a variant, or a homologue, or an epitope thereof, or a secreted form and/or the ECD thereof selected from SEQ ID NO:47-64 or a fragment, or a variant, or a homologue thereof, or a peptide selected from any one of SEQ ID NOs: 115-118, 121, 186, 191, 192. These antibodies are potentially useful as therapeutics and/or diagnostic agents (both in vitro and in vivo diagnostic methods).

According to at least some embodiments of the invention these antibodies are useful for generating and selecting for anti-idiotypic antibodies specific thereto which also are potentially useful as therapeutics and/or diagnostic agents (both in vitro and in vivo diagnostic methods).

According to at least some embodiments of the invention, the antibodies and fragments modulate the activity elicited by KRTCAP3, FAM26F, MGC52498, FAM70A, or TMEM154 polypeptides, and/or are immune activating or immune suppressing such as antibodies or fragments that target cells via ADCC (antibody dependent cellular cytotoxicity) or CDC (complement dependent cytotoxicity) activities.

In another embodiment the invention includes any of the foregoing antibodies or fragments thereof, wherein said antibody blocks or inhibits the interaction of any one of KRTCAP3, FAM26F, MGC52498, FAM70A, or TMEM154 polypeptides with a corresponding counterpart or cell component or tissue structure promoting an opposite activity or function.

In another embodiment the invention includes any of the foregoing antibodies or fragments wherein said antibody replaces or augments the interaction of any one of KRTCAP3, FAM26F, MGC52498, FAM70A, or TMEM154 polypeptides with a corresponding counterpart or cell component or tissue structure promoting an opposite function or activity.

According to at least some embodiments of the present invention, there is provided pharmaceutical and diagnostic compositions that comprise a therapeutically or diagnostically effective form of any of the foregoing antibody or antibody fragment.

According to at least some embodiments of the present invention, there is provided pharmaceutical compositions that comprise a therapeutically effective form of any of the foregoing antibody or antibody fragment and further comprising a pharmaceutically acceptable dilulent or carrier.

According to at least some embodiments of the present invention, there is provided any of the foregoing therapeutically effective polyclonal or monoclonal antibodies or fragments, or anti-idiotypic antibodies specific to any of the foregoing, or a pharmaceutical composition comprising same, for treating or preventing conditions wherein any one of KRTCAP3, FAM26F, MGC52498, FAM70A, or TMEM154 polypeptides, or its secreted or soluble form or ECD and/or fragments or variants or homologs thereof are differentially expressed, including cancer and immune related conditions.

According to at least some embodiments of the present invention, there is provided any of the foregoing therapeutically effective polyclonal or monoclonal antibodies or fragments, or anti-idiotypic antibodies, or a pharmaceutical composition comprising same, specific to any KRTCAP3 polypeptide, selected from a group consisting of any of SEQ ID NOs: 7, 8, 10-13, 47-51, 146-148, 115, 116, and/or fragments or variants or homologs thereof, for treating cancer. According to at least some embodiments of the present invention, there is provided any of the foregoing therapeutically effective polyclonal or monoclonal antibodies or fragments, or anti-idiotypic antibodies, or a pharmaceutical composition comprising same, specific to any KRTCAP3 polypeptide, for treating ovarian cancer, lung cancer, breast cancer and/or colon cancer.

According to at least some embodiments of the present invention, there is provided any of the foregoing therapeutically effective polyclonal or monoclonal antibodies or fragments, or anti-idiotypic antibodies, or a pharmaceutical composition comprising same, specific to any of one of FAM26F proteins, selected from a group consisting of any of SEQ ID NOs: 15-18, 52, 53, 127, 149, 117, 118, and/or fragments or variants or homologs thereof, for treating cancer and/or immune related conditions or disorders. According to at least some embodiments of the present invention, there is provided any of the foregoing therapeutically effective polyclonal or monoclonal antibodies or fragments, or anti-idiotypic antibodies, or a pharmaceutical composition comprising same, specific to any of one of FAM26F proteins, for treating ovarian cancer, breast cancer, prostate cancer, renal cancer, melanoma, acute lymphocytic leukemia, chronic lymphocytic leukemia, acute myelogenous leukemia, chronic myelogenous leukemia, multiple myeloma, Hodgkin's lymphoma or Non-Hodgkin's lymphoma, and/or immune related conditions or disorders.

According to at least some embodiments of the present invention, there is provided any of the foregoing therapeutically effective polyclonal or monoclonal antibodies or fragments, or anti-idiotypic antibodies, or a pharmaceutical composition comprising same, specific to any of one of MGC52498 proteins, selected from a group consisting of any of SEQ ID NOs: 19, 25, 60-62, 132-135, 150-154, 200 and/or fragments or variants or homologs thereof, for treating cancer and/or immune related conditions or disorders. According to at least some embodiments of the present invention, there is provided any of the foregoing therapeutically effective polyclonal or monoclonal antibodies or fragments, or anti-idiotypic antibodies, or a pharmaceutical composition comprising same, specific to any of one of MGC52498 proteins for treating lymphoma, especially Non-Hodgkin's Lymphoma, Multiple Myeloma, leukemia, especially T cell leukemia, and/or lung cancer, and/or immune related conditions or disorders.

According to at least some embodiments of the present invention, there is provided any of the foregoing therapeutically effective polyclonal or monoclonal antibodies or fragments, or anti-idiotypic antibodies, or a pharmaceutical composition comprising same, specific to any of one of FAM70A proteins, selected from a group consisting of any of SEQ ID NOs: 29-33, 35, 36, 54-59, 155-160, 121, 186, 196, 199, and/or fragments or variants or homologs thereof, for treating cancer and/or immune related conditions or disorders. According to at least some embodiments of the present invention, there is provided any of the foregoing therapeutically effective polyclonal or monoclonal antibodies or fragments, or anti-idiotypic antibodies, or a pharmaceutical composition comprising same, specific to any of one of FAM70A proteins for treating Multiple Myeloma, kidney cancer, lung cancer, liver cancer, and/or breast cancer, and/or immune related conditions or disorders.

According to at least some embodiments of the present invention, there is provided any of the foregoing therapeutically effective polyclonal or monoclonal antibodies or fragments, or anti-idiotypic antibodies, or a pharmaceutical composition comprising same, specific to any of one of TMEM154 proteins, selected from a group consisting of any of SEQ ID NOs: 42-46, 63, 64, 161, 162, 191, 192, and/or fragments or variants or homologs thereof, for treating cancer and/or immune related conditions or disorders. According to at least some embodiments of the present invention, there is provided any of the foregoing therapeutically effective polyclonal or monoclonal antibodies or fragments, or anti-idiotypic antibodies, or a pharmaceutical composition comprising same, specific to any of one of TMEM154 proteins for treating lymphoma, especially Non-Hodgkin's Lymphoma, , anti CD20 (i.e. Rituximab) resistant lymphoma, Multiple Myeloma, kidney cancer, and/or pancreatic cancer, and/or immune related conditions or disorders, especially SLE (systemic lupus erythematosus).

According to still other embodiments there is provided a use of any of the foregoing specific antibodies and antibody fragments, and conjugates thereof, and pharmaceutical composition comprising same, in modulating (enhancing or inhibiting) immunity.

According to still other embodiments there is provided antibodies and antibody fragments specific to polypeptides comprising discrete portions of KRTCAP3 proteins, including different portions of the extracellular domain corresponding to residues 42-62 of the KRTCAP3 protein sequence contained in the sequence of W93943_P2 (SEQ ID NO:7), W93943_P14 (SEQ ID NO:11), W93943_P17 (SEQ ID NO:12), and W93943_P18 (SEQ ID NO:13), or residues 115-162 KRTCAP3 protein sequence contained in the sequence of W93943_P2 (SEQ ID NO:7), W93943_P14 (SEQ ID NO:11), and W93943_P17 (SEQ ID NO:12), or residues 1-20 of the KRTCAP3 protein sequence contained in the sequence of W93943_P13 (SEQ ID NO:10), corresponding to amino acid sequence depicted in SEQ ID NO:49, or residues 77-91 of the KRTCAP3 protein sequence contained in the sequence of W93943_P13 (SEQ ID NO:10) corresponding to amino acid sequence depicted in SEQ ID NO:50, or residues 141-188 of the KRTCAP3 protein sequence contained in the sequence of W93943_P13 (SEQ ID NO:10) corresponding to amino acid sequence depicted in SEQ ID NO:48; or residues 115-171 of the KRTCAP3 protein sequence contained in the sequence of W93943_P18 (SEQ ID NO:13), corresponding to amino acid sequence depicted in SEQ ID NO:51, or to polypeptides comprising anyone of the unique bridge, edge portion, tail or head portion comprising any one of the amino acid sequences set forth in SEQ ID NOs: 146-147, or fragments thereof.

According to still other embodiments of the present invention there are provided antibodies and antibody fragments specific to polypeptides comprising discrete portions of FAM26F proteins including different portions of the extracellular domain corresponding to residues 40-48 of sequences of T82906_P4 (SEQ ID NO:18), corresponding to amino acid sequence depicted in SEQ ID NO: 52, or residues 125-175 of sequences of T82906_P4 (SEQ ID NO:18), corresponding to amino acid sequence depicted in SEQ ID NO: 53, or residues 27-143 of sequences of T82906_P3 (SEQ ID NO:16), corresponding to amino acid sequence depicted in SEQ ID NO: 127, or to polypeptides comprising anyone of the unique bridge, edge portion, tail or head portion comprising any one of the amino acid sequences set forth in SEQ ID NO:49, or fragments thereof.

According to still other embodiments of the present invention there are provided antibodies and antibody fragments specific to polypeptides comprising discrete portions of MGC52498 proteins including different portions of the extracellular domain corresponding to residues 1-55 of the sequence AA213820_P4 (SEQ ID NO:135), corresponding to amino acid sequence depicted in SEQ ID NO:60, or residues 91-190 of the sequences AA213820_P4 (SEQ ID NO:135), corresponding to amino acid sequence depicted in SEQ ID NO:61, or residues 1-71 of the sequences AA213820_P6 (SEQ ID NO:19), corresponding to amino acid sequence depicted in SEQ ID NO:62, or to polypeptides comprising anyone of the unique bridge, edge portion, tail or head portion comprising any one of the amino acid sequences set forth in SEQ ID NOs:25, 150-154, or fragments thereof.

According to still other embodiments of the present invention there are provided antibodies and antibody fragments specific to polypeptides comprising discrete portions of FAM70A proteins including different portions of the extracellular domain corresponding to residues 51-59 of the sequence F10649_P4 (SEQ ID NO:30), F10649_P5 (SEQ ID NO:33), or F10649_P7 (SEQ ID NO:35), corresponding to amino acid sequence depicted in SEQ ID NO:54, or residues 110-225 of the sequence F10649_P4 (SEQ ID NO:30), corresponding to amino acid sequence depicted in SEQ ID NO:55, or residues 110-201 of the sequence F10649_P5 (SEQ ID NO:33), corresponding to amino acid sequence depicted in SEQ ID NO:56, or residues 110-241 of the sequence F10649_P7 (SEQ ID NO:35), corresponding to amino acid sequence depicted in SEQ ID NO:58, or residues 51-65 of the sequence F10649_P8 (SEQ ID NO:36), corresponding to amino acid sequence depicted in SEQ ID NO:59, or residues 223-328 of the sequence F10649_P8 (SEQ ID NO:36), or residues 80-185 of the sequence F10649_P10 (SEQ ID NO:32), corresponding to amino acid sequence depicted in SEQ ID NO:57, or to polypeptides comprising anyone of the unique bridge, edge portion, tail or head portion comprising any one of the amino acid sequences set forth in SEQ ID NOs:155-160, or fragments thereof.

According to still other embodiments of the present invention there are provided antibodies and antibody fragments specific to polypeptides comprising discrete portions of TMEM154 proteins including different portions of the extracellular domain corresponding to residues 23-75 of the sequence W38346_P3 (SEQ ID NO:42) or W38346_P7 (SEQ ID NO:46), corresponding to amino acid sequence depicted in SEQ ID NO:63, or residues 20-105 of the sequence W38346_P4 (SEQ ID NO:45), corresponding to amino acid sequence depicted in SEQ ID NO:64, or to polypeptides comprising anyone of the unique bridge, edge portion, tail or head portion comprising any one of the amino acid sequences set forth in SEQ ID NOs:161-162, or fragments thereof.

According to still other embodiments there is provided a method to produce or select for anti-idiotypic antibodies specific to any of the foregoing.

According to still other embodiments there is provided a method to use any of the foregoing therapeutically effective polyclonal or monoclonal antibodies or fragments, or anti-idiotypic antibodies, or a pharmaceutical composition comprising same, for treatment or prevention of cancer, and/or immune related conditions.

According to at least some embodiments of the present invention there are provided methods for treating, or preventing cancer, and/or immune related conditions, comprising administering to a patient an effective amount of a foregoing antibody or fragment or a variant or a conjugate thereof, or a pharmaceutical composition comprising same.

According to at least some embodiments of the present invention there is provided any of the foregoing methods for treating, or preventing cancer, using any of the forgoing antibodies or fragments or a variant or a conjugate thereof, or a pharmaceutical composition comprising same.

According to at least some embodiments of the present invention there is provided any of the foregoing methods for treating, or preventing cancer, using any of the forgoing antibodies or fragments or a variant or a conjugate thereof, or a pharmaceutical composition comprising same, specific to any of one of KRTCAP3 proteins, wherein the cancer is including but not limited to ovarian cancer, lung cancer, breast cancer and/or colon cancer, wherein the cancer may be non-metastatic, invasive or metastatic. According to at least some embodiments of the present invention there is provided any of the foregoing methods for treating, or preventing cancer, using any of the forgoing antibodies or fragments or a variant or a conjugate thereof, or a pharmaceutical composition comprising same, specific to any of one of the FAM26F proteins, wherein the cancer is selected from but not limited to ovarian cancer, breast cancer, prostate cancer, renal cancer, melanoma, acute lymphocytic leukemia, chronic lymphocytic leukemia, acute myelogenous leukemia, chronic myelogenous leukemia, multiple myeloma, Hodgkin's lymphoma or Non-Hodgkin's lymphoma, wherein the cancer may be non-metastatic, invasive or metastatic, as well as for treating immune related conditions or disorders including but not limited to inflammatory or autoimmune diseases, transplant rejection and graft versus host disease.

According to at least some embodiments of the present invention there is provided any of the foregoing methods for treating, or preventing cancer, and/or immune related conditions or disorders, using any of the forgoing antibodies or fragments or a variant or a conjugate thereof, or a pharmaceutical composition comprising same, specific to any of one of the MGC52498 proteins, wherein the cancer is including but not limited to lymphoma, especially Non-Hodgkin's Lymphoma, Multiple Myeloma, leukemia, especially T cell leukemia, and/or lung cancer.

According to at least some embodiments of the present invention there is provided any of the foregoing methods for treating, or preventing cancer, and/or immune related conditions or disorders, using any of the forgoing antibodies or fragments or a variant or a conjugate thereof, or a pharmaceutical composition comprising same, specific to any of one of the FAM70A proteins, wherein the cancer is including but not limited to Multiple Myeloma, kidney cancer, lung cancer, liver cancer, and/or breast cancer.

According to at least some embodiments of the present invention there is provided any of the foregoing methods for treating, or preventing cancer, using any of the forgoing antibodies or fragments or a variant or a conjugate thereof, or a pharmaceutical composition comprising same, specific to any of one of the TMEM154 proteins, or its secreted or soluble form or ECD and/or portions or variants thereof, wherein the cancer is including but not limited to lymphoma, especially Non-Hodgkin's Lymphoma, anti CD20 (i.e. Rituximab) resistant lymphoma, Multiple Myeloma, kidney cancer, and/or pancreatic cancer, and/or immune related conditions or disorders, especially SLE.

In another embodiment the invention includes a method of inducing or enhancing an immune response, comprising administering to a patient in need thereof any of the foregoing antibodies or fragments and detecting induction or enhancement of said immune response.

In another embodiment the invention includes a method for potentiating a secondary immune response to an antigen in a patient, which method comprises administering effective amounts any of the foregoing antibodies or fragments. In another embodiment the invention includes the foregoing method, wherein the antigen is preferably a cancer antigen, a viral antigen or a bacterial antigen, and the patient has optionally received treatment with an anticancer vaccine or a viral vaccine.

In another embodiment the invention includes an antibody specific to any one of the KRTCAP3; FAM26F; MGC52498; FAM70A, or TMEM154 proteins, or a fragment or variant or a homolog thereof that elicits apoptosis or lysis of cancer cells that express said protein.

In another embodiment the invention includes any of the foregoing antibodies or fragments, wherein said apoptosis or lysis activity involves CDC or ADCC activity of the antibody.

According to at least some embodiments of the present invention there is provided a method for inhibiting the growth of cells that express any one of the KRTCAP3; FAM26F; MGC52498; FAM70A, or TMEM154 proteins in a subject, comprising: administering to the subject any of the corresponding foregoing antibody or a fragment or a variant conjugate thereof, or a pharmaceutical composition comprising same.

According to at least some embodiments the present invention provides the foregoing antibodies and fragments, wherein the antibody is a chimeric, humanized, primatized, or fully human antibody.

In another embodiment the invention includes any of the foregoing antibodies or fragments, wherein the antigen binding site contains from about 3-7 contiguous or non-contiguous amino acids, more typically at least 5 contiguous or non-contiguous amino acids. These binding sites include conformational and non-conformational epitopes.

According to other embodiments of the present invention there is provided antibody fragments and conjugates thereof including but not limited to Fab, F(ab')2, Fv or scFv fragment.

It is also an embodiment of the invention to directly or indirectly attach the subject antibodies and fragments to markers and other effector moieties such as a detectable marker, or to an effector moiety.

In another embodiment the invention includes any of the foregoing antibodies or fragments, wherein the effector moiety is selected from a drug, an enzyme (antibody-directed enzyme prodrug therapy (ADEPT)), a toxin, a radionuclide, a fluorophore, a therapeutic agent, or a chemotherapeutic agent.

In another embodiment the invention includes any of the foregoing antibodies or fragments, wherein the detectable marker is a radioisotope, a metal chelator, an enzyme, a fluorescent compound, a bioluminescent compound or a chemiluminescent compound.

According to at least some embodiments of the present invention there are provided compounds, including drugs which modulate (agonize or antagonize) at least one of the KRTCAP3-related, FAM26F-related, MGC52498-related, FAM70A-related, or TMEM154-related biological activity. Such drugs include by way of example small molecules, aptamers, peptides, antibodies and fragments that bind any of the polypeptides selected from SEQ ID NOs:7, 8, 10-13, 15-19, 25, 29-33, 35, 36, 42-64, 115-118, 121, 127, 132-135, 146-162, 186, 191-192, 196, 199, 200, as well as ribozymes or antisense or siRNAs which target nucleic acid sequence or fragments or variants thereof, selected from any of SEQ ID NOs:1-6, 9, 14, 20-24, 26-28, 38-41, 94, 97, 100, 103, 106, 109, 124, 125, 131, 193-195, 197, 198, 201. These molecules may directly bind or modulate an activity elicited by any of the KRTCAP3, FAM26F, MGC52498, FAM70A, and TMEM154 proteins or DNA/RNA or portions or variants thereof or may indirectly modulate a at least one of the KRTCAP3-related, FAM26F-related, MGC52498-related, FAM70A-related, or TMEM154-related activity or binding of molecules to any of the KRTCAP3, FAM26F, MGC52498, FAM70A, and TMEM154, and portions and variants thereof such as modulating the binding of any of the KRTCAP3, FAM26F, MGC52498, FAM70A, and TMEM154 to its corresponding counter receptor or endogenous ligand, and can be useful for treatment or prevention of cancer, immune related conditions, including but not limited to inflammatory and autoimmune diseases, transplant rejection and graft versus host disease, and/or for blocking or enhancing immune costimulation mediated by the KRTCAP3, FAM26F, MGC52498, FAM70A or TMEM154 polypeptide.

According to the present invention, each one of the following: TMEM154 ectodomain, or a fragment or a variant or a homologue or a conjugate thereof, or pharmaceutical composition comprising same, and/or specific antibodies and fragments that bind the KRTCAP3, FAM26F, MGC52498, FAM70A or TMEM154 polypeptides, or pharmaceutical composition comprising same, or compounds including drugs such as small molecules, aptamers, peptides, which target KRTCAP3, FAM26F, MGC52498, FAM70A or TMEM154 polypeptides, as well as ribozymes or antisense or siRNAs which target KRTCAP3, FAM26F, MGC52498, FAM70A or TMEM154 nucleic acid sequence or fragments or variants thereof, which are useful for treatment or prevention of cancer and/or immune related conditions, optionally may be used in combination therapy with other treatment methods known in the art selected from the group consisting of radiation therapy, antibody therapy, chemotherapy, surgery, or in combination therapy with other biological agents, conventional drugs, anti-cancer agents, immunosuppressants, cytotoxic drugs, chemotherapeutic agents, or in combination with therapeutic agents targeting other complement regulatory proteins (CRPs).

According to at least some embodiments of the present invention there is provided a use of any of the foregoing KRTCAP3, FAM26F, MGC52498, FAM70A, TMEM154 polypeptides, and/or polynucleotides, and/or antibodies, for diagnosis of a disease, wherein the disease is selected from cancer and/or immune related conditions.

As used herein, the term "diagnosis of a disease" encompasses screening for a disease, diagnosing a diseases, detecting the presence or a severity of a disease, prognosis of a diseases, monitoring of disease progression and/or treatment efficacy and/or relapse of a disease, disorder or condition, as well as selecting a therapy and/or a treatment for a disease, optimization of a given therapy for a disease, monitoring the treatment of a disease, and/or predicting the suitability of a therapy for specific patients or subpopulations or determining the appropriate dosing of a therapeutic product in patients or subpopulations.

In at least some embodiments of the present invention, there is a use of any of the foregoing KRTCAP3, FAM26F, MGC52498, FAM70A, TMEM154 polypeptides, and/or polynucleotides, and/or antibodies, for diagnosis of a cancer.

In at least some embodiments of the present invention, there is a use of any of the foregoing KRTCAP3 polypeptides, and/or polynucleotides, and/or antibodies, for diagnosis of a cancer, selected from but not limited to ovarian cancer, colon cancer, lung cancer, and/or breast cancer.

In at least some embodiments of the present invention, there is a use of any of the foregoing FAM26F polypeptides, and/or polynucleotides, and/or antibodies, for diagnosis of a cancer, selected from but not limited to ovarian cancer, breast cancer, prostate cancer, renal cancer, melanoma, acute lymphocytic leukemia, chronic lymphocytic leukemia, acute myelogenous leukemia, chronic myelogenous leukemia, multiple myeloma, Hodgkin's lymphoma or Non-Hodgkin's lymphoma, as well as for diagnosis of immune related conditions.

In at least some embodiments of the present invention, there is a use of any of the foregoing MGC52498 polypeptides, and/or polynucleotides, and/or antibodies, for diagnosis of a cancer, selected from but not limited to lymphoma, especially Non-Hodgkin's Lymphoma, Multiple Myeloma, leukemia, especially T cell leukemia, and/or lung cancer, as well as for diagnosis of immune related conditions.

In at least some embodiments of the present invention, there is a use of any of the foregoing FAM70A polypeptides, and/or polynucleotides, and/or antibodies, for diagnosis of a cancer, selected from but not limited to Multiple Myeloma, kidney cancer, lung cancer, liver cancer, and/or breast cancer, as well as for diagnosis of immune related conditions.

In at least some embodiments of the present invention, there is a use of any of the foregoing TMEM154 polypeptides, and/or polynucleotides, and/or antibodies, for diagnosis of a cancer, selected from but not limited to lymphoma, especially Non-Hodgkin's Lymphoma, anti CD20 (i.e. Rituximab) resistant lymphoma, Multiple Myeloma, kidney cancer, and/or pancreatic cancer, as well as for diagnosis of immune related conditions, especially SLE.

In at least some embodiments the present invention provides diagnostic methods for diagnosis of any of the foregoing diseases, disorders or conditions, comprising the detection of a polypeptide or polynucleotide according at least some embodiments the present invention. According to at least some embodiments the present invention the expression, the level or relative changes in the expression or the level of the polypeptide or polynucleotide herald the onset, severity, or prognosis of an individual with regard to a particular disease, disorder or condition. The detection may comprise detection of the expression or level of a specific polypeptide or polynucleotide according at least some embodiments the present invention, via any means known in the art, and as described herein.

According to one embodiment, detecting the presence of the polypeptide or polynucleotide is indicative of the presence of the disease and/or its severity and/or its progress. According to another embodiment, a change in the expression and/or the level of the polynucleotide or polypeptide compared to its expression and/or level in a healthy subject or a sample obtained therefrom is indicative of the presence of the disease and/or its severity and/or its progress. According to a further embodiment, a change in the expression and/or level of the polynucleotide or polypeptide compared to its level and/or expression in said subject or in a sample obtained therefrom at earlier stage is indicative of the progress of the disease. According to still further embodiment, detecting the presence and/or relative change in the expression and/or level of the polynucleotide or polypeptide is useful for selecting a treatment and/or monitoring a treatment of the disease. According to still further embodiment, detecting the presence and/or relative change in the expression and/or level of the polynucleotide or polypeptide is useful for prediction of the suitability of a therapeutic product for specific patients or subpopulations or for determining the appropriate dosing of a therapeutic product in patients or subpopulations. According to still further embodiment, the method comprising quantitatively and/or qualitatively determining or assessing expression of the polypeptides and/or polynucleotides, whereby differences in expression from an index sample, or a sample taken from a subject prior to the initiation of the therapy, or during the course of therapy, is indicative of the efficacy, or optimal activity of the therapy.

Thus, according to at least some embodiments, the present invention provides methods for diagnosis of any of the foregoing diseases, disorders or conditions, comprising detecting in a subject or in a sample obtained from the subject any nucleic acid sequence selected from the group consisting of SEQ ID NOs: 1-6, 9, 14, 20-24, 26-28, 38-41, 94, 97, 100, 103, 106, 109, 124, 125, 131, 193-195, 197, 198, 201 or fragments or variants or homologs thereof.

In at least some embodiments the present invention provide a method for diagnosing of any of the foregoing diseases, disorders or conditions in a subject, comprising (a) obtaining a sample from the subject and (b) detecting in the sample at least one polynucleotide and/or polypeptide being a member of a SEQ ID NOs: 1-6, 9, 14, 20-24, 26-28, 38-41, 94, 97, 100, 103, 106, 109, 124, 125, 131, 193-195, 197, 198, 201, or fragments or variants or homologs thereof.

In at least some embodiments of the present invention, the methods are conducted on a whole body.

In at least some embodiments of the present invention, the methods are conducted with a sample isolated from a subject having, predisposed to, or suspected of having the disease, disorder or condition. In at least some embodiments of the present invention, the sample is a cell or tissue or a body fluid sample.

In at least some embodiments, the subject invention therefore also relates to diagnostic methods and or assays for diagnosis a disease optionally in a biological sample taken from a subject (patient), which is optionally some type of body fluid or secretion including but not limited to seminal plasma, blood, serum, urine, prostatic fluid, seminal fluid, semen, the external secretions of the skin, respiratory, intestinal, and genitourinary tracts, tears, cerebrospinal fluid, sputum, saliva, milk, peritoneal fluid, pleural fluid, cyst fluid, broncho alveolar lavage, lavage of the reproductive system and/or lavage of any other part of the body or system in the body, and stool or a tissue sample. The term may also optionally encompass samples of in vivo cell culture constituents. The sample can optionally be diluted with a suitable eluant before contacting the sample to an antibody and/or performing any other diagnostic assay.

In at least some embodiments the present invention provides a method for diagnosis of a disease in a subject, comprising detecting in the subject or in a sample obtained from said subject at least one polypeptide selected from the group consisting of any of SEQ ID NOs: 7, 8, 10-13, 15-19, 25, 29-33, 35, 36, 42-64, 127, 132-135, 146-162, 196, 199, 200, or a homologue or a fragment thereof.

According to at least some embodiments of the present invention there are provided diagnostic methods that include the use of any of the foregoing antibodies according to at least some embodiments of the present invention, by way of example in immunohistochemical assay, radioimaging assays, in-vivo imaging, positron emission tomography (PET), single photon emission computer tomography (SPECT), magnetic resonance imaging (MRI), Ultra Sound, Optical Imaging, Computer Tomography, radioimmunoassay (RIA), ELISA (enzyme-linked immunosorbent assay), slot blot, competitive binding assays, fluorimetric imaging assays, Western blot, FACS, and the like. According to at least some embodiments, the present invention includes diagnostic methods and or assays which use any of the foregoing antibodies or fragments that specifically bind any polypeptide having an amino acid sequence as set forth in any one of SEQ ID NOs: 7, 8, 10-13, 15-19, 25, 29-33, 35, 36, 42-64, 127, 132-135, 146-162, 115-118, 121, 186, 191, 192, 196, 199, 200, or a fragment or a homolog thereof.

According to some embodiments of the present invention there is provided diagnostic methods and/or assays for detecting the presence of at least one of the polypeptides selected from a group consisting of SEQ ID NO: 7, 8, 10-13, 15-19, 25, 29-33, 35, 36, 42-64, 127, 132-135, 146-162, 115-118, 121, 186, 191, 192, 196, 199, 200, or a fragment or a variant or a homolog thereof, in vitro or in vivo in a biological sample or subject, comprising contacting the sample or the subject with an antibody having specificity for at least one of polypeptides having an amino acid sequence selected from the group consisting of SEQ ID NOs: 7, 8, 10-13, 15-19, 25, 29-33, 35, 36, 42-64, 127, 132-135, 146-162, 115-118, 121, 186, 191, 192, 196, 199, 200, or a fragment or a variant or a homologue thereof, or a combination thereof, and detecting the binding of any of the forgoing polypeptides, in the sample or in a subject to said antibody.

According to some embodiments of the present invention there are provided methods for diagnosis of a disease, comprising detecting the expression and or level in a subject or in a sample obtained from the subject, of at least one of KRTCAP3, FAM26F, MGC52498, FAM70A, and TMEM154 polypeptides.

According to at least some embodiments of the present invention there are provided diagnostic methods that include the detection of at least one of KRTCAP3, FAM26F, MGC52498, FAM70A, and TMEM154 polynucleotides, selected from the group consisting of SEQ ID NOs: 1-6, 9, 14, 20-24, 26-28, 38-41, 94, 97, 100, 103, 106, 109, 124, 125, 131, 193-195, 197, 198, 201, or a fragment or a variant or a homolog thereof, by employing a NAT-based technology.

In at least some embodiments of the present invention, the NAT-based assay is selected from the group consisting of a PCR, Real-Time PCR, LCR, Self-Sustained Synthetic Reaction, Q-Beta Replicase, Cycling Probe Reaction, Branched DNA, RFLP analysis, DGGE/TGGE, Single-Strand Conformation Polymorphism, Dideoxy Fingerprinting, Microarrays, Fluorescence In Situ Hybridization or Comparative Genomic Hybridization.

In another embodiment the invention relates to any isolated polynucleotide, comprising an amplicon having a nucleic acid sequence selected from the group consisting of SEQ ID NOs:94, 97, 100, 103, 106, 109, 124, 171, or a segment having a nucleic acid sequence set forth in SEQ ID NOs: 193-195, 197, 199, 201,, or fragments or polynucleotides homologous thereto.

In another embodiment the invention relates to any primer pair, comprising a pair of isolated oligonucleotides capable of amplifying the foregoing amplicon or segment.

In another embodiment the invention relates to the primer pair, comprising a pair of isolated oligonucleotides having a sequence selected from the group consisting of SEQ ID NOs: 92-93, 95-96, 98-99, 101-102, 104-105, 107-108, 122-123, 169-170; 163-168, 172, 173, 176-181, 187-188.

According to at least some embodiments of the present invention, detecting any of the forgoing KRTCAP3, FAM26F, MGC52498, FAM70A, and TMEM154 polynucleotides comprises employing a primer pair, comprising a pair of isolated oligonucleotides capable of specifically hybridizing to at least a portion of a polynucleotide having a nucleic acid sequence as set forth in SEQ ID NOs: 1-6, 9, 14, 20-24, 26-28, 38-41, 94, 97, 100, 103, 106, 109, 124, 125, 131, 171, 193-195, 197, 199, 201, or polynucleotides homologous thereto.

According to at least some embodiments of the present invention the detection is performed using an oligonucleotide pair capable of hybridizing to at least a portion of a nucleic acid sequence at least 85%, 90%, 95%, 96%, 97%, 98%, 99% homologous to the nucleic acid sequence set forth in SEQ ID NO: 1-6, 9, 14, 20-24, 26-28, 38-41, 94, 97, 100, 103, 106, 109, 124, 125, 131, 171, 193-195, 197, 199, 201.

According to at least some embodiments of the present invention, detecting any of the forgoing KRTCAP3, FAM26F, MGC52498, FAM70A, and TMEM154 polynucleotides according to at least some embodiments of the present invention, comprises employing a primer pair, comprising a pair of isolated oligonucleotides as set forth in SEQ ID NOs: 92-93, 95-96, 98-99, 101-102, 104-105, 107-108, 122-123, 169-170, 163-168, 172, 173, 176-181, 187-188.

In at least some embodiments the present invention provides a diagnostic kit for diagnosis of a disease, comprising markers and reagents for detecting qualitative and/or quantitative changes in the expression of a polypeptide or a polynucleotide according to at least some embodiments of the present invention.

In at least some embodiments of the present invention, the kit comprises markers and reagents for detecting the changes by employing a NAT-based technology.

In at least some embodiments of the present invention, the kit comprises at least one nucleotide probe or primer. In at least some embodiments of the present invention, the kit comprises at least one primer pair capable of selectively hybridizing to a nucleic acid sequence according to the teaching of the present invention. In at least some embodiments of the present invention, the kit comprises at least one oligonucleotide capable of selectively hybridizing to a nucleic acid sequence according to the teaching of the present invention.

In at least some embodiments of the present invention, the kit comprises an antibody capable of recognizing or interacting with a polypeptide or protein according to at least some embodiments of the present invention. In at least some embodiments of the present invention, the kit further comprises at least one reagent for performing an immunohistochemical assay, radioimaging assays, in-vivo imaging, positron emission tomography (PET), single photon emission computer tomography (SPECT), magnetic resonance imaging (MRI), Ultra Sound, Optical Imaging, Computer Tomography, radioimmunoassay (RIA), ELISA, slot blot, competitive binding assays, fluorimetric imaging assays, Western blot, FACS, and the like.

All nucleic acid sequences and/or amino acid sequences, according to at least some embodiments of the invention, relate to their isolated form.

It should be noted that oligonucleotide and polynucleotide, or peptide, polypeptide and protein, may optionally be used interchangeably.

### BRIEF DESCRIPTION OF THE FIGURES

Figure 1 shows a scatter plot, demonstrating the expression of KRTCAP3 transcripts, that encode the KRTCAP3 proteins, on a virtual panel of all tissues and conditions using MED discovery engine, demonstrating overexpression of KRTCAP3 transcripts in lung cancer compared to normal lung samples.
Figures 2A and 2B present a histogram showing over expression of the KRTCAP3 (keratinocyte associated protein 3) W93943 transcripts which are detectable by amplicon as depicted in sequence name W93943_seg7-10F1R1 (SEQ ID NO: 94) in cancerous ovarian samples relative to the normal samples (Figure 2B is a continuation of Figure 2A).
Figures 3A and 3B present a histogram showing over expression of the KRTCAP3 (keratinocyte associated protein 3) W93943 transcripts which are detectable by amplicon as depicted in sequence name W93943_seg7-10F1R1 (SEQ ID NO: 94) in different normal tissues (Figure 3B is a continuation of Figure 3A).
Figures 4A and 4B present a histogram showing over expression of the KRTCAP3 transcripts detectable by or according to W93943_seg3j4-6F2R1 amplicon (SEQ ID NO:171) in cancerous Ovary samples relative to the normal samples (Figure 4B is a continuation of Figure 4A).
Figures 5A and 5B present a histogram showing over expression of the KRTCAP3 transcripts detectable by or according to W93943_seg3j4-6F2R1 amplicon (SEQ ID NO:171) in different normal tissues (Figure 5B is a continuation of Figure 5A).
Figures 6A-6C show the DNA sequences of the KRTCAP3 full length, fused or non-fused to EGFP. Gene specific sequence corresponding to the target's full length sequence is marked in bold faced, EGFP sequence is in italics, and intermediate linker regions are unbold. Figure 6A represents the DNA sequence of KRTCAP3_EGFP (SEQ ID NO:110); Figure 6B represents the DNA sequence of EGFP_KRTCAP3 (SEQ ID NO:111); Figure 6C represents the DNA sequence of KRTCAP3 (SEQ ID NO:112).
Figures 7A and 7B show the amino acid sequences of the KRTCAP3_ORF_fused or non-fused to EGFP. Gene specific sequence corresponding to the full length sequence of the protein is marked in bold faced, EGFP sequence is in italics, and intermediate linker regions are unbold. Figure 7A represents the amino acid sequence of KRTCAP3_EGFP protein (SEQ ID NO:113) (484aa); Figure 7B represents the amino acid sequence of EGFP_KRTCAP3 protein (SEQ ID NO:114) (478aa); Figure 7C represents the amino acid sequence of KRTCAP3 protein (SEQ ID NO:7) (240aa).

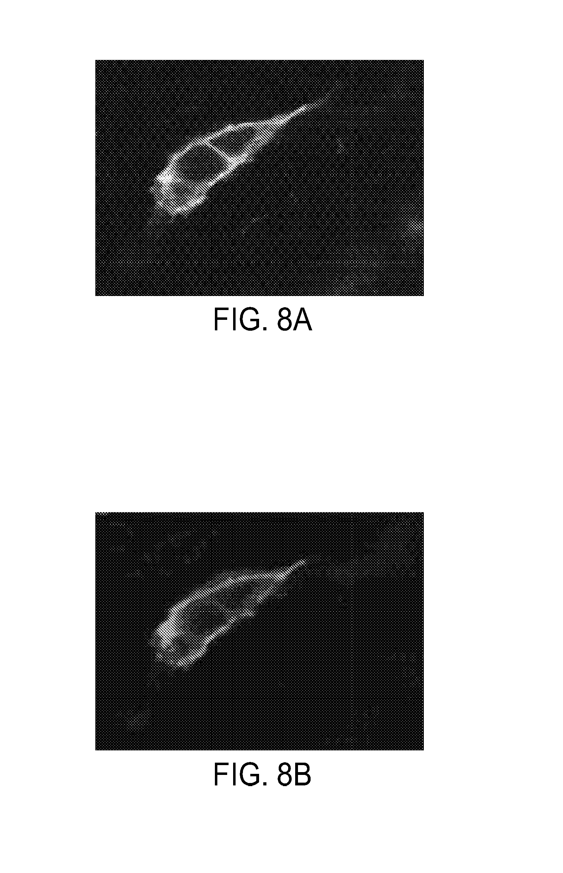

Figures 8A and 8B demonstrate the localization of the KRTCAP3 proteins of invention to cell membrane. Figure 8A demonstrates by green fluorescence of EGFP that the EGFP_KRTCAP3_P2 (SEQ ID NO: 114) fused protein localizes to the cell membrane upon expression in HEK 293T cells. The image was obtained using the 40x objective of the confocal microscope. Figure 8B demonstrates by red fluorescence of anti-GFP antibody that the EGFP_KRTCAP3_P2 (SEQ ID NO: 114) fused protein localizes to the cell membrane upon expression in HEK 293T cells. The image was obtained using the 40x objective of the confocal microscope.

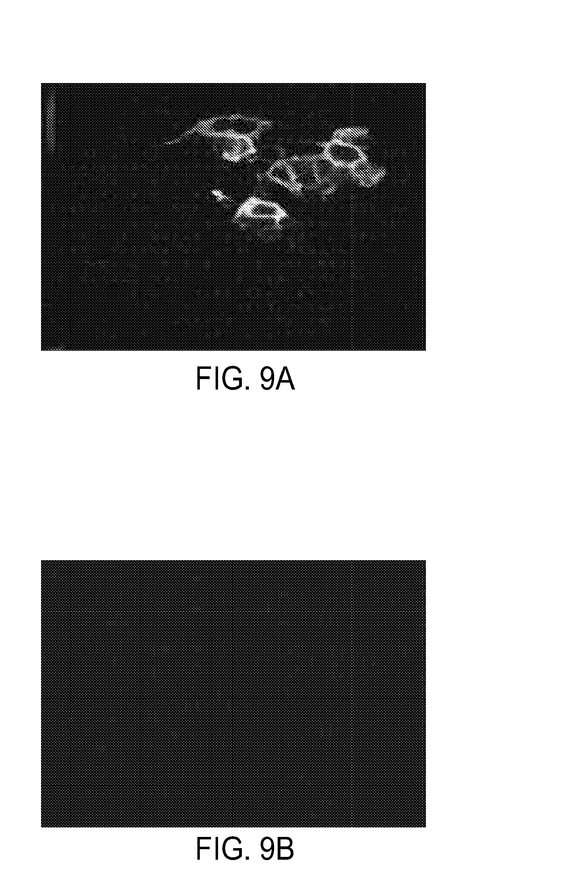

Figures 9A and 9B demonstrate the orientation of EGFP_KRTCAP3_P2 protein within the cell. Figure 9A demonstrates by green fluorescence of EGFP that the EGFP_KRTCAP3_P2 (SEQ ID NO: 114) fused protein localizes to the cell membrane of non permebealized EGFP-KRTCAP3 HEK 293T transfected cells. Figure 9B demonstrates the immunostaining with anti GFP of non permebealized EGFP-KRTCAP3 HEK 293T transfected cells immunostained. The absence of anti-GFP red fluorescence (as compared with Figure 8B) indicates that the EGFP_KRTCAP3_P2 (SEQ ID NO:114) fused protein is positioned in the plasma membrane with its amino terminus facing the cytosol. The images were obtained using the 40x objective of the confocal microscope.
Figures 10A-10D demonstrate Western blot analysis using KRTCAP3 antibodies on HEK 293T transfected cell lysates. Figure 10A-10B show Western blot analysis using KRT223 antibodies (corresponding to rabbits marked RB5257 and RB5258), on KRTCAP3-HEK293T cell lysates (lane 1) and pIRESpuro3-HEK293T cell lystaes (lane 2). Figure 10C-D show Western blot analysis using KRT143 antibodies (corresponding to rabbits marked RB5259 and RB5261), on KRTCAP3-HEK293T cell lysates (lane 1) and pIRESpuro3-HEK293T cell lystaes (lane 2).

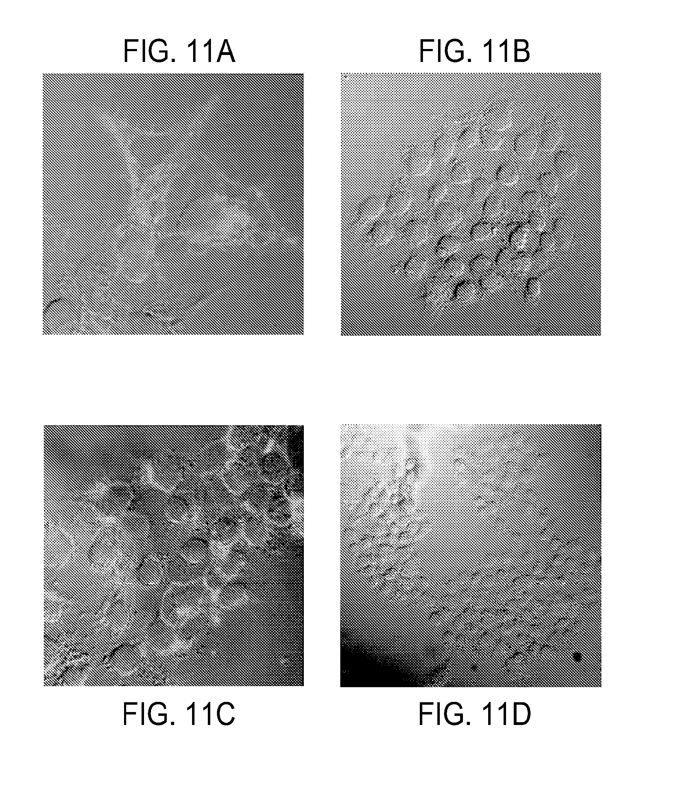

Figures 11A-11D demonstrate immunostaining of HEK-293T cells using purified KRTCAP3 antibodies. Figures 11A-11B present imunnostaining using KRT143 antibodies on KRTCAP3 HEK-293T transfected cells (Figure 11A) or pIRESpuro3 HEK-293T transfected cells (Figure 11B). KRT143 antibodies shows a specific signal in the KRTCAP3 transfected cells which is absent in the pIRESpuro3 transfected cells. Figures 11C-11D present imunnostaining using KRT223 antibodies on KRTCAP3 HEK-293T transfected cells (Figure 11C) or pIRESpuro3 HEK-293T transfected cells (Figure 11D). KRT223 antibodies shows a specific signal in the KRTCAP3 transfected cells which is absent in the pIRESpuro3 transfected cells. The image was obtained using the 40x objective of the confocal microscope.
Figure 12 demonstrates intense immunohistochemical staining of an ovary carcinoma sample obtained from a 52- year old female, using Antibody KRT223. The signal was quantified using a 0-4 scale, and was given the signal intensity 2.
Figure 13 demonstrates prominent immunohistochemical staining of an adenocarcinoma sample from a metastatic gastrointestinal tumor obtained from a 31-year-old female, using Antibody KRT223. The signal was quantified using a 0-4 scale, and was given the signal intensity 3.
Figure 14 shows a scatter plot, demonstrating the expression of FAM26F transcripts that encode the FAM26F proteins, on a virtual panel of all tissues and conditions using MED discovery engine, demonstrating overexpression of FAM26F transcripts in breast cancer compared to normal breast samples.
Figure 15 shows a scatter plot, demonstrating the expression of FAM26F transcripts that encode the FAM26F proteins, on a virtual panel of all tissues and conditions using MED discovery engine, demonstrating overexpression of FAM26F transcripts in ovarian cancer compared to normal ovarian samples.
Figures 16A-16H show a scatter plot, demonstrating the overall expression of FAM26F transcripts in various diseased, normal and cancer tissues, using MED discovery engine. Figures 16A-16H are contiguous and in a sequential order.
Figures 17A-17C show a histogram representing the overexpression of FAM26F transcripts detectable by FAM26F F1/R1 primers (SEQ ID NOs: 95 and 96) in kidney cancer, liver cancer, lung cancer, NHL lymphomas and melanoma. Figures 17A-17C are contiguous and in a sequential order.
Figures 18A and 18B show a histogram showing the expression of FAM26F T82906 transcripts which are detectable by amplicon as depicted in sequence name T82906_seg5-10F7R5 (SEQ ID NO:124) in different normal tissues (Figure 18B is a continuation of Figure 18A).
Figures 19A and 19B show a histogram showing the expression of FAM26F T82906 transcripts which are detectable by amplicon as depicted in sequence name T82906_seg5-10F7R5 (SEQ ID NO:124) in blood-specific panel (Figure 19B is a continuation of Figure 19A).
Figure 20 presents the DNA sequence of the FAM26_P4_FLAG (SEQ ID NO: 174). The FLAG sequence is in underlined.
Figure 21 presents the amino acid sequence of FAM26_P4_FLAG (SEQ ID NO:175). The FLAG sequence is in underlined.
Figures 22A and 22B demonstrate the cellular localization of FAM26_P4 protein.
Figures 23A and 23B demonstrate a specific cell staining localized to cell membrane observed using anti FAM26F antibodies on FAM26F transfected cells (Figure 23A); as opposed to no staining observed using same antibodies on pIRESpuro3 HEK-293T transfected cells (Figure 23B).
Figure 24 shows a scatter plot, demonstrating the expression of MGC52498 transcripts that encode the MGC52498 proteins, on a virtual panel of all tissues and conditions using MED discovery engine, demonstrating overexpression of MGC52498 transcripts in lung cancer compared to normal lung samples.
Figures 25A and 25B show a scatter plot, demonstrating the expression of MGC52498 transcripts, that encode the MGC52498 proteins, on a virtual panel of all tissues and conditions using MED discovery engine, demonstrating overexpression of MGC52498 transcripts in various leukemia samples compared to normal blood samples (Figure 25B is a continuation of Figure 25A).
Figures 26A and 26B present a histogram showing expression of hypothetical protein MGC52498 AA213820 transcripts which are detectable by amplicon as depicted in sequence name AA213820_seg8-11F2R2 (SEQ ID NO: 109) in different normal tissues (Figure 26B is a continuation of Figure 26A).
Figures 27A and 27B present a histogram showing expression of hypothetical protein MGC52498 AA213820 transcripts which are detectable by amplicon as depicted in sequence name AA213820_seg8-11F2R2 (SEQ ID NO: 109) in blood specific panel (Figure 27B is a continuation of Figure 27A).
Figures 28A and 28B represent the DNA sequence of FLAG_MGC_T1_P4-(SEQ ID NO:182) and MGC_T1_P4_FLAG (SEQ ID NO:183), respectively; FLAG sequence is underlined.
Figures 29A and 29B represent the amino acid sequence of FLAG_MGC_T1_P4 protein (SEQ ID NO:184) and MGC_T1_P4_FLAG (SEQ ID NO:185), respectively; FLAG sequence is underlined.
Figures 30A and 30B show a scatter plot, demonstrating the expression of FAM70A transcripts that encode the FAM70A proteins, on a virtual panel of all tissues and conditions using MED discovery engine, demonstrating overexpression of FAM70A transcripts in lung cancer samples compared to normal lung samples (Figure 30B is a continuation of Figure 30A).
Figure 31 shows a scatter plot, demonstrating the expression of FAM70A transcripts that encode the FAM70A proteins, on a virtual panel of all tissues and conditions using MED discovery engine, demonstrating overexpression of FAM70A transcripts in liver cancer samples compared to normal liver samples.
Figure 32 shows a scatter plot, demonstrating the expression of FAM70A transcripts that encode the FAM70A proteins, on a virtual panel of all tissues and conditions using MED discovery engine, demonstrating overexpression of FAM70A transcripts in breast cancer samples compared to normal breast samples.
Figures 33A and 33B show a scatter plot, demonstrating the expression of FAM70A transcripts that encode the FAM70A proteins, on a virtual panel of all tissues and conditions using MED discovery engine, demonstrating overexpression of FAM70A transcripts in kidney cancer samples compared to normal kidney samples (Figure 33B is a continuation of Figure 33A).
Figures 34A and 34B show a histogram showing the expression of hypothetical protein FLJ20716-FAM70A F10649 transcripts which are detectable by amplicon as depicted in sequence name F10649_seg10-12F1R1 (SEQ ID NO: 103) in different normal tissues (Figure 34B is a continuation of Figure 34A).
Figures 35A and 35B show a histogram showing the expression of hypothetical protein FLJ20716-FAM70A F10649 transcripts which are detectable by amplicon as depicted in sequence name F10649_seg10-12F1R1 (SEQ ID NO: 103) in blood specific panel (Figure 35B is a continuation of Figure 35A).
Figure 36 represents the DNA sequence of FAM70_T1_P5_FLAG (SEQ ID NO: 119). Gene specific sequence corresponding to the target's full length sequence is marked in bold faced, FLAG sequence is unbold.
Figure 37 represents the amino acid sequence of FAM70A_T1_P5_FLAG protein (SEQ ID NO:120); gene specific sequence corresponding to the full length sequence of the protein is marked in bold faced, FLAG sequence is unbold.
Figures 38A-38D demonstrate that the FAM70A_T1_P5_FLAG (SEQ ID NO:120) fused protein localizes to cell membrane upon expression in HEK 293T cells. The image was obtained using the 40x objective of the confocal microscope.
Figures 39A and 39B present the specificity of antibodies raised against selected peptide of FAM70A. Figures 39A and 39B present the results of immuno-precipitation followed by western blot analysis using purified serum from rabbits #5663 and #5664, respectively, and FAM70 HEK-293T stable transfectants cell lysates as well as HEK-293T nontrasfected cell lysates. Lane 1 represents HEK-293T transfected cell lysates followed by IP; lane 2 represents HEK-293T non trasfected cell lysates followed by IP; lanes 3 and 4 represent the whole cell lysate of HEK-293T transfected cells.
Figures 40A-40F present immunostaning of various cells using purified anti FAM70 antibodies (rabbits #5663). Figures 40A and 40B present the results on HEK-293T transfected cells, using 1:200 or 1:1000 dillutions, respectively. Figures 40C and 40D present the results on HEK-293T non transfected cells using 1:200 or 1:1000 dillutions, respectively. Figure 40E presents the results on CHO-K1 (ATCC, CCL-61) cells and Figure 40F presents the results on MC/CAR (ATCC, CRL-8083) cells. Similar results were obtained using rabbit#5664 (data not shown).
Figures 41A-41D demonstrate red fluorescence signal of 293T transfected cells followed by incubation with 0, 5times, 25times, 50 times FAM70 peptide, respectively.
Figures 41E-41H demonstrate red fluorescence signal of 293T non transfected cells followed by incubation with 0, 5times,25times, 50 times FAM70 peptide, respectively.
Figure 42 shows a scatter plot, demonstrating the expression of TMEM154 transcripts that encode the TMEM154 proteins, on a virtual panel of all tissues and conditions using MED discovery engine, demonstrating overexpression of TMEM154 transcripts in kidney cancer samples compared to normal kidney samples.
Figures 43A and 43B demonstrate the expression of TMEM154 transcripts that encode the TMEM154 proteins, on a virtual panel of all tissues and conditions using MED discovery engine. Figure 43 shows a scatter plot, demonstrating overexpression of TMEM154 transcripts in pancreas cancer samples compared to normal pancreas samples. Figure 43B presents Kaplan-Meier survival curves of Rituximab treated DLBCL in corelation to TMEM154 expression. In Figure 43B the time scale is shown in years; solid line represents high TMEM154 expression; fragmented line represents low TMEM154 expression.
Figures 44A and 44B show a histogram showing the expression of hypothetical protein FLJ32028, TMEM154 W38346 transcripts which are detectable by amplicon as depicted in sequence name W38346_seg6-20F1R1 (SEQ ID NO: 106) in different normal tissues (Figure 44B is a continuation of Figure 44A).
Figures 45A and 45B show a histogram showing the expression of hypothetical protein FLJ32028, TMEM154 W38346 transcripts which are detectable by amplicon as depicted in sequence name W38346_seg6-20F1R1 (SEQ ID NO: 106) in blood specific panel (Figure 54B is a continuation of Figure 45A).
Figure 46 presents the DNA sequence of the TMEM154_TO_FLAG (SEQ ID NO:189); FLAG sequence is in underlined.
Figure 47 presents the amino acid sequence of TMEM154_P3_FLAG (SEQ ID NO:190); FLAG sequence is in underlined.
Figures 48A and 48B present the localization results for TMEM154_P3.
Figures 49A and 49B present the specific cell staining localized to the cell membrane, observed using purified TM21 antibodies on TMEM154 transfected cells. Figure 49A and 49B present the results obtained using TM21 antibodies purified from rabbit #6285 and rabbit #6286, respectively.
Figures 50A and 50B present the specific cell staining localized to the cell membrane, observed using purified TM101 antibodies on TMEM154 transfected cells. Figures 50A and 50B present the results obtained using TM101 antibodies purified from rabbit #6248 and rabbit #6249, respectively.
Figures 51A-51C present the results of cell staining observed using purified TM21 and TM101 antibodies on the negative control pIRESpuro3 HEK-293T transfected cells. Figures 51A and 51B present the results obtained using TM21 antibodies purified from rabbit #6285 and rabbit #6286, respectively. Figure 51C presents the results obtained using TM101 antibodies purified from rabbit #6249.

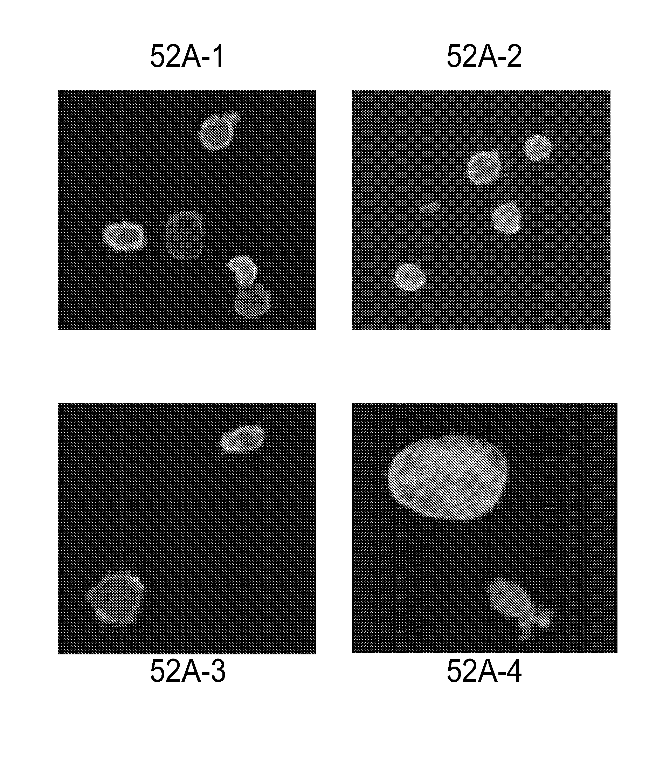

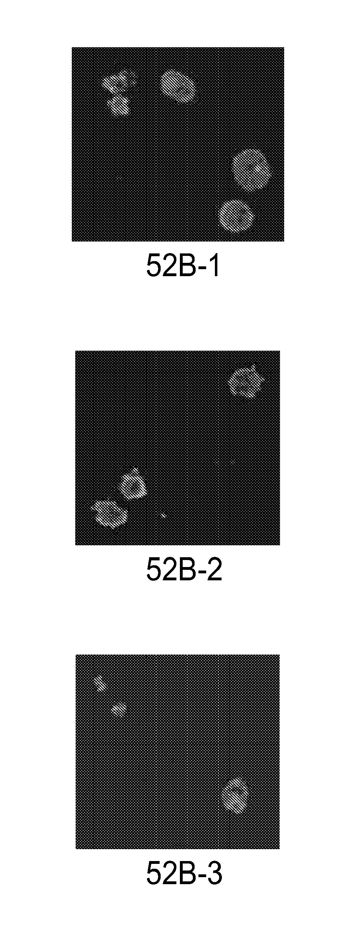

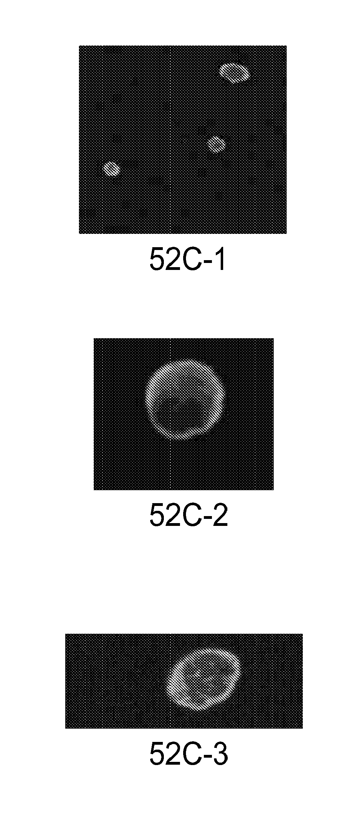

Figures 52A-52C present specific cell staining localized to the cell membrane, observed using purified TM21 and TM101 antibodies on three different cell lines: Figures 52A-1 -- 52A-4 present the results on CESS (ATCC cat no TIB-190) calls; Figures 52B-1-52B-3 present the results on Ramos (ATCC cat no CRL-1923) cells; and Figures 52C-1-52C-3 present the results on Daudi (ATCC cat no CCL-213) cells. Figures 52A-1 and 52A-2 present the results obtained using TM21 antibodies purified from rabbit #6285 and rabbit #6286, respectively. Figures 52A-3 and 52A-4 present the results obtained using TM101 antibodies purified from rabbit #6248 and rabbit #6249, respectively. Figures 52B-1 and 52B-2 present the results obtained using TM21 antibodies purified from rabbit #6285 and rabbit #6286, respectively. Figure 52B-3 presents the results obtained using TM101 antibodies purified from rabbit #6248. Figures 52C-1 and 52C-2 present the results obtained using TM21 antibodies purified from rabbit #6285 and rabbit #6286, respectively. Figure 52C-3 presents the results obtained using TM101 antibodies purified from rabbit #6248.

### DETAILED DESCRIPTION OF THE INVENTION

The present invention, in some embodiments, relates to any one of the polypeptides referred to as KRTCAP3, FAM26F, MGC52498, FAM70A, TMEM154 polypeptides, and its corresponding nucleic acid sequence, and fragments and variants and homologs thereof, and the use thereof as a therapeutic and/or diagnostic target. According to at least some embodiments, there are provided uses of these polypeptides and discrete portions thereof as a drug target for therapeutic small molecules, peptides, antibodies, antisense RNAs, siRNAs, ribozymes, and the like. According to at least some embodiments, the invention relates to diagnostic and therapeutic polyclonal and monoclonal antibodies and fragments thereof that specifically bind KRTCAP3, FAM26F, MGC52498, FAM70A, TMEM154 polypeptides and portions and variants thereof, especially those that target the extracellular domains or portions or variants thereof, or the unique bridge, edge, tail or head portion, or fragment or variant thereof. According to at least some embodiments, the invention provides human or chimeric monoclonal antibodies and fragments thereof and anti-idiotypic antibodies, that bind specifically to any of the amino acid sequences as set forth in SEQ ID NOs: 7, 8, 10-13, 15-19, 25, 29-33, 35, 36, 42-64, 115-118, 121, 127, 132-135, 146-162, 186, 191-192, 196, 199, 200, and variants and fragments and homologs thereof.

According to at least some embodiments of the invention, the antibodies are derived from particular heavy and light chain germline sequences and/or comprise particular structural features such as CDR regions comprising particular amino acid sequences. The invention provides isolated antibodies, methods of making such antibodies, immunoconjugates and bispecific molecules comprising such antibodies and pharmaceutical and diagnostic compositions containing the antibodies, immunoconjugates or bispecific molecules.

According to at least some embodiments of the invention, the specific antibodies may be used for the treatment and/or diagnosis of cancer and/or immune related conditions, as described herein.

In order that the present invention may be more readily understood, certain terms are first defined. Additional definitions are set forth throughout the detailed description.

The term "KRTCAP3 protein", as used herein, includes any protein encoded by a KRTCAP3 gene product, including the known or "wild type" protein, any splice variants thereof, any other variants thereof, or any fragments thereof (including but not limited to any extracellular portions thereof).

The term "KRTCAP3 polypeptide" refers to a polypeptide encoded by any one of the nucleic acid sequences set forth in any one of SEQ ID NOs:1-6, 9, 94, 171, 193-195, and fragments and homologous thereof, especially those possessing at least 80, 85, 90, 95, 96, 97, 98, 99% sequence identity therewith. These nucleic acid sequences are referred to herein as "KRTCAP3 polynucleotide". The term also refers to any one of the polypeptides set forth in any one of SEQ ID NOs:7, 8, 10-13; extracellular portions thereof, set forth in any one of SEQ ID NOs:47-51; unique bridge, edge portion, tail or head portion thereof, set forth in any one of SEQ ID NOs: 146-148; protein fragments selected from any of the isolated polypeptides, that are used for rabbit, mouse or other mammal immunization and specific antibodies production, set forth in any one of SEQ ID NOs: 115, 116; and fragments and homologous thereof, especially those possessing at least 80, 85, 90, 95, 96, 97, 98, 99% sequence identity therewith. The term "KRTCAP3 polynucleotide" or "KRTCAP3 polypeptide", as used herein, further refers to any one of the foregoing polynucleotides and polypeptides, respectively, that are differentially expressed e.g., in cancer, including but not limited to lung cancer, breast cancer, colon cancer and ovarian cancer, wherein the cancer may be non-metastatic, invasive or metastatic.

The term "KRTCAP3 variant(s)", as used herein, refers to a protein encoded by any one of the nucleic acid sequences set forth in SEQ ID NOs: 2, 3, 4, 6, 9, 94, 171, 193-195, and fragments and homologous thereof, especially those possessing at least 80, 85, 90, 95, 96, 97, 98, 99% sequence identity therewith. The term "KRTCAP3 novel variant(s)", as used herein, further refers to any one of the proteins set forth in any one of SEQ ID NOs:10, 11, 13, 47-51, 146-148, and fragments and homologous thereof, especially those possessing at least 80, 85, 90, 95, 96, 97, 98, 99% sequence identity therewith.

The term "KRTCAP3 proteins" as used herein encompass any protein within the groups of "KRTCAP3 polypeptide", "KRTCAP3 variant(s)" and "KRTCAP3 novel variant(s)".

The term "FAM26F protein", as used herein, includes any protein encoded by a FAM26F gene product, including the known or "wild type" protein, any splice variants thereof, any other variants thereof, or any fragments thereof (including but not limited to any extracellular portions thereof).

The term "FAM26F polypeptide", as used herein, refers to a polypeptide encoded by any one of the nucleic acid sequences set forth in any one of SEQ ID NOs:14, 125, 97, 124, and fragments and homologous thereof, especially those possessing at least 80, 85, 90, 95, 96, 97, 98, 99% sequence identity therewith. These nucleic acid sequences are referred to herein as "FAM26F polynucleotides". The term also refers to any polypeptide set forth in any one of SEQ ID NOs:15-18; extracellular portions thereof, set forth in any one of SEQ ID NOs: 52, 53; unique bridge, edge portion, tail or head portion thereof, set forth in any one of SEQ ID NOs: 127, 149; protein fragments selected from any of the isolated polypeptides, that are used for rabbit, mouse or other mammal immunization and specific antibodies production, set forth in any one of SEQ ID NOs: 117, 118; and fragments and homologous thereof, especially those possessing at least 80, 85, 90, 95, 96, 97, 98, 99% sequence identity therewith. The term "FAM26F polynucleotide" or "FAM26F polypeptide", as used herein, further refers to any of the foregoing polynucleotides and polypeptides, respectively, that are differentially expressed e.g., in cancer, including but not limited to ovarian cancer, breast cancer, prostate cancer, renal cancer, melanoma, acute lymphocytic leukemia, chronic lymphocytic leukemia, acute myelogenous leukemia, chronic myelogenous leukemia, multiple myeloma, Hodgkin's lymphoma or Non-Hodgkin's lymphoma, wherein the cancer may be non-metastatic, invasive or metastatic as well as non-malignant disorders such as immune related conditions or disorders including but not limited to inflammatory or autoimmune diseases, transplant rejection and graft versus host disease.

The term "MGC52498 protein", as used herein, includes any protein encoded by a MGC52498 gene product, including the known or "wild type" protein, any splice variants thereof, any other variants thereof, or any fragments thereof (including but not limited to any extracellular portions thereof).

The term "MGC52498 polypeptide", as used herein, refers to a polypeptide encoded by any one of the nucleic acid sequences set forth in any one of SEQ ID NOs:20, 27, 109, 131, 201, and fragments and homologous thereof, especially those possessing at least 80, 85, 90, 95, 96, 97, 98, 99% sequence identity therewith. These nucleic acid sequences are referred to herein as "KRTCAP3 polynucleotides". The term also refers to any one of the polypeptides set forth in any one of SEQ ID NOs:19, 132-135; extracellular portions thereof, set forth in any one of SEQ ID NOs:60-62, unique bridge, edge portion, tail or head portion thereof, set forth in any one of SEQ ID NOs:25, 150-154, 200, and fragments and homologous thereof, especially those possessing at least 80, 85, 90, 95, 96, 97, 98, 99% sequence identity therewith. The term "MGC52498 polynucleotide" or "MGC52498 polypeptide", as used herein, further refers to any of the foregoing polynucleotides and polypeptides, respectively, that are differentially expressed e.g., in cancer, including but not limited to lung cancer, multiple myeloma, lymphomas, especially non-Hodgkins lymphoma, leukemia, especially T cell leukemia, wherein the cancer may be non-metastatic, invasive or metastatic as well as non-malignant disorders such as immune related conditions or disorders including but not limited to inflammatory or autoimmune diseases, transplant rejection and graft versus host disease.

The term "MGC52498 variant(s)", as used herein, refers to a protein encoded by any one of the nucleic acid sequences set forth in SEQ ID NOs:20, 27, 109, 201, and fragments and homologous thereof, especially those possessing at least 80, 85, 90, 95, 96, 97, 98, 99% sequence identity therewith. The term "MGC52498 novel variant(s)", as used herein, further refers to any one of the proteins set forth in any one of SEQ ID NOs:19, 25, 60-62, 150-154, 200, and fragments and homologous thereof, especially those possessing at least 80, 85, 90, 95, 96, 97, 98, 99% sequence identity therewith.

The term "MGC52498 proteins" as used herein encompass any protein within the groups of "MGC52498 polypeptides", "MGC52498 variant(s)" and "MGC52498 novel variant(s)".

The term "FAM70A protein", as used herein, includes any protein encoded by a FAM70A gene product, including the known or "wild type" protein, any splice variants thereof, any other variants thereof, or any fragments thereof (including but not limited to any extracellular portions thereof).

The term "FAM70A polypeptide", as used herein, refers to a polypeptide encoded by any one of the nucleic acid sequences set forth in any one of SEQ ID NOs:21, 22, 24, 26, 28, 103, 197, 198, and fragments and homologous thereof, especially those possessing at least 80, 85, 90, 95, 96, 97, 98, 99% sequence identity therewith. These nucleic acid sequences are referred to herein as "FAM70A polynucleotides". The term also refers to any one of the polypeptides as set forth in any one of SEQ ID NOs:29-33, 35, 36; extracellular portions thereof, set forth in any one of SEQ ID NOs:54-59; unique bridge, edge portion, tail or head portion thereof, set forth in any one of SEQ ID NOs: 155-160, 196, 199; protein fragments selected from any of the isolated polypeptides, used for rabbit immunization and specific antibodies production, set forth in any one of SEQ ID NOs:121, 186,and fragments and homologous thereof, especially those possessing at least 80, 85, 90, 95, 96, 97, 98, 99% sequence identity therewith. The term "FAM70A polynucleotide" or "FAM70A polypeptide", as used herein, further refers to any one of the foregoing polynucleotides and polypeptides, respectively, that are differentially expressed e.g., in cancer, including but not limited to lung cancer, liver cancer, breast cancer, kidney cancer, multiple myeloma, and wherein the cancer may be non-metastatic, invasive or metastatic as well as non-malignant disorders such as immune related conditions or disorders including but not limited to inflammatory or autoimmune diseases, transplant rejection and graft versus host disease.

The term "FAM70A variant(s)", as used herein, refers to a protein encoded by any one of the nucleic acid sequences set forth in SEQ ID NOs:26, 103, 197, 198, and fragments and homologous thereof, especially those possessing at least 80, 85, 90, 95, 96, 97, 98, 99% sequence identity therewith. The term "FAM70A novel variant(s)", as used herein, further refers to any one of the proteins set forth in any one of SEQ ID NOs:36, 54-59, 155-160, 196, 199, and fragments and homologous thereof, especially those possessing at least 80, 85, 90, 95, 96, 97, 98, 99% sequence identity therewith.

The term "FAM70A proteins" as used herein encompass any protein within the groups of "FAM70A polypeptides", "FAM70A variant(s)", and "FAM70A novel variant(s)".

The term "TMEM154 protein", as used herein, includes any protein encoded by a TMEM154 gene product, including the known or "wild type" protein, any splice variants thereof, any other variants thereof, or any fragments thereof (including but not limited to any extracellular portions thereof).

The term "TMEM154 polypeptide", as used herein, refers to a polypeptide encoded by any one of the nucleic acid sequences set forth in any one of SEQ ID NOs:23, 38-41, 106, and fragments and homologous thereof, especially those possessing at least 80, 85, 90, 95, 96, 97, 98, 99% sequence identity therewith. These nucleic acid sequences are referred to herein as " TMEM154 polynucleotide". The term also refers to any one of the polypeptides set forth in any one of SEQ ID NOs:42-46; extracellular portions thereof, set forth in any one of SEQ ID NOs:63, 64; unique bridge, edge portion, tail or head portion thereof, set forth in any one of SEQ ID NOs: 161, 162; protein fragments selected from any of the isolated polypeptides, used for rabbit immunization and specific antibodies production, set forth in any one of SEQ ID NOs: 191, 192; and fragments and homologous thereof, especially those possessing at least 80, 85, 90, 95, 96, 97, 98, 99% sequence identity therewith. The term "TMEM154 polynucleotide" or "TMEM154 polypeptide", as used herein, further refers to any one of the foregoing polynucleotides and polypeptides, respectively, that are differentially expressed e.g., in cancer, including but not limited to kidney cancer, pancreatic cancer, multiple myeloma, lymphomas, especially non-Hodgkins lymphoma, wherein the cancer may be non-metastatic, invasive or metastatic as well as non-malignant disorders such as immune related conditions or disorders including but not limited to inflammatory or autoimmune diseases, transplant rejection and graft versus host disease, specifically SLE.

The term the "soluble ectodomain (ECD)" or "ectodomain" or "extracellular ectodomain" of a KRTCAP3 polypeptide refers to the polypeptide sequences listed below or the corresponding nucleic acid sequences (which do not comprise the signal peptide and the TM (transmembrane portion) of the KRTCAP3 polypeptide):
Two ECD regions of the polypeptide W93943_P2 (SEQ ID NO:7): W93943_P2_42-62 (SEQ ID NO:47) - sequence: TVLRHVANPRGAVTPEYTVAN (and optionally bridging amino acids of any of one, two, three, four, five, six, seven, eight, nine or 10 amino acids on either side, starting anywhere from residue 32 and ending anywhere up to residue 72; and also non-linear epitopes incorporating this sequence or a portion thereof, as well as any of one, two, three, four, five, six, seven, eight, nine or 10 non-contiguous amino acids of the sequence);
W93943_P2_115-162 (SEQ ID NO:48) - sequence: LAVSLTVANGGRRLIADCHPGLLDPLVPLDEGPGHTDCPFDPTRIYDT (and optionally bridging amino acids of any of one, two, three, four, five, six, seven, eight, nine or 10 amino acids on either side, starting anywhere from residue 105 and ending anywhere up to residue 172; and also non-linear epitopes incorporating this sequence or a portion thereof, as well as any of one, two, three, four, five, six, seven, eight, nine or 10 non-contiguous amino acids of the sequence);
Three ECD regions of the polypeptide W93943_P13 (SEQ ID NO:10): W93943_P13_1-20 (SEQ ID NO:49) - sequence: MRRCSLCAFDAARGPRRLMR (and optionally bridging amino acids of any of one, two, three, four, five, six, seven, eight, nine or 10 amino acids, starting anywhere from residue 1 and ending anywhere up to residue 30; and also non-linear epitopes incorporating this sequence or a portion thereof, as well as any of one, two, three, four, five, six, seven, eight, nine or 10 non-contiguous amino acids of the sequence);
W93943_P13_77-91 (SEQ ID NO:50) - sequence: DPGGGRAPGEPSRPK (and optionally bridging amino acids of any of one, two, three, four, five, six, seven, eight, nine or 10 amino acids on either side, starting anywhere from residue 67 and ending anywhere up to residue 101; and also non-linear epitopes incorporating this sequence or a portion thereof, as well as any of one, two, three, four, five, six, seven, eight, nine or 10 non-contiguous amino acids of the sequence);
W93943_P13_141-188 (SEQ ID NO:48) - sequence: LAVSLTVANGGRRLIADCHPGLLDPLVPLDEGPGHTDCPFDPTRIYDT (and optionally bridging amino acids of any of one, two, three, four, five, six, seven, eight, nine or 10 amino acids on either side, starting anywhere from residue 131 and ending anywhere up to residue 198; and also non-linear epitopes incorporating this sequence or a portion thereof, as well as any of one, two, three, four, five, six, seven, eight, nine or 10 non-contiguous amino acids of the sequence);
Two ECD regions of the polypeptide W93943_P14 (SEQ ID NO:11): W93943_P14_42-62 (SEQ ID NO:47) - sequence: TVLRHVANPRGAVTPEYTVAN (and optionally bridging amino acids of any of one, two, three, four, five, six, seven, eight, nine or 10 amino acids on either side, starting anywhere from residue 32 and ending anywhere up to residue 72; and also non-linear epitopes incorporating this sequence or a portion thereof, as well as any of one, two, three, four, five, six, seven, eight, nine or 10 non-contiguous amino acids of the sequence);
W93943_P14_115-162 (SEQ ID NO:48) - sequence: LAVSLTVANGGRRLIADCHPGLLDPLVPLDEGPGHTDCPFDPTRIYDT (and optionally bridging amino acids of any of one, two, three, four, five, six, seven, eight, nine or 10 amino acids on either side, starting anywhere from residue 105 and ending anywhere up to residue 172; and also non-linear epitopes incorporating this sequence or a portion thereof, as well as any of one, two, three, four, five, six, seven, eight, nine or 10 non-contiguous amino acids of the sequence);
Two ECD regions of the polypeptide W93943_P17 (SEQ ID NO:12): W93943_P17_42-62 (SEQ ID NO:47) - sequence: TVLRHVANPRGAVTPEYTVAN (and optionally bridging amino acids of any of one, two, three, four, five, six, seven, eight, nine or 10 amino acids on either side, starting anywhere from residue 32 and ending anywhere up to residue 72; and also non-linear epitopes incorporating this sequence or a portion thereof, as well as any of one, two, three, four, five, six, seven, eight, nine or 10 non-contiguous amino acids of the sequence);
W93943_P17_115-162 (SEQ ID NO:48) - sequence: LAVSLTVANGGRRLIADCHPGLLDPLVPLDEGPGHTDCPFDPTRIYDT (and optionally bridging amino acids of any of one, two, three, four, five, six, seven, eight, nine or 10 amino acids on either side, starting anywhere from residue 105 and ending anywhere up to residue 172; and also non-linear epitopes incorporating this sequence or a portion thereof, as well as any of one, two, three, four, five, six, seven, eight, nine or 10 non-contiguous amino acids of the sequence);
Two ECD regions of the polypeptide W93943_P18 (SEQ ID NO:13): W93943_P18_42-62 (SEQ ID NO:47) - sequence: TVLRHVANPRGAVTPEYTVAN (and optionally bridging amino acids of any of one, two, three, four, five, six, seven, eight, nine or 10 amino acids on either side, starting anywhere from residue 32 and ending anywhere up to residue 72; and also non-linear epitopes incorporating this sequence or a portion thereof, as well as any of one, two, three, four, five, six, seven, eight, nine or 10 non-contiguous amino acids of the sequence);
W93943_P18_115-171 (SEQ ID NO:51) - sequence: CCVAALTLRGVGPCRKDGLQGQLEEMTELESPKCKRQENEQLLDQNQEIRASQRS WV (and optionally bridging amino acids of any of one, two, three, four, five, six, seven, eight, nine or 10 amino acids on either side, starting anywhere from residue 105 and ending anywhere up to residue 181; and also non-linear epitopes incorporating this sequence or a portion thereof, as well as any of one, two, three, four, five, six, seven, eight, nine or 10 non-contiguous amino acids of the sequence);
and fragments and variants and homologs thereof possessing at least 80%, at least 85%, at least 90%, at least 95, at least 96, at least 97, at least 98 or at least 99% sequence identity therewith.

The term the "soluble ectodomain (ECD)" or "ectodomain" or "extracellular ectodomain" of a FAM26F polypeptide refers to the polypeptide sequences listed below or the corresponding nucleic acid sequences (which do not comprise the signal peptide and the TM (transmembrane portion) of the FAM26F polypeptide):
Two ECD regions of the polypeptide T82906_P4 (SEQ ID NO:18): T82906_P4_40-48 (SEQ ID NO:52) - sequence: QCPCSAAWN (and optionally bridging amino acids of any of one, two, three, four, five, six, seven, eight, nine or 10 amino acids on either side, starting anywhere from residue 30 and ending anywhere up to residue 58; and also non-linear epitopes incorporating this sequence or a portion thereof, as well as any of one, two, three, four, five, six, seven, eight, nine or 10 non-contiguous amino acids of the sequence);
T82906_P4_125-175 (SEQ ID NO:53) - sequence: ECAATGSAAFAQRLCLGRNRSCAAELPLVPCNQAKASDVQDLLKDLKAQSQ (and optionally bridging amino acids of any of one, two, three, four, five, six, seven, eight, nine or 10 amino acids on either side, starting anywhere from residue 115 and ending anywhere up to residue 185; and also non-linear epitopes incorporating this sequence or a portion thereof, as well as any of one, two, three, four, five, six, seven, eight, nine or 10 non-contiguous amino acids of the sequence);
One ECD region of the polypeptide T82906_P3 (SEQ ID NO:16): T82906_P3_27-143 (SEQ ID NO:127) - sequence LSPVSFLQLKFWKIYLEQEQQILKSKATEHATELAKENIKCFFEGSHPKEYNTPSMK EWQQISSLYTFNPKGQYYSMLHKYVNRKEKTHSIRSTEGDTVIPVLGFVDSSGINST PEL (and optionally bridging amino acids of any of one, two, three, four, five, six, seven, eight, nine or 10 amino acids on either side, starting anywhere from residue 17 and ending anywhere up to residue 153; and also non-linear epitopes incorporating this sequence or a portion thereof, as well as any of one, two, three, four, five, six, seven, eight, nine or 10 non-contiguous amino acids of the sequence);
and fragments and variants and homologs thereof possessing at least 80%, at least 85%, at least 90%, at least 95, at least 96, at least 97, at least 98 or at least 99% sequence identity therewith.

The term the "soluble ectodomain (ECD)" or "ectodomain" or "extracellular ectodomain" of a MGC52498 polypeptide refers to the polypeptide sequences listed below or the corresponding nucleic acid sequences (which do not comprise the signal peptide and the TM (transmembrane portion) of the MGC52498 protein):
Three ECD regions of the polypeptide AA213820_P4 (SEQ ID NO:135): AA213820_P4_1-55 (SEQ ID NO:60) - sequence: MSGACTSYVSAEQEVVRGFSCPRPGGEAAAVFCCGFRDHKYCCDDPHSFFPYEHS (and optionally bridging amino acids of any of one, two, three, four, five, six, seven, eight, nine or 10 amino acids, starting anywhere from residue 1 and ending anywhere up to residue 65; and also non-linear epitopes incorporating this sequence or a portion thereof, as well as any of one, two, three, four, five, six, seven, eight, nine or 10 non-contiguous amino acids of the sequence);
AA213820_P4_91-190 (SEQ ID NO:61) - sequence: SSKPHTKLDLGLSLQTAGPEEVSPDCQGVNTGMAAEVPKVSPLQQSYSCLNPQLES NEGQAVNSKRLLHHCFMATVTTSDIPGSPEEASVPNPDLCGPVP (and optionally bridging amino acids of any of one, two, three, four, five, six, seven, eight, nine or 10 amino acids on either side, starting anywhere from residue 81 and ending anywhere up to residue 200; and also non-linear epitopes incorporating this sequence or a portion thereof, as well as any of one, two, three, four, five, six, seven, eight, nine or 10 non-contiguous amino acids of the sequence);
AA213820_P_61-71 (SEQ ID NO:62) - sequence: MASLWPSALTFNTDANIPGPLGFCGGWVRLCSLSSLTPPCGRRLVPCLSAPAPNAPR LPAPARCSIGALIG (and optionally bridging amino acids of any of one, two, three, four, five, six, seven, eight, nine or 10 amino acids on either side, starting anywhere from residue 51 and ending anywhere up to residue 81; and also non-linear epitopes incorporating this sequence or a portion thereof, as well as any of one, two, three, four, five, six, seven, eight, nine or 10 non-contiguous amino acids of the sequence),
and fragments and variants and homologs thereof possessing at least 80%, at least 85%, at least 90%, at least 95, at least 96, at least 97, at least 98 or at least 99% sequence identity therewith.

The term the "soluble ectodomain (ECD)" or "ectodomain" or "extracellular ectodomain" of a FAM70A polypeptide refers to the polypeptide sequences listed below or the corresponding nucleic acid sequences (which do not comprise the signal peptide and the TM (transmembrane portion) of the FAM70A protein):
Two ECD regions of the polypeptide F10649_P4 (SEQ ID NO:30): F10649_P4_51-59 (SEQ ID NO:54) - sequence: TTRTQNVTV (and optionally bridging amino acids of any of one, two, three, four, five, six, seven, eight, nine or 10 amino acids on either side, starting anywhere from residue 41 and ending anywhere up to residue 69; and also non-linear epitopes incorporating this sequence or a portion thereof, as well as any of one, two, three, four, five, six, seven, eight, nine or 10 non-contiguous amino acids of the sequence);
F10649_P4_110-225 (SEQ ID NO:55) - sequence: DGVFAARHIDLKPLYANRCHYVPKTSQKEAEEVISSSTKNSPSTRVMRNLTQAARE VNCPHLSREFCTPRIRGNTCFCCDLYNCGNRVEITGGYYEYIDVSSCQDIIHLYHLL WSA (and optionally bridging amino acids of any of one, two, three, four, five, six, seven, eight, nine or 10 amino acids on either side, starting anywhere from residue 100 and ending anywhere up to residue 235; and also non-linear epitopes incorporating this sequence or a portion thereof, as well as any of one, two, three, four, five, six, seven, eight, nine or 10 non-contiguous amino acids of the sequence);
Two ECD regions of the polypeptide F10649_P5 (SEQ ID NO:33): F10649_P5_51-59 (SEQ ID NO:54) - sequence: TTRTQNVTV (and optionally bridging amino acids of any of one, two, three, four, five, six, seven, eight, nine or 10 amino acids on either side, starting anywhere from residue 41 and ending anywhere up to residue 69; and also non-linear epitopes incorporating this sequence or a portion thereof, as well as any of one, two, three, four, five, six, seven, eight, nine or 10 non-contiguous amino acids of the sequence);
F10649_P5_110-201 (SEQ ID NO:56) - sequence: DGVFAARHIDLKPLYANRCHYVPKTSQKEAEEVNCPHLSREFCTPRIRGNTCFCCD LYNCGNRVEITGGYYEYIDVSSCQDIIHLYHLLWSA (and optionally bridging amino acids of any of one, two, three, four, five, six, seven, eight, nine or 10 amino acids on either side, starting anywhere from residue 100 and ending anywhere up to residue 211; and also non-linear epitopes incorporating this sequence or a portion thereof, as well as any of one, two, three, four, five, six, seven, eight, nine or 10 non-contiguous amino acids of the sequence);
Two ECD regions of the polypeptide F10649_P7 (SEQ ID NO:35): F10649_P7_51-59 (SEQ ID NO:54) - sequence: TTRTQNVTV (and optionally bridging amino acids of any of one, two, three, four, five, six, seven, eight, nine or 10 amino acids on either side, starting anywhere from residue 41 and ending anywhere up to residue 69; and also non-linear epitopes incorporating this sequence or a portion thereof, as well as any of one, two, three, four, five, six, seven, eight, nine or 10 non-contiguous amino acids of the sequence);
F10649_P7_110-241 (SEQ ID NO:58)- sequence : DGVFAARHIDLKPLYANRCHYVPKTSQKEAEENPTLPALNCSVENTHPTVSYYAHP QVASYNTYYHSPPHLPPYSAYDFQHSGVFPSSPPSGLSDEPQSASPSPSYMWSSSAPP RYSPPYYPPFEKPPPYSP (and optionally bridging amino acids of any of one, two, three, four, five, six, seven, eight, nine or 10 amino acids on either side, starting anywhere from residue 100 and ending anywhere up to residue 251; and also non-linear epitopes incorporating this sequence or a portion thereof, as well as any of one, two, three, four, five, six, seven, eight, nine or 10 non-contiguous amino acids of the sequence);
Two ECD regions of the polypeptide F10649_P8 (SEQ ID NO:36): F10649_P8_51-65 (SEQ ID NO:59) - sequence: TTRTQNVTVGGYYPG (and optionally bridging amino acids of any of one, two, three, four, five, six, seven, eight, nine or 10 amino acids on either side, starting anywhere from residue 41 and ending anywhere up to residue 75; and also non-linear epitopes incorporating this sequence or a portion thereof, as well as any of one, two, three, four, five, six, seven, eight, nine or 10 non-contiguous amino acids of the sequence);
F10649_P8_223-328 (SEQ ID NO:57) - sequence: GGFKDMNPTLPALNCSVENTHPTVSYYAHPQVASYNTYYHSPPHLPPYSAYDFQHS GVFPSSPPSGLSDEPQSASPSPSYMWSSSAPPRYSPPYYPPFEKPPPYSP (and optionally bridging amino acids of any of one, two, three, four, five, six, seven, eight, nine or 10 amino acids on either side, starting anywhere from residue 213 and ending anywhere up to residue 338; and also non-linear epitopes incorporating this sequence or a portion thereof, as well as any of one, two, three, four, five, six, seven, eight, nine or 10 non-contiguous amino acids of the sequence);
One ECD region of the polypeptide F10649_P10 (SEQ ID NO:32): F10649_P10_80-185 (SEQ ID NO:57) - sequence: GGFKDMNPTLPALNCSVENTHPTVSYYAHPQVASYNTYYHSPPHLPPYSAYDFQHS GVFPSSPPSGLSDEPQSASPSPSYMWSSSAPPRYSPPYYPPFEKPPPYSP (and optionally bridging amino acids of any of one, two, three, four, five, six, seven, eight, nine or 10 amino acids on either side, starting anywhere from residue 70 and ending anywhere up to residue 195; and also non-linear epitopes incorporating this sequence or a portion thereof, as well as any of one, two, three, four, five, six, seven, eight, nine or 10 non-contiguous amino acids of the sequence);
and fragments and variants and homologs thereof possessing at least 80%, at least 85%, at least 90%, at least 95, at least 96, at least 97, at least 98 or at least99% sequence identity therewith.

The term the "soluble ectodomain (ECD)" or "ectodomain" or "extracellular ectodomain" of a polypeptide TMEM154 refers to the polypeptide sequences below or the corresponding nucleic acid sequences (which do not comprise the signal peptide and the TM (transmembrane portion) of the TMEM154 protein):
One ECD region of the polypeptide W38346_P3 (SEQ ID NO:42): W38346_P3_23-75 (SEQ ID NO:63) - sequence: EELENSGDTTVESERPNKVTIPSTFAAVTIKETLNANINSTNFAPDENQLE (and optionally bridging amino acids of any of one, two, three, four, five, six, seven, eight, nine or 10 amino acids on either side, starting anywhere from residue 13 and ending anywhere up to residue 85; and also non-linear epitopes incorporating this sequence or a portion thereof, as well as any of one, two, three, four, five, six, seven, eight, nine or 10 non-contiguous amino acids of the sequence);
One ECD region of the polypeptide W38346_P4 (SEQ ID NO:45): W38346_P4_20-105 (SEQ ID NO:64) - sequence: ATYYKRKRTKQEPSSQGSQSALQTYELGSENVKVPIFEEDTPSVMEIEMEELDKWM NSMNRNADFECLPTLKEEKESNHNPSDSES (and optionally bridging amino acids of any of one, two, three, four, five, six, seven, eight, nine or 10 amino acids on either side, starting anywhere from residue 10 and ending anywhere up to residue 115; and also non-linear epitopes incorporating this sequence or a portion thereof, as well as any of one, two, three, four, five, six, seven, eight, nine or 10 non-contiguous amino acids of the sequence);
One ECD region of the polypeptide W38346_P7 (SEQ ID NO:46): W38346_P7_23-75 (SEQ ID NO:63) - sequence: EELENSGDTTVESERPNKVTIPSTFAAVTIKETLNANINSTNFAPDENQLE (and optionally bridging amino acids of any of one, two, three, four, five, six, seven, eight, nine or 10 amino acids on either side, starting anywhere from residue 13 and ending anywhere up to residue 85; and also non-linear epitopes incorporating this sequence or a portion thereof, as well as any of one, two, three, four, five, six, seven, eight, nine or 10 non-contiguous amino acids of the sequence);
and fragments and variants and homologs thereof possessing at least 80%, at least 85%, at least 90%, at least 95, at least 96, at least 97, at least 98 or at least 99% sequence identity therewith.

The term "immune response" refers to the action of, for example, lymphocytes, antigen presenting cells, phagocytic cells, granulocytes, and soluble macromolecules produced by the above cells or cells produced by the liver or spleen (including antibodies, cytokines, and complement) that results in selective damage to, destruction of, or elimination from the human body of invading pathogens, cells or tissues infected with pathogens, cancerous cells, or, in cases of autoimmunity or pathological inflammation, normal human cells or tissues.

The term "antibody" as referred to herein includes whole polyclonal and monoclonal antibodies and any antigen binding fragment (i.e., "antigen-binding portion") or single chains thereof. An "antibody" refers to a glycoprotein comprising at least two heavy (H) chains and two light (L) chains inter-connected by disulfide bonds, or an antigen binding portion thereof. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The term "antigen-binding portion" of an antibody (or simply "antibody portion"), as used herein, refers to one or more fragments of an antibody that retain the ability to specifically bind to an antigen (e.g., KRTCAP3, FAM26F, MGC52498, FAM70A, TMEM154 polypeptides and proteins). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the V Light, V Heavy, Constant light (CL) and CH1 domains; (ii) a F(ab').2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies.

An "isolated antibody", as used herein, is intended to refer to an antibody that is substantially free of other antibodies having different antigenic specificities (e.g., an isolated antibody that specifically binds KRTCAP3, FAM26F, MGC52498, FAM70A, TMEM154 proteins or KRTCAP3, FAM26F, MGC52498, FAM70A, or TMEM154 polypeptides is substantially free of antibodies that specifically bind antigens other than KRTCAP3, FAM26F, MGC52498, FAM70A, TMEM154 proteins or polypeptides, respectively. An isolated antibody that specifically binds KRTCAP3, FAM26F, MGC52498, FAM70A, TMEM154 proteins or polypeptides may, however, have cross-reactivity to other antigens, such as KRTCAP3, FAM26F, MGC52498, FAM70A, TMEM154 proteins or KRTCAP3, FAM26F, MGC52498, FAM70A, or TMEM154 polypeptides from other species, respectively. Moreover, an isolated antibody may be substantially free of other cellular material and/or chemicals.

The terms "monoclonal antibody" or "monoclonal antibody composition" as used herein refer to a preparation of antibody molecules of single molecular composition. A monoclonal antibody composition displays a single binding specificity and affinity for a particular epitope.

The term "human antibody", as used herein, is intended to include antibodies having variable regions in which both the framework and CDR regions are derived from human germline immunoglobulin sequences. Furthermore, if the antibody contains a constant region, the constant region also is derived from human germline immunoglobulin sequences. The human antibodies according to at least some embodiments of the invention may include amino acid residues not encoded by human germline immunoglobulin sequences (e.g., mutations introduced by random or site-specific mutagenesis in vitro or by somatic mutation in vivo). However, the term "human antibody", as used herein, is not intended to include antibodies in which CDR sequences derived from the germline of another mammalian species, such as a mouse, have been grafted onto human framework sequences.

The term "human monoclonal antibody" refers to antibodies displaying a single binding specificity which have variable regions in which both the framework and CDR regions are derived from human germline immunoglobulin sequences. In one embodiment, the human monoclonal antibodies are produced by a hybridoma which includes a B cell obtained from a transgenic nonhuman animal, e.g., a transgenic mouse, having a genome comprising a human heavy chain transgene and a light chain transgene fused to an immortalized cell.

The term "recombinant human antibody", as used herein, includes all human antibodies that are prepared, expressed, created or isolated by recombinant means, such as (a) antibodies isolated from an animal (e.g., a mouse) that is transgenic or transchromosomal for human immunoglobulin genes or a hybridoma prepared therefrom (described further below), (b) antibodies isolated from a host cell transformed to express the human antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable regions in which the framework and CDR regions are derived from human germline immunoglobulin sequences. In certain embodiments, however, such recombinant human antibodies can be subjected to in vitro mutagenesis (or, when an animal transgenic for human Ig sequences is used, in vivo somatic mutagenesis) and thus the amino acid sequences of the VH and VL regions of the recombinant antibodies are sequences that, while derived from and related to human germline VH and VL sequences, may not naturally exist within the human antibody germline repertoire in vivo.

As used herein, "isotype" refers to the antibody class (e.g., IgM or IgG1) that is encoded by the heavy chain constant region genes.

The phrases "an antibody recognizing an antigen" and "an antibody specific for an antigen" are used interchangeably herein with the term "an antibody which binds specifically to an antigen."

As used herein, an antibody that "specifically binds" to human KRTCAP3, FAM26F, MGC52498, FAM70A, TMEM154 proteins or polypeptides is intended to refer to an antibody that binds to human KRTCAP3, FAM26F, MGC52498, FAM70A, TMEM154 proteins or polypeptides optionally one with a KD of 5X10 -8 M or less, 3X10 - 8 M or less, or 1X.10 -9 M or less.

The term "K-assoc" or "Ka", as used herein, is intended to refer to the association rate of a particular antibody-antigen interaction, whereas the term "Kdiss" or "Kd," as used herein, is intended to refer to the dissociation rate of a particular antibody-antigen interaction. The term "KD", as used herein, is intended to refer to the dissociation constant, which is obtained from the ratio of Kd to Ka (i.e., Kd/Ka) and is expressed as a molar concentration (M). KD values for antibodies can be determined using methods well established in the art. A preferred method for determining the KD of an antibody is by using surface Plasmon resonance, optionally using a biosensor system such as a Biacore® system.

As used herein, the term "high affinity" for an IgG antibody refers to an antibody having a KD of 10⁻⁸ M or less, 10⁻⁹ M or less or 10⁻¹⁰ M or less for a target antigen. However, "high affinity" binding can vary for other antibody isotypes. For example, "high affinity" binding for an IgM isotype refers to an antibody having a KD of 10⁻⁷ M or less, or 10⁻⁸ M or less.

As used herein, the term "tail" refers to a peptide sequence at the end of an amino acid sequence that is unique to a splice variant according to the present invention. Therefore, a splice variant having such a tail may optionally be considered as a chimera, in that at least a first portion of the splice variant is typically highly homologous (often 100% identical) to a portion of the corresponding known protein, while at least a second portion of the variant comprises the tail.

As used herein, the term "head" refers to a peptide sequence at the beginning of an amino acid sequence that is unique to a splice variant according to the present invention. Therefore, a splice variant having such a head may optionally be considered as a chimera, in that at least a first portion of the splice variant comprises the head, while at least a second portion is typically highly homologous (often 100% identical) to a portion of the corresponding known protein.

As used herein, the term "an edge portion" refers to a connection between two portions of a splice variant according to the present invention that were not joined in the wild type or known protein. An edge may optionally arise due to a join between the above "known protein" portion of a variant and the tail, for example, and/or may occur if an internal portion of the wild type sequence is no longer present, such that two portions of the sequence are now contiguous in the splice variant that were not contiguous in the known protein. A "bridge" may optionally be an edge portion as described above, but may also include a join between a head and a "known protein" portion of a variant, or a join between a tail and a "known protein" portion of a variant, or a join between an insertion and a "known protein" portion of a variant.

In some embodiments, a bridge between a tail or a head or a unique insertion, and a "known protein" portion of a variant, comprises at least about 10 amino acids, or in some embodiments at least about 20 amino acids, or in some embodiments at least about 30 amino acids, or in some embodiments at least about 40 amino acids, in which at least one amino acid is from the tail/head/insertion and at least one amino acid is from the "known protein" portion of a variant. In some embodiments, the bridge may comprise any number of amino acids from about 10 to about 40 amino acids (for example, 10, 11, 12, 13...37, 38, 39, 40 amino acids in length, or any number in between).

It should be noted that a bridge cannot be extended beyond the length of the sequence in either direction, and it should be assumed that every bridge description is to be read in such manner that the bridge length does not extend beyond the sequence itself.

Furthermore, bridges are described with regard to a sliding window in certain contexts below. For example, certain descriptions of the bridges feature the following format: a bridge between two edges (in which a portion of the known protein is not present in the variant) may optionally be described as follows: a bridge portion of CONTIG-NAME_P1 (representing the name of the protein), comprising a polypeptide having a length "n", wherein n is at least about 10 amino acids in length, optionally at least about 20 amino acids, at least about 30 amino acids, at least about 40 amino acids, or at least about 50 amino acids in length, wherein at least two amino acids comprise XX (2 amino acids in the center of the bridge, one from each end of the edge), having a structure as follows (numbering according to the sequence of CONTIG-NAME_P1): a sequence starting from any of amino acid numbers 49-x to 49 (for example); and ending at any of amino acid numbers 50 + ((n-2) - x) (for example), in which x varies from 0 to n-2. In this example, it should also be read as including bridges in which n is any number of amino acids between 10-50 amino acids in length. Furthermore, the bridge polypeptide cannot extend beyond the sequence, so it should be read such that 49-x (for example) is not less than 1, nor 50 + ((n-2) - x) (for example) greater than the total sequence length.

The term *"cancer"* as used herein should be understood to encompass any neoplastic disease (whether invasive or metastatic) which is characterized by abnormal and uncontrolled cell division causing malignant growth or tumor. Non-limiting examples of cancer which may be treated with a composition according to at least some embodiments of the present invention are solid tumors, sarcomas, hematological malignancies, including but not limited to breast cancer (e.g. breast carcinoma), cervical cancer, ovary cancer (ovary carcinoma), endometrial cancer, melanoma, bladder cancer (bladder carcinoma), lung cancer (e.g. adenocarcinoma and non-small cell lung cancer), pancreatic cancer (e.g. pancreatic carcinoma such as exocrine pancreatic carcinoma), colon cancer (e.g. colorectal carcinoma, such ascolon adenocarcinoma and colon adenoma), prostate cancer including the advanced disease, hematopoietic tumors of lymphoid lineage (e.g. leukemia, acute lymphocytic leukemia, chronic lymphocytic leukemia, B-cell lymphoma, Burkitt's lymphoma, multiple myeloma, Hodgkin's lymphoma, Non-Hodgkin's lymphoma, anti CD20 (i.e. Rituximab) resistant lymphoma), myeloid leukemia (for example, acute myelogenous leukemia (AML), chronic myelogenous leukemia), thyroid cancer, thyroid follicular cancer, myelodysplastic syndrome (MDS), tumors of mesenchymal origin (e.g. fibrosarcomas and rhabdomyosarcomas), melanoma, uveal melanoma, teratocarcinoma, neuroblastoma, glioma, glioblastoma, benign tumor of the skin (e.g. keratoacanthomas), renal cancer, anaplastic large-cell lymphoma, esophageal squamous cells carcinoma, hepatocellular carcinoma, follicular dendritic cell carcinoma, intestinal cancer, muscle-invasive cancer, seminal vesicle tumor, epidermal carcinoma, spleen cancer, bladder cancer, head and neck cancer, stomach cancer, liver cancer, bone cancer, brain cancer, cancer of the retina, biliary cancer, small bowel cancer, salivary gland cancer, cancer of uterus, cancer of testicles, cancer of connective tissue, prostatic hypertrophy, myelodysplasia, Waldenstrom's macroglobinaemia, nasopharyngeal, neuroendocrine cancer, myelodysplastic syndrome, mesothelioma, angiosarcoma, Kaposi's sarcoma, carcinoid, oesophagogastric, fallopian tube cancer, peritoneal cancer, papillary serous mullerian cancer, malignant ascites, gastrointestinal stromal tumor (GIST), and a hereditary cancer syndrome such as Li-Fraumeni syndrome and Von Hippel-Lindau syndrome (VHL).

With regard to ovarian cancer, the disease is selected from the group including but not limited to primary and metastatic cancer of the ovary, including epithelial ovarian cancer such as serous, mucinous, endometroid, clear cell, mixed epithelial, undifferentiated carcinomas and Brenner tumor, as well as other non-epithelial neoplasms of the ovary, including germ cell malignancies.

With regard to breast cancer, the disease is selected from the group including but not limited to primary and metastatic cancer of the breast, including mammary carcinomas such as Infiltrating Ductal carcinoma, Ductal carcinoma in-situ, Infiltrating Lobular carcinoma, Lobular carcinoma in-situ, Inflammatory breast cancer, Paget's disease of the breast, and other non-epithelial neoplasms of the breast, including fibrosarcomas, leiomyosarcomas, rhapdomyosarcomas, angiosarcomas, cystosarcoma phyllodes.

With regard to lung cancer, the disease is selected from the group consisting of but not limited to squamous cell lung carcinoma, lung adenocarcinoma, carcinoid, small cell lung cancer or non-small cell lung cancer.

With regard to liver cancer, the disease is selected from the group consisting of but not limited to primary and metastatic cancers of the liver and intrahepatic bile duct, including hepatocellular carcinoma, cholangiocarcinoma, hepatic angiosarcoma and hepatoblastoma.

With regard to renal cancer, the disease is selected from the group consisting of but not limited to primary and metastatic cancer of the kidney, including renal cell carcinoma (i.e. renal adenocarcinoma), as well as other non-epithelial neoplasms of the ovary, including nephroblastoma (i.e. Wilm's tumor), transitional cell neoplasms of the renal pelvis, and various sarcomas of renal origin.

With regard to colon cancer, the disease is selected from the group consisting of but not limited to primary and metastatic cancer of the colon, including adenocarcinoma, mucinous carcinoma (including signet ring cell-type and medullary), adenosquamous carcinoma, carcinoid, small cell carcinoma, squamous cell carcinoma, undifferentiated carcinoma, as well as other non-epithelial neoplasms of the colon, including lymphoma, melanoma and sarcoma.

With regard to pancreatic cancer, the disease is selected from the group consisting of but not limited to primary and metastatic cancers of the exocrine pancreas, including adenocarcinoma, serous and mucinous cystadenocarcinomas, acinar cell carcinoma, undifferentiated carcinoma, pancreatoblastoma and neuroendocrine tumors such as insulinoma.

With regard to melanoma, the disease is selected from the group consisting of but not limited to primary and metastatic malignant melanoma, including cutaneous melanoma such as superficial spreading melanoma, nodular melanoma, acral lentiginous melanoma and lentigo maligna melanoma, as well as mucosal melanoma, intraocular melanoma, desmoplastic/neurotropic melanoma and melanoma of soft parts (clear cell sarcoma).

With regard to prostate cancer, the disease is selected from the group consisting of but not limited to primary and metastatic cancer of the prostate, including prostatic intraepithelial neoplasia, atypical adenomatous hyperplasia, prostate adenocarcinoma, mucinous or signet ring tumor, adenoid cystic carcinoma, prostatic duct carcinoma, carcinoid and small-cell undifferentiated cancer.

As used herein the term *"hematological malignancies"* refers to acute lymphocytic leukemia, chronic lymphocytic leukemia (CLL), acute lymphoblastic leukemia, acute myelogenous leukemia, chronic myelogenous leukemia, multiple myeloma, B-cell lymphoma, such as Hodgkin's lymphoma, non-Hodgkin's lymphoma (NHL), anti CD20 (i.e. Rituximab) resistant lymphoma, low grade/follicular NHL, small cell lymphocytic (SL) NHL, small cell NHL, grade I small cell follicular NHL, grade II mixed small and large cell follicular NHL, grade III large cell follicular NHL, large cell NHL, Diffuse Large B-Cell NHL, intermediate grade diffuse NHL, high grade immunoblastic NHL, high grade lymphoblastic NHL, high grade small non- cleaved cell NHL, bulky disease NHL, mantle cell lymphoma, Mucosa-Associated Lymphatic Tissue lymphoma (MALT), Burkitt lymphoma, Mediastinal large B cell lymphoma, Nodal marginal zone B cell lymphoma (NMZL), Splenic marginal zone lymphoma (SMZL), Extranodal marginal zone B cell lymphoma, Intravascular large B cell lymphoma, Primary effusion lymphoma, Lymphomatoid granulomatosis, B-cell prolymphocytic leukemia, Precursor B lymphoblastic leukemia, Hairy cell leukemia, AIDS-related lymphoma and Waldenstrom's Macroglobulinernia.

The term "immune related condition" as used herein will encompass any disease disorder or condition selected from the group including but not limited to multiple sclerosis; psoriasis; rheumatoid arthritis; psoriatic arthritis, systemic lupus erythematosus (SLE); ulcerative colitis; Crohn's disease; benign lymphocytic angiitis, thrombocytopenic purpura, idiopathic thrombocytopenia, idiopathic autoimmune hemolytic anemia, pure red cell aplasia, Sjogren's syndrome, rheumatic disease, connective tissue disease, inflammatory rheumatism, degenerative rheumatism, extra-articular rheumatism, juvenile rheumatoid arthritis, arthritis uratica, muscular rheumatism, chronic polyarthritis, cryoglobulinemic vasculitis, ANCA-associated vasculitis, antiphospholipid syndrome, myasthenia gravis, autoimmune haemolytic anaemia, Guillian-Barre syndrome, chronic immune polyneuropathy, autoimmune thyroiditis, insulin dependent diabetes mellitus, type I diabetes, Addison's disease, membranous glomerulonephropathy, Goodpasture's disease, autoimmune gastritis, pernicious anaemia, pemphigus vulgarus, cirrhosis, primary biliary cirrhosis, dermatomyositis, polymyositis, fibromyositis, myogelosis, celiac disease, immunoglobulin A nephropathy, Henoch-Schonlein purpura, Evans syndrome, atopic dermatitis, psoriasis, psoriasis arthropathica, Graves' disease, Graves' ophthalmopathy, scleroderma, systemic scleroderma, asthma, allergy, primary biliary cirrhosis, Hashimoto's thyroiditis, primary myxedema, sympathetic ophthalmia, autoimmune uveitis, hepatitis, chronic action hepatitis, collagen diseases, ankylosing spondylitis, periarthritis humeroscapularis, panarteritis nodosa, chondrocalcinosis, Wegener's granulomatosis, microscopic polyangiitis, chronic urticaria, bullous skin disorders, pemphigoid, atopic eczema, Devic's disease, childhood autoimmune hemolytic anemia, Refractory or chronic Autoimmune Cytopenias, Prevention of development of Autoimmune Anti-Factor VIII Antibodies in Acquired Hemophilia A, Cold Agglutinin Disease, Neuromyelitis Optica, Stiff Person Syndrome, gingivitis, periodontitis, pancreatitis, myocarditis, vasculitis, gastritis, gout, gouty arthritis, and inflammatory skin disorders, selected from the group consisting of psoriasis, atopic dermatitis, eczema, rosacea, urticaria, and acne, normocomplementemic urticarial vasculitis, pericarditis, myositis, anti-synthetase syndrome, scleritis, macrophage activation syndrome, Bechet's Syndrome, PAPA Syndrome, Blau's Syndrome, gout, adult and juvenile Still's disease, cryropyrinopathy, Muckle-Wells syndrome, familial cold-induced auto-inflammatory syndrome, neonatal onset multisystemic inflammatory disease, familial Mediterranean fever, chronic infantile neurologic, cutaneous and articular syndrome, systemic juvenile idiopathic arthritis, Hyper IgD syndrome, Schnitzler's syndrome, and TNF receptor-associated periodic syndrome (TRAPS), inflammatory bowel disease, Good pasture's syndrome, pernicious anemia, autoimmune atrophic gastritis, ulceratis colitis, mixed connective tissue disease, panarteritis nodosa, progressive systemic scleroderma, peptic ulcers, ulcers, chronic bronchitis, acute lung injury, pulmonary inflammation, airway hyper-responsiveness, septic shock, inflammatory skin disorders, myogelosis, chondrocalcinosis, thyroditis, allergic oedema, granulomas, immune disorders associated with graft transplantation rejection, such as acute and chronic rejection of organ transplantation, allogenic stem cell transplantation, autologous stem cell transplantation, bone marrow tranplantation, and graft versus host disease.

As used herein the term "***treatment***" refers to care provided to relieve illness and refers to both a therapeutic treatment or prophylactic/preventative measures, wherein the objective is to prevent or slow down (lessen) the targeted pathologic condition or disorder. Those in need of treatment include those already with the disorder as well as those prone to have the disorder or those in whom the disorder is to be prevented. The term treatment as used herein refers also to "maintenance therapy", which is a treatment that is given to keep a pathologic condition or disorder from coming back after it has disappeared following the initial therapy.

The term "therapeutically effective amount" refers to an amount of agent according to the present invention that is effective to treat a disease or disorder in a mammal.

As used herein the term "***diagnosis***" refers to the process of identifying a medical condition or disease by its signs, symptoms, and in particular from the results of various diagnostic procedures, including e.g. detecting the expression of the nucleic acids or polypeptides according to at least some embodiments of the invention in a biological sample (e.g. in cells, tissue or serum, as defined below) obtained from an individual. Furthermore, as used herein the term "diagnosis" encompasses screening for a disease, detecting a presence or a severity of a disease, distinguishing a disease from other diseases including those diseases that may feature one or more similar or identical symptoms, providing prognosis of a disease, monitoring disease progression or relapse, as well as assessment of treatment efficacy and/or relapse of a disease, disorder or condition, as well as selecting a therapy and/or a treatment for a disease, optimization of a given therapy for a disease, monitoring the treatment of a disease, and/or predicting the suitability of a therapy for specific patients or subpopulations or determining the appropriate dosing of a therapeutic product in patients or subpopulations. The diagnostic procedure can be performed in vivo or in vitro. It should be noted that a "biological sample obtained from the subject" may also optionally comprise a sample that has not been physically removed from the subject.

As used herein the term ***"combination therapy"*** refers to the simultaneous or consecutive administration of two or more medications or types of therapy to treat a single disease. In particular, the term refers to the use of any of the polypeptides, polynucleotides, antibodies or pharmaceutical compositions according to at least some embodiments of the invention in combination with at least one additional medication or therapy. Thus, treatment of a disease using the agents according to at least some embodiments of the present invention may be combined with therapies well known in the art that include, but are not limited to, radiation therapy, antibody therapy, chemotherapy or surgery or in combination therapy with other biological agents, conventional drugs, anti-cancer agents, immunosuppressants, cytotoxic drugs for cancer, chemotherapeutic agents. According to at least some embodiments, treatment of Multiple Myeloma using the agents according to at least some embodiments of the present invention may be combined with an agent including but not limited to Melphalan, prednisone, thalidomide (MPT), or combination Bortezomib (Velcade), melphalan, prednisone (VMP) or a combination of Lenalidomide plus low-dose dexamethasone.

According to at least some embodiments, treatment of ovarian cancer using the agents according to at least some embodiments of the present invention may be combined with an agent including but not limited to paclitaxol and cisplatin.

According to at least some embodiments, treatment of rheumatoid arthritis disorder using the agents according to at least some embodiments of the present invention may be combined with but not limited to a first-line combination treatment with a drug such as aspirin and cortisone (corticosteroids), which are used to reduce pain and inflammation, and one or more second-line combination drugs, such as gold, methotrexate, and hydroxychloroquine (Plaquenil), promote disease remission and prevent progressive joint destruction. According to at least some embodiments, treatment of rheumatoid arthritis disorder may optionally feature an agent according to the present invention combined with an agent including but not limited to methotrexate and rituximab.

As used herein, the term "subject" includes any human or nonhuman animal. The term "nonhuman animal" includes all vertebrates, e.g., mammals and non-mammals, such as nonhuman primates, sheep, dogs, cats, horses, cows, chickens, amphibians, reptiles, etc.

Various aspects of the invention are described in further detail in the following subsections.

### NUCLEIC ACIDS

A "nucleic acid fragment" or an "oligonucleotide" or a "polynucleotide" are used herein interchangeably to refer to a polymer of nucleic acid residues. A polynucleotide sequence according to at least some embodiments of the present invention refers to a single or double stranded nucleic acid sequences which is isolated and provided in the form of an RNA sequence, a complementary polynucleotide sequence (cDNA), a genomic polynucleotide sequence and/or a composite polynucleotide sequences (e.g., a combination of the above).

Thus, the present invention encompasses nucleic acid sequences described hereinabove; fragments thereof, sequences hybridizable therewith, sequences homologous thereto [e.g., at least 90%, at least 95, 96, 97, 98 or 99 % or more identical to the nucleic acid sequences set forth herein], sequences encoding similar polypeptides with different codon usage, altered sequences characterized by mutations, such as deletion, insertion or substitution of one or more nucleotides, either naturally occurring or man induced, either randomly or in a targeted fashion. The present invention also encompasses homologous nucleic acid sequences (i.e., which form a part of a polynucleotide sequence according to at least some embodiments of the present invention), which include sequence regions unique to the polynucleotides according to at least some embodiments of the present invention.

In cases where the polynucleotide sequences according to at least some embodiments of the present invention encode previously unidentified polypeptides, the present invention also encompasses novel polypeptides or portions thereof, which are encoded by the isolated polynucleotide and respective nucleic acid fragments thereof described hereinabove.

Thus, the present invention also encompasses polypeptides encoded by the polynucleotide sequences according to at least some embodiments of the present invention. The present invention also encompasses homologues of these polypeptides, such homologues can be at least 90 %, at least 95, 96, 97, 98 or 99 % or more homologous to the amino acid sequences set forth below, as can be determined using BlastP software of the National Center of Biotechnology Information (NCBI) using default parameters. Finally, the present invention also encompasses fragments of the above described polypeptides and polypeptides having mutations, such as deletions, insertions or substitutions of one or more amino acids, either naturally occurring or man induced, either randomly or in a targeted fashion.

Oligonucleotides designed for carrying out the methods according to at least some embodiments of the present invention for any of the sequences provided herein (designed as described above) can be generated according to any oligonucleotide synthesis method known in the art such as enzymatic synthesis or solid phase synthesis. Equipment and reagents for executing solid-phase synthesis are commercially available from, for example, Applied Biosystems. Any other means for such synthesis may also be employed; the actual synthesis of the oligonucleotides is well within the capabilities of one skilled in the art.

Oligonucleotides used according to this aspect according to at least some embodiments of the present invention are those having a length selected from a range of about 10 to about 200 bases, optionally about 15 to about 150 bases, about 20 to about 100 bases, or about 20 to about 50 bases.

The oligonucleotides according to at least some embodiments of the present invention may comprise heterocyclic nucleosides consisting of prunes and the pyrimidines bases, bonded in a 3' to 5' phosphodiester linkage.

### PEPTIDES

The terms "polypeptide," "peptide" and "protein" are used interchangeably herein to refer to a polymer of amino acid residues. The terms apply to amino acid polymers in which one or more amino acid residue is an analog or mimetic of a corresponding naturally occurring amino acid, as well as to naturally occurring amino acid polymers. Polypeptides can be modified, e.g., by the addition of carbohydrate residues to form glycoproteins. The terms "polypeptide," "peptide" and "protein" include glycoproteins, as well as non-glycoproteins.

Polypeptide products can be biochemically synthesized such as by employing standard solid phase techniques. Such methods include exclusive solid phase synthesis, partial solid phase synthesis methods, fragment condensation, classical solution synthesis. These methods are optionally used when the peptide is relatively short (i.e., 10 kDa) and/or when it cannot be produced by recombinant techniques (i.e., not encoded by a nucleic acid sequence) and therefore involves different chemistry.

Solid phase polypeptide synthesis procedures are well known in the art and further described by John Morrow Stewart and Janis Dillaha Young, Solid Phase Peptide Syntheses (2nd Ed., Pierce Chemical Company, 1984).

Synthetic polypeptides can be purified by preparative high performance liquid chromatography [Creighton T. (1983) Proteins, structures and molecular principles. WH Freeman and Co. N.Y.] and the composition of which can be confirmed via amino acid sequencing.

In cases where large amounts of a polypeptide are desired, it can be generated using recombinant techniques such as described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

It will be appreciated that peptides according to at least some embodiments of the present invention may be degradation products, synthetic peptides or recombinant peptides as well as peptidomimetics, typically, synthetic peptides and peptoids and semipeptoids which are peptide analogs, which may have, for example, modifications rendering the peptides more stable while in a body or more capable of penetrating into cells. Such modifications include, but are not limited to N terminus modification, C terminus modification, peptide bond modification, including, but not limited to, CH2-NH, CH2-S, CH2-S=O, O=C-NH, CH2-O, CH2-CH2, S=C-NH, CH=CH or CF=CH, backbone modifications, and residue modification. Methods for preparing peptidomimetic compounds are well known in the art and are specified, for example, in Quantitative Drug Design, C.A. Ramsden Gd., Chapter 17.2, F. Choplin Pergamon Press (1992), which is incorporated by reference as if fully set forth herein. Further details in this respect are provided hereinunder.

Peptide bonds (-CO-NH-) within the peptide may be substituted, for example, by N-methylated bonds (-N(CH3)-CO-), ester bonds (-C(R)H-C-O-O-C(R)-N-), ketomethylen bonds (-CO-CH2-), α-aza bonds (-NH-N(R)-CO-), wherein R is any alkyl, e.g., methyl, carba bonds (-CH2-NH-), hydroxyethylene bonds (-CH(OH)-CH2-), thioamide bonds (-CS-NH-), olefinic double bonds (-CH=CH-), retro amide bonds (-NH-CO-), peptide derivatives (-N(R)-CH2-CO-), wherein R is the "normal" side chain, naturally presented on the carbon atom.

These modifications can occur at any of the bonds along the peptide chain and even at several (2-3) at the same time.

Natural aromatic amino acids, Trp, Tyr and Phe, may be substituted by synthetic non-natural acid such as Phenylglycine, TIC, naphthylelanine (Nol), ring-methylated derivatives of Phe, halogenated derivatives of Phe or o-methyl-Tyr.

In addition to the above, the peptides according to at least some embodiments of the present invention may also include one or more modified amino acids or one or more non-amino acid monomers (e.g. fatty acids, complex carbohydrates etc).

As used herein in the specification and in the claims section below the term "amino acid" or "amino acids" is understood to include the 20 naturally occurring amino acids; those amino acids often modified post-translationally in vivo, including, for example, hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids.

The peptides according to at least some embodiments of the present invention might include one or more non-natural or natural polar amino acids, including but not limited to serine and threonine which are capable of increasing peptide solubility due to their hydroxyl-containing side chain.

The peptides according to at least some embodiments of the present invention can be biochemically synthesized such as by using standard solid phase techniques. These methods include exclusive solid phase synthesis, partial solid phase synthesis methods, fragment condensation, classical solution synthesis. These methods are optionally used when the peptide is relatively short (i.e., 10 kDa) and/or when it cannot be produced by recombinant techniques (i.e., not encoded by a nucleic acid sequence) and therefore involves different chemistry.

Solid phase peptide synthesis procedures are well known in the art and further described by John Morrow Stewart and Janis Dillaha Young, Solid Phase Peptide Syntheses (2nd Ed., Pierce Chemical Company, 1984).

Synthetic peptides can be purified by preparative high performance liquid chromatography [Creighton T. (1983) Proteins, structures and molecular principles. WH Freeman and Co. N.Y.] and the composition of which can be confirmed via amino acid sequencing.

In cases where large amounts of the peptides according to at least some embodiments of the present invention are desired, the peptides can be generated using recombinant techniques such as described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

### RECOMBINANT EXPRESSION OF POLYPEPTIDES

Methods for introduction of heterologous polynucleotides into mammalian cells are well known in the art and include dextran-mediated transfection, calcium phosphate precipitation, polybrene-mediated transfection, protoplast fusion, electroporation, encapsulation of the polynucleotide(s) in liposomes, biolistic injection and direct microinjection of the DNA into nuclei. In addition, nucleic acid molecules may be introduced into mammalian cells by viral vectors. Methods of transforming cells are well known in the art. See, e.g., U.S. Patent Nos. 4,399,216, 4,912,040, 4,740,461, and 4,959,455 (which patents are hereby incorporated herein by reference). Methods of transforming plant cells are well known in the art, including, e.g., Agrobacterium-mediated transformation, biolistic transformation, direct injection, electroporation and viral transformation. Methods of transforming bacterial and yeast cells are also well known in the art.

Mammalian cell lines available as hosts for expression are well known in the art and include many immortalized cell lines available from e.g. the American Type Culture Collection (ATCC). These include, inter alia, Chinese hamster ovary (CHO) cells, NS0, SP2 cells, HEK-293T cells, NIH-3T3 cells, HeLa cells, baby hamster kidney (BHK) cells, monkey kidney cells (COS), human hepatocellular carcinoma cells (e.g., Hep G2), A549 cells, 3T3 cells, and a number of other cell lines. Mammalian host cells include human, mouse, rat, dog, monkey, pig, goat, bovine, horse and hamster cells. Cell lines of particular preference are selected through determining which cell lines have high expression levels. Other cell lines that may be used are insect cell lines, such as Sf9 cells, amphibian cells, bacterial cells, plant cells and fungal cells. When recombinant expression vectors encoding the polypeptides according to at least some embodiments of the invention or fragments thereof are introduced into mammalian host cells, the polypeptides are produced by culturing the host cells for a period of time sufficient to allow for expression of the polypeptide in the host cells or, more preferably, secretion of the polypeptide into the culture medium in which the host cells are grown. Polypeptides can be recovered from the culture medium using standard protein purification methods. Plant host cells include, e.g., Nicotiana, Arabidopsis, duckweed, corn, wheat, potato, etc. Bacterial host cells include E. coli and Streptomyces species. Yeast host cells include Schizosaccharomyces pombe, Saccharomyces cerevisiae and Pichia pastoris.

Further, expression of the polypeptides according to at least some embodiments of the invention (or other moieties derived therefrom) from production cell lines can be enhanced using a number of known techniques. For example, the glutamine synthetase gene expression system (the GS system) is a common approach for enhancing expression under certain conditions. The GS system is discussed in whole or part in connection with European Patent Nos. 0 216 846, 0 256 055, 0 338 841 and 0 323 997.

It is likely that polypeptides expressed by different cell lines or in transgenic animals will have different glycosylation patterns. However, all polypeptides encoded by the nucleic acid molecules provided herein, or comprising the amino acid sequences provided herein are part of the instant invention, regardless of their glycosylation pattern.

### VECTORS

According to at least some embodiments, the invention provides vectors comprising the nucleic acid molecules that encode the polypeptides, fusion proteins, modified polypeptides, and polypeptide fragments of at least some embodiments the invention.

To express the polypeptides according to at least some embodiments of the invention, or fragments thereof, DNAs encoding partial or full-length polypeptides, obtained as described above, are inserted into expression vectors such that the genes are operatively linked to transcriptional and translational control sequences. Expression vectors include plasmids, retroviruses, adenoviruses, adeno-associated viruses (AAV), plant viruses such as cauliflower mosaic virus, tobacco mosaic virus, cosmids, YACs, EBV derived episomes, and the like. The gene is ligated into a vector such that transcriptional and translational control sequences within the vector serve their intended function of regulating the transcription and translation of the gene. The expression vector and expression control sequences are chosen to be compatible with the expression host cell used. The gene is inserted into the expression vector by standard methods (e.g., ligation of complementary restriction sites on the gene fragment and vector, or blunt end ligation if no restriction sites are present).

A convenient vector is one that encodes a functionally complete sequence, with appropriate restriction sites engineered so that any sequence can be easily inserted and expressed, as described above. Polyadenylation and transcription termination occur at native chromosomal sites downstream of the coding regions. The recombinant expression vector can also encode a signal peptide that facilitates secretion of the polypeptide from a host cell. The gene may be cloned into the vector such that the signal peptide is linked in-frame to the amino terminus of the gene.

In addition to the nucleic acid according to at least some embodiments of the invention, the recombinant expression vectors carry regulatory sequences that control the expression of the gene in a host cell. It will be appreciated by those skilled in the art that the design of the expression vector, including the selection of regulatory sequences may depend on such factors as the choice of the host cell to be transformed, the level of expression of protein desired, etc. Preferred regulatory sequences for mammalian host cell expression include viral elements that direct high levels of protein expression in mammalian cells, such as promoters and/or enhancers derived from retroviral LTRs, cytomegalovirus (CMV) (such as the CMV promoter/enhancer), Simian Virus 40 (SV40) (such as the SV40 promoter/enhancer), adenovirus, (e.g., the adenovirus major late promoter (AdMLP)), polyoma and strong mammalian promoters such as native immunoglobulin and actin promoters. For further description of viral regulatory elements, and sequences thereof, see e.g., U.S. Pat. Nos. 5,168,062, 4,510,245, and 4,968,615, each of which is hereby incorporated by reference. Methods of expressing polypeptides in bacterial cells or fungal cells, e.g., yeast cells, are also well known in the art.

In addition to the nucleic acids according to at least some embodiments of the invention and regulatory sequences, the recombinant expression vectors according to at least some embodiments of the invention may carry additional sequences, such as sequences that regulate replication of the vector in host cells (e.g., origins of replication) and selectable marker genes. The selectable marker gene facilitates selection of host cells into which the vector has been introduced (see, e.g., U.S. Pat. Nos. 4,399,216, 4,634,665 and 5,179,017). For example, typically the selectable marker gene confers resistance to drugs, such as G418, hygromycin or methotrexate, on a host cell into which the vector has been introduced. Preferred selectable marker genes include the dihydrofolate reductase (DHFR) gene, the neo gene (for G418 selection), and the glutamate synthetase gene.

### PROTEIN MODIFICATIONS

### FUSION PROTEINS

The present invention encompasses fusion proteins (conjugates) for use in therapy, comprising the TMEM154 soluble portions including the ectodomain or portions or variants thereof. For example the invention encompasses conjugates wherein the ECD of the TMEM154 is attached to an immunoglobulin or fragment thereof. The invention contemplates the use thereof for treating cancer and/or immune related conditions, diseases or disorders described herein.

According to at least some embodiments, a fusion protein may be prepared from a protein according to at least some embodiments of the invention by fusion with a portion of an immunoglobulin comprising a constant region of an immunoglobulin. Optionally, the portion of the immunoglobulin comprises a heavy chain constant region which is optionally and more preferably a human heavy chain constant region. The heavy chain constant region is optionally an IgG heavy chain constant region, and optionally an Fc chain, or an IgG Fc fragment that comprises CH2 and CH3 domains. Although any IgG subtype may optionally be used, the IgG1 subtype is preferred. The Fc chain may optionally be a known or "wild type" Fc chain, or alternatively may be mutated. Non-limiting, illustrative, exemplary types of mutations are described in US Patent Application No. 20060034852, published on February 16, 2006, hereby incorporated by reference as if fully set forth herein. The term "Fc chain" also optionally comprises any type of Fc fragment.

Several of the specific amino acid residues that are important for antibody constant region-mediated activity in the IgG subclass have been identified. Inclusion, substitution or exclusion of these specific amino acids therefore allows for inclusion or exclusion of specific immunoglobulin constant region-mediated activity. Furthermore, specific changes may result in aglycosylation for example and/or other desired changes to the Fc chain. At least some changes may optionally be made to block a function of Fc which is considered to be undesirable, such as an undesirable immune system effect, as described in greater detail below.

Non-limiting, illustrative examples of mutations to Fc which may be made to modulate the activity of the fusion protein include the following changes (given with regard to the Fc sequence nomenclature as given by Kabat, from Kabat EA et al: Sequences of Proteins of Immunological Interest. US Department of Health and Human Services, NIH, 1991): 220C - > S; 233-238 ELLGGP - > EAEGAP; 265D - > A, preferably in combination with 434N -> A; 297N - > A (for example to block N-glycosylation); 318-322 EYKCK - > AYACA; 330-331AP - > SS; or a combination thereof (see for example M. Clark, "Chemical Immunol and Antibody Engineering", pp 1-31 for a description of these mutations and their effect). The construct for the Fc chain which features the above changes optionally and preferably comprises a combination of the hinge region with the CH2 and CH3 domains.

The above mutations may optionally be implemented to enhance desired properties or alternatively to block non-desired properties. For example, aglycosylation of antibodies was shown to maintain the desired binding functionality while blocking depletion of T-cells or triggering cytokine release, which may optionally be undesired functions (see M. Clark, "Chemical Immunol and Antibody Engineering", pp 1-31). Substitution of 331 proline for serine may block the ability to activate complement, which may optionally be considered an undesired function (see M. Clark, "Chemical Immunol and Antibody Engineering", pp 1-31). Changing 330alanine to serine in combination with this change may also enhance the desired effect of blocking the ability to activate complement.

Residues 235 and 237 were shown to be involved in antibody-dependent cell-mediated cytotoxicity (ADCC), such that changing the block of residues from 233-238 as described may also block such activity if ADCC is considered to be an undesirable function.

Residue 220 is normally a cysteine for Fc from IgG1, which is the site at which the heavy chain forms a covalent linkage with the light chain. Optionally, this residue may be changed to a serine, to avoid any type of covalent linkage (see M. Clark, "Chemical Immunol and Antibody Engineering", pp 1-31).

The above changes to residues 265 and 434 may optionally be implemented to reduce or block binding to the Fc receptor, which may optionally block undesired functionality of Fc related to its immune system functions (see "Binding site on Human IgG1 for Fc Receptors", Shields et al, Vol 276, pp 6591-6604, 2001).

The above changes are intended as illustrations only of optional changes and are not meant to be limiting in any way. Furthermore, the above explanation is provided for descriptive purposes only, without wishing to be bound by a single hypothesis.

### ADDITION OF GROUPS

If a protein according to the present invention is a linear molecule, it is possible to place various functional groups at various points on the linear molecule which are susceptible to or suitable for chemical modification. Functional groups can be added to the termini of linear forms of the protein according to at least some embodiments of the invention. In some embodiments, the functional groups improve the activity of the protein with regard to one or more characteristics, including but not limited to, improvement in stability, penetration (through cellular membranes and/or tissue barriers), tissue localization, efficacy, decreased clearance, decreased toxicity, improved selectivity, improved resistance to expulsion by cellular pumps, and the like. For convenience sake and without wishing to be limiting, the free N-terminus of one of the sequences contained in the compositions according to at least some embodiments of the invention will be termed as the N-terminus of the composition, and the free C-terminal of the sequence will be considered as the C-terminus of the composition. Either the C-terminus or the N-terminus of the sequences, or both, can be linked to a carboxylic acid functional groups or an amine functional group, respectively.

Non-limiting examples of suitable functional groups are described in Green and Wuts, "Protecting Groups in Organic Synthesis", John Wiley and Sons, Chapters 5 and 7, 1991, the teachings of which are incorporated herein by reference. Preferred protecting groups are those that facilitate transport of the active ingredient attached thereto into a cell, for example, by reducing the hydrophilicity and increasing the lipophilicity of the active ingredient, these being an example for "a moiety for transport across cellular membranes".

These moieties can optionally be cleaved in vivo, either by hydrolysis or enzymatically, inside the cell. (Ditter et al., J. Pharm. Sci. 57:783 (1968); Ditter et al., J. Pharm. Sci. 57:828 (1968); Ditter et al., J. Pharm. Sci. 58:557 (1969); King et al., Biochemistry 26:2294 (1987); Lindberg et al., Drug Metabolism and Disposition 17:311 (1989); and Tunek et al., Biochem. Pharm. 37:3867 (1988), Anderson et al., Arch. Biochem. Biophys. 239:538 (1985) and Singhal et al., FASEB J. 1:220 (1987)). Hydroxyl protecting groups include esters, carbonates and carbamate protecting groups. Amine protecting groups include alkoxy and aryloxy carbonyl groups, as described above for N-terminal protecting groups. Carboxylic acid protecting groups include aliphatic, benzylic and aryl esters, as described above for C-terminal protecting groups. In one embodiment, the carboxylic acid group in the side chain of one or more glutamic acid or aspartic acid residue in a composition according to at least some embodiments of the present invention is protected, optionally with a methyl, ethyl, benzyl or substituted benzyl ester.

Non-limiting, illustrative examples of N-terminal protecting groups include acyl groups (-CO-R1) and alkoxy carbonyl or aryloxy carbonyl groups (-CO-O-R1), wherein R1 is an aliphatic, substituted aliphatic, benzyl, substituted benzyl, aromatic or a substituted aromatic group. Specific examples of acyl groups include but are not limited to acetyl, (ethyl)-CO-, n-propyl-CO-, iso-propyl-CO-, n-butyl-CO-, sec-butyl-CO-, t-butyl-CO-, hexyl, lauroyl, palmitoyl, myristoyl, stearyl, oleoyl phenyl-CO-, substituted phenyl-CO-, benzyl-CO- and (substituted benzyl)-CO-. Examples of alkoxy carbonyl and aryloxy carbonyl groups include CH3-O-CO-, (ethyl)-O-CO-, n-propyl-O-CO-, iso-propyl-O-CO-, n-butyl-O-CO-, sec-butyl-O-CO-, t-butyl-O-CO-, phenyl-O- CO-, substituted phenyl-O-CO- and benzyl-O-CO-, (substituted benzyl)- O-CO-, Adamantan, naphtalen, myristoleyl, toluen, biphenyl, cinnamoyl, nitrobenzoy, toluoyl, furoyl, benzoyl, cyclohexane, norbornane, or Z-caproic. In order to facilitate the N-acylation, one to four glycine residues can be present in the N-terminus of the molecule.

The carboxyl group at the C-terminus of the compound can be protected, for example, by a group including but not limited to an amide (i.e., the hydroxyl group at the C-terminus is replaced with -NH₂, -NHR₂ and -NR₂R₃) or ester (i.e. the hydroxyl group at the C-terminus is replaced with -OR₂). R₂ and R₃ are optionally independently an aliphatic, substituted aliphatic, benzyl, substituted benzyl, aryl or a substituted aryl group. In addition, taken together with the nitrogen atom, R₂ and R₃ can optionally form a C4 to C8 heterocyclic ring with from about 0-2 additional heteroatoms such as nitrogen, oxygen or sulfur. Non-limiting suitable examples of suitable heterocyclic rings include piperidinyl, pyrrolidinyl, morpholino, thiomorpholino or piperazinyl. Examples of C-terminal protecting groups include but are not limited to -NH₂, -NHCH₃, -N(CH₃)₂, -NH(ethyl), -N(ethyl)₂, -N(methyl) (ethyl), -NH(benzyl), -N(C1-C4 alkyl)(benzyl), -NH(phenyl), -N(C1-C4 alkyl) (phenyl), -OCH₃, -O-(ethyl), -O-(n-propyl), -O-(n-butyl), -O-(iso-propyl), -O-(sec- butyl), -O-(t-butyl), -O-benzyl and -O-phenyl.

### SUBSTITUTION BY PEPTIDOMIMETIC MOIETIES

A "peptidomimetic organic moiety" can optionally be substituted for amino acid residues in the composition of this invention both as conservative and as non-conservative substitutions. These moieties are also termed "non-natural amino acids" and may optionally replace amino acid residues, amino acids or act as spacer groups within the peptides in lieu of deleted amino acids. The peptidomimetic organic moieties optionally have steric, electronic or configurational properties similar to the replaced amino acid and such peptidomimetics are used to replace amino acids in the essential positions, and are considered conservative substitutions. However such similarities are not necessarily required. According to at least some embodiments of the present invention, one or more peptidomimetics are selected such that the composition at least substantially retains its physiological activity as compared to the native protein according to the present invention.

Peptidomimetics may optionally be used to inhibit degradation of the peptides by enzymatic or other degradative processes. The peptidomimetics can optionally be produced by organic synthetic techniques. Non-limiting examples of suitable peptidomimetics include D amino acids of the corresponding L amino acids, tetrazol (Zabrocki et al., J. Am. Chem. Soc. 110:5875-5880 (1988)); isosteres of amide bonds (Jones et al., Tetrahedron Lett. 29: 3853-3856 (1988)); LL-3-amino-2-propenidone-6-carboxylic acid (LL-Acp) (Kemp et al., J. Org. Chem. 50:5834-5838 (1985)). Similar analogs are shown in Kemp et al., Tetrahedron Lett. 29:5081-5082 (1988) as well as Kemp et al., Tetrahedron Lett. 29:5057-5060 (1988), Kemp et al., Tetrahedron Lett. 29:4935-4938 (1988) and Kemp et al., J. Org. Chem. 54:109-115 (1987). Other suitable but exemplary peptidomimetics are shown in Nagai and Sato, Tetrahedron Lett. 26:647-650 (1985); Di Maio et al., J. Chem. Soc. Perkin Trans., 1687 (1985); Kahn et al., Tetrahedron Lett. 30:2317 (1989); Olson et al., J. Am. Chem. Soc. 112:323-333 (1990); Garvey et al., J. Org. Chem. 56:436 (1990). Further suitable exemplary peptidomimetics include hydroxy-1,2,3,4-tetrahydroisoquinoline- 3-carboxylate (Miyake et al., J. Takeda Res. Labs 43:53-76 (1989)); 1,2,3,4-tetrahydro- isoquinoline-3-carboxylate (Kazmierski et al., J. Am. Chem. Soc. 133:2275-2283 (1991)); histidine isoquinolone carboxylic acid (HIC) (Zechel et al., Int. J. Pep. Protein Res. 43 (1991)); (2S, 3S)-methyl-phenylalanine, (2S, 3R)-methyl-phenylalanine, (2R, 3S)-methyl- phenylalanine and (2R, 3R)-methyl-phenylalanine (Kazmierski and Hruby, Tetrahedron Lett. (1991)).

Exemplary, illustrative but non-limiting non-natural amino acids include beta-amino acids (beta3 and beta2), homo-amino acids, cyclic amino acids, aromatic amino acids, Pro and Pyr derivatives, 3-substituted Alanine derivatives, Glycine derivatives, ring-substituted Phe and Tyr Derivatives, linear core amino acids or diamino acids. They are available from a variety of suppliers, such as Sigma-Aldrich (USA) for example.

### CHEMICAL MODIFICATIONS

In the present invention any part of a protein according to at least some embodiments of the invention may optionally be chemically modified, i.e. changed by addition of functional groups. For example the side amino acid residues appearing in the native sequence may optionally be modified, although as described below alternatively other parts of the protein may optionally be modified, in addition to or in place of the side amino acid residues. The modification may optionally be performed during synthesis of the molecule if a chemical synthetic process is followed, for example by adding a chemically modified amino acid. However, chemical modification of an amino acid when it is already present in the molecule ("in situ" modification) is also possible.

The amino acid of any of the sequence regions of the molecule can optionally be modified according to any one of the following exemplary types of modification (in the peptide conceptually viewed as "chemically modified"). Non-limiting exemplary types of modification include carboxymethylation, acylation, phosphorylation, glycosylation or fatty acylation. Ether bonds can optionally be used to join the serine or threonine hydroxyl to the hydroxyl of a sugar. Amide bonds can optionally be used to join the glutamate or aspartate carboxyl groups to an amino group on a sugar (Garg and Jeanloz, Advances in Carbohydrate Chemistry and Biochemistry, Vol. 43, Academic Press (1985); Kunz, Ang. Chem. Int. Ed. English 26:294-308 (1987)). Acetal and ketal bonds can also optionally be formed between amino acids and carbohydrates. Fatty acid acyl derivatives can optionally be made, for example, by acylation of a free amino group (e.g., lysine) (Toth et al., Peptides: Chemistry, Structure and Biology, Rivier and Marshal, eds., ESCOM Publ., Leiden, 1078-1079 (1990)).

As used herein the term "chemical modification", when referring to a protein or peptide according to the present invention, refers to a protein or peptide where at least one of its amino acid residues is modified either by natural processes, such as processing or other post-translational modifications, or by chemical modification techniques which are well known in the art. Examples of the numerous known modifications typically include, but are not limited to: acetylation, acylation, amidation, ADP-ribosylation, glycosylation, GPI anchor formation, covalent attachment of a lipid or lipid derivative, methylation, myristylation, pegylation, prenylation, phosphorylation, ubiquitination, or any similar process.

Other types of modifications optionally include the addition of a cycloalkane moiety to a biological molecule, such as a protein, as described in PCT Application No. WO 2006/050262, hereby incorporated by reference as if fully set forth herein. These moieties are designed for use with biomolecules and may optionally be used to impart various properties to proteins.

Furthermore, optionally any point on a protein may be modified. For example, pegylation of a glycosylation moiety on a protein may optionally be performed, as described in PCT Application No. WO 2006/050247, hereby incorporated by reference as if fully set forth herein. One or more polyethylene glycol (PEG) groups may optionally be added to O-linked and/or N-linked glycosylation. The PEG group may optionally be branched or linear. Optionally any type of water-soluble polymer may be attached to a glycosylation site on a protein through a glycosyl linker.

### ALTERED GLYCOSYLATION

Proteins according to at least some embodiments of the invention may be modified to have an altered glycosylation pattern (i.e., altered from the original or native glycosylation pattern). As used herein, "altered" means having one or more carbohydrate moieties deleted, and/or having at least one glycosylation site added to the original protein.

Glycosylation of proteins is typically either N-linked or O-linked. N-linked refers to the attachment of the carbohydrate moiety to the side chain of an asparagine residue. The tripeptide sequences, asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-acetylgalactosamine, galactose, or xylose to a hydroxyamino acid, most commonly serine or threonine, although 5-hydroxyproline or 5-hydroxylysine may also be used.

Addition of glycosylation sites to proteins according to at least some embodiments of the invention is conveniently accomplished by altering the amino acid sequence of the protein such that it contains one or more of the above-described tripeptide sequences (for N-linked glycosylation sites). The alteration may also be made by the addition of, or substitution by, one or more serine or threonine residues in the sequence of the original protein (for O-linked glycosylation sites). The protein's amino acid sequence may also be altered by introducing changes at the DNA level.

Another means of increasing the number of carbohydrate moieties on proteins is by chemical or enzymatic coupling of glycosides to the amino acid residues of the protein. Depending on the coupling mode used, the sugars may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330, and in Aplin and Wriston, CRC Crit. Rev. Biochem., 22: 259-306 (1981).

Removal of any carbohydrate moieties present on proteins according to at least some embodiments of the invention may be accomplished chemically or enzymatically. Chemical deglycosylation requires exposure of the protein to trifluoromethanesulfonic acid, or an equivalent compound. This treatment results in the cleavage of most or all sugars except the linking sugar (N-acetylglucosamine or N-acetylgalactosamine), leaving the amino acid sequence intact.

Chemical deglycosylation is described by Hakimuddin et al., Arch. Biochem. Biophys., 259: 52 (1987); and Edge et al., Anal. Biochem., 118: 131 (1981). Enzymatic cleavage of carbohydrate moieties on proteins can be achieved by the use of a variety of endo- and exo-glycosidases as described by Thotakura et al., Meth. Enzymol., 138: 350 (1987).

### METHODS OF TREATMENT USING TMEM154 POLYPEPTIDES AND PROTEINS

As mentioned hereinabove the TMEM154 proteins and polypeptides according to at least some embodiments of the present invention or nucleic acid sequence or fragments thereof especially the ectodomain or secreted forms of TMEM154 proteins and polypeptides, can be used to treat cancer, including but not limited to lymphoma, especially Non-Hodgkin's Lymphoma, anti CD20 (i.e. Rituximab) resistant lymphoma, Multiple Myeloma, kidney cancer, and pancreatic cancer, and/or immune related conditions or disorders.

Thus, according to at least some embodiments of the present invention there is provided a method of treating cancer, and/or immune related conditions or disorders

As used herein the term "treating" refers to preventing, curing, reversing, attenuating, alleviating, minimizing, suppressing or halting the deleterious effects of the above-described diseases, disorders or conditions. The term treatment as used herein refers also to "maintenance therapy", which is a treatment that is given to keep a pathologic condition or disorder from coming back after it has disappeared following the initial therapy.

Treating, according to the present invention, can be effected by specifically upregulating the expression of at least one of the polypeptides according to at least some embodiments of the present invention in the subject.

Optionally, upregulation may be effected by administering to the subject at least one of the polypeptides according to at least some embodiments of the present invention (e.g., recombinant or synthetic) or an active portion thereof, as described herein. However, since the bioavailability of large polypeptides may potentially be relatively small due to high degradation rate and low penetration rate, administration of polypeptides is preferably confined to small peptide fragments (e.g., about 100 amino acids). The polypeptide or peptide may optionally be administered in as part of a pharmaceutical composition, described in more detail below.

It will be appreciated that treatment of the above-described diseases according to the present invention may be combined with other treatment methods known in the art (i.e., combination therapy).

### ANTI-KRTCAP3, ANTI-FAM26F, ANTI-MGC52498, ANTI-FAM70A, ANTI-TMEM154 ANTIBODIES

The antibodies according to at least some embodiments of the invention including those having the particular germline sequences, homologous antibodies, antibodies with conservative modifications, engineered and modified antibodies are characterized by particular functional features or properties of the antibodies. For example, the antibodies bind specifically to human KRTCAP3, FAM26F, MGC52498, FAM70A, or TMEM154 polypeptides. Optionally, an antibody according to at least some embodiments of the invention binds to corresponding KRTCAP3, FAM26F, MGC52498, FAM70A, or TMEM154 polypeptides with high affinity, for example with a KD of 10 -8 M or less or 10 -9 M or less or even 10 -10 M or less. The Anti-KRTCAP3, Anti-FAM26F, Anti-MGC52498, Anti-FAM70A, Anti-TMEM154 antibodies according to at least some embodiments of the invention optionally exhibit one or more of the following characteristics:
(i) binds to one of the corresponding human KRTCAP3, FAM26F, MGC52498, FAM70A, or TMEM154 polypeptides with a KD of 5.X10 -8 M or less;
(ii) binds to one of the KRTCAP3, FAM26F, MGC52498, FAM70A, or TMEM154 antigen expressed by cancer cells including for example ovarian cancer, lung cancer, colon cancer, breast cancer, kidney cancer, liver cancer, pancreatic cancer, prostate cancer, melanoma and hematological malignancies such as Multiple Myeloma, lymphoma, Non-Hodgkin's lymphoma, anti CD20 (i.e. Rituximab) resistant lymphoma, leukemia, T cell leukemia, but does not substantially bind to normal cells. In addition, optionally these antibodies and conjugates thereof will be effective in eliciting selective killing of such cancer cells and for modulating immune responses involved in autoimmunity and cancer.

Optionally, the antibody binds to one of the corresponding human KRTCAP3, FAM26F, MGC52498, FAM70A, or TMEM154 antigens with a KD of 3X10 -8 M or less, or with a KD of 1X10 -9 M or less, or with a KD of 0.1.X10 -9 M or less, or with a KD Of 0.05.X10 -9 M or less or with a KD of between 1X10 -9 and 1X10 -11 M.

Standard assays to evaluate the binding ability of the antibodies toward KRTCAP3, FAM26F, MGC52498, FAM70A, or TMEM154 polypeptides are known in the art, including for example, ELISAs, Western blots and RIAs. Suitable assays are described in detail in the Examples. The binding kinetics (e.g., binding affinity) of the antibodies also can be assessed by standard assays known in the art, such as by Biacore analysis.

Upon production of Anti-KRTCAP3, Anti-FAM26F, Anti-MGC52498, Anti-FAM70A, Anti-TMEM154 antibody sequences from antibodies can bind to KRTCAP3, FAM26F, MGC52498, FAM70A, or TMEM154 the VH and VL sequences can be "mixed and matched" to create other anti-KRTCAP3, FAM26F, MGC52498, FAM70A, or TMEM154 binding molecules according to at least some embodiments of the invention. KRTCAP3, FAM26F, MGC52498, FAM70A, or TMEM154 binding of such "mixed and matched" antibodies can be tested using the binding assays described above. e.g., ELISAs). Optionally, when VH and VL chains are mixed and matched, a VH sequence from a particular VH/VL pairing is replaced with a structurally similar VH sequence. Likewise, optionally a VL sequence from a particular VH/VL pairing is replaced with a structurally similar VL sequence. For example, the VH and VL sequences of homologous antibodies are particularly amenable for mixing and matching.

### ANTIBODIES HAVING PARTICULAR GERMLINE SEQUENCES

In certain embodiments, an antibody according to at least some embodiments of the invention comprises a heavy chain variable region from a particular germline heavy chain immunoglobulin gene and/or a light chain variable region from a particular germline light chain immunoglobulin gene.

As used herein, a human antibody comprises heavy or light chain variable regions that is "the product of" or "derived from" a particular germline sequence if the variable regions of the antibody are obtained from a system that uses human germline immunoglobulin genes. Such systems include immunizing a transgenic mouse carrying human immunoglobulin genes with the antigen of interest or screening a human immunoglobulin gene library displayed on phage with the antigen of interest. A human antibody that is "the product of" or "derived from" a human germline immunoglobulin sequence can be identified as such by comparing the amino acid sequence of the human antibody to the amino acid sequences of human germline immunoglobulins and selecting the human germline immunoglobulin sequence that is closest in sequence (i.e., greatest % identity) to the sequence of the human antibody.

A human antibody that is "the product of" or "derived from" a particular human germline immunoglobulin sequence may contain amino acid differences as compared to the germline sequence, due to, for example, naturally-occurring somatic mutations or intentional introduction of site-directed mutation. However, a selected human antibody typically is at least 90% identical in amino acids sequence to an amino acid sequence encoded by a human germline immunoglobulin gene and contains amino acid residues that identify the human antibody as being human when compared to the germline immunoglobulin amino acid sequences of other species (e.g., murine germline sequences). In certain cases, a human antibody may be at least 95, 96, 97, 98 or 99%, or even at least 96%, 97%, 98%, or 99% identical in amino acid sequence to the amino acid sequence encoded by the germline immunoglobulin gene. Typically, a human antibody derived from a particular human germline sequence will display no more than 10 amino acid differences from the amino acid sequence encoded by the human germline immunoglobulin gene. In certain cases, the human antibody may display no more than 5, or even no more than 4, 3, 2, or 1 amino acid difference from the amino acid sequence encoded by the germline immunoglobulin gene.

### HOMOLOGOUS ANTIBODIES

In yet another embodiment, an antibody according to at least some embodiments of the invention comprises heavy and light chain variable regions comprising amino acid sequences that are homologous to isolated Anti-KRTCAP3, Anti-FAM26F, Anti-MGC52498, Anti-FAM70A, Anti-TMEM154 amino acid sequences of preferred Anti-KRTCAP3, Anti-FAM26F, Anti-MGC52498, Anti-FAM70A, Anti-TMEM154 antibodies, respectively, wherein the antibodies retain the desired functional properties of the parent Anti-KRTCAP3, Anti-FAM26F, Anti-MGC52498, Anti-FAM70A, Anti-TMEM154 antibodies.

As used herein, the percent homology between two amino acid sequences is equivalent to the percent identity between the two sequences. The percent identity between the two sequences is a function of the number of identical positions shared by the sequences (i.e., % homology=# of identical positions/total # of positions X 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent identity between two amino acid sequences can be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48:444-453 (1970)) algorithm which has been incorporated into the GAP program in the GCG software package (available commercially), using either a Blossum 62 matrix or a PAM250 matrix, and a gap weight of 16, 14, 12, 10, 8, 6, or 4 and a length weight of 1, 2, 3, 4, 5, or 6.

Additionally or alternatively, the protein sequences according to at least some embodiments of the present invention can further be used as a "query sequence" to perform a search against public databases to, for example, identify related sequences. Such searches can be performed using the XBLAST program (version 2.0) of Altschul, et al. (1990) J Mol. Biol. 215:403-10. BLAST protein searches can be performed with the XBLAST program, score=50, wordlength=3 to obtain amino acid sequences homologous to the antibody molecules according to at least some embodiments of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (e.g., XBLAST and NBLAST) can be used.

### Antibodies with Conservative Modifications

In certain embodiments, an antibody according to at least some embodiments of the invention comprises a heavy chain variable region comprising CDR1, CDR2 and CDR3 sequences and a light chain variable region comprising CDR1, CDR2 and CDR3 sequences, wherein one or more of these CDR sequences comprise specified amino acid sequences based on preferred Anti-KRTCAP3, Anti-FAM26F, Anti-MGC52498, Anti-FAM70A, Anti-TMEM154 antibodies isolated and produced using methods herein, or conservative modifications thereof, and wherein the antibodies retain the desired functional properties of the Anti-KRTCAP3, Anti-FAM26F, Anti-MGC52498, Anti-FAM70A, Anti-TMEM154 antibodies according to at least some embodiments of the invention, respectively.

In various embodiments, the Anti-KRTCAP3, Anti-FAM26F, Anti-MGC52498, Anti-FAM70A, Anti-TMEM154 antibody can be, for example, human antibodies, humanized antibodies or chimeric antibodies.

As used herein, the term "conservative sequence modifications" is intended to refer to amino acid modifications that do not significantly affect or alter the binding characteristics of the antibody containing the amino acid sequence. Such conservative modifications include amino acid substitutions, additions and deletions. Modifications can be introduced into an antibody according to at least some embodiments of the invention by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within the CDR regions of an antibody according to at least some embodiments of the invention can be replaced with other amino acid residues from the same side chain family and the altered antibody can be tested for retained function (i.e., the functions set forth in (c) through (j) above) using the functional assays described herein.

### ENGINEERED AND MODIFIED ANTIBODIES

An antibody according to at least some embodiments of the invention can be prepared using an antibody having one or more of the VH and/or VL sequences derived from an Anti-KRTCAP3, Anti-FAM26F, Anti-MGC52498, Anti-FAM70A, Anti-TMEM154antibody starting material to engineer a modified antibody, which modified antibody may have altered properties from the starting antibody. An antibody can be engineered by modifying one or more residues within one or both variable regions (i.e., VH and/or VL), for example within one or more CDR regions and/or within one or more framework regions. Additionally or alternatively, an antibody can be engineered by modifying residues within the constant regions, for example to alter the effector functions of the antibody.

One type of variable region engineering that can be performed is CDR grafting. Antibodies interact with target antigens predominantly through amino acid residues that are located in the six heavy and light chain complementarity determining regions (CDRs). For this reason, the amino acid sequences within CDRs are more diverse between individual antibodies than sequences outside of CDRs. Because CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties (see, e.g., Riechmann, L. et al. (1998) Nature 332:323-327; Jones, P. et al. (1986) Nature 321:522-525; Queen, C. et al. (1989) Proc. Natl. Acad. See. U.S.A. 86:10029-10033; U.S. Pat. No. 5,225,539 to Winter, and U.S. Pat. Nos. 5,530,101; 5,585,089; 5,693,762 and 6,180,370 to Queen et al.)

Suitable framework sequences can be obtained from public DNA databases or published references that include germline antibody gene sequences. For example, germline DNA sequences for human heavy and light chain variable region genes can be found in the "VBase" human germline sequence database (available on the Internet), as well as in Kabat, E. A., et al. (1991) Sequences of Proteins of Immunological Interest, Fifth Edition, U.S. Department of Health and Human Services, NIH Publication No. 91-3242; Tomlinson, I. M., et al. (1992) "The Repertoire of Human Germline VH Sequences Reveals about Fifty Groups of VH Segments with Different Hypervariable Loops" J. Mol. Biol. 227:776-798; and Cox, J. P. L. et al. (1994) "A Directory of Human Germ-line VH Segments Reveals a Strong Bias in their Usage" Eur. J Immunol. 24:827-836; the contents of each of which are expressly incorporated herein by reference.

Another type of variable region modification is to mutate amino acid residues within the VH and/or VL CDR 1, CDR2 and/or CDR3 regions to thereby improve one or more binding properties (e.g., affinity) of the antibody of interest. Site-directed mutagenesis or PCR-mediated mutagenesis can be performed to introduce the mutations and the effect on antibody binding, or other functional property of interest, can be evaluated in appropriate in vitro or in vivo assays. Optionally conservative modifications (as discussed above) are introduced. The mutations may be amino acid substitutions, additions or deletions, but are preferably substitutions. Moreover, typically no more than one, two, three, four or five residues within a CDR region are altered.

Engineered antibodies according to at least some embodiments of the invention include those in which modifications have been made to framework residues within VH and/or VL, e.g. to improve the properties of the antibody. Typically such framework modifications are made to decrease the immunogenicity of the antibody. For example, one approach is to "backmutate" one or more framework residues to the corresponding germline sequence. More specifically, an antibody that has undergone somatic mutation may contain framework residues that differ from the germline sequence from which the antibody is derived. Such residues can be identified by comparing the antibody framework sequences to the germline sequences from which the antibody is derived.

In addition or alternative to modifications made within the framework or CDR regions, antibodies according to at least some embodiments of the invention may be engineered to include modifications within the Fc region, typically to alter one or more functional properties of the antibody, such as serum half-life, complement fixation, Fc receptor binding, and/or antigen-dependent cellular cytotoxicity. Furthermore, an antibody according to at least some embodiments of the invention may be chemically modified (e.g., one or more chemical moieties can be attached to the antibody) or be modified to alter its glycosylation, again to alter one or more functional properties of the antibody. Such embodiments are described further below. The numbering of residues in the Fc region is that of the EU index of Kabat.

In one embodiment, the hinge region of CH1 is modified such that the number of cysteine residues in the hinge region is altered, e.g., increased or decreased. This approach is described further in U.S. Pat. No. 5,677,425 by Bodmer et al. The number of cysteine residues in the hinge region of CH1 is altered to, for example, facilitate assembly of the light and heavy chains or to increase or decrease the stability of the antibody.

In another embodiment, the Fc hinge region of an antibody is mutated to decrease the biological half life of the antibody. More specifically, one or more amino acid mutations are introduced into the CH2-CH3 domain interface region of the Fc-hinge fragment such that the antibody has impaired Staphylococcyl protein A (SpA) binding relative to native Fc-hinge domain SpA binding. This approach is described in further detail in U.S. Pat. No. 6,165,745 by Ward et al.

In another embodiment, the antibody is modified to increase its biological half life. Various approaches are possible. For example, one or more of the following mutations can be introduced: T252L, T254S, T256F, as described in U.S. Pat. No. 6,277,375 to Ward. Alternatively, to increase the biological half life, the antibody can be altered within the CH1 or CL region to contain a salvage receptor binding epitope taken from two loops of a CH2 domain of an Fc region of an IgG, as described in U.S. Pat. Nos. 5,869,046 and 6,121,022 by Presta et al.

In yet other embodiments, the Fc region is altered by replacing at least one amino acid residue with a different amino acid residue to alter the effector functions of the antibody. For example, one or more amino acids selected from amino acid residues 234, 235, 236, 237, 297, 318, 320 and 322 can be replaced with a different amino acid residue such that the antibody has an altered affinity for an effector ligand but retains the antigen-binding ability of the parent antibody. The effector ligand to which affinity is altered can be, for example, an Fc receptor or the C1 component of complement. This approach is described in further detail in U.S. Pat. Nos. 5,624,821 and 5,648,260, both by Winter et al.

In another example, one or more amino acids selected from amino acid residues 329, 331 and 322 can be replaced with a different amino acid residue such that the antibody has altered C1q binding and/or reduced or abolished complement dependent cytotoxicity (CDC). This approach is described in further detail in U.S. Pat. Nos. 6,194,551 by Idusogie et al.

In another example, one or more amino acid residues within amino acid positions 231 and 239 are altered to thereby alter the ability of the antibody to fix complement. This approach is described further in PCT Publication WO 94/29351 by Bodmer et al.

In yet another example, the Fc region is modified to increase the ability of the antibody to mediate antibody dependent cellular cytotoxicity (ADCC) and/or to increase the affinity of the antibody for an Fcy receptor by modifying one or more amino acids at the following positions: 238, 239, 248, 249, 252, 254, 255, 256, 258, 265, 267, 268, 269, 270, 272, 276, 278, 280, 283, 285, 286, 289, 290, 292, 293, 294, 295, 296, 298, 301, 303, 305, 307, 309, 312, 315, 320, 322, 324, 326, 327, 329, 330, 331, 333, 334, 335, 337, 338, 340, 360, 373, 376, 378, 382, 388, 389, 398, 414, 416, 419, 430, 434, 435, 437, 438 or 439. This approach is described further in PCT Publication WO 00/42072 by Presta. Moreover, the binding sites on human IgG1 for Fc grammar, Fc gamma RII, Fc gammaRIII and FcRn have been mapped and variants with improved binding have been described (see Shields, R. L. et al. (2001) J. Biol. Chem. 276:6591-6604). Specific mutations at positions 256, 290, 298, 333, 334 and 339 are shown to improve binding to FcyRIII. Additionally, the following combination mutants are shown to improve Fcgamma.RIII binding: T256A/S298A, S298A/E333A, S298A/K224A and S298A/E333A/K334A.

In still another embodiment, the glycosylation of an antibody is modified. For example, an aglycoslated antibody can be made (i.e., the antibody lacks glycosylation). Glycosylation can be altered to, for example, increase the affinity of the antibody for antigen. Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Such aglycosylation may increase the affinity of the antibody for antigen. Such an approach is described in further detail in U.S. Pat. Nos. 5,714,350 and 6,350,861 by Co et al.

Additionally or alternatively, an antibody can be made that has an altered type of glycosylation, such as a hypofucosylated antibody having reduced amounts of fucosyl residues or an antibody having increased bisecting GlcNac structures. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies according to at least some embodiments of the invention to thereby produce an antibody with altered glycosylation. For example, the cell lines Ms704, Ms705, and Ms709 lack the fucosyltransferase gene, FUT8 (alpha (1,6) fucosyltransferase), such that antibodies expressed in the Ms704, Ms705, and Ms709 cell lines lack fucose on their carbohydrates. The Ms704, Ms705, and Ms709 FUT8.-/- cell lines are created by the targeted disruption of the FUT8 gene in CHO/DG44 cells using two replacement vectors (see U.S. Patent Publication No. 20040110704 by Yamane et al. and Yamane-Ohnuki et al. (2004) Biotechnol Bioeng 87:614-22). As another example, EP 1,176,195 by Hanai et al. describes a cell line with a functionally disrupted FUT8 gene, which encodes a fucosyl transferase, such that antibodies expressed in such a cell line exhibit hypofucosylation by reducing or eliminating the alpha 1,6 bond-related enzyme. Hanai et al. also describe cell lines which have a low enzyme activity for adding fucose to the N-acetylglucosamine that binds to the Fc region of the antibody or does not have the enzyme activity, for example the rat myeloma cell line YB2/0 (ATCC CRL 1662). PCT Publication WO 03/035835 by Presta describes a variant CHO cell line, Lec13 cells, with reduced ability to attach fucose to Asn(297)-linked carbohydrates, also resulting in hypofucosylation of antibodies expressed in that host cell (see also Shields, R. L. et al. (2002) J. Biol. Chem. 277:26733-26740). PCT Publication WO 99/54342 by Umana et al. describes cell lines engineered to express glycoprotein-modifying glycosyl transferases (e.g., beta(1,4)-N-acetylglucosaminyltransferase III (GnTIII)) such that antibodies expressed in the engineered cell lines exhibit increased bisecting GlcNac structures which results in increased ADCC activity of the antibodies (see also Umana et al. (1999) Nat. Biotech. 17:176-180). Alternatively, the fucose residues of the antibody may be cleaved off using a fucosidase enzyme. For example, the fucosidase alpha-L-fucosidase removes fucosyl residues from antibodies (Tarentino, A. L. et al. (1975) Biochem. 14:5516-23).

Another modification of the antibodies herein that is contemplated by the invention is pegylation. An antibody can be pegylated to, for example, increase the biological (e.g., serum) half life of the antibody. To pegylate an antibody, the antibody, or fragment thereof, typically is reacted with polyethylene glycol (PEG), such as a reactive ester or aldehyde derivative of PEG, under conditions in which one or more PEG groups become attached to the antibody or antibody fragment. Optionally, the pegylation is carried out via an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (C1-C10) alkoxy- or aryloxy-polyethylene glycol or polyethylene glycol-maleimide. In certain embodiments, the antibody to be pegylated is an aglycosylated antibody. Methods for pegylating proteins are known in the art and can be applied to the antibodies according to at least some embodiments of the invention. See for example, EP 0 154 316 by Nishimura et al. and EP 0 401 384 by Ishikawa et al.

### METHODS OF ENGINEERING ANTIBODIES

As discussed above, the Anti-KRTCAP3, Anti-FAM26F, Anti-MGC52498, Anti-FAM70A, Anti-TMEM154 antibodies having VH and VK sequences disclosed herein can be used to create new Anti-KRTCAP3, Anti-FAM26F, Anti-MGC52498, Anti-FAM70A, Anti-TMEM154antibodies, respectively, by modifying the VH and/or VL sequences, or the constant regions attached thereto. Thus, according to at least some embodiments of the invention, the structural features of an Anti-KRTCAP3, Anti-FAM26F, Anti-MGC52498, Anti-FAM70A, Anti-TMEM154 antibody according to at least some embodiments of the invention, are used to create structurally related Anti-KRTCAP3, Anti-FAM26F, Anti-MGC52498, Anti-FAM70A, Anti-TMEM154 antibodies that retain at least one functional property of the antibodies according to at least some embodiments of the invention, such as binding to human KRTCAP3, FAM26F, MGC52498, FAM70A, or TMEM154, respectively. For example, one or more CDR regions of one KRTCAP3, FAM26F, MGC52498, FAM70A, or TMEM154 antibody or mutations thereof, can be combined recombinantly with known framework regions and/or other CDRs to create additional, recombinantly-engineered, Anti-KRTCAP3, Anti-FAM26FF, Anti-MGC52498, Anti-FAM70A, Anti-TMEM154 antibodies according to at least some embodiments of the invention, as discussed above. Other types of modifications include those described in the previous section. The starting material for the engineering method is one or more of the VH and/or VK sequences provided herein, or one or more CDR regions thereof. To create the engineered antibody, it is not necessary to actually prepare (i.e., express as a protein) an antibody having one or more of the VH and/or VK sequences provided herein, or one or more CDR regions thereof. Rather, the information contained in the sequences is used as the starting material to create a "second generation" sequences derived from the original sequences and then the "second generation" sequences is prepared and expressed as a protein.

Standard molecular biology techniques can be used to prepare and express altered antibody sequence.

OPtionally, the antibody encoded by the altered antibody sequences is one that retains one, some or all of the functional properties of the Anti-KRTCAP3, Anti-FAM26F, Anti-MGC52498, Anti-FAM70A, Anti-TMEM154 antibodies, respectively, produced by methods and with sequences provided herein, which functional properties include binding to KRTCAP3, FAM26F, MGC52498, FAM70A, or TMEM154 antigen with a specific KD level or less and/or selectively binding to desired target cells such as ovarian cancer, lung cancer, breast cancer, colon cancer, kidney cancer, liver cancer, pancreatic cancer, prostate cancer, melanoma and hematological malignancies such as Multiple Myeloma, lymphoma, Non-Hodgkin's lymphoma, anti CD20 (i.e. Rituximab) resistant lymphoma, leukemia, T cell leukemia, that express KRTCAP3, FAM26F, MGC52498, FAM70A, or TMEM154 antigen.

The functional properties of the altered antibodies can be assessed using standard assays available in the art and/or described herein.

In certain embodiments of the methods of engineering antibodies according to at least some embodiments of the invention, mutations can be introduced randomly or selectively along all or part of an Anti-KRTCAP3, Anti-FAM26F, Anti-MGC52498, Anti-FAM70A, Anti-TMEM154 antibody coding sequence and the resulting modified Anti-KRTCAP3, Anti-FAM26F, Anti-MGC52498, Anti-FAM70A, Anti-TMEM154 antibodies can be screened for binding activity and/or other desired functional properties.

Mutational methods have been described in the art. For example, PCT Publication WO 02/092780 by Short describes methods for creating and screening antibody mutations using saturation mutagenesis, synthetic ligation assembly, or a combination thereof. Alternatively, PCT Publication WO 03/074679 by Lazar et al. describes methods of using computational screening methods to optimize physiochemical properties of antibodies.

### NUCLEIC ACID MOLECULES ENCODING ANTIBODIES

According to at least some embodiments of the inventionpertains to nucleic acid molecules that encode the antibodies according to at least some embodiments of the invention. The nucleic acids may be present in whole cells, in a cell lysate, or in a partially purified or substantially pure form. A nucleic acid is "isolated" or "rendered substantially pure" when purified away from other cellular components or other contaminants, e.g., other cellular nucleic acids or proteins, by standard techniques, including alkaline/SDS treatment, CsCI banding, column chromatography, agarose gel electrophoresis and others well known in the art. See, F. Ausubel, et al., ed. (1987) Current Protocols in Molecular Biology, Greene Publishing and Wiley Interscience, New York. A nucleic acid according to at least some embodiments of the invention can be, for example, DNA or RNA and may or may not contain intronic sequences. In a preferred embodiment, the nucleic acid is a cDNA molecule.

Nucleic acids according to at least some embodiments of the invention can be obtained using standard molecular biology techniques. For antibodies expressed by hybridomas (e.g., hybridomas prepared from transgenic mice carrying human immunoglobulin genes as described further below), cDNAs encoding the light and heavy chains of the antibody made by the hybridoma can be obtained by standard PCR amplification or cDNA cloning techniques. For antibodies obtained from an immunoglobulin gene library (e.g., using phage display techniques), nucleic acid encoding the antibody can be recovered from the library.

Once DNA fragments encoding VH and VL segments are obtained, these DNA fragments can be further manipulated by standard recombinant DNA techniques, for example to convert the variable region genes to full-length antibody chain genes, to Fab fragment genes or to a scFv gene. In these manipulations, a VL- or VH-encoding DNA fragment is operatively linked to another DNA fragment encoding another protein, such as an antibody constant region or a flexible linker.

The term "operatively linked", as used in this context, is intended to mean that the two DNA fragments are joined such that the amino acid sequences encoded by the two DNA fragments remain in-frame.

The isolated DNA encoding the VH region can be converted to a full-length heavy chain gene by operatively linking the VH-encoding DNA to another DNA molecule encoding heavy chain constant regions (CH1, CH2 and CH3). The sequences of human heavy chain constant region genes are known in the art (see e.g., Kabat, E. A., el al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The heavy chain constant region can be an IgG1, IgG2, IgG3, IgG4, IgA, IgE, IgM or IgD constant region. For a Fab fragment heavy chain gene, the VH-encoding DNA can be operatively linked to another DNA molecule encoding only the heavy chain CH1 constant region.

The isolated DNA encoding the VL region can be converted to a full-length light chain gene (as well as a Fab light chain gene) by operatively linking the VL-encoding DNA to another DNA molecule encoding the light chain constant region, CL. The sequences of human light chain constant region genes are known in the art (see e.g., Kabat, E. A., et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The light chain constant region can be a kappa or lambda constant region.

To create a scFv gene, the VH- and VL-encoding DNA fragments are operatively linked to another fragment encoding a flexible linker, e.g., encoding the amino acid sequence (Gly4-Ser)3, such that the VH and VL sequences can be expressed as a contiguous single-chain protein, with the VL and VH regions joined by the flexible linker (see e.g., Bird et al. (1988) Science 242:423-426; Huston et al. (1988) Proc. Natl. Acad. Sci. USA 85:5879-5883; McCafferty et al., (1990) Nature 348:552-554).

### Production Of Anti-KRTCAP3, Anti-FAM26F, Anti-MGC52498, Anti-FAM70A, Anti-TMEM154 Monoclonal Antibodies

Monoclonal antibodies (mAbs) according to at least some embodiments of the present invention can be produced by a variety of techniques, including conventional monoclonal antibody methodology e.g., the standard somatic cell hybridization technique of Kohler and Milstein (1975) Nature 256:495. Although somatic cell hybridization procedures are preferred, in principle, other techniques for producing monoclonal antibody can be employed e.g., viral or oncogenic transformation of B lymphocytes.

A preferred animal system for preparing hybridomas is the murine system. Hybridoma production in the mouse is a very well-established procedure. Immunization protocols and techniques for isolation of immunized splenocytes for fusion are known in the art. Fusion partners (e.g., murine myeloma cells) and fusion procedures are also known.

Chimeric or humanized antibodies according to at least some embodiments of the present invention can be prepared based on the sequence of a murine monoclonal antibody prepared as described above. DNA encoding the heavy and light chain immunoglobulins can be obtained from the murine hybridoma of interest and engineered to contain non-murine (e.g.,. human) immunoglobulin sequences using standard molecular biology techniques. For example, to create a chimeric antibody, the murine variable regions can be linked to human constant regions using methods known in the art (see e.g., U.S. Pat. No. 4,816,567 to Cabilly et al.). To create a humanized antibody, the murine CDR regions can be inserted into a human framework using methods known in the art (see e.g., U.S. Pat. No. 5,225,539 to Winter, and U.S. Pat. Nos. 5,530,101; 5,585,089; 5,693,762 and 6,180,370 to Queen et al.).

In a preferred embodiment, the antibodies according to at least some embodiments of the invention are human monoclonal antibodies. Such human monoclonal antibodies directed against KRTCAP3, FAM26F, MGC52498, FAM70A or TMEM154 can be generated using transgenic or transchromosomic mice carrying parts of the human immune system rather than the mouse system. These transgenic and transchromosomic mice include mice referred to herein as the HuMAb Mouse RTM and KM Mouse. RTM. respectively, and are collectively referred to herein as "human Ig mice." The HuMAb Mouse TM. (Medarex. Inc.) contains human immunoglobulin gene miniloci that encode unrearranged human heavy (.mu. and.gamma.) and.kappa. light chain immunoglobulin sequences, together with targeted mutations that inactivate the endogenous.mu. and.kappa. chain loci (see e.g., Lonberg, et al. (1994) Nature 368(6474): 856-859). Accordingly, the mice exhibit reduced expression of mouse IgM or.kappa., and in response to immunization, the introduced human heavy and light chain transgenes undergo class switching and somatic mutation to generate high affinity human IgGkappa. monoclonal (Lonberg, N. et al. (1994), supra; reviewed in Lonberg, N. (1994) Handbook of Experimental Pharmacology 113:49-101; Lonberg, N. and Huszar, D. (1995) Intern. Rev. Immunol. 13: 65-93, and Harding, F. and Lonberg, N. (1995) Ann. N.Y. Acad. Sci. 764:536-546). The preparation and use of the HuMab Mouse RTM., and the genomic modifications carried by such mice, is further described in Taylor, L. et al. (1992) Nucleic Acids Research 20:6287-6295; Chen, J. et al. (1993) International Immunology 5:647-656; Tuaillon et al. (1993) Proc. Natl. Acad. Sci. USA 90:3720-3724; Choi et al. (1993) Nature Genetics 4:117-123; Chen, J. et al. (1993) EMBO J. 12: 821-830; Tuaillon et al. (1994) J. Immunol. 152:2912-2920; Taylor, L. et al. (1994) International Immunology 6:579-591; and Fishwild, D. et al. (1996) Nature Biotechnology 14: 845-851, the contents of all of which are hereby specifically incorporated by reference in their entirety. See further, U.S. Pat. Nos. 5,545,806; 5,569,825; 5,625,126; 5,633,425; 5,789,650; 5,877,397; 5,661,016; 5,814,318; 5,874,299; and 5,770,429; all to Lonberg and Kay; U.S. Pat. No. 5,545,807 to Surani et al.; PCT Publication Nos. WO 92/03918, WO 93/12227, WO 94/25585, WO 97/13852, WO 98/24884 and WO 99/45962, all to Lonberg and Kay; and PCT Publication No. WO 01/14424 to Korman et al.

In another embodiment, human antibodies according to at least some embodiments of the invention can be raised using a mouse that carries human immunoglobulin sequences on transgenes and transchomosomes, such as a mouse that carries a human heavy chain transgene and a human light chain transchromosome. Such mice, referred to herein as "KM mice TM.", are described in detail in PCT Publication WO 02/43478 to Ishida et al.

Still further, alternative transgenic animal systems expressing human immunoglobulin genes are available in the art and can be used to raise anti- KRTCAP3, FAM26F, MGC52498, FAM70A or TMEM154 antibodies according to at least some embodiments of the invention. For example, an alternative transgenic system referred to as the Xenomouse (Abgenix, Inc.) can be used; such mice are described in, for example, U.S. Pat. Nos. 5,939,598; 6,075,181; 6,114,598; 6, 150,584 and 6,162,963 to Kucherlapati et al.

Moreover, alternative transchromosomic animal systems expressing human immunoglobulin genes are available in the art and can be used to raise Anti-KRTCAP3, Anti-FAM26F, Anti-MGC52498, Anti-FAM70A, Anti-TMEM154 antibodies according to at least some embodiments of the invention. For example, mice carrying both a human heavy chain transchromosome and a human light chain transchromosome, referred to as "TC mice" can be used; such mice are described in Tomizuka et al. (2000) Proc. Natl. Acad Sci. USA 97:722-727. Furthermore, cows carrying human heavy and light chain transchromosomes have been described in the art (Kuroiwa et al. (2002) Nature Biotechnology 20:889-894) and can be used to raise Anti-KRTCAP3, Anti-FAM26F, Anti-MGC52498, Anti-FAM70A, Anti-TMEM154 antibodies according to at least some embodiments of the invention.

Human monoclonal antibodies according to at least some embodiments of the invention can also be prepared using phage display methods for screening libraries of human immunoglobulin genes. Such phage display methods for isolating human antibodies are established in the art. See for example: U.S. Pat. Nos. 5,223,409; 5,403,484; and 5,571,698 to Ladner et al.; U.S. Pat. Nos. 5,427,908 and 5,580,717 to Dower et al.; U.S. Pat. Nos. 5,969,108 and 6,172,197 to McCafferty et al.; and U.S. Pat. Nos. 5,885,793; 6,521,404; 6,544,73 1; 6,555,313; 6,582,915 and 6,593,081 to Griffiths et al.

Human monoclonal antibodies according to at least some embodiments of the invention can also be prepared using SCID mice into which human immune cells have been reconstituted such that a human antibody response can be generated upon immunization. Such mice are described in, for example, U.S. Pat. Nos. 5,476,996 and 5,698,767 to Wilson et al.

### IMMUNIZATION OF HUMAN IG MICE

When human Ig mice are used to raise human antibodies according to at least some embodiments of the invention, such mice can be immunized with a purified or enriched preparation of KRTCAP3, FAM26F, MGC52498, FAM70A, or TMEM154 antigen and/or recombinant KRTCAP3, FAM26F, MGC52498, FAM70A, or TMEM154, or an KRTCAP3, FAM26F, MGC52498, FAM70A, or TMEM154 fusion protein, as described by Lonberg, N. et al. (1994) Nature 368(6474): 856-859; Fishwild, D. et al. (1996) Nature Biotechnology 14: 845-851; and PCT Publication WO 98/24884 and WO 01/14424. Preferably, the mice will be 6-16 weeks of age upon the first infusion. For example, a purified or recombinant preparation (5-50.mu.g) of KRTCAP3, FAM26F, MGC52498, FAM70A, or TMEM154 antigen can be used to immunize the human Ig mice intraperitoneally.

Prior experience with various antigens by others has shown that the transgenic mice respond when initially immunized intraperitoneally (IP) with antigen in complete Freund's adjuvant, followed by every other week IP immunizations (up to a total of 6) with antigen in incomplete Freund's adjuvant. However, adjuvants other than Freund's are also found to be effective. In addition, whole cells in the absence of adjuvant are found to be highly immunogenic. The immune response can be monitored over the course of the immunization protocol with plasma samples being obtained by retroorbital bleeds. The plasma can be screened by ELISA (as described below), and mice with sufficient titers of anti- KRTCAP3, anti-FAM26F, anti-MGC52498, anti-FAM70A, or anti-TMEM154 human immunoglobulin can be used for fusions. Mice can be boosted intravenously with antigen 3 days before sacrifice and removal of the spleen. It is expected that 2-3 fusions for each immunization may need to be performed. Between 6 and 24 mice are typically immunized for each antigen. Usually both HCo7 and HCo12 strains are used. In addition, both HCo7 and HCo12 transgene can be bred together into a single mouse having two different human heavy chain transgenes (HCo7/HCo 12). Alternatively or additionally, the KM Mouse. RTM. strain can be used.

### GENERATION OF HYBRIDOMAS PRODUCING HUMAN MONOCLONAL ANTIBODIES

To generate hybridomas producing human monoclonal antibodies according to at least some embodiments of the invention, splenocytes and/or lymph node cells from immunized mice can be isolated and fused to an appropriate immortalized cell line, such as a mouse myeloma cell line. The resulting hybridomas can be screened for the production of antigen-specific antibodies. For example, single cell suspensions of splenic lymphocytes from immunized mice can be fused to one-sixth the number of P3X63-Ag8.653 nonsecreting mouse myeloma cells (ATCC, CRL 1580) with 50% PEG. Cells are plated at approximately 2 X 10 -5 in flat bottom microtiter plate, followed by a two week incubation in selective medium containing 20% fetal Clone Serum, 18% "653" conditioned media, 5% origen (IGEN), 4 mM L-glutamine, 1 mM sodium pyruvate, 5 mM HEPES, 0.055 mM 2-mercaptoethanol, 50 units/ml penicillin, 50 mg/ml streptomycin, 50 mg/ml gentamycin and 1X HAT (Sigma; the HAT is added 24 hours after the fusion). After approximately two weeks, cells can be cultured in medium in which the HAT is replaced with HT. Individual wells can then be screened by ELISA for human monoclonal IgM and IgG antibodies. Once extensive hybridoma growth occurs, medium can be observed usually after 10-14 days. The antibody secreting hybridomas can be replated, screened again, and if still positive for human IgG, the monoclonal antibodies can be subcloned at least twice by limiting dilution. The stable subclones can then be cultured in vitro to generate small amounts of antibody in tissue culture medium for characterization.

To purify human monoclonal antibodies, selected hybridomas can be grown in two-liter spinner-flasks for monoclonal antibody purification. Supernatants can be filtered and concentrated before affinity chromatography with protein A-Sepharose (Pharmacia, Piscataway, N.J.). Eluted IgG can be checked by gel electrophoresis and high performance liquid chromatography to ensure purity. The buffer solution can be exchanged into PBS, and the concentration can be determined by OD280 using 1.43 extinction coefficient. The monoclonal antibodies can be aliquoted and stored at -80 degrees C.

### GENERATION OF TRANSFECTOMAS PRODUCING MONOCLONAL ANTIBODIES

Antibodies according to at least some embodiments of the invention also can be produced in a host cell transfectoma using, for example, a combination of recombinant DNA techniques and gene transfection methods as is well known in the art (e.g., Morrison, S. (1985) Science 229:1202).

For example, to express the antibodies, or antibody fragments thereof, DNAs encoding partial or full-length light and heavy chains, can be obtained by standard molecular biology techniques (e.g., PCR amplification or cDNA cloning using a hybridoma that expresses the antibody of interest) and the DNAs can be inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vector or, more typically, both genes are inserted into the same expression vector. The antibody genes are inserted into the expression vector by standard methods (e.g., ligation of complementary restriction sites on the antibody gene fragment and vector, or blunt end ligation if no restriction sites are present). The light and heavy chain variable regions of the antibodies described herein can be used to create full-length antibody genes of any antibody isotype by inserting them into expression vectors already encoding heavy chain constant and light chain constant regions of the desired isotype such that the VH segment is operatively linked to the CH segments within the vector and the VK segment is operatively linked to the CL segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (i.e., a signal peptide from a non-immunoglobulin protein).

In addition to the antibody chain genes, the recombinant expression vectors according to at least some embodiments of the invention carry regulatory sequences that control the expression of the antibody chain genes in a host cell. The term "regulatory sequence" is intended to include promoters, enhancers and other expression control elements (e.g., polyadenylation signals) that control the transcription or translation of the antibody chain genes. Such regulatory sequences are described, for example, in Goeddel (Gene Expression Technology. Methods in Enzymology 185, Academic Press, San Diego, Calif. (1990)). It will be appreciated by those skilled in the art that the design of the expression vector, including the selection of regulatory sequences, may depend on such factors as the choice of the host cell to be transformed, the level of expression of protein desired, etc. Preferred regulatory sequences for mammalian host cell expression include viral elements that direct high levels of protein expression in mammalian cells, such as promoters and/or enhancers derived from cytomegalovirus (CMV), Simian Virus 40 (SV40), adenovirus, (e.g., the adenovirus major late promoter (AdMLP) and polyoma. Alternatively, nonviral regulatory sequences may be used, such as the ubiquitin promoter or.beta.-globin promoter. Still further, regulatory elements composed of sequences from different sources, such as the SR alpha. promoter system, which contains sequences from the SV40 early promoter and the long terminal repeat of human T cell leukemia virus type 1 (Takebe, Y. et al. (1988) Mol. Cell. Biol. 8:466-472).

In addition to the antibody chain genes and regulatory sequences, the recombinant expression vectors according to at least some embodiments of the invention may carry additional sequences, such as sequences that regulate replication of the vector in host cells (e.g., origins of replication) and selectable marker genes. The selectable marker gene facilitates selection of host cells into which the vector has been introduced (see, e.g., U.S. Pat. Nos. 4,399,216, 4,634,665 and 5,179,017, all by Axel et al.). For example, typically the selectable marker gene confers resistance to drugs, such as G418, hygromycin or methotrexate, on a host cell into which the vector has been introduced. Preferred selectable marker genes include the dihydrofolate reductase (DHFR) gene (for use in dhfr-host cells with methotrexate selection/amplification) and the neo gene (for G418 selection).

For expression of the light and heavy chains, the expression vectors encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection and the like. Although it is theoretically possible to express the antibodies according to at least some embodiments of the invention in either prokaryotic or eukaryotic host cells, expression of antibodies in eukaryotic cells, and most preferably mammalian host cells, is the most preferred because such eukaryotic cells, and in particular mammalian cells, are more likely than prokaryotic cells to assemble and secrete a properly folded and immunologically active antibody. Prokaryotic expression of antibody genes has been reported to be ineffective for production of high yields of active antibody (Boss, M. A. and Wood, C. R. (1985) Immunology Today 6:12-13).

Preferred mammalian host cells for expressing the recombinant antibodies according to at least some embodiments of the invention include Chinese Hamster Ovary (CHO cells) (including dhfr- CHO cells, described in Urlaub and Chasin, (1980) Proc. Natl. Acad. Sci. USA 77:4216-4220, used with a DHFR selectable marker, e.g., as described in R. J. Kaufman and P. A. Sharp (1982) Mol. Biol. 159:601-621), NSO myeloma cells, COS cells and SP2 cells. In particular, for use with NSO myeloma cells, another preferred expression system is the GS gene expression system disclosed in WO 87/04462, WO 89/01036 and EP 338,841. When recombinant expression vectors encoding antibody genes are introduced into mammalian host cells, the antibodies are produced by culturing the host cells for a period of time sufficient to allow for expression of the antibody in the host cells or, preferably, secretion of the antibody into the culture medium in which the host cells are grown. Antibodies can be recovered from the culture medium using standard protein purification methods.

### CHARACTERIZATION OF ANTIBODY BINDING TO ANTIGEN

Antibodies according to at least some embodiments of the invention can be tested for binding to KRTCAP3, FAM26F, MGC52498, FAM70A, or TMEM154 by, for example, standard ELISA. Briefly, microtiter plates are coated with purified KRTCAP3, FAM26F, MGC52498, FAM70A, or TMEM154 at 0.25.mu.g/ml in PBS, and then blocked with 5% bovine serum albumin in PBS. Dilutions of antibody (e.g., dilutions of plasma from KRTCAP3, FAM26F, MGC52498, FAM70A, or TMEM154-immunized mice) are added to each well and incubated for 1-2 hours at 37 degrees C. The plates are washed with PBS/Tween and then incubated with secondary reagent (e.g., for human antibodies, a goat-anti-human IgG Fc-specific polyclonal reagent) conjugated to alkaline phosphatase for 1 hour at 37 degrees C. After washing, the plates are developed with pNPP substrate (1 mg/ml), and analyzed at OD of 405-650. Preferably, mice which develop the highest titers will be used for fusions.

An ELISA assay as described above can also be used to screen for hybridomas that show positive reactivity with KRTCAP3, FAM26F, MGC52498, FAM70A, or TMEM154 immunogen. Hybridomas that bind with high avidity to KRTCAP3, FAM26F, MGC52498, FAM70A, or TMEM154 are subcloned and further characterized. One clone from each hybridoma, which retains the reactivity of the parent cells (by ELISA), can be chosen for making a 5-10 vial cell bank stored at -140 degrees C., and for antibody purification.

To purify anti-KRTCAP3, anti-FAM26F, anti-MGC52498, anti-FAM70A, or anti-TMEM154 antibodies, selected hybridomas can be grown in two-liter spinner-flasks for monoclonal antibody purification. Supernatants can be filtered and concentrated before affinity chromatography with protein A-sepharose (Pharmacia, Piscataway, N.J.). Eluted IgG can be checked by gel electrophoresis and high performance liquid chromatography to ensure purity. The buffer solution can be exchanged into PBS, and the concentration can be determined by OD280 using 1.43 extinction coefficient. The monoclonal antibodies can be aliquoted and stored at -80 degrees C.

To determine if the selected anti-KRTCAP3, anti-FAM26F, anti-MGC52498, anti-FAM70A, or anti-TMEM154 monoclonal antibodies bind to unique epitopes, each antibody can be biotinylated using commercially available reagents (Pierce, Rockford, I11.). Competition studies using unlabeled monoclonal antibodies and biotinylated monoclonal antibodies can be performed using KRTCAP3, FAM26F, MGC52498, FAM70A, or TMEM154 coated-ELISA plates as described above. Biotinylated mAb binding can be detected with a strep-avidin-alkaline phosphatase probe.

To determine the isotype of purified antibodies, isotype ELISAs can be performed using reagents specific for antibodies of a particular isotype. For example, to determine the isotype of a human monoclonal antibody, wells of microtiter plates can be coated with 1.mu.g/ml of anti-human immunoglobulin overnight at 4 degrees C. After blocking with 1% BSA, the plates are reacted with 1mug /ml or less of test monoclonal antibodies or purified isotype controls, at ambient temperature for one to two hours. The wells can then be reacted with either human IgG1 or human IgM-specific alkaline phosphatase-conjugated probes. Plates are developed and analyzed as described above.

Anti-KRTCAP3, anti-FAM26F, anti-MGC52498, anti-FAM70A, or anti-TMEM154 human IgGs can be further tested for reactivity with KRTCAP3, FAM26F, MGC52498, FAM70A, or TMEM154 antigen, respectively, by Western blotting. Briefly, KRTCAP3, FAM26F, MGC52498, FAM70A, or TMEM154 antigen can be prepared and subjected to sodium dodecyl sulfate polyacrylamide gel electrophoresis. After electrophoresis, the separated antigens are transferred to nitrocellulose membranes, blocked with 10% fetal calf serum, and probed with the monoclonal antibodies to be tested. Human IgG binding can be detected using anti-human IgG alkaline phosphatase and developed with BCIP/NBT substrate tablets (Sigma Chem. Co., St. Louis, Mo.).

### CONJUGATES OR IMMUNOCONJUGATES

According to at least some embodiments, the present invention features immunoconjugates comprising an anti-KRTCAP3, anti-FAM26F, anti-MGC52498, anti-FAM70A, or anti-TMEM154 antibody, or a fragment thereof, conjugated to a therapeutic moiety, such as a cytotoxin, a drug (e.g., an immunosuppressant) or a radiotoxin. Such conjugates are referred to herein as "immunoconjugates". Immunoconjugates that include one or more cytotoxins are referred to as "immunotoxins." A cytotoxin or cytotoxic agent includes any agent that is detrimental to (e.g., kills) cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents also include, for example, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine).

Other preferred examples of therapeutic cytotoxins that can be conjugated to an antibody according to at least some embodiments of the invention include duocarmycins, calicheamicins, maytansines and auristatins, and derivatives thereof. An example of a calicheamicin antibody conjugate is commercially available (Mylotarg.TM.; Wyeth).

Cytotoxins can be conjugated to antibodies according to at least some embodiments of the invention using linker technology available in the art. Examples of linker types that have been used to conjugate a cytotoxin to an antibody include, but are not limited to, hydrazones, thioethers, esters, disulfides and peptide-containing linkers. A linker can be chosen that is, for example, susceptible to cleavage by low pH within the lysosomal compartment or susceptible to cleavage by proteases, such as proteases preferentially expressed in tumor tissue such as cathepsins (e.g., cathepsins B, C, D).

For further discussion of types of cytotoxins, linkers and methods for conjugating therapeutic agents to antibodies, see also Saito, G. et al. (2003) Adv. Drug Deliv. Rev. 55:199-215; Trail, P. A. et al. (2003) Cancer Immunol. Immunother. 52:328-337; Payne, G. (2003) Cancer Cell 3:207-212; Allen, T. M. (2002) Nat. Rev. Cancer 2:750-763; Pastan, I. and Kreitman, R. J. (2002) Curr. Opin. Investig. Drugs 3:1089-1091; Senter, P. D. and Springer, C. J. (2001) Adv. Drug Deliv. Rev. 53:247-264.

Antibodies according to at least some embodiments of the present invention also can be conjugated to a radioactive isotope to generate cytotoxic radiopharmaceuticals, also referred to as radioimmunoconjugates. Examples of radioactive isotopes that can be conjugated to antibodies for use diagnostically or therapeutically include, but are not limited to, iodine 131, indium 111, yttrium 90 and lutetium 177. Method for preparing radioimmunconjugates are established in the art. Examples of radioimmunoconjugates are commercially available, including Zevalin.TM. (IDEC Pharmaceuticals) and Bexxar.TM. (Corixa Pharmaceuticals), and similar methods can be used to prepare radioimmunoconjugates using the antibodies according to at least some embodiments of the invention.

The antibody conjugates according to at least some embodiments of the invention can be used to modify a given biological response, and the drug moiety is not to be construed as limited to classical chemical therapeutic agents. For example, the drug moiety may be a protein or polypeptide possessing a desired biological activity. Such proteins may include, for example, an enzymatically active toxin, or active fragment thereof, such as abrin, ricin A, pseudomonas exotoxin, or diphtheria toxin; a protein such as tumor necrosis factor or interferon-.gamma.; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors.

Techniques for conjugating such therapeutic moiety to antibodies are well known, see, e.g., Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982).

### BISPECIFIC MOLECULES

In another aspect, the present invention features bispecific molecules comprising an anti-KRTCAP3, anti-FAM26F, anti-MGC52498, anti-FAM70A, or anti-TMEM154 antibody, or a fragment thereof, according to at least some embodiments of the invention. An antibody according to at least some embodiments of the invention, or antigen-binding portions thereof, can be derivatized or linked to another functional molecule, e.g., another peptide or protein (e.g., another antibody or ligand for a receptor) to generate a bispecific molecule that binds to at least two different binding sites or target molecules. The antibody according to at least some embodiments of the invention may in fact be derivatized or linked to more than one other functional molecule to generate multispecific molecules that bind to more than two different binding sites and/or target molecules; such multispecific molecules are also intended to be encompassed by the term "bispecific molecule" as used herein. To create a bispecific molecule according to at least some embodiments of the invention, an antibody according to at least some embodiments of the invention can be functionally linked (e.g., by chemical coupling, genetic fusion, noncovalent association or otherwise) to one or more other binding molecules, such as another antibody, antibody fragment, peptide or binding mimetic, such that a bispecific molecule results.

Accordingly, the present invention includes bispecific molecules comprising at least one first binding specificity for a KRTCAP3, FAM26F, MGC52498, FAM70A, or TMEM154 polypeptide and a second binding specificity for a second target epitope. In a particular embodiment according to at least some embodiments of the invention, the second target epitope is an Fc receptor, e.g., human Fc gamma RI (CD64) or a human Fc alpha receptor (CD89). Therefore, the invention includes bispecific molecules capable of binding both to Fc gamma. R, Fc alpha R or Fc epsilon R expressing effector cells (e.g., monocytes, macrophages or polymorphonuclear cells (PMNs)), and to target cells expressing a KRTCAP3, FAM26F, MGC52498, FAM70A, or TMEM154 polypeptide, respectively. These bispecific molecules target KRTCAP3, FAM26F, MGC52498, FAM70A, or TMEM154 polypeptide expressing cells to effector cell and trigger Fc receptor-mediated effector cell activities, such as phagocytosis of KRTCAP3, FAM26F, MGC52498, FAM70A, or TMEM154 polypeptide expressing cells, antibody dependent cell-mediated cytotoxicity (ADCC), cytokine release, or generation of superoxide anion.

In an embodiment according to at least some embodiments of the invention in which the bispecific molecule is multispecific, the molecule can further include a third binding specificity, in addition to an anti-Fc binding specificity and an anti-6f binding specificity. In one embodiment, the third binding specificity is an anti-enhancement factor (EF) portion, e.g., a molecule which binds to a surface protein involved in cytotoxic activity and thereby increases the immune response against the target cell.

The "anti-enhancement factor portion" can be an antibody, functional antibody fragment or a ligand that binds to a given molecule, e.g., an antigen or a receptor, and thereby results in an enhancement of the effect of the binding determinants for the Fc receptor or target cell antigen. The "anti-enhancement factor portion" can bind an Fc receptor or a target cell antigen. Alternatively, the anti-enhancement factor portion can bind to an entity that is different from the entity to which the first and second binding specificities bind. For example, the anti-enhancement factor portion can bind a cytotoxic T-cell (e.g., via CD2, CD3, CD8, CD28, CD4, CD40, ICAM-1 or other immune cell that results in an increased immune response against the target cell).

In one embodiment, the bispecific molecules according to at least some embodiments of the invention comprise as a binding specificity at least one antibody, or an antibody fragment thereof, including, e.g., an Fab, Fab', F(ab')₂, Fv, or a single chain Fv. The antibody may also be a light chain or heavy chain dimer, or any minimal fragment thereof such as a Fv or a single chain construct as described in Ladner et al. U.S. Pat. No. 4,946,778, the contents of which is expressly incorporated by reference.

The production and characterization of certain preferred anti-Fc gamma. monoclonal antibodies are described by Fanger et al. in PCT Publication WO 88/00052 and in U.S. Pat. No. 4,954,617, the teachings of which are fully incorporated by reference herein. These antibodies bind to an epitope of Fc R1, FcyRII or FcyRIII at a site which is distinct from the Fc binding site of the receptor and, thus, their binding is not blocked substantially by physiological levels of IgG. Specific anti-Fc RI antibodies useful in this invention are mAb 22, mAb 32, mAb 44, mAb 62 and mAb 197. The hybridoma producing mAb 32 is available from the American Type Culture Collection, ATCC Accession No. HB9469. In other embodiments, the anti-Fcy receptor antibody is a humanized form of monoclonal antibody 22 (H22). The production and characterization of the H22 antibody is described in Graziano, R.F. et al. (1995) J. Immunol. 155 (10): 4996-5002 and PCT Publication WO 94/10332. The H22 antibody producing cell line is deposited at the American Type Culture Collection under the designation HAO22CLI and has the accession no. CRL 11177.

In still other preferred embodiments, the binding specificity for an Fc receptor is provided by an antibody that binds to a human IgA receptor, e.g., an Fc-alpha receptor (Fc alpha RI(CD89)), the binding of which is preferably not blocked by human immunoglobulin A (IgA). The term "IgA receptor" is intended to include the gene product of one alpha.-gene (Fc alpha RI) located on chromosome 19. This gene is known to encode several alternatively spliced transmembrane isoforms of 55 to 10 kDa.

Fc alpha RI (CD89) is constitutively expressed on monocytes/macrophages, eosinophilic and neutrophilic granulocytes, but not on non-effector cell populations. Fc alpha RI has medium affinity (Approximately 5X10-7 M-1) for both IgA1 and IgA2, which is increased upon exposure to cytokines such as G-CSF or GM-CSF (Morton, H. C. et al. (1996) Critical Reviews in Immunology 16:423-440). Four Fca RI-specific monoclonal antibodies, identified as A3, A59, A62 and A77, which bind Fc alpha RI outside the IgA ligand binding domain, have been described (Monteiro, R. C. et al. (1992) J. Immunol. 148:1764).

Fc alpha RI and Fc gamma RI are preferred trigger receptors for use in the bispecific molecules according to at least some embodiments of the invention because they are (1) expressed primarily on immune effector cells, e.g., monocytes, PMNs, macrophages and dendritic cells; (2) expressed at high levels (e.g., 5,000-100,000 per cell); (3) mediators of cytotoxic activities (e.g., ADCC, phagocytosis); (4) mediate enhanced antigen presentation of antigens, including self-antigens, targeted to them.

While human monoclonal antibodies are preferred, other antibodies which can be employed in the bispecific molecules according to at least some embodiments of the invention are murine, chimeric and humanized monoclonal antibodies.

The bispecific molecules according to at least some embodiments of the present invention can be prepared by conjugating the constituent binding specificities, e.g., the anti-FcR and anti-KRTCAP3, anti-FAM26F, anti-MGC52498, anti-FAM70A, or anti-TMEM154 polypeptide binding specificities, using methods known in the art. For example, each binding specificity of the bispecific molecule can be generated separately and then conjugated to one another. When the binding specificities are proteins or peptides, a variety of coupling or cross-linking agents can be used for covalent conjugation. Examples of cross-linking agents include protein A, carbodiimide, N-succinimidyl-S-acetyl-thioacetate (SATA), 5,5'-dithiobis(2-nitrobenzoic acid) (DTNB), o-phenylenedimaleimide (oPDM), N-succinimidyl-3-(2-pyridyld- ithio)propionate (SPDP), and sulfosuccinimidyl 4-(N-maleimidomethyl) cyclohaxane-1-carboxylate (sulfo-SMCC) (see e.g., Karpovsky et al. (1984) J. Exp. Med. 160:1686; Liu, M A et al. (1985) Proc. Natl. Acad. Sci. USA 82:8648). Other methods include those described in Paulus (1985) Behring Ins. Mitt. No. 78, 118-132; Brennan et al. (1985) Science 229:81-83), and Glennie et al. (1987) J. Immunol. 139: 2367-2375). Preferred conjugating agents are SATA and sulfo-SMCC, both available from Pierce Chemical Co. (Rockford, Ill.).

When the binding specificities are antibodies, they can be conjugated via sulfhydryl bonding of the C-terminus hinge regions of the two heavy chains. In a particularly preferred embodiment, the hinge region is modified to contain an odd number of sulfhydryl residues, preferably one, prior to conjugation.

Alternatively, both binding specificities can be encoded in the same vector and expressed and assembled in the same host cell. This method is particularly useful where the bispecific molecule is a mAbXmAb, mAbXFab, FabXF(ab')2 or ligandXFab fusion protein. A bispecific molecule according to at least some embodiments of the invention can be a single chain molecule comprising one single chain antibody and a binding determinant, or a single chain bispecific molecule comprising two binding determinants. Bispecific molecules may comprise at least two single chain molecules. Methods for preparing bispecific molecules are described for example in U.S. Pat. No. 5,260,203; U.S. Pat. No. 5,455,030; U.S. Pat. No. 4,881,175; U.S. Pat. No. 5,132,405; U.S. Pat. No. 5,091,513; U.S. Pat. No. 5,476,786; U.S. Pat. No. 5,013,653; U.S. Pat. No. 5,258,498; and U.S. Pat. No. 5,482,858.

Binding of the bispecific molecules to their specific targets can be confirmed by, for example, enzyme-linked immunosorbent assay (ELISA), radioimmunoassay (RIA), FACS analysis, bioassay (e.g., growth inhibition), or Western Blot assay. Each of these assays generally detects the presence of protein-antibody complexes of particular interest by employing a labeled reagent (e.g., an antibody) specific for the complex of interest. For example, the FcR-antibody complexes can be detected using e.g., an enzyme-linked antibody or antibody fragment which recognizes and specifically binds to the antibody-FcR complexes. Alternatively, the complexes can be detected using any of a variety of other immunoassays. For example, the antibody can be radioactively labeled and used in a radioimmunoassay (RIA) (see, for example, Weintraub, B., Principles of Radioimmunoassays, Seventh Training Course on Radioligand Assay Techniques, The Endocrine Society, March, 1986, which is incorporated by reference herein). The radioactive isotope can be detected by such means as the use of a gamma. counter or a scintillation counter or by autoradiography.

### PHARMACEUTICAL COMPOSITIONS

In another aspect, the present invention provides a composition, e.g., a pharmaceutical composition, containing one or a combination of monoclonal antibodies, or antigen-binding portions thereof, according to at least some embodiments of the present invention, formulated together with a pharmaceutically acceptable carrier. Such compositions may include one or a combination of (e.g., two or more different) antibodies, or immunoconjugates or bispecific molecules according to at least some embodiments of the invention. For example, a pharmaceutical composition according to at least some embodiments of the invention can comprise a combination of antibodies (or immunoconjugates or bispecifics) that bind to different epitopes on the target antigen or that have complementary activities.

As discussed supra, at least some embodiments of the present invention further embrace identifying other molecules such as small organic molecules, peptides, ribozymes, carbohydrates, glycoprotein, siRNAs, antisense RNAs and the like which specifically bind and/or modulate (enhance or inhibit) an activity elicited by the KRTCAP3, FAM26F, MGC52498, FAM70A, or TMEM154 antigen or polypeptides, respectively. These molecules may be identified by known screening methods such as binding assays. Typically these assays will be high throughput and will screen a large library of synthesized or native compounds in order to identify putative drug candidates that bind and/or modulate KRTCAP3, FAM26F, MGC52498, FAM70A, or TMEM154 related activities.

Specifically, the invention embraces the development of drugs containing the ectodomain of the TMEM154 antigen or polypeptide, or a fragment or variant thereof or a corresponding nucleic acid sequence encoding.

Thus, the present invention features a pharmaceutical composition comprising a therapeutically effective amount of a therapeutic agent according to the present invention. According to the present invention the therapeutic agent could be any one of TMEM154 ectodomain, or a fragment or a variant or a conjugate thereof, or a corresponding nucleic acid sequence encoding same.

The pharmaceutical composition according to the present invention is further optionally used for the treatment of cancer and/or immune related conditions or disorders.

The therapeutic agents according to at least some embodiments of the present invention can be provided to the subject alone, or as part of a pharmaceutical composition where they are mixed with a pharmaceutically acceptable carrier.

Pharmaceutical compositions according to at least some embodiments of the invention also can be administered in combination therapy, i.e., combined with other agents. For example, the combination therapy can include an anti-KRTCAP3, anti-FAM26F, anti-MGC52498, anti-FAM70A, or anti-TMEM154 antibody or KRTCAP3, FAM26F, MGC52498, FAM70A, or TMEM154 modulating agent according to the present invention such as a soluble polypeptide conjugate containing the ectodomain of the TMEM154 polypeptide or a small molecule such as a peptide, ribozyme, siRNA, or other drug that binds a KRTCAP3, FAM26F, MGC52498, FAM70A, or TMEM154 polypeptide, combined with at least one other therapeutic or immune modulatory agent.

A composition according to at least some embodiments of the present invention can be administered via one or more routes of administration using one or more of a variety of methods known in the art. As will be appreciated by the skilled artisan, the route and/or mode of administration will vary depending upon the desired results. Preferred routes of administration for antibodies according to at least some embodiments of the invention include intravenous, intramuscular, intradermal, intraperitoneal, subcutaneous, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g., by injection or infusion). Depending on the route of administration, the active compound, i.e., antibody, immunoconjugate, or bispecific molecule, may be coated in a material to protect the compound from the action of acids and other natural conditions that may inactivate the compound.

A pharmaceutical composition according to at least some embodiments of the invention also may include a pharmaceutically acceptable anti-oxidant. Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like. Examples of suitable aqueous and nonaqueous carriers that may be employed in the pharmaceutical compositions according to at least some embodiments of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions according to at least some embodiments of the invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin. Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect. Generally, out of one hundred per cent, this amount will range from about 0.01 per cent to about ninety-nine percent of active ingredient, optionally from about 0.1 per cent to about 70 per cent, optionally from about 1 per cent to about 30 per cent of active ingredient in combination with a pharmaceutically acceptable carrier.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms according to at least some embodiments of the invention are dictated by and directly dependent on (a) the unique characteristics of the active compound and the particular therapeutic effect to be achieved, and (b) the limitations inherent in the art of compounding such an active compound for the treatment of sensitivity in individuals.

For administration of the antibody, the dosage ranges from about 0.0001 to 100 mg/kg, and more usually 0.01 to 5 mg/kg, of the host body weight. For example dosages can be 0.3 mg/kg body weight, 1 mg/kg body weight, 3 mg/kg body weight, 5 mg/kg body weight or 10 mg/kg body weight or within the range of 1-10 mg/kg. An exemplary treatment regime entails administration once per week, once every two weeks, once every three weeks, once every four weeks, once a month, once every 3 months or once every three to 6 months.

Alternatively, antibody can be administered as a sustained release formulation, in which case less frequent administration is required. Dosage and frequency vary depending on the half-life of the antibody in the patient. In general, human antibodies show the longest half life, followed by humanized antibodies, chimeric antibodies, and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

Actual dosage levels of the active ingredients in the pharmaceutical compositions according to at least some embodiments of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions according to at least some embodiments of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A "therapeutically effective dosage" of an anti-KRTCAP3, anti-FAM26F, anti-MGC52498, anti-FAM70A, or anti-TMEM154 antibody according to at least some embodiments of the invention preferably results in a decrease in severity of disease symptoms, an increase in frequency and duration of disease symptom-free periods, an increase in lifespan, disease remission, or a prevention of impairment or disability due to the disease affliction. For example, for the treatment of KRTCAP3, FAM26F, MGC52498, FAM70A, or TMEM154 polypeptide positive tumors, e.g., ovarian tumors, lung tumors, breast tumors, colon tumors, kidney tumors, liver tumors, pancreatic tumors, prostate cancer, melanoma and hematological malignancies such as Multiple Myeloma, lymphoma, Non-Hodgkin's lymphoma, anti CD20 (i.e. Rituximab) resistant lymphoma, leukemia, T cell leukemia, a "therapeutically effective dosage" optionally inhibits cell growth or tumor growth by at least about 20%, 40%, 60%, 80% relative to untreated subjects. The ability of a compound to inhibit tumor growth can be evaluated in an animal model system predictive of efficacy in human tumors. Alternatively, this property of a composition can be evaluated by examining the ability of the compound to inhibit, such inhibition in vitro by assays known to the skilled practitioner.

Alternatively or additionally, a "therapeutically effective dosage" preferably results in at least stable disease, preferably partial response, more preferably complete response, as assessed by the WHO or RECIST criteria for tumor response (Natl Cancer Inst 1999;91:523-8 and Cancer 1981;47:207-14).

A therapeutically effective amount of a therapeutic compound can decrease tumor size, or otherwise ameliorate symptoms in a subject, or otherwise support partial or complete stable disease and/or partial or complete response as determined above. One of ordinary skill in the art would be able to determine such amounts based on such factors as the subject's size, the severity of the subject's symptoms, and the particular composition or route of administration selected.

Therapeutic compositions can be administered with medical devices known in the art. For example, in a preferred embodiment, a therapeutic composition according to at least some embodiments of the invention can be administered with a needles hypodermic injection device, such as the devices disclosed in U.S. Pat. Nos. 5,399,163; 5,383,851; 5,312,335; 5,064,413; 4,941,880; 4,790,824; or 4,596,556. Examples of well-known implants and modules useful in the present invention include: U.S. Pat. No. 4,487,603, which discloses an implantable micro-infusion pump for dispensing medication at a controlled rate; U.S. Pat. No. 4,486,194, which discloses a therapeutic device for administering medicaments through the skin; U.S. Pat. No. 4,447,233, which discloses a medication infusion pump for delivering medication at a precise infusion rate; U.S. Pat. No. 4,447,224, which discloses a variable flow implantable infusion apparatus for continuous drug delivery; U.S. Pat. No. 4,439,196, which discloses an osmotic drug delivery system having multi-chamber compartments; and U.S. Pat. No. 4,475,196, which discloses an osmotic drug delivery system. These patents are incorporated herein by reference. Many other such implants, delivery systems, and modules are known to those skilled in the art.

In certain embodiments, the antibodies or other KRTCAP3, FAM26F, MGC52498, FAM70A, or TMEM154 related drugs according to at least some embodiments of the invention can be formulated to ensure proper distribution in vivo. For example, the blood-brain barrier (BBB) excludes many highly hydrophilic compounds. To ensure that the therapeutic compounds according to at least some embodiments of the invention cross the BBB (if desired), they can be formulated, for example, in liposomes. For methods of manufacturing liposomes, see, e.g., U.S. Pat. Nos. 4,522,811; 5,374,548; and 5,399,331. The liposomes may comprise one or more moieties which are selectively transported into specific cells or organs, thus enhance targeted drug delivery (see, e.g., V. V. Ranade (1989) J. Clin. Pharmacol. 29:685). Exemplary targeting moieties include folate or biotin (see, e.g., U.S. Pat. No. 5,416,016 to Low et al.); mannosides (Umezawa et al., (1988) Biochem. Biophys. Res. Commun. 153:1038); antibodies (P. G. Bloeman et al. (1995) FEBS Lett. 357:140; M. Owais et al. (1995) Antimicrob. Agents Chemother. 39:180); surfactant protein A receptor (Briscoe et al. (1995) Am. J Physiol. 1233:134); p120 (Schreier et al. (1994) J. Biol. Chem. 269:9090); see also K. Keinanen; M. L. Laukkanen (1994) FEBS Lett. 346:123; J. J. Killion; I. J. Fidler (1994) Immunomethods 4:273.

Given the specific binding of the antibodies according to at least some embodiments of the invention for KRTCAP3, FAM26F, MGC52498, FAM70A or TMEM154 polypeptides, the antibodies can be used to specifically detect KRTCAP3, FAM26F, MGC52498, FAM70A or TMEM154 expression on the surface of cells and, moreover, can be used to purify KRTCAP3, FAM26F, MGC52498, FAM70A or TMEM154 antigen via immunoaffinity purification.

Furthermore, given the expression of KRTCAP3, FAM26F, MGC52498, FAM70A or TMEM154 polypeptides on various tumor cells, the human antibodies, antibody compositions and methods according to at least some embodiments of the present invention can be used to treat a subject with a tumorigenic disorder, e.g., a disorder characterized by the presence of tumor cells expressing KRTCAP3, FAM26F, MGC52498, FAM70A or TMEM154 antigen such as ovarian cancer, colon cancer, lung cancer, breast cancer, kidney cancer, liver cancer, pancreatic cancer, prostate cancer, melanoma and hematological malignancies such as Multiple Myeloma, lymphoma, Non-Hodgkin's lymphoma, anti CD20 (i.e. Rituximab) resistant lymphoma, leukemia, T cell leukemia, as mentioned.

In one embodiment, the antibodies (e.g., human monoclonal antibodies, multispecific and bispecific molecules and compositions) according to at least some embodiments of the invention can be used to detect levels of a KRTCAP3, FAM26F, MGC52498, FAM70A or TMEM154 polypeptide or levels of cells which contain a KRTCAP3, FAM26F, MGC52498, FAM70A or TMEM154 polypeptide, respectively, on their membrane surface, which levels can then be linked to certain disease symptoms.

Alternatively, the antibodies can be used to inhibit or block functioning of KRTCAP3, FAM26F, MGC52498, FAM70A or TMEM154 polypeptides which, in turn, can be linked to the prevention or amelioration of certain disease symptoms, thereby implicating KRTCAP3, FAM26F, MGC52498, FAM70A or TMEM154 polypeptides, respectively, as a mediator of the disease. This can be achieved by contacting a sample and a control sample with the anti- KRTCAP3, anti-FAM26F, anti-MGC52498, anti-FAM70A or anti-TMEM154 antibody under conditions that allow for the formation of a complex between the corresponding antibody and KRTCAP3, FAM26F, MGC52498, FAM70A or TMEM154 polypeptides, respectively. Any complexes formed between the antibody and KRTCAP3, FAM26F, MGC52498, FAM70A or TMEM154 polypeptides are detected and compared in the sample and the control.

In another embodiment, the antibodies (e.g., human antibodies, multispecific and bispecific molecules and compositions) according to at least some embodiments of the invention can be initially tested for binding activity associated with therapeutic or diagnostic use in vitro. For example, compositions according to at least some embodiments of the invention can be tested using low cytometric assays.

As previously described, human anti-KRTCAP3, anti-FAM26F, anti-MGC52498, anti-FAM70A or anti-TMEM154 antibodies according to at least some embodiments of the invention can be co-administered with one or other more therapeutic agents, e.g., an cytotoxic agent, a radiotoxic agent or an immunosuppressive agent. The antibody can be linked to the agent (as an immunocomplex) or can be administered separate from the agent. In the latter case (separate administration), the antibody can be administered before, after or concurrently with the agent or can be co-administered with other known therapies, e.g., an anti-cancer therapy, e.g., radiation. Such therapeutic agents include, among others, anti-neoplastic agents such as doxorubicin (adriamycin), cisplatin bleomycin sulfate, carmustine, chlorambucil, and cyclophosphamide hydroxyurea which, by themselves, are only effective at levels which are toxic or subtoxic to a patient. Cisplatin is intravenously administered as a 100 mg/dose once every four weeks and adriamycin is intravenously administered as a 60-75 mg/ml dose once every 21 days. Co-administration of the human anti- KRTCAP3, anti-FAM26F, anti-MGC52498, anti-FAM70A or anti-TMEM154 antibodies, or antigen binding fragments thereof, according to at least some embodiments of the present invention with chemotherapeutic agents provides two anti-cancer agents which operate via different mechanisms which yield a cytotoxic effect to human tumor cells. Such co-administration can solve problems due to development of resistance to drugs or a change in the antigenicity of the tumor cells which would render them unreactive with the antibody.

Also within the scope according to at least some embodiments of the present invention are kits comprising the KRTCAP3, FAM26F, MGC52498, FAM70A or TMEM154 polypeptide or antibody compositions according to at least some embodiments of the invention (e.g., human antibodies, bispecific or multispecific molecules, or immunoconjugates) and instructions for use. The kit can further contain one ore more additional reagents, such as an immunosuppressive reagent, a cytotoxic agent or a radiotoxic agent, or one or more additional human antibodies according to at least some embodiments of the invention (e.g., a human antibody having a complementary activity which binds to an epitope in the KRTCAP3, FAM26F, MGC52498, FAM70A or TMEM154 antigen distinct from the first human antibody).

In other embodiments, the subject can be additionally treated with an agent that modulates, e.g., enhances or inhibits, the expression or activity of Fcy or Fcy receptors by, for example, treating the subject with a cytokine. Preferred cytokines for administration during treatment with the multispecific molecule include of granulocyte colony-stimulating factor (G-CSF), granulocyte- macrophage colony-stimulating factor (GM-CSF), interferon-.gamma. (IFN-.gamma.), and tumor necrosis factor (TNF).

The compositions (e.g., human antibodies, multispecific and bispecific molecules) according to at least some embodiments of the invention can also be used to target cells expressing Fc gamma R or KRTCAP3, FAM26F, MGC52498, FAM70A or TMEM154, for example for labeling such cells. For such use, the binding agent can be linked to a molecule that can be detected. Thus, the invention provides methods for localizing ex vivo or in vitro cells expressing Fc receptors, such as FcgammaR, or KRTCAP3, FAM26F, MGC52498, FAM70A or TMEM154 antigen. The detectable label can be, e.g., a radioisotope, a fluorescent compound, an enzyme, or an enzyme co-factor.

According to an aspect of the invention, here designated as item 1, there is provided a polyclonal or monoclonal antibody or fragment that specifically binds to at least one of the TMEM154 polypeptides selected from the group consisting of SEQ ID NOs: 42-46, 63, 64, 161, 162, 191, 192, or a fragment or a variant, or a homolog thereof possessing at least 85% sequence identity therewith.

According to an aspect of the invention, here designated as item 2, an antibody or fragment according to item 1 is provided, wherein said antibody blocks or inhibits the interaction of at least one of the polypeptides selected from the group consisting of SEQ ID NOs: 42-46 with a counterpart.

According to an aspect of the invention, here designated as item 3, an antibody or fragment according to item 1 is provided, wherein said antibody or fragment replaces or augments the interaction of at least one of the polypeptides selected from the group consisting of SEQ ID NOs: 42-46 with a counterpart.

According to an aspect of the invention, an antibody or fragment according to item 1 is provided, here designated as item 4, which is suitable for treatment or prevention of cancer or immune related condition, by modulating the activity of at least one of the TMEM154 proteins selected from the group consisting of SEQ ID NOs: 42-46.

According to an aspect of the invention, an antibody or fragment according to item 4 is provided, here designated as item 5, wherein the cancer is selected from the group consisting of Non-Hodgkin's Lymphoma, Hodgkin's Lymphoma, anti CD20 (i.e. Rituximab) resistant lymphoma, Multiple Myeloma, kidney cancer and pancreatic cancer.

According to an aspect of the invention, an antibody or fragment according to item 4 is provided, here designated as item 6, wherein the immune related condition is SLE (systemic lupus erythematosus).

According to an aspect of the invention, an antibody or fragment according to item 1 is provided, here designated as item 7, wherein the antigen binding site contains from about 3-7 contiguous or non-contiguous amino acids of any of the above sequences.

According to an aspect of the invention, an antibody or fragment according to item 1 is provided, here designated as item 8, wherein the antibody is a fully human antibody, a humanized or primatized antibody, or a chimeric antibody.

According to an aspect of the invention, an antibody or fragment according to item 1 is provided, here designated as item 9, wherein the antibody is selected from the group consisting of Fab, Fab', F(ab')2, F(ab'), F(ab), Fv or scFv fragment and minimal recognition unit.

According to an aspect of the invention, an antibody or fragment according to item 1 is provided, here designated as item 10, wherein the antibody is coupled to a detectable marker, or to an effector moiety.

According to an aspect of the invention, an antibody or fragment according to item 10 is provided, here designated as item 11, wherein the effector moiety is one or more of a radionuclide, fluorophore, an enzyme, a toxin, a therapeutic agent, a chemotherapeutic agent, a cytokine antibody, a cytokine receptor, or an immunomodulatory agent.

According to an aspect of the invention, an antibody or fragment according to item 10 is provided, here designated as item 12, wherein the detectable marker is one or more of a radioisotope, a metal chelator, an enzyme, a fluorescent compound, a bioluminescent compound or a chemiluminescent compound.

According to an aspect of the invention, a pharmaceutical composition is provided, here designated as item 13, which comprises an antibody or a fragment according to any one of items 1-12.

According to an aspect of the invention, a method for modulating lymphocyte activity is provided, here designated as item 14, comprising contacting a lymphocyte, positive for a TMEM154 polypeptide selected from the group consisting of SEQ ID NOs: 42-46, with a bioactive agent capable of modulating TMEM154-mediated signaling in an amount effective to modulate at least one lymphocyte activity.

According to an aspect of the invention, a method according to item 14 is provided, here designated as item 15, wherein said agent comprises an antagonist of TMEM154-mediated signaling, and wherein said contacting inhibits the attenuation of lymphocyte activity mediated by such signaling.

According to an aspect of the invention, a method according to item 15 is provided, here designated as item 16, wherein said contacting increases lymphocyte activity.

According to an aspect of the invention, a method according to item 15 is provided, here designated as item 17, wherein said antagonist comprises a blocking agent capable of interfering with the functional interaction of TMEM154 antigen and its counterpart.

According to an aspect of the invention, a method according to item 15 is provided, here designated as item 18, wherein the administered antagonist is an antibody or fragment which is suitable for treatment or prevention of cancer by modulating the activity of any one of the TMEM154 proteins.

According to an aspect of the invention, a method according to item 15 is provided, here designated as item 19, wherein the administered antibody or fragment inhibits negative stimulation of T cell activity against cancer cells.

According to an aspect of the invention, a method of treating or preventing TMEM154 positive cancer or immune related condition is provided, here designated as item 20, comprising administering to the patient a therapeutically effective amount of an antibody or fragment or pharmaceutical composition according to anyone of items 1-13.

According to an aspect of the invention, a method according to item 20 is provided, here designated as item 21, wherein the treatment is provided in combination with another medicament or therapeutic method.

According to an aspect of the invention, a method according to item 20 is provided, here designated as item 22, wherein the cancer is selected from the group consisting of Non-Hodgkin's Lymphoma, Hodgkin's Lymphoma, anti CD20 (i.e. Rituximab) resistant lymphoma, Multiple Myeloma, kidney cancer and pancreatic cancer.

According to an aspect of the invention, a method according to item 20 is provided, here designated as item 23, wherein the immune related condition is SLE (systemic lupus erythematosus).

(///original claim 24///) According to an aspect of the invention, an antibody or fragment is provided, here designated as item 24, which specifically binds at least one polypeptide of any of SEQ ID NOs:42-46, 63, 64, 161, 162, 191, 192, or a fragment or variant thereof, for diagnosing cancer or immune related condition characterized by differential expression of at least one of the TMEM154 polypeptides selected from the group consisting of SEQ ID NOs: 42-46, or a fragment or variant thereof.

According to an aspect of the invention, an antibody or fragment according to item 24 is provided, here designated as item 25, wherein the cancer is selected from the group consisting of Non-Hodgkin's Lymphoma, Hodgkin's Lymphoma, anti CD20 (i.e. Rituximab) resistant lymphoma, Multiple Myeloma, kidney cancer and pancreatic cancer.

According to an aspect of the invention, an antibody or fragment according to item 24 is provided, here designated as item 26, wherein the immune related condition is SLE (systemic lupus erythematosus).

According to an aspect of the invention, a method for diagnosing a cancer or an immune related condition in a subject is provided, here designated as item 27, comprising detecting in the subject or in a sample obtained from said subject the presence of a polypeptide and/or an overexpressed level of said polypeptide having a sequence at least 85% homologous to any one of the TMEM154 polypeptides, having an amino acid sequence selected from the group consisting of SEQ ID NOs: 42-46, 63, 64, 161, 162, or a fragment thereof; wherein said overexpressed level is determined with regard to a normal level of said polypeptide in a corresponding normal tissue.

According to an aspect of the invention, a method according to item 27 is provided, here designated as item 28, wherein the detection is conducted by immunoassay.

According to an aspect of the invention, a method according to item 28 is provided, here designated as item 29, wherein the immunoassay utilizes an antibody according to any of items 1, 10, or 24.

According to an aspect of the invention, an assay is provided, here designated as item 30, for detecting the presence of any one of the TMEM154 proteins, selected from the group consisting of any of SEQ ID NOs: 42-46, or a fragment or variant thereof in a biological sample comprising contacting the sample with an antibody or a fragment according to any of items 1, 10, or 24, in the sample.

According to an aspect of the invention, a method according to item 27 is provided, here designated as item 31, wherein detecting the presence and/or the overexpressed level of the TMEM154 polypeptides, or a fragment or variant thereof is performed in vivo or in vitro.

According to an aspect of the invention, a method for diagnosing a cancer or an immune related condition in a subject is provided, here designated as item 32, comprising detecting in the subject or in a sample obtained from said subject a polynucleotide and/or an overexpressed level of said polynucleotide having a sequence at least 85% homologous to the nucleic acid sequence as set forth in at least one of SEQ ID NOs:23, 38-41, or 106; wherein said overexpressed level is determined with regard to a normal level of said polynucleotide in a corresponding normal tissue.

According to an aspect of the invention, a method according to item 27 or 32 is provided, here designated as item 33, wherein diagnosing comprises screening for cancer or immune related condition in a subject, detecting a presence or a severity of cancer or immune related condition in a subject, distinguishing cancer or immune related condition from other diseases, providing prognosis of cancer or immune related condition, monitoring progression or relapse of cancer or immune related condition in a subject, assessment of treatment efficacy or relapse of cancer or immune related condition in a subject, selecting a therapy and a treatment for cancer or immune related condition in a subject, optimization of a given therapy for cancer or immune related condition in a subject, monitoring the treatment of cancer or immune related condition in a subject, predicting the suitability of a therapy for specific patients or subpopulations, determining the appropriate dosing of a therapeutic product in patients or subpopulations.

According to an aspect of the invention, a method according to item 27 or 32 is provided, here designated as item 34, wherein the cancer is selected from the group consisting of Non-Hodgkin's Lymphoma, Hodgkin's Lymphoma, anti CD20 (i.e. Rituximab) resistant lymphoma, Multiple Myeloma, kidney cancer and pancreatic cancer.

According to an aspect of the invention, a method according to item 27 or 32 is provided, here designated as item 35, wherein the immune related condition is SLE (systemic lupus erythematosus).

According to an aspect of the invention, a method according to item 32 is provided, here designated as item 36, wherein the detection is performed using an oligonucleotide pair capable of hybridizing to at least a portion of a nucleic acid sequence at least 85% homologous to the nucleic acid sequence set forth in any of SEQ. ID NOs: 23, 38-41, or 106.

According to an aspect of the invention, a method according to item 36 is provided, here designated as item 37, wherein the detection is performed using an oligonucleotide pair as set forth in any one of SEQ. ID NOs:104-105.

According to an aspect of the invention, an isolated polynucleotide is provided, here designated as item 38, comprising an amplicon having a nucleic acid sequence set forth in SEQ ID NO:106, or fragments thereof, or polynucleotides homologous thereto.

According to an aspect of the invention, a primer pair is provided, here designated as item 39, comprising a pair of isolated oligonucleotides capable of amplifying the nucleic acid sequence as set forth in at least one of SEQ ID NOs:23, 38-41, or 106 or fragments thereof, or polynucleotides homologous thereto.

According to an aspect of the invention, a primer pair according to item 39 is provided, here designated as item 40, comprising a pair of isolated oligonucleotides having a sequence selected from the group consisting of SEQ. ID NOs:104-105.

According to an aspect of the invention, an isolated polypeptide is provided, here designated as item 41, of TMEM154 ectodomain or fragment or variant thereof that possesses at least 95% sequence identity therewith.

According to an aspect of the invention, an isolated polypeptide according to item 41 is provided, here designated as item 42, comprising an amino acid sequence having at least 95% sequence identity with a sequence selected from the group consisting of amino acid residues 23-75 of the sequences W38346_P3 (SEQ ID NO:42), or W38346_P7 (SEQ ID NO:46), corresponding to amino acid sequence depicted in SEQ ID NO:63, or amino acid residues 20-105 of the sequence W38346_P4 (SEQ ID NO:45), corresponding to the amino acid sequence depicted in SEQ ID NO:64, or fragment thereof, having at least 95% sequence identity with the amino acid sequence set forth in any one of SEQ ID NOs: 63, 64, 161, 162, 191, or 192.

According to an aspect of the invention, a polypeptide according to either item 41 or 42 is provided, here designated as item 43, which is fused to a non-TMEM154 protein sequence, or attached to a detectable or therapeutic moiety.

According to an aspect of the invention, a fused protein according to item 43 is provided, here designated as item 44, wherein the non-TMEM154 protein is at least a portion of an immunoglobulin molecule.

According to an aspect of the invention, a nucleic acid sequence is provided, here designated as item 45, encoding a TMEM154 ectodomain polypeptide according to any of items 41, 42, 43 or 44.

According to an aspect of the invention, a nucleic acid sequence according to item 45 is provided, here designated as item 46, having a sequence as set forth in SEQ ID NO:23, or fragment or variant thereof that possesses at least 95% sequence identity therewith.

According to an aspect of the invention, an expression vector is provided, here designated as item 47, containing a nucleic acid sequence according to either items 45 or 46.

According to an aspect of the invention, a host cell is provided, here designated as item 48, comprising an expression vector of claim 47.

According to an aspect of the invention, a method of producing a TMEM154 ectodomain polypeptide, or fragment or conjugate thereof is provided, here designated as item 49, comprising culturing the host cell according to item 48, under conditions whereby the cell expresses the polypeptide encoded by the DNA segment or nucleic acid and recovering said polypeptide.

According to an aspect of the invention, a pharmaceutical composition is provided, here designated as item 50, comprising at least one polypeptide according to any of items 41, 42, 43, or 44, and further comprising a pharmaceutically acceptable diluent or carrier.

According to an aspect of the invention, a method for treating or preventing cancer or immune related condition is provided, here designated as item 51, comprising administering to a subject in need thereof a pharmaceutical composition according to item 50.

According to an aspect of the invention, a method according to item 51 is provided, here designated as item 52, wherein the cancer is selected from the group consisting of Non-Hodgkin's Lymphoma, Hodgkin's Lymphoma, anti CD20 (i.e. Rituximab) resistant lymphoma, Multiple Myeloma, kidney cancer, and pancreatic cancer.

According to an aspect of the invention, a method according to item 51 is provided, here designated as item 53, wherein the immune related condition is systemic lupus erythematosus (SLE).

According to an aspect of the invention, an siRNA, antisense RNA, or ribozyme is provided, here designated item 54, which binds the transcript encoding any one of the TMEM154 polypeptides, and inhibits its expression.

According to an aspect of the invention, a pharmaceutical composition according to either of items 13 or 50 is provided, here designated item 55, for use in combination with another medicament or therapeutic method.

### DIAGNOSTIC USES OF KRTCAP3, FAM26F, MGC52498, FAM70A, OR TMEM154 POLYPEPTIDES, POLYNUCLEOTIDES AND ANTIBODIES

In certain embodiments the polypeptides and/or polynucleotides according to at least some embodiments of the present invention are used as markers for diagnosis of diseases wherein KRTCAP3, FAM26F, MGC52498, FAM70A, OR TMEM154 polypeptides and/or polynucleotides are differentially present. According to at least some embodiments, the diseases are selected from but not limited to cancer, and immune related conditions (as defined herein).

According to further embodiments markers according to at least some embodiments of the present invention might optionally be used alone or in combination one or more other compounds described herein, and/or in combination with known markers for lung cancer, including but not limited to CEA, CA15-3, Beta-2-microglobulin, CA19-9, TPA, and/or in combination with the known proteins for the variant marker as described herein.

According to further embodiments markers according to at least some embodiments of the present invention might optionally be used alone or in combination with one or more other compounds described herein, and/or in combination known markers for ovarian cancer, including but not limited to CEA, CA125 (Mucin 16), CA72-4TAG, CA-50, CA 54-61, CA-195 and CA 19-9 in combination with CA-125, and/or in combination with the known proteins for the variant marker as described herein.

According to further embodiments markers according to at least some embodiments of the present invention might optionally be used alone or in combination with one or more other compounds described herein, and/or in combination with known markers for breast cancer, including but not limited to Calcitonin, CA15-3 (Mucin1), CA27-29, TPA, a combination of CA 15-3 and CEA, CA 27.29 (monoclonal antibody directed against MUC1), Estrogen 2 (beta), HER-2 (c-erbB2), and/or in combination with the known proteins for the variant marker as described herein.

According to further embodiments markers according to at least some embodiments of the present invention might optionally be used alone or in combination with one or more other compounds described herein, and/or in combination with known markers for renal cancer, including but not limited to urinary protein, creatinine or creatinine clearance, and/or markers used for the diagnosis or assessment of prognosis of renal cancer, specifically of renal cell carcinoma, including but not limited to vascular endothelial growth factor, interleukin-12, the soluble interleukin-2 receptor, intercellular adhesion molecule-1, human chorionic gonadotropin beta, insulin-like growth factor-1 receptor, Carbonic anhydrase 9 (CA 9), endostatin, Thymidine phosphorylase and/or in combination with the known proteins for the variant marker as described herein.

According to further embodiments markers according to at least some embodiments of the present invention might optionally be used alone or in combination with one or more other compounds described herein, and/or in combination with known markers for liver cancer, including but not limited to Alpha fetoprotein (AFP), des-gamma-carboxyprothrombin (DCP), Squamous cell carcinoma antigen (SCCA)-immunoglobulin M (IgM), AFP (L3), or fucosylated AFP, GP73 (a golgi protein marker) and its fucosylated form, (TGF)-beta1, HS-GGT, free insulin-like growth factor (IGF)-II.

According to further embodiments markers according to at least some embodiments of the present invention might optionally be used alone or in combination with one or more other compounds described herein, and/or in combination with known markers for melanoma cancer, including but not limited to S100-beta, melanoma inhibitory activity (MIA), lactate dehydrogenase (LDH), tyrosinase, 5-S-Cysteinyldopa, L-Dopa/L-tyrosine, VEGF, bFGF, IL-8, ICAM-1, MMPs, IL-6, IL-10, sIL-2R (soluble interleukin-2-receptor), sHLA-DR (soluble HLA-DR), sHLA-class-1 (soluble HLA-class I), TuM2-PK, Fas/CD95, sHLA-class-I (soluble HLA-class I), Albumin, TuM2-PK (Tumour pyruvate kinase type M2), sFas/CD95, YKL-40, CYT-MAA (cytoplasmic melanoma-associated antigen), HMW-MAA (high-molecular-weight melanoma-associated antigen), STAT3, STAT1, gp100/HMB45, p16 INK4A, PTEN, pRb (retinoblastoma protein), EGFR, p-Akt, c-Kit, c-myc, AP-2, HDM2, bcl-6, Ki67 (detected by Mib1), Cyclin A, B, D, E, p21CIP1, Geminin, PCNA (proliferating cell nuclear antigen), bcl-2, bax, bak, APAF-1, LYVE-1 (lymphatic vascular endothelial hyaluronan receptor-1), PTN, P-Cadherin, E-Cadherin, Beta-catenin, Integrins beta1 and beta3, MMPs (matrix metalloproteinases), Dysadherin, CEACAM1 (carcinoembryonic-antigen-related cell-adhesion molecule 1), Osteonectin, TA, Melastatin, ALCAM/CD166 (Activated leukocyte cell adhesion molecule), CXCR4, Metallothionein.

According to further embodiments n markers according to at least some embodiments of the present invention might optionally be used alone or in combination with one or more other compounds described herein, and/or in combination with known markers for prostate cancer, including but not limited to PSA, PAP (prostatic acid phosphatase), CPK-BB, PSMA, PCA3, DD3, and/or in combination with the known protein(s) for the variant marker as described herein.

According to further embodiments markers according to at least some embodiments of the present invention might optionally be used alone or in combination with one or more other compounds described herein, and/or in combination with known markers for pancreatic cancer, including but not limited to CA 19-9, and/or in combination with the known protein(s) for the variant marker as described herein.

According to further embodiments markers according to at least some embodiments of the present invention might optionally be used alone or in combination with one or more other compounds described herein, and/or in combination with known markers for hematological cancer, including but not limited to soluble forms of tumor markers like P-Selectin, CD-22, interleukins, cytokines, and/or in combination with the known protein(s) for the variant marker as described herein.

According to further embodiments markers according to at least some embodiments of the present invention might optionally be used alone or in combination with one or more other compounds described herein, and/or in combination with known markers for colon cancer, including but not limited to CEA, CA19-9, CA50, and/or in combination with the known proteins for the variant marker as described herein. The diagnostic assay is performed in a subject or in a sample obtained from a subject.

According to some embodiments, the sample taken from a subject to perform a diagnostic assay according to at least some embodiments of the present invention is selected from the group consisting of a body fluid or secretion including but not limited to blood, serum, urine, plasma, prostate fluid, seminal fluid, semen, the external secretions of the skin, respiratory, intestinal, and genitourinary tracts, tears, cerebrospinal fluid, sputum, saliva, milk, peritoneal fluid, pleural fluid, cyst fluid, secretions of the breast ductal system (and/or lavage thereof), broncho alveolar lavage, lavage of the reproductive system and lavage of any other part of the body or system in the body; samples of any organ including isolated cells or tissues, wherein the cell or tissue can be obtained from an organ selected from, but not limited to lung, colon, kidney, pancreas, ovary, prostate, liver, skin, bone marrow, lymph node, breast, and/or blood tissue; stool or a tissue sample, or any combination thereof. Prior to be subjected to the diagnostic assay, the sample can optionally be diluted with a suitable diluent. In certain embodiments, cells obtained from the sample are cultured in vitro prior to performing the diagnostic assay.

Numerous well known tissue or fluid collection methods can be utilized to collect the biological sample from a subject in order to determine the level of nucleic acid and/or polypeptide of the marker of interest in the subject.

Examples include, but are not limited to, fine needle biopsy, needle biopsy, core needle biopsy and surgical biopsy (e.g., brain biopsy), and lavage. Regardless of the procedure employed, once a biopsy/sample is obtained the level of the marker can be determined and a diagnosis can thus be made.

In at least some embodiments the present invention provides variant proteins, which may optionally be used as diagnostic markers, optionally as markers for in vivo imaging. According to at least some embodiments the present invention therefore overcomes the many deficiencies of the background art with regard to the need to obtain tissue samples and subjective interpretations of results. As in vivo imaging markers, the markers according to at least some embodiments of the present invention may also provide different and/or better measurement parameters for various diseases and/or pathological conditions. Molecular imaging using these markers could be performed in conjunction with other imaging modalities as CT and MRI which capture body anatomy and overlap it with the in-vivo marker distribution.

In at least some embodiments the present invention further relates to diagnostic assays for detecting a disease, particularly in a sample taken from a subject (patient), optionally a blood sample or a body secretion sample. In at least some embodiments of the present invention, the diagnostic assays are immunoassays, including, for example, immunohistochemical assay, radioimaging assays, in-vivo imaging, positron emission tomography (PET), single photon emission computer tomography (SPECT), magnetic resonance imaging (MRI), Ultra Sound, Optical Imaging, Computer Tomography, radioimmunoassay (RIA), ELISA, slot blot, competitive binding assays, fluorimetric imaging assays, Western blot, FACS, and the like. According to another embodiments, the diagnostic assays are NAT (nucleic acid amplification technology)-based assays, including, for example, nucleic acid hybridization assays, PCR or variations thereof, e.g. real-time PCR. The diagnostic assays can be qualitative or quantitative.

In some embodiments, the phrase "differentially present" refers to differences in the quantity of a marker present in a sample taken from subjects having one of the herein-described diseases or conditions as compared to a comparable sample taken from subjects who do not have one of the herein-described diseases or conditions. For example, a nucleic acid fragment may optionally be differentially present between the two samples if the amount of the nucleic acid fragment in one sample is significantly different from the amount of the nucleic acid fragment in the other sample, for example as measured by hybridization and/or NAT-based assays. A polypeptide is differentially present between the two samples if the amount of the polypeptide in one sample is significantly different from the amount of the polypeptide in the other sample. It should be noted that if the marker is detectable in one sample and not detectable in the other, then such a marker can be considered to be differentially present. Optionally, a relatively low amount of up-regulation may serve as the marker, as described herein. One of ordinary skill in the art could easily determine such relative levels of the markers; further guidance is provided in the description of each individual marker below.

The term "marker" in the context of the present invention refers to a nucleic acid fragment, a peptide, or a polypeptide, which is differentially present in a sample taken from subjects having one of the herein-described diseases or conditions, as compared to a comparable sample taken from subjects who do not have one the above-described diseases or conditions.

According to at least some embodiments of the present invention, a diagnostic assay can provide qualitative or quantitative information on the level of the markers in the sample.

In some embodiments, the phrase "qualitative" when in reference to differences in expression levels of a polynucleotide or polypeptide as described herein, refers to the presence versus absence of expression, or in some embodiments, the temporal regulation of expression, or in some embodiments, the timing of expression, or in some embodiments, any post-translational modifications to the expressed molecule, and others, as will be appreciated by one skilled in the art. In some embodiments, the phrase "quantitative" when in reference to differences in expression levels of a polynucleotide or polypeptide as described herein, refers to absolute differences in quantity of expression, as determined by any means, known in the art, or in other embodiments, relative differences, which may be statistically significant, or in some embodiments, when viewed as a whole or over a prolonged period of time, etc., indicate a trend in terms of differences in expression.

The term "level" refers to expression levels of nucleic acids (e.g. RNA) and/or polypeptides of the marker according to at least some embodiments of the present invention.

In certain embodiments, the diagnostic markers according to at least some embodiments of the invention are correlated to a condition or disease by their mere presence or absence. In other embodiments, threshold levels of the diagnostic markers can be established, and the level of the markers in a patient's sample can be compared to the threshold levels.

In some embodiments, the term "test amount" of a marker refers to an amount of a marker in a subject's sample that is consistent with a diagnosis of a particular disease or condition. A test amount can be either in absolute amount (e.g., microgram/ml) or a relative amount (e.g., relative intensity of signals).

In some embodiments, the term "control amount" of a marker can be any amount or a range of amounts to be compared against a test amount of a marker. For example, a control amount of a marker can be the amount of a marker in a patient with a particular disease or condition or a person without such a disease or condition. A control amount can be either in absolute amount (e.g., microgram/ml) or a relative amount (e.g., relative intensity of signals).

In some embodiments, the term "detect" refers to identifying the presence, absence or amount of the object to be detected.

In some embodiments, the term "label" includes any moiety or item detectable by spectroscopic, photo chemical, biochemical, immunochemical, or chemical means. For example, useful labels include 32P, 35S, fluorescent dyes, electron-dense reagents, enzymes (e.g., as commonly used in an ELISA), biotin-streptavadin, dioxigenin, haptens and proteins for which antisera or monoclonal antibodies are available, or nucleic acid molecules with a sequence complementary to a target. The label often generates a measurable signal, such as a radioactive, chromogenic, or fluorescent signal, that can be used to quantify the amount of bound label in a sample. The label can be incorporated in or attached to a primer or probe either covalently, or through ionic, van der Waals or hydrogen bonds, e.g., incorporation of radioactive nucleotides, or biotinylated nucleotides that are recognized by streptavadin. The label may be directly or indirectly detectable. Indirect detection can involve the binding of a second label to the first label, directly or indirectly. For example, the label can be the ligand of a binding partner, such as biotin, which is a binding partner for streptavadin, or a nucleotide sequence, which is the binding partner for a complementary sequence, to which it can specifically hybridize. The binding partner may itself be directly detectable, for example, an antibody may be itself labeled with a fluorescent molecule. The binding partner also may be indirectly detectable, for example, a nucleic acid having a complementary nucleotide sequence can be a part of a branched DNA molecule that is in turn detectable through hybridization with other labeled nucleic acid molecules (see, e.g., P. D. Fahrlander and A. Klausner, Bio/Technology 6:1165 (1988)). Quantitation of the signal is achieved by, e.g., scintillation counting, densitometry, or flow cytometry.

Exemplary detectable labels, optionally for use with immunoassays, include but are not limited to magnetic beads, fluorescent dyes, radiolabels, enzymes (e.g., horse radish peroxide, alkaline phosphatase and others commonly used in an ELISA), and calorimetric labels such as colloidal gold or colored glass or plastic beads. Alternatively, the marker in the sample can be detected using an indirect assay, wherein, for example, a second, labeled antibody is used to detect bound marker-specific antibody, and/or in a competition or inhibition assay wherein, for example, a monoclonal antibody which binds to a distinct epitope of the marker are incubated simultaneously with the mixture.

The phrase "specifically (or selectively) binds" to an antibody or "specifically (or selectively) immunoreactive with," or "specifically interacts or binds" when referring to a protein or peptide (or other epitope), refers, in some embodiments, to a binding reaction that is determinative of the presence of the protein in a heterogeneous population of proteins and other biologies. Thus, under designated immunoassay conditions, the specified antibodies bind to a particular protein at least two times greater than the background (non-specific signal) and do not substantially bind in a significant amount to other proteins present in the sample. Specific binding to an antibody under such conditions may require an antibody that is selected for its specificity for a particular protein. For example, polyclonal antibodies raised to seminal basic protein from specific species such as rat, mouse, or human can be selected to obtain only those polyclonal antibodies that are specifically immunoreactive with seminal basic protein and not with other proteins, except for polymorphic variants and alleles of seminal basic protein. This selection may be achieved by subtracting out antibodies that cross-react with seminal basic protein molecules from other species. A variety of immunoassay formats may be used to select antibodies specifically immunoreactive with a particular protein. For example, solid-phase ELISA immunoassays are routinely used to select antibodies specifically immunoreactive with a protein (see, e.g., Harlow & Lane, Antibodies, A Laboratory Manual (1988), for a description of immunoassay formats and conditions that can be used to determine specific immunoreactivity). Typically a specific or selective reaction will be at least twice background signal or noise and more typically more than 10 to 100 times background.Diagnostic assays according to at least some embodiments of the present invention include, but are not limited to immunoassays and nucleic acid based assays. "Immunoassay" is an assay that uses an antibody to specifically bind an antigen. The immunoassay is characterized by the use of specific binding properties of a particular antibody to isolate, target, and/or quantify the antigen.

According to at least some embodiments, the present invention provides a method for detecting the polypeptides according to at least some embodiments of the invention in a biological sample, comprising: contacting a biological sample with an antibody specifically recognizing a polypeptide according to at least some embodiments of the present invention and detecting said interaction; wherein the presence of an interaction correlates with the presence of a polypeptide in the biological sample.

According to at least some embodiments, the present invention provides a method for detecting a polynucleotide according to at least some embodiments of the invention in a biological sample, using NAT based assays, comprising: hybridizing the isolated nucleic acid molecules or oligonucleotide fragments of at least about a minimum length to a nucleic acid material of a biological sample and detecting a hybridization complex; wherein the presence of a hybridization complex correlates with the presence of the polynucleotide in the biological sample.

Non-limiting examples of methods or assays are described below.

The present invention also relates to kits based upon such diagnostic methods or assays.

### IMMUNOASSAYS

Immunological binding assays include, for example, an enzyme immune assay (EIA) such as enzyme-linked immunosorbent assay (ELISA), a radioimmune assay (RIA), a Western blot assay, or a slot blot assay (see, e.g., U.S. Pat. No 4,366,241; 4,376,110; 4,517,288; and 4,837,168). Generally, a subject or a sample obtained from a subject is contacted with an antibody that specifically binds a polypeptide according to at least some embodiments of the invention, or a fragment thereof. Optionally, the antibody can be fixed to a solid support prior to contacting the antibody with a sample. Examples of solid supports include but are not limited to glass or plastic in the form of, e.g., a microtiter plate, a stick, a bead, or a microbead. After incubating the sample with antibodies, the mixture is washed and the antibody-marker complex formed can be detected. This can be accomplished by incubating the washed mixture with a detection reagent. Alternatively, the marker in the sample can be detected using an indirect assay, wherein, for example, a second, labeled antibody is used to detect bound marker-specific antibody. Throughout the assays, incubation and/or washing steps may be required after each combination of reagents. Incubation steps can vary from about 5 seconds to several hours, preferably from about 5 minutes to about 24 hours. However, the incubation time will depend upon the assay format, marker, volume, concentrations and the like. Usually the assays will be carried out at ambient temperature, although they can be conducted over a range of temperatures, such as 10 °C to 40 °C.

The amount of an antibody-marker complex can optionally be determined by comparing to a standard or to a control amount and/or signal.

Radio-immunoassay (RIA): According to one embodiment, this method involves contacting the biological sample with a specific antibody followed by a radiolabeled secondary antibody or antibody binding protein (e.g., protein A labeled with I125) immobilized on a precipitable carrier such as agarose beads. The number of counts in the precipitated pellet is proportional to the amount of the marker polypeptide in the sample.

Enzyme linked immunosorbent assay (ELISA): This method involves fixation of a sample containing the target polypeptide to a surface such as a well of a microtiter plate. A substrate specific antibody coupled to an enzyme is applied and allowed to bind to the target polypeptide. Presence of the antibody is then detected and quantitated by a colorimetric reaction employing the enzyme coupled to the antibody. Enzymes commonly employed in this method include horseradish peroxidase and alkaline phosphatase. The amount of substrate present in the sample is proportional to the amount of color produced. A substrate standard is generally employed to improve quantitative accuracy.

Western blot: This method involves separation of a solution containing the target polypeptide by means of an acrylamide gel followed by transfer of the polypeptides to a membrane (e.g., nylon or PVDF). Presence of the target polypeptide is then detected by specific antibodies, which are in turn detected by antibody binding reagents. Antibody binding reagents may be, for example, protein A, or secondary antibodies. Antibody binding reagents may be radiolabeled or enzyme linked as described hereinabove. Detection may be by autoradiography, colorimetric reaction or chemiluminescence. This method allows both quantitative analysis of the amount of target polypeptide and determination of its identity by a relative position on the membrane which is indicative of a migration distance in the acrylamide gel during electrophoresis.

Immunohistochemical analysis: This method involves detection of a substrate in situ in fixed cells by specific antibodies. The antibodies may be enzyme linked or linked to fluorophores. Detection is by microscopy and subjective evaluation. If enzyme linked antibodies are employed, a colorimetric reaction may be required.

Fluorescence activated cell sorting (FACS): This method involves detection of a target polypeptide in situ in cells by specific antibodies. The antibodies are linked to fluorophores. Detection is by means of a cell sorting machine which reads the wavelength of light emitted from each cell as it passes through a light beam. This method may employ two or more antibodies simultaneously.

### NUCLEIC ACID TECHNOLOGY (NAT) BASED ASSAYS:

According to at least some embodiments the invention also contemplates nucleic acids which selectively hybridize with the polynucleotides according to at least some embodiments of the invention. The following are non-limiting examples of Nucleic Acid Technology-based assays: polymerase chain reaction (PCR), Real-Time PCR, ligase chain reaction (LCR), Self-Sustained Synthetic Reaction, Q-Beta Replicase, Cycling probe reaction, Branched DNA, RFLP analysis, DGGE/TGGE, Single-Strand Conformation Polymorphism, Dideoxy fingerprinting, microarrays, Fluorescense In Situ Hybridization and Comparative Genomic Hybridization. Detection of a nucleic acid of interest in a biological sample may be effected by assays which involve nucleic acid amplification technology. Amplification of a target nucleic acid sequence may be carried out by a number of suitable methods known in the art. Non-limiting examples of amplification techniques include primer based-PCR, LCR, strand displacement amplification (SDA), transcription-based amplification, the q3 replicase system and NASBA (Kwoh et al., 1989, Proc. NatI. Acad. Sci. USA 86, 1173-1177; Lizardi et al., 1988, BioTechnology 6:1197-1202; Malek et al., 1994, Methods Mol. Biol., 28:253-260; and Sambrook et al., 1989, supra). As used herein, a "primer" refers to an oligonucleotide which is capable of annealing to (hybridizing with) a target sequence, thereby creating a double stranded region which can serve as an initiation point for DNA synthesis under suitable conditions. The terminology "amplification pair" (or "primer pair") refers herein to a pair of oligonucleotides (oligos), which are selected to be used together in amplifying a selected nucleic acid sequence by one of a number of types of amplification processes, preferably a polymerase chain reaction.

Oligonucleotide primers according to at least some embodiments of the present invention may be of any suitable length, depending on the particular assay format and the particular needs and targeted genomes employed. Optionally, the oligonucleotide primers are at least 12 nucleotides in length, preferably between 15 and 24 nucleotides, and they may be adapted to be especially suited to a chosen nucleic acid amplification system. As commonly known in the art, the oligonucleotide primers can be designed by taking into consideration the melting point of hybridization thereof with its targeted sequence (Sambrook et al., 1989, Molecular Cloning -A Laboratory Manual, 2nd Edition, CSH Laboratories; Ausubel et al., 1989, in Current Protocols in Molecular Biology, John Wiley & Sons Inc., N.Y.).

### RADIO-IMAGING METHODS

These methods include but are not limited to, positron emission tomography (PET) and single photon emission computed tomography (SPECT). Both of these techniques are non-invasive, and can be used to detect and/or measure a wide variety of tissue events and/or functions, such as detecting cancerous cells for example. Unlike PET, SPECT can optionally be used with two labels simultaneously. SPECT has some other advantages as well, for example with regard to cost and the types of labels that can be used. For example, US Patent No. 6,696,686 describes the use of SPECT for detection of breast cancer.

According to at least some embodiments the present invention also relates to kits based upon such diagnostic methods or assays.

### THERANOSTICS:

According to at least some embodiments the present invention also relates to the ude of markers and antibodies according to at least some embodiments of the invention for theranostics. The term theranostics describes the use of diagnostic testing to diagnose the disease, choose the correct treatment regime according to the results of diagnostic testing and/or monitor the patient response to therapy according to the results of diagnostic testing. Theranostic tests optionally may be used to select patients for treatments that are particularly likely to benefit them and unlikely to produce side-effects. They can also provide an early and objective indication of treatment efficacy in individual patients, so that (if necessary) the treatment can be altered with a minimum of delay. For example: DAKO and Genentech together created HercepTest and Herceptin (trastuzumab) for the treatment of breast cancer, the first theranostic test approved simultaneously with a new therapeutic drug. In addition to HercepTest (which is an immunohistochemical test), other theranostic tests are in development which use traditional clinical chemistry, immunoassay, cell-based technologies and nucleic acid tests. PPGx's recently launched TPMT (thiopurine S-methyltransferase) test, which is enabling doctors to identify patients at risk for potentially fatal adverse reactions to 6-mercaptopurine, an agent used in the treatment of leukemia. Also, Nova Molecular pioneered SNP genotyping of the apolipoprotein E gene to predict Alzheimer's disease patients' responses to cholinomimetic therapies and it is now widely used in clinical trials of new drugs for this indication. Thus, the field of theranostics represents the intersection of diagnostic testing information that predicts the response of a patient to a treatment with the selection of the appropriate treatment for that particular patient.

### SURROGATE MARKERS:

According to at least some embodiments the present invention also relates to the ude of markers and antibodies according to at least some embodiments of the invention as Surrogate markers. A surrogate marker is a marker, that is detectable in a laboratory and/or according to a physical sign or symptom on the patient, and that is used in therapeutic trials as a substitute for a clinically meaningful endpoint. The surrogate marker is a direct measure of how a patient feels, functions, or survives which is expected to predict the effect of the therapy. The need for surrogate markers mainly arises when such markers can be measured earlier, more conveniently, or more frequently than the endpoints of interest in terms of the effect of a treatment on a patient, which are referred to as the clinical endpoints. Ideally, a surrogate marker will be biologically plausible, predictive of disease progression and measurable by standardized assays (including but not limited to traditional clinical chemistry, immunoassay, cell-based technologies, nucleic acid tests and imaging modalities).

Surrogate endpoints were used first mainly in the cardiovascular area. For example, antihypertensive drugs have been approved based on their effectiveness in lowering blood pressure. Similarly, in the past, cholesterol-lowering agents have been approved based on their ability to decrease serum cholesterol, not on the direct evidence that they decrease mortality from atherosclerotic heart disease. The measurement of cholesterol levels is now an accepted surrogate marker of atherosclerosis. In addition, currently two commonly used surrogate markers in HIV studies are CD4+ T cell counts and quantitative plasma HIV RNA (viral load). In some embodiments of this invention, the polypeptide/polynucleotide expression pattern may serve as a surrogate marker for a particular disease, as will be appreciated by one skilled in the art.

### SMALL INTERFERING NUCLEIC ACIDS AND ANTISENSE MOLECULES

According to at least some embodiments the present invention further relates to small interfering nucleic acids, in particular siNA comprising complementary sequences capable of specifically hybridizing with the polynucleotides according to at least some embodiments of the invention (i.e. with portions of F04175 T5 and F04175 T15) and specifically silencing these genes. According to at least some embodiments the present invention also relates to sequences and constructs encoding such nucleic acids and to the uses of such nucleic acids or constructs to modify F04175 T5 or F04175 T15 gene expression, particularly to reduce or inhibit gene expression.

Certain single stranded nucleic acid molecules are able to form a self- complementary double stranded region where part of the nucleotide sequence is able to interact with another part of the sequence by Watson-Crick base pairing between inverted repeats of the sequence. Where the repeated regions are adjacent or in close proximity to each other, the double stranded regions may form structures known as hairpin structures. The hairpin structure forms with an unpaired "loop" of nucleotides at one end of the hairpin structure, with the inverted repeat sequence annealed. The loop may also facilitate the folding of the nucleic acid chain.

Hairpin RNA sequences have been used in interfering RNA and gene silencing technologies. Such techniques are described for example in US patent no. 6,573,099 and in Grimm D. (Adv. Drug Deliv. Rev. 2009 61 (9): 672-703). According to at least some embodiments the present invention further contemplates antisense RNA molecules complementary to the polynucleotides according to at least some embodiments of the invention, or to any fragment thereof. Antisense RNA may be introduced into a cell to inhibit translation of the complementary mRNA by hybridizing with the polynucleotides of the according to at least some embodiments of the invention and obstructing the translation machinery.

siNA or antisense molecules according to at least some embodiments of the invention may be used as a therapeutic tool to inhibit F04175 T5 and F04175 T15 gene expression in vivo.

The following examples are offered for illustrative purposes only, and are not intended to limit the scope of the present invention in any way.

All patent and literature references cited in the present specification are hereby incorporated by reference in their entirety.

### EXAMPLES

### EXAMPLE 1:

### METHODS USED TO ANALYZE THE EXPRESSION OF THE RNA

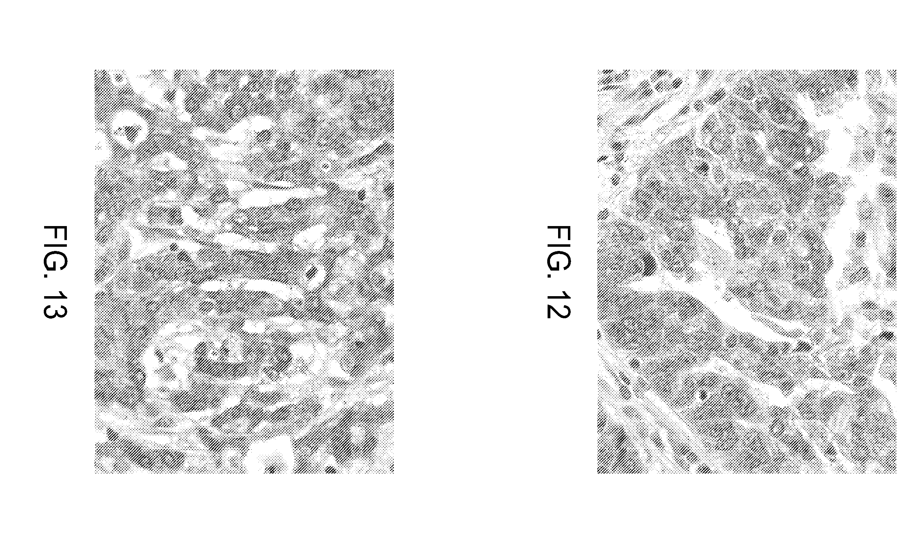

The targets according to at least some embodiments of the present invention were tested with regard to their expression in various cancerous and non-cancerous tissue samples and/or with regard to its expression in a wide panel of human samples which contains various types of immune cells, and hematological malignancies samples and cell lines, as well as several samples of normal tissues. A description of the samples used in the normal and cancerous tissue panels used in Example 3_2 (presented in Figure 13) is provided in Table 1. The list of the blood specific RNA samples used for the qRT-PCR analysis is provided in Table 2 below. A description of the Multiple Myeloma samples from the blood panel described in Table 2, is provided in Table 2_1. A description of the samples used in the normal tissue panels are provided in Table 3. A description of the samples used in the ovary cancer testing panel is provided in Table 4 below. The key for the table 4 is given in table 4_1. Tests were then performed as described in the "Materials and Experimental Procedures" section below.

**Table 1**

| Well | Sample Name | Ct | Quantity | Well | Sample Name | Ct | Quantity |
|---|---|---|---|---|---|---|---|
| 1 | A1 | 31.988285 | 100 | 91 | N.Testis (#276) | 32.4772 | 48.8715 |
| 2 | A2 | 28.521492 | 1000 | 94 | N.Trach ea (#314) | 31.908 | 71.3431 |
| 3 | A3 | 24.747038 | 10000 | 106 | Head/Ne ckT. (Larynx, #402) | 31.7226 | 80.7009 |
| 4 | A4 | 21.234554 | 100000 | 107 | Head/Ne ckT. (Pharyn x, #403) | 30.1814 | 224.8 |
| 5 | A5 | 17.710257 | 1000000 | 108 | Head/Ne ckT. (Tongue , #404) | 31.1225 | 120.26 |
| 6 | A6 | 31.214949 | 100 | 109 | Head/Ne ckT. (Tonsil, #405) | 33.464 | 25.3618 |
| 7 | A7 | 27.833765 | 1000 | 110 | Kidney T. (#167) | 28.9694 | 503.108 |
| 8 | A8 | 24.486034 | 10000 | 111 | Kidney T. (#168) | 30.175 | 225.761 |
| 9 | A9 | 20.853727 | 100000 | 112 | Kidney T. (#169) | 34.01 | 17.6431 |
| 10 | A10 | 17.610092 | 1000000 | 113 | Kidney T. (#170) | 30.4578 | 187.069 |
| 11 | A11 | 30.853067 | 100 | 114 | Kidney T. (#171) | 30.5402 | 177.099 |
| 12 | A12 | 27.679554 | 1000 | 115 | Liver T. (#326) | 31.9156 | 70.9841 |
| 13 | A13 | 24.258177 | 10000 | 116 | Liver T. (#327) | 31.0544 | 125.828 |
| 14 | A14 | 20.678856 | 100000 | 117 | Liver T. (#328) | 28.3689 | 749.942 |
| 15 | A15 | 17.408792 | 1000000 | 118 | Liver T. (#329) | 29.0953 | 462.736 |
| 17 | Water | Undetermi ned | 0 | 123 | Lung T. (NSC, #157) | 31.9388 | 69.8983 |
| 28 | N. Bone Marrow Stromal Cells (#394) | 37.212994 | 2.098594 | 124 | Lung T. (NSC, #158) | 33.0217 | 34.0301 |
| 30 | N.Brain Cerebellum (#123) | 34.93981 | 9.509247 | 125 | Lung T. (NSC, #159) | 29.9511 | 261.985 |
| 36 | N.Brain Thalamus (#131) | 34.98086 | 9.253285 | 126 | Lung T. (NSC, #160) | 31.4862 | 94.4371 |
| 37 | N.Breast (#259) | 31.53689 | 91.30512 | 134 | Lympho ma (#287) | 28.2315 | 821.668 |
| 39 | N.Cervix (#260) | 33.32578 | 27.80238 | 135 | Lympho ma (#288) | 33.1223 | 31.8298 |
| 40 | N.Colon (#261) | 31.046032 | 126.5304 | 136 | Lymphoma (#290) | 27.9259 | 1006.72 |
| 45 | N.Esophagus (#307) | 34.37151 | 13.87403 | 137 | Lymphoma (#289) | 29.1648 | 441.834 |
| 46 | N.Heart (#118) | 34.899815 | 9.765454 | 138 | Lymphoma (#291) | 29.3026 | 403.177 |
| 53 | N.Kidney (#264) | 32.810036 | 39.17111 | 139 | Lymphoma (#292) | 33.3113 | 28.0704 |
| 54 | N.Kidney (#265) | 31.380625 | 101.2991 | 140 | Lymphoma (#293) | 28.0062 | 954.41 |
| 55 | N.Kidney (#311) | 35.80812 | 5.3393 | 141 | Lymphoma (#294) | 28.9552 | 507.88 |
| 56 | N.Liver (#266) | 36.25151 | 3.976376 | 142 | Lymphoma (#295) | 27.6835 | 1182.74 |
| 57 | N.Liver (#267) | 34.554146 | 12.28795 | 143 | Lymphoma (#296) | 30.0861 | 239.499 |
| 67 | N.Lung (#268) | 32.817394 | 38.97998 | 144 | Lymphoma (#297) | 30.4276 | 190.865 |
| 68 | N.Lung (#313) | 32.38373 | 52.00325 | 145 | Lymphoma (#298) | 30.8015 | 148.863 |
| 70 | N.Lymph Node (#269) | 30.1909 | 223.3843 | 146 | Lymphoma (#299) | 30.1717 | 226.256 |
| 71 | N.Lymph Node (#315) | 28.42075 | 724.5305 | 147 | Lymphoma (#300) | 30.2177 | 219.437 |
| 73 | N.Ovary (#270) | 33.379852 | 26.82083 | 148 | Melanoma (#162) | 32.4551 | 49.5932 |
| 74 | N.Pancreas (#271) | 35.97763 | 4.770353 | 149 | Melanoma (#163) | 28.9535 | 508.481 |
| 76 | N.Peripheral Blood Leukocytes (#302) | 29.496643 | 354.3804 | 150 | Melanoma (#166) | 37.4152 | 1.83466 |
| 77 | N.Prostate (#272) | 36.119064 | 4.34232 | 151 | Melanoma (#165) | 27.2872 | 1539.16 |
| 81 | N.Skin (#273) | 38.019768 | 1.227525 | 152 | Melanoma (#164) | 33.0435 | 33.5416 |
| 82 | N.Skin (#319) | 33.879433 | 19.24218 | 162 | Pancreas T. (#186) | 30.7932 | 149.691 |
| 83 | N.Smal 1 Intestine (#320) | 31.034052 | 127.5421 | 163 | Prostate T. (#378) | 33.5837 | 23.422 |
| 84 | N.Smal 1 Intestine: Jejunum (#321) | 32.072933 | 63.93684 | 164 | Prostate T. (#379) | 33.1311 | 31.6443 |
| 87 | N.Spleen (#274) | 33.140545 | 31.44526 | 165 | Prostate T. (#380) | 31.9457 | 69.5817 |
| 88 | N.Spleen (#322) | 26.349798 | 2870.087 | 166 | Prostate T. (#381) | 32.1304 | 61.5407 |
| 89 | N.Stomach (#275) | Undetermined | 0 | 167 | Prostate T. (#382) | 31.5726 | 89.1632 |
| 90 | N.Stomach (#323) | 31.639095 | 85.30818 | 168 | Prostate T. (#383) | 34.3888 | 13.7151 |

**Table 2**

| Blood panel sample | Description | Organ/ Cell type | Tumor Type |
|---|---|---|---|
| 1_PBMC2 | PBMCs | blood-derived cells | |
| 2_PBMC3 | PBMCs | blood-derived cells | |
| 3_Bcell1 | B cells | blood-derived cells | |
| 4_Bcell2 | B cells | blood-derived cells | |
| 5_J_Bcell | B cells | blood-derived cells | |
| 6_K_Bcells_act | B cells activated | blood-derived cells | |
| 7_Tcelll | T cells | blood-derived cells | |
| 8_Tcell2 | T cells | blood-derived cells | |
| 9_M_CD8 | CD4+ T cells | blood-derived cells | |
| 10_G_CD4_unt | CD8+ T cells | blood-derived cells | |
| 11_H_CD4_Beads | CD4+ w Activation beads | blood-derived cells | |
| 12_I_CD4_Beads_IL12 | CD4 w act. Beads+IL12 | blood-derived cells | |
| 13_95_CD4+CD25- | CD4+CD25- | blood-derived cells | |
| 15_NK | NK cells | blood-derived cells | |
| 16_CD34+_1548 | CD34+(PCBM1548) | blood-derived cells | |
| 17_CD34+_1028 | CD34+(PCBM1028) | blood-derived cells | |
| 18_PMN | PMNs | blood-derived cells | |
| 19_A_Mono | Monocytes | blood-derived cells | |
| 20_B_Macro_imma | Macrophages immature | blood-derived cells | |
| 21_C_Macro_mat | Macrophages mature | blood-derived cells | |
| 22_D_DCs_immat | DCs immature | blood-derived cells | |
| 23_E_DCs_mat_LPS | DCs mature LPS | blood-derived cells | |
| 24_F_DCs_mat_CK | DCs mature CK | blood-derived cells | |
| 25_L_DCs+T | DCs +T cells | blood-derived cells | |
| 26_Lym1 | 13987A1 | Lymph Node | Lymphoma |
| 27_Lym2 | 43594B1 | Muscle | lymphoma |
| 28_Lym3 | 65493A1 | Testis | Lymphoma |
| 29_Ma1Lym3 | 75894A1 | Brain | Lymphoma |
| 30_NonHod_SCLym | 83325A1 | Lymph Node | NHL Small Cell |
| 31_NonHod_FolLym | 76943A1(5 tubes) | Lymph Node | NHL Follicular |
| 32_Lym_Fol_GI | CN_4_ASRBNA35 | | NHL Follicular Grade I (Small Cell) |
| 33_Lym_Fol_GII | CN_1_113GHA8J | | NHL Follicular Grade II (mixed Small & Large Cell) |
| 34_Lym_Fol_GIII | CN_8_VXML6AXI | | NHL Follicular Grade III (Large Cell) |
| 35_MalLym1 | 76218B1 | Testis | NHL Large Cell |
| 36_MalLym2 | 76102A1 | Lymph Node | NHL Large Cell |
| 37_Lym_DifBCell1 | CN_2_4HDLNA2R | | NHL Diffuse Large B-Cell |
| 38_Lym_DifBCell2 | CN_3_4M4S7AAM | | NHL Diffuse Large B-Cell |
| 39_Lym_DifBCell3 | CN_5_HEODOAR2 | | NHL Diffuse Large B-Cell |
| 40_NonHod_Lym1 | 77332A1(5 tubes) | Colon | NHL Diffuse Large B-Cell |
| 41_MalLym4 | 76161A1 | Spleen | NHL Diffuse Large B-Cell |
| 42_Lym_MantleCell1 | CN_6_MAE47AOY | | NHL Mantle Cell |
| 43_Lym_MantleCell2 | CN_7_VJU9OAO9 | | NHL Mantle Cell |
| 44_NonHod_Lym2 | 95377A1(5 tubes) | Spleen | NHL |
| 45_THP_1 | THP-1 | monocytes | AML cell line |
| 77_MM_Patient1 | Multiple Myeloma Patient | See Table 2_1 | |
| 78-MM_Patient2 | Multiple Myeloma Patient | See Table 2_1 | |
| 79-MM_Patient3 | Multiple Myeloma Patient | See Table 2_1 | |
| 80-MM_Patient4 | Multiple Myeloma Patient | See Table 2_1 | |
| 81-MM_Patient5 | Multiple Myeloma Patient | See Table 2_1 | |
| 82-MM_Patient6 | Multiple Myeloma Patient | See Table 2_1 | |
| 83-MM_Patient7 | Multiple Myeloma Patient | See Table 2_1 | |
| 84-MM_Tumor1 | Multiple Myeloma Tumor | See Table 2_1 | |
| 85-MM_Tumor2 | Multiple Myeloma Tumor | See Table 2_1 | |
| 87-MM_Tumor4 | Multiple Myeloma Tumor | See Table 2_1 | |
| 59_NCI_H929 | NCI-H929 | B lymphoblasts | Multiple Myeloma cell line |
| 60_MC/CAR | MC/CAR | B lymphoblasts | Multiple Myeloma cell line |
| 61_U266 | U266 | B lymphoblasts | Multiple Myeloma cell line |
| 62_RPMI8226 | RPMI8226 | B lymphoblasts | Multiple Myeloma cell line |
| 63_IM_9 | IM-9 | B lymphoblasts | Multiple Myeloma cell line |
| 64_cereN | cerebellum normal | cerebellum normal | |
| 65_kidneyN1 | kidney normal | kidney normal | |
| 66_kidneyN2 | kidney normal | kidney normal | |
| 67_Kidney3 | kidney normal | kidney normal | |
| 68_colonN1 | colon normal | colon normal | |
| 69_colonN2 | colon normal | colon normal | |
| 70_stomN | stomach normal | stomach normal | |
| 71_liverN | liver normal | liver normal | |
| 72_lungN1 | lung normal | lung normal | |
| 73_lungN2 | lung normal | lung normal | |
| 74_small intestineN | small intestine | small intestine | |
| 75_brainN | brain normal mix | brain normal mix | |
| 76_heartN | heart normal mix | heart normal mix | |

**Table 2_1: Multiple Myeloma samples details**

| | patient ID | tumor ID | Diagnosis | Bone disease | Heavy chain isotype | Light chain isotype | ISS | Sex |
|---|---|---|---|---|---|---|---|---|
| 77-MM_Pat ient1 | 1289 | | Amyloidosis | UD | IgG | kappa | | |
| 78-MM_Pat ient2 | 1441 | | myeloma | No | IgG | - | satge 2 | |
| 79-MM_Pat ient3 | 1647 | | myeloma | UD | IgG | kappa | stage 3 | |
| 80-MM_Pat ient4 | 1434 | | myeloma | UD | IgG | kappa | satge 3 | |
| 81-MM_Pat ient5 | 1650 | | Undetermained (myeloma vs.MGUS) | UD | IgG | lambda | stage 2 | |
| 82-MM_Pat ient6 | 1661 | | myeloma | Yes | urine secretor | lambda | stage 1 | |
| 83-MM_Pat ient7 | 1058 | | myeloma | Yes | IgG | lambda | | |
| 84-MM_Tu mor1 | 1016 | LAP CLκ-1 | Plasma cell leukemia | UD | IgG | kappa | | - |
| 85-MM_Tu mor2 | 1065 | LAG κ-1A | myeloma | Yes | IgG | kappa | | F |
| 87-MM_Tu mor4 | 1178 | LAG κ-2 | myeloma | Yes | IgG | kappa | | M |

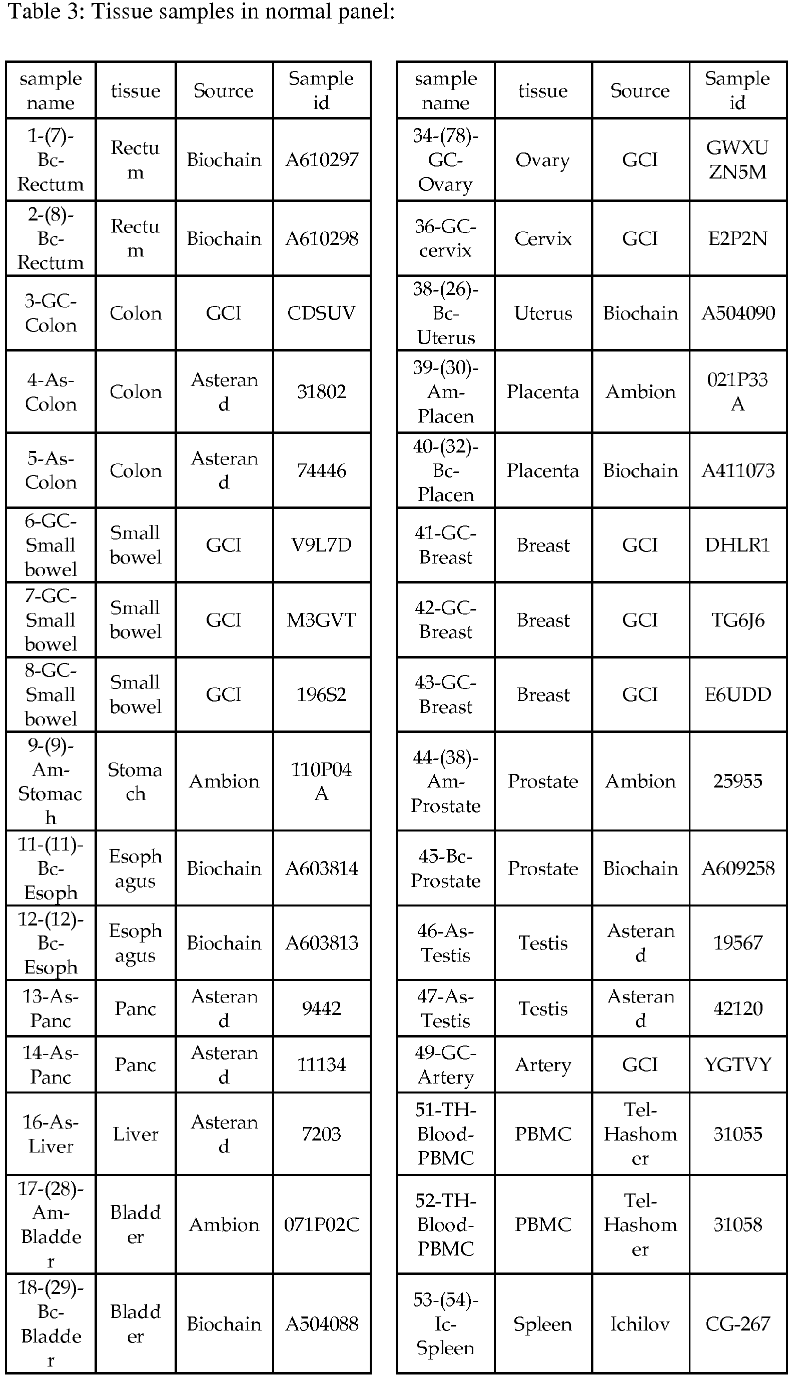

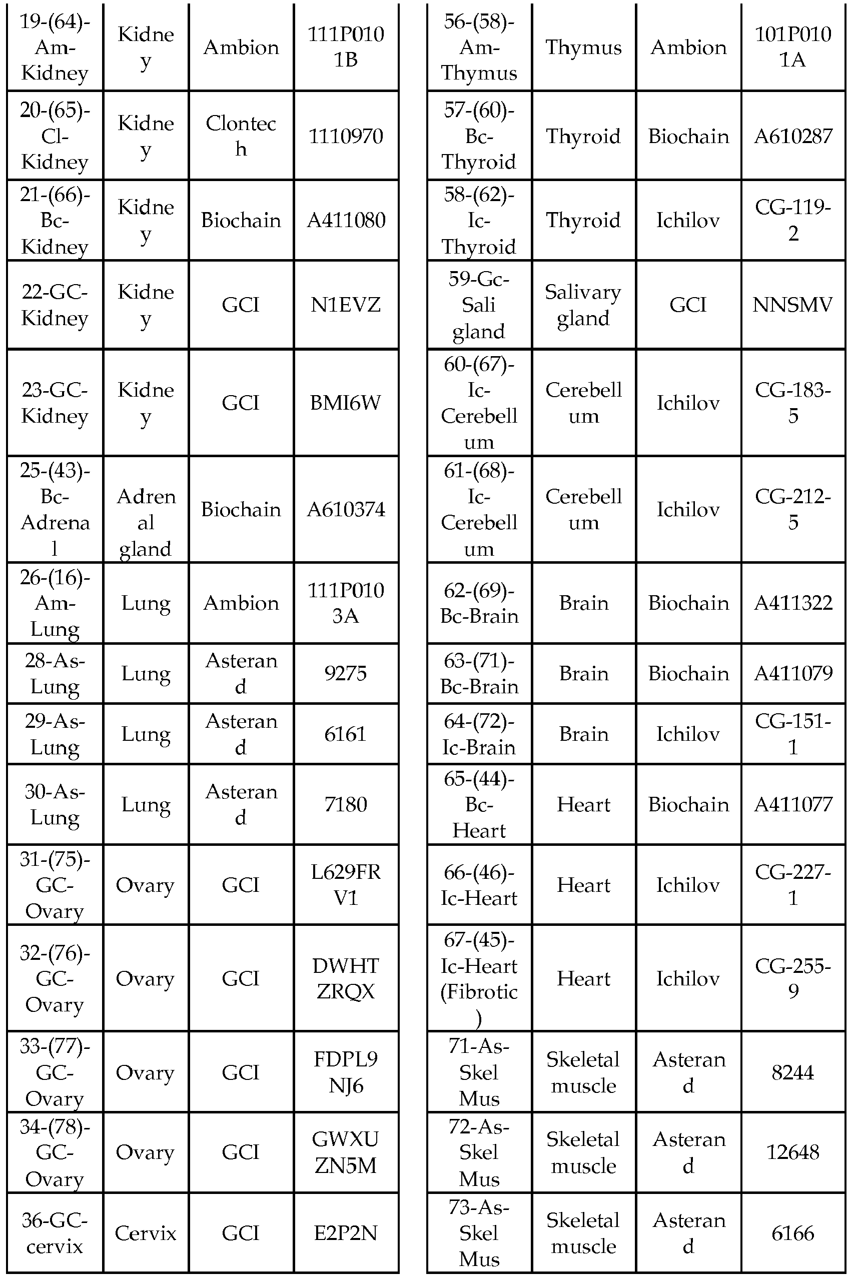

**Table 4: Tissue samples in ovary panel**

| Source | sample name | Ovary tissue type | Stage | Sample id |
|---|---|---|---|---|
| Asterand | 1-As-Ser SI | Serous carcinoma | I | 71900A2 |
| Asterand | 2-As-Ser SI | Serous carcinoma | I | 70270A1 |
| Asterand | 3-As-Ser SIB | Serous carcinoma | IB | 40771B1 |
| Asterand | 4-As-Ser SIB | Serous carcinoma | IB | 32667B1 |
| Asterand | 5-As-Ser SIC | Serous carcinoma | IC | 22996A1 |
| Asterand | 6-As-Ser SIIA | Serous carcinoma | IIA | 40773C1 |
| GCI | 7-GC-Ser SIIB | Serous carcinoma | IIB | 20370 |
| GCI | 8-GC-Ser SIIB | Serous carcinoma | IIB | 7B3DP |
| Asterand | 9-As-Ser SIIIC | Serous carcinoma | IIIC | 19832A1 |
| GCI | 10-GC-Ser SIIIC | Serous carcinoma | IIIC | 3NTIS |
| GCI | 11-GC-Ser SIIIC | Serous carcinoma | IIIC | CEJUS |
| GCI | 12-GC-Ser SIIIC | Serous carcinoma | IIIC | 5NCLK |
| GCI | 13-GC-Ser SIIIC | Serous carcinoma | IIIC | 1HI5H |
| GCI | 14-GC-Ser SIIIC | Serous carcinoma | IIIC | 7RMHZ |
| GCI | 15-GC-Ser SIIIC | Serous carcinoma | IIIC | 4WAAB |
| GCI | 16-GC-Ser SIIIC | Serous carcinoma | IIIC | 79Z67 |
| GCI | 17-GC-Ser SIIIC | Serous carcinoma | IIIC | DDSNL |
| GCI | 18-GC-Ser SIV | Serous carcinoma | IV | DH8PH |
| GCI | 19-GC-Endo SIA | Endometrioid Carcinoma | IA | E2WKF |
| GCI | 20-GC-Endo SIA | Endometrioid Carcinoma | IA | 5895C |
| GCI | 21-GC-Endo SIA | Endometrioid Carcinoma | IA | 533DX |
| GCI | 22-GC-Endo SIA | Endometrioid Carcinoma | IA | HZ2EY |
| GCI | 23-GC-Endo SIA | Endometrioid Carcinoma | IA | RWOIV |
| GCI | 24-GC-Endo SIIA | Endometrioid Carcinoma | IIA | 1U52X |
| GCI | 25-GC-Endo SIIB | Endometrioid Carcinoma | IIB | A17WS |
| GCI | 26-GC-Endo SIIIC | Endometrioid Carcinoma | IIIC | 1VT3I |
| GCI | 27-GC-Endo SIIIC | Endometrioid Carcinoma | IIIC | PZQXH |
| GCI | 28-GC-Endo SIV | Endometrioid Carcinoma | IV | I8VHZ |
| GOG | 29-(21)-GO-Muc SIA | Mucinous Carcinoma | IA | 95-10-G020 |
| GCI | 30-GC-Muc SIC | Mucinous Carcinoma | IC | IMDA1 |
| Asterand | 31-As-Muc SIC | Mucinous Carcinoma | IC | 18920A1 |
| ABS | 32-(22)-AB-Muc SIC | Mucinous Carcinoma | IC | A0139 |
| GCI | 33-GC-Muc SIIA | Mucinous Carcinoma | IIA | NJM4U |
| ABS | 34-(20)- AB-Muc SIIIA | Mucinous Carcinoma | IIIA | USA-00273 |
| GCI | 35-GC-Muc SIIIA | Mucinous Carcinoma | IIIA | RAFCW |
| Asterand | 36-As-Muc SIIIC | Mucinous Carcinoma | IIIC | 72888A1 |
| Asterand | 37-As-Muc SIIIC | Mucinous Carcinoma | IIIC | 29374B1 |
| GCI | 38-GC-Muc Border SIA | Mucinous borderline tumor | IA | SC656 |
| GCI | 39-GC-Muc Border SIA | Mucinous borderline tumor | IA | 3D5FO |
| GCI | 40-GC-Muc Border SIA | Mucinous borderline tumor | IA | 7JP3F |
| GOG | 41-(62)-Go-Ben Muc | Benign mucinous | | 99-10-G442 |
| GCI | 43-GC-Ben Muc | Benign mucinous | | QLIKY |
| Asterand | 44-As-Ben Muc | Benign mucinous | | 30534A1 |
| GOG | 45-(56)-GO-Ben Muc | Benign mucinous | | 99-01-G407 |
| GCI | 46-GC-Ben Muc | Benign mucinous | | 943EC |
| GCI | 47-GC-Ben Muc | Benign mucinous | | JO8W7 |
| Asterand | 48-As-Ben Ser | Benign seruos | | 30645B1 |
| GOG | 49-(64)-GO-Ben Ser | Benign seruos | | 99-06-G039 |
| GCI | 50-GC-Ben Ser | Benign seruos | | DQQ2F |
| Asterand | 51-As-Ben Ser | Benign seruos | | 8275A1 |
| Asterand | 52-As-N BM | Normal | | 23054A1 |
| Asterand | 53-As-N BM | Normal | | 30488A1 |
| Asterand | 54-As-N BM | Normal | | 30496B1 |
| Asterand | 55-As-N BM | Normal | | 30499C1 |
| GCI | 56-GC-N PS | Normal | | WPU1U |
| GCI | 58-GC-N PS | Normal | | 76VM9 |
| GCI | 59-GC-N PS | Normal | | DWHTZ |
| GCI | 60-GC-N PS | Normal | | SJ2R2 |
| GCI | 61-GC-N PS | Normal | | 9RQMN |
| GCI | 62-GC-N PS | Normal | | TOAE5 |
| GCI | 63-GC-N PS | Normal | | TW9PM |
| GCI | 64-GC-N PS | Normal | | 2VND2 |
| GCI | 65-GC-N PS | Normal | | L629F |
| GCI | 66-GC-N PS | Normal | | XLB23 |
| GCI | 67-GC-N PS | Normal | | IDUVY |
| GCI | 68-GC-N PS | Normal | | ZCXAD |
| GCI | 69-GC-N PS | Normal | | PEQ6C |
| GCI | 70-GC-N PS | Normal | | DD73B |
| GCI | 71-GC-N PS | Normal | | E2UF7 |
| GCI | 74-GC-N PS | Normal | | FDPL9 |
| BioChain | 76-(46)-Bc-N PM | Normal | | A504086 |
| Ichilov | 77-(71)-Ic-N PM | Normal | | CG-188-7 |
| BioChain | 78-(48)-Bc-N PM | Normal | | A504087 |

### Materials and Experimental Procedures Used to Obtain Expression Data

### RNA preparation -

RNA was obtained from ABS (Wilmington, DE 19801, USA, http://www.absbioreagents.com), BioChain Inst. Inc. (Hayward, CA 94545 USA www.biochain.com), GOG for ovary samples- Pediatic Cooperative Human Tissue Network, Gynecologic Oncology Group Tissue Bank, Children Hospital of Columbus (Columbus OH 43205 USA), Clontech (Franklin Lakes, NJ USA 07417, www.clontech.com), Ambion (Austin, TX 78744 USA, http://www.ambion.com), Asternad (Detroit, MI 48202-3420, USA, www.asterand.com), AllCells, LLC. (Emeryville, CA 94608 USA, www,allcells.co,), IMBCR- Institute for Myeloma and Bone cancer research (West Hollywood, CA 90069, USA, www.imbcr.org) and from Genomics Collaborative Inc.a Division of Seracare (Cambridge, MA 02139, USA, www.genomicsinc.com). Alternatively, RNA was generated from blood cells, cell lines or tissue samples using TRI-Reagent (Molecular Research Center), according to Manufacturer's instructions. Tissue and RNA samples were obtained from patients or from postmortem. Most total RNA samples were treated with DNaseI (Ambion).

RT PCR - Purified RNA (2-10 µg) was mixed with 300-1500 ng Random Hexamer primers (Invitrogen) and 500 µM dNTP in a total volume of 31.2 to 156 µl. The mixture was incubated for 5 min at 65 °C and then quickly chilled on ice. Thereafter, 10-50 µl of 5X SuperscliptII first strand buffer (Invitrogen), 4.8 to 24 µl 0.1M DTT and 80-400 units RNasin (Promega) were added, and the mixture was incubated for 10 min at 25 °C, followed by further incubation at 42 °C for 2 min. Then, 2-10 µl (400-2000 units) of SuperscriptII (Invitrogen) was added and the reaction (final volume of 50-250µl) was incubated for 50 min at 42 °C and then inactivated at 70 °C for 15min. The resulting cDNA was diluted 1:20 in TE buffer (10 mM Tris pH=8, 1 mM EDTA pH=8).

Real-Time RT-PCR analysis carried out as described below- cDNA (5µl), prepared as described above, was used as a template in Real-Time PCR reactions (final volume of 20 µl) using the SYBR Green I assay (PE Applied Biosystem) with specific primers and UNG Enzyme (Eurogentech or ABI or Roche). The amplification was effected as follows: 50 °C for 2 min, 95 °C for 10 min, and then 40 cycles of 95 °C for 15 sec, followed by 60 °C for 1 min, following by dissociation step. Detection was performed by using the PE Applied Biosystem SDS 7000. The cycle in which the reactions achieved a threshold level of fluorescence (Ct= Threshold Cycle, described in detail below) was registered and was used to calculate the relative transcript quantity in the RT reactions. The relative quantity was calculated using the equation Q=efficiency^-Ct. The efficiency of the PCR reaction was calculated from a standard curve, created by using different dilutions of several reverse transcription (RT) reactions. To minimize inherent differences in the RT reaction, the resulting relative quantities were normalized using a normalization factor calculated in the following way:

The expression of several housekeeping (HSKP) genes was checked on every panel. The relative quantity (Q) of each housekeeping gene in each sample, calculated as described above, was divided by the median quantity of this gene in all panel samples to obtain the "relative Q rel to MED". Then, for each sample the median of the "relative Q rel to MED" of the selected housekeeping genes was calculated and served as normalization factor of this sample for further calculations. It should be noted that this type of analysis provides relative quantification.

For each RT sample, the expression of the specific amplicon was normalized to the normalization factor calculated from the expression of different house keeping genes as described in section above.

These house keeping genes are different for each panel.

The sequences for primers and amplicons of the housekeeping genes measured in all the ovary cancer examples are HPRT1, SDHA and G6PD.

SDHA (GenBank Accession No. NM_004168 (SEQ ID NO:136); amplicon - SDHA-amplicon (SEQ ID NO:85)), SDHA Forward primer (SEQ ID NO:83); SDHA Reverse primer (SEQ ID NO:84);

HPRT1 (GenBank Accession No. NM_000194 (SEQ ID NO:137); amplicon - HPRT1-amplicon (SEQ ID NO:88)); HPRT1 Forward primer (SEQ ID NO:86)), HPRT1 Reverse primer (SEQ ID NO:87);

G6PD (GenBank Accession No. NM_000402 (SEQ ID NO:138); G6PD amplicon (SEQ ID NO: 91)), G6PD Forward primer (SEQ ID NO:89), G6PD Reverse primer (SEQ ID NO:90).

The sequences of the housekeeping genes measured in all the examples on normal tissue samples panel were as follows:

SDHA (GenBank Accession No. NM_004168 (SEQ ID NO:136); amplicon - SDHA-amplicon (SEQ ID NO:85)), Forward primer (SEQ ID NO:83), SDHA Reverse primer (SEQ ID NO:84).

Ubiquitin (GenBank Accession No. BC000449 (SEQ ID NO:139); amplicon - Ubiquitin-amplicon (SEQ ID NO: 82)), Ubiquitn Forward primer (SEQ ID NO:80), Ubiquitin Reverse primer (SEQ ID NO:81).

TATA box (GenBank Accession No. NM_003194 (SEQ ID NO:140); TATA amplicon (SEQ ID NO: 79)), TATA box Forward primer (SEQ ID NO:77), TATA box Reverse primer (SEQ ID NO:78).

The sequences of the housekeeping genes measured in all the examples of blood panel were as follows:

HSB1L_HUMAN (Accession No. Q9Y450 (SEQ ID NO:141)), T05337_seg30-34F1-Forward primer (SEQ ID NO:68), T05337_seg30-34R1 Reverse primer (SEQ ID NO:69), T05337_seg30-34Amplicon (SEQ ID NO:70).

DHSA_HUMAN (Accession No P31040 (SEQ ID NO:142)), M78124_seg45-48F1 Forward primer (SEQ ID NO:71), M78124_seg45-48R1-Reverse primer (SEQ ID NO:72), M78124_seg45-48Amplicon (SEQ ID NO:73).

SLC25A3 (Accession No Q7Z7N7 (SEQ ID NO:144)), SSMPCPseg24-25-29F1- Forward primer (SEQ ID NO:74), SSMPCPseg24-25-29R1- Reverse primer (SEQ ID NO:75), SSMPCPseg24-25-29Amplicon (SEQ ID NO:76).

SFRS4_HUMSRP75A (Accession NO Q08170 (SEQ ID NO:143)), HUMSRP75Aseg30-33F1 Forward primer (SEQ ID NO:65), HUMSRP75Aseg30-33R1 Reverse primer (SEQ ID NO:66), HUMSRP75Aseg30-33Amplicon (SEQ ID NO:67).

HPRT1 (Accession No. NM_000194 (SEQ ID NO:137), HUMHPRTCseg5-7F1 - forward primer (SEQ ID NO:34), HUMHPRTCseg5-7R1 - reverse primer (SEQ ID NO:37), HUMHPRTCseg5-7 Amplicon (SEQ ID NO:126).

TBP -TATA Box binding protein (Accession NO P20226 (SEQ ID NO:145)), HSTFIIDXseg7-9F1 - forward primer (SEQ ID NO:128), HSTFIIDXseg7-9R1 - reverse primer (SEQ ID NO:129), HSTFIIDXseg7-9 Amplicon (SEQ ID NO:130).

Another methodology used to predict the expression pattern of the proteins according to at least some embodiments of the invention was MED discovery engine:

MED is a platform for collection of public gene-expression data, normalization, annotation and performance of various queries. Expression data from the most widely used Affymetrix microarrays is downloaded from the Gene Expression Omnibus (GEO - www.ncbi.nlm.nih.gov/GEO). Data is multiplicatively normalized by setting the 95 percentile to a constant value (normalized expression=1200), and noise is filtered by setting the lower 30% to 0. Experiments are annotated, first automatically, and then manually, to identify tissue and condition, and chips are grouped according to this annotation, and cross verification of this grouping by comparing the overall expression pattern of the genes of each chip to the overall average expression pattern of the genes in this group. Each probeset in each group is assigned an expression value which is the median of the expressions of that probeset in all chips included in the group. The vector of expression of all probesets within a certain group is the virtual chip of that group, and the collection of all such virtual chips is a virtual panel. The panel (or sub-panels) can be queried to identify probesets with a required behavior (e.g. specific expression in a sub-set of tissues, or differential expression between disease and healthy tissues). These probesets are linked to LEADS contigs and to RefSeqs (http://www.ncbi.nlm.nih.gov/RefSeq/) by probe-level mapping, for further analysis.

The Affymetrix platforms that are downloaded are HG-U95A and the HG-U133 family (A,B, A2.0 and PLUS 2.0). Than three virtual panels were created: U95 and U133 Plus 2.0, based on the corresponding platforms, and U133 which uses the set of common probesets for HG-U133A, HG-U133A2.0 and HG-U133 PLUS 2.0+.

The results of the MED discovery engine are presented in scatter plots. The scatter plot is a compact representation of a given panel (collection of groups). The y-axis is the (normalized) expression and the x-axis describes the groups in the panel. For each group, the median expression is represented by a solid marker., and the expression values of the different chips in the group are represented by small dashes ("-"). The groups are ordered and marked as follows - "Other" groups (e.g. benign, non-cancer diseases, etc.) with a triangle, Treated cells with a square, Normal with a circle, Matched with a cross, and Cancer with a diamond. The number of chips in each group is also written adjacent to it's name.

### EXAMPLE 2: KRTCAP3 POLYPEPTIDES AND POLYNUCLEOTIDES, AND USES THEREOF AS A DRUG TARGET FOR PRODUCING DRUGS AND BIOLOGICS

### EXAMPLE 2_1: DESCRIPTION FOR CLUSTER W93943

Cluster W93943 (internal ID 72425829) features 6 transcripts and of interest, the names for which are given in Table 5. The selected protein variants are given in table 6.

**Table 5 - Transcripts of interest**

| **Transcript Name** |
|---|
| W93943_T0 (SEQ ID NO:1) |
| W93943_T5 (SEQ ID NO:3) |
| W93943_T8 (SEQ ID NO:4) |
| W93943_T13 (SEQ ID NO:5) |
| W93943_T14 (SEQ ID NO:6) |

**Table 6 - Proteins of interest**

| **Protein Name** | **Coresponding Transcript(s)** |
|---|---|
| W93943_P2 (SEQ ID NO:7) | W93943_T0 (SEQ ID NO:1) |
| W93943_P13 (SEQ ID NO:10) | W93943_T5 (SEQ ID NO:3) |
| W93943_P14 (SEQ ID NO:11) | W93943_T8 (SEQ ID NO:4) |
| W93943_P17 (SEQ ID NO:12) | W93943_T13 (SEQ ID NO:5) |
| W93943_P18 (SEQ ID NO:13) | W93943_T14 (SEQ ID NO:6) |

These sequences are variants of the known protein Keratinocytes-associated protein 3 (SwissProt accession identifier KCP3_HUMAN (SEQ ID NO:7); known also according to the synonyms KCP-3, KRTCAP3).

KRTCAP3 (keratinocyte associated protein 3) was identified in several in large scale studies, such as the identification of secreted and membrane protein in keratinocytes (Bonkobara et al. 2003, Br J Dermatol. 148(4):654-64), the secreted protein initiative (Clark et al. 2003, Genome Research 13(10): 2265-70), annotation of chromosomes 2 and 4 (Hillier et al. 2005, Nature 434(7034): 724-31), and full length cDNA projects (Gerhard et al. 2004, Genome Res. 14(10B): 2121-7; Strausberg et al. 2002, PNAS 99(26): 16899-903). However no specific information was published about KRTCAP3.

Sequence depicted in W93943_P17 (SEQ ID NO:12) encoded by the corresponding W93943_T13 (SEQ ID NO:5), was reported in WO2000000506, among other human proteins having hydrophobic domains. The WO2000000506 patent application does not teach, however, that sequence corresponding to W93943_P17 (SEQ ID NO:12) or W93943_T13 (SEQ ID NO:5) are differentially expressed in ovarian cancer, lung cancer or in any other pathology. Also, there is no teaching in WO2000000506 application that W93943_P17 (SEQ ID NO:12) or W93943_T13 (SEQ ID NO:5) can be used as drug target for treatment of cancer and/or immune related conditions or for diagnosis thereof. Also, there is no teaching in WO2000000506 application that antibodies specific W93943_P17 (SEQ ID NO:12), its soluble ectodomain, and/or fragments thereof can be used as therapeutic or diagnostic agents for treatment of cancer and/or immune related conditions.

Sequence depicted in W93943_P2 (SEQ ID NO:7) was reported in several patent applications. For example, US20070065888 reports W93943_P2 (SEQ ID NO:7) among very large number of other genes. US20070065888 purports to disclose methods and reagents including antibodies specific to various tumor antigens for evaluating cancer prognosis and for use in therapies. The US20070065888 patent application does not teach, however, that expression of the sequence corresponding to W93943_P2 (SEQ ID NO:7) or the use of antibodies thereto is correlated specifically to the treatment or diagnosis of cancer, or breast, colon, or ovarian cancer, and/or immune related conditions.

WO200190304 reports W93943_P2 (SEQ ID NO:7) sequence among very large number of other genes. WO200190304 purports to disclose isolated nucleic acid molecules encoding novel polypeptides and antibodies that bind to these polypeptides. The application further purportedly relates to diagnostic and therapeutic methods useful for diagnosing, treating, preventing and/or prognosing disorders related to these novel polypeptides and to screening methods for identifying agonists and antagonists of these polynucleotides and polypeptides. The application also purports to provide methods and/or compositions for inhibiting or enhancing the production and function of these polypeptides including antibody based therapies. However, The WO200190304 patent application does not provide any specific teaching or incentive that would direct a skilled artisan to use antibodies specific to the polypeptide encoded by the sequence corresponding to KRTCAP3 for the treatment or diagnosis of cancer or specifically ovarian, lung, breast or colon cancer, and/or immune related conditions.

WO2004091511 reports KRTCAP3 among very large number of other genes. This application predominantly relates to compositions and methods purported to be useful for aiding in the diagnoses of the neoplastic condition of a lung cell, and methods of screening for a potential therapeutic agent for the reversal of the neoplastic condition. Also allegedly provided are therapeutic compositions and methods to inhibit the growth of neoplastic lung cells and to treat subjects harboring neoplastic lung cells. The WO2004091511 patent application does not teach, however, that sequence corresponding to KRTCAP3 is differentially expressed in ovarian cancer or breast cancer or colon cancer or in immune related conditions. Also, there is no teaching in WO2004091511 application that antibodies specific to KRTCAP3, its soluble ectodomain, and/or fragments thereof can be used as therapeutic or diagnostic agents for treatment of cancer, especially ovarian and/or breast and/or colon cancer cancer and/or immune related conditions.

US2003100727 patent application and it's related counterparts disclose PRO9898 (keratinocyte associated protein 3) and purport to teach the production and use of antibodies to this and other secreted proteins for the treatment of cancers. However, these applications do not appear to teach or suggest the treatment or diagnosis of ovarian cancer. Also, there is no teaching in US2003100727 application that antibodies specific to KRTCAP3, its soluble ectodomain, and/or fragments thereof can be used as therapeutic or diagnostic agents for treatment of ovarian, breast, colon, or lung cancer and/or immune related conditions.

WO06110593 patent application purports to describe methods for detecting, diagnosing, monitoring and prognosing cancer by detecting differences in the expression of one or more, or any combination of genes which includes KRTCAP3. WO06110593 also purport to describe methods for screening and identifying compounds that modulate the expression of one or more, or any combination of such genes and corresponding gene products. It further mentions the use of such compounds in the prevention, treatment, management and amelioration of cancer. The application purports to describe the administration of an effective amount of one or more therapeutics including antibodies that modulate the expression and/or activity of one or more cancer targets disclosed for the prevention, treatment, management and amelioration of cancer. However, there is no teaching in WO06110593 application that antibodies specific to KRTCAP3, its soluble ectodomain, and/or fragments thereof and specific antibodies against it can be used as therapeutic or diagnostic agents for treatment of ovarian, breast, colon or lung cancer and/or immune related conditions.

The amino acid sequence of W93943_P13 (SEQ ID NO:10) and W93943_P14 (SEQ ID NO:11) were previously disclosed by the applicants of the current application in a US patent application No: 11/043,860. However, there is no teaching in US 11/043,860 application that KRTCAP3 soluble ectodomain, as well as fragments thereof and specific antibodies against it can be used as therapeutic or diagnostic agents for treatment of ovarian, breast, colon or lung cancer and/or immune related conditions.

In particular, at least some embodiments of the present invention relate to the use of novel KRTCAP3 variants and discrete portions thereof as a drug target for therapeutic small molecules, peptides, antibodies, antisense RNAs, siRNAs, ribozymes, and the like. More particularly the invention relates to diagnostic and therapeutic polyclonal and monoclonal antibodies and fragments thereof that bind KRTCAP3 variants, and portions and variants thereof. It is a specific object according to at least some embodiments of the present invention to use antibodies and antibody fragments against KRTCAP3 antigens, its secreted or soluble form conjugates, or fragments thereof for treating and diagnosing ovarian cancer and/or breast cancer, and/or colon cancer and/or immune related conditions, wherein this antigen is differentially expressed.

Known polymorphisms A to T at position 79; G to R at position 14; and L to P at position 114 of KRTCAP3 protein (SEQ ID NO:7) were previously reported.

Keratinocytes-associated protein 3 (SEQ ID NO:7) is believed to be multi-pass membrane protein.

As noted above, cluster W93943 features 5 transcripts, which were listed in Table 5 above. These transcripts encode for proteins which are variants of protein Keratinocytes-associated protein 3 (SEQ ID NO:7). A description of each variant protein according to at least some embodiments of the invention is now provided.

Protein W93943_P2 (SEQ ID NO:7) is encoded by the following transcripts: W93943_T0 (SEQ ID NO:1). The coding portion of transcript W93943_T0 (SEQ ID NO:1) starts at position 77 and ends at position 796. The transcript also has the following SNPs as listed in Table 7 (given according to their position on the nucleotide sequence, with the alternative nucleic acid listed).

**Table 7 - Nucleic acid SNPs**

| **Polymorphism** | **SNP position(s) on nucleotide sequence** |
|---|---|
| G→A | 116,859 |
| G->C | 116 |
| T->C | 417 |

Variant protein W93943_P13 (SEQ ID NO:10) according to at least some embodiments of the invention has an amino acid sequence encoded by transcript W93943_T5 (SEQ ID NO:3). A description of the relationship of the variant protein according to at least some embodiments of the invention to known proteins is as follows:
1. Comparison report between W93943_P13 (SEQ ID NO:10) and known protein(s) KCP3_HUMAN (SEQ ID NO:7) :
   A. An isolated chimeric polypeptide, comprising a first amino acid sequence being at least 90% homologous to MRRCSLCAFDAARGPRRLMRVGLALILVGHVNLLLGAVLHGTVLRHVANPRGAV TPEYTVANVISVGSGLL corresponding to amino acids 1 - 71 of known protein KCP3_HUMAN (SEQ ID NO:7), which also corresponds to amino acids 1 - 71 of W93943_P13 (SEQ ID NO:10), a second amino acid sequence being at least 70%, optionally at least 80%, preferably at least 85%, more preferably at least 90% and most preferably at least 95% homologous to a polypeptide having the sequence VSAAGDPGGGRAPGEPSRPKALCLPQ (SEQ ID NO: 146) corresponding to amino acids 72 - 97 of W93943_P13 (SEQ ID NO:10), and a third amino acid sequence being at least 90% homologous to SVSVGLVALLASRNLLRPPLHWVLLALALVNLLLSVACSLGLLLAVSLTVANGGRR LIADCHPGLLDPLVPLDEGPGHTDCPFDPTRIYDTALALWIPSLLMSAGEAALSGYC CVAALTLRGVGPCRKDGLQGQLEEMTELESPKCKRQENEQLLDQNQEIRASQRSW V corresponding to amino acids 72 - 240 of known protein KCP3_HUMAN (SEQ ID NO:7), which also corresponds to amino acids 98 - 266 of W93943_P13 (SEQ ID NO:10), wherein said first amino acid sequence, second amino acid sequence and third amino acid sequence are contiguous and in a sequential order.
   B. An isolated polypeptide corresponding to an edge portion of W93943_P13 (SEQ ID NO:10), comprising an amino acid sequence being at least 70%, optionally at least about 80%, preferably at least about 85%, more preferably at least about 90% and most preferably at least about 95% homologous to the sequence VSAAGDPGGGRAPGEPSRPKALCLPQ (SEQ ID NO: 146) of W93943_P13 (SEQ ID NO:10).
2. Comparison report between W93943_P13 (SEQ ID NO:10) and known protein NP_776252 (SEQ ID NO:8) :
   A. An isolated chimeric polypeptide, comprising a first amino acid sequence being at least 90% homologous to MRRCSLCAFDAARGPRRLMRVGLALILVGHVNLLLGAVLHGTVLRHVANPRGAV TPEYTVANVISVGSGLL corresponding to amino acids 1 - 71 of known protein NP_776252 (SEQ ID NO:8), which also corresponds to amino acids 1 - 71 of W93943_P13 (SEQ ID NO:10), a second amino acid sequence being at least 70%, optionally at least 80%, preferably at least 85%, more preferably at least 90% and most preferably at least 95% homologous to a polypeptide having the sequence VSAAGDPGGGRAPGEPSRPKALCLPQ (SEQ ID NO: 146) corresponding to amino acids 72 - 97 of W93943_P13 (SEQ ID NO:10), a third amino acid sequence being at least 90% homologous to SVSVGLV corresponding to amino acids 72 - 78 of known protein NP_776252 (SEQ ID NO:8), which also corresponds to amino acids 98 - 104 of W93943_P13 (SEQ ID NO:10), a bridging amino acid A corresponding to amino acid 105 of W93943_P13 (SEQ ID NO:10), and a fourth amino acid sequence being at least 90% homologous to LLASRNLLRPPLHWVLLALALVNLLLSVACSLGLLLAVSLTVANGGRRLIADCHPG LLDPLVPLDEGPGHTDCPFDPTRIYDTALALWIPSLLMSAGEAALSGYCCVAALTLR GVGPCRKDGLQGQLEEMTELESPKCKRQENEQLLDQNQEIRASQRSWV corresponding to amino acids 80 - 240 of known protein NP_776252 (SEQ ID NO:8), which also corresponds to amino acids 106 - 266 of W93943_P13 (SEQ ID NO:10), wherein said first amino acid sequence, second amino acid sequence, third amino acid sequence, bridging amino acid and fourth amino acid sequence are contiguous and in a sequential order.
   B. An isolated polypeptide corresponding to an edge portion of W93943_P13 (SEQ ID NO:10), comprising an amino acid sequence being at least 70%, optionally at least about 80%, preferably at least about 85%, more preferably at least about 90% and most preferably at least about 95% homologous to the sequence VSAAGDPGGGRAPGEPSRPKALCLPQ (SEQ ID NO: 146) of W93943_P13 (SEQ ID NO:10).

The localization of the variant protein was determined according to results from a number of different software programs and analyses, including analyses from SignalP and other specialized programs. The variant protein is believed to be located as follows with regard to the cell: membrane.

Variant protein W93943_P13 (SEQ ID NO:10) also has the following non-silent SNPs (Single Nucleotide Polymorphisms) as listed in Table 8, (given according to their position(s) on the amino acid sequence, with the alternative amino acid(s) listed).

**Table 8 - Amino acid mutations**

| **SNP position(s) on amino acid sequence** | **Alternative amino acid(s)** |
|---|---|
| 14 | G -> R |
| 140 | L -> P |

The coding portion of transcript W93943_T5 (SEQ ID NO:3) starts at position 77 and ends at position 874. The transcript also has the following SNPs as listed in Table 9 (given according to their position on the nucleotide sequence, with the alternative nucleic acid listed).

**Table 9 - Nucleic acid SNPs**

| **Polymorphism** | **SNP position(s) on nucleotide sequence** |
|---|---|
| G -> A | 116,937 |
| G -> C | 116 |
| T -> C | 495 |

Variant protein W93943_P14 (SEQ ID NO:11) according to at least some embodiments of the invention has an amino acid sequence encoded by transcriptW93943_T8 (SEQ ID NO:4). A description of the relationship of the variant protein according to at least some embodiments of the invention to known proteins is as follows:
1. Comparison report between W93943_P14 (SEQ ID NO:11) and known protein KCP3_HUMAN (SEQ ID NO:7) :
   A. An isolated chimeric polypeptide, comprising a first amino acid sequence being at least 90% homologous to MRRCSLCAFDAARGPRRLMRVGLALILVGHVNLLLGAVLHGTVLRHVANPRGAV TPEYTVANVISVGSGLLSVSVGLVALLASRNLLRPPLHWVLLALALVNLLLSVACS LGLLLAVSLTVANGGRRLIADCHPGLLDPLVPLDEGPGHTDCPFDPTRIYDTALAL WIPSLLMSAGEAALSGYCCVAALTLRGVGPCRKDGLQGQ corresponding to amino acids 1 - 205 of known protein KCP3_HUMAN (SEQ ID NO:7), which also corresponds to amino acids 1 - 205 of W93943_P14 (SEQ ID NO:11), and a second amino acid sequence being at least 70%, optionally at least 80%, preferably at least 85%, more preferably at least 90% and most preferably at least 95% homologous to a polypeptide having the sequence VRKANRKGSFHRDWLC (SEQ ID NO: 147) corresponding to amino acids 206 - 221 of W93943_P14 (SEQ ID NO:11), wherein said first amino acid sequence and second amino acid sequence are contiguous and in a sequential order.
   B. An isolated polypeptide corresponding to an edge portion of W93943_P14 (SEQ ID NO:11), comprising an amino acid sequence being at least 70%, optionally at least about 80%, preferably at least about 85%, more preferably at least about 90% and most preferably at least about 95% homologous to the sequence VRKANRKGSFHRDWLC (SEQ ID NO: 147) of W93943_P14 (SEQ ID NO:11).
2. Comparison report between W93943_P14 (SEQ ID NO:11) and known protein NP_776252 (SEQ ID NO:8) :
   A. An isolated chimeric polypeptide, comprising a first amino acid sequence being at least 90% homologous to MRRCSLCAFDAARGPRRLMRVGLALILVGHVNLLLGAVLHGTVLRHVANPRGAV TPEYTVANVISVGSGLLSVSVGLV corresponding to amino acids 1 - 78 of known protein NP_776252 (SEQ ID NO:8), which also corresponds to amino acids 1 - 78 of W93943_P14 (SEQ ID NO:11), a bridging amino acid A corresponding to amino acid 79 of W93943_P14 (SEQ ID NO:11), a second amino acid sequence being at least 90% homologous to LLASRNLLRPPLHWVLLALALVNLLLSVACSLGLLLAVSLTVANGGRRLIADCHPG LLDPLVPLDEGPGHTDCPFDPTRIYDTALALWIPSLLMSAGEAALSGYCCVAALTLR GVGPCRKDGLQGQ corresponding to amino acids 80 - 205 of known protein NP_776252 (SEQ ID NO:8), which also corresponds to amino acids 80 - 205 of W93943_P14 (SEQ ID NO:11), and a third amino acid sequence being at least 70%, optionally at least 80%, preferably at least 85%, more preferably at least 90% and most preferably at least 95% homologous to a polypeptide having the sequence VRKANRKGSFHRDWLC (SEQ ID NO: 147) corresponding to amino acids 206 - 221 of W93943_P14 (SEQ ID NO:11), wherein said first amino acid sequence, bridging amino acid, second amino acid sequence and third amino acid sequence are contiguous and in a sequential order.
   B. An isolated polypeptide corresponding to an edge portion of W93943_P14 (SEQ ID NO:11), comprising an amino acid sequence being at least 70%, optionally at least about 80%, preferably at least about 85%, more preferably at least about 90% and most preferably at least about 95% homologous to the sequence VRKANRKGSFHRDWLC (SEQ ID NO: 147) of W93943_P14 (SEQ ID NO:11).

The localization of the variant protein was determined according to results from a number of different software programs and analyses, including analyses from SignalP and other specialized programs. The variant protein is believed to be located as follows with regard to the cell: membrane.

Variant protein W93943_P14 (SEQ ID NO:11) also has the following non-silent SNPs (Single Nucleotide Polymorphisms) as listed in Table 10, (given according to their position(s) on the amino acid sequence, with the alternative amino acid(s) listed).

**Table 10 - Amino acid mutations**

| **SNP position(s) on amino acid sequence** | **Alternative amino acid(s)** |
|---|---|
| 14 | G -> R |
| 114 | L -> P |

The coding portion of transcript W93943_T8 (SEQ ID NO:4) starts at position 77 and ends at position 739. The transcript also has the following SNPs as listed in Table 11 (given according to their position on the nucleotide sequence, with the alternative nucleic acid listed).

**Table 11 - Nucleic acid SNPs**

| **Polymorphism** | **SNP position(s) on nucleotide sequence** |
|---|---|
| G -> A | 116, 1275 |
| G -> C | 116 |
| T -> C | 417 |

Variant protein W93943_P17 (SEQ ID NO:12) according to at least some embodiments of the invention has an amino acid sequence as encoded by transcriptW93943_T13 (SEQ ID NO:5). A description of the relationship of the variant protein according to at least some embodiments of the invention to known proteins is as follows:
1. Comparison report between W93943_P17 (SEQ ID NO:12) and known protein KCP3_HUMAN (SEQ ID NO:7) :
   A. An isolated chimeric polypeptide, comprising a first amino acid sequence being at least 90% homologous to MRRCSLCAFDAARGPRRLMRVGLALILVGHVNLLLGAVLHGTVLRHVANPRGAV TPEYTVANVISVGSGLLSVSVGLVALLASRNLLRPPLHWVLLALALVNLLLSVACS LGLLLAVSLTVANGGRRLIADCHPGLLDPLVPLDEGPGHTDCPFDPTRIYDTALAL WIPSLLMSAGEAALSGYCCVAALTLRGVGPCRKDGLQGQ corresponding to amino acids 1 - 205 of known protein KCP3_HUMAN (SEQ ID NO:7), which also corresponds to amino acids 1 - 205 of W93943_P17 (SEQ ID NO:12), and a second amino acid sequence being at least 70%, optionally at least 80%, preferably at least 85%, more preferably at least 90% and most preferably at least 95% homologous to a polypeptide having the sequence VVAGCDARVKQKAWQPRFPGIKVKAL (SEQ ID NO: 148) corresponding to amino acids 206 - 231 of W93943_P17 (SEQ ID NO:12), wherein said first amino acid sequence and second amino acid sequence are contiguous and in a sequential order.
   B. An isolated polypeptide corresponding to an edge portion of W93943_P17 (SEQ ID NO:12), comprising an amino acid sequence being at least 70%, optionally at least about 80%, preferably at least about 85%, more preferably at least about 90% and most preferably at least about 95% homologous to the sequence VVAGCDARVKQKAWQPRFPGIKVKAL (SEQ ID NO: 148) of W93943_P17 (SEQ ID NO:12).
2. Comparison report between W93943_P17 (SEQ ID NO:12) and known protein NP_776252 (SEQ ID NO:8) :
   A. An isolated chimeric polypeptide, comprising a first amino acid sequence being at least 90% homologous to MRRCSLCAFDAARGPRRLMRVGLALILVGHVNLLLGAVLHGTVLRHVANPRGAV TPEYTVANVISVGSGLLSVSVGLV corresponding to amino acids 1 - 78 of known protein NP_776252 (SEQ ID NO:8), which also corresponds to amino acids 1 - 78 of W93943_P17 (SEQ ID NO:12), a bridging amino acid A corresponding to amino acid 79 of W93943_P17 (SEQ ID NO:12), a second amino acid sequence being at least 90% homologous to LLASRNLLRPPLHWVLLALALVNLLLSVACSLGLLLAVSLTVANGGRRLIADCHPG LLDPLVPLDEGPGHTDCPFDPTRIYDTALALWIPSLLMSAGEAALSGYCCVAALTLR GVGPCRKDGLQGQ corresponding to amino acids 80 - 205 of known protein NP_776252 (SEQ ID NO:8), which also corresponds to amino acids 80 - 205 of W93943_P17 (SEQ ID NO:12), and a third amino acid sequence being at least 70%, optionally at least 80%, preferably at least 85%, more preferably at least 90% and most preferably at least 95% homologous to a polypeptide having the sequence VVAGCDARVKQKAWQPRFPGIKVKAL (SEQ ID NO: 148) corresponding to amino acids 206 - 231 of W93943_P17 (SEQ ID NO:12), wherein said first amino acid sequence, bridging amino acid, second amino acid sequence and third amino acid sequence are contiguous and in a sequential order.
   B. An isolated polypeptide corresponding to an edge portion of W93943_P17 (SEQ ID NO:12), comprising an amino acid sequence being at least 70%, optionally at least about 80%, preferably at least about 85%, more preferably at least about 90% and most preferably at least about 95% homologous to the sequence VVAGCDARVKQKAWQPRFPGIKVKAL (SEQ ID NO: 148) of W93943_P17 (SEQ ID NO:12).

The localization of the variant protein was determined according to results from a number of different software programs and analyses, including analyses from SignalP and other specialized programs. The variant protein is believed to be located as follows with regard to the cell: membrane.

Variant protein W93943_P17 (SEQ ID NO:12) also has the following non-silent SNPs (Single Nucleotide Polymorphisms) as listed in Table 12, (given according to their position(s) on the amino acid sequence, with the alternative amino acid(s) listed).

**Table 12 - Amino acid mutations**

| **SNP position(s) on amino acid sequence** | **Alternative amino acid(s)** |
|---|---|
| 14 | G -> R |
| 114 | L -> P |

The coding portion of transcript W93943_T13 (SEQ ID NO:5) starts at position 77 and ends at position 769. The transcript also has the following SNPs as listed in Table 13 (given according to their position on the nucleotide sequence, with the alternative nucleic acid listed).

**Table 13 - Nucleic acid SNPs**

| **Polymorphism** | **SNP position(s) on nucleotide sequence** |
|---|---|
| G -> A | 116 |
| G -> C | 116 |
| T -> C | 417 |
| A -> | 865 |
| A -> T | 865 |

Variant protein W93943_P18 (SEQ ID NO:13) according to at least some embodiments of the invention has an amino acid sequence as encoded by transcript W93943_T14 (SEQ ID NO:6). A description of the relationship of the variant protein according to at least some embodiments of the invention to known proteins is as follows:
1. Comparison report between W93943_P18 (SEQ ID NO:13) and known protein KCP3_HUMAN (SEQ ID NO:7) :
   A. An isolated chimeric, comprising a first amino acid sequence being at least 90% homologous to MRRCSLCAFDAARGPRRLMRVGLALILVGHVNLLLGAVLHGTVLRHVANPRGAV TPEYTVANVISVGSGLLSVSVGLVALLASRNLLRPPL corresponding to amino acids 1 - 91 of known protein KCP3_HUMAN (SEQ ID NO:7), which also corresponds to amino acids 1 - 91 of W93943_P18 (SEQ ID NO:13), and a second amino acid sequence being at least 90% homologous to DTALALWIPSLLMSAGEAALSGYCCVAALTLRGVGPCRKDGLQGQLEEMTELESP KCKRQENEQLLDQNQEIRASQRSWV corresponding to amino acids 161 - 240 of known protein KCP3_HUMAN (SEQ ID NO:7), which also corresponds to amino acids 92 - 171 of W93943_P18 (SEQ ID NO:13), wherein said first amino acid sequence and second amino acid sequence are contiguous and in a sequential order.
   B. An isolated chimeric polypeptide corresponding to an edge portion of W93943_P18 (SEQ ID NO:13), comprising a polypeptide having a length "n", wherein n is at least about 10 amino acids in length, optionally at least about 20 amino acids in length, preferably at least about 30 amino acids in length, more preferably at least about 40 amino acids in length and most preferably at least about 50 amino acids in length, wherein at least two amino acids comprise LD, having a structure as follows: a sequence starting from any of amino acid numbers 91-x to 91; and ending at any of amino acid numbers 92 + ((n-2)-x), in which x varies from 0 to n-2.
2. Comparison report between W93943_P18 (SEQ ID NO:13) and known protein NP_776252 (SEQ ID NO:8) :
   A. An isolated chimeric polypeptide, comprising a first amino acid sequence being at least 90% homologous to MRRCSLCAFDAARGPRRLMRVGLALILVGHVNLLLGAVLHGTVLRHVANPRGAV TPEYTVANVISVGSGLLSVSVGLV corresponding to amino acids 1 - 78 of known protein NP_776252 (SEQ ID NO:8), which also corresponds to amino acids 1 - 78 of W93943_P18 (SEQ ID NO:13), a bridging amino acid A corresponding to amino acid 79 of W93943_P18 (SEQ ID NO:13), a second amino acid sequence being at least 90% homologous to LLASRNLLRPPL corresponding to amino acids 80 - 91 of known protein NP_776252 (SEQ ID NO:8), which also corresponds to amino acids 80 - 91 of W93943_P18 (SEQ ID NO:13), and a third amino acid sequence being at least 90% homologous to DTALALWIPSLLMSAGEAALSGYCCVAALTLRGVGPCRKDGLQGQLEEMTELESP KCKRQENEQLLDQNQEIRASQRSWV corresponding to amino acids 161 - 240 of known protein NP_776252 (SEQ ID NO:8), which also corresponds to amino acids 92 - 171 of W93943_P18 (SEQ ID NO:13), wherein said first amino acid sequence, bridging amino acid, second amino acid sequence and third amino acid sequence are contiguous and in a sequential order.
   B. An isolated chimeric polypeptide corresponding to an edge portion of W93943_P18 (SEQ ID NO:13), comprising a polypeptide having a length "n", wherein n is at least about 10 amino acids in length, optionally at least about 20 amino acids in length, preferably at least about 30 amino acids in length, more preferably at least about 40 amino acids in length and most preferably at least about 50 amino acids in length, wherein at least two amino acids comprise LD, having a structure as follows: a sequence starting from any of amino acid numbers 91-x to 91; and ending at any of amino acid numbers 92 + ((n-2)-x), in which x varies from 0 to n-2.

The localization of the variant protein was determined according to results from a number of different software programs and analyses, including analyses from SignalP and other specialized programs. The variant protein is believed to be located as follows with regard to the cell: membrane.

Variant protein W93943_P18 (SEQ ID NO:13) also has the following non-silent SNPs (Single Nucleotide Polymorphisms) as listed in Table 14, (given according to their position(s) on the amino acid sequence, with the alternative amino acid(s) listed).

**Table 14 - Amino acid mutations**

| **SNP position(s) on amino acid sequence** | **Alternative amino acid(s)** |
|---|---|
| 14 | G -> R |

The coding portion of transcript W93943_T14 (SEQ ID NO:6) starts at position 77 and ends at position 589. The transcript also has the following SNPs as listed in Table 15 (given according to their position on the nucleotide sequence, with the alternative nucleic acid listed).

**Table 15 - Nucleic acid SNPs**

| **Polymorphism** | **SNP position(s) on nucleotide sequence** |
|---|---|
| G -> A | 116, 652 |
| G -> C | 116 |

### EXAMPLE 2_2: ANALYSIS OF THE EXPRESSION OF KRTCAP3 TRANSCRIPTS

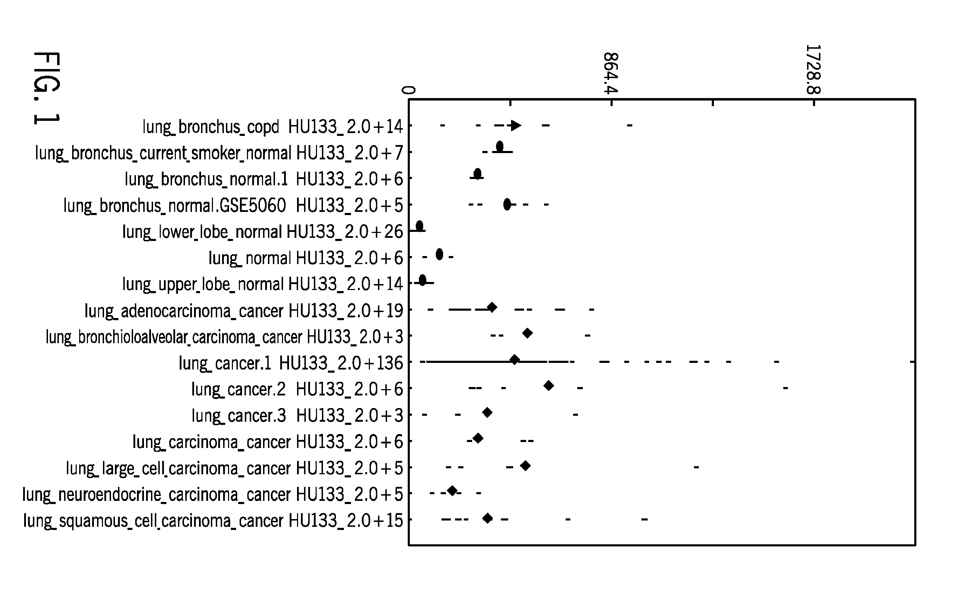

MED discovery engine described in Example 1 herein, was used to assess the expression of KRTCAP3 transcripts. KRTCAP3 transcripts were found to be over expressed in lung cancer, as is demonstrated in Figure 1. Figure 1 shows expression graphs of Affymetrix probe set 235148_at. Figure 1 shows the expression of KRTCAP3 transcripts in microarray chips from lung cancer and lung normal experiments. As can be seen KRTCAP3 transcripts are overexpressed in lung cancer tissues (diamond markers) relative to its expression in normal lung (circle markers).

### Expression of KRTCAP3 (keratinocyte associated protein 3) W93943 transcripts which are detectable by amplicon as depicted in sequence name W93943_seg7-10F1R1 (SEQ ID NO: 94) in normal and cancerous Ovary tissues

Expression of KRTCAP3 (keratinocyte associated protein 3) transcripts detectable by or according to seg7-10F1R1 - W93943_seg7-10F1R1 (SEQ ID NO: 94) amplicon and primers W93943_seg7-10F1 (SEQ ID NO: 92) and W93943_seg7-10R1 (SEQ ID NO: 93) was measured by real time PCR in ovary panel and normal panel. The samples used are detailed in Tables 3 and 4 accordingly, in Example 1.

### Ovary panel -

For each RT sample, the expression of the above amplicon was normalized to the normalization factor calculated from the expression of several house keeping genes as described in example 1. The normalized quantity of each RT sample was then divided by the median of the quantities of the normal samples (sample numbers 52-78, Table 4 above), to obtain a value of fold up-regulation for each sample relative to median of the normal samples.

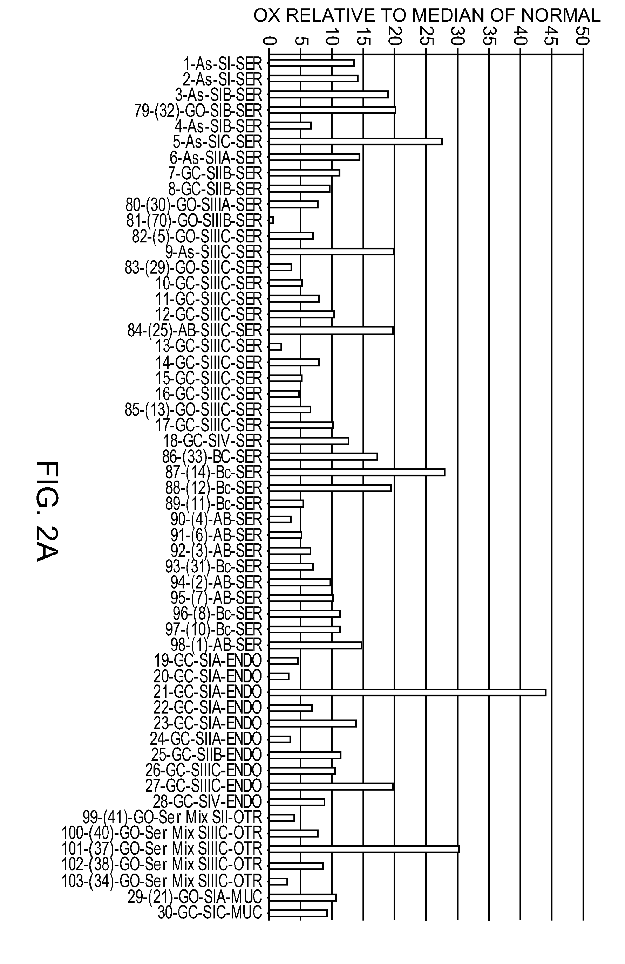

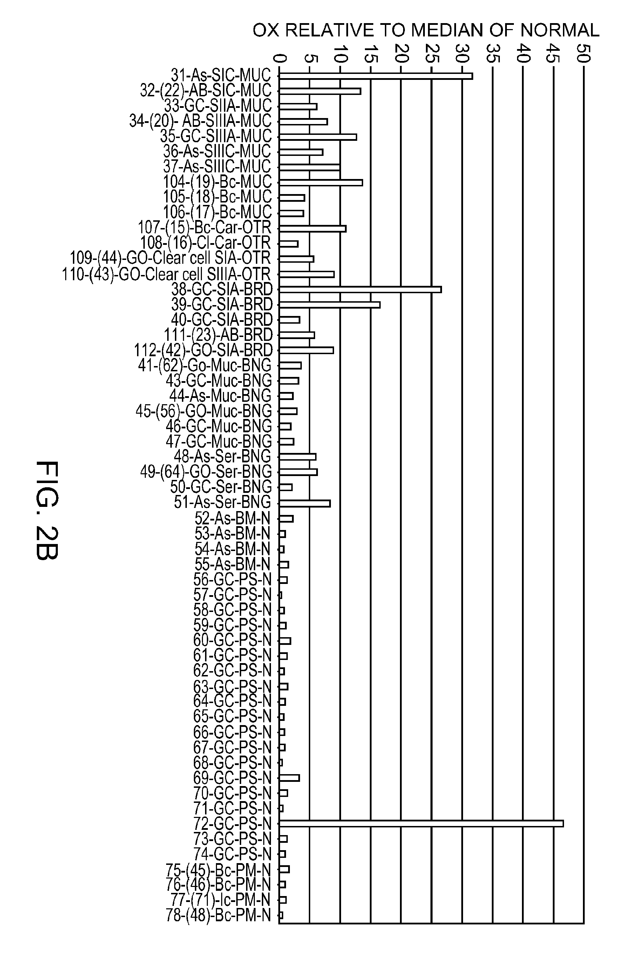

Figure 2 is a histogram showing over expression of the above-indicated KRTCAP3 (keratinocyte associated protein 3) transcripts in cancerous Ovary samples relative to the normal samples.

As is evident from Figure 2, the expression of KRTCAP3 transcripts detectable by the above amplicon in serous carcinoma, mucinous carcinoma, endometroid and adenocarcinoma samples was significantly higher than in the non-cancerous samples (sample numbers 52-78, Table 4 above). Notably an over-expression of at least 5 fold was found in 33 out of 38 serous carcinoma samples, in 10 out of 12 mucinous carcinoma samples, in 7 out of 10 endometroid samples and 56 out of 69 adenocarcinoma samples.

Statistical analysis was applied to verify the significance of these results, as described below.

The P value for the difference in the expression levels of KRTCAP3 transcripts detectable by the above amplicon in ovary serous carcinoma samples, ovary mucinous carcinoma samples, ovary endometroid samples and ovary adenocarcinoma samples versus the normal tissue samples was determined by T test as 6.32e-005, 8.72e-003, 1.04e-002 and 2.33e-005, respectively.

Threshold of 5 fold over expression was found to differentiate between serous carcinoma, mucinous carcinoma, endometriod and adenocarcinoma and normal samples with P value of 4.01e-012, 1.07e-006, 8.51e-005 and 8.77e-013, respectively as checked by exact Fisher test.

The above values demonstrate statistical significance of the results.

### Normal panel -

For each RT sample, the expression of the above amplicon was normalized to the normalization factor calculated from the expression of several house keeping genes as described in example 1. The normalized quantity of each RT sample was then divided by the median of the quantities of the ovary samples (sample numbers 31-34, Table 3 above), to obtain a value of relative expression of each sample relative to median of the ovary samples.

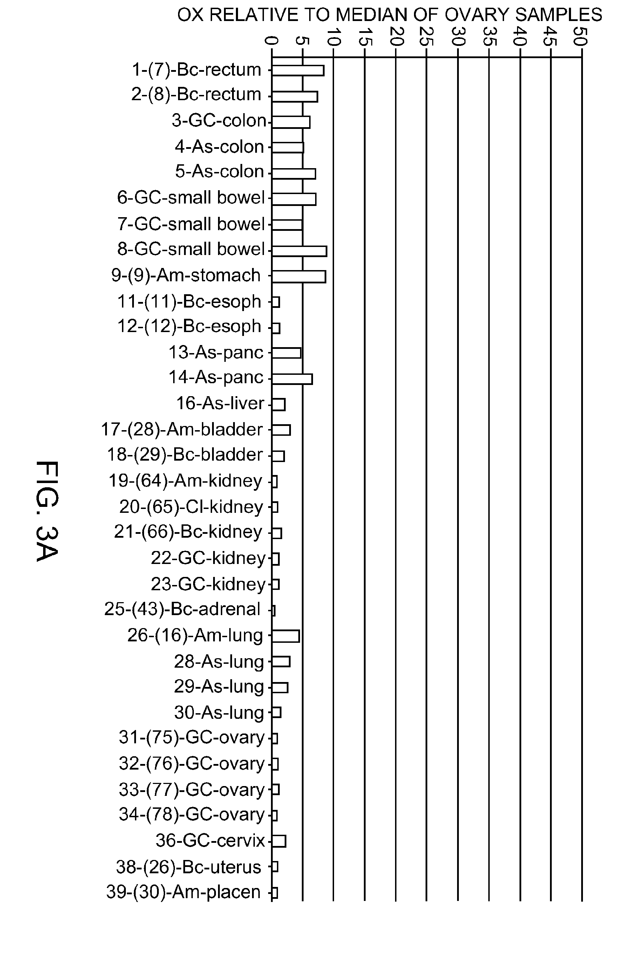

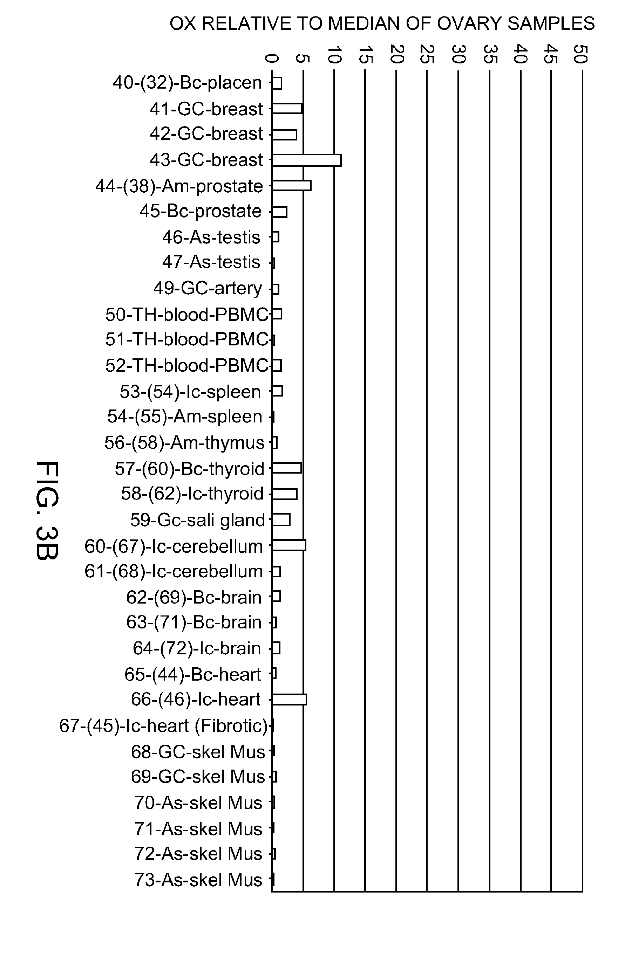

Figure 3 is a histogram showing over expression of the KRTCAP3 (keratinocyte associated protein 3) W93943 transcripts which are detectable by amplicon as depicted in sequence name W93943_seg7-10F1R1 (SEQ ID NO: 94) in different normal tissues.

Primer pairs are also optionally and preferably encompassed within the present invention; for example, for the above experiment, the following primer pair was used as a non-limiting illustrative example only of a suitable primer pair: W93943_seg7-10F1 forward primer (SEQ ID NO: 92); and W93943_seg7-10R1 reverse primer (SEQ ID NO: 93).

The present invention also preferably encompasses any amplicon obtained through the use of any suitable primer pair; for example, for the above experiment, the following amplicon was obtained as a non-limiting illustrative example only of a suitable amplicon: W93943_seg7-10F1R1 (SEQ ID NO: 94).
**Forward Primer (W93943_seg7-10F1) (SEQ ID NO:92):**
CCCCTTTGACCCCACAAGA
**Reverse Primer (W93943_seg7-10R1) (SEQ ID NO:93):**
CAGCCACACAGCAGTAACCAG

Amplicon (W93943_seg7-10F1R1 (SEQ ID NO: 94)):
CCCCTTTGACCCCACAAGAATCTATGATACAGCCTTGGCTCTCTGGATCC
CTTCTTTGCTCATGTCTGCAGGGGAGGCTGCTCTATCTGGTTACTGCTGT
GTGGCTG

### Expression of Homo sapiens keratinocyte associated protein 3 (KRTCAP3) W93943 transcripts which are detectable by amplicon as depicted in sequence name W93943_seg3j4-6F2R1 (SEQ ID NO:171) in normal and cancerous Ovary tissues

Expression of Homo sapiens keratinocyte associated protein 3 (KRTCAP3) transcripts detectable by or according to W93943_seg3j4-6F2R1 amplicon (SEQ ID NO:171) and primers W93943_seg3j4-6F2 (SEQ ID NO:169) and W93943_seg3j4-6R1 (SEQ ID NO:170) was measured by real time PCR. In parallel the expression of several housekeeping genes - SDHA (GenBank Accession No. NM_004168; amplicon - SDHA-amplicon), HPRT1 (GenBank Accession No. NM_000194; amplicon - HPRT1-amplicon) and G6PD (GenBank Accession No. NM_000402; amplicon - G6PD-amplicon) was measured similarly. For each RT sample, the expression of the above amplicon was normalized to the normalization factor calculated from the expression of these house keeping genes as described in normalization method 2 in the "materials and methods" section. The normalized quantity of each RT sample was then divided by the median of the quantities of the normal samples (sample numbers 53, 60, 61, 63, 64, 65, 66, 67, 68, 71, 72, 73, 74, 76 and 77, Table 4 above), to obtain a value of fold up-regulation for each sample relative to median of the normal samples.

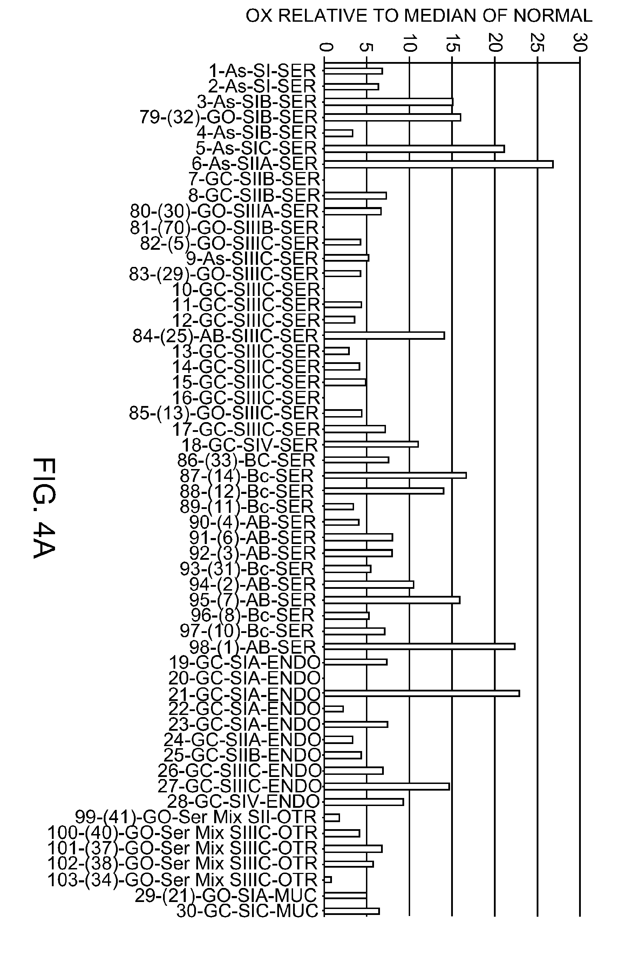

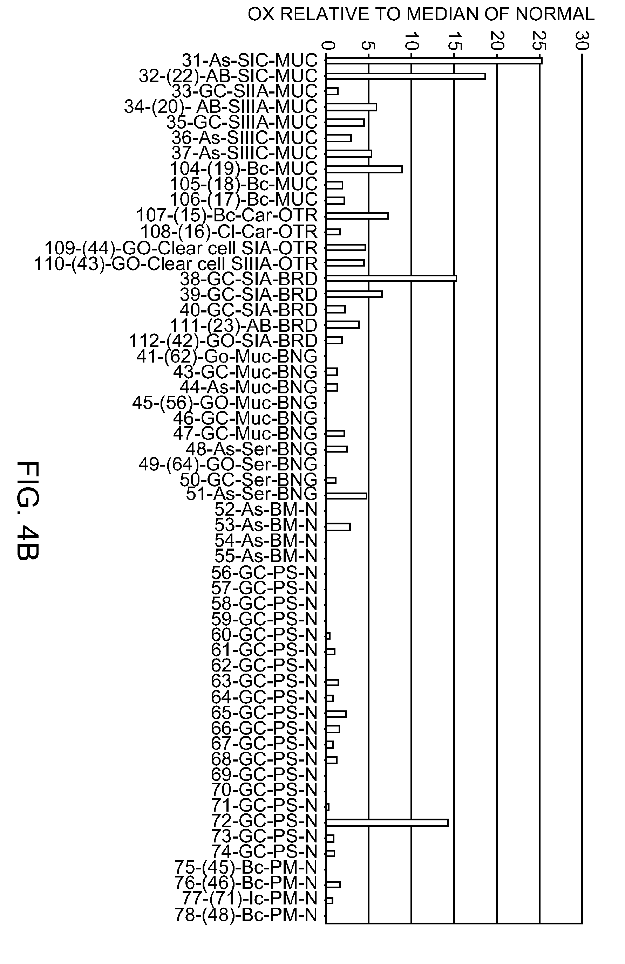

Figure 4 is a histogram showing over expression of the above-indicated Homo sapiens keratinocyte associated protein 3 (KRTCAP3) transcripts in cancerous Ovary samples relative to the normal samples.

As is evident from Figure 4, the expression of Homo sapiens keratinocyte associated protein 3 (KRTCAP3) transcripts detectable by the above amplicon in serous carcinoma, mucinous carcinoma and adenocarcinoma samples was significantly higher than in the non-cancerous samples (sample numbers 53, 60, 61, 63, 64, 65, 66, 67, 68, 71, 72, 73, 74, 76 and 77, Table 4 above). Notably an over-expression of at least 5 fold was found in 25 out of 39 serous carcinoma samples, in 6 out of 12 mucinous carcinoma samples and in 6 out of 9 endometroid carcinoma samples.

Statistical analysis was applied to verify the significance of these results, as described below.

The P value for the difference in the expression levels of Homo sapiens keratinocyte associated protein 3 (KRTCAP3) transcripts detectable by the above amplicon in Ovary serous carcinoma samples versus the normal tissue samples was determined by T test as 8.93e-006. The P value for the difference in the expression levels of Homo sapiens keratinocyte associated protein 3 (KRTCAP3) transcripts detectable by the above amplicon in Ovary mucinous carcinoma samples versus the normal tissue samples was determined by T test as 1.76e-002. The P value for the difference in the expression levels of Homo sapiens keratinocyte associated protein 3 (KRTCAP3) transcripts detectable by the above amplicon in Ovary endometroid carcinoma samples versus the normal tissue samples was determined by T test as 7.94e-003. The P value for the difference in the expression levels of Homo sapiens keratinocyte associated protein 3 (KRTCAP3) transcripts detectable by the above amplicon in Ovary adenocarcinoma samples versus the normal tissue samples was determined by T test as 5.75e-006.

Threshold of 5 fold over expression was found to differentiate between serous carcinoma and normal samples with P value of 1.25e-004 as checked by exact Fisher test. Threshold of 5 fold over expression was found to differentiate between mucinous carcinoma and normal samples with P value of 1.64e-002 as checked by exact Fisher test. Threshold of 5 fold over expression was found to differentiate between endometroid carcinoma and normal samples with P value of 3.71e-003 as checked by exact Fisher test. Threshold of 5 fold over expression was found to differentiate between adenocarcinoma and normal samples with P value of 1.75e-004 as checked by exact Fisher test.

The above values demonstrate statistical significance of the results.

Primer pairs are also optionally and preferably encompassed within the present invention; for example, for the above experiment, the following primer pair was used as a non-limiting illustrative example only of a suitable primer pair: W93943_seg3j4-6F2 forward primer (SEQ ID NO:169); and W93943_seg3j4-6R1 reverse primer (SEQ ID NO:170).

The present invention also preferably encompasses any amplicon obtained through the use of any suitable primer pair; for example, for the above experiment, the following amplicon was obtained as a non-limiting illustrative example only of a suitable amplicon: W93943_seg3j4-6F2R1 (SEQ ID NO:171).
**Forward Primer (W93943_seg3j4-6F2) (SEQ ID NO:169): AGAGCCCAGCAGGCCAAAG**
**Reverse Primer (W93943_seg3j4-6R1) (SEQ ID NO:170): AGCAGGACCCAGTGCAGTG**

Amplicon (W93943_seg3j4-6F2R1) (SEQ ID NO:171):
AGAGCCCAGCAGGCCAAAGGCTTTGTGTCTTCCACAGAGCGTTTCCGTGGGACTTGTGGCC
CTCCTGGCGTCCAGGAACCTTCTTCGCCCTCCACTGCACTGGGTCCTGCT

### Expression of Homo sapiens keratinocyte associated protein 3 (KRTCAP3) W93943 transcripts which are detectable by amplicon as depicted in sequence name W93943_seg3j4-6F2R1 (SEQ ID NO:171) in different normal tissues

Expression of Homo sapiens keratinocyte associated protein 3 (KRTCAP3) transcripts detectable by or according to W93943_seg3j4-6F2R1 amplicon (SEQ ID NO:171) and primers W93943_seg3j4-6F2 (SEQ ID NO:169) and W93943_seg3j4-6R1 (SEQ ID NO:170) was measured by real time PCR. In parallel the expression of several housekeeping genes - SDHA (GenBank Accession No. NM_004168; amplicon - SDHA-amplicon), Ubiquitin (GenBank Accession No. BC000449; amplicon - Ubiquitin-amplicon) and TATA box (GenBank Accession No. NM_003194; TATA amplicon) was measured similarly. For each RT sample, the expression of the above amplicon was normalized to the normalization factor calculated from the expression of these house keeping genes as described in the "materials and methods" section. The normalized quantity of each RT sample was then divided by the median of the quantities of the ovary samples (sample numbers 31, 32, 33 and 34, Table 3 above), to obtain a value of relative expression of each sample relative to median of the ovary samples.
**Forward Primer (W93943_seg3j4-6F2): AGAGCCCAGCAGGCCAAAG**
**Reverse Primer (W93943_seg3j4-6R1): AGCAGGACCCAGTGCAGTG**

Amplicon (W93943_seg3j4-6F2R1):
AGAGCCCAGCAGGCCAAAGGCTTTGTGTCTTCCACAGAGCGTTTCCGTGGGACTTGTGGCC
CTCCTGGCGTCCAGGAACCTTCTTCGCCCTCCACTGCACTGGGTCCTGCT

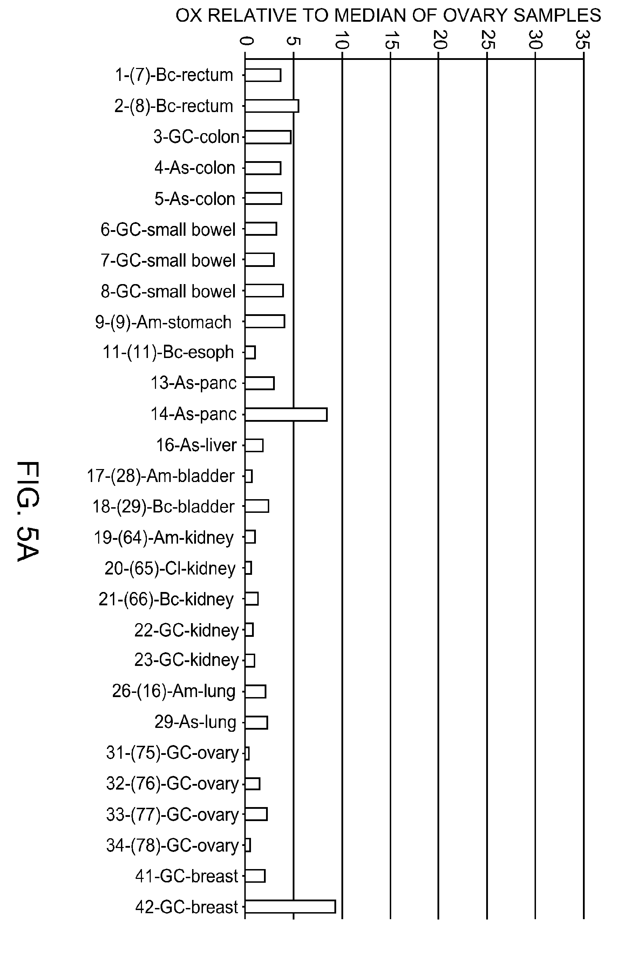

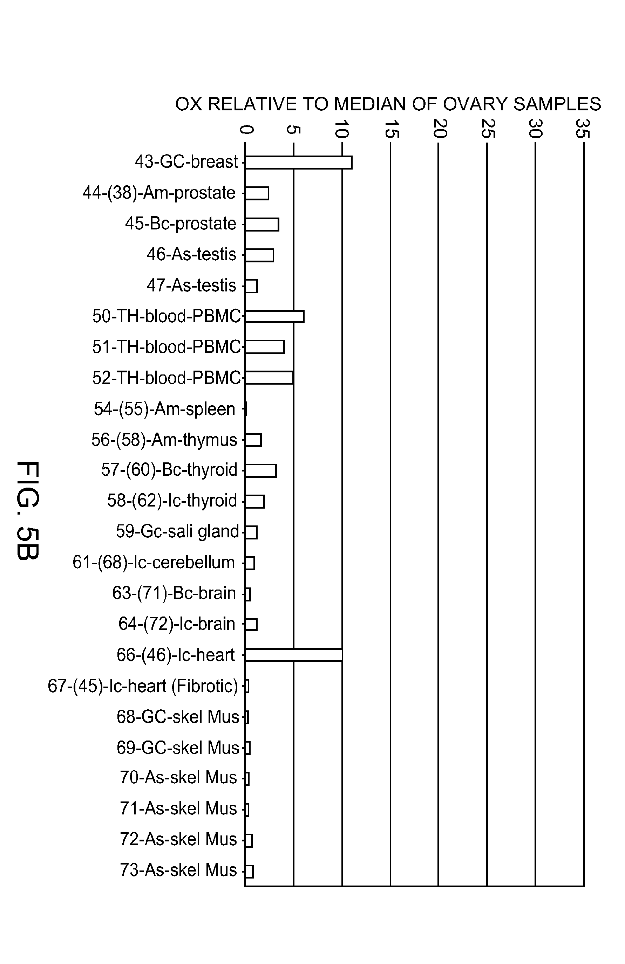

Figure 5 is a histogram showing over expression of the KRTCAP3 transcripts detectable by or according to W93943_seg3j4-6F2R1 amplicon (SEQ ID NO:171) in different normal tissues.

### EXAMPLE 2_3

### CLONING OF KRTCAP3 ORF NON-FUSED and FUSED TO EGFP

Cloning of KRTCAP3 open reading frame (ORF) fused to EGFP was carried out as described below, following that, the non fused KRTCAP3 ORF cloning was carried out.

The cloning of KRTCAP3-EGFP (SEQ ID NO:110) was done in two steps. In the first step an EGFP expression vector was constructed followed by a second step of subcloning KRTCAP3 ORF into the EGFP expression construct. EGFP expression vector was constructed as follows: EGFP-N1 vector (Clontech cataloge number: 6085-1) was digested with NheI and NotI to excise the EGFP gene. The EGFP insert was then ligated into pIRESpuro3 (Clontech cataloge number: 631619), which was previously digested with the same enzymes, in order to obtain the EGFP-pIRESpuro3 vector. Cloning of the KRTCAP3 open reading frame (ORF) was done using the following steps:

1. A reverse transcription reaction was carried out as follows: 10µg of purified lung cancer RNA was mixed with 150ng Random Hexamer primers (Invitrogen, Carlsbad, CA, USA, catalog number: 48190-011) and 500µM dNTPs in a total volume of 156µl. The mixture was incubated for 5 min at 65°C and then quickly chilled on ice. Thereafter, 50µl of 5X SuperscriptII first strand buffer (Invitrogen, catalog number: 18064-014, part number: Y00146), 24µl 0.1M DTT and 400 units RNasin (Promega, Milwaukee, WS, U.S.A., catalog number: N2511) were added, and the mixture was incubated for 10 min at 25°C, followed by further incubation at 42°C for 2 min. Then, 10µl (2000 units) of SuperscriptII (Invitrogen, catalog number: 18064-014) was added and the reaction (final volume of 250µl) was incubated for 50 min at 42°C and then inactivated at 70°C for 15min. The resulting cDNA was diluted 1:20 in TE buffer (10mM Tris, 1 mM EDTA pH 8).

PCR details concerning the subcloning of KRTCAP3 ORF are given in Table 16. PCR #1 was designed to yield KRTCAP3 ORF DNA (SEQ ID NO: 112) which then was subcloned upstream to the EGFP in the EGFP pIRESpuro3 described above, while PCR #2 was designed to yield KRTCAP3 ORF DNA which was subcloned downstream to the EGFP pIRESpuro from above.

2. PCR was done using Platinum PFX™ (Invitrogen., Carlsbad, CA, USA, catalog number: 1178-021) under the following conditions: 5µl Platinum PFX 10x buffer; 5□µl - cDNA from the above; 2µl - 10 mM dNTPs (2.5mM of each nucleotide); 0.5µl - Platinum PFX enzyme; 37□µl - H2O; and 1.5µl - of each primer (10µM) in a total reaction volume of 50µl; with a reaction program of 5 minutes in 95°C; 35 cycles of: 30 seconds at 94°C, 30 seconds at 55°C, 50 seconds at 68°C; then 10 minutes at 68°C. Primers which were used include gene specific sequences corresponding to the desired coordinates of the protein, restriction enzyme sites and Kozak sequence, as listed in Table 16, below. Bold letters in Table 16 represent the specific gene sequence while the restriction site extensions utilized for cloning purposes are in Italic and kozak sequences are underlined.

5µl of PCR product was loaded onto a 1% agarose gel stained with ethidium bromide, electrophoresed in 1xTBE solution at 100V, and visualized with UV light. After verification of expected band size, remaining PCR product was processed for DNA purification using Qiaquick PCR purification kit (Qiagen™, Valencia, CA, U.S.A., catalog number 28106). The extracted PCR products were digested with the appropriate restriction enzymes (New England Biolabs, Beverly, MA, U.S.A.), as listed in Table 16. After digestion, DNAs were loaded onto a 1 % agarose gel as described above. The expected band size was excised and extracted from the gel using QiaQuick™ Gel Extraction kit (Qiagen, catalog number: 28707).

The digested ORF DNAs were ligated to EGFP_pIRESpuro3 vector using the LigaFastTM Rapid DNA Ligation System (Promega, catalog number: M8221.). The resulting DNAs were transformed into competent E.Coli bacteria DH5α (RBC Bioscience, Taipei, Taiwan, catalog number: RH816) according to manufacturer's instructions, then plated on LB-ampicillin agar plates for selection of recombinant plasmids, and incubated overnight at 37°C.

The following day, a number of colonies from each transformation that grew on the selective plates were taken for further analysis by streak-plating on another selective plate and by PCR using GoTaq ReadyMix (Promega, catalog number: M7122). Screening positive clones was performed by PCR using pIRESpuro3 vector specific primer and gene specific primer (data not shown). After completion of all PCR cycles, half of the reaction was analyzed using 1% agarose gel as described above. After verification of expected band size, 2 positive colonies from each ligation reactions were grown in 5 ml Terrific Broth supplemented with 100µg/ml ampicillin, with shaking overnight at 37°C. Plasmid DNA was isolated from bacterial cultures using Qiaprep™ Spin Miniprep Kit (Qiagen, catalog number: 27106). Accurate cloning was verified by sequencing the inserts (Weizmann Institute, Rehovot, Israel). Upon verification of an error-free colony (i.e. no mutations within the ORF), recombinant plasmids were processed for further analyses.

The two KRTCAP3-EGFP constructs from above were used for subcloning KRTCAP3 pIRESpuro3 construct. Subcloning was done as follows: KRTCAP3-EGFP pIRESpuro3 was double digested with BlpI and NheI restriction enzymes (New England Biolabs, Beverly, MA, U.S.A.) and a 220 base pair fragment, corresponding to the 5' end of KRTCAP3 was excised. Following that, EGFP-KRTCAP3 pIRESpuro3 was also double digested with the same restriction enzymes (New England Biolabs, Beverly, MA, U.S.A.) and a 5629 base pair fragment, corresponding to the 3' end of KRTCAP3 and pIRESpuro sequences was excised. The two fragments were ligated and transformed into E.coli as described above. The resulting construct was named KRTCAP3 pIRESpuro3.

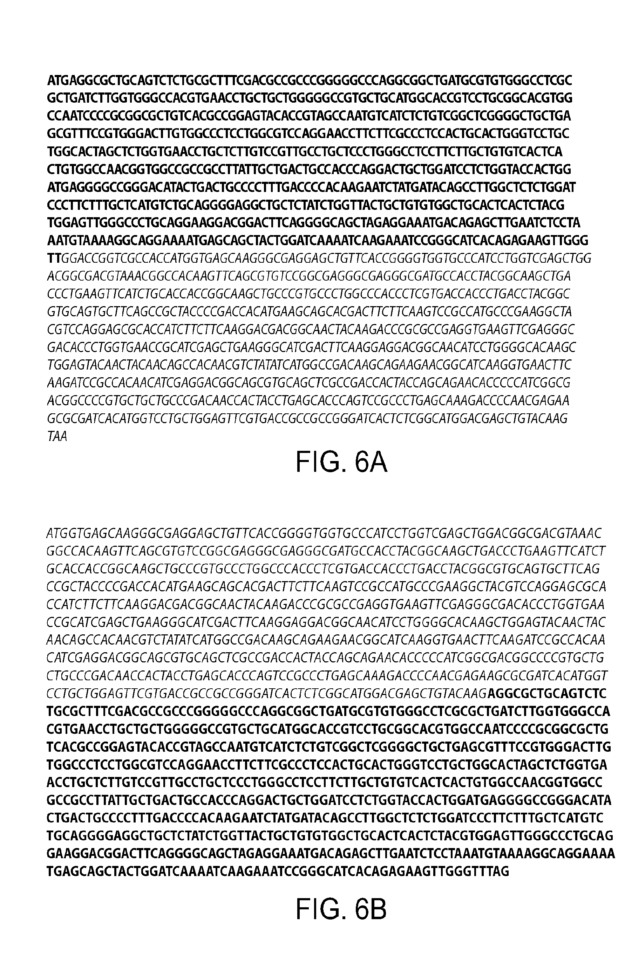

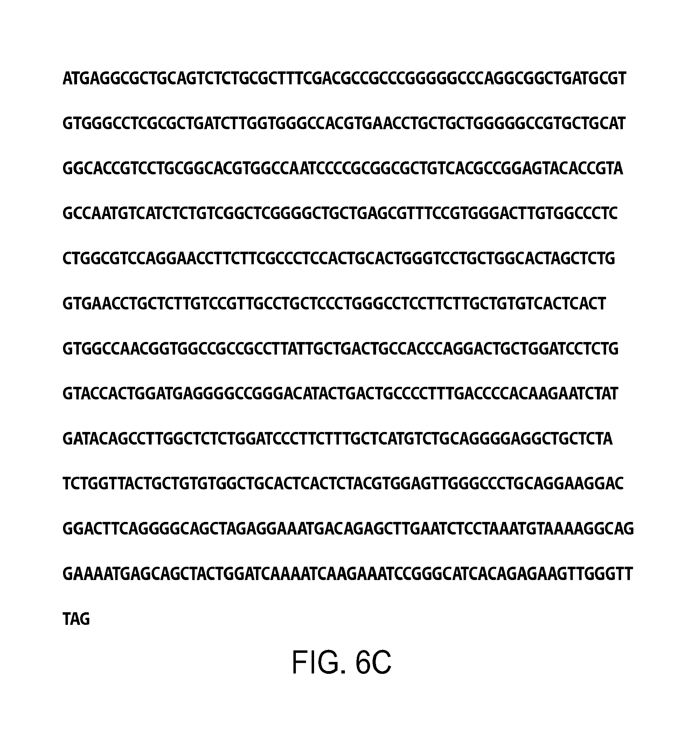

The DNA sequences of the resulting KRTCAP3-EGFP; EGFP-KRTCAP3 and KRTCAP3 are shown in Figures 6A-C; gene specific sequence corresponding to KRTCAP3 ORF sequence is marked in bold faced, EGFP sequence is in italics, and intermediate linker regions are unbold. Figure 6A represents the DNA sequence of KRTCAP3_EGFP (SEQ ID NO:110); Figure 6B represents the DNA sequence of EGFP_KRTCAP3 (SEQ ID NO:111); Figure 6C represents the DNA sequence of KRTCAP3 (SEQ ID NO: 112).

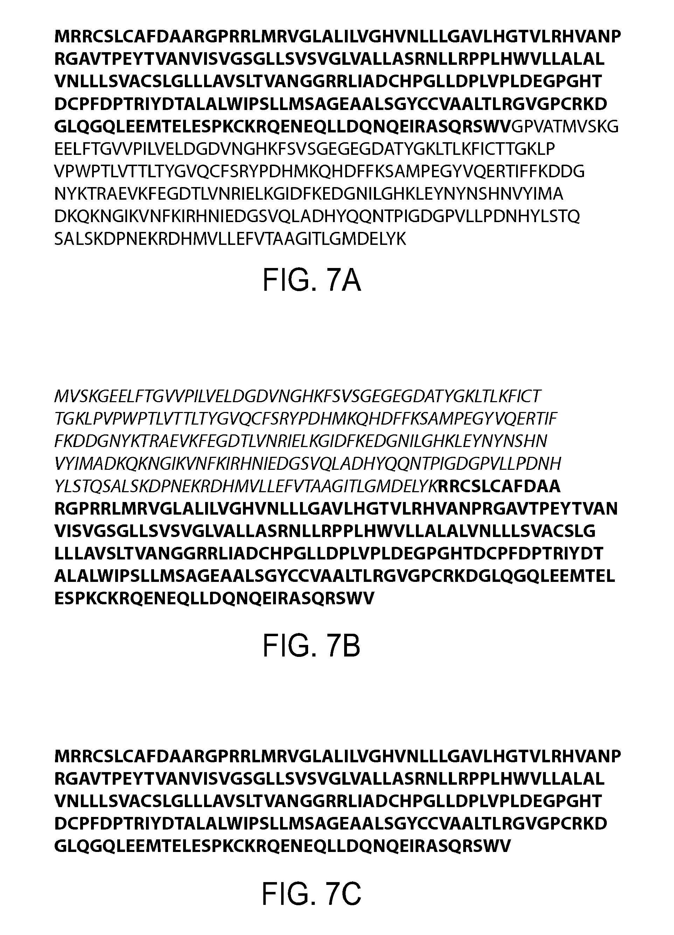

The amino acid sequences of KRTCAP3-EGFP; EGFP-KRTCAP3 and KRTCAP3 are shown in Figures 7A-C; amino acid sequence corresponding to KRTCAP3 ORF is marked in bold faced, EGFP sequence is in italics, and intermediate linker regions are unbold. Figure 7A represents the amino acid sequence of KRTCAP3_EGFP protein (SEQ ID NO: 113); Figure 7B represents the amino acid sequence of EGFP_KRTCAP3 protein (SEQ ID NO:114); Figure 7C represents the amino acid sequence of KRTCAP3 protein (SEQ ID NO:7).

**Table 20: KRT fused to EGFP cloning details**

| Construct name | PC R# | DNA template | Primer ID | Primer sequence | Primer orientation | Restriction site |
|---|---|---|---|---|---|---|
| KRTCAP3_EGFP_pIRESpuro3 | 1 | lung cancer cDNA | 100-857 (SEQ ID NO:163) | CTAGCTAGCCAC CATGAGGCGCT GCAGTCTCTG | For | NheI |
| | | | 100-858 (SEQ ID NO:164) | CGCGACCGGTCC AACCCAACTTC TCTGTGATG | Rev | AgeI |
| EGFP_KRTCAP3 | 2 | lung cDNA | 100-859 (SEQ ID | CGATTGTACAAG AGGCGCTGCAG | For | BsrGI |
| _pIRESpuro3 | | | NO:165) | TCTCTGCGCTT | | |
| | | | 100-860 (SEQ ID NO:166) | GCGCGCGGCCG CCTAAACCCAA CTTCTCTGT | Rev | NotI |

### EXAMPLE 2_4 DETERMINING CELL LOCALIZATION OF KRTCAP3

KRTCAP3 protein was predicted to be a transmembrane protein with four transmembrane domains. In order to verify KRTCAP3 cellular localization, KRTCAP3 was cloned as EGFP (Enhanced Green Fluorescent Protein) fusion proteins, as described above. Protein localization was observed upon transient transfection (Chen et al., Molecular Vision 2002; 8; 372-388) using confocal microscopy. The cells were observed for the presence of fluorescent products 48 hours following transfection.

The EGFP-KRTCAP3 pIRESpuro3 (SEQ ID NO:111) and KRTCAP3-EGFP pIRESpuro3 (SEQ ID NO:110) constructs were subsequently transiently transfected into HEK-293T cells as follows:

HEK-293T (ATCC, CRL-11268) cells were plated on sterile glass coverslips, 13mm diameter (Marienfeld, catalog number: 01 115 30), which were placed in a 6 well plate, using 2ml pre-warmed DMEM [Dulbecco's modified Eagle's Media, Biological Industries (Beit Ha'Emek, Israel), catalog number: 01-055-1A] + 10% FBS [Fetal Bovine Serum, Biological Industries (Beit Ha'Emek, Israel), catalog number: 04-001-1A] + 4mM L-Glutamine [Biological Industries (Beit Ha'Emek, Israel), catalog number: 03-020-1A]. 500,000 cells per well were transfected with 2µg of DNA construct using 6µl FuGENE 6 reagent (Roche, catalog number: 11-814-443-001) diluted into 94ul DMEM. The mixture was incubated at room temperature for 15 minutes. The complex mixture was added dropwise to the cells and swirled. Cells were placed in incubator maintained at 37°C with 5% CO₂ content.

48 hours post transient transfection, cells on coverslips were further processed for immunostaining and analysis by confocal microscopy. The cover slips were washed in phosphate buffered saline (PBS), then fixed for 15 minutes with a solution of 3.7% paraformaldehyde (PFA) (Sigma, catalog number: P-6148)/3% glucose (Sigma, catalog number: G5767) (diluted in PBS). Quenching of PFA was done by a 5 minute incubation in 3mM glycine (Sigma, catalog number: G7126) (diluted in PBS). After two 5-minute washes in PBS, cells were permeabilized with 0.1% triton-X100 (diluted in PBS) for 5 minutes. After two 5-minute washes in PBS, blocking of non-specific regions was done with 5% bovine serum albumin (BSA) (Sigma, catalog number: A4503) (diluted in PBS) for 20 minutes. The coverslips were then incubated, in a humid chamber for 1 hour, with rabbit anti-GFP antibody (MBL International Corporation, catalog number: 598), diluted 1:500 in 5% BSA in PBS, followed by three 5-minute washes in PBS. The coverslips were then incubated, in a humid chamber for 1 hour, with secondary antibody: donkey anti-rabbit conjugated to Cy-3 flurophore (Jackson ImmunoResearch, catalog number: 711-165-152), diluted 1:200 in 3% BSA in PBS. After three 5-minute washes in PBS, the fixed coverslips were mounted on slides with Gel Mount Aqueous medium (Sigma, catalog number: G0918) and cells were observed for the presence of fluorescent product using confocal microscopy. The results are presented in Figure 8.

KRTCAP3 plasma membrane localization was demonstrated using both EGFP fused constructs (EGFP-KRTCAP3 pIRESpuro3 and KRTCAP3-EGFP pIRESpuro3). Cell localization was observed by either detecting EGFP fusion protein fluorescence (Figure 8A) or by immunostaining using anti GFP (Figure 8B). Data is shown for only one construct (EGFP-KRTCAP3 pIRESpuro3)

Figure 8A demonstrates by green fluorescence of EGFP that the EGFP_KRTCAP3_P2 (SEQ ID NO: 114) fused protein localizes to the cell membrane upon expression in HEK 293T cells. The image was obtained using the 40x objective of the confocal microscope.

Figure 8B demonstrates by red fluorescence of anti-GFP antibody conjugated to Cy3 flurophore that the EGFP_KRTCAP3_P2 (SEQ ID NO: 114) fused protein localizes to the cell membrane upon expression in HEK 293T cells. The image was obtained using the 40x objective of the confocal microscope.

### EXAMPLE 2_5

### DETERMINING ORIENTATION OF KRTCAP3 IN THE CELL MEMBRANE

KRTCAP3 protein's orientation within the cell was determined by immunostating of the above EGFP-KRTCAP3 transiently transfected cells. Immunostaining was done as described above, but this time antibody staining was done using non permeabilized transiently transfected HEK 293T cells (as opposed to Example 2_4). Cell permeabilization enables antibody penetration into the cell, therefore, immunostaining of non permeabilized cells, will result in detection of proteins epitopes which are located at the extracellular region of the cell, while internal epitopes will not be detected.

48 hours post transient transfection, the cells on coverslips were further processed for immunostaining and analysis by confocal microscopy. The coverslips were washed 2 times in cold PBS and non-specific regions of coverslips blocked with 5% BSA (Sigma, catalog number: A4503) (diluted in PBS) for 20 minutes on ice. The coverslips were then incubated, in a humid chamber on ice for 1hr, with rabbit anti-GFP antibody (MBL International Corporation, catalog number: 598), diluted 1:500 in 5% BSA in PBS. After 3 5-minute washes in cold PBS, cells on coverslips were fixed for 15 minutes with a solution of 3% paraformaldehyde (PFA) (Sigma, catalog number: P-6148)/3% glucose (Sigma, catalog number: G5767)(diluted in PBS). Quenching of PFA was done by a 5 minute incubation in 3mM glycine (Sigma, catalog number: G7126) (diluted in PBS), followed by two 5 minutes wash in PBS. The coverslips were then incubated, in a humid chamber for 1hr, with secondary antibody: donkey anti-rabbit conjugated to Cy-3 flurophore (Jackson ImmunoResearch, catalog number: 711-165-152), diluted 1:200 in 3% BSA in PBS. After 3 5-minute washes in PBS, the fixed coverslips were mounted on slides with Gel Mount Aqueous medium (Sigma, catalog number: G0918) and cells were observed for the presence of fluorescent product using confocal microscopy.

The results presented in Figure 9 indicate that the amino terminal region of KRTCAP3 is internal to the cell surface. The green fluorescence of EGFP, which is observed in the non permeabilized transiently transfected EGFP_KRTCAP3 HEK 293T cells, demonstrates the localization of the fusion protein to the cell membrane (Figure 9A), however, the absence of red fluorescence of anti-GFP antibody in these cells, indicates that the EGFP_KRTCAP3 fused protein is positioned in the plasma membrane with its amino terminal facing the cytosol (Figure 9B). The images were obtained using the 40x objective of the confocal microscope.

### EXAMPLE 2_6

### PRODUCTION OF POLYCLONAL ANTIBODIES SPECIFIC TO KRTCAP3 VARIANT

All polyclonal Abs production procedure, including peptides synthesis, peptides conjugation, animal immunizations, bleeding and antibodies purification were performed at Sigma-Aldrich (Israel).

### Animals

Two pairs of rabbits were injected to prepare antibodies for KRTCAP3 (rabbit numbers 5257 and 5258, 5259 and 5261). All animal care, handling and injections were performed by Sigma (Israel).

### Peptide synthesis

The peptides which were used for rabbit immunization were as follows: EQLLDQNQEIRASQRS-C (KRT223 (SEQ ID NO:115), a sequence taken from the C'terminus correspond to aa 223-238 of the KRTCAP3 protein (KRTCAP3_P2; SEQ ID NO:7), in which Cystein was added to the C' terminus of the peptide for KLH conjugation, and LDEGPGHTDCPFDPTR (KRT143 SEQ ID NO:116), a sequence taken from the ECD loop correspond to aa143-160 of the KRTCAP3 protein (KRTCAP3_P2; SEQ ID NO: 7). 25mg of each peptides were synthesized with 95% purity of which 10mg were conjugated to KLH carrier.

### Immunization

Each pair of rabbits was immunized with the corresponding conjugated peptide as follows: rabbits 5257 and 5258 were immunized with KRT223 peptide (KRT223 SEQ ID NO:115), and rabbits 5259 and 5261 were immunized with KRT143peptide (KRT143 SEQ ID NO:116). Animals were immunized every two weeks. 3 test bleeds of 2-3ml were collected and analyzed by ELISA. 100ml production bleeds from each rabbit were collected.

### Antibody purification

Antibodies were purified from two rabbit's serum: rabbit 5257 (immunized with peptide KRT223) and rabbit 5261 (immunized with peptide KRT143). Affinity purification was performed with the peptide against which the respective antibodies were raised. The purified antibodies were analyzed by ELISA.

### EXAMPLE 2_7

### CHARACTERIZATION OF PURIFIED KRTCAP3 ANTIBODIES BY WESTERN BLOT USING KRTCAP3 TRANSFECTED CELLS LYSATES

In order to verify the specificity of antibodies raised against selected peptides of KRTCAP3, Western blot analysis was done using non purified serum from rabbits 5257; 5258; 5259 and 5261 described above, and KRTCAP3 stable transfectants cell lysates.

Two stably transfected pool were generated, KRTCAP3 pIRESpuro3 and the negative control empty pIRESpuro3. Both constructs were transfected into HEK-293T cells as follows:

HEK-293T (ATCC, CRL-11268) cells were plated in a sterile 6 well plate suitable for tissue culture, using 2ml pre-warmed of complete media, DMEM [Dulbecco's modified Eagle's Media, Biological Industries (Beit Ha'Emek, Israel), catalog number: 01-055-1A] + 10% FBS [Fetal Bovine Serum, Biological Industries (Beit Ha'Emek, Israel), catalog number: 04-001-1A] + 4mM L-Glutamine [Biological Industries (Beit Ha'Emek, Israel), catalog number: 03-020-1A]. 500,000 cells per well were transfected with 2µg of DNA construct using 6µl FuGENE 6 reagent (Roche, catalog number: 11-814-443-001) diluted into 94ul DMEM. The mixture was incubated at room temperature for 15 minutes. The complex mixture was added dropwise to the cells and swirled. Cells were placed in incubator maintained at 37°C with 5% CO₂ content. 48 hours following transfection, transfected cells were transferred to a 75cm² tissue culture flask containing 15ml of selection media: complete media supplemented with 5µg\ml puromycin (Sigma, catalog number P8833). Cells were placed in incubator, and media was changed every 3-4 days, until clone formation observed.

Upon sufficient quantities of cells passing through selection, 3-5 million cells were harvested. Cells were lysed in 300ul RIPA buffer (50mM Tris HCl pH 8, 150 mM NaCl, 1% NP-40, 0.5% sodium Deoxycholate, 0.1% SDS) supplemented with protease inhibitors (Roche, catalog number: 11873580001), for 1.5hrs at 4°C. Following centrifugation at 4°C for 15 minutes at 20,000xg, the clear supernatants were transferred to clean tubes and 100ul of 4X NuPAGE® LDS sample buffer (Invitrogen, catalog number: NP0007) was added. In addition, 1,4-Dithiothreitol (DTT; a reducing agent) was added to a final concentration of 100mM. The samples were then incubated at 100°C for 3 minutes, followed by a 1 minute spin at 20,000xg. SDS-PAGE (Laemmli, U.K., Nature 1970; 227; 680-685) was performed upon loading of 25ul of sample per lane into a 12% NuPAGE® Bis-Tris gels (Invitrogen, catalog number: NP0341), and gels were run in 1xMOPS SDS running buffer (Invitrogen, catalog number: NP0001), using the XCell *SureLock*™ Mini-Cell (Invitrogen, catalog number: E10001), according to manufacturer's instructions. The separated proteins were transferred to a nitrocellulose membrane (Schleicher & Schuell, catalog number: 401385) using the XCell™ II blotting apparatus (Invitrogen, catalog number E19051), according to manufacturer's instructions.

The membrane containing blotted proteins was processed for antibody detection as follows:

Non-specific regions of the membrane were blocked by incubation in 10% skim-milk diluted in Tris buffered saline (TBS) supplemented with 0.05% Tween-20 (TBST) for 1 hour at room temperature (all subsequent incubations occur for 1 hour at room temperature). Blocking solution was then replaced with primary antibody solution: 3^{rd} bleed (before purification) of rabbit anti-KRT antibodies described above diluted 1:500 in blocking solution. After 3 10-minute washes, secondary antibody was applied: goat anti-rabbit conjugated to horse radish-peroxidase (Jackson ImmunoResearch, catalog number 111-035-144) diluted 1:10,000 in blocking solution. After 3 10-minute washes, ECL substrate (GE-Amersham, catalog number: RPN2209) was applied for 1 minute, followed by exposure to X-ray film (Fuji, catalog number: 100NIF).

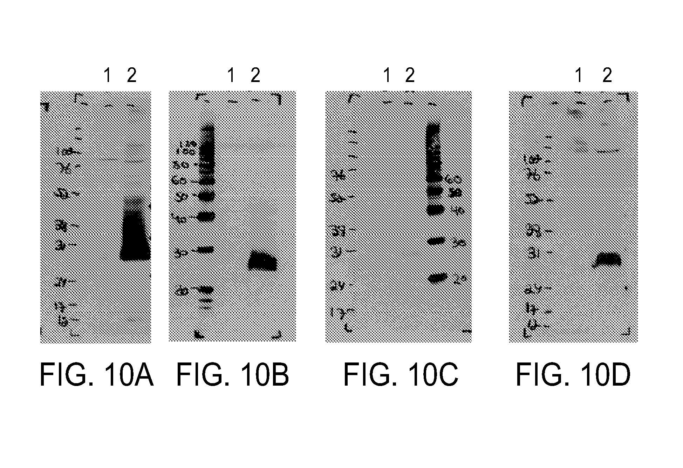

Figure 10A-D demonstrate that all four sera 5257; 5258; 5259 and 5261 recognize KRTCAP3 protein respectively. Specificity is demonstarted by the differential signal obtained for the KRTCAP3 transfected cells which is absent in empty pIRESpuro3 transfected cells.

### EXAMPLE 2_8

### CHARACTERIZATION OF PURIFIED KRTCAP3 ANTIBODIES BY IMMUNOSTAINING OF KRTCAP3 TRANSFECTED CELLS

In order to further characterize the affinity purified antibodies raised against KRTCAP3 polypeptide, antibody-protein interaction was studied using immunostaining of KRTCAP3 stable transfected HEK293T cells.

500,000 cells per well of HEK-293T (ATCC, CRL-11268) stably expressing KRTCAP3 or the empty vector pIRES puro3, described above, were plated on sterile glass coverslips, 13mm diameter (Marienfeld, catalog number: 01 115 30), which were placed in a 6 well plate, using 2ml pre-warmed DMEM [Dulbecco's modified Eagle's Media, Biological Industries (Beit Ha'Emek, Israel), catalog number: 01-055-1A] + 10% FBS [Fetal Bovine Serum, Biological Industries (Beit Ha'Emek, Israel), catalog number: 04-001-1A] + 4mM L-Glutamine [Biological Industries (Beit Ha'Emek, Israel), catalog number: 03-020-1A].

48 hours post plating the cells on coverslips they were further processed for immunostaining and analysis by confocal microscopy. The cover slips were washed in phosphate buffered saline (PBS), then fixed for 15 minutes with a solution of Formalin solution, natural buffered 10% (Sigma, catalog number: HT501128). After 2 5-minute washes in PBS, cells were permeabilized with 0.1% triton-X100 (diluted in PBS) for 5 minutes. After two 5-minute washes in PBS, blocking of non-specific regions was done with 5% bovine serum albumin (BSA) (Sigma, catalog number: A4503) (diluted in PBS) for 20 minutes. The coverslips were then incubated, in a humid chamber for 1 hour, with purified rabbit anti-KRT antibodies described above: anti KRT143 (RB 5261, 0.9mg/ml) was diluted 1:2000 in 5% BSA in PBS and anti KRT223 (RB5257, 1mg/ml) was diluted 1:1000 in 5% BSA. The antibodies were washed 3 times for 5-minutes in PBS. The coverslips were then incubated, in a humid chamber for 1 hour, with secondary antibody: donkey anti-rabbit conjugated to Cy-3 flurophore (Jackson ImmunoResearch, catalog number: 711-165-152), diluted 1:200 in 3% BSA in PBS. After 3 5-minute washes in PBS, the fixed coverslips were mounted on slides with Gel Mount Aqueous medium (Sigma, catalog number: G0918) and cells were observed for the presence of fluorescent product using confocal microscopy.

Specific cell staining was observed using purified KRT143 and KRT223 antibodies on KRTCAP3 transfected cells (Figures 11A and 11C respectively), however, no staining was observed using these antibodies on pIRESpuro3 HEK-293T transfected cells (Figures 11B and 11D respectively). The red fluorescence obtained in Figure 11A and 11C as opposed to the absence of signal in Figure 11B and 11D demonstrates the specificity of KRT143 and KRT223 antibodies to KRTCAP3_P2 (SEQ ID NO: 7).

### EXAMPLE 2_9: IMMUNOHISTOCHEMISTRY ANALYSIS OF KRT223 and KRT143 ANTIBODIES ON TISSUE SAMPLES

### Antibody Titration Protocol and Positive Control Study Results:

Antibody titration experiments were conducted at LifeSpan Biosciences (USA) with rabbit polyclonal antibodies KRT223 and KRT143 to establish concentrations that would result in minimal background and maximal detection of signal. Serial dilutions were performed at 20ug/ml, 10ug/ml, 5ug/ml, and 2.5ug/ml on formalin-fixed, paraffin-embedded tissues supplied by LifeSpan BioSciences (USA) and on HEK-293T (ATCC, CRL-11268) cell lines transiently transfected with KRTCAP3 as positive control or empty vector as negative control. This study demonstrated the highest signal-to-noise ratio at a concentration of 2.5ug/ml for antibody KRT223 and 2.5ug/ml and 1.25ug/ml for antibody KRT143. Antibodies KRT223 and KRT143 were used as the primary antibody, and the principal detection system consisted of a Vector anti-rabbit secondary (BA-1000) and a Vector ABC-AP kit (AK-5000) with a Vector Red substrate kit (SK-5100),which was used to produce a fuchsia-colored deposit. Antigen retrieval was carried out by steam-heat in sodium citrate buffer. Tissues were stained with a positive control antibody (CD31 andvimentin to ensure that the tissue antigens were preserved and accessible for Immunohistochemical analysis. Only tissues that showed positive CD31 and vimentin staining were selected for the study. The negative control consisted of performing the entire immunohistochemistry procedure on adjacent sections in the absence of primary antibody. Slides were imaged with a DVC1310C digital camera coupled to a Nikon microscope. Antibody KRT223 performed well in the positive control cells and showed a specific-appearing distribution of staining in tissues. Staining was membranous and cytoplasmic (data not shown), while antibody KRT143 showed a broader spectrum of positivity.

### Immunohistochemistry analysis of KRT223 on ovarian tissue samples

Antibody KRT223 was examined in samples of normal ovary, as well as in a series of ovarian carcinomas and metastatic carcinoma from gastrointestinal tumors. Staining was carried out as described above, with Antibody KRT223 at a concentration of 2.5ug/ml. In the normal ovary samples, positive staining was identified in benign ovarian surface epithelium (mesothelium), and weaker staining was frequently present in oocytes, granulosa cells, theca cells, and ovarian stroma. The ovarian carcinomas showed weak positive staining in approximately 20 percent of samples, as shown in Figure 12, while more prominent staining, shown in Figure 13, was identified in metastatic carcinoma samples from gastrointestinal tumors, which often metastasize to ovary, and are sometimes referred to as "Krukenberg" tumors.

Figure 12 demonstrates intense immunohistochemical staining of an ovary carcinoma sample obtained from a 52- year old female, using Antibody KRT223. The signal was quantified using a 0-4 scale, and was given the signal intensity 2.

Figure 13 demonstrates prominent immunohistochemical staining of an adenomacarcinoma sample from a metastatic gastrointestinal tumor obtained from a 31-year-old female, using Antibody KRT223. The signal was quantified using a 0-4 scale, and was given the signal intensity 3.

### Immunohistochemistry analysis of KRT143 on common cancer tissue samples

Antibody KRT143was examined at concentrations of 2.5ug/ml and 1.25ug/ml in a series of common cancers, including breast, colon, lung, ovarian, prostatic, and pancreatic carcinomas. The quality of staining obtained in this study was very good, with variable cytoplasmic, membranous, nuclear, and occasional extracellular staining in secretions, serum, and necrobiotic debris (data not shown). Positive staining was identified in the majority of the cancers. The most prominent staining was identified in individual samples of ovarian, lung, and prostate carcinoma, in which focal intense cytoplasmic staining was present in malignant cells. Most of the remaining cancer subtypes showed weaker but uniform nuclear and cytoplasmic staining in malignant cells (data not shown). Additional positive cell types included inflammatory cells, peripheral nerves, and ganglion cells. Vascular smooth muscle and benign prostatic fibromuscular stroma were also occasionally positive (dsata not shown). Further studies and calibrations are needed for the evaluation of antibody KRT143 specificity.

### EXAMPLE 3: FAM26F POLYPEPTIDES AND POLYNUCLEOTIDES, AND USES THEREOF AS A DRUG TARGET FOR PRODUCING DRUGS AND BIOLOGICS

### EXAMPLE 3_1: DESCRIPTION FOR CLUSTER T82906

Cluster T82906 (internal ID 72304721) features 3 transcript(s) of interest, the names for which are given in Table 17. The selected protein variants are given in table 18.

**Table 17 - Transcripts of interest**

| **Transcript Name** |
|---|
| T82906_T0 (SEQ ID NO:125) |
| T82906_T1 (SEQ ID NO:14) |

**Table 18 - Proteins of interest**

| **Protein Name** | **Corresponding Transcript(s)** |
|---|---|
| T82906_P3 (SEQ ID NO:16) | T82906_T0 (SEQ ID NO:125) |
| T82906_P4 (SEQ ID NO:18) | T82906_T1 (SEQ ID NO:14) |

These sequences are variants of the known protein hypothetical protein LOC441168 (SEQ ID NO:15) (SwissProt accession identifier NP_001010919, synonims: FAM26F).

FAM26F (family with sequence similarity 26, member F) is one of the genes identified in the annotation of chromosome 6 (Mungall et al. 2003, Nature 425(6960):805-11). No specific information was published on FAM26F. Only one other member of the FAM26 has been annotated: FAM26C- a Calcium homeostasis modulator protein (Dreses_Werringloer et al. 2008, Cell 133:1149-1161).

FAM26F antigen has been reported in WO2003025138 patent application, which purports that sequence of AI796870 and Hs.54277, corresponding to FAM26F, is differentially expressed in melanoma, kidney cancer and fibrotic diseases. The WO2003025138 patent application does not teach, however, that sequence corresponding to FAM26F is differentially expressed in ovarian cancer, breast cancer, prostate cancer, acute lymphocytic leukemia, chronic lymphocytic leukemia, acute myelogenous leukemia, chronic myelogenous leukemia, multiple myeloma, Hodgkin's lymphoma or Non-Hodgkin's lymphoma and/or immune related conditions. Also, there is no teaching in WO2003025138 application that FAM26F can be used as drug target for treatment of ovarian cancer, breast cancer, prostate cancer, renal cancer, melanoma, acute lymphocytic leukemia, chronic lymphocytic leukemia, acute myelogenous leukemia, chronic myelogenous leukemia, multiple myeloma, Hodgkin's lymphoma or Non-Hodgkin's lymphoma and/or immune related conditions, or for diagnosis thereof. Also, there is no teaching in WO2003025138 application that antibodies specific for FAM26F, its soluble ectodomain, and/or fragments thereof can be used as therapeutics for treatment of ovarian cancer, breast cancer, prostate cancer, renal cancer, melanoma, acute lymphocytic leukemia, chronic lymphocytic leukemia, acute myelogenous leukemia, chronic myelogenous leukemia, multiple myeloma, Hodgkin's lymphoma or Non-Hodgkin's lymphoma and/or immune related conditions, or for diagnosis thereof.

FAM26F antigen has been also reported in the following patent applications: WO9961471, WO2005019258, WO200501692, WO2008079406, which purports that sequence corresponding to FAM26F is useful for diagnosing, treating or preventing selected immune related disorders. However, there is no teaching or suggestion in these applications that FAM26F can be used as drug target for treatment prevention or diagnosing of ovarian cancer, breast cancer, prostate cancer, renal cancer, melanoma, acute lymphocytic leukemia, chronic lymphocytic leukemia, acute myelogenous leukemia, chronic myelogenous leukemia, multiple myeloma, Hodgkin's lymphoma or Non-Hodgkin's lymphoma.

FAM26F antigen has been also reported in the following patent applications:, WO200177292, WO200006719, however, there is no teaching no suggestion in these applications that FAM26F can be used as drug target for treatment prevention or diagnosing of ovarian cancer, breast cancer, prostate cancer, renal cancer, melanoma, acute lymphocytic leukemia, chronic lymphocytic leukemia, acute myelogenous leukemia, chronic myelogenous leukemia, multiple myeloma, Hodgkin's lymphoma or Non-Hodgkin's lymphoma.

By contrast and surprisingly, the present inventors have found that FAM26F antigen and discrete portions thereof may optionally be used as a drug target for therapeutic small molecules, peptides, antibodies, antisense RNAs, siRNAs, ribozymes, and the like. Diagnostic and therapeutic polyclonal and monoclonal antibodies and fragments thereof that bind FAM26F, and portions and variants thereof, may also optionally be produced. According to at least some embodiments of the invention, there is provided a use of antibodies and antibody fragments against FAM26F antigen, its secreted or soluble form or ECD and/or variants, conjugates, or fragments thereof and fragments and variants thereof for treating and diagnosing ovarian cancer, breast cancer, prostate cancer, renal cancer, melanoma, acute lymphocytic leukemia, chronic lymphocytic leukemia, acute myelogenous leukemia, chronic myelogenous leukemia, multiple myeloma, Hodgkin's lymphoma or Non-Hodgkin's lymphoma, wherein this antigen is differentially expressed.

As noted above, cluster T82906 features 3 transcript(s), which were listed in Table 17 above. These transcript(s) encode for protein(s) which are variant(s) of protein hypothetical protein LOC441168 (SEQ ID NO:15). A description of each variant protein according to at least some embodiments of the invention is now provided.

Variant protein T82906_P3 (SEQ ID NO:16) according to at least some embodiments of the invention is encoded by transcript T82906_T0 (SEQ ID NO:125). A description of the relationship of the variant protein according to at least some embodiments of the invention to known proteins is as follows:
Comparison report between T82906_P3 (SEQ ID NO:16) and known protein Q5R3K2_HUMAN (SEQ ID NO:17):
   A. An isolated chimeric polypeptide, comprising a first amino acid sequence being at least 70%, optionally at least 80%, preferably at least 85%, more preferably at least 90% and most preferably at least 95% homologous to a polypeptide having the sequence MFP corresponding to amino acids 1 - 3 of T82906_P3 (SEQ ID NO:16), and a second amino acid sequence being at least 90% homologous to VLGWILIAVVIIILLIFTSVTRCLSPVSFLQLKFWKIYLEQEQQILKSKATEHATELAK ENIKCFFEGSHPKEYNTPSMKEWQQISSLYTFNPKGQYYSMLHKYVNRKEKTHSIR STEGDTVIPVLGFVDSSGINSTPEL corresponding to amino acids 19 - 158 of known protein Q5R3K2_HUMAN (SEQ ID NO:17), which also corresponds to amino acids 4 - 143 of T82906_P3 (SEQ ID NO:16), wherein said first amino acid sequence and second amino acid sequence are contiguous and in a sequential order.

The localization of the variant protein was determined according to results from a number of different software programs and analyses, including analyses from SignalP and other specialized programs. The variant protein is believed to be located as follows with regard to the cell: secreted.

Variant protein T82906_P3 (SEQ ID NO:16) also has the following non-silent SNPs (Single Nucleotide Polymorphisms) as listed in Table 19, (given according to their position(s) on the amino acid sequence, with the alternative amino acid(s) listed).

**Table 19 - Amino acid mutations**

| **SNP position(s) on amino acide sequence** | **Alternatice amino acid(s)** |
|---|---|
| 87 | Q → R |
| 111 | K → T |

The coding portion of transcript T82906_T0 (SEQ ID NO:125) starts at position 165 and ends at position 593. The transcript also has the following SNPs as listed in Table 20 (given according to their position on the nucleotide sequence, with the alternative nucleic acid listed).

**Table 20 - Nucleic acid SNPs**

| **Polymorphism** | **SNP position(s) on nucleotide sequence** |
|---|---|
| A → T | 68 |
| A → C | 68, 496 |
| A → G | 424, 431 |
| T → A | 524 |
| T → G | 524 |
| G → | 612 |
| G → A | 612 |

Variant protein T82906_P4 (SEQ ID NO:18) according to at least some embodiments of the invention is encoded by transcript T82906_T1 (SEQ ID NO:14).

The localization of the FAM26F protein was determined according to results from a number of different software programs and analyses, including analyses from SignalP and other specialized programs. The FAM26F protein is believed to be located as follows with regard to the cell: membrane.

Variant protein T82906_P4 (SEQ ID NO:18) also has the following non-silent SNPs (Single Nucleotide Polymorphisms) as listed in Table 21, (given according to their position(s) on the amino acid sequence, with the alternative amino acid(s) listed).

**Table 21 - Amino acid mutations**

| **SNP position(s) on amino acid sequence** | **Alternative amino acid(s)** |
|---|---|
| 80 | G → R |
| 99 | C → * |
| 99 | C → W |
| 100 | A → T |
| 110 | P → L |
| 115 | A → G |
| 259 | Q → R |
| 283 | K → T |

The coding portion of transcript T82906_T1 (SEQ ID NO:14) starts at position 232 and ends at position 1176. The transcript also has the following SNPs as listed in Table 22 (given according to their position on the nucleotide sequence, with the alternative nucleic acid listed).

**Table 22 - Nucleic acid SNPs**

| **Polymorphism** | **SNP position(s) on nucleotide sequence** |
|---|---|
| A → T | 68 |
| A → C | 68, 1079 |
| C → A | 468, 528 |
| C → G | 468, 528, 575 |
| G → A | 469, 529, 1195 |
| C → T | 560, 597 |
| A → G | 1007, 1014 |
| T → A | 1107 |
| T → G | 1107 |
| G → | 1195 |

### EXAMPLE 3_2: ANALYSIS OF THE EXPRESSION OF FAM26F TRANSCRIPTS

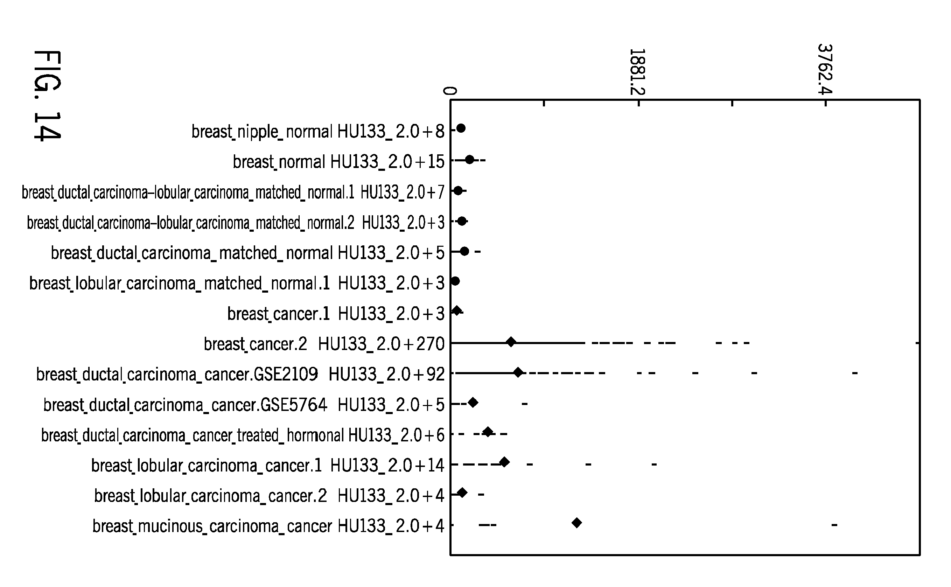

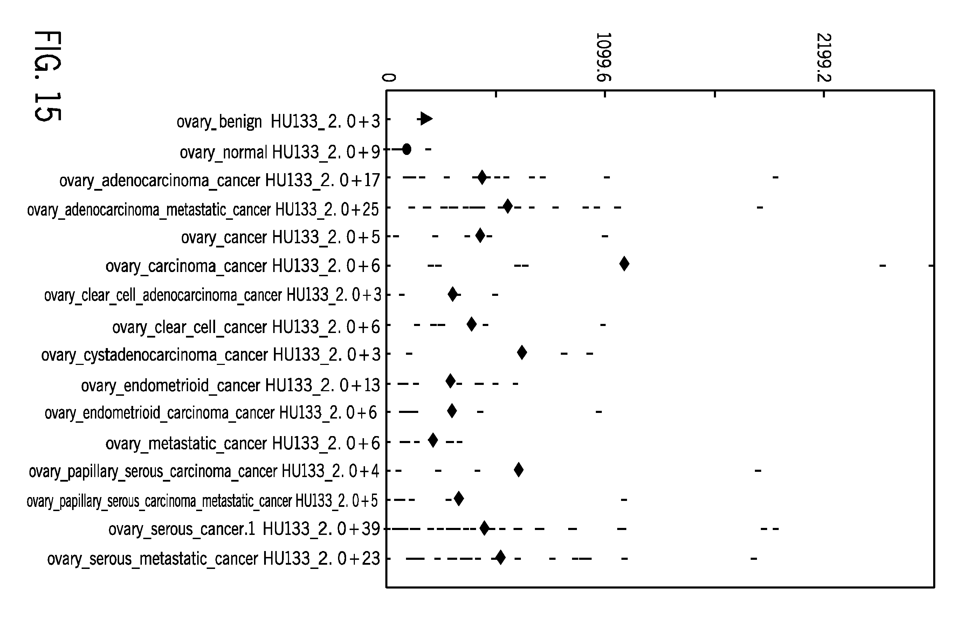

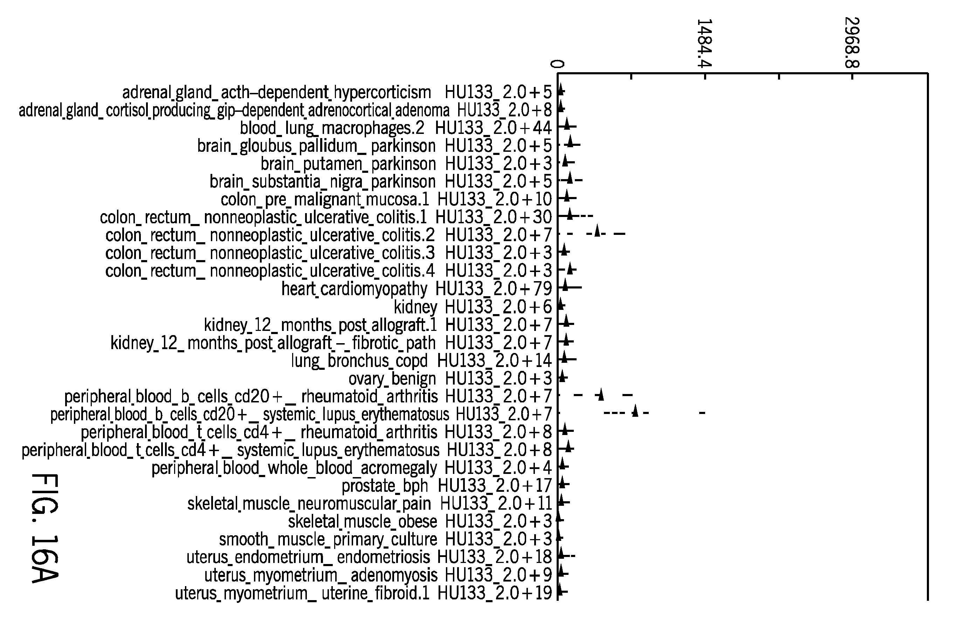

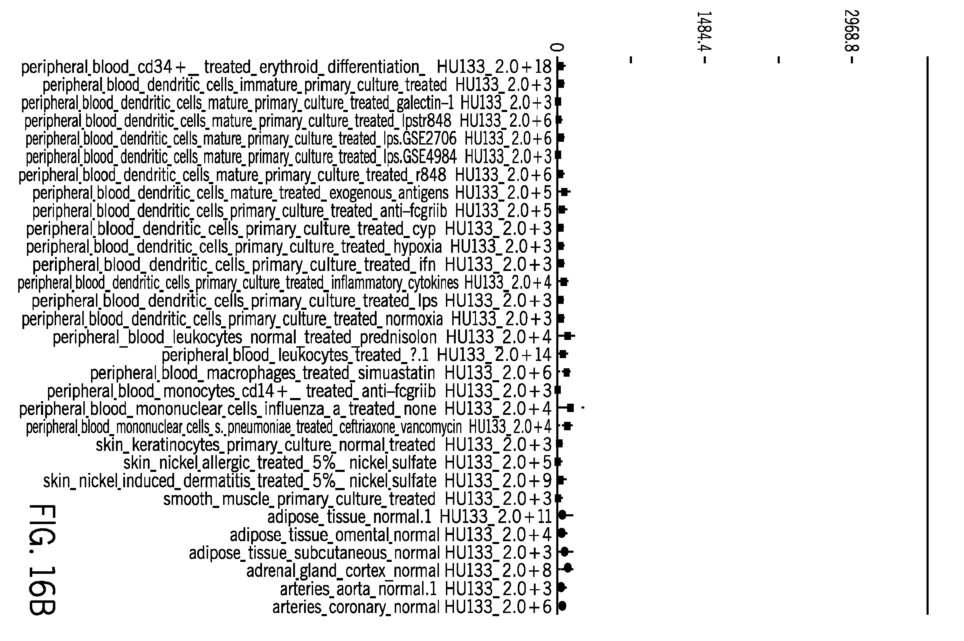

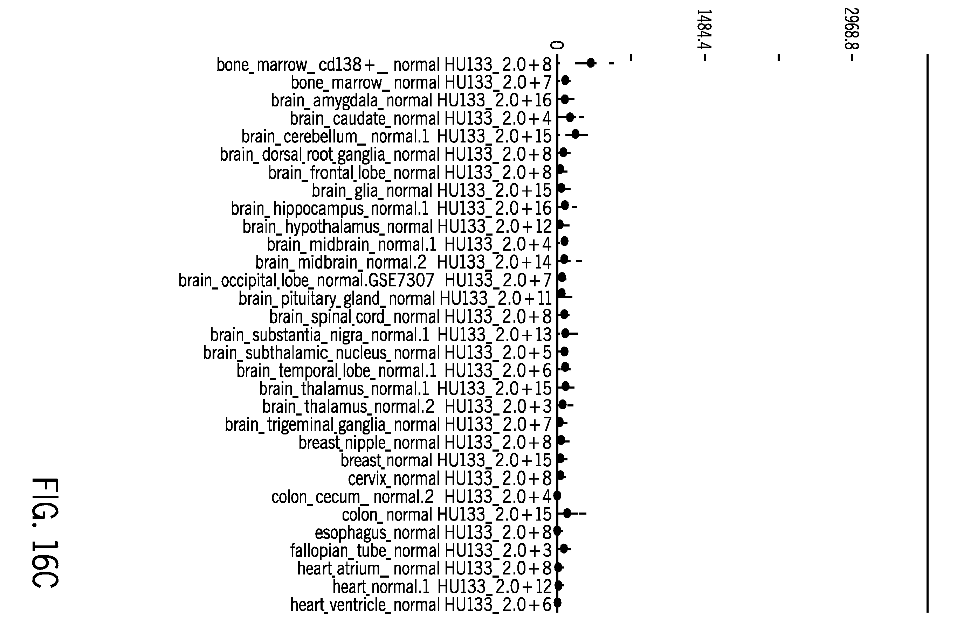

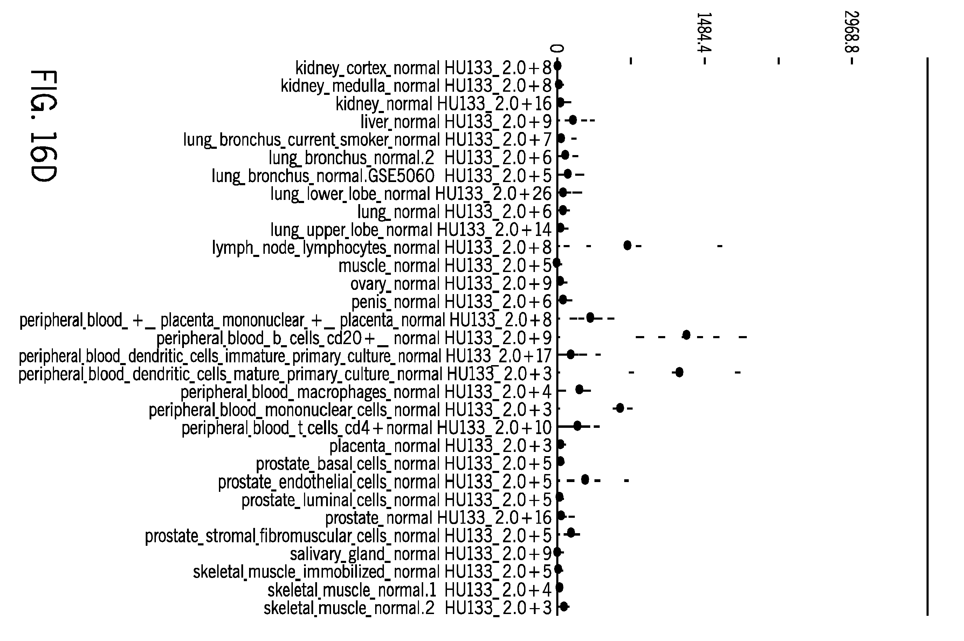

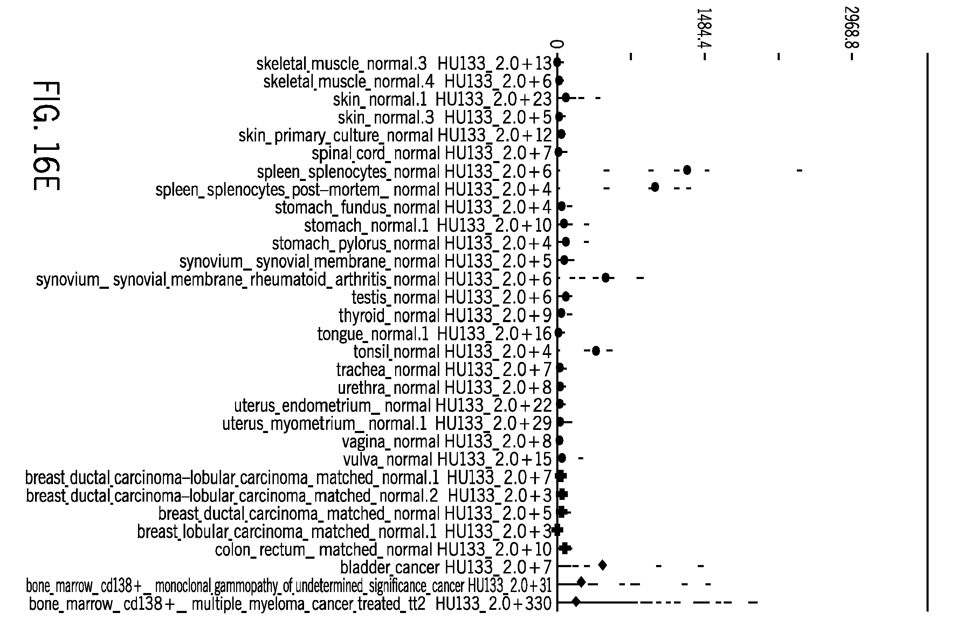

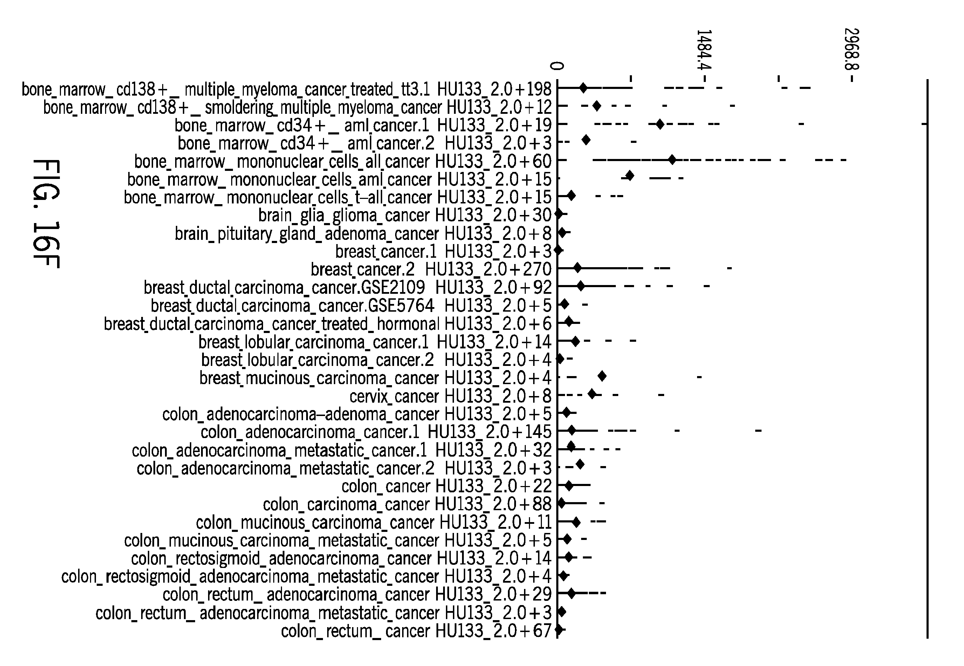

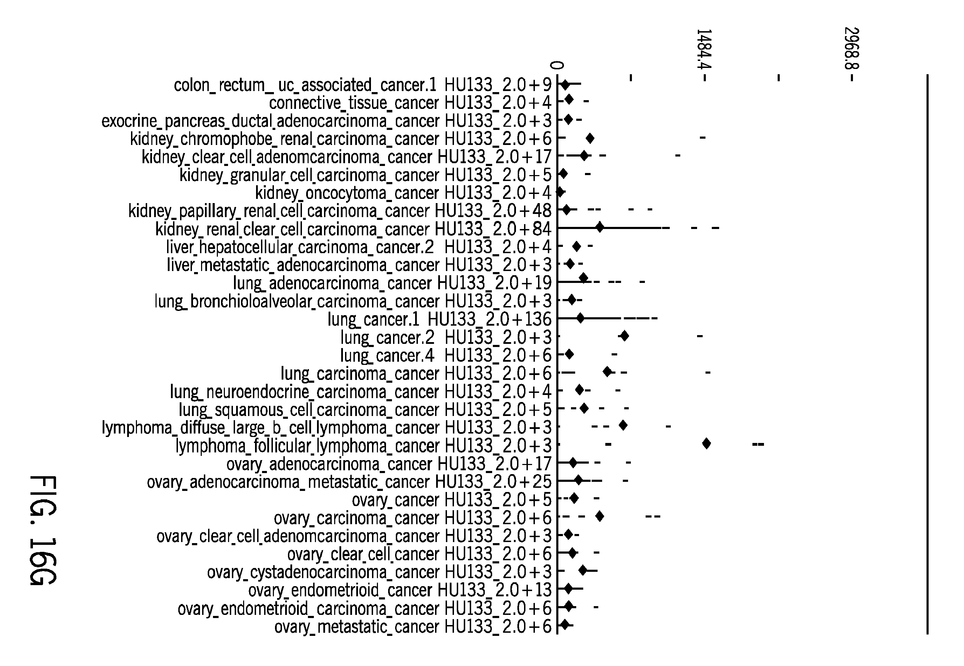

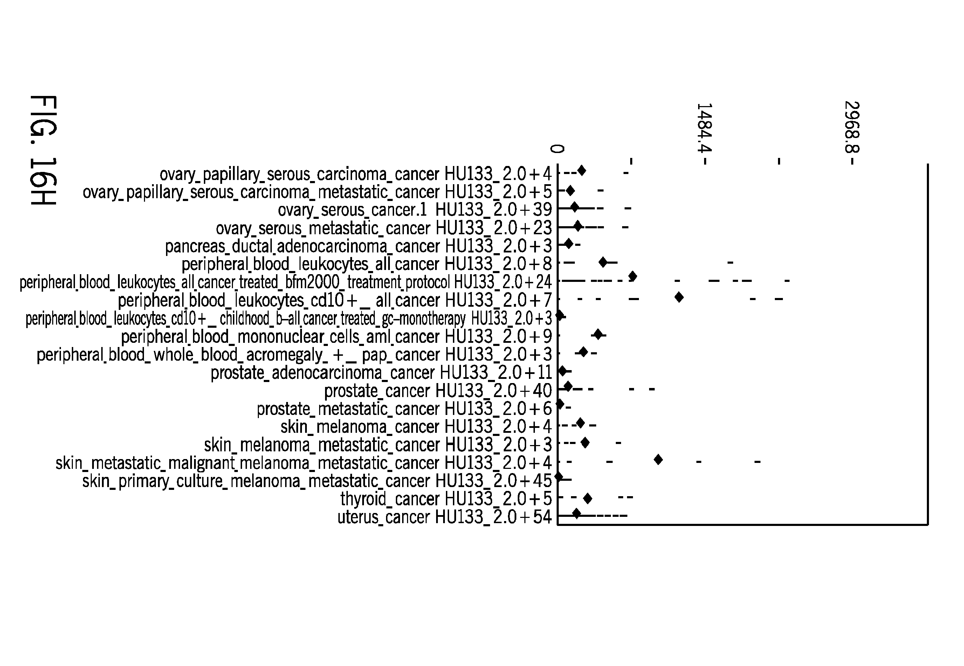

MED discovery engine described in Example 1 herein, was used to assess the expression of FAM26F transcripts. FAM26F transcripts were found to be over expressed in breast cancer and ovarian cancer, as is demonstrated in Figures 14 and 15, respectively. Figures 14 and 15 show expression graphs of Affymetrix probe set 229390_at. Figure 14 shows the expression of FAM26F transcripts in microarray chips from breast cancer and breast normal experiments. As can be seen FAM26F transcripts is overexpressed in breast cancer tissues (diamond markers) relative to its expression in normal breast (circle markers). Figure 15 shows the expression of FAM26F transcripts in microarray chips from ovarian cancer experiments. As can seen in Figure 16, FAM26F transcripts are overexpressed in ovarian cancer relative to normal ovary samples. Figure 16 shows the overall expression of FAM26F transcripts in various diseased, normal and cancer tissues.

### FAM26F qRT PCR results:

Real-Time RT-PCR analysis carried out on 5ng template in 5ul volume, in final volume of 12 µl in 384 well plates. The amplification was effected as follows: 50 °C for 2 min, 95 °C for 10 min, and then 40 cycles of 95 °C for 15 sec, followed by 60 °C for 30 sec, following by dissociation step of 95 °C for 15 sec, followed by 65 °C for 15 sec and final step of 95 °C for 15 sec. The quantity was calculated using standard curve of serial dilutions of the PCR product.

### Primers:

T82906 _seg5-10F1 (SEQ ID NO:95): GGCCAAGGCGTCGGAC
T82906 _seg5-10R1 (SEQ ID NO:96): GAAAACTAACTGGAGATAGGCATCG
**Amplicon:** T82906_DB63_seg5-10F1R1 (SEQ ID NO:97):

GGCCAAGGCGTCGGACGTGCAGGACCTCCTGAAGGATCTGAAGGCTCAGTCGC
AGGTGTTGGGCTGGATCTTGATAGCAGTTGTTATCATCATTCTTCTGATTTTTAC
ATCTGTCACCCGATGCCTATCTCCAGTTAGTTTTC

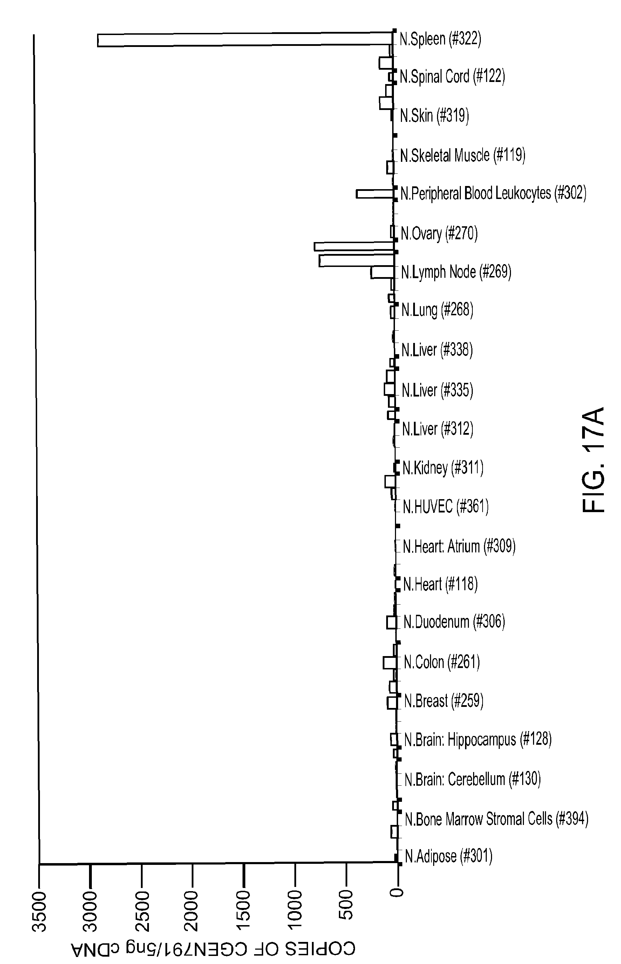

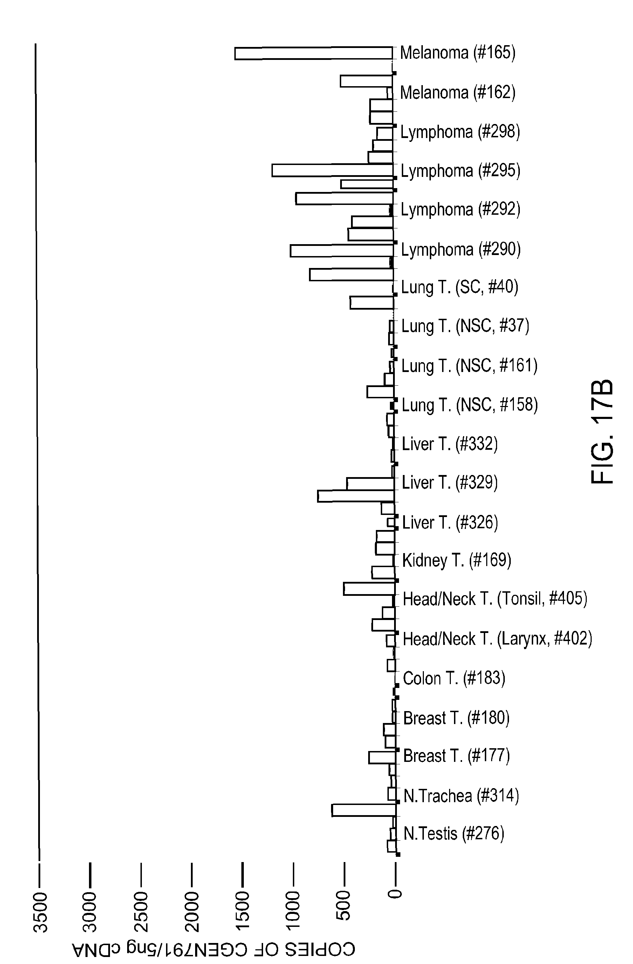

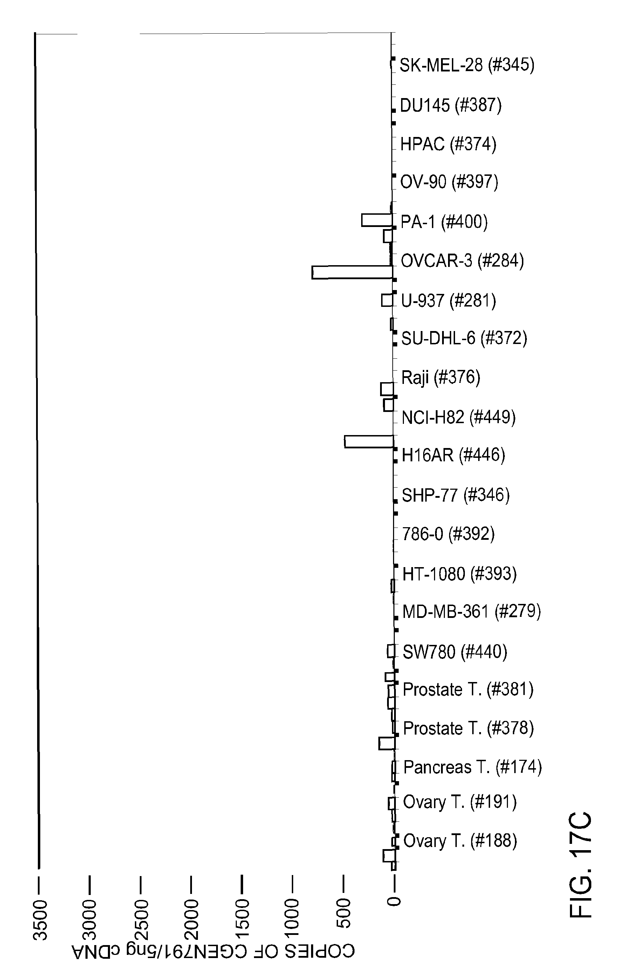

The tissue panel used for this qRT-PCR analysis is described in Table 1 herein. The histogram representing the qRT PCR results is shown in Figure 17. The results show the overexpression of FAM26F in kidney cancer, liver cancer, lung cancer, NHL lymphomas, melanoma, pancreas cancer and prostate cancer.

### Expression of LOC441168-FAM26 T82906 transcripts which are detectable by amplicon as depicted in sequence name T82906_seg5-10F7R5 (SEQ ID NO:124) in blood specific panel and in different normal tissues

Expression of LOC441168-FAM26 transcripts detectable by or according to seg5-10F7R5 - T82906_seg5-10F7R5 (SEQ ID NO:124) amplicon and primers T82906_seg5-10F7 (SEQ ID NO:122) and T82906_seg5-10R5 (SEQ ID NO:123) was measured by real time PCR in blood panel (Table 2) and normal panel (Table 3).

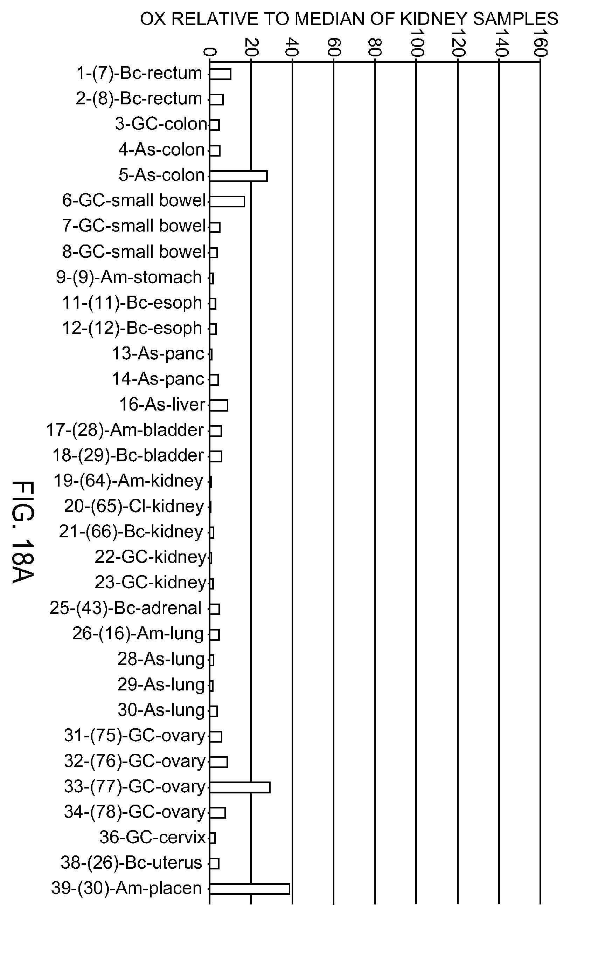

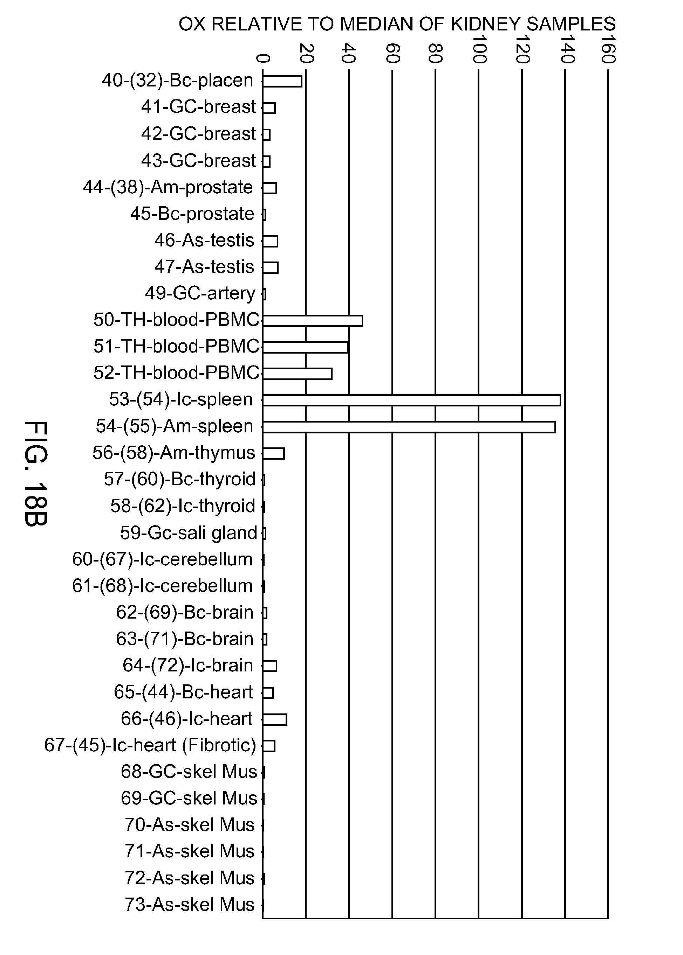

Normal panel - For each RT sample, the expression of the above amplicon was normalized to the normalization factor calculated from the expression of several house keeping genes as described in example 1 herein. The normalized quantity of each RT sample was then divided by the median of the quantities of the kidney samples (sample numbers 19-23, Table 3), to obtain a value of relative expression of each sample relative to median of the kidney samples, as shown in Figure 18. High expression was observed in spleen and PBMC samples.

Blood panel - For blood panel -The normalized quantity of each RT sample was then divided by the median of the quantities of the kidney normal samples (sample numbers 65-67, Table 2 above), to obtain a value of relative expression of each sample relative to median of the kidney normal samples.

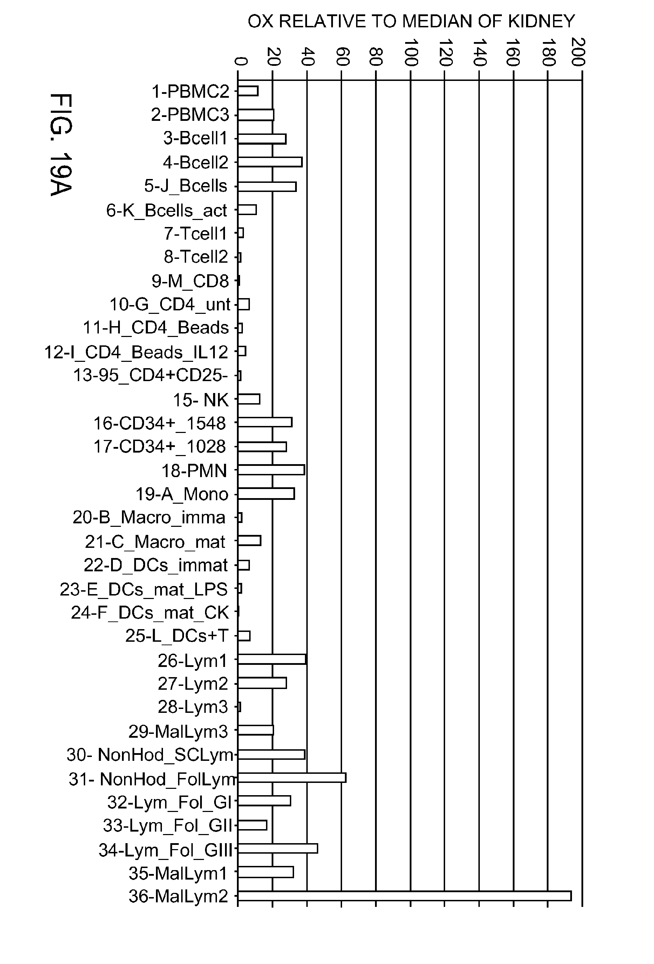

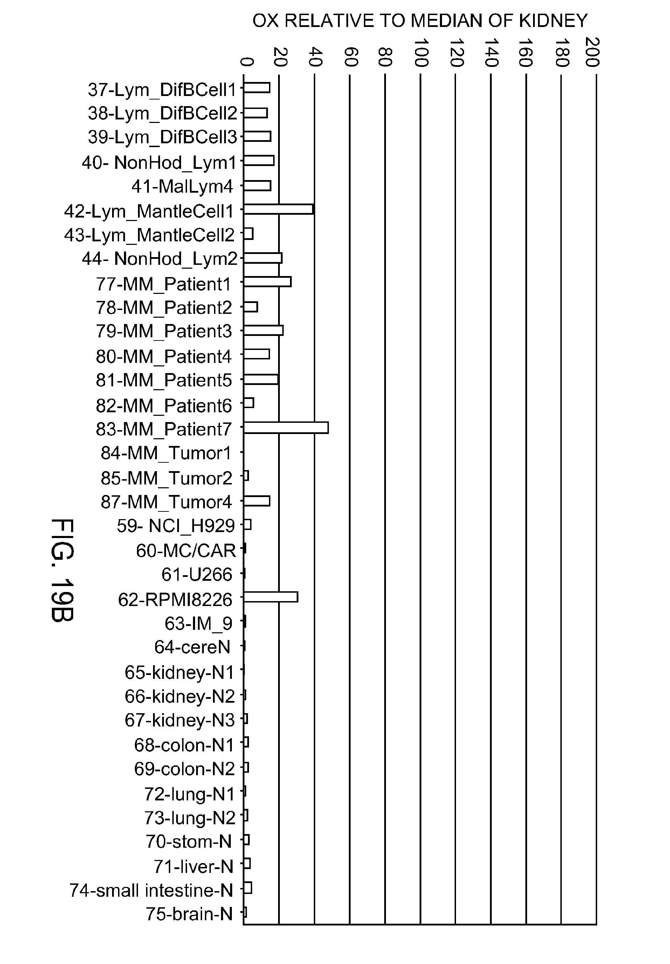

The results of this analysis are depicted in the histogram in Figure 19. Expression of the above-indicated LOC441168-FAM26 transcripts is high in PBMC, B cell, CD34+, lymphomas and multiple myelome patient samples.
Forward Primer (T82906_seg5-10F7) (SEQ ID NO:122):
CTGAAGGATCTGAAGGCTCAGTC
Reverse Primer (T82906_seg5-10R5) (SEQ ID NO:123)
GGATCTGCTGCTCCTGTTCC

Amplicon (T82906_seg5-10F7R5) (SEQ ID NO:124):

CTGAAGGATCTGAAGGCTCAGTCGCAGGTGTTGGGCTGGATCTTGATAGC
AGTTGTTATCATCATTCTTCTGATTTTTACATCTGTCACCCGATGCCTATCTCCA
GTTAGTTTTCTGCAGCTGAAATTCTGGAAAATCTATTTGGAACAGGAGCAGCAG
ATCC

### EXAMPLE 3_3

### Production of polyclonal antibodies specific to FAM26F_P4 protein

All polyclonal antibody production procedures, including peptides synthesis, peptides conjugation, animals immunizations, animals care and handling, bleeding and antibodies purification were performed at EZBiolab (IN,USA).

Peptide synthesis - The peptides which were used for rabbit immunization were as follows: EKFRAVLDLHVKH (FAM26F-1 (SEQ ID NO:117), a sequence taken from the N'terminus corresponding to aa 2-14 of the FAM26F_P4 protein (SEQ ID NO:), in which Cystein was added to the peptide N' terminus for KLH conjugation, and CNQAKASDVQDLLKD (FAM26F-2 (SEQ ID NO:118), a sequence taken from the ECD loop corresponding to aa 155-169 of the FAM26F_P4 protein (SEQ ID NO:18) peptides were synthesized with 95% purity and were conjugated to KLH carrier.

Immunization -Each two pairs of rabbits were immunized with the above peptides. Total of 200ml serum for each antibody are being collected.

Antibody purification - Antibodies will be purified from rabbits' serum. Affinity purification will be performed with the peptide against which the respective antibodies were raised using an immuno affinity column.
FAM26F-1 peptide sequence SEQ ID NO:117
EKFRAVLDLHVKH
FAM26F-2 peptide sequence SEQ ID NO:118
CNQAKASDVQDLLKD

### EXAMPLE 3_4

### CLONING OF FAM26F_P4 FUSED TO FLAG TAG

Cloning of FAM26F open reading frame (ORF) fused to FLAG was carried out by PCR as described below.

PCR was done using Super-Therm (Roche, catalog number: JMR-801) with 5% DMSO using 10□µl cDNA of MalLym2 from the blood panel described above and 0.5µl (10µM) of each primer #100-921 (SEQ ID NO: 172) and #100-922 (SEQ ID NO:173) in a total reaction volume of 25µl; with a reaction program of 2 minutes in 94°C; 45 cycles of: 30 seconds at 94°C, 30 seconds at 50°C, 1 minute at 72°C; then 10 minutes at 72°C. Primers which were used include gene specific sequences; restriction enzyme sites; Kozak sequence and FLAG tag.

5µl of PCR product were loaded onto a 1.2% agarose gel stained with ethidium bromide, electrophoresed in 1xTAE solution at 100V, and visualized with UV light. After verification of expected band size, PCR product was purified using QiaQuick™ PCR Purification kit (Qiagen, catalog number: 28004). The purified PCR product was digested with NheI and AgeI restriction enzymes (New England Biolabs, Beverly, MA, U.S.A.). After digestion, DNA was loaded onto a 1.2 % agarose gel as described above. The expected band size was excised and extracted from the gel using QiaQuick™ Gel Extraction kit (Qiagen, catalog number: 28707). The digested DNA was then ligated into pIRESpuro3 vector, previously digested with the above restriction enzymes, using LigaFastTM Rapid DNA Ligation System (Promega, catalog number: M8221). The resulting DNA was transformed into competent E.Coli bacteria DH5α (RBC Bioscience, Taipei, Taiwan, catalog number: RH816) according to manufacturer's instructions, then plated on LB-ampicillin agar plates for selection of recombinant plasmids, and incubated overnight at 37°C. The following day, positive colonies were screened by PCR using pIRESpuro3 vector specific primer and gene specific primer (data not shown). The PCR product was analyzed using 1.2% agarose gel as described above. After verification of expected band size, positive colonies were grown in 5 ml Terrific Broth supplemented with 100µg/ml ampicillin, with shaking overnight at 37°C. Plasmid DNA was isolated from bacterial cultures using Qiaprep™ Spin Miniprep Kit (Qiagen, catalog number: 27106). Accurate cloning was verified by sequencing the inserts (Weizmann Institute, Rehovot, Israel). Upon verification of an error-free colony (i.e. no mutations within the ORF), recombinant plasmids were processed for further analyses.

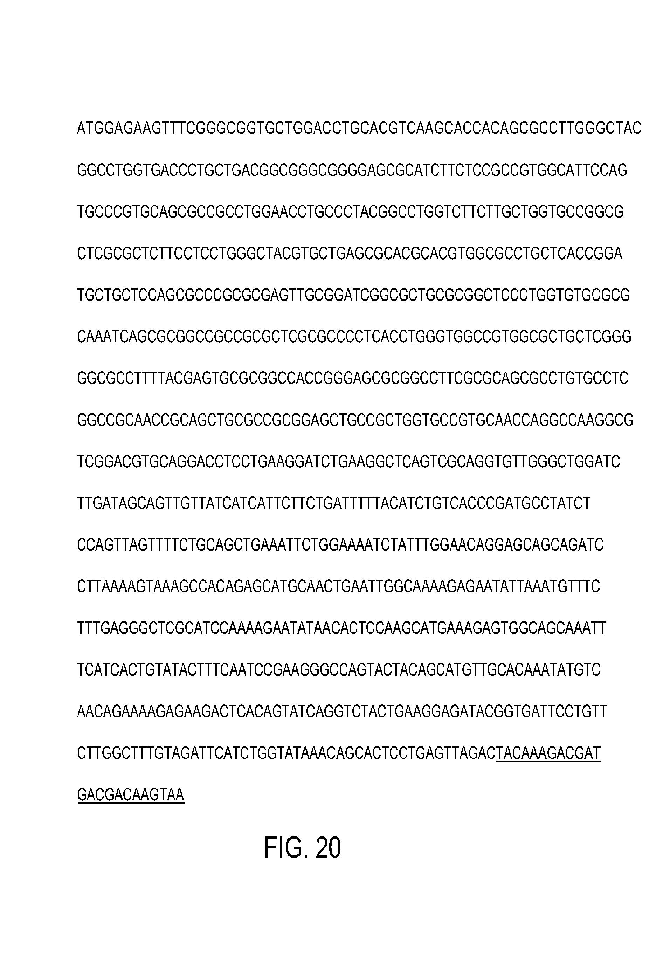

The DNA sequence of the resulting FAM26_P4_FLAG (SEQ ID NO: 174) is shown in Figure 20; FLAG sequence is in underlined.

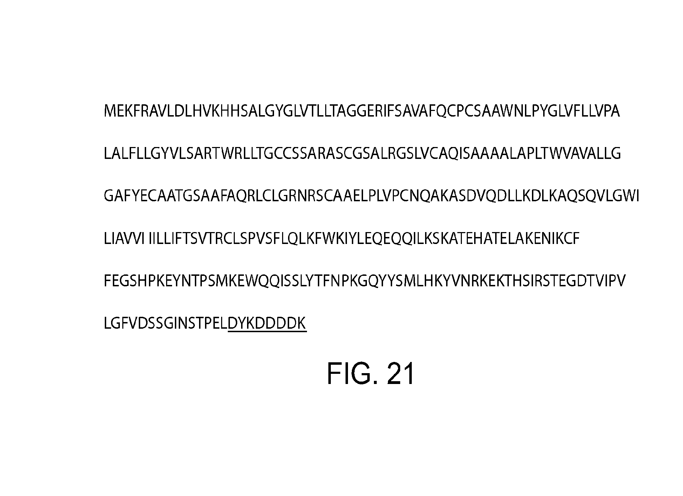

The amino acid sequence of FAM26_P4_FLAG (SEQ ID NO:175) is shown in Figure 21; FLAG sequence is in underlined.

### EXAMPLE 3_5

### DETERMINING CELL LOCALIZATION OF FAM26_P4

In order to determine FAM26_P4 cellular localization, FAM26_P4 was cloned in frame to FLAG tag, as described above. Protein localization was observed upon transient transfection (as described in Chen et al., Molecular Vision 2002; 8; 372-388) using confocal microscopy. 48 hours following transfection, the cells were stained with anti FLAG antibodies conjugated to Cy-3 flurophore (Sigma, catalog number: A9594) and were observed for the presence of fluorescent signal.

FAM26_P4_FLAG (SEQ ID NO:174) pIRESpuro3 construct was transiently transfected into HEK-293T cells as follows: HEK-293T (ATCC, CRL-11268) cells were plated on sterile glass coverslips, 13mm diameter (Marienfeld, catalog number: 01 115 30), which were placed in a 6 well plate, using 2ml pre-warmed DMEM [Dulbecco's modified Eagle's Media, Biological Industries (Beit Ha'Emek, Israel), catalog number: 01-055-1A] + 10% FBS [Fetal Bovine Serum, Biological Industries (Beit Ha'Emek, Israel), catalog number: 04-001-1A] + 4mM L-Glutamine [Biological Industries (Beit Ha'Emek, Israel), catalog number: 03-020-1A]. 500,000 cells per well were transfected with 2µg of DNA construct using 6µl FuGENE 6 reagent (Roche, catalog number: 11-814-443-001) diluted into 94µl DMEM. The mixture was incubated at room temperature for 15 minutes. The complex mixture was added dropwise to the cells and swirled. Cells were placed in incubator maintained at 37°C with 5% CO₂ content. 48 hours post transient transfection, cells on coverslip were further processed for immunostaining and analysis by confocal microscopy. The cover slip was washed in phosphate buffered saline (PBS), then fixed for 15 minutes with a solution of 3.7% paraformaldehyde (PFA) (Sigma, catalog number: P-6148)/3% glucose (Sigma, catalog number: G5767) (diluted in PBS). Quenching of PFA was done by a 5 minute incubation in 3mM glycine (Sigma, catalog number: G7126) (diluted in PBS). After two 5-minute washes in PBS, cells were permeabilized with 0.1% triton-X100 (diluted in PBS) for 5 minutes. After two 5-minute washes in PBS, blocking of non-specific regions was done with 5% bovine serum albumin (BSA) (Sigma, catalog number: A4503) (diluted in PBS) for 20 minutes. The coverslip was then incubated, in a humid chamber for 1 hour, with mouse anti FLAG-Cy3 antibodies (Sigma, catalog number: A9594), diluted 1:100 in 5% BSA in PBS, followed by three 5-minute washes in PBS. The coverslip was then mounted on a slide with Gel Mount Aqueous medium (Sigma, catalog number: G0918) and cells were observed for the presence of fluorescent product using confocal microscopy.

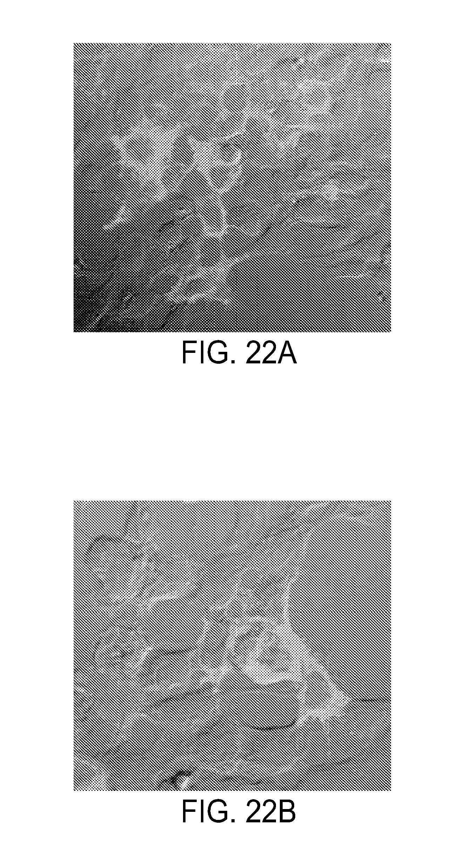

Cell localization is shown in Figure 22. FAM26_P4 was localized to the cell membrane.

### EXAMPLE 3_6

### CHARACTERIZATION OF PURIFIED FAM26F_P4 ANTIBODIES BY IMMUNOSTAINING OF FAM26F_P4 TRANSFECTED CELLS

In order to further characterize the affinity purified antibodies raised against FAM26_P4, antibody-protein interaction was studied using immunostaining of FAM26_P4 stable transfected HEK293T cells.

Two stably transfected pools were generated, FAM26_P4 pIRESpuro3 and the negative control, empty pIRESpuro3. Both constructs were transfected into HEK-293T cells as follows:
HEK-293T (ATCC, CRL-11268) cells were plated in a sterile 6 well plate suitable for tissue culture, using 2ml pre-warmed of complete media, DMEM [Dulbecco's modified Eagle's Media, Biological Industries (Beit Ha'Emek, Israel), catalog number: 01-055-1A] + 10% FBS [Fetal Bovine Serum, Biological Industries (Beit Ha'Emek, Israel), catalog number: 04-001-1A] + 4mM L-Glutamine [Biological Industries (Beit Ha'Emek, Israel), catalog number: 03-020-1A]. 500,000 cells per well were transfected with 2µg of DNA construct using 6µl FuGENE 6 reagent (Roche, catalog number: 11-814-443-001) diluted into 94ul DMEM. The mixture was incubated at room temperature for 15 minutes. The complex mixture was added dropwise to the cells and swirled. Cells were placed in incubator maintained at 37°C with 5% CO₂ content. 48 hours following transfection, transfected cells were transferred to a 75cm² tissue culture flask containing 15ml of selection media: complete media supplemented with 5µg\ml puromycin (Sigma, catalog number P8833). Cells were placed in incubator, and media was changed every 3-4 days, until clone formation observed.

### IMMUNOSTAINING OF FAM26F TRANSFECTED CELLS

500,000 cells per well of HEK-293T (ATCC, CRL-11268) stably expressing FAM26F or the empty vector pIRES puro3, described above, were plated on sterile glass coverslips, 13mm diameter (Marienfeld, catalog number: 01 115 30), which were placed in a 6 well plate, using 2ml pre-warmed DMEM [Dulbecco's modified Eagle's Media, Biological Industries (Beit Ha'Emek, Israel), catalog number: 01-055-1A] + 10% FBS [Fetal Bovine Serum, Biological Industries (Beit Ha'Emek, Israel), catalog number: 04-001-1A] + 4mM L-Glutamine [Biological Industries (Beit Ha'Emek, Israel), catalog number: 03-020-1A].

48 hours post plating the cells on coverslips they were further processed for immunostaining and analysis by confocal microscopy. The cover slips were washed in phosphate buffered saline (PBS), then fixed for 25 minutes with a 3.7% paraformaldehyde (PFA) (Sigma, catalog number: P-6148)/3% glucose (Sigma, catalog number: G5767). After 2 5-minute washes in PBS, cells were permeabilized with 0.1% triton-X100 (diluted in PBS) for 5 minutes. After two 5-minute washes in PBS, blocking of non-specific regions was done with 5% bovine serum albumin (BSA) (Sigma, catalog number: A4503) (diluted in PBS) for 20 minutes. The coverslips were then incubated, in a humid chamber for 1 hour, with purified rabbit anti-FAM26F antibodies raised in EZBiolabs described above were washed 3 times for 5-minutes in PBS. The coverslips were then incubated, in a humid chamber for 1 hour, with secondary antibody: donkey anti-rabbit conjugated to Cy-3 flurophore (Jackson ImmunoResearch, catalog number: 711-165-152), diluted 1:200 in 3% BSA in PBS. After 3 5-minute washes in PBS, the fixed coverslips were mounted on slides with Gel Mount Aqueous medium (Sigma, catalog number: G0918) and cells were observed for the presence of fluorescent product using confocal microscopy.

Cell staining was not specific, staining was observed in the nucleus of both on FAM26F transfected cells as well as pIRESpuro3 HEK-293T using purified anti FAM26F antibodies raised in EZBiolabs transfected cells negative control (data not shown), raising a need to seek after additional FAM26F antibodies.

In order to further analyze expression and localization of FAM26F protein in trasfectants, cells were immunostained as described above using commercial antibody specific to FAM26F protein (Sigma, catalog number: HPA017948) diluted 1:50 in 5%BSA.

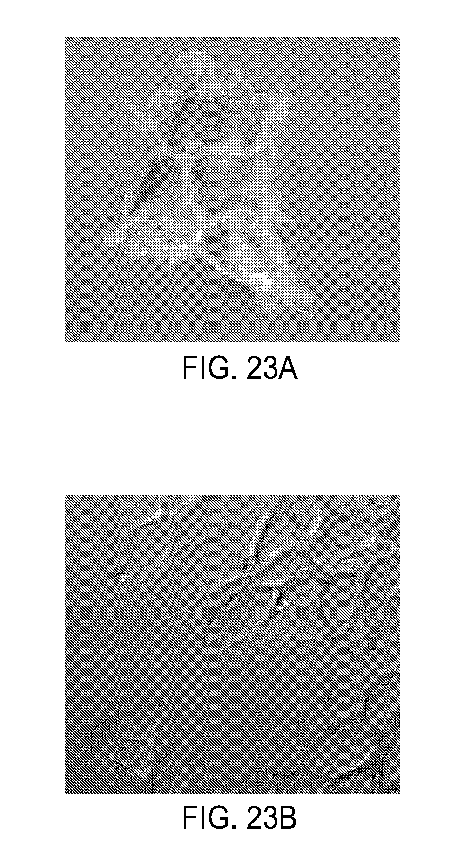

Specific cell staining localized to cell membrane was observed using anti FAM26F antibodies on FAM26F transfected cells (Figure 23A); however, no staining was observed using these antibodies on pIRESpuro3 HEK-293T transfected cells (Figure 23B). The red fluorescence obtained in Figure 23A as opposed to the absence of signal in Figure 23B demonstrates the specificity of FAM26F antibodies to FAM26F_P4.

In order to determine endogenous expression of FAM26F, RPMI8226 cells (ATCC cat#CCL-155) were immunostained as described above using specific antibodies against FAM26_P4 protein (Sigma, catalog number HPA017948).

Cell staining was observed using the specific antibodies (Sigma, catalog number HPA017948) on RPMI8226 cell line. However, localization could not be determined as membranal (data not shown).

### EXAMPLE 3_7: IMMUNOHISTOCHEMISTRY ANALYSIS OF FAM26 ANTIBODY ON TISSUE SAMPLES

### Antibody Titration Protocol and Positive Control Study Results:

Antibody titration experiments were conducted at LifeSpan Biosciences (USA) with rabbit polyclonal antibody FAM26 (SIGMA cat # HPA017948) to establish concentrations that would result in minimal background and maximal detection of signal. Serial dilutions were performed at 20ug/ml, 10ug/ml, 5ug/ml, and 2.5ug/ml on formalin-fixed, paraffin-embedded tissues supplied by LifeSpan BioSciences (USA) and on HEK-293T (ATCC, CRL-11268) cell lines transiently transfected with FAM26 as positive control or empty vector as negative control. Antibody FAM26 (SIGMA cat # HPA017948) was used as the primary antibody, and the principal detection system consisted of a Vector anti-rabbit secondary (BA-1000) and a Vector ABC-AP kit (AK-5000) with a Vector Red substrate kit (SK-5100),which was used to produce a fuchsia-colored deposit.

Antibody titration experiments were conducted with antibody to CD20 (DAKO, Cat. # MO755, mouse monoclonal) to establish dilutions that would result in minimal background and maximal detection of signal. Serial dilutions were performed at 1:1000, 1:2000 and 1:4000. Antibody to CD20 was used as the primary antibody, and the principal detection system consisted of a Vector anti-rabbit secondary (BA-2000) and a Vector ABC-AP kit (AK-5000) with a Vector Red substrate kit (SK-5100),which was used to produce a fuchsia-colored deposit.

Tissues were also stained with a positive control antibody (CD31 and vimentin) to ensure that the tissue antigens were preserved and accessible for immunohistochemical analysis. Only tissues that showed positive CD31 and vimentin staining were selected for the study. The negative control consisted of performing the entire immunohistochemistry procedure on adjacent sections in the absence of primary antibody. Slides were imaged with a DVC1310C digital camera coupled to a Nikon microscope.

Antibody FAM26 at 5 micrograms/mL showed prominent cytoplasmic and membranous staining in the positive cell line,and only weak staining in the negative cell line (data not shown). In normal tissues, the most prominent staining was present in subsets of monocyte-derived cell types (macrophages and histiocytes) and in occasional lymphocytes, which showed both cytoplasmic and nuclear staining (data not shown). Compared to antibody to CD20, the staining pattern was more prominent in monocyte cell types, and lymphocyte staining was not limited to typical B-lymphocyte zones. The antibody showed weak cytoplasmic and nuclear staining in most other cell types. Further characterization of antibody to FAM26 is under the process.

### EXAMPLE 4: MGC52498 POLYPEPTIDES AND POLYNUCLEOTIDES, AND USES THEREOF AS A DRUG TARGET FOR PRODUCING DRUGS AND BIOLOGICS

### EXAMPLE 4_1: DESCRIPTION FOR CLUSTER AA213820

Cluster AA213820 (internal ID 69312991) features 2 transcripts of interest, the names for which are given in Table 23. The selected protein variants are given in table 24.

**Table 23 - Transcripts of interest**

| **Transcript Name** |
|---|
| AA213820_T1 (SEQ ID NO:131) |
| AA213820_T6 (SEQ ID NO:20) |

**Table 24 - Proteins of interest**

| **Protein Name** | **Corresponding Transcript(s)** |
|---|---|
| AA213820_P4 (SEQ ID NO:135) | AA213820_T1 (SEQ ID NO:131) |
| AA213820_P6 (SEQ ID NO:19) | AA213820_T6 (SEQ ID NO:20) |

These sequences are variants of the known hypothetical protein LOC348378 (SEQ ID NO:132) (SwissProt accession identifier NP_872427; synonims: MGC52498).

MGC52498 also known as FAM159A (family with sequence similarity 159, member A) was identified in two large scale studes: the secreted protein initiative (Clark et al. 2003, Genome Research 13(10): 2265-70), and a full length cDNA project (Strausberg et al. 2002, PNAS 99(26): 16899-903). However no specific study was published on this protein.

The sequence depicted in PRO90951, corresponding to sequence of AA213820_P4 (SEQ ID NO: 135) was reported in WO2004081199, among other human genes showing altered patterns of expression in autoimmune disease for use in diagnosis, prevention and treatment. The sequence corresponding to AA213820_P4 (SEQ ID NO:135) was also reported in WO2004047728, among other human genes having expression profile in activated human CD4+T cells useful for the diagnosis and treatment of immune-related diseases.

However, none of the WO2004081199 or WO2004047728 applications teach or suggest that sequence corresponding to AA213820_P4 (SEQ ID NO:135) is differentially expressed in cancer. WO2004081199 and WO2004047728 also do not teach or suggest that AA213820_P4 (SEQ ID NO:135) is differentially expressed in lymphoma, especially Non-Hodgkin's Lymphoma, Multiple Myeloma, leukemia, especially T cell leukemia, or lung cancer. Also, there is no teaching or suggestion in the WO2004081199 or WO2004047728 applications that AA213820_P4 (SEQ ID NO:135) can be used as drug target for treatment of cancer or for cancer diagnosis. Also, there is no teaching or suggestion in the WO2004081199 application that antibodies specific for AA213820_P4 (SEQ ID NO:135), its soluble ectodomain, and/or fragments thereof can be used as therapeutic or diagnostic agents for treatment of cancer.

By contrast and surprisingly, the present inventors have found that MGC52498 antigen and discrete portions thereof may optionally use as a drug target for therapeutic small molecules, peptides, antibodies, antisense RNAs, siRNAs, ribozymes, and the like. Diagnostic and therapeutic polyclonal and monoclonal antibodies and fragments thereof that bind MGC52498, and portions and variants thereof may optionally be produced. According to at least some embodiments of the invention, there is provided the use of antibodies and antibody fragments against MGC52498 antigen, its secreted or soluble form or ECD and/or variants, conjugates, or fragments thereof and fragments and variants thereof for treating and diagnosing lymphoma, especially Non-Hodgkin's Lymphoma, Multiple Myeloma, leukemia, especially T cell leukemia, and lung cancer, wherein this antigen is differentially expressed.

As noted above, cluster AA213820 features 2 transcript), which were listed in Table 23 above. These transcript(s) encode for protein(s) which are variant(s) of protein hypothetical protein LOC348378 (SEQ ID NO:132). A description of each variant protein according to at least some embodiments of the invention is now provided.

Variant protein AA213820_P4 (SEQ ID NO:135) according to at least some embodiments of the invention has an amino acid sequence as encoded by transcript AA213820_T1 (SEQ ID NO:131).

The localization of the variant protein was determined according to results from a number of different software programs and analyses, including analyses from SignalP and other specialized programs. The variant protein is believed to be located as follows with regard to the cell: membrane.

The coding portion of transcript AA213820_T1 (SEQ ID NO:131) starts at position 287 and ends at position 856.

Variant protein AA213820_P6 (SEQ ID NO:19) according to at least some embodiments of the invention has an amino acid sequence as encoded by transcript AA213820_T6 (SEQ ID NO:20). A description of the relationship of the variant protein according to at least some embodiments of the invention to known proteins is as follows:
1. Comparison report between AA213820_P6 (SEQ ID NO:19) and known protein Q6UWV7_HUMAN (SEQ ID NO:135) :
   A. An isolated chimeric polypeptide, comprising a first amino acid sequence being at least 70%, optionally at least 80%, preferably at least 85%, more preferably at least 90% and most preferably at least 95%, homologous to a polypeptide having the sequence MASLWPSALTFNTDANIPGPLGFCGGWVRLCSLSSLTPPCGRRLVPCLSAPAPNAPR LPAPARC (SEQ ID NO: 153) corresponding to amino acids 1 - 64 of AA213820_P6 (SEQ ID NO:19), and a second amino acid sequence being at least 90% homologous to SIGALIGLSVAAVVLLAFIVTACVLCYLFISSKPHTKLDLGLSLQTAGPEEVSPDCQG VNTGMAAEVPKVSPLQQSYSCLNPQLESNEGQAVNSKRLLHHCFMATVTTSDIPGS PEEASVPNPDLCGPVP (SEQ ID NO: 152), corresponding to amino acids 5 - 134 of known protein Q6UWV7_HUMAN (SEQ ID NO:135), which also corresponds to amino acids 65 - 194 of AA213820_P6 (SEQ ID NO:19), wherein said first amino acid sequence and second amino acid sequence are contiguous and in a sequential order.
   B. An isolated polypeptide corresponding to a head of AA213820_P6 (SEQ ID NO:19), comprising a polypeptide being at least 70%, optionally at least about 80%, preferably at least about 85%, more preferably at least about 90% and most preferably at least about 95% homologous to the sequence MASLWPSALTFNTDANIPGPLGFCGGWVRLCSLSSLTPPCGRRLVPCLSAPAPNAPR LPAPARC (SEQ ID NO: 153) of AA213820_P6 (SEQ ID NO:19).
2. Comparison report between AA213820_P6 (SEQ ID NO:19) and known protein Q6ZRG4_HUMAN (SEQ ID NO:135) :
   A. An isolated chimeric polypeptide, comprising a first amino acid sequence being at least 70%, optionally at least 80%, preferably at least 85%, more preferably at least 90% and most preferably at least 95%, homologous to a polypeptide having the sequence MASLWPSALTFNTDANIPGPLGFCGGWVRLCSLSSLTPPCGRRLVPCLSAPAPNAPR LPAPARC (SEQ ID NO: 153) corresponding to amino acids 1 - 64 of AA213820_P6 (SEQ ID NO:19), a second amino acid sequence being at least 90% homologous to SIGALIGLSVAAVVLLAFIVTACVLCYLFISSKPHTKLDLGLSLQTAGP corresponding to amino acids 61 - 109 of known protein Q6ZRG4_HUMAN (SEQ ID NO:135), which also corresponds to amino acids 65 - 113 of AA213820_P6 (SEQ ID NO:19), and a third amino acid sequence being at least 70%, optionally at least 80%, preferably at least 85%, more preferably at least 90% and most preferably at least 95% homologous to a polypeptide having the sequence EEVSPDCQGVNTGMAAEVPKVSPLQQSYSCLNPQLESNEGQAVNSKRLLHHCFMA TVTTSDIPGSPEEASVPNPDLCGPVP (SEQ ID NO: 151) corresponding to amino acids 114 - 194 of AA213820_P6 (SEQ ID NO:19), wherein said first amino acid sequence, second amino acid sequence and third amino acid sequence are contiguous and in a sequential order.
   B. An isolated polypeptide corresponding to a head of AA213820_P6 (SEQ ID NO:19), comprising a polypeptide being at least 70%, optionally at least about 80%, preferably at least about 85%, more preferably at least about 90% and most preferably at least about 95% homologous to the sequence MASLWPSALTFNTDANIPGPLGFCGGWVRLCSLSSLTPPCGRRLVPCLSAPAPNAPR LPAPARC (SEQ ID NO: 153) of AA213820_P6 (SEQ ID NO:19).
   C. An isolated polypeptide corresponding to an edge portion of AA213820_P6 (SEQ ID NO:19), comprising an amino acid sequence being at least 70%, optionally at least about 80%, preferably at least about 85%, more preferably at least about 90% and most preferably at least about 95% homologous to the sequence EEVSPDCQGVNTGMAAEVPKVSPLQQSYSCLNPQLESNEGQAVNSKRLLHHCFMA TVTTSDIPGSPEEASVPNPDLCGPVP (SEQ ID NO: 151) of AA213820_P6 (SEQ ID NO:19).

The localization of the variant protein was determined according to results from a number of different software programs and analyses, including analyses from SignalP and other specialized programs. The variant protein is believed to be located as follows with regard to the cell: membrane.

The coding portion of transcript AA213820_T6 (SEQ ID NO:20) starts at position 2 and ends at position 496.

### EXAMPLE 4_2 EXPRESSION ANALYSIS OF MGC52498 TRANSCRIPTS

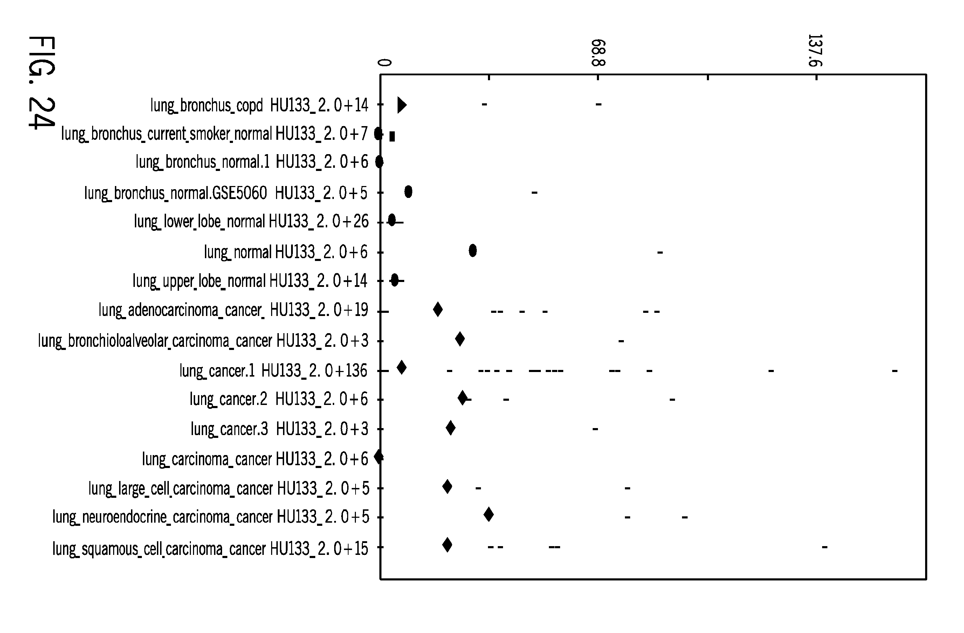

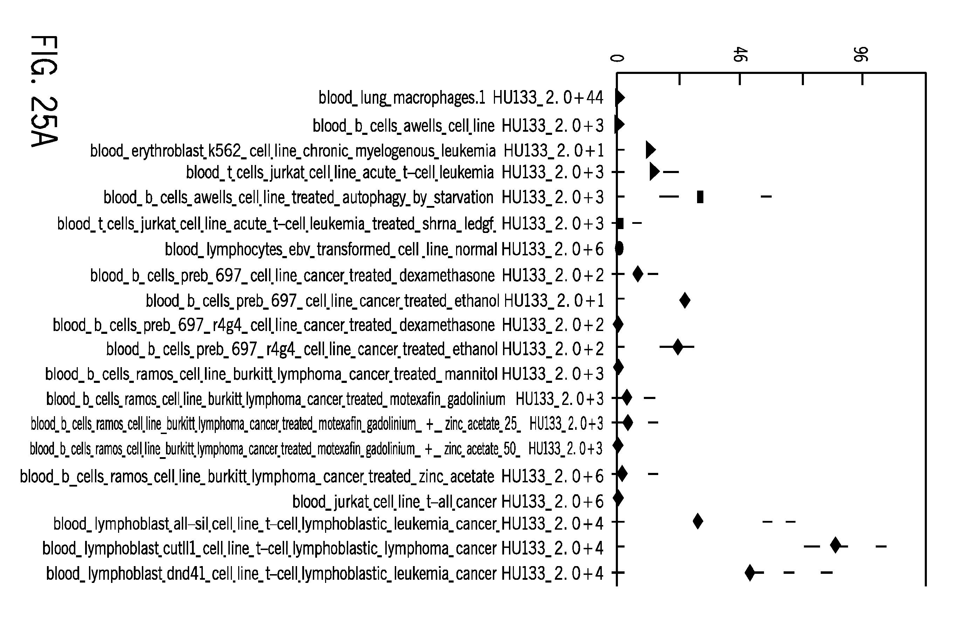

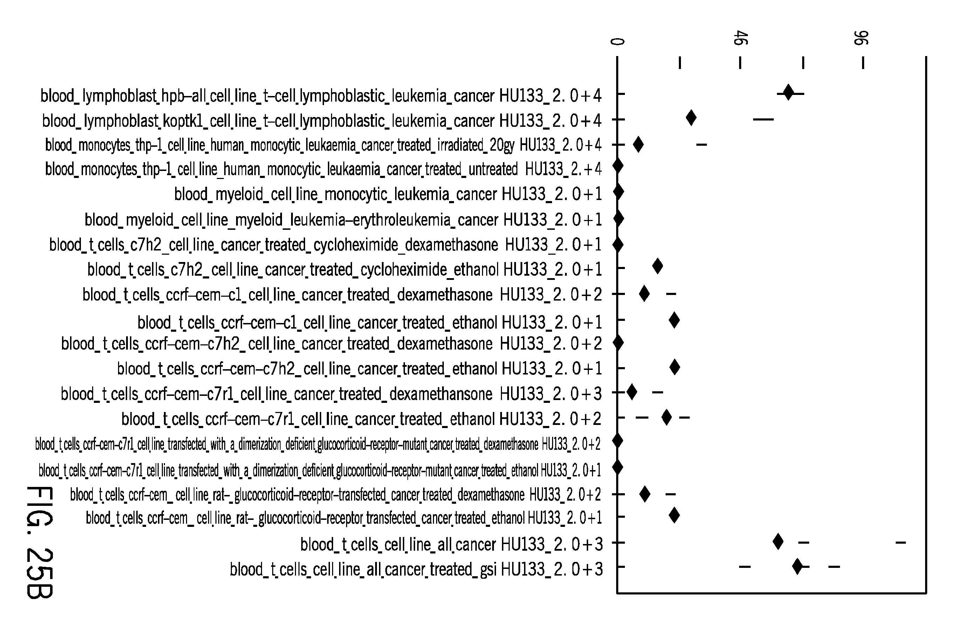

The MED discovery engine, described in Example 1 herein, was used to assess the expression of MGC52498 transcripts. MGC52498 transcripts were found to be over expressed in lung cancer, as demonstrated in Figure 24, and in leukemias, as demonstrated in Figure 25. Figures 24 and 25 show expression graphs of Affymetrix probe set 1555379_at. Figure 24 shows the expression of MGC52498 transcripts in microarray chips from lung cancer and lung normal experiments. As can be seen MGC52498 transcripts are overexpressed in lung cancer tissues (diamond markers) relative to their expression in normal lung (circle markers).

Figure 25 shows the expression of MGC52498 transcripts in microarray chips from blood cancers and normal blood experiments. As can be seen MGC52498 transcripts are overexpressed in various leukemia samples (diamonds markers) relative to its expression in normal blood samples (circle, square and triangle markers).

### Expression of hypothetical protein MGC52498 AA213820 transcripts which are detectable by amplicon as depicted in sequence name AA213820_seg8-11F2R2 (SEQ ID NO: 109) in blood specific panel and in different normal tissues

Expression of hypothetical protein MGC52498 transcripts detectable by or according to seg8-11F2R2 - AA213820_seg8-11F2R2 (SEQ ID NO: 109) amplicon and primers AA213820_seg8-11F2 (SEQ ID NO: 107) and AA213820_seg8-11R2 (SEQ ID NO: 108) was measured by real time PCR in blood panel and normal panel. The samples used for blood panel are detailed in Tables 2 and 2_1. The samples used for normal panel are detailed in Table 3.

### Normal panel -

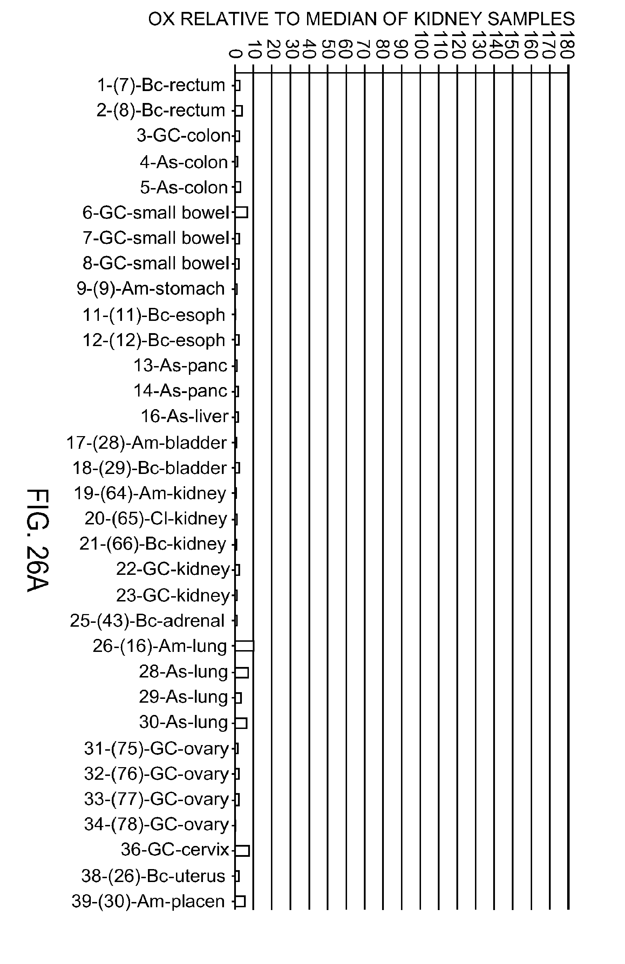

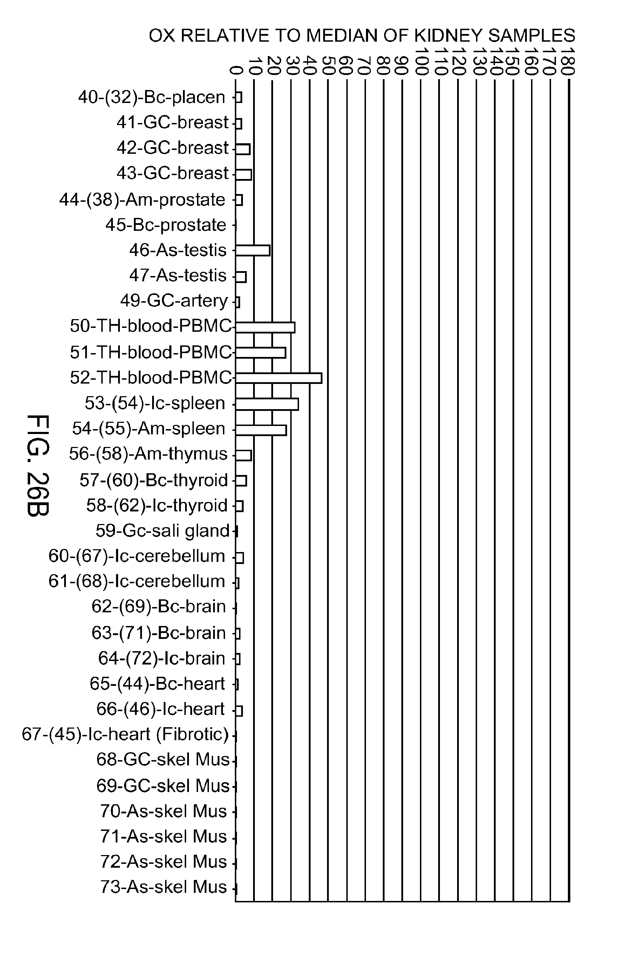

For each RT sample, the expression of the above amplicon was normalized to the normalization factor calculated from the expression of several house keeping genes as described in Example 1. The normalized quantity of each RT sample was then divided by the median of the quantities of the kidney samples (sample numbers 19-23, Table 3 above), to obtain a value of relative expression of each sample relative to median of the kidney samples, as shown in Figure 26. High expression was observed in PBMCs and spleen normal samples.

### Blood panel -

For blood panel - For each RT sample, the expression of the above amplicon was normalized to the normalization factor calculated from the expression of several house keeping genes as described in Example 1. The normalized quantity of each RT sample was then divided by the median of the quantities of the kidney normal samples (sample numbers 65-67, Table 2 above), to obtain a value of relative expression of each sample relative to median of the kidney normal samples.

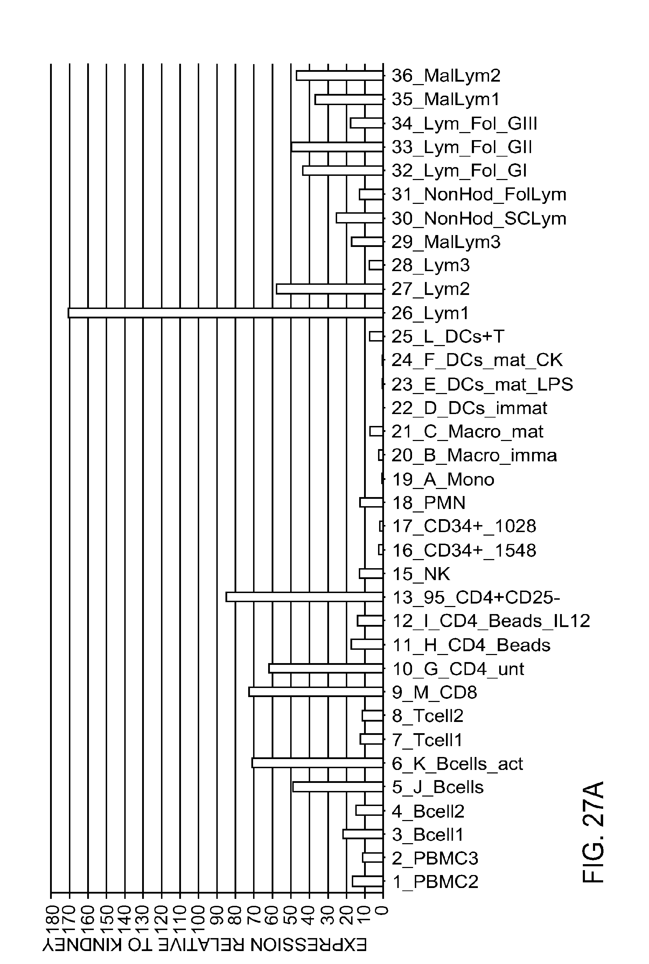

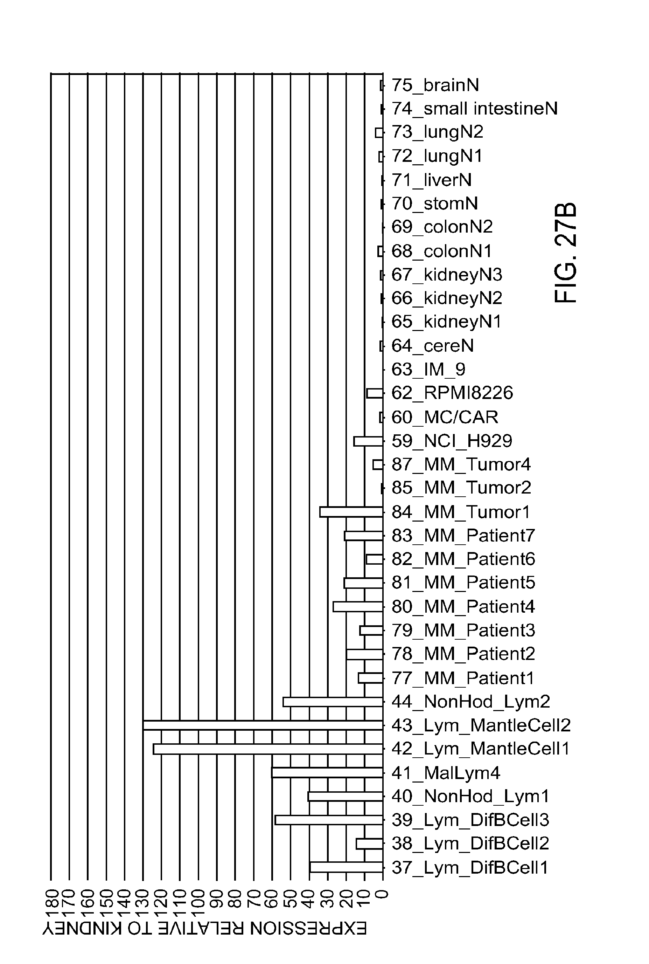

The results of this analysis are depicted in the histogram in Figure 27. Expression of the above-indicated hypothetical protein MGC52498 transcripts is high in different blood-derived cells, different lymphomas and multiple myeloma patients samples
Forward Primer (AA213820_seg8-11F2) (SEQ ID NO: 107): CAGCATTGGCGCTCTCATAGG
Reverse Primer (AA213820_seg8-11R2) (SEQ ID NO:108): GTGTTCACACCTTGGCAGTCAG

Amplicon (AA213820_seg8-11F2R2 (SEQ ID NO: 109)):
CAGCATTGGCGCTCTCATAGGCCTGTCCGTAGCAGCAGTGGTTCTTCTCGCCTTC
ATTGTTACCGCCTGTGTGCTCTGCTACCTGTTCATCAGCTCTAAGCCCCACACAA
AGTTGGACCTGGGCTTGAGCTTACAGACAGCAGGCCCTGAGGAGGTTTCTCCTG
ACTGCCAAGGTGTGAACAC

### EXAMPLE 4_3: CLONING OF FULL LENGTH ORF ENCODING MGC52498 T1_P4 FUSED TO FLAG

Cloning of Full Length ORF encoding MGC fused to FLAG tag, either at the C terminus or the N Terminus, was done as described below.

PCR was done using GoTaq ReadyMix (Promega, catalog number M7122) under the following conditions: 10.5□µl - cDNA from the blood panel described above (XXXLym_MantleCell1); 1µl - of each primer (10µM); 12.5µl ReadyMix with a reaction program of 3 minutes in 95°C; 40 cycles of 30 seconds at 94°C, 30 seconds at 50°C, 1.5 minutes at 72°C; then 10 minutes at 72°C. Primers which were used were primer #100-946 (SEQ ID NO:176) and primer #100-947 (SEQ ID NO:177). In order to enhance the PCR product and to generate Flag tag at the N/C terminus followed by NheI restriction site, and Kozak sequence, as well as EcoRI restriction site at the C terminus, second PCR was done using first PCR product as a template and specific primers as follows: primer #100-948 (SEQ ID NO:178) and primer #100-949 (SEQ ID NO:179), for the N terminus Flag or primer #100-954 (SEQ ID NO:180) and primer #100-955 (SEQ ID NO:181) for the C terminus Flag. PCR conditions were the same as described above.

The PCR product was loaded onto a 1% agarose gel stained with ethidium bromide, electrophoresed in 1xTBE solution at 100V, and visualized with UV light. After verification of expected size band, it was extracted using Qiaquick gel extraction purification kit (Qiagen™, Valencia, CA, U.S.A., catalog number 28706). The extracted PCR product was digested with the appropriate restriction enzymes: NheI and EcoRI (New England Biolabs, Beverly, MA, U.S.A.). After digestion, the DNA was loaded onto a 1 % agarose gel as described above. The expected band size was excised and extracted from the gel using QiaQuick™ Gel Extraction kit (Qiagen, catalog number: 28706).

The digested target ORF DNA was ligated into pIRESpuro3 vector previously digested with the same enzymes, using the LigaFastTM Rapid DNA Ligation System (Promega, catalog number: M8221). The resulting DNA were transformed into competent E.Coli bacteria DH5α (RBC Bioscience, Taipei, Taiwan, catalog number: RH816) according to manufacturer's instructions, then plated on LB-ampicillin agar plates for selection of recombinant plasmids, and incubated overnight at 37°C.

The following day, a number of colonies from the transformation were screen by PCR using GoTaq ReadyMix (Promega, catalog number: M7122) using pIRESpuro3 vector specific primer and gene specific primer (data not shown). After verification of expected band size, two positive colonies were grown in 5 ml Terrific Broth supplemented with 100µg/ml ampicillin, with shaking overnight at 37°C. Plasmid DNA was isolated from bacterial cultures using Qiaprep™ Spin Miniprep Kit (Qiagen, catalog number: 27106). Accurate cloning was verified by sequencing the inserts (Weizmann Institute, Rehovot, Israel). Upon verification of an error-free colony (i.e. no mutations within the ORF), recombinant plasmid was processed for further analysis.

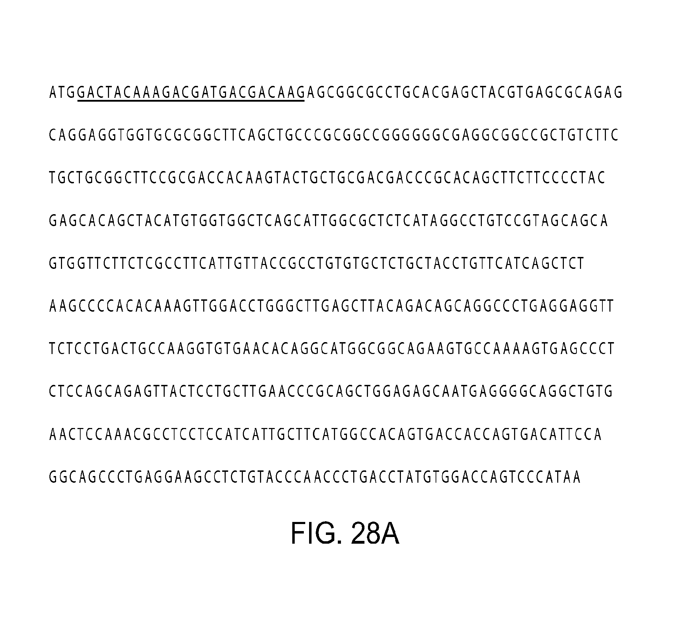

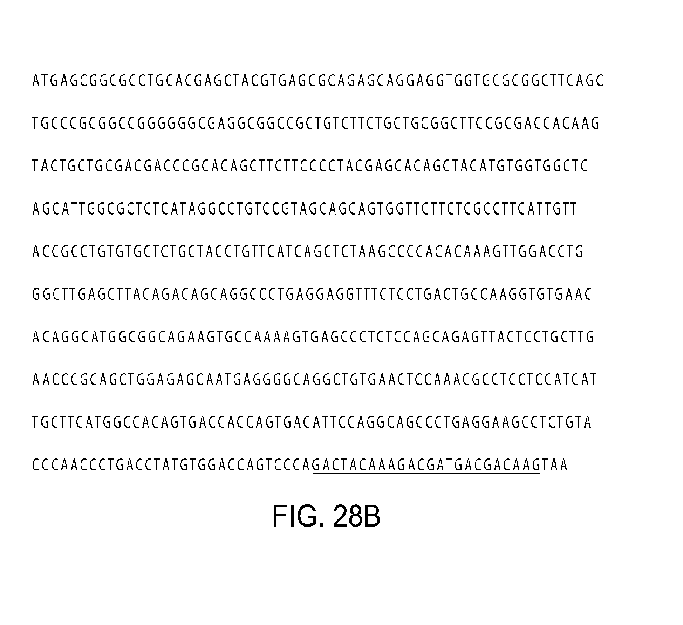

Figures 28A and 28B represent the DNA sequence of FLAG_MGC_T1_P4 - (SEQ ID NO:182) and MGC_T1_P4_FLAG (SEQ ID NO:183) respectively; FLAG sequence is underlined.

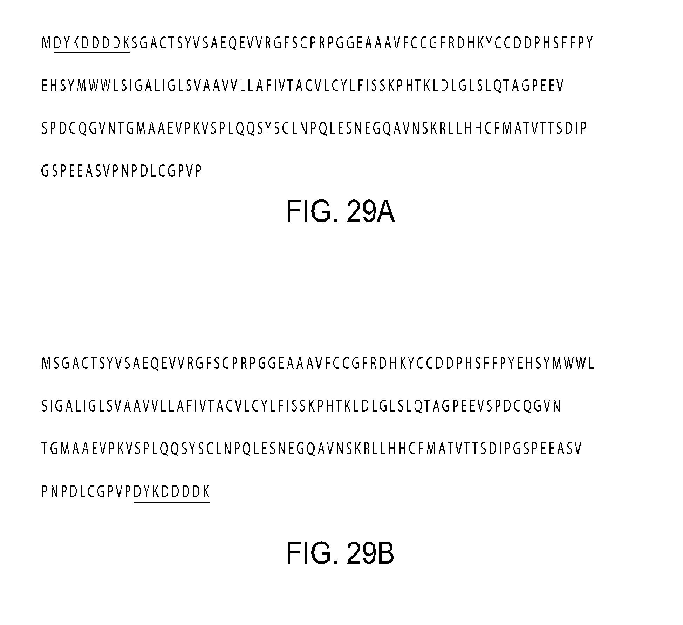

Figures 29A and 29B represent the amino acid sequence of FLAG_MGC_T1_P4 protein (SEQ ID NO:184) and MGC_T1_P4_FLAG (SEQ ID NO:185) respectively; FLAG sequence is underlined.

### EXAMPLE 4_4 DETERMINING CELL LOCALIZATION OF MGC52498 PROTEIN

Determining cell localization of MGC52498 was done using the confocal microscope. MGC_T1_P4_FLAG pIRESpuro3 or FLAG_MGC_T1_P4 pIRESpuro3 constructs described above or pIRESpuro3 empty vector were subsequently transiently transfected into HEK-293T cells as follows:

HEK-293T (ATCC, CRL-11268) cells were plated on sterile glass coverslips, 13mm diameter (Marienfeld, catalog number: 01 115 30), which were placed in a 6 well plate, using 2ml pre-warmed DMEM [Dulbecco's modified Eagle's Media, Biological Industries (Beit Ha'Emek, Israel), cataloge number: 01-055-1A] + 10% FBS (Fetal Bovin Serum) + 4mM L-Glutamine. 500,000 cells per well were transfected with 2□g of the DNA construct using 6µl FuGENE 6 reagent (Roche, catalog number: 11-814-443-001) diluted into 94ul DMEM. The mixture was incubated at room temperature for 15 minutes. The complex mixture was added dropwise to the cells and swirled. Cells were placed in incubator maintained at 37°C with 5% CO2 content.

48 hours post transient transfection, the cells were further processed for analysis in confocal microscopy. The cover slips were washed 3 times in phosphate buffered saline (PBS) and fixed for 15 minutes with a fixing solution composed of 3.7% paraformaldehyde (PFA) (Sigma, catalog number: P-6148) and 3% glucose (Sigma, catalog number: G5767), followed by 5 minutes incubation with 3mM glycine (Sigma, catalog number: G7126). After 1 wash in PBS, cells were permeabilized by incubation with 0.1% triton X-100/PBS solution for 5 minutes. After 2 washes in PBS cells were incubated in 5% bovine serum albumin (BSA) (Sigma, catalog number: A4503) in PBS solution for 20 minutes. The cells were then incubated with anti FLAG antibody conjugated to cy3 (Sigma, catalog number: A9594) diluted 1:100 in 5% BSA in PBS for 1 hr, followed by three 5-minute washes in PBS. The coverslips were then mounted on a slide with Gel Mount Aqueous medium (Sigma, catalog number: G0918) and cells were observed for the presence of fluorescent product using confocal microscopy.

The red fluorescence signal obtained from cells expressing MGC_T1_P4_FLAG (SEQ ID NO:184) or FLAG_MGC_T1_P4 (SEQ ID NO:185), as opposed to the absence of signal obtained from pIRESpuro3 empty vector indicates for ectopic expression of MGC. Notwithstanding, the recombinant protein could not be detected in the cell membrane of HEK 293T cells (data not shown). In order to further understand MGC P4 cell localization, endogenous expression instead of ectopic expression will be tested.

### EXAMPLE 5: FAM70A POLYPEPTIDES AND POLYNUCLEOTIDES, AND USES THEREOF AS A DRUG TARGET FOR PRODUCING DRUGS AND BIOLOGICS

### EXAMPLE 5_1: DESCRIPTION FOR CLUSTER F10649

Cluster F10649 (internal ID 72834556) features 8 transcripts of interest, the names for which are given in Table 25. The selected protein variants are given in table 26.

**Table 25 - Transcripts of interest**

| **Transcript Name** |
|---|
| F10649_T0 (SEQ ID NO:21) |
| F10649_T1 (SEQ ID NO:22) |
| F10649_T4 (SEQ ID NO:24) |
| F10649_T6 (SEQ ID NO:26) |
| F10649_T8 (SEQ ID NO:28) |

**Table 26 - Proteins of interest**

| **Protein Name** | **Corresponding Transcript(s)** |
|---|---|
| F10649_P4 (SEQ ID NO:30) | F10649_T0 (SEQ ID NO:21) |
| F10649_P5 (SEQ ID NO:33) | F10649_T1 (SEQ ID NO:22) |
| F10649_P7 (SEQ ID NO:35) | F10649_T4 (SEQ ID NO:24) |
| F10649_P8 (SEQ ID NO:36) | F10649_T6 (SEQ ID NO:26) |
| F10649_P10 (SEQ ID NO:32) | F10649_T8 (SEQ ID NO:28) |

These sequences are variants of the known hypothetical protein LOC55026 (SEQ ID NO:29) (SwissProt accession identifier NP_060408; synonims: FAM70A).

FAM70A (family with sequence similarity 70, member A) was identified in several large scale studies, such as identification and characterization of putative alternative promoters of human genes (Kimura et al. 2006, Genome Res. 16(1): 55-65), annotation of chromosome X (Ross et al. 2005, Nature 434(7031): 325-37), and full length cDNA projects (Gerhard et al. 2004, Genome Res. 14(10B): 2121-7; Strausberg et al. 2002, PNAS 99(26): 16899-903; Ota et al. 2004, Nat Genet 36(1): 40-5). However no research was published about FAM70A specifically.

Sequences corresponding to F10649_P4 (SEQ ID NO:30) and F10649_P5 (SEQ ID NO:33) were reported in WO2003083039 among other novel polypeptides, and the nucleic acids encoding them, as having properties related to stimulation of biochemical or physiological responses in a cell, a tissue, an organ or an organism. Sequences corresponding to F10649_P4 (SEQ ID NO:30) and F10649_P5 (SEQ ID NO:33) were also reported in EP1293569, among other novel polypeptides, and the nucleic acids encoding them, as being involved in neural cell differentiation. Neither WO2003083039 nor EP1293569 teach or suggest, however, that sequences corresponding to F10649_P4 (SEQ ID NO:30) and F10649_P5 (SEQ ID NO:33) are differentially expressed in Multiple Myeloma, kidney cancer, lung cancer, liver cancer, and breast cancer. Also, there is no teaching or suggestion in these applications that F10649_P4 (SEQ ID NO:30) and F10649_P5 (SEQ ID NO:33) can be used as drug target for treatment of cancer, especially for treatment of Multiple Myeloma, kidney cancer, lung cancer, liver cancer, and breast cancer, and/or immune related conditions, or for diagnosis thereof. Also, there is no teaching or suggestion in these applications that antibodies specific for F10649_P4 (SEQ ID NO:30), F10649_P5 (SEQ ID NO:33), its soluble ectodomain, and/or fragments thereof can be used as therapeutics for treatment of cancer, especially for treatment of Multiple Myeloma, kidney cancer, lung cancer, liver cancer, and breast cancer, and/or immune related conditions, or for diagnosis thereof.

A sequence corresponding to F10649_P10 (SEQ ID NO:32) was reported in WO2003057160, WO200222660, WO2004039956 and WO2004041170, among many other polypeptides related to cancer and/or immune related diseases. In contrary to the present application, WO2003057160 demonstrates deregulation of the sequence corresponding to F10649_P10 in kidney tumor as compared to normal kidney tissues. None of the WO2003057160, WO200222660, WO2004039956 and WO2004041170 applications teaches or suggests that the sequence corresponding to F10649_P10 can be used as drug target for treatment of Multiple Myeloma, kidney cancer, lung cancer, liver cancer, and breast cancer, and/or immune related conditions, or for diagnosis thereof. Also, there is no teaching or suggestion in these applications that antibodies specific to F10649_P10, its soluble ectodomain, and/or fragments thereof can be used as therapeutics for treatment of cancer, especially for treatment of Multiple Myeloma, kidney cancer, lung cancer, liver cancer, and breast cancer, or for diagnosis thereof.

By contrast and surprisingly, the present inventors have found that FAM70A antigen and discrete portions thereof may optionally be used as a drug target for therapeutic small molecules, peptides, antibodies, antisense RNAs, siRNAs, ribozymes, and the like. Diagnostic and therapeutic polyclonal and monoclonal antibodies and fragments thereof that bind FAM70A, and portions and variants thereof, may optionally be produced. According to at least some embodiments of the present invention, there is provided the use of antibodies and antibody fragments against FAM70A antigen, its secreted or soluble form or ECD and/or variants, conjugates, or fragments thereof and fragments and variants thereof for treating and diagnosing cancer, especially for treatment of Multiple Myeloma, kidney cancer, lung cancer, liver cancer, and breast cancer, and/or immune related conditions, wherein this antigen is differentially expressed.

As noted above, cluster F10649 features 8 transcripts, which were listed in Table 25 above. These transcripts encode for proteins which are variants of protein hypothetical protein LOC55026 (SEQ ID NO:29). A description of each variant protein according to at least some embodiments of the invention is now provided.

Variant protein F10649_P4 (SEQ ID NO:30) according to at least some embodiments of the present invention has an amino acid sequence as encoded by transcript F10649_T0 (SEQ ID NO:21). A description of the relationship of the variant protein according to at least some embodiments of the invention to known proteins is as follows:
1. Comparison report between F10649_P4 (SEQ ID NO:30) and known protein Q7Z4S8_HUMAN (SEQ ID NO:31) :
   A. An isolated chimeric polypeptide, comprising a first amino acid sequence being at least 90% homologous to MHQSLTQQRSSDMSLPDSMGAFNRRKRNSIYVTVTLLIVSVLILTVGLAATTRTQN VTVGGYYPGVILGFGSFLGIIGSNLIENKRQMLVASIVFISFGVIAAFCCAIVDGVFAA RHIDLKPLYANRCHYVPKTSQKEAEEVISSSTKNSPSTRVMRNLTQAAREVNCPHLS REFCTPRIRGNTCFCCDLYNCGNRVEITGGYYEYIDVSSCQDIIHLYHLLWSATILNI VGLFLGIITAAVLGGFKDMNPTLPALNCSVENTHPTVSYYAHPQVASYNTYYHSPP HLPPYSAYDFQHSGVFPSSPPSGLSDEPQSAS corresponding to amino acids 1 - 318 of known protein Q7Z4S8_HUMAN (SEQ ID NO:31), which also corresponds to amino acids 1 - 318 of F10649_P4 (SEQ ID NO:30), a bridging amino acid P corresponding to amino acid 319 of F10649_P4 (SEQ ID NO:30), and a second amino acid sequence being at least 90% homologous to SPSYMWSSSAPPRYSPPYYPPFEKPPPYSP corresponding to amino acids 320 - 349 of known protein Q7Z4S8_HUMAN (SEQ ID NO:31), which also corresponds to amino acids 320 - 349 of F10649_P4 (SEQ ID NO:30), wherein said first amino acid sequence, bridging amino acid and second amino acid sequence are contiguous and in a sequential order.
2. Comparison report between F10649_P4 (SEQ ID NO:30) and known proteins NP_060408 and Q86Y72_HUMAN:
   A. An isolated chimeric polypeptide, comprising a first amino acid sequence being at least 90% homologous to MHQSLTQQRSSDMSLPDSMGAFNRRKRNSIYVTVTLLIVSVLILTVGLAATTRTQN VTVGGYYPGVILGFGSFLGIIGSNLIENKRQMLVASIVFISFGVIAAFCCAIVDGVFAA RHIDLKPLYANRCHYVPKTSQKEAEEVISSSTKNSPSTRVMRNLTQAAREVNCPHLS REFCTPRIRGNTCFCCDLYNCGNRVEITGGYYEYIDVSSCQDIIHLYHLLWSATILNI VGLFLGIITAAVLGGFKDMNPTLPALNCSVENTHPTVSYYAHPQVASYNTYYHSPP HLPPYSAYDFQHSGVFPSSPPSGLSDEPQSASPSPSYMWSSSAPPRYSPPYYPPFEKP corresponding to amino acids 1 - 344 of known proteins NP_060408 and Q86Y72_HUMAN, which also corresponds to amino acids 1 - 344 of F10649_P4 (SEQ ID NO:30), a bridging amino acid P corresponding to amino acid 345 of F10649_P4 (SEQ ID NO:30), and a second amino acid sequence being at least 90% homologous to PYSP corresponding to amino acids 346 - 349 of known proteins NP_060408 and Q86Y72_HUMAN, which also corresponds to amino acids 346 - 349 of F10649_P4 (SEQ ID NO:30), wherein said first amino acid sequence, bridging amino acid and second amino acid sequence are contiguous and in a sequential order.
3. Comparison report between F10649_P4 (SEQ ID NO:30) and known protein Q9NWN8_HUMAN (SEQ ID NO:32) :
   A. An isolated chimeric polypeptide, comprising a first amino acid sequence being at least 70%, optionally at least 80%, preferably at least 85%, more preferably at least 90% and most preferably at least 95%, homologous to a polypeptide having the sequence MHQSLTQQRSSDMSLPDSMGAFNRRKRNSIYVTVTLLIVSVLILTVGLAATTRTQN VTVGGYYPGVILGFGSFLGIIGSNLIENKRQMLVASIVFISFGVIAAFCCAIVDGVFAA RHIDLKPLYANRCHYVPKTSQKEAEEVISSSTKNSPSTRVMRNLTQAARE (SEQ ID NO: 155) corresponding to amino acids 1 - 165 of F10649_P4 (SEQ ID NO:30), and a second amino acid sequence being at least 90% homologous to VNCPHLSREFCTPRIRGNTCFCCDLYNCGNRVEITGGYYEYIDVSSCQDIIHLYHLL WSATILNIVGLFLGIITAAVLGGFKDMNPTLPALNCSVENTHPTVSYYAHPQVASYN TYYHSPPHLPPYSAYDFQHSGVFPSSPPSGLSDEPQSASPSPSYMWSSSAPPRYSPPY YPPFEKPPPYSP corresponding to amino acids 2 - 185 of known protein Q9NWN8_HUMAN (SEQ ID NO:32), which also corresponds to amino acids 166 - 349 of F10649_P4 (SEQ ID NO:30), wherein said first amino acid sequence and second amino acid sequence are contiguous and in a sequential order.
   B. An isolated polypeptide corresponding to a head of F10649_P4 (SEQ ID NO:30), comprising a polypeptide being at least 70%, optionally at least about 80%, preferably at least about 85%, more preferably at least about 90% and most preferably at least about 95% homologous to the sequence MHQSLTQQRSSDMSLPDSMGAFNRRKRNSIYVTVTLLIVSVLILTVGLAATTRTQN VTVGGYYPGVILGFGSFLGIIGSNLIENKRQMLVASIVFISFGVIAAFCCAIVDGVFAA RHIDLKPLYANRCHYVPKTSQKEAEEVISSSTKNSPSTRVMRNLTQAARE (SEQ ID NO: 155) of F10649_P4 (SEQ ID NO:30).

The localization of the variant protein was determined according to results from a number of different software programs and analyses, including analyses from SignalP and other specialized programs. The variant protein is believed to be located as follows with regard to the cell: membrane.

Variant protein F10649_P4 (SEQ ID NO:30) also has the following non-silent SNPs (Single Nucleotide Polymorphisms) as listed in Table 27, (given according to their position(s) on the amino acid sequence, with the alternative amino acid(s) listed).

**Table 27 - Amino acid mutations**

| **SNP position(s) on amino acid sequence** | **Alternative amino acid(s)** |
|---|---|
| 127 | R->P |
| 127 | R -> Q |
| 319 | P -> S |

The coding portion of transcript F10649_T0 (SEQ ID NO:21) starts at position 248 and ends at position 1294. The transcript also has the following SNPs as listed in Table 28 (given according to their position on the nucleotide sequence, with the alternative nucleic acid listed).

**Table 28 - Nucleic acid SNPs**

| **Polymorphism** | **SNP position(s) on nucleaotide sequence** |
|---|---|
| C -> | 95 |
| G -> A | 627 |
| G -> C | 627 |
| C -> T | 1202 |
| A -> C | 1282, 3227 |
| C -> A | 1523, 1826 |
| C -> G | 1523, 1826 |
| A -> | 1820 |
| T -> A | 2423 |
| T -> G | 2423 |
| T -> C | 2433 |
| A -> T | 3227 |

Variant protein F10649_P5 (SEQ ID NO:33) according to at least some embodiments of the invention has an amino acid sequence encoded by transcript F10649_T1 (SEQ ID NO:22). A description of the relationship of the variant protein according to at least some embodiments of the invention to known proteins is as follows:
1. Comparison report between F10649_P5 (SEQ ID NO:33) and known protein Q5JRV8_HUMAN (SEQ ID NO:30) :
   A. An isolated chimeric polypeptide, comprising a first amino acid sequence being at least 90% homologous to MHQSLTQQRSSDMSLPDSMGAFNRRKRNSIYVTVTLLIVSVLILTVGLAATTRTQN VTVGGYYPGVILGFGSFLGIIGSNLIENKRQMLVASIVFISFGVIAAFCCAIVDGVFAA RHIDLKPLYANRCHYVPKTSQKEAEE (SEQ ID NO: 156) corresponding to amino acids 1 - 141 of known protein Q5JRV8_HUMAN (SEQ ID NO:30), which also corresponds to amino acids 1 - 141 of F10649_P5 (SEQ ID NO:33), and a second amino acid sequence being at least 90% homologous to VNCPHLSREFCTPRIRGNTCFCCDLYNCGNRVEITGGYYEYIDVSSCQDIIHLYHLL WSATILNIVGLFLGIITAAVLGGFKDMNPTLPALNCSVENTHPTVSYYAHPQVASYN TYYHSPPHLPPYSAYDFQHSGVFPSSPPSGLSDEPQSASPSPSYMWSSSAPPRYSPPY YPPFEKPPPYSP corresponding to amino acids 166 - 349 of known protein Q5JRV8_HUMAN (SEQ ID NO:30), which also corresponds to amino acids 142 - 325 of F10649_P5 (SEQ ID NO:33), wherein said first amino acid sequence and second amino acid sequence are contiguous and in a sequential order.
   B. An isolated chimeric polypeptide corresponding to an edge portion of F10649_P5 (SEQ ID NO:33), comprising a polypeptide having a length "n", wherein n is at least about 10 amino acids in length, optionally at least about 20 amino acids in length, preferably at least about 30 amino acids in length, more preferably at least about 40 amino acids in length and most preferably at least about 50 amino acids in length, wherein at least two amino acids comprise EV, having a structure as follows: a sequence starting from any of amino acid numbers 141-x to 141; and ending at any of amino acid numbers 142 + ((n-2) - x), in which x varies from 0 to n-2.
2. Comparison report between F10649_P5 (SEQ ID NO:33) and known protein Q7Z4S8_HUMAN (SEQ ID NO:31) :
   A. An isolated chimeric polypeptide, comprising a first amino acid sequence being at least 90% homologous to MHQSLTQQRSSDMSLPDSMGAFNRRKRNSIYVTVTLLIVSVLILTVGLAATTRTQN VTVGGYYPGVILGFGSFLGIIGSNLIENKRQMLVASIVFISFGVIAAFCCAIVDGVFAA RHIDLKPLYANRCHYVPKTSQKEAEE (SEQ ID NO: 156) corresponding to amino acids 1 - 141 of known protein Q7Z4S8_HUMAN (SEQ ID NO:31), which also corresponds to amino acids 1 - 141 of F10649_P5 (SEQ ID NO:33), a second amino acid sequence being at least 90% homologous to VNCPHLSREFCTPRIRGNTCFCCDLYNCGNRVEITGGYYEYIDVSSCQDIIHLYHLL WSATILNIVGLFLGIITAAVLGGFKDMNPTLPALNCSVENTHPTVSYYAHPQVASYN TYYHSPPHLPPYSAYDFQHSGVFPSSPPSGLSDEPQSAS corresponding to amino acids 166 - 318 of known protein Q7Z4S8_HUMAN (SEQ ID NO:31), which also corresponds to amino acids 142 - 294 of F10649_P5 (SEQ ID NO:33), a bridging amino acid P corresponding to amino acid 295 of F10649_P5 (SEQ ID NO:33), and a third amino acid sequence being at least 90% homologous to SPSYMWSSSAPPRYSPPYYPPFEKPPPYSP corresponding to amino acids 320 - 349 of known protein Q7Z4S8_HUMAN (SEQ ID NO:31), which also corresponds to amino acids 296 - 325 of F10649_P5 (SEQ ID NO:33), wherein said first amino acid sequence, second amino acid sequence, bridging amino acid and third amino acid sequence are contiguous and in a sequential order.
   B. An isolated chimeric polypeptide corresponding to an edge portion of F10649_P5 (SEQ ID NO:33), comprising a polypeptide having a length "n", wherein n is at least about 10 amino acids in length, optionally at least about 20 amino acids in length, preferably at least about 30 amino acids in length, more preferably at least about 40 amino acids in length and most preferably at least about 50 amino acids in length, wherein at least two amino acids comprise EV, having a structure as follows: a sequence starting from any of amino acid numbers 141-x to 141; and ending at any of amino acid numbers 142 + ((n-2) - x), in which x varies from 0 to n-2.
3. Comparison report between F10649_P5 (SEQ ID NO:33) and known proteins NP_060408 and Q86Y72_HUMAN (SEQ ID NO:29):
   A. An isolated chimeric polypeptide, comprising a first amino acid sequence being at least 90% homologous to MHQSLTQQRSSDMSLPDSMGAFNRRKRNSIYVTVTLLIVSVLILTVGLAATTRTQN VTVGGYYPGVILGFGSFLGIIGSNLIENKRQMLVASIVFISFGVIAAFCCAIVDGVFAA RHIDLKPLYANRCHYVPKTSQKEAEE (SEQ ID NO: 156) corresponding to amino acids 1 - 141 of known proteins NP_060408 and Q86Y72_HUMAN (SEQ ID NO:29), which also corresponds to amino acids 1 - 141 of F10649_P5 (SEQ ID NO:33), a second amino acid sequence being at least 90% homologous to VNCPHLSREFCTPRIRGNTCFCCDLYNCGNRVEITGGYYEYIDVSSCQDIIHLYHLL WSATILNIVGLFLGIITAAVLGGFKDMNPTLPALNCSVENTHPTVSYYAHPQVASYN TYYHSPPHLPPYSAYDFQHSGVFPSSPPSGLSDEPQSASPSPSYMWSSSAPPRYSPPY YPPFEKP corresponding to amino acids 166 - 344 of known proteins NP_060408 and Q86Y72_HUMAN (SEQ ID NO:29), which also corresponds to amino acids 142 - 320 of F10649_P5 (SEQ ID NO:33), a bridging amino acid P corresponding to amino acid 321 of F10649_P5 (SEQ ID NO:33), and a third amino acid sequence being at least 90% homologous to PYSP corresponding to amino acids 346 - 349 of known proteins NP_060408 and Q86Y72_HUMAN, which also corresponds to amino acids 322 - 325 of F10649_P5 (SEQ ID NO:33), wherein said first amino acid sequence, second amino acid sequence, bridging amino acid and third amino acid sequence are contiguous and in a sequential order.
   B. An isolated chimeric polypeptide corresponding to an edge portion of F10649_P5 (SEQ ID NO:33), comprising a polypeptide having a length "n", wherein n is at least about 10 amino acids in length, optionally at least about 20 amino acids in length, preferably at least about 30 amino acids in length, more preferably at least about 40 amino acids in length and most preferably at least about 50 amino acids in length, wherein at least two amino acids comprise EV, having a structure as follows: a sequence starting from any of amino acid numbers 141-x to 141; and ending at any of amino acid numbers 142 + ((n-2) - x), in which x varies from 0 to n-2.
4. Comparison report between F10649_P5 (SEQ ID NO:33) and known protein Q9NWN8_HUMAN (SEQ ID NO:32) :
   A. An isolated chimeric polypeptide, comprising a first amino acid sequence being at least 70%, optionally at least 80%, preferably at least 85%, more preferably at least 90% and most preferably at least 95%, homologous to a polypeptide having the sequence MHQSLTQQRSSDMSLPDSMGAFNRRKRNSIYVTVTLLIVSVLILTVGLAATTRTQN VTVGGYYPGVILGFGSFLGIIGSNLIENKRQMLVASIVFISFGVIAAFCCAIVDGVFAA RHIDLKPLYANRCHYVPKTSQKEAEE (SEQ ID NO: 156) corresponding to amino acids 1 - 141 of F10649_P5 (SEQ ID NO:33), and a second amino acid sequence being at least 90% homologous to VNCPHLSREFCTPRIRGNTCFCCDLYNCGNRVEITGGYYEYIDVSSCQDIIHLYHLL WSATILNIVGLFLGIITAAVLGGFKDMNPTLPALNCSVENTHPTVSYYAHPQVASYN TYYHSPPHLPPYSAYDFQHSGVFPSSPPSGLSDEPQSASPSPSYMWSSSAPPRYSPPY YPPFEKPPPYSP corresponding to amino acids 2 - 185 of known protein Q9NWN8_HUMAN (SEQ ID NO:32), which also corresponds to amino acids 142 - 325 of F10649_P5 (SEQ ID NO:33), wherein said first amino acid sequence and second amino acid sequence are contiguous and in a sequential order.
   B. An isolated polypeptide corresponding to a head of F10649_P5 (SEQ ID NO:33), comprising a polypeptide being at least 70%, optionally at least about 80%, preferably at least about 85%, more preferably at least about 90% and most preferably at least about 95% homologous to the sequence MHQSLTQQRSSDMSLPDSMGAFNRRKRNSIYVTVTLLIVSVLILTVGLAATTRTQN VTVGGYYPGVILGFGSFLGIIGSNLIENKRQMLVASIVFISFGVIAAFCCAIVDGVFAA RHIDLKPLYANRCHYVPKTSQKEAEE (SEQ ID NO: 156) of F10649_P5 (SEQ ID NO:33).

The localization of the variant protein was determined according to results from a number of different software programs and analyses, including analyses from SignalP and other specialized programs. The variant protein is believed to be located as follows with regard to the cell: membrane.

Variant protein F10649_P5 (SEQ ID NO:33) also has the following non-silent SNPs (Single Nucleotide Polymorphisms) as listed in Table 29, (given according to their position(s) on the amino acid sequence, with the alternative amino acid(s) listed).

**Table 29 - Amino acid mutations**

| **SNP position(s) on amino acid sequence** | **Alternative amino acid(s)** |
|---|---|
| 127 | R → P |
| 127 | R → Q |
| 295 | P → S |

The coding portion of transcript F10649_T1 (SEQ ID NO:22) starts at position 248 and ends at position 1222. The transcript also has the following SNPs as listed in Table 30 (given according to their position on the nucleotide sequence, with the alternative nucleic acid listed).

**Table 30 - Nucleic acid SNPs**

| **Polymorphism** | **SNP position(s) on nucleotide sequence** |
|---|---|
| C → | 95 |
| G → A | 627 |
| G → C | 627 |
| C → T | 1130 |
| A → C | 1210, 3155 |
| C → A | 1451, 1754 |
| C → G | 1451, 1754 |
| A → | 1748 |
| T → A | 2351 |
| T → G | 2351 |
| T → C | 2361 |
| A → T | 3155 |

Variant protein F10649_P7 (SEQ ID NO:35) according to at least some embodiments of the invention has an amino acid sequence as encoded by transcripts F10649_T4 (SEQ ID NO:24). A description of the relationship of the variant protein according to at least some embodiments of the invention to known proteins is as follows:
1. Comparison report between F10649_P7 (SEQ ID NO:35) and known protein Q5JRV8_HUMAN (SEQ ID NO:30) :
   A. An isolated chimeric polypeptide, comprising a first amino acid sequence being at least 90% homologous to MHQSLTQQRSSDMSLPDSMGAFNRRKRNSIYVTVTLLIVSVLILTVGLAATTRTQN VTVGGYYPGVILGFGSFLGIIGSNLIENKRQMLVASIVFISFGVIAAFCCAIVDGVFAA RHIDLKPLYANRCHYVPKTSQKEAEE (SEQ ID NO: 156) corresponding to amino acids 1 - 141 of known protein Q5JRV8_HUMAN (SEQ ID NO:30), which also corresponds to amino acids 1 - 141 of F10649_P7 (SEQ ID NO:35), and a second amino acid sequence being at least 90% homologous to NPTLPALNCSVENTHPTVSYYAHPQVASYNTYYHSPPHLPPYSAYDFQHSGVFPSSP PSGLSDEPQSASPSPSYMWSSSAPPRYSPPYYPPFEKPPPYSP corresponding to amino acids 250 - 349 of known protein Q5JRV8_HUMAN (SEQ ID NO:30), which also corresponds to amino acids 142 - 241 of F10649_P7 (SEQ ID NO:35), wherein said first amino acid sequence and second amino acid sequence are contiguous and in a sequential order.
   B. An isolated chimeric polypeptide corresponding to an edge portion of F10649_P7 (SEQ ID NO:35), comprising a polypeptide having a length "n", wherein n is at least about 10 amino acids in length, optionally at least about 20 amino acids in length, preferably at least about 30 amino acids in length, more preferably at least about 40 amino acids in length and most preferably at least about 50 amino acids in length, wherein at least two amino acids comprise EN, having a structure as follows: a sequence starting from any of amino acid numbers 141-x to 141; and ending at any of amino acid numbers 142 + ((n-2) - x), in which x varies from 0 to n-2.
2. Comparison report between F10649_P7 (SEQ ID NO:35) and known protein Q9NWN8_HUMAN (SEQ ID NO:32):
   A. An isolated chimeric polypeptide, comprising a first amino acid sequence being at least 70%, optionally at least 80%, preferably at least 85%, more preferably at least 90% and most preferably at least 95%, homologous to a polypeptide having the sequence MHQSLTQQRSSDMSLPDSMGAFNRRKRNSIYVTVTLLIVSVLILTVGLAATTRTQN VTVGGYYPGVILGFGSFLGIIGSNLIENKRQMLVASIVFISFGVIAAFCCAIVDGVFAA RHIDLKPLYANRCHYVPKTSQKEAEE (SEQ ID NO: 156) corresponding to amino acids 1 - 141 of F10649_P7 (SEQ ID NO:35), and a second amino acid sequence being at least 90% homologous to NPTLPALNCSVENTHPTVSYYAHPQVASYNTYYHSPPHLPPYSAYDFQHSGVFPSSP PSGLSDEPQSASPSPSYMWSSSAPPRYSPPYYPPFEKPPPYSP corresponding to amino acids 86 - 185 of known protein Q9NWN8_HUMAN (SEQ ID NO:32), which also corresponds to amino acids 142 - 241 of F10649_P7 (SEQ ID NO:35), wherein said first amino acid sequence and second amino acid sequence are contiguous and in a sequential order.
   B. An isolated polypeptide corresponding to a head portion of F10649_P7 (SEQ ID NO:35), comprising a polypeptide being at least 70%, optionally at least about 80%, preferably at least about 85%, more preferably at least about 90% and most preferably at least about 95% homologous to the sequence MHQSLTQQRSSDMSLPDSMGAFNRRKRNSIYVTVTLLIVSVLILTVGLAATTRTQN VTVGGYYPGVILGFGSFLGIIGSNLIENKRQMLVASIVFISFGVIAAFCCAIVDGVFAA RHIDLKPLYANRCHYVPKTSQKEAEE (SEQ ID NO: 156) of F10649_P7 (SEQ ID NO:35).

The localization of the variant protein was determined according to results from a number of different software programs and analyses, including analyses from SignalP and other specialized programs. The variant protein is believed to be located as follows with regard to the cell: membrane.

Variant protein F10649_P7 (SEQ ID NO:35) also has the following non-silent SNPs (Single Nucleotide Polymorphisms) as listed in Table 31, (given according to their position(s) on the amino acid sequence, with the alternative amino acid(s) listed).

**Table 31 - Amino acid mutations**

| **SNP position(s) on amino acid sequence** | **Alternative amino acid(s)** |
|---|---|
| 127 | R → P |
| 127 | R → Q |
| 211 | P → S |

The coding portion of transcript F10649_T4 (SEQ ID NO:24) starts at position 248 and ends at position 970. The transcript also has the following SNPs as listed in Table 32 (given according to their position on the nucleotide sequence, with the alternative nucleic acid listed).

**Table 32 - Nucleic acid SNPs**

| **Polymorphism** | **SNP position(s) on nucleotide sequence** |
|---|---|
| C → | 95 |
| G → A | 627 |
| G → C | 627 |
| C → T | 878 |
| A → C | 958, 2903 |
| C → A | 1199, 1502 |
| C → G | 1199, 1502 |
| A → | 1496 |
| T → A | 2099 |
| T → G | 2099 |
| T → C | 2109 |
| A → T | 2903 |

Variant protein F10649_P8 (SEQ ID NO:36) according to at least some embodiments of the invention has an amino acid sequence as encoded by transcript F10649_T6 (SEQ ID NO:26). A description of the relationship of the variant protein according to at least some embodiments of the invention to known proteins is as follows:
1. Comparison report between F10649_P8 (SEQ ID NO:36) and known protein Q5JRV8_HUMAN (SEQ ID NO:30):
   A. An isolated chimeric polypeptide, comprising a first amino acid sequence being at least 90% homologous to MHQSLTQQRSSDMSLPDSMGAFNRRKRNSIYVTVTLLIVSVLILTVGLAATTRTQN VTVGGYYPGVI (SEQ ID NO: 159), corresponding to amino acids 1 - 67 of known protein Q5JRV8_HUMAN (SEQ ID NO:30), which also corresponds to amino acids 1 - 67 of F10649_P8 (SEQ ID NO:36), and a second amino acid sequence being at least 90% homologous to LVASIVFISFGVIAAFCCAIVDGVFAARHIDLKPLYANRCHYVPKTSQKEAEEVISSS TKNSPSTRVMRNLTQAAREVNCPHLSREFCTPRIRGNTCFCCDLYNCGNRVEITGG YYEYIDVSSCQDIIHLYHLLWSATILNIVGLFLGIITAAVLGGFKDMNPTLPALNCSV ENTHPTVSYYAHPQVASYNTYYHSPPHLPPYSAYDFQHSGVFPSSPPSGLSDEPQSA SPSPSYMWSSSAPPRYSPPYYPPFEKPPPYSP corresponding to amino acids 89 - 349 of known protein Q5JRV8_HUMAN (SEQ ID NO:30), which also corresponds to amino acids 68 - 328 of F10649_P8 (SEQ ID NO:36), wherein said first amino acid sequence and second amino acid sequence are contiguous and in a sequential order.
   B. An isolated chimeric polypeptide corresponding to an edge portion of F10649_P8 (SEQ ID NO:36), comprising a polypeptide having a length "n", wherein n is at least about 10 amino acids in length, optionally at least about 20 amino acids in length, preferably at least about 30 amino acids in length, more preferably at least about 40 amino acids in length and most preferably at least about 50 amino acids in length, wherein at least two amino acids comprise IL, having a structure as follows: a sequence starting from any of amino acid numbers 67-x to 67; and ending at any of amino acid numbers 68 + ((n-2) - x), in which x varies from 0 to n-2.
2. Comparison report between F10649_P8 (SEQ ID NO:36) and known protein Q9NWN8_HUMAN (SEQ ID NO:32):
   A. An isolated chimeric polypeptide, comprising a first amino acid sequence being at least 70%, optionally at least 80%, preferably at least 85%, more preferably at least 90% and most preferably at least 95%, homologous to a polypeptide having the sequence MHQSLTQQRSSDMSLPDSMGAFNRRKRNSIYVTVTLLIVSVLILTVGLAATTRTQN VTVGGYYPGVILVASIVFISFGVIAAFCCAIVDGVFAARHIDLKPLYANRCHYVPKT SQKEAEEVISSSTKNSPSTRVMRNLTQAARE (SEQ ID NO: 159) corresponding to amino acids 1 - 144 of F10649_P8 (SEQ ID NO:36), and a second amino acid sequence being at least 90% homologous to VNCPHLSREFCTPRIRGNTCFCCDLYNCGNRVEITGGYYEYIDVSSCQDIIHLYHLL WSATILNIVGLFLGIITAAVLGGFKDMNPTLPALNCSVENTHPTVSYYAHPQVASYN TYYHSPPHLPPYSAYDFQHSGVFPSSPPSGLSDEPQSASPSPSYMWSSSAPPRYSPPY YPPFEKPPPYSP corresponding to amino acids 2 - 185 of known protein Q9NWN8_HUMAN (SEQ ID NO:32), which also corresponds to amino acids 145 - 328 of F10649_P8 (SEQ ID NO:36), wherein said first amino acid sequence and second amino acid sequence are contiguous and in a sequential order.
   B. An isolated polypeptide corresponding to a head portion of F10649_P8 (SEQ ID NO:36), comprising a polypeptide being at least 70%, optionally at least about 80%, preferably at least about 85%, more preferably at least about 90% and most preferably at least about 95% homologous to the sequence MHQSLTQQRSSDMSLPDSMGAFNRRKRNSIYVTVTLLIVSVLILTVGLAATTRTQN VTVGGYYPGVILVASIVFISFGVIAAFCCAIVDGVFAARHIDLKPLYANRCHYVPKT SQKEAEEVISSSTKNSPSTRVMRNLTQAARE (SEQ ID NO: 159) of F10649_P8 (SEQ ID NO:36).

The localization of the variant protein was determined according to results from a number of different software programs and analyses, including analyses from SignalP and other specialized programs. The variant protein is believed to be located as follows with regard to the cell: membrane.

Variant protein F10649_P8 (SEQ ID NO:36) also has the following non-silent SNPs (Single Nucleotide Polymorphisms) as listed in Table 33, (given according to their position(s) on the amino acid sequence, with the alternative amino acid(s) listed).

**Table 33 - Amino acid mutations**

| **SNP position(s) on amino acid sequence** | **Alternative amino acid(s)** |
|---|---|
| 106 | R → P |
| 106 | R → Q |
| 298 | P → S |

The coding portion of transcript F10649_T6 (SEQ ID NO:26) starts at position 248 and ends at position 1231. The transcript also has the following SNPs as listed in Table 34 (given according to their position on the nucleotide sequence, with the alternative nucleic acid listed).

**Table 34 - Nucleic acid SNPs**

| **Polymorphism** | **SNP Position(s) on nucleotide sequence** |
|---|---|
| C → | 95 |
| G → A | 564 |
| G → C | 564 |
| C → T | 1139 |
| A → C | 1219, 3164 |
| C → A | 1460, 1763 |
| C → G | 1460, 1763 |
| A → | 1757 |
| T → A | 2360 |
| T → G | 2360 |
| T → C | 2370 |
| A → T | 3164 |

Variant protein F10649_P10 (SEQ ID NO:32) according to at least some embodiments of the invention has an amino acid sequence as encoded by transcript) F10649_T8 (SEQ ID NO:28). A description of the relationship of the variant protein according to at least some embodiments of the invention to known proteins is as follows:
1. Comparison report between F10649_P10 (SEQ ID NO:32) and known protein Q5JRV8_HUMAN (SEQ ID NO:30):
   A. An isolated chimeric polypeptide, comprising a first amino acid sequence being at least 70%, optionally at least 80%, preferably at least 85%, more preferably at least 90% and most preferably at least 95% homologous to a polypeptide having the sequence M corresponding to amino acids 1 - 1 of F10649_P10 (SEQ ID NO:32), and a second amino acid sequence being at least 90% homologous to VNCPHLSREFCTPRIRGNTCFCCDLYNCGNRVEITGGYYEYIDVSSCQDIIHLYHLL WSATILNIVGLFLGIITAAVLGGFKDMNPTLPALNCSVENTHPTVSYYAHPQVASYN TYYHSPPHLPPYSAYDFQHSGVFPSSPPSGLSDEPQSASPSPSYMWSSSAPPRYSPPY YPPFEKPPPYSP corresponding to amino acids 166 - 349 of known protein Q5JRV8_HUMAN (SEQ ID NO:30), which also corresponds to amino acids 2 - 185 of F10649_P10 (SEQ ID NO:32), wherein said first amino acid sequence and second amino acid sequence are contiguous and in a sequential order.
2. Comparison report between F10649_P10 (SEQ ID NO:32) and known protein Q7Z4S8_HUMAN (SEQ ID NO:31):
   A. An isolated chimeric polypeptide, comprising a first amino acid sequence being at least 70%, optionally at least 80%, preferably at least 85%, more preferably at least 90% and most preferably at least 95% homologous to a polypeptide having the sequence M corresponding to amino acids 1 - 1 of F10649_P10 (SEQ ID NO:32), a second amino acid sequence being at least 90% homologous to VNCPHLSREFCTPRIRGNTCFCCDLYNCGNRVEITGGYYEYIDVSSCQDIIHLYHLL WSATILNIVGLFLGIITAAVLGGFKDMNPTLPALNCSVENTHPTVSYYAHPQVASYN TYYHSPPHLPPYSAYDFQHSGVFPSSPPSGLSDEPQSAS corresponding to amino acids 166 - 318 of known protein Q7Z4S8_HUMAN (SEQ ID NO:31), which also corresponds to amino acids 2 - 154 of F10649_P10 (SEQ ID NO:32), a bridging amino acid P corresponding to amino acid 155 of F10649_P10 (SEQ ID NO:32), and a third amino acid sequence being at least 90% homologous to SPSYMWSSSAPPRYSPPYYPPFEKPPPYSP corresponding to amino acids 320 - 349 of known protein Q7Z4S8_HUMAN (SEQ ID NO:31), which also corresponds to amino acids 156 - 185 of F10649_P10 (SEQ ID NO:32), wherein said first amino acid sequence, second amino acid sequence, bridging amino acid and third amino acid sequence are contiguous and in a sequential order.
3. Comparison report between F10649_P10 (SEQ ID NO:32) and known proteins NP_060408 and Q86Y72_HUMAN :
   A. An isolated chimeric polypeptide, comprising a first amino acid sequence being at least 70%, optionally at least 80%, preferably at least 85%, more preferably at least 90% and most preferably at least 95% homologous to a polypeptide having the sequence M corresponding to amino acids 1 - 1 of F10649_P10 (SEQ ID NO:32), a second amino acid sequence being at least 90% homologous to VNCPHLSREFCTPRIRGNTCFCCDLYNCGNRVEITGGYYEYIDVSSCQDIIHLYHLL WSATILNIVGLFLGIITAAVLGGFKDMNPTLPALNCSVENTHPTVSYYAHPQVASYN TYYHSPPHLPPYSAYDFQHSGVFPSSPPSGLSDEPQSASPSPSYMWSSSAPPRYSPPY YPPFEKP corresponding to amino acids 166 - 344 of known proteins NP_060408 and Q86Y72_HUMAN, which also corresponds to amino acids 2 - 180 of F10649_P10 (SEQ ID NO:32), a bridging amino acid P corresponding to amino acid 181 of F10649_P10 (SEQ ID NO:32), and a third amino acid sequence being at least 90% homologous to PYSP corresponding to amino acids 346 - 349 of known proteins NP_060408 and Q86Y72_HUMAN, which also corresponds to amino acids 182 - 185 of F10649_P10 (SEQ ID NO:32), wherein said first amino acid sequence, second amino acid sequence, bridging amino acid and third amino acid sequence are contiguous and in a sequential order.

The localization of the variant protein was determined according to results from a number of different software programs and analyses, including analyses from SignalP and other specialized programs. The variant protein is believed to be located as follows with regard to the cell: membrane.

Variant protein F10649_P10 (SEQ ID NO:32) also has the following non-silent SNPs (Single Nucleotide Polymorphisms) as listed in Table 35, (given according to their position(s) on the amino acid sequence, with the alternative amino acid(s) listed).

**Table 35 - Amino acid mutations**

| **SNP position(s) on amino acid sequence** | **Alternative amino acid(s)** |
|---|---|
| 155 | P → S |

The coding portion of transcript F10649_T8 (SEQ ID NO:28) starts at position 103 and ends at position 657. The transcript also has the following SNPs as listed in Table 36 (given according to their position on the nucleotide sequence, with the alternative nucleic acid listed).

**Table 36 - Nucleic acid SNPs**

| **Polymorphism** | **SNP position(s) on nucleotide sequence** |
|---|---|
| C → T | 565 |
| A → C | 645, 2590 |
| C → A | 886, 1189 |
| C → G | 886, 1189 |
| A → | 1183 |
| T → A | 1786 |
| T → G | 1786 |
| T → C | 1796 |
| A → T | 2590 |

### EXAMPLE 5_2: EXPRESSION ANALYSIS OF FAM70A TRANSCRIPTS

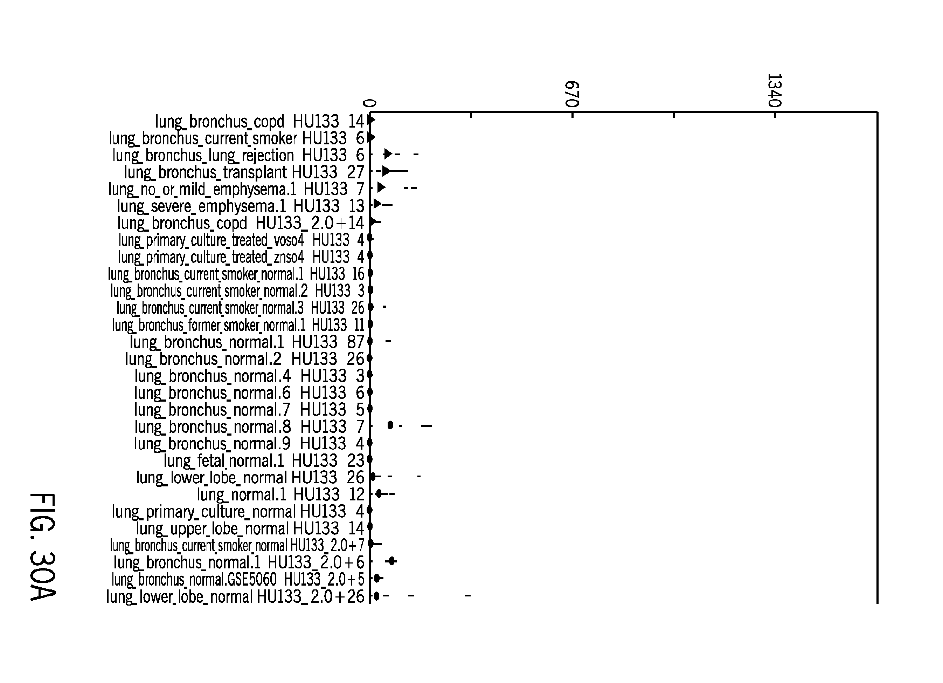

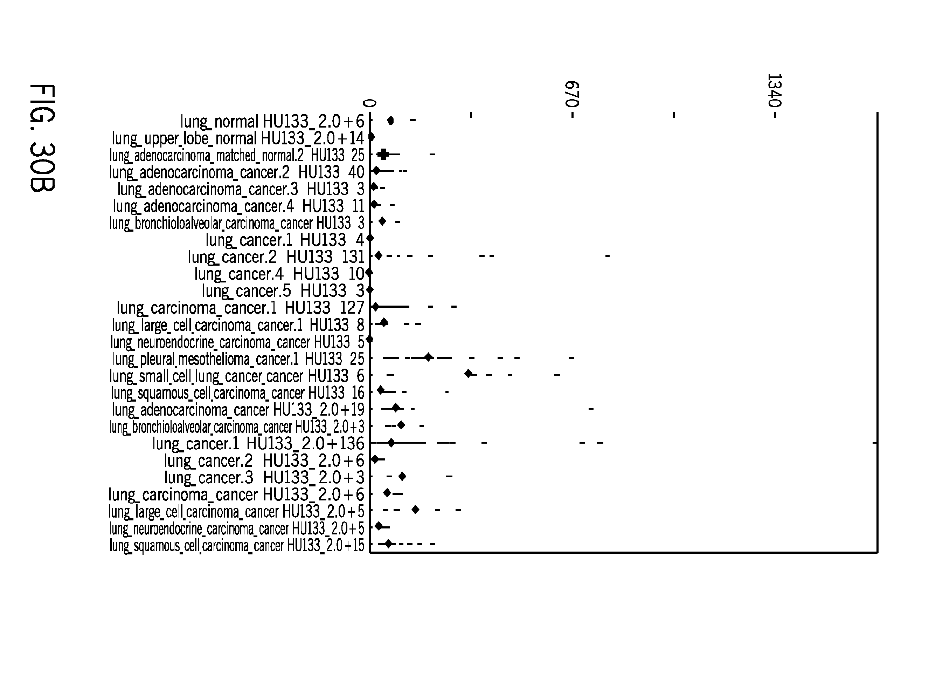

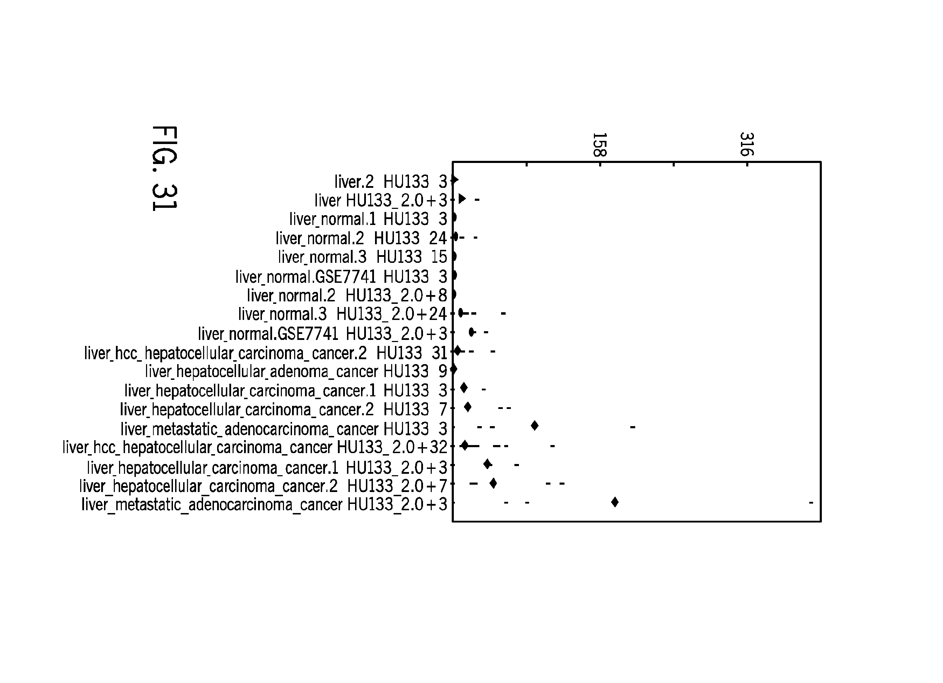

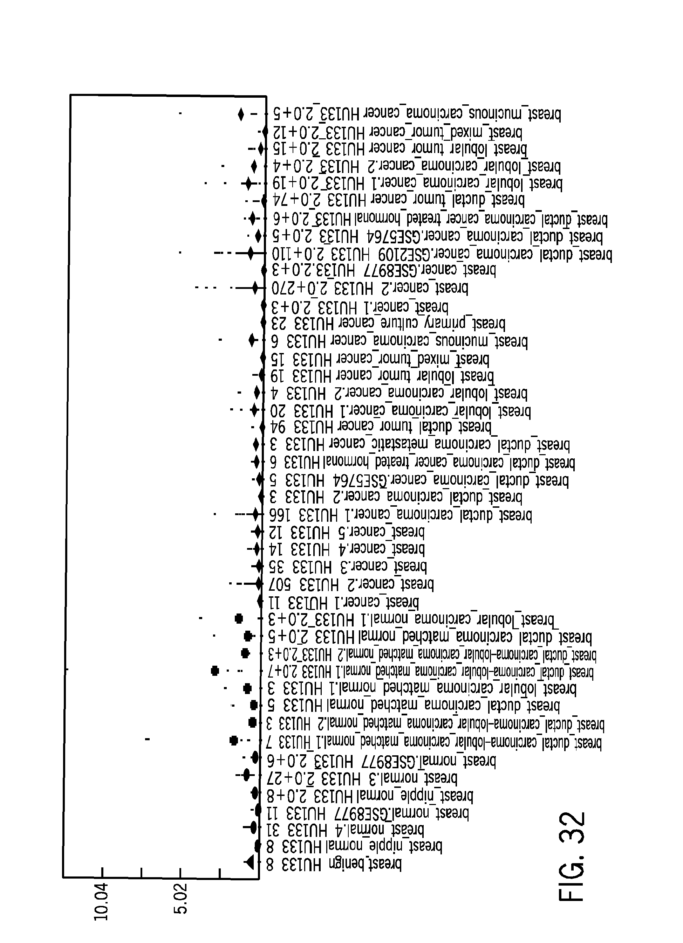

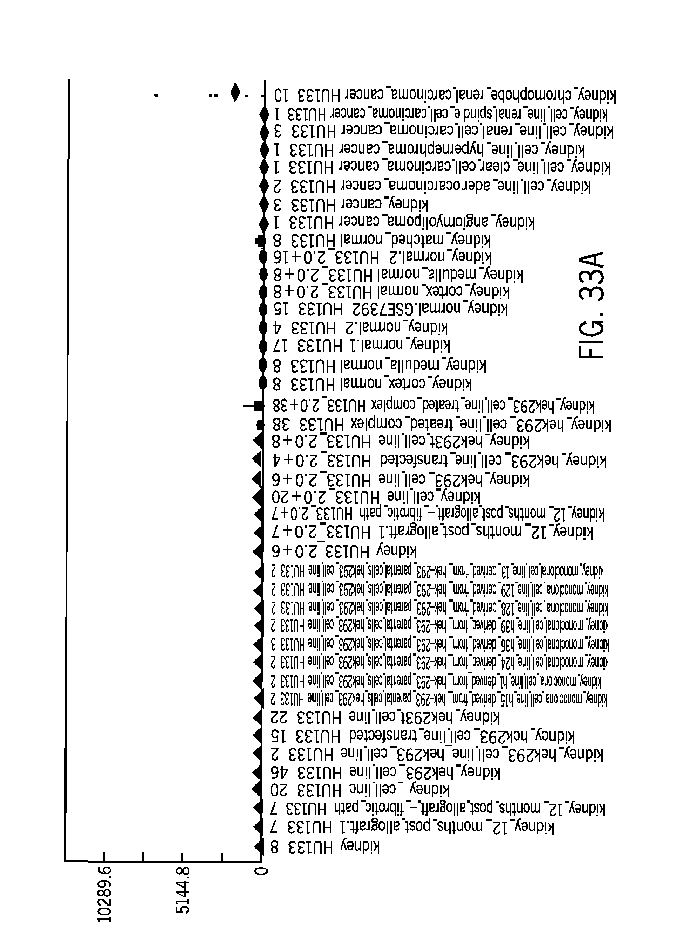

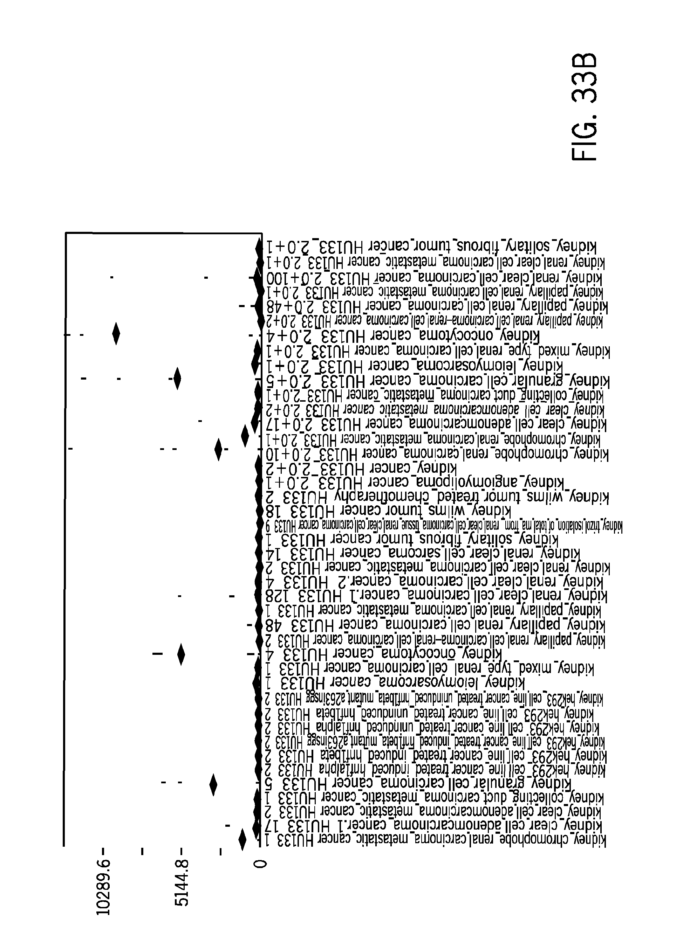

MED discovery engine described in Example 1 herein, was used to assess the expression of FAM70A transcripts. FAM70A transcripts were found to be over expressed in lung cancer, as is demonstrated in Figures 30, in liver cancer, as is demonstrated in Figure 31, in breast cancer, as is demonstrated in Figure 32, and in kidney cancer, as is demonstrated in Figure 33. Figures 30-33 show expression graphs of Affymetrix probe set 219895_at. Figure 30 shows the expression of FAM70A transcripts in microarray chips from lung cancer and lung normal experiments. As can be seen FAM70A transcripts are overexpressed in lung cancer tissues (diamond markers) relative to its expression in normal lung (circle markers).

Figure 31 shows the expression of FAM70A transcripts in microarray chips from liver cancer and normal liver experiments. As can be seen FAM70A transcripts are overexpressed in liver cancer tissues (diamond markers) relative to its expression in normal liver (circle and triangle markers).

Figure 32 shows the expression of FAM70A transcripts in microarray chips from breast cancer and normal breast experiments. As can be seen FAM70A transcripts are overexpressed in breast cancer tissues (diamond markers) relative to its expression in breast lung (circle, cross and triangle markers).

Figure 33 shows the expression of FAM70A transcripts in microarray chips from kidney cancer and normal kidney experiments. As can be seen FAM70A transcripts are overexpressed in kidney cancer tissues (diamond markers) relative to its expression in normal kidney (circle, cross and triangle markers).

### Expression of hypothetical protein FLJ20716-FAM70A F10649 transcripts which are detectable by amplicon as depicted in sequence name F10649_seg10-12F1R1 (SEQ ID NO: 103) in blood specific panel and in different normal tissues

Expression of hypothetical protein FLJ20716-FAM70A transcripts detectable by or according to seg10-12F1R1 - F10649_seg10-12F1R1 (SEQ ID NO: 103) amplicon and primers F10649_seg10-12F (SEQ ID NO: 101) and F10649_seg10-12R (SEQ ID NO: 102) was measured by real time PCR in blood panel and normal panel. The samples used for blood panel are detailed in Tables 2 and 2_1. The samples used for normal panel are detailed in Table 3.

### Normal panel -

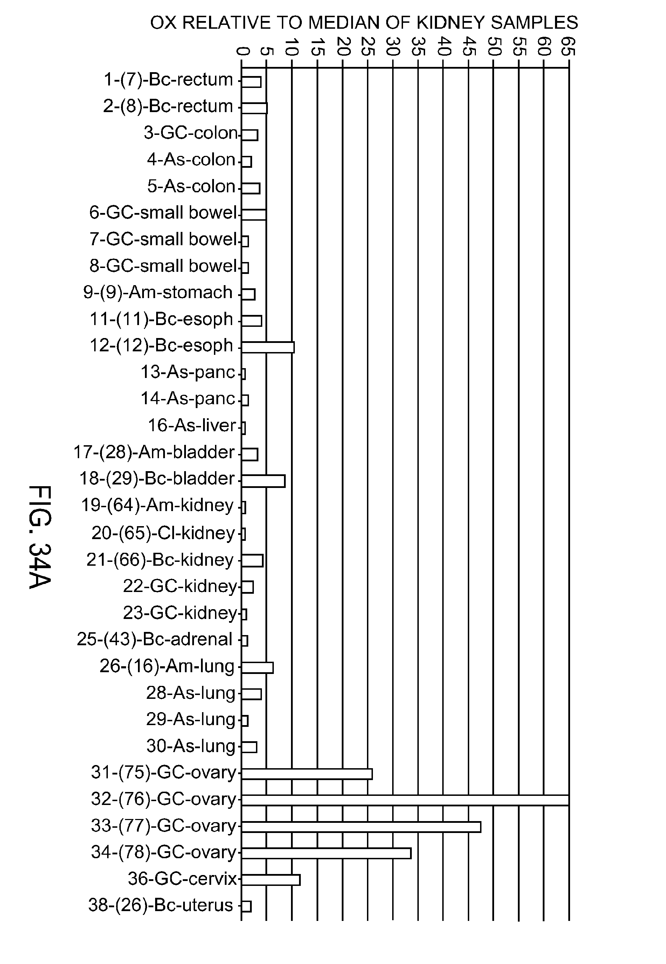

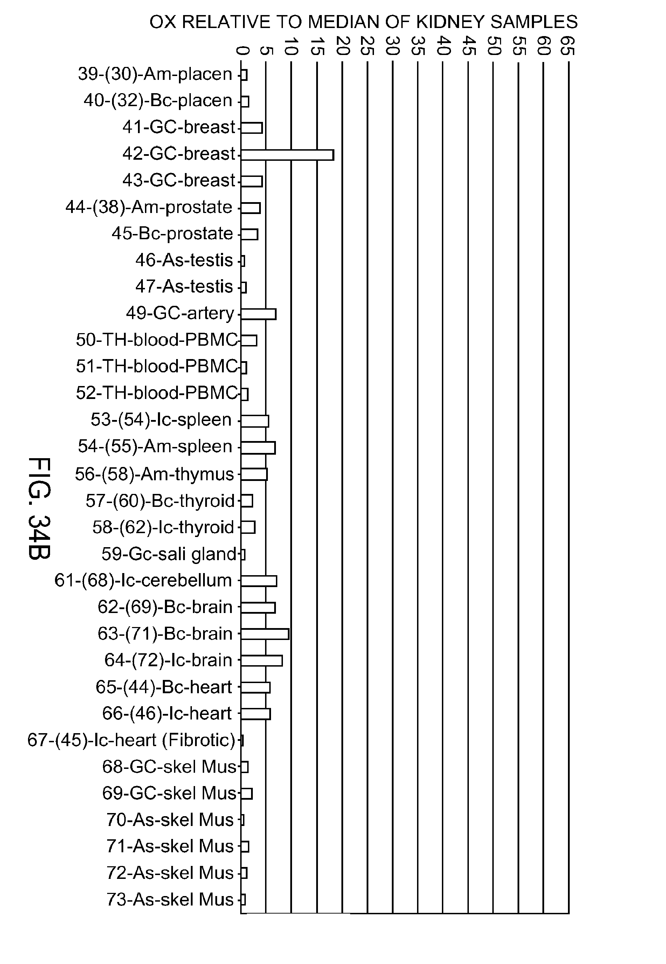

For each RT sample, the expression of the above amplicon was normalized to the normalization factor calculated from the expression of several house keeping genes as described in Example 1. The normalized quantity of each RT sample was then divided by the median of the quantities of the kidney samples (sample numbers 19-23, Table 3 above), to obtain a value of relative expression of each sample relative to median of the kidney samples, as shown in Figure 34. Very high expression was observed in ovary normal samples, high expression was observed in normal brain and heart.

For blood panel - For each RT sample, the expression of the above amplicon was normalized to the normalization factor calculated from the expression of several house keeping genes as described in Example 1. The normalized quantity of each RT sample was then divided by the median of the quantities of the kidney normal samples (sample numbers 65-67, Table 2 above), to obtain a value of relative expression of each sample relative to median of the kidney normal samples.

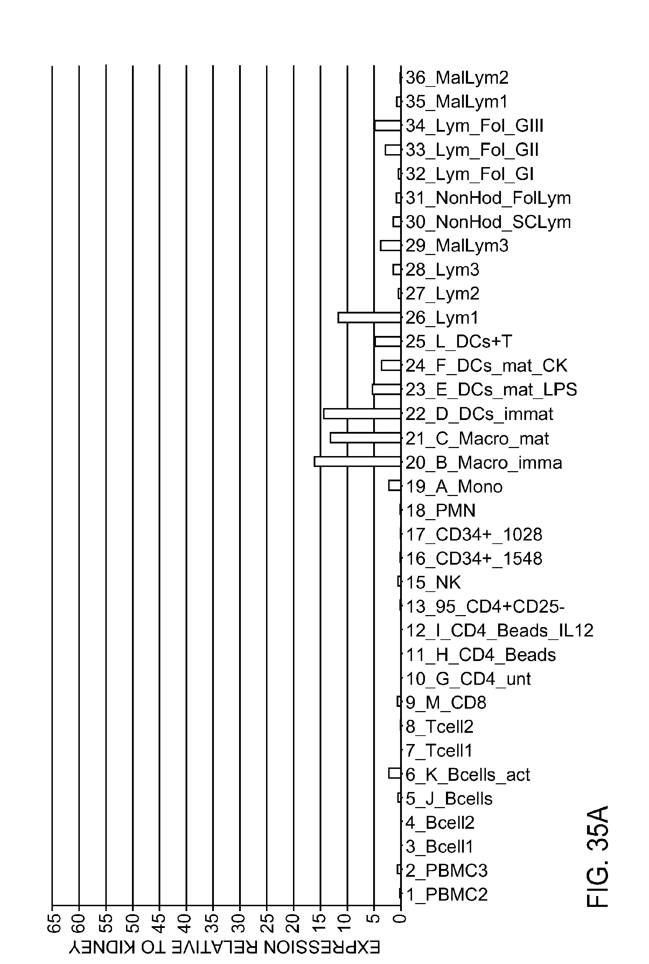

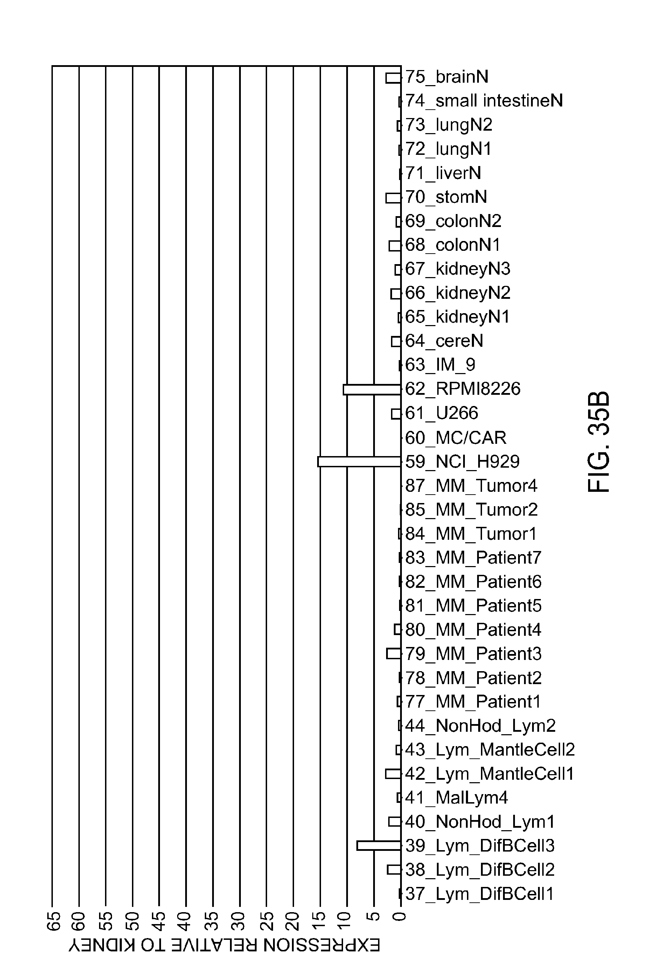

The results of this analysis are depicted in the histogram in Figure 35. Expression of the above-indicated hypothetical protein FLJ20716-FAM70A transcripts is very high in mature and immature macrophages and dendritic cells samples, and in two out of the five multiple myeloma cell lines.
Forward Primer (F10649_seg10-12F) (SEQ ID NO:101):
CTGGTGGCTTCTATCGTGTTTATCAG
Reverse Primer (F10649_seg10-12R) (SEQ ID NO:102):
CGGTTAGCGTAGAGTGGTTTCAG

Amplicon (F10649_seg10-12F1R1 (SEQ ID NO: 103)) (SEQ ID NO:103):
CTGGTGGCTTCTATCGTGTTTATCAGCTTTGGTGTGATTGCGGCTTTTTGT
TGTGCCATAGTTGACGGGGTCTTTGCTGCCAGACACATTGATCTGAAACC
ACTCTACGCTAACCG

### EXAMPLE 5_3:

### CLONING OF FULL LENGTH ORF ENCODING FAM70A T1_P5 FUSED TO FLAG

Cloning of Full Length ORF encoding FAM70A fused to FLAG was done as described below.
1. A reverse transcription reaction was carried out as follows: 10µg of purified RNA extracted from RPMI 8226 cell line (ATCC CCL-155) was mixed with 150ng Random Hexamer primers (Invitrogen, Carlsbad, CA, USA, catalog number: 48190-011) and 500µM dNTPs in a total volume of 156µl. The mixture was incubated for 5 min at 65µC and then quickly chilled on ice. Thereafter, 50µl of 5X SuperscriptII first strand buffer (Invitrogen, catalog number: 18064-014, part number: Y00146), 24µl 0.1M DTT and 400 units RNasin (Promega, Milwaukee, WS, U.S.A., catalog number: N2511) were added, and the mixture was incubated for 10 min at 25°C, followed by further incubation at 42°C for 2 min. Then, 10µl (2000 units) of SuperscriptII (Invitrogen, catalog number: 18064-014) was added and the reaction (final volume of 250µl) was incubated for 50 min at 42°C and then inactivated at 70°C for 15min. The resulting cDNA was diluted 1:20 in TE buffer (10mM Tris, 1 mM EDTA pH 8).
2. PCR was done using GoTaq ReadyMix (Promega, catalog number M122) under the following conditions: 10.5□µl - cDNA from the above; 1µl - of each primer (10µM) (Table 48); 12.5µl ReadyMix with a reaction program of 3 minutes in 95°C; 30 cycles of 30 seconds at 94°C, 30 seconds at 52°C, 1.5 minutes at 72°C; then 10 minutes at 72°C. Primers which were used include gene specific sequences corresponding to the desired coordinates of the protein and restriction enzyme sites and Kozak sequence, as listed in Table 37 below. Bold letters in Table 37 represent the specific gene sequence while the restriction site extensions utilized for cloning purposes are in Italic and kozak sequences are underlined. FLAG tag is in Italic bold. In order to enhance the PCR product, a second PCR was done using Platinum PFX™ (Invitrogen., Carlsbad, CA, USA, catalog number: 1178-021) and the above PCR product as a template (5µl). PCR conditions were as follows: 1 □µl Platinum PFX enzyme; 1µl- 10 mM dNTPs (2.5mM of each nucleotide); 0.5µl - 50mM MgSO₄; and 1µl - of each primer (10µM) in a total reaction volume of 50µl; PCR program was as follows: 3 minutes in 95°C; 30 cycles of 30 seconds at 94°C, 30 seconds at 52°C, 1.5 minutes at 68°C; then 10 minutes at 68°C.Table 1- FAM70A cloning primers details

**Table 37**

| Primer ID | Primer sequence | Primer orientation | Restr iction site |
|---|---|---|---|
| 100-923 (SEQ ID NO:167) | CTA*GCTA**G*CCACCA**TGCA CAGTCCCTGACTC** | For | NheI |
| 10□-924 (SEQ ID NO:168) | ATAAGAATGCGGCCGCTCATTAC TGTCGTCATCGTC TTTGTAGTCGGGACTGTAAGGTGGTGGCTTTTCAA AAG | Rev | NotI |

The Platinum PFX™ PCR product was loaded onto a 1 % agarose gel stained with ethidium bromide, electrophoresed in 1xTBE solution at 100V, and visualized with UV light. After verification of expected size band, it was extracted using Qiaquick gel extraction purification kit (Qiagen™, Valencia, CA, U.S.A., catalog number 28706). The extracted PCR product was digested with NheI and NotI (New England Biolabs, Beverly, MA, U.S.A.). After digestion, the DNA was loaded onto a 1 % agarose gel as described above. The expected band size was excised and extracted from the gel using QiaQuick™ Gel Extraction kit (Qiagen, catalog number: 28706).

The digested PCR product from above was ligated into pIRESpuro3 vector using the LigaFastTM Rapid DNA Ligation System (Promega, □catalog number: M8221□. The resulting DNA was transformed into competent E.Coli bacteria DH5α (RBC Bioscience, Taipei, Taiwan, catalog number: RH816) according to manufacturer's instructions, then plated on LB-ampicillin agar plates for selection of recombinant plasmids, and incubated overnight at 37°C.

The following day, a number of colonies from the transformation that grew on the selective plate was taken for further analysis by streak-plating on another selective plate and by PCR using GoTaq ReadyMix (Promega, catalog number: M7122). Screening positive clones was performed by PCR using pIRESpuro3 vector specific primer and gene specific primer (data not shown). After completion of all PCR cycles, half of the reaction was analyzed using 1 % agarose gel as described above. After verification of expected band size, 2 positive colonies were grown in 5 ml Terrific Broth supplemented with 100µg/ml ampicillin, with shaking overnight at 37°C. Plasmid DNA was isolated from bacterial cultures using Qiaprep™ Spin Miniprep Kit (Qiagen, catalog number: 27106). Accurate cloning was verified by sequencing the inserts (Weizmann Institute, Rehovot, Israel). Upon verification of an error-free colony (i.e. no mutations within the ORF), recombinant plasmid was processed for further analysis.

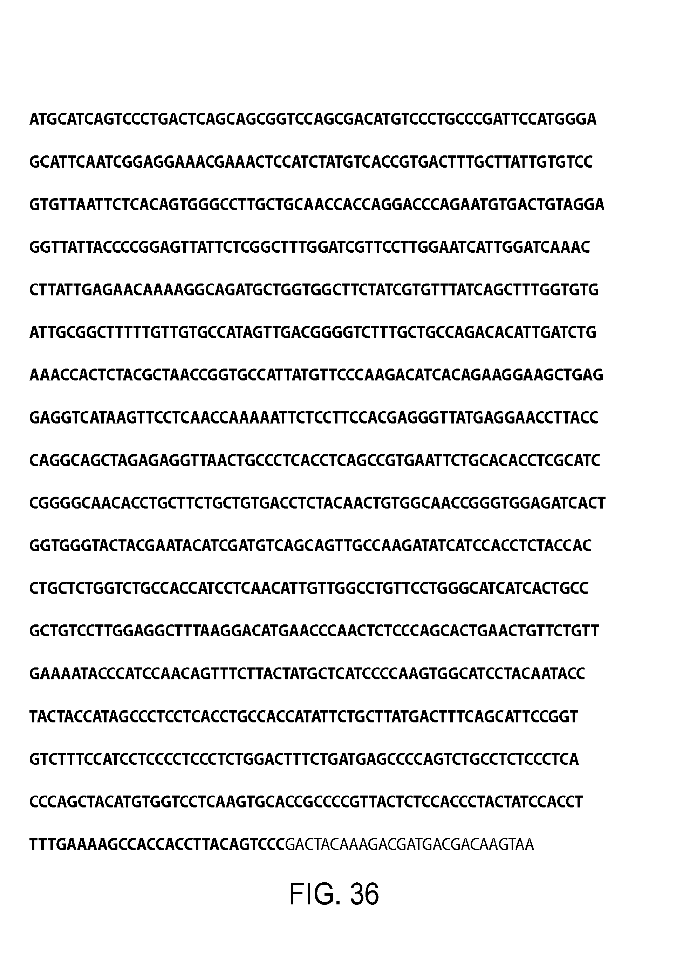

Figure 36 represents the DNA sequence of FAM70_T1_P5_FLAG (SEQ ID NO: 119) gene specific sequence corresponding to the target's full length sequence is marked in bold faced, FLAG sequence is unbold.

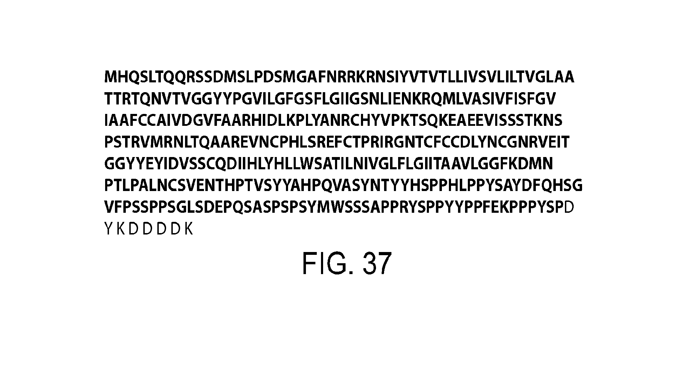

Figure 37 represents the amino acid sequence of FAM70A_T1_P5_FLAG protein (SEQ ID NO:120); gene specific sequence corresponding to the full length sequence of the protein is marked in bold faced, FLAG sequence is unbold.

### EXAMPLE 5_4: DETERMINING CELL LOCALIZATION OF FAM70A

Determining cell localization of FAM70A was done using the confocal microscope. The FAM70A-FLAG pIRESpuro3 construct was subsequently transiently transfected into HEK-293T cells as follows:

HEK-293T (ATCC, CRL-11268) cells were plated on sterile glass coverslips, 13mm diameter (Marienfeld, catalog number: 01 115 30), which were placed in a 6 well plate, using 2ml pre-warmed DMEM [Dulbecco's modified Eagle's Media, Biological Industries (Beit Ha'Emek, Israel), cataloge number: 01-055-1A] + 10% FBS (Fetal Bovin Serum) + 4mM L-Glutamine. 500,000 cells per well were transfected with 2µg of the DNA construct using 6µl FuGENE 6 reagent (Roche, catalog number: 11-814-443-001) diluted into 94ul DMEM. The mixture was incubated at room temperature for 15 minutes. The complex mixture was added dropwise to the cells and swirled. Cells were placed in incubator maintained at 37°C with 5% CO2 content.

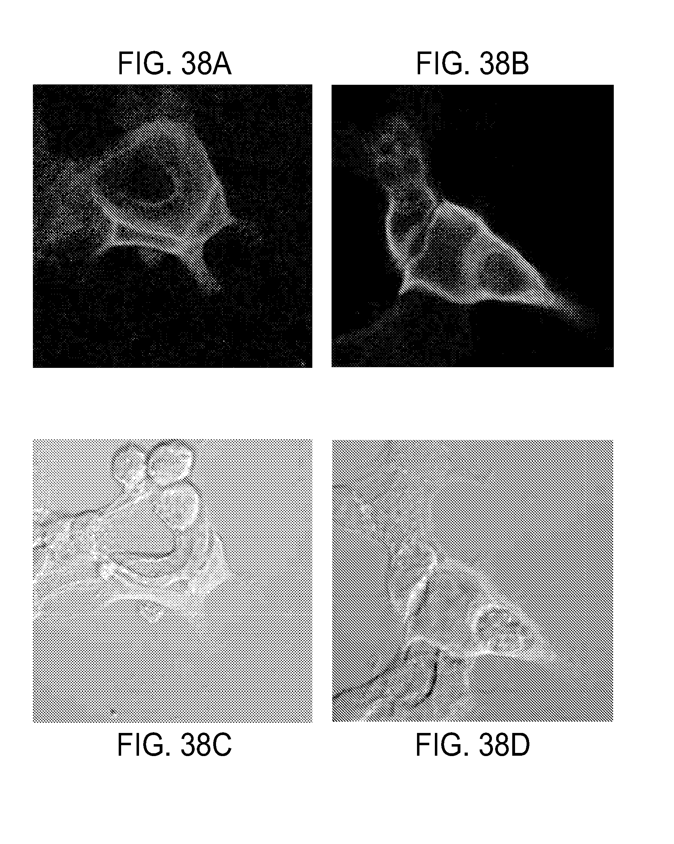

48 hours post transient transfection, the cells were further processed for analysis in confocal microscopy. The cover slips were washed 3 times in phosphate buffered saline (PBS) and fixed for 15 minutes with a fixing solution composed of 3.7% paraformaldehyde (PFA) (Sigma, catalog number: P-6148) and 3% glucose (Sigma, catalog number: G5767), followed by 5 minutes incubation with 3mM glycine (Sigma, catalog number: G7126). After one wash in PBS, the cells were permeabilized by incubation with 0.1% triton X-100/PBS solution for 5 minutes. After two washes in PBS the cells were incubated in 5% bovine serum albumin (BSA) (Sigma, catalog number: A4503) in PBS solution for 20 minutes. The cells were then incubated with anti FLAG antibody conjugated to cy3 (Sigma, catalog number: A9594) diluted 1:100 in 3% BSA in PBS for 1 hr. After 3 washes in PBS the coverslips were glued to a slide using mounting solution (Sigma, catalog number: G0918) and cells were observed for the presence of fluorescence using confocal microscope. The results are presented in Figure 38.

Figure 38 demonstrates that the FAM70A_T1_P5_FLAG (SEQ ID NO:**119**) fused protein localizes to cell membrane upon expression in HEK 293T cells. The image was obtained using the 40x objective of the confocal microscope.

### EXAMPLE 5_5: PRODUCTION OF POLYCLONAL ANTIBODIES SPECIFIC TO FAM70 VARIANT

All polyclonal Abs production procedure, including peptides synthesis, peptides conjugation, animal immunizations, bleeding and antibodies purification were performed at Sigma-Aldrich (Israel).

Peptide synthesis - The peptide sequence which was used for rabbit immunization was as follows: CHYVPKTSQKEAEEV (FAM70_128 SEQ ID NO:121), a sequence taken from the ECD loop correspond to aa128-142 of the FAM70_P5 protein (FAM70_P5; SEQ ID NO:33). 25mg peptide were synthesized with 95% purity of which 10mg were conjugated to KLH carrier.

Immunization -two rabbits (#5663, #5664) were immunized with the conjugated peptide as follows: Animals were immunized every two weeks. 3 test bleeds of 2-3ml were collected and analyzed by ELISA. 100ml production bleeds from each rabbit will be collected.

Antibody purification - Antibodies will be purified from the rabbits' serum. Affinity purification will be performed using the peptide against which the respective antibodies were raised, in an immuno-affinity column. The purified antibodies will be analyzed by ELISA and by Western blot on the recombinant FAM70_P5 expressed in HEK-293T cell line. Endogenous protein localization will be determined using these Abs by tissue Immuno HystoChemistry (IHC)
FAM70 peptide sequence (SEQ ID NO: 121) CHYVPKTSQKEAEEV

### EXAMPLE 5_6: GENERATION OF STABLE POOL EXPRESSING FAM70_P5 PROTEIN

In order to generate stable pool of HEK-293T cells expressing FAM70A_P5 protein, FAM70_P5_Flag pIRESpuro3 construct was transfected into HEK-293T cells as follows:
HEK-293T (ATCC, CRL-11268) cells were plated in a sterile 6 well plate suitable for tissue culture, using 2ml pre-warmed of complete media, DMEM [Dulbecco's modified Eagle's Media, Biological Industries (Beit Ha'Emek, Israel), catalog number: 01-055-1A] + 10% FBS [Fetal Bovine Serum, Biological Industries (Beit Ha'Emek, Israel), catalog number: 04-001-1A] + 4mM L-Glutamine [Biological Industries (Beit Ha'Emek, Israel), catalog number: 03-020-1A]. 500,000 cells per well were transfected with 2µg of DNA construct using 6µl FuGENE 6 reagent (Roche, catalog number: 11-814-443-001) diluted into 94ul DMEM. The mixture was incubated at room temperature for 15 minutes. The complex mixture was added dropwise to the cells and swirled. Cells were placed in incubator maintained at 37°C with 5% CO₂ content. 48 hours following transfection, transfected cells were transferred to a 75cm² tissue culture flask containing 15ml of selection media: complete media supplemented with 5µg\ml puromycin (Sigma, catalog number P8833). Cells were placed in incubator, and media was changed every 3-4 days, until clone formation observed.

### EXAMPLE 5_7: CHARACTERIZATION OF PURIFIED FAM70A ANTIBODIES BY WESTERN BLOT ANALYSIS ON FAM70A_P5 TRANSFECTED CELLS

In order to verify the specificity of antibodies raised against selected peptide of FAM70A, immuno-precipitation followed by western blot analysis was done using purified serum from rabbits 5663 and 5664 described above, and FAM70 HEK-293T stable transfectants cell lysates as well as HEK-293T nontrasfected cell lysates.

HEK-293T stably expressing FAM70_P5_FLAG and untransfected cell lysates were immuno- precipitated using anti Flag antibody (Anti Flag M2 affinity Gel Freezer-Safe, Sigma. Cat: A220) and were analyzed by Western blot using the purified antibodies raised against FAM70 peptide diluted 1:250 as previously described.

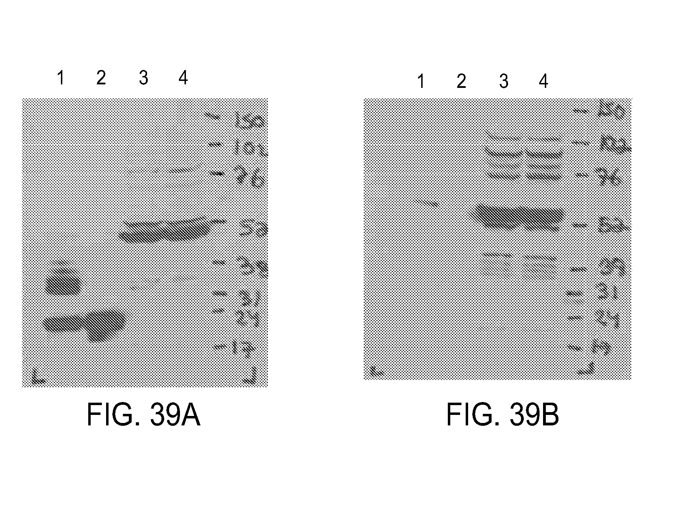

Figures 39A and 39B represent the signal obtained from purified serum of rabbits #5663 and #5664 respectively. Figure 39A demonstrates that specific band size of 36kDa was observed from purified serum of rabbit #5663 on HEK-293T transfected cell lysates followed by IP (lane 1 in Figure 39A). However, whole cell lysate extraction did not revealed expected band size (lane 3). Nonspecific band of 52kDa was observed using serum purified from rabbit #5663 or #5664 (lanes 3 and 4 in Figures 39A and 39B respectively).

### EXAMPLE 5_8: CHARACTERIZATION OF PURIFIED FAM70A ANTIBODIES BY IMMUNOSTAINING OF FAM70A_P5 TRANSFECTED CELLS

In order to further characterize the affinity purified antibodies raised against FAM70A, antibody-protein interaction was studied using immunostaining of FAM70_P5 stable transfected HEK293T cells.

### Immunostaining of FAM70A transfected cells:

500,000 cells per well of HEK-293T (ATCC, CRL-11268) stably expressing FAM70A or HEK-293T non trasfected, were plated on sterile glass coverslips, 13mm diameter (Marienfeld, catalog number: 01 115 30), which were placed in a 6 well plate, using 2ml pre-warmed DMEM [Dulbecco's modified Eagle's Media, Biological Industries (Beit Ha'Emek, Israel), catalog number: 01-055-1A] + 10% FBS [Fetal Bovine Serum, Biological Industries (Beit Ha'Emek, Israel), catalog number: 04-001-1A] + 4mM L-Glutamine [Biological Industries (Beit Ha'Emek, Israel), catalog number: 03-020-1A].

48 hours post plating the cells on coverslips they were further processed for immunostaining and analysis by confocal microscopy. The cover slips were washed in phosphate buffered saline (PBS), then fixed for 15 minutes with a 3.7% paraformaldehyde (PFA) (Sigma, catalog number: P-6148)/3% glucose (Sigma, catalog number: G5767). After 2 5-minute washes in PBS, cells were permeabilized with 0.1% triton-X100 (diluted in PBS) for 5 minutes. After two 5-minute washes in PBS, blocking of non-specific regions was done with 5% bovine serum albumin (BSA) (Sigma, catalog number: A4503) (diluted in PBS) for 20 minutes. The coverslips were then incubated, in a humid chamber for 1 hour, with purified serum of rabbit 5663 antibodies diluted 1:200/ 1:1000 in 5% BSA, described above and then, were washed 3 times for 5-minutes in PBS. The coverslips were then incubated, in a humid chamber for 1 hour, with secondary antibody: donkey anti-rabbit conjugated to Cy-3 flurophore (Jackson ImmunoResearch, catalog number: 711-165-152), diluted 1:200 in 3% BSA in PBS. After 3 5-minute washes in PBS, the fixed coverslips were mounted on slides with Gel Mount Aqueous medium (Sigma, catalog number: G0918) and cells were observed for the presence of fluorescent product using confocal microscopy.

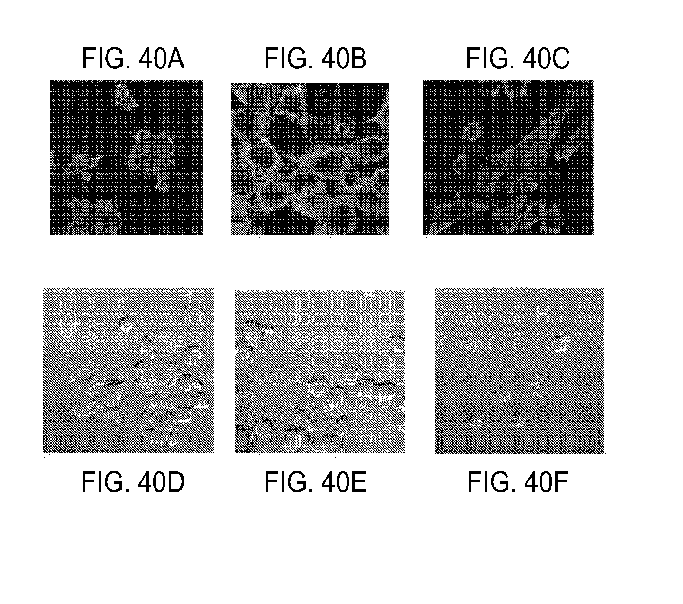

As shown in Figure 40, cell staining was observed localized to the cell membrane using purified anti FAM70 antibodies (rabbits #5663 and #5664 described above) 1:200 or 1:1000 on HEK-293T transfected cells (Figures 40A and 40B, respectively). However, positive signal was observed on HEK-293T non transfected cells using 1:200 or 1:1000 (Figures 40C and 40D, respectively) as well as on CHO-K1 (ATCC, CCL-61) cells (Figure 40E) and MC/CAR (ATCC, CRL-8083) cells (Figure 40F).

In order to further determine whether the positive signal obtained in the non transfected cells was due to endogenous expression or lack of specificity, RTPCR was performed on HEK-293T as well as on MC/CAR cDNAs as previously described, using GoTaq_ReadyMix (Promega, M712B). The primers used were FAM70 specific: primer #100-923 (SEQ ID NO 167) and primer #100-924 (SEQ ID NO 168).

The expected PCR product size obtained from HEK-293 cDNA was purified and verified by sequence as previously described. Sequence results indicated FAM70A expression in HEK-293T non transfected cells. However, no product was observed when using MC/CAR cDNA as a template (data not shown).

In order to further analyze specificity, FAM70A HEK-293Ttransfected cells described above were immunostained using anti FAM70A antibodies described above with or without pre incubation with FAM70A peptide.

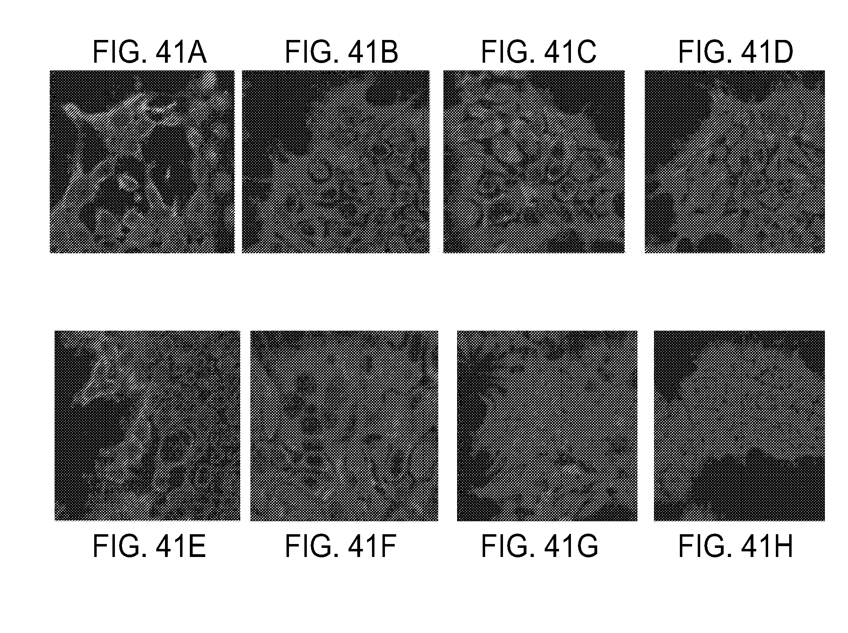

Figures 41A-41D demonstrate red fluorescence signal of 293T transfected cells followed by incubation with 0, 5times, 25times, 50 times FAM70 peptide, respectively.

Figures 41E-41H demonstrate red fluorescence signal of 293T non transfected cells followed by incubation with 0, 5times,25times, 50 times FAM70 peptide, respectively.

As shown in Figure 41A and Figure 41E, membranalic localization was observed both in HEK-293T transfected cells and HEK-293Tnon transfected cells, respectively.

However, non specific signal was observed followed by peptide blocking.

In order to further analyze FAM70A expression, polyclonal antibodies against an additional peptide sequence were raised.

The peptide sequence which was used for rabbit immunization was as follows: MHQSLTQQRSSDMSLPDS (FAM70_1, SEQ ID NO:186) a sequence taken from the N terminus correspond to amino acid residues 1-18 of the FAM70_P5 protein (SEQ ID NO:33). 25mg peptide were synthesized at Sigma (Israel) with 95% purity of which 10mg were conjugated to KLH carrier.

Immunization: two rabbits were immunized with the conjugated peptide as follows: Animals were immunized every two weeks. 60ml production bleeds from each rabbit were collected.

Antibody purification: antibodies are purified from the rabbits' serum. Affinity purification is performed using the peptide against which the respective antibodies are raised, in an immuno-affinity column. The purified antibodies are analyzed by immunostaining on FAM70_P5 HEK-293T transfected cell line described above. After immune-staining verification, these antibodies are further used for endogenous protein localization in human tissue array by Immuno HystoChemistry (IHC).

### EXAMPLE 6

### EXAMPLE 6_1

### DESCRIPTION FOR CLUSTER W38346

Cluster W38346 (internal ID 70579958) features 4 transcripts of interest, the names for which are given in Table 38. The selected protein variants are given in table 39.

**Table 38 - Transcripts of interest**

| **Transcipt Name** |
|---|
| W38346_T0 (SEQ ID NO:38) |
| W38346_T1 (SEQ ID NO:39) |
| W38346_T2 (SEQ ID NO:40) |
| W38346_T5 (SEQ ID NO:41) |

**Table 39 - Proteins of interest**

| **Protein Name** | **Corresponding Transcript(s)** |
|---|---|
| W38346_P3 (SEQ ID NO:42) | W38346_T0 (SEQ ID NO:38); W38346_T1 (SEQ ID NO:39) |
| W38346_P4 (SEQ ID NO:45) | W38346_T2 (SEQ ID NO:40) |
| W38346_P7 (SEQ ID NO:46) | W38346_T5 (SEQ ID NO:41) |

These sequences are variants of the known hypothetical protein LOC201799 (SEQ ID NO:42) (SwissProt accession identifier NP_689893; synonims: TMEM154).

TMEM154 (transmembrane protein 154) was identified in 2 full length cDNA projects (Strausberg et al. 2002, PNAS 99(26): 16899-903; Ota et al. 2004, Nat Genet 36(1): 40-5). However no research was published about TMEM154 specifically.

Sequence corresponding to W38346_P3 (SEQ ID NO:42) has been reported in WO2004110369 patent application, which purports that sequence of FLJ32028, corresponding to W38346_P3 (SEQ ID NO:42), is associated with human chronic lymphocytic leukemia. The application further relates to monoclonal antibodies and methods for antibody screening and production and their use as diagnostic marker or therapeutic target for B-CLL. However, The WO2004110369 patent application does not provide any specific teaching or incentive that would direct a skilled artisan to use antibodies specific to the TMEM154 for the treatment or diagnosis of lymphoma, especially Non-Hodgkin's Lymphoma, anti CD20 (i.e. Rituximab) resistant lymphoma, Multiple Myeloma, kidney cancer or pancreatic cancer, and/or immune related conditions, particularly SLE. The WO2004110369 patent application does not teach also, that TMEM154 soluble ectodomain, as well as fragments thereof and conjugates and antibodies against it can be used as therapeutic or diagnostic agents for treatment of of lymphoma, especially Non-Hodgkin's Lymphoma, anti CD20 (i.e. Rituximab) resistant lymphoma, Multiple Myeloma, kidney cancer or pancreatic cancer, and/or immune related conditions, particularly SLE.

PRO92173, corresponding to W38346_P3 (SEQ ID NO:42) protein, has been reported in WO2004081199 patent application, among other genes showing altered patterns of expression in autoimmune diseases for use in diagnosis, prevention and treatment thereof. The WO2004081199 patent application does not teach however, that that sequence corresponding to PRO92173 differentially expressed in systemic lupus erythematosus (SLE), and/or in cancer, especially in lymphoma, especially Non-Hodgkin's Lymphoma, anti CD20 (i.e. Rituximab) resistant lymphoma, Multiple Myeloma, kidney cancer or pancreatic cancer. The WO2004081199 patent application does not teach also, that that sequence corresponding to PRO92173 can be used as drug target for treatment of SLE, and/or of cancer, including lymphoma, especially Non-Hodgkin's Lymphoma, anti CD20 (i.e. Rituximab) resistant lymphoma, Multiple Myeloma, kidney cancer or pancreatic cancer, and/or diagnosis thereof. The WO2004081199 patent application does not teach also, that PRO92173 soluble ectodomain, as well as fragments thereof and conjugates and antibodies against it can be used as therapeutic or diagnostic agents for treatment of cancer including lymphoma, especially Non-Hodgkin's Lymphoma, anti CD20 (i.e. Rituximab) resistant lymphoma, Multiple Myeloma, kidney cancer or pancreatic cancer, and/or immune related conditions, especially SLE.

TMEM154 sequence corresponding to W38346_P3 (SEQ ID NO:42) has been also reported in WO03090694 patent application, among other genes used for diagnosing or monitoring autoimmune and chronic inflammatory diseases. The WO03090694 patent application does not teach however, that sequence corresponding to TMEM154 can be used as drug targets for treatment of immune related conditions, particularly SLE. The WO03090694 patent application does not teach also, that sequence corresponding to TMEM154 are differentially expressed in cancer, especially in lymphoma, especially Non-Hodgkin's Lymphoma, anti CD20 (i.e. Rituximab) resistant lymphoma, Multiple Myeloma, kidney cancer or pancreatic cancer. The WO03090694 patent application does not teach also, that sequences corresponding to TMEM154 can be used as drug targets for treatment of lymphoma, especially Non-Hodgkin's Lymphoma, anti CD20 (i.e. Rituximab) resistant lymphoma, Multiple Myeloma, kidney cancer or pancreatic cancer, or diagnosis thereof. The WO03090694 patent application does not teach also, that TMEM154 soluble ectodomain, as well as fragments thereof and conjugates and antibodies against it can be used as therapeutic or diagnostic agents for treatment of lymphoma, especially Non-Hodgkin's Lymphoma, anti CD20 (i.e. Rituximab) resistant lymphoma, Multiple Myeloma, kidney cancer or pancreatic cancer, and/or immune related conditions, especially SLE.

TMEM154 antigen corresponding to W38346_P3 (SEQ ID NO:42) has been reported in WO06020266 patent application, which purports to disclose polypeptides and antibodies derived from chronic lymphocytic leukemia cells, and uses thereof. WO06020266 does not teach however, that sequences corresponding to TMEM154 can be used as drug targets for treatment of lymphoma, especially Non-Hodgkin's Lymphoma, anti CD20 (i.e. Rituximab) resistant lymphoma, Multiple Myeloma, kidney cancer or pancreatic cancer, and/or immune related conditions, especially SLE. The WO06020266 patent application does not provide any specific teaching or incentive that would direct a skilled artisan to use antibodies specific to the TMEM154 for the treatment or diagnosis of lymphoma, especially Non-Hodgkin's Lymphoma, anti CD20 (i.e. Rituximab) resistant lymphoma, Multiple Myeloma, kidney cancer or pancreatic cancer, and/or immune related conditions, especially SLE. The WO06020266 patent application does not teach also, that TMEM154 soluble ectodomain, as well as fragments thereof and conjugates and antibodies against it can be used as therapeutic or diagnostic agents for treatment of lymphoma, especially Non-Hodgkin's Lymphoma, anti CD20 (i.e. Rituximab) resistant lymphoma, Multiple Myeloma, kidney cancer or pancreatic cancer, and/or immune related conditions, especially SLE (systemic lupus erythematosus).

TMEM154 antigen corresponding to W38346_P3 (SEQ ID NO:42) has been reported in WO2008112177, WO200270539 and EP1293569 patent applications. TMEM154 antigen corresponding to W38346_P4 (SEQ ID NO:45) has been reported in WO2008112177 patent application. However, none of these patent applications teaches that sequences corresponding to TMEM154 can be used as drug targets for treatment or diagnosing of lymphoma, especially Non-Hodgkin's Lymphoma, anti CD20 (i.e. Rituximab) resistant lymphoma, Multiple Myeloma, kidney cancer or pancreatic cancer, and/or immune related conditions, especially SLE (systemic lupus erythematosus). These applications do not teach also, that TMEM154 soluble ectodomain, as well as fragments thereof and conjugates and antibodies against it can be used as therapeutic or diagnostic agents for treatment of lymphoma, especially Non-Hodgkin's Lymphoma, anti CD20 (i.e. Rituximab) resistant lymphoma, Multiple Myeloma, kidney cancer or pancreatic cancer, and/or immune related conditions, especially SLE (systemic lupus erythematosus).

TMEM154 antigen corresponding to W38346_P7 (SEQ ID NO:46) has been reported by the applicants of the present application in US patent application No: 11/043,860. However, there is no teaching in US 11/043,860 application that W38346_P7 (SEQ ID NO:46) soluble ectodomain, as well as fragments thereof and specific antibodies against it can be used as therapeutic or diagnostic agents for treatment of lymphoma, especially Non-Hodgkin's Lymphoma, anti CD20 (i.e. Rituximab) resistant lymphoma, Multiple Myeloma, kidney cancer or pancreatic cancer, and/or immune related conditions, especially SLE (systemic lupus erythematosus).

In particular this invention uses TMEM154 antigen and discrete portions thereof as a drug target for therapeutic small molecules, peptides, antibodies, antisense RNAs, siRNAs, ribozymes, and the like. More particularly the invention relates to diagnostic and therapeutic polyclonal and monoclonal antibodies and fragments thereof that bind TMEM154, and portions and variants thereof. According to at least some embodiments of the invention there is a use antibodies and antibody fragments against TMEM154 antigen, its secreted or soluble form or ECD and/or variants, conjugates, or fragments thereof and fragments and variants thereof for treating and diagnosing cancer, especially for treatment of lymphoma, especially Non-Hodgkin's Lymphoma, anti CD20 (i.e. Rituximab) resistant lymphoma, Multiple Myeloma, kidney cancer or pancreatic cancer, and/or immune related conditions, especially SLE (systemic lupus erythematosus), wherein this antigen is differentially expressed.

As noted above, cluster W38346 features 4 transcripts, which were listed in Table 38 above. These transcripts encode for proteins which are variants of protein hypothetical protein LOC201799 (SEQ ID NO:42). A description of each variant protein according to at least some embodiments of the invention is now provided.

Variant protein W38346_P3 (SEQ ID NO:42) according to at least some embodiments of the invention has an amino acid sequence as encoded by transcriptsW38346_T0 (SEQ ID NO:38) and W38346_T1 (SEQ ID NO:39).

The localization of the variant protein was determined according to results from a number of different software programs and analyses, including analyses from SignalP and other specialized programs. The variant protein is believed to be located as follows with regard to the cell: membrane.

The coding portion of transcript W38346_T0 (SEQ ID NO:38) starts at position 233 and ends at position 781. The transcript also has the following SNPs as listed in Table 40 (given according to their position on the nucleotide sequence, with the alternative nucleic acid listed).

**Table 40 - Nucleic acid SNPs**

| **Polymorphism** | **SNP position(s) on nucleotide sequence** |
|---|---|
| C → T | 511 |
| A → C | 1104 |
| A → T | 2446 |
| A → G | 2446, 3013, 3075 |
| C → A | 2611, 3030 |
| C → G | 2611 |

The coding portion of transcript W38346_T1 (SEQ ID NO:39) starts at position 233 and ends at position 781. The transcript also has the following SNPs as listed in Table 41 (given according to their position on the nucleotide sequence, with the alternative nucleic acid listed).

**Table 41 - Nucleic acid SNPs**

| **Polymorphisme** | **SNP position(s) on nucleotide sequence** |
|---|---|
| C → T | 511 |
| A → C | 1104 |
| A → T | 2446 |
| A → G | 2446 |
| C → A | 2611 |
| C → G | 2611 |

Variant protein W38346_P4 (SEQ ID NO:45) according to at least some embodiments of the invention has an amino acid sequence as encoded by transcripts W38346_T2 (SEQ ID NO:40). A description of the relationship of the variant protein according to at least some embodiments of the invention to known proteins is as follows:
2. Comparison report between W38346_P4 and known proteins Q96MQ8_HUMAN:
   A. An isolated chimeric polypeptide encoding for W38346_P4, comprising a amino acid sequence being at least 90% homologous to MVLIPLILLVLLLLSVVFLATYYKRKRTKQEPSSQGSQSALQTYELGSENVKVPIFEE DTPSVMEIEMEELDKWMNSMNRNADFECLPTLKEEKESNHNPSDSES corresponding to amino acids 79 - 183 of known proteins Q96MQ8_HUMAN, which also corresponds to amino acids 1 - 105 of W38346_P4.

The localization of the variant protein was determined according to results from a number of different software programs and analyses, including analyses from SignalP and other specialized programs. The variant protein is believed to be located as follows with regard to the cell: secreted.

The coding portion of transcript W38346_T2 (SEQ ID NO:40) starts at position 516 and ends at position 830. The transcript also has the following SNPs as listed in Table 42 (given according to their position on the nucleotide sequence, with the alternative nucleic acid listed).

**Table 42 - Nucleic acid SNPs**

| **Polymorphism** | **SNP position(s) on nucleotide sequence** |
|---|---|
| C → T | 560 |
| A → C | 1153 |
| A → T | 2495 |
| A → G | 2495, 3062, 3124 |
| C → A | 2660, 3079 |
| C → G | 2660 |

Variant protein W38346_P7 (SEQ ID NO:46) according to at least some embodiments of the invention has an amino acid sequence as encoded by transcripts W38346_T5 (SEQ ID NO:41). A description of the relationship of the variant protein according to at least some embodiments of the invention to known proteins is as follows:
1. Comparison report between W38346_P7 (SEQ ID NO:46) and known proteins Q96MQ8_HUMAN and NP_689893:
   A. An isolated chimeric polypeptide, comprising a first amino acid sequence being at least 90% homologous to MQAPRAALVFALVIALVPVGRGNYEELENSGDTTVESERPNKVTIPSTFAAVTIKET LNANINSTNFAPDENQLEFILMVLIPLILLVLLLLSVVFLATYYKRKRTKQEPSSQGS QSALQT corresponding to amino acids 1 - 121 of known proteins Q96MQ8_HUMAN and NP_689893, which also corresponds to amino acids 1 - 121 of W38346_P7 (SEQ ID NO:46), and a second amino acid sequence being at least 70%, optionally at least 80%, preferably at least 85%, more preferably at least 90% and most preferably at least 95% homologous to a polypeptide having the sequence CKIQLSWKVIPAFCLESSHRNAL (SEQ ID NO: 162) corresponding to amino acids 122 - 144 of W38346_P7 (SEQ ID NO:46), wherein said first amino acid sequence and second amino acid sequence are contiguous and in a sequential order.
   B. An isolated polypeptide of an edge portion of W38346_P7 (SEQ ID NO:46), comprising an amino acid sequence being at least 70%, optionally at least about 80%, preferably at least about 85%, more preferably at least about 90% and most preferably at least about 95% homologous to the sequence CKIQLSWKVIPAFCLESSHRNAL (SEQ ID NO: 162) of W38346_P7 (SEQ ID NO:46).
2. Comparison report between W38346_P7 (SEQ ID NO:46) and known protein Q6P9G4_HUMAN (SEQ ID NO:44):
   A. An isolated chimeric polypeptide, comprising a first amino acid sequence being at least 90% homologous to MQAPRAALVFALVIALVPVGRGNYEELENSGDTTVESERPNKVTIPSTFAAVTIKET LNANINSTNFAPDENQLEFILMVLIPLILLVLLLL corresponding to amino acids 1 - 92 of known protein Q6P9G4_HUMAN (SEQ ID NO:44), which also corresponds to amino acids 1 - 92 of W38346_P7 (SEQ ID NO:46), a bridging amino acid S corresponding to amino acid 93 of W38346_P7 (SEQ ID NO:46), a second amino acid sequence being at least 90% homologous to VVFLATYYKRKRTKQEPSSQGSQSALQT corresponding to amino acids 94 - 121 of known protein Q6P9G4_HUMAN (SEQ ID NO:44), which also corresponds to amino acids 94 - 121 of W38346_P7 (SEQ ID NO:46), and a third amino acid sequence being at least 70%, optionally at least 80%, preferably at least 85%, more preferably at least 90% and most preferably at least 95% homologous to a polypeptide having the sequence CKIQLSWKVIPAFCLESSHRNAL (SEQ ID NO: 162) corresponding to amino acids 122 - 144 of W38346_P7 (SEQ ID NO:46), wherein said first amino acid sequence, bridging amino acid, second amino acid sequence and third amino acid sequence are contiguous and in a sequential order.
   B. An isolated polypeptide of an edge portion of W38346_P7 (SEQ ID NO:46), comprising an amino acid sequence being at least 70%, optionally at least about 80%, preferably at least about 85%, more preferably at least about 90% and most preferably at least about 95% homologous to the sequence CKIQLSWKVIPAFCLESSHRNAL (SEQ ID NO: 162) of W38346_P7 (SEQ ID NO:46).

The localization of the variant protein was determined according to results from a number of different software programs and analyses, including analyses from SignalP and other specialized programs. The variant protein is believed to be located as follows with regard to the cell: membrane.

Variant protein W38346_P7 (SEQ ID NO:46) also has the following non-silent SNPs (Single Nucleotide Polymorphisms) as listed in Table 43, (given according to their position(s) on the amino acid sequence, with the alternative amino acid(s) listed).

**Table 43 - Amino acid mutations**

| **SNP position(s) on amino acid sequence** | **Alternative amino acid(s)** |
|---|---|
| 137 | E → D |

The coding portion of transcript W38346_T5 (SEQ ID NO:41) starts at position 233 and ends at position 664. The transcript also has the following SNPs as listed in Table 44 (given according to their position on the nucleotide sequence, with the alternative nucleic acid listed).

**Table 44 - Nucleic acid SNPs**

| **Polymorphism** | **SNP position(s) on nucleotide sequence** |
|---|---|
| C → T | 511 |
| A → C | 643 |
| G → | 701 |
| A → G | 703 |
| T → A | 738 |

### EXAMPLE 6_2: EXPRESSION ANALYSIS OF TMEM154 TRANSCRIPTS

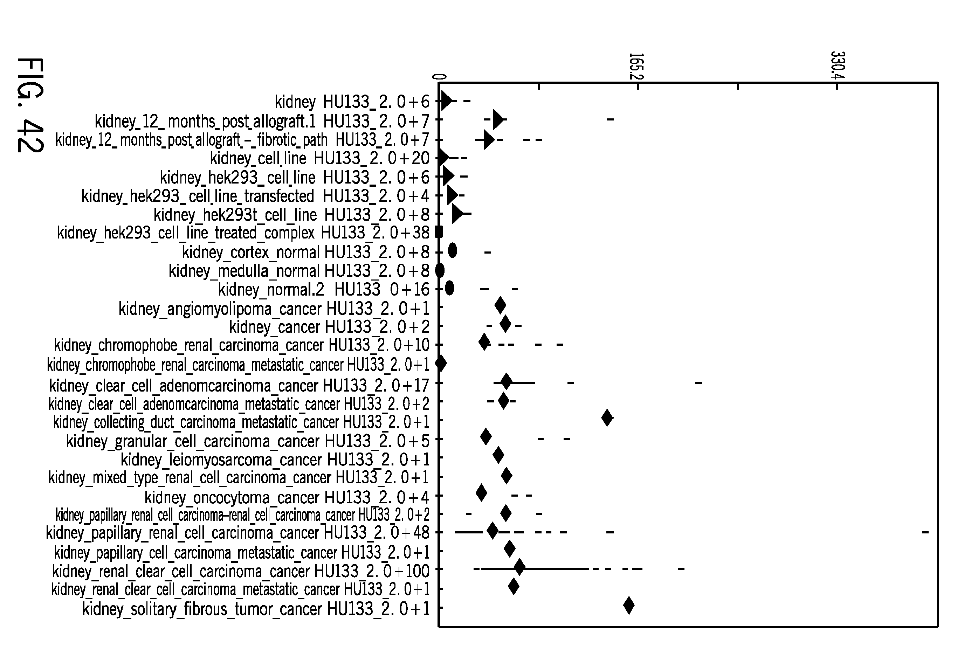

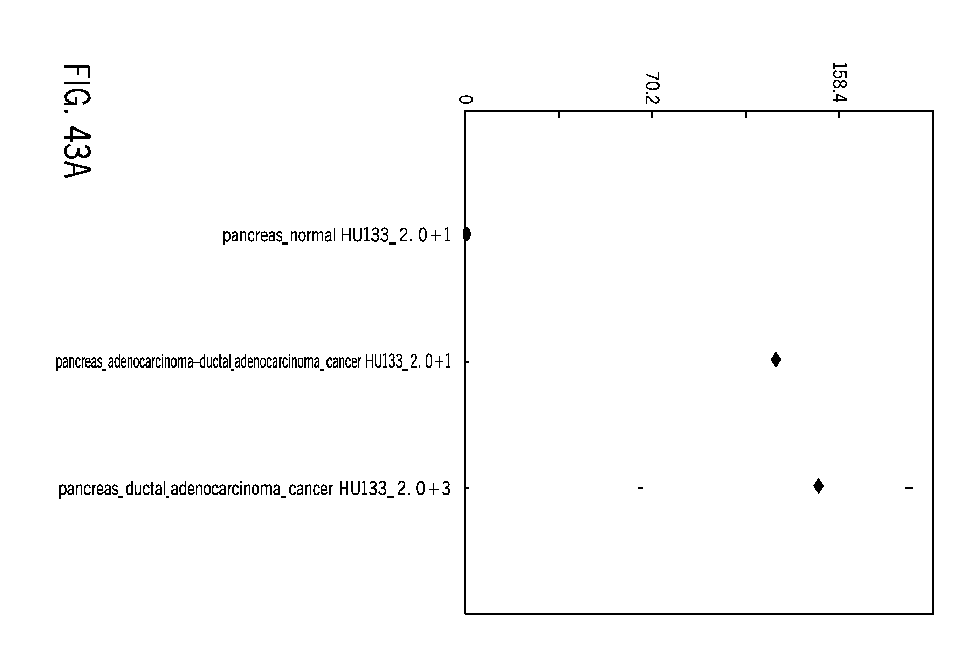

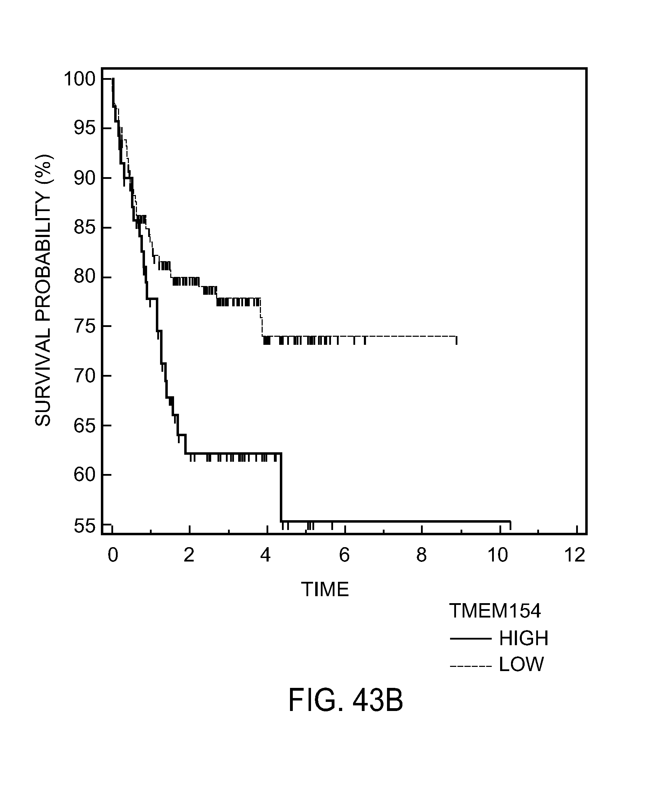

TMEM154 transcripts were also found to be over expressed in kidney cancer, as is demonstrated in Figure 42 and in pancreatic cancer, as is demonstrated in Figure 43. Figures 42-43 show expression graphs of Affymetrix probe set 238063_at. Figure 42 shows the expression of TMEM154 transcripts in microarray chips from kidney cancer and normal kidney experiments. As can be seen TMEM154 transcripts are overexpressed in kidney cancer tissues (diamond markers) relative to its expression in normal kidney (circle and triangle markers).

Figure 43A shows the expression of TMEM154 transcripts in microarray chips from pancreatic cancer and normal pancreas experiments. As can be seen TMEM154 transcripts are overexpressed in pnacreatic cancer tissues (diamond markers).

Figure 43B shows Kaplan-Meier survival curves of the high expression of the TMEM154 probe (238063_at, samples over median expression), and low expression (below median) in a samples from Rituximab treated DLBCL (Diffuse large B-cell lymphoma). It is evident that TMEM154 high expression is corelated with poor survival, and hance can serve as a potential treatment for anti CD20 resistant lymphoma paetients. In Figure 43B the time scale is shown in years; solid line represents high TMEM154 expression; fragmented line represents low TMEM154 expression.

### Expression of hypothetical protein FLJ32028, TMEM154 W38346 transcripts which are detectable by amplicon as depicted in sequence name W38346_seg6-20F1R1 (SEQ ID NO: 106) in different normal tissues and blood specific panel

Expression of hypothetical protein FLJ32028, TMEM154 transcripts detectable by or according to seg6-20F1R1 - W38346_seg6-20F1R1 (SEQ ID NO: 106) amplicon and primers W38346_seg6-20F (SEQ ID NO: 104) and W38346_seg6-20R (SEQ ID NO: 105) was measured by real time PCR in blood panel and normal panel. The samples used for blood panel are detailed in Tables 2 and 2_1. The samples used for normal panel are detailed in Table 3.

### Normal panel -

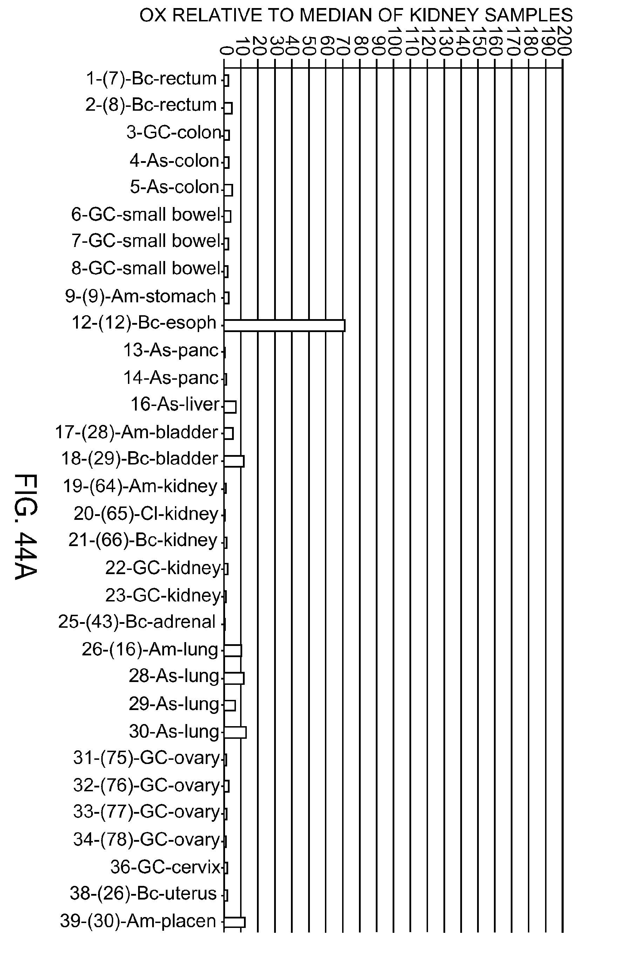

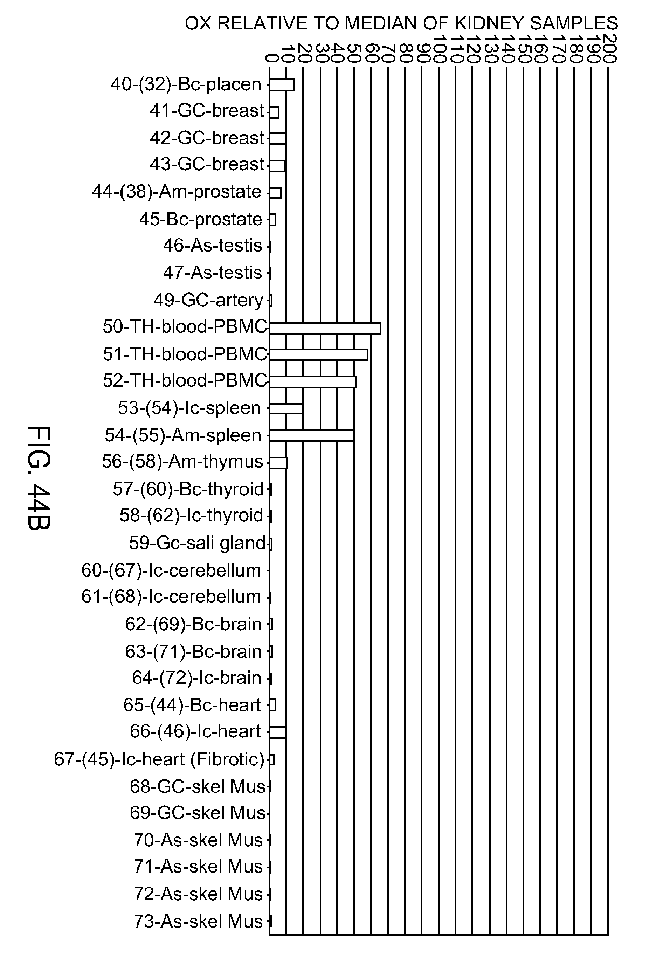

For each RT sample, the expression of the above amplicon was normalized to the normalization factor calculated from the expression of several house keeping genes as described in example 1. The normalized quantity of each RT sample was then divided by the median of the quantities of the kidney samples (sample numbers 19-23, Table 3 above), to obtain a value of relative expression of each sample relative to median of the kidney samples, as shown in Figure 44. High expression was observed in normal PBMCs, spleen and esophagus.

For blood panel - For each RT sample, the expression of the above amplicon was normalized to the normalization factor calculated from the expression of several house keeping genes as described in example 1. The normalized quantity of each RT sample was then divided by the median of the quantities of the kidney normal samples (sample numbers 65-67, Table 2 above), to obtain a value of relative expression of each sample relative to median of the kidney normal samples.

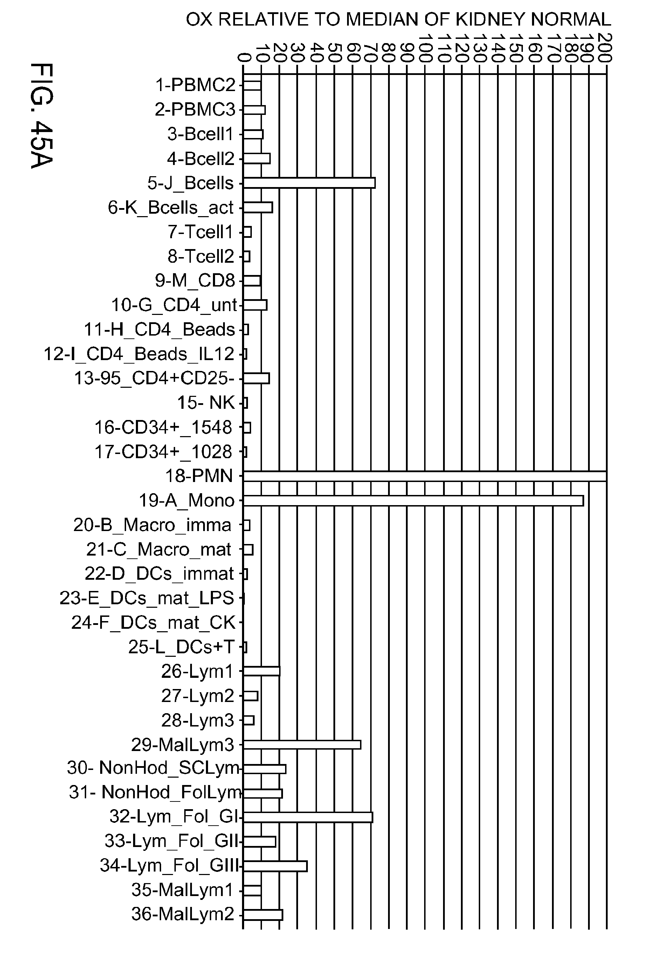

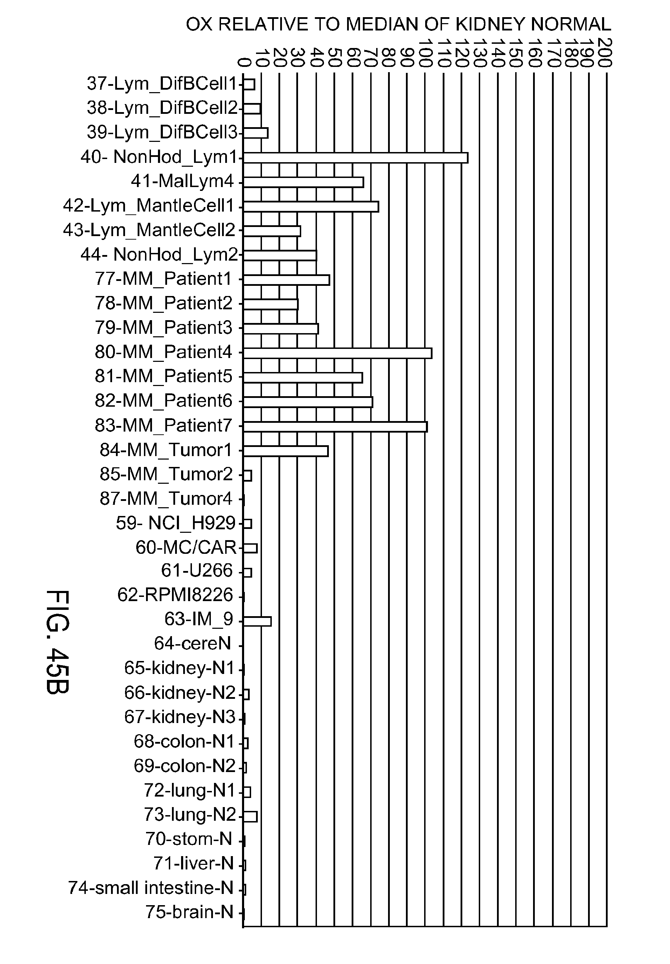

The results of this analysis are depicted in the histogram in Figure 45. Expression of the above-indicated hypothetical protein FLJ32028, TMEM154 transcripts is high in PMN, monocytes, multiple myeloma patients and several lymphomas samples.
Forward Primer (W38346_seg6-20F) (SEQ ID NO:104):
CCTTCTAGCCAAGGATCTCAGAGTG
Reverse Primer (W38346_seg6-20R) (SEQ ID NO: 105):
CTTGGGTTGTGATTTGATTCCTTCTC

Amplicon (W38346_seg6-20F1R1 (SEQ ID NO: 106)) (SEQ ID NO:106):

CCTTCTAGCCAAGGATCTCAGAGTGCTTTACAGACATATGAACTGGGAAG
TGAAAACGTGAAAGTCCCTATTTTTGAGGAAGATACACCCTCTGTTATGGAAAT
TGAAATGGAAGAGCTTGATAAATGGATGAACAGCATGAATAGAAATGCCGACT
TTGAATGTTTACCTACCTTGAAGGAAGAGAAGGAATCAAATCACAACCCAAG

### EXAMPLE 6_3: CLONING OF CGEN928 TMEM154_T0P3 ORF FUSED TO FLAG TAG

Cloning of TMEM154_T0_P3 open reading frame (ORF) fused to FLAG was carried out by RT PCR as described below.

RT18 PMNs (RT-PCR product resulted from sample 18, Table 2) and RT19 monocytes (RT-PCR product resulted from sample 19, Table 2) from the blood panel were diluted 1:20 in TE buffer (10mM Tris, 1 mM EDTA pH 8) and served as a template for PCR.

PCR was done using GoTaq ReadyMix (Promega, catalog number M122) under the following conditions: 10□µl - cDNA described above; 1.5 □µl - H2O; and 0.5µl (10µM) - of each primer #100-952 (SEQ ID NO:187) and #100-953 (SEQ ID NO:188) in a total reaction volume of 25µl; with a reaction program of 2 minutes in 94°C; 35 cycles of: 30 seconds at 94°C, 30 seconds at 55°C, 1 minute at 72°C; then 10 minutes at 72°C. Primers which were used include gene specific sequences; restriction enzyme sites; Kozak sequence and FLAG tag.

5µl of PCR product were loaded onto a 1.2% agarose gel stained with ethidium bromide, electrophoresed in 1xTAE solution at 100V, and visualized with UV light. After verification of expected band size, PCR product was purified using QiaQuick™ PCR Purification kit (Qiagen, catalog number: 28004). The purified PCR product was digested with NheI and AgeI restriction enzymes (New England Biolabs, Beverly, MA, U.S.A.). After digestion, DNA was loaded onto a 1.2 % agarose gel as described above. The expected band size was excised and extracted from the gel using QiaQuick™ Gel Extraction kit (Qiagen, catalog number: 28707). The digested DNA was then ligated into pIRESpuro3 vector, previously digested with the above restriction enzymes, using LigaFastTM Rapid DNA Ligation System (Promega, catalog number: M8221). The resulting DNA was transformed into competent E.Coli bacteria DH5α (RBC Bioscience, Taipei, Taiwan, catalog number: RH816) according to manufacturer's instructions, then plated on LB-ampicillin agar plates for selection of recombinant plasmids, and incubated overnight at 37°C. The following day, positive colonies were screened by PCR using pIRESpuro3 vector specific primer and gene specific primer (data not shown). The PCR product was analyzed using 1.2% agarose gel as described above. After verification of expected band size, positive colonies were grown in 5 ml Terrific Broth supplemented with 100µg/ml ampicillin, with shaking overnight at 37°C. Plasmid DNA was isolated from bacterial cultures using Qiaprep™ Spin Miniprep Kit (Qiagen, catalog number: 27106). Accurate cloning was verified by sequencing the inserts (Weizmann Institute, Rehovot, Israel). Upon verification of an error-free colony (i.e. no mutations within the ORF), recombinant plasmids were processed for further analyses.

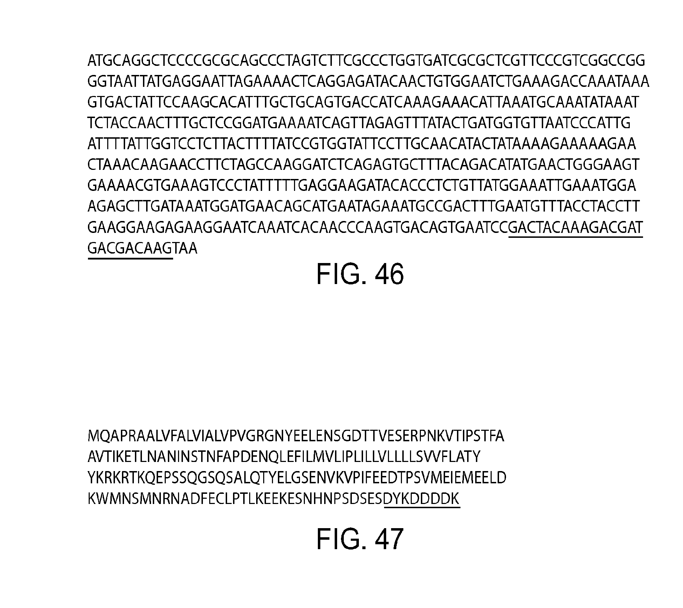

The DNA sequence of the resulting TMEM154_T0_FLAG (SEQ ID NO:189) is shown in Figure 46; FLAG sequence is in underlined.

The amino acid sequence of TMEM154_P3_FLAG (SEQ ID NO:190) is shown in Figure 47; FLAG sequence is in underlined.

### EXAMPLE 6_4: DETERMINING CELL LOCALIZATION OF TMEM154_P3

In order to determine **TMEM154_P3** cellular localization, TMEM154_T0_P3 was cloned in frame to FLAG tag, as described above. Protein localization was observed upon transient transfection (Chen et al., Molecular Vision 2002; 8; 372-388) using confocal microscopy. 48 hours following transfection, the cells were stained with anti FLAG antibodies conjugated to Cy-3 flurophore and were observed for the presence of fluorescent signal.

TMEM154_T0_P3_FLAG (SEQ ID NO:189) pIRESpuro3 construct was transiently transfected into HEK-293T cells as described above. 48 hours post transient transfection, cells on coverslip were further processed for immunostaining and analysis by confocal microscopy. The cover slip was washed in phosphate buffered saline (PBS), then fixed for 15 minutes with a solution of 3.7% paraformaldehyde (PFA) (Sigma, catalog number: P-6148)/3% glucose (Sigma, catalog number: G5767) (diluted in PBS). Quenching of PFA was done by a 5 minute incubation in 3mM glycine (Sigma, catalog number: G7126) (diluted in PBS). After two 5-minute washes in PBS, cells were permeabilized with 0.1% triton-X100 (diluted in PBS) for 5 minutes. After two 5-minute washes in PBS, blocking of non-specific regions was done with 5% bovine serum albumin (BSA) (Sigma, catalog number: A4503) (diluted in PBS) for 20 minutes. The coverslip was then incubated, in a humid chamber for 1 hour, with mouse anti FLAG-Cy3 antibodies (Sigma, catalog number: A9594), diluted 1:100 in 5% BSA in PBS, followed by three 5-minute washes in PBS. The coverslip was then mounted on a slide with Gel Mount Aqueous medium (Sigma, catalog number: G0918) and cells were observed for the presence of fluorescent product using confocal microscopy.

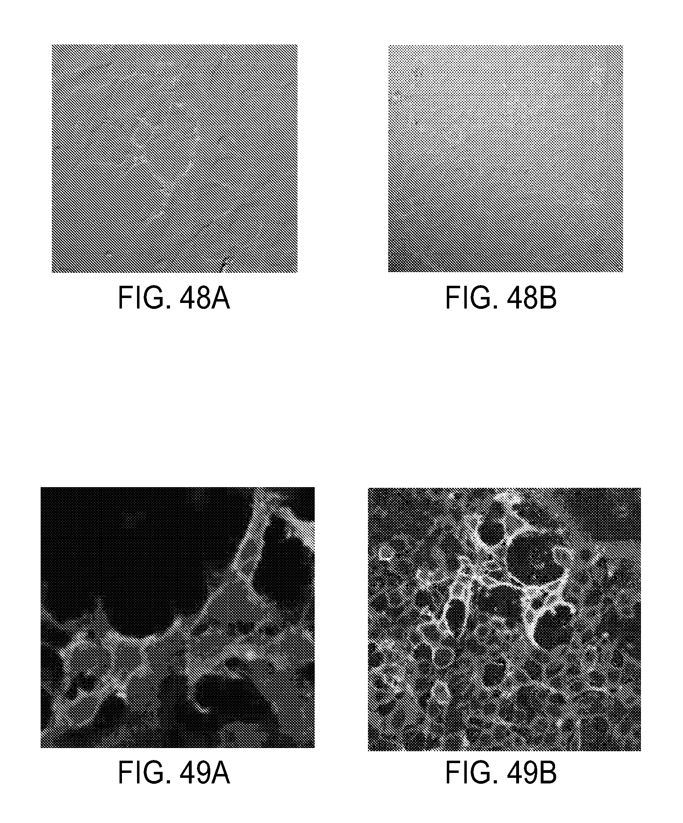

Cell localization is shown in Figure 48. **TMEM154_P3** is localized to the cell membrane.

### EXAMPLE 6_5: PRODUCTION OF POLYCLONAL ANTIBODIES SPECIFIC TO TMEM154_P3 PROTEIN

All polyclonal Abs production procedure, including peptides synthesis, peptides conjugation, animal immunizations, bleeding and antibodies purification were performed at Sigma-Aldrich (Israel). Two pairs of rabbits (one pair per epitope) were injected to prepare antibodies for **TMEM154_P3** (rabbit numbers 6285 and 6286, 6248 and 6249 respectively). All animal care, handling and injections were performed by Sigma (Israel).

Peptides which were used for rabbit immunization were as follows: RGNYEELENSGDTTVESER designated TM21 (SEQ ID NO:191) a sequence taken from the N' terminus corresponding to amino acids 21-39 of **TMEM154_P3** protein (SEQ ID NO:42). The second peptide sequence to be used was: YKRKRTKQEPSSQGSQS designated TM101 (SEQ ID NO:192), a sequence taken from the C' terminus, corresponding to amino acids 101-117 of TMEM154_P3 protein (SEQ ID NO:42). 25mg of each peptide were synthesized with 95% purity of which 10mg were conjugated to KLH carrier. Each pair of rabbits was immunized with the corresponding conjugated peptide as follows: rabbits 6285 and 6286 were immunized with TM21 peptide (SEQ ID NO:191), and rabbits 6248 and 6249 were immunized with TM101 peptide (SEQ ID NO:192). Animals were immunized every two weeks. 60ml production bleeds from each rabbit were collected and affinity purification was performed with the peptide against which the respective antibodies were raised.

### EXAMPLE 6_6: CHARACTERIZATION OF PURIFIED TMEM154_P3 ANTIBODIES BY IMMUNOSTAINING OF TMEM154 TRANSFECTED CELLS

In order to further characterize the affinity purified antibodies raised against TMEM154_P3, antibody-protein interaction was studied using immunostaining of **TMEM154_P3** stable transfected HEK293T cells.

### GENERATION OF STABLE POOL EXPRESSING TMEM154_P3 PROTEIN:

Two stably transfected pool were generated, **TMEM154_P3** pIRESpuro3 and the negative control empty pIRESpuro3. Both constructs were transfected into HEK-293T cells as previously described.

### IMMUNOSTAINING OF TMEM154 TRANSFECTED CELLS

500,000 cells per well of HEK-293T (ATCC, CRL-11268) stably expressing TMEM154 or the empty vector pIRES puro3, described above, were plated on sterile glass coverslips, 13mm diameter (Marienfeld, catalog number: 01 115 30), which were placed in a 6 well plate, using 2ml pre-warmed DMEM [Dulbecco's modified Eagle's Media, Biological Industries (Beit Ha'Emek, Israel), catalog number: 01-055-1A] + 10% FBS [Fetal Bovine Serum, Biological Industries (Beit Ha'Emek, Israel), catalog number: 04-001-1A] + 4mM L-Glutamine [Biological Industries (Beit Ha'Emek, Israel), catalog number: 03-020-1A].

48 hours post plating the cells on coverslips they were further processed for immunostaining and analysis by confocal microscopy. The cover slips were washed in phosphate buffered saline (PBS), then fixed for 25 minutes with a 3.7% paraformaldehyde (PFA) (Sigma, catalog number: P-6148)/3% glucose (Sigma, catalog number: G5767). After 2 5-minute washes in PBS, cells were permeabilized with 0.1% triton-X100 (diluted in PBS) for 5 minutes. After two 5-minute washes in PBS, blocking of non-specific regions was done with 5% bovine serum albumin (BSA) (Sigma, catalog number: A4503) (diluted in PBS) for 20 minutes. The coverslips were then incubated, in a humid chamber for 1 hour, with purified rabbit anti-TMEM154 antibodies described above: TM21 (Rabbit 6285, 6286 1mg/ml) was diluted 1:1000 in 5% BSA in PBS and TM101 (Rabbit 6248 6249, 1mg/ml) was diluted 1:1000 in 5% BSA. The antibodies were washed 3 times for 5-minutes in PBS. The coverslips were then incubated, in a humid chamber for 1 hour, with secondary antibody: donkey anti-rabbit conjugated to Cy-3 flurophore (Jackson ImmunoResearch, catalog number: 711-165-152), diluted 1:200 in 3% BSA in PBS. After 3 5-minute washes in PBS, the fixed coverslips were mounted on slides with Gel Mount Aqueous medium (Sigma, catalog number: G0918) and cells were observed for the presence of fluorescent product using confocal microscopy.

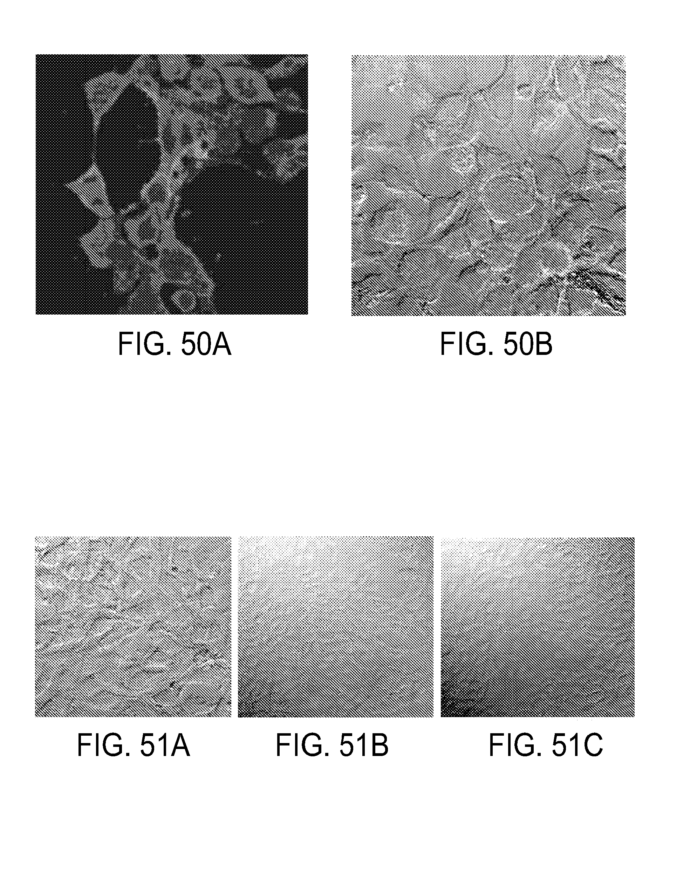

Specific cell staining localized to the cell membrane was observed using purified TM21 and TM101 antibodies on TMEM154 transfected cells (Figure 49 and Figure 50 respectively), while, no staining was observed using these antibodies on the negative control pIRESpuro3 HEK-293T transfected cells (Figure 51). The red fluorescence obtained in Figures 49 and 50 as opposed to the absence of signal in Figure 51 demonstrates the specificity of TM21 and TM101 antibodies to TMEM154_P3 (SEQ ID NO:42).

### EXAMPLE 6_7: DEMONSTRATION OF ENDOGENOUS EXPRESSION OF TMEM154_P3 BY IMMUNOSTAINING OF LYMPHOBLAST CELL LINES

In order to determine endogenous expression of TMEM154, three cell lines were selected for immustaining using specific antibodies against TMEM154_P3 protein described above.

500000 cells from each cell line: Ramos (ATCC cat no CRL-1923), CESS (ATCC cat no TIB-190), Daudi (ATCC cat no CCL-213) were fixed with 3.7% PFA containing 3% Glucose and plated on coverslips previously treated with poly-L-Lysin 0.01% (Sigma cat no P4832). Cells were further processed for immunostaining as describe above and analyzed by confocal microscopy.

Specific cell staining localized to the cell membrane was observed using purified TM21 and TM101 antibodies on all three cell lines as shown in Figure 52.

### EXAMPLE 7

### DEVELOPMENT OF FULLY HUMAN ANTI-KRTCAP3, ANTI-FAM26F, ANTI-MGC52498, ANTI-FAM70A AND ANTI-TMEM154 ANTIBODIES

Generation Of Human Monoclonal Antibodies Against KRTCAP3, FAM26F, MGC52498, FAM70A and TMEM154 Antigen

Fusion proteins composed of the extracellular domain of the KRTCAP3, FAM26F, MGC52498, FAM70A and TMEM154 linked to an IgG2 Fc polypeptide are generated by standard recombinant methods and used as antigen for immunization.

### Transgenic HuMab Mouse.

Fully human monoclonal antibodies to KRTCAP3, FAM26F, MGC52498, FAM70A and TMEM154 are prepared using mice from the HCo7 strain of the transgenic HuMab Mouse®, which expresses human antibody genes. In this mouse strain, the endogenous mouse kappa light chain gene has been homozygously disrupted as described in Chen et al. (1993) EMBO J. 12:811-820 and the endogenous mouse heavy chain gene has been homozygously disrupted as described in Example 1 of PCT Publication WO 01/09187. Furthermore, this mouse strain carries a human kappa light chain transgene, KCo5, as described in Fishwild et al. (1996) Nature Biotechnology 14:845-851, and a human heavy chain transgene, HCo7, as described in U.S. Pat. Nos. 5,545,806; 5,625,825; and 5,545,807.

### HuMab Immunizations:

To generate fully human monoclonal antibodies to KRTCAP3, FAM26F, MGC52498, FAM70A and TMEM154 polypeptides, mice of the HCo7 HuMab Mouse® strain can be immunized with purified recombinant KRTCAP3, FAM26F, MGC52498, FAM70A or TMEM154 fusion protein derived from mammalian cells that are transfected with an expression vector containing the gene encoding the fusion protein. General immunization schemes for the HuMab Mouse® are described in Lonberg, N. et al (1994) Nature 368(6474): 856-859; Fishwild, D. et al. (1996) Nature Biotechnology 14: 845-851 and PCT Publication WO 98/24884. The mice are 6-16 weeks of age upon the first infusion of antigen. A purified recombinant KRTCAP3, FAM26F, MGC52498, FAM70A or TMEM154 antigen preparation (5-50 micro-grams, purified from transfected mammalian cells expressing KRTCAP3, FAM26F, MGC52498, FAM70A or TMEM154 fusion protein) is used to immunize the HuMab mice intraperitoneally.

Transgenic mice are immunized twice with antigen in complete Freund's adjuvant or Ribi adjuvant IP, followed by 3-21 days IP (up to a total of 11 immunizations) with the antigen in incomplete Freund's or Ribi adjuvant. The immune response is monitored by retroorbital bleeds. The plasma is screened by ELISA (as described below), and mice with sufficient titers of anti- KRTCAP3, anti-FAM26F, anti-MGC52498, anti-FAM70A or anti-TMEM154 human immunoglobulin are used for fusions. Mice are boosted intravenously with antigen 3 days before sacrifice and removal of the spleen.

### Selection of HuMab mice® Producing Anti- KRTCAP3, Anti-FAM26F, Anti-MGC52498, Anti-FAM70A or Anti-TMEM154 Antibodies:

To select HuMab mice® producing antibodies that bind KRTCAP3, FAM26F, MGC52498, FAM70A or TMEM154 polypeptides, sera from immunized mice is tested by a modified ELISA as originally described by Fishwild, D. et al. (1996). Briefly, microtiter plates are coated with purified recombinant KRTCAP3, FAM26F, MGC52498, FAM70A or TMEM154 fusion protein at 1-2.mu.g/ml in PBS, 50.mu.l/wells incubated 4 degrees C. overnight then blocked with 200.mu.l/well of 5% BSA in PBS. Dilutions of plasma from KRTCAP3, FAM26F, MGC52498, FAM70A or TMEM154 -immunized mice are added to each well and incubated for 1-2 hours at ambient temperature. The plates are washed with PBS/Tween and then incubated with a goat-anti-human kappa light chain polyclonal antibody conjugated with alkaline phosphatase for 1 hour at room temperature. After washing, the plates are developed with pNPP substrate and analyzed by spectrophotometer at OD 415-650. Mice that developed the highest titers of anti-KRTCAP3, anti-FAM26F, anti-MGC52498, anti-FAM70A or anti-TMEM154 antibodies are used for fusions. Fusions are performed as described below and hybridoma supernatants are tested for anti-KRTCAP3, anti-FAM26F, anti-MGC52498, anti-FAM70A or anti-TMEM154 activity by ELISA.

### Generation Of Hybridomas Producing Human Monoclonal Antibodies To KRTCAP3, FAM26F, MGC52498, FAM70A or TMEM154 polypeptides

The mouse splenocytes, isolated from the HuMab mice, are fused with PEG to a mouse myeloma cell line based upon standard protocols. The resulting hybridomas are then screened for the production of antigen-specific antibodies. Single cell suspensions of splenic lymphocytes from immunized mice are fused to one-fourth the number of P3X63 Ag8.6.53 (ATCC CRL 1580) nonsecreting mouse myeloma cells with 50% PEG (Sigma). Cells are plated at approximately 1X10 -5 /well in flat bottom microtiter plate, followed by about two week incubation in selective medium containing 10% fetal calf serum, supplemented with origen (IGEN) in RPMI, L-glutamine, sodium pyruvate, HEPES, penicillin, streptamycin, gentamycin, 1x HAT, and beta-mercaptoethanol. After 1-2 weeks, cells are cultured in medium in which the HAT is replaced with HT. Individual wells are then screened by ELISA (described above) for human anti-KRTCAP3, anti-FAM26F, anti-MGC52498, anti-FAM70A or anti-TMEM154 monoclonal IgG antibodies. Once extensive hybridoma growth occurred, medium is monitored usually after 10-14 days. The antibody secreting hybridomas are replated, screened again and, if still positive for human IgG, anti-KRTCAP3, anti-FAM26F, anti-MGC52498, anti-FAM70A or anti-TMEM154 monoclonal antibodies are subcloned at least twice by limiting dilution. The stable subclones are then cultured in vitro to generate small amounts of antibody in tissue culture medium for further characterization.

### Hybridoma clones are selected for further analysis.

### Structural Characterization Of Desired anti-KRTCAP3, anti-FAM26F, anti-MGC52498, anti-FAM70A or anti-TMEM154Human Monoclonal Antibodies

The cDNA sequences encoding the heavy and light chain variable regions of the obtained anti-KRTCAP3, anti-FAM26F, anti-MGC52498, anti-FAM70A or anti-TMEM154 monoclonal antibodies are obtained from the resultant hybridomas, respectively, using standard PCR techniques and are sequenced using standard DNA sequencing techniques.

The nucleotide and amino acid sequences of the heavy chain variable region and of the light chain variable region are identified. These sequences may be compared to known human germline immunoglobulin light and heavy chain sequences and the CDRs of each heavy and light of the obtained anti-KRTCAP3, anti-FAM26F, anti-MGC52498, anti-FAM70A or anti-TMEM154 sequences identified.

### Characterization Of Binding Specificity And Binding Kinetics Of anti-KRTCAP3, anti-FAM26F, anti-MGC52498, anti-FAM70A or anti-TMEM154 Human Monoclonal Antibodies

The binding affinity, binding kinetics, binding specificity, and cross-competition of anti-KRTCAP3, anti-FAM26F, anti-MGC52498, anti-FAM70A or anti-TMEM154 antibodies are examined by Biacore analysis. Also, binding specificity is examined by flow cytometry.

### Binding affinity and kinetics

Anti-KRTCAP3, anti-FAM26F, anti-MGC52498, anti-FAM70A or anti-TMEM154 antibodies produced according to the invention are characterized for affinities and binding kinetics by Biacore analysis (Biacore AB, Uppsala, Sweden). Purified recombinant human KRTCAP3, FAM26F, MGC52498, FAM70A or TMEM154 fusion protein is covalently linked to a CM5 chip (carboxy methyl dextran coated chip) via primary amines, using standard amine coupling chemistry and kit provided by Biacore. Binding is measured by providing the antibodies in HBS EP buffer (provided by BIAcore AB) at a concentration of 267 nM and a flow rate of 50.mu.l/min. The antigen- antibody association kinetics are followed for 3 minutes and the dissociation kinetics are followed for 7 minutes. The association and dissociation curves are fit to a 1:1 Langmuir binding model using BlAevaluation software (Biacore AB). To minimize the effects of avidity in the estimation of the binding constants, only the initial segments of data corresponding to association and dissociation phases are used for fitting.

### Epitope Mapping of Obtained anti-KRTCAP3, anti-FAM26F, anti-MGC52498, anti-FAM70A or anti-TMEM154 Antibodies

Biacore is used to determine epitope grouping of anti-KRTCAP3, anti-FAM26F, anti-MGC52498, anti-FAM70A or anti-TMEM154 antibodies are used to map their epitopes on the KRTCAP3, FAM26F, MGC52498, FAM70A or TMEM154 antigen, respectively. These different antibodies are coated on three different surfaces of the same chip to 8000 RUs each. Dilutions of each of the mAbs are made, starting at 10 mu.g/mL and is incubated with Fc fused KRTCAP3, FAM26F, MGC52498, FAM70A or TMEM154 (50 nM) for one hour. The incubated complex is injected over all the three surfaces (and a blank surface) at the same time for 1.5 minutes at a flow rate of 20.mu.L/min. Signal from each surface at end of 1.5 minutes, after subtraction of appropriate blanks, has been plotted against concentration of mAb in the complex. Upon analysis of the data, the anti-KRTCAP3, anti-FAM26F, anti-MGC52498, anti-FAM70A or anti-TMEM154 antibodies are categorized into different epitope groups depending on the epitope mapping results. The functional properties thereof are also compared.

Chinese hamster ovary (CHO) cell lines that express KRTCAP3, FAM26F, MGC52498, FAM70A or TMEM154 protein at the cell surface are developed and used to determine the specificity of the KRTCAP3, FAM26F, MGC52498, FAM70A or TMEM154 HuMAbs by flow cytometry. CHO cells are transfected with expression plasmids containing full length cDNA encoding transmembrane forms of KRTCAP3, FAM26F, MGC52498, FAM70A or TMEM154 antigen or a variant thereof. The transfected proteins contained an epitope tag at the N-terminus are used for detection by an antibody specific for the epitope. Binding of an anti-KRTCAP3, anti-FAM26F, anti-MGC52498, anti-FAM70A or anti-TMEM154 MAb is assessed by incubating the transfected cells with each of the KRTCAP3, FAM26F, MGC52498, FAM70A or TMEM154 antibodies at a concentration of 10 micro-grams/ml. The cells are washed and binding is detected with a FITC-labeled anti-human IgG Ab. A murine anti-epitope tag Ab, followed by labeled anti-murine IgG, is used as the positive control. Non-specific human and murine Abs are used as negative controls. The obtained data is used to assess the specificity of the HuMAbs for the KRTCAP3, FAM26F, MGC52498, FAM70A or TMEM154 antigen target.

These antibodies and other antibodies specific to KRTCAP3, FAM26F, MGC52498, FAM70A or TMEM154 polypeptides may be used in the afore-described anti-KRTCAP3, anti-FAM26F, anti-MGC52498, anti-FAM70A or anti-TMEM154 related therapies such as treatment of cancers wherein KRTCAP3, FAM26F, MGC52498, FAM70A or TMEM154 antigen is differentially expressed, such as ovarian cancer, lung cancer, breast cancer, kidney cancer, liver cancer, pancreatic cancer, prostate cancer, melanoma and hematological malignancies such as Multiple Myeloma, lymphoma, Non-Hodgkin's lymphoma, anti CD20 (i.e. Rituximab) resistant lymphoma, leukemia and T cell leukemia, involving the KRTCAP3, FAM26F, MGC52498, FAM70A or TMEM154 antigen, such as in the treatment of cancers and inflammatory or autoimmune diseases wherein such antibodies will e.g., prevent negative stimulation of T cell activity against desired target cancer cells or prevent the positive stimulation of T cell activity thereby eliciting a desired anti-autoimmune effect.

The invention has been described and prophetic embodiments provided relating to manufacture and selection of desired anti-KRTCAP3, anti-FAM26F, anti-MGC52498, anti-FAM70A or anti-TMEM154 antibodies for use as therapeutics and diagnostic methods wherein the disease or condition is associated with KRTCAP3, FAM26F, MGC52498, FAM70A or TMEM154 antigen. The invention is now further described by the claims which follow.

## Claims

1. A polyclonal or monoclonal antibody or antibody binding fragment that specifically binds to a FAM26F polypeptide selected from the group consisting of SEQ ID NO: 15-18, or fragment thereof.

2. The antibody or fragment of claim 1, wherein said antibody or fragment blocks or inhibits the interaction of the FAM26F polypeptide with a counterpart by specifically binding to an amino acid sequence as set forth in any one of SEQ ID NOs 15-18, 52, 53, 117, 118, 127, or 149.

3. The antibody or fragment according to claims 1 or 2, adapted for treatment and/or diagnosis of cancer or an immune related condition, disease or disorder.

4. The antibody or fragment according to claim 3, wherein said cancer is selected from the group consisting of: ovarian cancer, breast cancer, prostate cancer, acute lymphocytic leukemia, chronic lymphocytic leukemia, acute myelogenous leukemia, chronic myelogenous leukemia, multiple myeloma, Hodgkin's lymphoma or Non-Hodgkin's lymphoma; or
wherein said immune related condition, disease or disorder is selected from the group consisting of: multiple sclerosis; psoriasis; rheumatoid arthritis; psoriatic arthritis; systemic lupus erythematosus (SLE); ulcerative colitis; Crohn's disease; benign lymphocytic angiitis; thrombocytopenic purpura; idiopathic thrombocytopenia; idiopathic autoimmune hemolytic anemia; pure red cell aplasia; Sjogren's syndrome; rheumatic disease; connective tissue disease; inflammatory rheumatism; degenerative rheumatism; extra-articular rheumatism; juvenile rheumatoid arthritis; arthritis uratica; muscular rheumatism; chronic polyarthritis; cryoglobulinemic vasculitis; ANCA-associated vasculitis; antiphospholipid syndrome; myasthenia gravis; autoimmune haemolytic anaemia; Guillian-Barre syndrome; chronic immune polyneuropathy; autoimmune thyroiditis; insulin dependent diabetes mellitus; type I diabetes; Addison's disease; membranous glomerulonephropathy; Goodpasture's disease; autoimmune gastritis; pernicious anaemia; pemphigus vulgarus; cirrhosis; primary biliary cirrhosis; dermatomyositis; polymyositis; fibromyositis; myogelosis; celiac disease; immunoglobulin A nephropathy; Henoch-Schonlein purpura; Evans syndrome; atopic dermatitis; psoriasis arthropathica; Graves' disease; Graves' ophthalmopathy; scleroderma; systemic scleroderma; asthma; allergy; Hashimoto's thyroiditis; primary myxedema; sympathetic ophthalmia; autoimmune uveitis; hepatitis; chronic action hepatitis; collagen diseases; ankylosing spondylitis; periarthritis humeroscapularis; panarteritis nodosa; chondrocalcinosis; Wegener's granulomatosis; microscopic polyangiitis; chronic urticaria; bullous skin disorders; pemphigoid; atopic eczema; Devic's disease; childhood autoimmune hemolytic anemia; refractory or chronic autoimmune cytopenias; prevention of development of autoimmune anti-factor VIII antibodies in acquired hemophilia A; cold agglutinin disease; neuromyelitis optica; stiff person syndrome; gingivitis; periodontitis; pancreatitis; myocarditis; vasculitis; gastritis; gout; gouty arthritis; inflammatory skin disorders selected from the group consisting of psoriasis, atopic dermatitis, eczema, rosacea, urticaria, and acne; normocomplementemic urticarial vasculitis; pericarditis; myositis; anti-synthetase syndrome; scleritis; macrophage activation syndrome; Bechet's Syndrome; PAPA Syndrome; Blau's Syndrome; adult and juvenile Still's disease; cryropyrinopathy; Muckle-Wells syndrome; familial cold-induced auto-inflammatory syndrome; neonatal onset multisystemic inflammatory disease; familial Mediterranean fever; chronic infantile neurologic, cutaneous and articular syndrome; systemic juvenile idiopathic arthritis; hyper IgD syndrome; Schnitzler's syndrome; TNF receptor-associated periodic syndrome (TRAPS); inflammatory bowel disease; Good pasture's syndrome; pernicious anemia; autoimmune atrophic gastritis; ulceratis colitis; mixed connective tissue disease; panarteuitis nodosa; progressive systemic scleroderma; peptic ulcers; ulcers; chronic bronchitis; acute lung injury; pulmonary inflammation; airway hyper-responsiveness; septic shock; inflammatory skin disorders, myogelosis; chondrocalcinosis; thyroditis; allergic oedema; granulomas; and immune disorders associated with graft transplantation rejection including acute and chronic rejection of organ transplantation, allogenic stem cell transplantation, autologous stem cell transplantation, bone marrow transplantation and graft versus host disease.

5. The antibody or fragment according to any of claims 1-4, wherein the antibody or fragment is selected from the group consisting of a fully human antibody, a humanized or primatized antibody, a chimeric antibody, Fab, Fab', F(ab')2, F(ab'), F(ab), Fv or scFv fragment and minimal recognition unit.

6. The antibody or fragment according to any of claims 1-5, wherein the antibody or fragment is coupled to a detectable marker or to an effector moiety.

7. The antibody or fragment according to claim 6, wherein the effector moiety is one or more of a radionuclide, fluorophore, an enzyme, a toxin, a therapeutic agent, a chemotherapeutic agent, a cytokine antibody, a cytokine receptor or an immunomodulatory agent, and wherein the detectable marker is one or more of a radioisotope, a metal chelator, an enzyme, a fluorescent compound, a bioluminescent compound or a chemiluminescent compound.

8. A pharmaceutical composition that comprises the antibody or fragment according to any one of claims 1 to 7.

9. The pharmaceutical composition of claim 8 for modulating the activity of a FAM26F protein as represented by SEQ ID NO: 15-18 in the treatment or prevention of a cancer or an immune related condition, disease or disorder.

10. The pharmaceutical composition of claim 9, wherein said cancer is selected from the group consisting of: ovarian cancer, breast cancer, prostate cancer, acute lymphocytic leukemia, chronic lymphocytic leukemia, acute myelogenous leukemia, chronic myelogenous leukemia, multiple myeloma, Hodgkin's lymphoma or Non-Hodgkin's lymphoma, or
wherein said immune related condition, disease or disorder is selected from the group consisting of: multiple sclerosis; psoriasis; rheumatoid arthritis; psoriatic arthritis; systemic lupus erythematosus (SLE); ulcerative colitis; Crohn's disease; benign lymphocytic angiitis; thrombocytopenic purpura; idiopathic thrombocytopenia; idiopathic autoimmune hemolytic anemia; pure red cell aplasia; Sjogren's syndrome; rheumatic disease; connective tissue disease; inflammatory rheumatism; degenerative rheumatism; extra-articular rheumatism; juvenile rheumatoid arthritis; arthritis uratica; muscular rheumatism; chronic polyarthritis; cryoglobulinemic vasculitis; ANCA-associated vasculitis; antiphospholipid syndrome; myasthenia gravis; autoimmune haemolytic anaemia; Guillian-Barre syndrome; chronic immune polyneuropathy; autoimmune thyroiditis; insulin dependent diabetes mellitus; type I diabetes; Addison's disease; membranous glomerulonephropathy; Goodpasture's disease; autoimmune gastritis; pernicious anaemia; pemphigus vulgarus; cirrhosis; primary biliary cirrhosis; dermatomyositis; polymyositis; fibromyositis; myogelosis; celiac disease; immunoglobulin A nephropathy; Henoch-Schonlein purpura; Evans syndrome; atopic dermatitis; psoriasis arthropathica; Graves' disease; Graves' ophthalmopathy; scleroderma; systemic scleroderma; asthma; allergy; Hashimoto's thyroiditis; primary myxedema; sympathetic ophthalmia; autoimmune uveitis; hepatitis; chronic action hepatitis; collagen diseases; ankylosing spondylitis; periarthritis humeroscapularis; panarteritis nodosa; chondrocalcinosis; Wegener's granulomatosis; microscopic polyangiitis; chronic urticaria; bullous skin disorders; pemphigoid; atopic eczema; Devic's disease; childhood autoimmune hemolytic anemia; refractory or chronic autoimmune cytopenias; prevention of development of autoimmune anti-factor VIII antibodies in acquired hemophilia A; cold agglutinin disease; neuromyelitis optica; stiff person syndrome; gingivitis; periodontitis; pancreatitis; myocarditis; vasculitis; gastritis; gout; gouty arthritis; inflammatory skin disorders selected from the group consisting of psoriasis, atopic dermatitis, eczema, rosacea, urticaria, and acne; normocomplementemic urticarial vasculitis; pericarditis; myositis; anti-synthetase syndrome; scleritis; macrophage activation syndrome; Bechet's Syndrome; PAPA Syndrome; Blau's Syndrome; adult and juvenile Still's disease; cryropyrinopathy; Muckle-Wells syndrome; familial cold-induced auto-inflammatory syndrome; neonatal onset multisystemic inflammatory disease; familial Mediterranean fever; chronic infantile neurologic, cutaneous and articular syndrome; systemic juvenile idiopathic arthritis; hyper IgD syndrome; Schnitzler's syndrome; TNF receptor-associated periodic syndrome (TRAPS); inflammatory bowel disease; Good pasture's syndrome; pernicious anemia; autoimmune atrophic gastritis; ulceratis colitis; mixed connective tissue disease; panarteriitis nodosa; progressive systemic scleroderma; peptic ulcers; ulcers; chronic bronchitis; acute lung injury; pulmonary inflammation; airway hyper-responsiveness; septic shock; inflammatory skin disorders, myogelosis; chondrocalcinosis; thyroditis; allergic oedema; granulomas; and immune disorders associated with graft transplantation rejection including acute and chronic rejection of organ transplantation, allogenic stem cell transplantation, autologous stem cell transplantation, bone marrow transplantation and graft versus host disease.

11. The pharmaceutical composition of any of claims 8-10, further comprising another medicament or therapy selected from the group consisting of radiation therapy, antibody therapy, chemotherapy, surgery, or in combination therapy with other biological agents, conventional drugs, anti-cancer agents, immunosuppressants, cytotoxic drugs, chemotherapeutic agents, or in combination with therapeutic agents targeting other complement regulatory proteins (CRPs), administered simultaneously or consecutively.

12. A method of diagnosing a cancer using the antibody or fragment of any one of claims 1 to 7, said method comprising detecting in a sample obtained from a subject the presence of a polypeptide and/or an over expressed level of said polypeptide having a sequence of a FAM26F polypeptide having an amino acid sequence consisting of SEQ ID NO: 15-18, or a fragment thereof,
wherein said over expressed level is determined with regard to a normal level of said polypeptide in a corresponding normal tissue and wherein said detecting is performed with an immunoassay.

13. The method of claim 12, wherein the cancer is selected from the group consisting of ovarian cancer, breast cancer, prostate cancer, acute lymphocytic leukemia, chronic lymphocytic leukemia, acute myelogenous leukemia, chronic myelogenous leukemia, multiple myeloma, Hodgkin's lymphoma or Non-Hodgkin's lymphoma.

14. An assay for detecting the presence of a polypeptide selected from the group consisting of SEQ ID NO: 15-18, or a fragment or variant thereof in a biological sample, said assay comprising contacting an isolated sample from a subject with the antibody or fragment according to any one of claims 1 to 7.

15. An antibody or an antibody fragment specifically binding to a FAM26F polypeptide for treatment of a cancer in a subject, wherein said cancer is selected from the group consisting of: ovarian cancer, prostate cancer, acute lymphocytic leukemia, chronic lymphocytic leukemia, acute myelogenous leukemia, chronic myelogenous leukemia, multiple myeloma, Hodgkin's lymphoma and Non-Hodgkin's lymphoma.
